# EUROPEAN PATENT SPECIFICATION

(11) **EP 4 222 151 B1**
(45) Date of publication and mention of the grant of the patent: **18.11.2026**
(21) Application number: 21871521.7
(22) Date of filing: 23.09.2021
(51) Int. Cl.: C07D 233/88, C07D 263/48, C07D 277/46, C07D 277/48, C07D 417/12, C07D 417/14, A61K 31/4178, A61K 31/422, A61K 31/427, A61P 29/00, A61P 35/00, A61P 25/06

(54) **ALPHA PROTEIN KINASE 1 INHIBITORS AND METHODS OF USE**
ALPHA-PROTEINKINASE-1-HEMMER UND VERFAHREN ZUR VERWENDUNG
INHIBITEURS DE LA PROTÉINE ALPHA KINASE 1 ET PROCÉDÉS D'UTILISATION

(30) Priority: 24.09.2020 WO PCT/CN2020/117436
(43) Date of publication of application: 09.08.2023
(73) Proprietor: Shanghai Yao Yuan Biotechnology Co., Ltd., Shanghai 200444 (CN)
(72) Inventor: LIU, Danyang, Shanghai 200444 (CN); XU, Cong, Shanghai 200444 (CN); MELVIN, Lawrence S., Jr., Shanghai 200444 (CN); WEI, Xiong, Shanghai 200444 (CN); LI, Tongruei Raymond, Shanghai 200444 (CN); FAN, Jieqing, Shanghai 200444 (CN); PAN, Yanfang, Shanghai 200444 (CN); DANG, Huaixin, Shanghai 200444 (CN); LICHENSTEIN, Henri, Shanghai 200444 (CN); XU, Tian, Shanghai 200444 (CN)
(74) Representative: Haseltine Lake Kempner LLP
(86) International application number: PCT/CN2021/119801
(87) International publication number: WO 2022/063152

(56) References cited:
- WO-A1-2009/002933
- WO-A1-2013/131018
- WO-A1-2019/007362
- WO-A1-2019/046668
- WO-A1-2020/176863
- WO-A1-2022/063153
- WO-A1-2022/222888
- WO-A2-2008/057862
- US-A- 3 591 600
- US-B1- 6 410 533
- DATABASE BIOSIS [online] BIOSCIENCES INFORMATION SERVICE, PHILADELPHIA, PA, US; February 2011 (2011-02-01), STRAUB CHRISTOPHER S: "Targeting IAPs as An Approach to Anti-Cancer Therapy", XP002812245, Database accession no. PREV201100096707
- STRAUB CHRISTOPHER S: "Targeting IAPs as An Approach to Anti-Cancer Therapy", CURRENT TOPICS IN MEDICINAL CHEMISTRY, vol. 11, no. 3, February 2011 (2011-02-01), pages 291 - 316
- OLIVIER BEZENÇON ET AL: "Discovery of a Potent, Selective T-type Calcium Channel Blocker as a Drug Candidate for the Treatment of Generalized Epilepsies", JOURNAL OF MEDICINAL CHEMISTRY, vol. 60, no. 23, 14 December 2017 (2017-12-14), US, pages 9769 - 9789, XP055447039, ISSN: 0022-2623, DOI: 10.1021/acs.jmedchem.7b01236
- PARK C M ET AL: "Non-peptidic small molecule inhibitors of XIAP", BIOORGANIC & MEDICINAL CHEMISTRY LETTERS, ELSEVIER, AMSTERDAM NL, vol. 15, no. 3, 1 February 2005 (2005-02-01), pages 771 - 775, XP027801959, ISSN: 0960-894X, [retrieved on 20050201]
- INOUE TAKAYUKI ET AL: "Synthesis and SAR study of new thiazole derivatives as vascular adhesion protein-1 (VAP-1) inhibitors for the treatment of diabetic macular edema", BIOORGANIC & MEDICINAL CHEMISTRY, ELSEVIER, AMSTERDAM, NL, vol. 21, no. 5, 29 December 2012 (2012-12-29), pages 1219 - 1233, XP028977166, ISSN: 0968-0896, DOI: 10.1016/J.BMC.2012.12.025
- DATABASE REGISTRY 13 May 2005 (2005-05-13), ANONYMOUS: "/1 -(C) FILE REGISTRY RN -850402-11-8 REGISTRY ED -Entered STN: 13 May 2005 CN -Formamide, N-[4-[4-(3,4-dimethoxyphenyl)tetrahydro-2H-pyran-4-yl]-2- thiazolyl]-(CA INDEX NAME)", XP055915146, retrieved from STN Database accession no. 850402-11-8
- DATABASE REGISTRY 22 September 2020 (2020-09-22), ANONYMOUS: "/1 -(C) FILE REGISTRY RN -2484862-72-6 REGISTRY ED -Entered STN: 22 Sep 2020 CN -1H-Pyrrole-2-carboxamide, N-[4-(1-phenylethyl)-2-thiazolyl]-1-(4- pyridinylmethyl)-(CA INDEX NAME)", XP055915150, retrieved from STN Database accession no. 2484862-72-6
- DATABASE REGISTRY 21 December 2017 (2017-12-21), ANONYMOUS: "/1 -(C) FILE REGISTRY RN -2162783-43-7 REGISTRY ED -Entered STN: 21 Dec 2017 CN -Carbamic acid, N-[6-[hydroxy(3-methylphenyl)methyl]-2-pyridinyl]-, 1,1-dimethylethyl ester (CA INDEX NAME)", XP055915152, retrieved from STN Database accession no. 2162783-43-7

## Description

### FIELD OF THE INVENTION

The present invention relates to compounds that are inhibitors of alpha protein kinase 1 (ALPK1) and related compositions.

### BACKGROUND OF THE INVENTION

Alpha-kinases display little sequence similarity to conventional protein kinases. A total of six alpha kinase members have been identified. These include alpha-protein kinase 1 (ALPK1), ALPK2, ALPK3, elongated factor-2 kinase (eEF2K), and transient receptor potential cation channel M6 and M7 (TRPM6 and TRPM7). *See* Ryazanov et al., Curr Biol 9:R43-45 (1999) and Ryazanov et al., Proc Natl Acad Sci USA 94:4884-4889 (1997).

ALPK1 is an intracytoplasmic serine threonine protein kinase that plays an important role in activating the innate immune response to bacteria *via* TRAF-interacting protein with forkhead-associated domain (TIFA) dependent proinflammatory nuclear factor-kappa-B (NFkB) signaling. *See* Zimmermann et al. Cell Rep. 20:2384-2395 (2017); Milivojevic et al., PLoS Pathog. 13:E1006224-E1006224 (2017); and Zhou et al., Nature 561:122-126 (2018).

Inappropriate activation of ALPK1 signaling has been implicated in diseases and disorders associated with excessive or inappropriate inflammation. For example, ALPK1 has been implicated in monosodium urate monohydrate (MSU)-induced inflammation and gout. Lee et al., Sci. Rep. 6:25740-25740(2016). Elevated ALPK1 expression has also been associated with lymph node metastasis and tumor growth in oral squamous cell carcinoma. Chen et al., Am J Pathol 189:190-199 (2019). In addition, genetic mutations in ALPK1 have been associated with spiroandenoma, spiroandenocarcinoma, "Retinal dystrophy, Optic nerve edema, Splenomegaly, Anhidrosis and migraine Headache" ("ROSAH") syndrome, and "Periodic Fever, Aphthous Stomatitis, Pharyngitis, and Adenitis" ("PFAPA") syndrome. *See e.g.,* Rashid et al., Nature Communications (2019); Williams et al., Genetics in Medicine 21:2103-2115 (2019); and Sangiorgi et al. Eur. J. Human Genetics (2019).Prior art WO 2022/222888 discloses methods for inhibiting ALPK1 kinase activity. WO2019/007362 discloses ALPK1 inhibitors. WO 2022/063153 discloses compounds of Formula (I) or (II) and related compositions and methods for their inhibitors of alpha-kinase 1(ALPK1).

### SUMMARY OF THE INVENTION

The invention is set out in the appended set of claims.

The references to the methods of treatment by therapy or surgery or in vivo diagnosis methods of this description are to be interpreted as references to compounds, pharmaceutical compositions and medicaments of the present invention for use in those methods.

Provided herein are compounds of Formula XI, 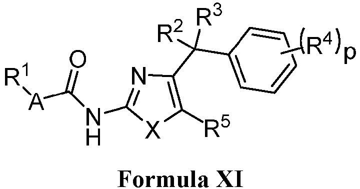 wherein X, A, p, R¹, R², R³, R⁴ and R⁵ are as defined herein.

In some embodiments, compounds of Formula XI are represented by Formula XI-A, 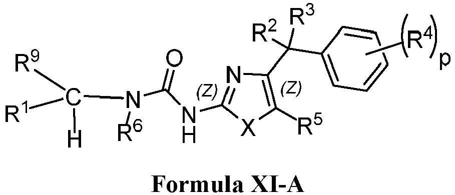 wherein X, p, R¹, R², R³, R⁴,R⁵, R⁶, and R⁹ are as defined herein.

In some embodiments, compounds of Formula XI are represented by Formula XI-A-1, 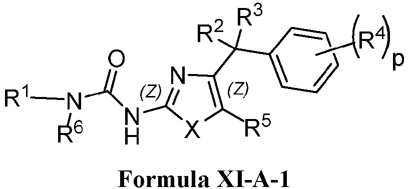 wherein X, p, R¹, R², R³, R⁴,R⁵, R⁶, and R⁹ are as defined herein.

In some embodiments, compounds of Formula XI are represented by Formula XI-A-1-a, 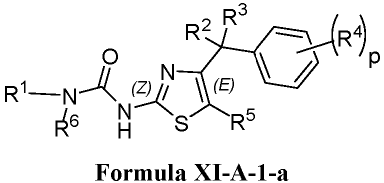 wherein p, R¹, R², R³, R⁴,R⁵, R⁶, and R⁹ are as defined herein.

In some embodiments, compounds of Formula XI are represented by Formula XI-B, 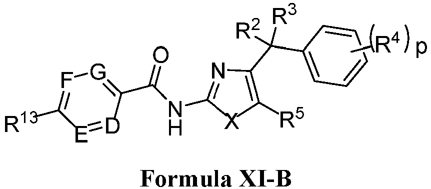 wherein X, p, R², R³, R⁴, R⁵, R¹³, D, E, F, and G are as defined herein.

In some embodiments, compounds of Formula XI are represented by Formula XI-B-1, 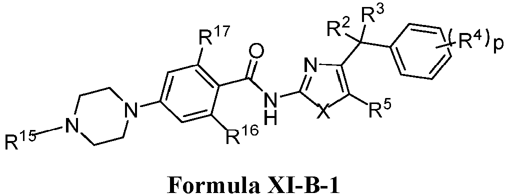 wherein X, p, R², R³, R⁴, R⁵, R¹⁵, R¹⁶, and R¹⁷ are as defined herein.

In some embodiments, compounds of Formula XI are represented by Formula XI-B-1-a, 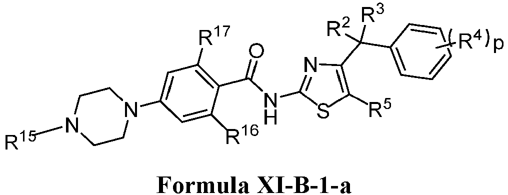 wherein p, R², R³, R⁴, R⁵, R¹⁵, R¹⁶, and R¹⁷ are as defined herein.

In some embodiments, compounds of Formula XI are represented by Formula XI-C, 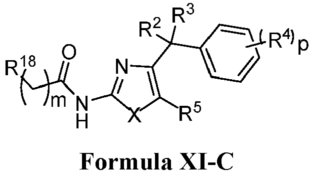 wherein X, p, m, R², R³, R⁴, R⁵, R¹⁸ are as defined herein.

In some embodiments, compounds of Formula XI are represented by Formula XI-C-1, 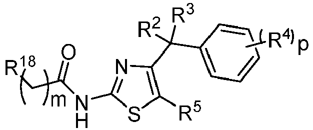 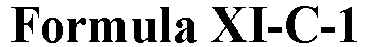 wherein p, m, R², R³, R⁴, R⁵, R¹⁸ are as defined herein.

In embodiments, the disclosure provides a pharmaceutical composition comprising a compound of Formula XI, XI-A, XI-B, or XI-C, or a subembodiment thereof, as described herein.

### BRIEF DESCRIPTION OF THE FIGURES

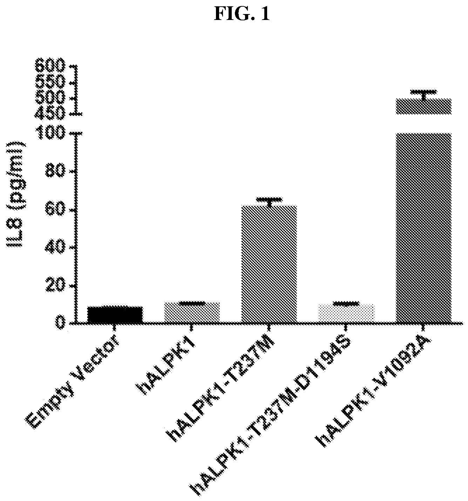

FIG. 1: Bar graph showing IL-8 secretion (pg/ml) in HEK293 cells transiently transfected with empty vector, or expression vectors encoding human ALPK1 (hALPK1), an activating mutation in hALPK1 (T237M, V1092A) or an activating mutation combined with a kinase dead mutation in ALPK1 (hALPK1-T237M-D1194S).

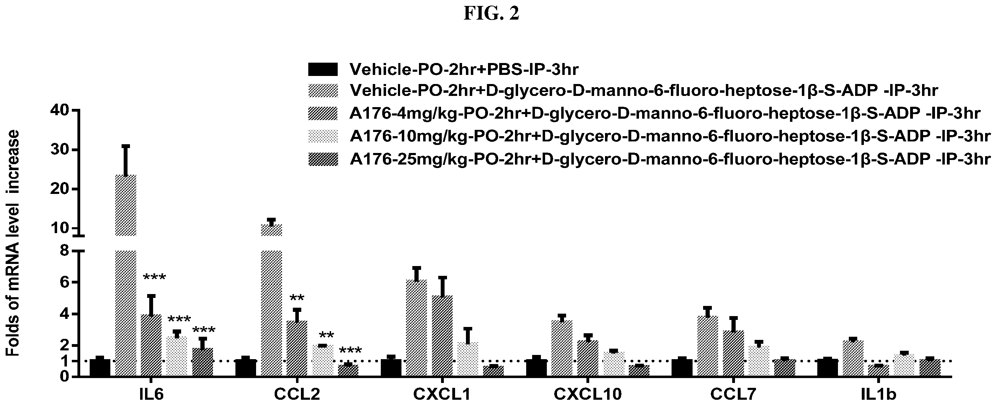

FIG.2: Treatment groups were administered 4, 10 or 25 mg/kg of the ALPK1 inhibitor A0176 2 hours prior to the agonist, D-glycero-D-manno-6-fluoro-heptose-1β-S-ADP. 3 hours after agonist administration, the kidney tissues were examined for inhibition of gene expression of innate immunity genes including MCP-1 (CCL-2), CCL-7, CXCL-1, CXCL-10, IL-1β, IL-6 mRNA. A0176 showed a dose-dependent inhibition of gene expression levels. **p<0.01, ***p<0.001 vs Vehicle-PO+A0176-IP-3hr by two-way-ANOVA

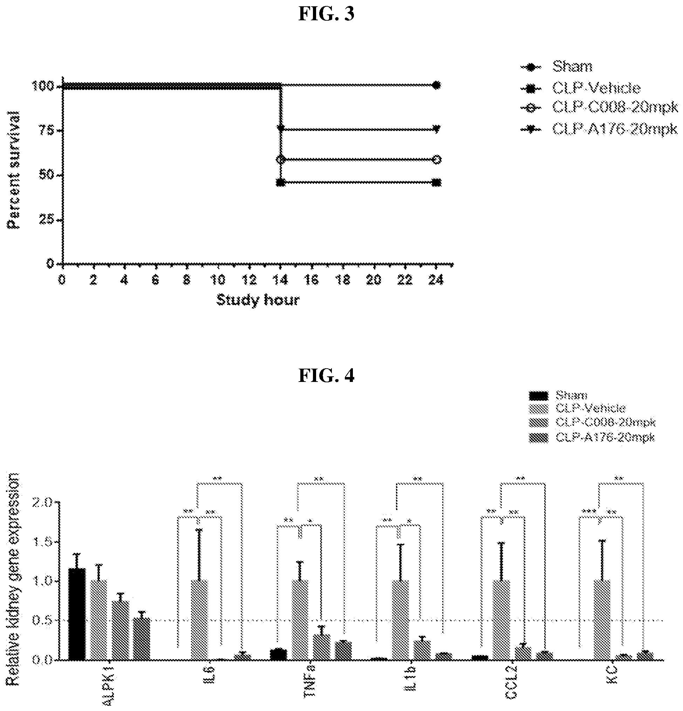

FIG. 3: In the sepsis induced acute kidney injury animal model, compounds C008 and A0176 (20 mg/kg) were administered to treatment groups of animals 2 hours prior to the surgery. Survival was recorded over the following 24 hours. Both compounds improved the animals' survival rate.
FIG 4: In the sepsis induced acute kidney injury animal model, compounds C008 and A0176 (20 mg/kg) were administered to treatment groups of animals 2 hours prior to the surgery. 24 hours post-surgery, the kidneys were collected for gene expression analysis by Q-PCR. The data show that ALPK1 inhibitors decreased expression of kidney proinflammatory genes including IL6, TNFa, IL-1b, CCl2 and Keratinocyte chemoattractant (KC) chemokine. *p<0.05, **p<0.01, p<0.001, vs. CLP-Vehicle

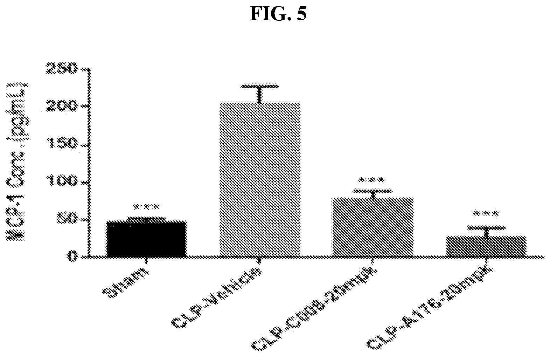

FIG 5: In the sepsis induced acute kidney injury animal model, compounds C008 and A0176 (20 mg/kg) were administered to treatment groups of animals 2 hours prior to the surgery. 24 hours post-surgery, plasma MCP-1 concentration was measured by ELISA. ALPK1 inhibitors improved the plasma MCP-1 levels. ***p<0.001 vs. CLP vehicle by one-way ANOVA

### DETAILED DESCRIPTION

The disclosure provides compounds that are inhibitors of ALPK1, compositions comprising same.

The term "ALPK1" is used herein to refer interchangeably to isoform 1 (Q96QP1-1) or the alternative splice variant isoform 2 (Q96QP1-2) of the human sequence identified by UniProtKB - Q96QP1 (ALPK1_HUMAN).

As used herein, the term "alkyl" refers to a straight or branched, saturated, aliphatic radical having the number of carbon atoms indicated. Alkyl can include any number of carbons, such as C₁₋₂, C₁₋₃, C₁₋₄, C₁₋₅, C₁₋₆, C₁₋₇, C₁₋₈, C₁₋₉, C₁₋₁₀, C₂₋₃, C₂₋₄, C₂₋₅, C₂₋₆, C₃₋₄, C₃₋₅, C₃₋₆, C₄₋₅, C₄₋₆ and C₅₋₆. For example, C₁₋₆ alkyl includes, but is not limited to, methyl, ethyl, propyl, isopropyl, butyl, isobutyl, sec-butyl, tert-butyl, pentyl, isopentyl, hexyl, etc. Alkyl can also refer to alkyl groups having up to 20 carbons atoms, such as, but not limited to heptyl, octyl, nonyl, decyl, etc. Alkyl groups can be substituted or unsubstituted.

As used herein, "alkenyl" refers to a straight chain or branched hydrocarbon having at least 2 carbon atoms and at least one double bond. Alkenyl can include any number of carbons, such as C₂, C₂₋₃, C₂₋₄, C₂₋₅, C₂₋₆, C₂₋₇, C₂₋₈, C₂₋₉, C₂₋₁₀, C₃, C₃₋₄, C₃₋₅, C₃₋₆, C₄, C₄₋₅, C₄₋₆, C₅, C₅₋₆, and C₆. Alkenyl groups can have any suitable number of double bonds, including, but not limited to, 1, 2, 3, 4, 5 or more. In some embodiments, an alkenyl group has 1 double bond. Alkenyl groups can be substituted or unsubstituted.

As used herein, "alkynyl" refers to a straight chain or branched hydrocarbon having at least 2 carbon atoms and at least one triple bond. Alkenyl can include any number of carbons, such as C₂, C₂₋₃, C₂₋₄, C₂₋₅, C₂₋₆, C₂₋₇, C₂₋₈, C₂₋₉, C₂₋₁₀, C₃, C₃₋₄, C₃₋₅, C₃₋₆, C₄, C₄₋₅, C₄₋₆, C₅, C₅₋₆, and C₆. Alkynyl groups can have any suitable number of triple bonds, including, but not limited to, 1, 2, 3, 4, 5 or more. In some embodiments, an alkynyl group has 1 triple bond. Alkynyl groups can be substituted or unsubstituted.

As used herein, the term "alkylene" refers to a straight or branched, saturated, aliphatic radical having the number of carbon atoms indicated, and linking at least two other groups, i.e., a divalent hydrocarbon radical. The two moieties linked to the alkylene can be linked to the same atom or different atoms of the alkylene group. For instance, a straight chain alkylene can be the bivalent radical of -(CH₂)n-, where n is 1, 2, 3, 4, 5 or 6. Representative alkylene groups include, but are not limited to, methylene, ethylene, propylene, isopropylene, butylene, isobutylene, sec-butylene, pentylene and hexylene. Alkylene groups can be substituted or unsubstituted. In some embodiments, alkylene groups are substituted with 1-2 substituents. As a non-limiting example, suitable substituents include halogen and hydroxyl.

As used herein, the term "alkoxy" or "alkoxyl" refers to an alkyl group having an oxygen atom that connects the alkyl group to the point of attachment: alkyl-O-. As for alkyl group, alkoxyl groups can have any suitable number of carbon atoms, such as C1-6. Alkoxyl groups include, for example, methoxy, ethoxy, propoxy, iso-propoxy, butoxy, 2-butoxy, iso-butoxy, sec-butoxy, tert-butoxy, pentoxy, hexoxy, etc. The alkoxy groups can be substituted or unsubstituted.

As used herein, the term "alkenyloxy" or "alkenyloxyl" refers to an alkenyl group, as defined above, having an oxygen atom that connects the alkenyl group to the point of attachment: alkenyl-O-. Alkenyloxyl groups can have any suitable number of carbon atoms, such as C1-6. Alkenyloxyl groups can be further substituted with a variety of substituents described within. Alkenyloxyl groups can be substituted or unsubstituted.

"Aminoalkyl" means a linear monovalent hydrocarbon radical of one to six carbon atoms or a branched monovalent hydrocarbon radical of three to six carbons substituted with -NR'R" where R' and R" are independently hydrogen, alkyl, haloalkyl, or hydroxyalkyl, each as defined herein, e.g., aminomethyl, aminoethyl, methylaminomethyl, and the like.

As used herein, the term "halogen" or "halo" refers to fluorine, chlorine, bromine and iodine.

As used herein, the term "haloalkyl" refers to alkyl, as defined above, where some or all of the hydrogen atoms are replaced with halogen atoms. As for alkyl group, haloalkyl groups can have any suitable number of carbon atoms, such as C₁₋₆. For example, haloalkyl includes trifluoromethyl, fluoromethyl, etc.

As used herein, the term "haloalkoxyl" or "haloalkoxy" refers to an alkoxyl group where some or all of the hydrogen atoms are substituted with halogen atoms. As for an alkyl group, haloalkoxy groups can have any suitable number of carbon atoms, such as C₁₋₆. The alkoxy groups can be substituted with 1, 2, 3, or more halogens.

As used herein, the term "deuteroalkyl" means an alkyl radical as defined above wherein one to six hydrogen atoms in the alkyl radical are replaced by deuterium, e.g., -CH₂D, -CHD₂, -CD₃, -CH₂CD₃, and the like.

As used herein, the term "hydroxyalkyl" refers to an alkyl radical wherein at least one of the hydrogen atoms of the alkyl radical is replaced by OH. Examples of hydroxyalkyl include, but are not limited to, hydroxy-methyl, 2-hydroxy-ethyl, 2-hydroxy-propyl, 3-hydroxy-propyl and 4-hydroxy-butyl.

As used herein, the term "oxo" refers to an oxygen atom connected to the point of attachment by a double bond (=O).

As used herein, the term "aryl" refers to an aromatic ring system having any suitable number of ring atoms and any suitable number of rings. Aryl groups can include any suitable number of ring atoms, such as, 6, 7, 8, 9, 10, 11, 12, 13, 14, 15 or 16 ring atoms, as well as from 6 to 10, 6 to 12, or 6 to 14 ring members. Aryl groups can be monocyclic, fused to form bicyclic or tricyclic groups, or linked by a bond to form a biaryl group. Representative aryl groups include phenyl, naphthyl and biphenyl. Other aryl groups include benzyl, having a methylene linking group. Some aryl groups have from 6 to 12 ring members, such as phenyl, naphthyl or biphenyl. Other aryl groups have from 6 to 10 ring members, such as phenyl or naphthyl. Some other aryl groups have 6 ring members, such as phenyl. Aryl groups can be substituted or unsubstituted.

As used herein, the term "heteroaryl" refers to a monocyclic or fused bicyclic aromatic ring assembly containing 5 to 12 ring atoms, where from 1 to 5 of the ring atoms are a heteroatom such as N, O or S. Additional heteroatoms can also be useful, including, but not limited to, B, Al, Si and P. The heteroatoms can also be oxidized, such as, but not limited to, -S(O)- and -S(O)₂-. Heteroaryl groups can include any number of ring atoms, such as, 3 to 6, 4 to 6, 5 to 6, 3 to 8, 4 to 8, 5 to 8, 6 to 8, 3 to 9, 3 to 10, 3 to 11, or 3 to 12 ring members. Any suitable number of heteroatoms can be included in the heteroaryl groups, such as 1, 2, 3, 4, or 5, or 1 to 2, 1 to 3, 1 to 4, 1 to 5, 2 to 3, 2 to 4, 2 to 5, 3 to 4, or 3 to 5. Heteroaryl groups can have from 5 to 9 ring members and from 1 to 4 heteroatoms, or from 5 to 9 ring members and from 1 to 3 heteroatoms, or from 5 to 6 ring members and from 1 to 4 heteroatoms, or from 5 to 6 ring members and from 1 to 3 heteroatoms. The heteroaryl group can include groups such as pyrrole, pyridine, imidazole, pyrazole, triazole, tetrazole, pyrazine, pyrimidine, pyridazine, triazine (1,2,3-, 1,2,4- and 1,3,5-isomers), purine. The heteroaryl groups can also be fused to aromatic ring systems, such as a phenyl ring, to form members including, but not limited to, benzopyrroles such as indole and isoindole, benzopyridines such as quinoline and isoquinoline, benzopyrazine (quinoxaline), benzopyrimidine (quinazoline), benzopyridazines such as phthalazine and cinnoline, benzothiophene, and benzofuran. Other heteroaryl groups include heteroaryl rings linked by a bond, such as bipyridine. Heteroaryl groups can be substituted or unsubstituted.

As used herein, "cycloalkyl" refers to a saturated ring assembly containing from 3 to 10 ring atoms, or the number of atoms indicated. Cycloalkyl can include any number of carbons, such as C₃₋₆, C₄₋₆, C₅₋₆, C₃₋₈, C₄₋₈, C₅₋₈, C₆₋₈. Cycloalkyl rings can be saturated or unsaturated, when unsaturated cycloalkyl rings can have one or two double bonds. Cycloalkyl rings include, for example, cyclopropyl, cyclobutyl, cyclopentyl, cyclohexyl, and cyclooctyl. Cycloalkyl groups can be substituted or unsubstituted.

As used herein, the term"heterocyclyl" or"heterocyclic" refers to a heterocyclic group that is saturated or partially saturated and is a monocyclic or a polycyclic ring; which has 3 to 16, most preferably 5 to 10 and most preferably 1 or 4 ring atoms; wherein one or more, preferably one to four, especially one or two ring atoms are a heteroatom selected from oxygen, nitrogen and sulfur (the remaining ring atoms therefore being carbon). The term heterocyclyl excludes heteroaryl. The heterocyclic group can be attached to the rest of the molecule through a heteroatom, selected from oxygen, nitrogen and sulfur, or a carbon atom. The heterocyclyl can include fused or bridged rings as well as spirocyclic rings. Examples of heterocyclyl include dihydrofuranyl, dioxolanyl, dioxanyl, dithianyl, piperazinyl, pyrrolidine, dihydropyranyl, oxathiolanyl, dithiolane, oxathianyl, thiomorpholino, oxiranyl, aziridinyl, oxetanyl, oxepanyl, azetidinyl, tetrahydrofuranyl, tetrahydrothiophenyl, pyrrolidinyl, tetrahydropyranyl, piperidinyl, morpholino, piperazinyl, azepinyl, oxapinyl, oxaazepanyl, oxathianyl, thiepanyl, azepanyl, dioxepanyl, and diazepanyl.

As used herein, "spiroheterocyclyl" refers to a specific bicyclic heterocyclic group wherein the 2 ring systems are connected through a single carbon atom. For example, the term"spiroheterocyclyl" can refer to a 6-10 spiro heterocyclyl. Examples of include, but not limited to, 6,9-diazaspiro[4.5]decane, 2-oxa-6,9-diazaspiro[4.5]decane, 2-Oxa-6-azaspiro[3.4]octane, 6-azaspiro[3.4]octane, 2,6-diazaspiro[3.4]octane, 1,6-diazaspiro[3.4]octane, 2,8-diazaspiro[4.5]decane,2,7-diazaspiro[4.4]nonane, 1-thia-8-azaspiro[4.5]decane 1,1-dioxide, 1-oxa-7-azaspiro[4.4]nonane and 1-oxa-9-azaspiro[5.5]undecane.

As used herein, "bridged heterocyclyl" refers to a C₃₋₆ cycloalkyl ring or a 3-to 6- memberd heterocyclyl ring, as defined above, where two non-adjacent ring vertices ("bridgehead atoms") of the cycloalkyl ring or the heterocyclyl ring are linked to form an additional cyclic moiety (a "bridge"). The bridge comprises 1 to 4 ring vertices, not including the bridgehead atoms. Examples include, but not limited to, 2,5-diazabicyclo[2.2.1]heptane, 3,6-diazabicyclo[3.1.1]heptane, 3,8-diazabicyclo[3.2.1]octane, 2,5-diazabicyclo[2.2.2]octane, 3,9-diazabicyclo[3.3.1]nonane, 2-thia-5-azabicyclo[2.2.1]heptane 2,2-dioxide, 2-azabicyclo[2.2.1]hept-5-ene, 3-oxa-8-azabicyclo[3.2.1]octane, 3-oxa-6-azabicyclo[3.1.1]heptane, 6-oxa-3-azabicyclo[3.1.1]heptane and 2-oxa-5-azabicyclo[2.2.1]heptane.

The term "bicyclic heterocyclyl" refers to a heterocyclic group as defined above where the two ring systems are connected through two adjacent ring vertices (e.g., a fused ring system). Typical "bicyclic heterocyclyl" rings include 6 to 11 ring members having 1 to 4 heteroatom ring vertices selected from N, O, and S (the remaining ring atoms therefore being carbon). Examples include, but not limited to, benzodioxolyl, benzimidazolyl, benzisoxazolyl, benzofurazanyl, benzopyranyl, benzothiopyranyl, benzofuryl, benzothiazolyl, benzothienyl, benzotriazolyl, benzoxazolyl, chromanyl, cinnolinyl, dihydrobenzofuryl, dihydroisobenzofuranyl, dihydrobenzothienyl, dihydrobenzothiopyranyl, dihydrobenzothiopyranyl sulfone, indolinyl, indolyl, isochromanyl, isoindolinyl, isoquinolinyl, isothiazolidinyl, naphthyridinyl, pyrazolopyridinyl, quinazolinyl, quinolinyl, quinoxalinyl, tetrahydroisoquinolinyl, tetrahydroquinolinyl.

As used herein, "saturated or unsaturated" refers to a cyclic system where two of the atoms in the group may be bound to one another by a single bond, a double bond, or a triple bond. Saturated moieties are those having only single bonds, where moieties having multiple bonds (e.g., at least one double bond or at least one triple bondare referred to as unsaturated.

When needed, any definition herein may be used in combination with any other definition to describe a composite structural group. By convention, the trailing element of any such definition is that which attaches to the parent moiety. For example, the composite group cycloalkoxyl means that a cycloalkyl group is attached to the parent molecule through an oxyl group.

The term "pharmaceutically acceptable salts" is meant to include salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of salts derived from pharmaceutically-acceptable inorganic bases include aluminum, ammonium, calcium, copper, ferric, ferrous, lithium, magnesium, manganic, manganous, potassium, sodium, zinc and the like. Salts derived from pharmaceutically-acceptable organic bases include salts of primary, secondary and tertiary amines, including substituted amines, cyclic amines, naturally-occuring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge, S.M., et al, "Pharmaceutical Salts", Journal of Pharmaceutical Science, 1977, 66, 1-19). Certain specific compounds of the present invention contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present disclosure.

### Detailed Description of the Embodiments

The present invention discloses novel heterocyclic compounds as inhibitors of ALPK1. The compounds are represented by formula XI, 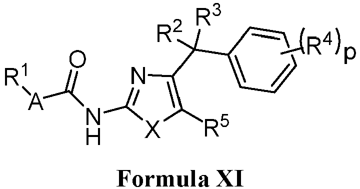
wherein A, p, R¹, R², R³, R⁴ and R⁵ are as defined herein; and
   X is selected from -S-, -O-, -NR^{a}-, -CH=N-, and -CH=CH-, wherein R^{a} is H, or C₁-C₆ alkyl;
A is selected from a bond, azetidinyl, -O-, -N(R⁶)-, -CH₂-N(R⁶)-, -CHR⁹-N(R⁶)-, wherein
   R⁶ is selected from H, D, -OH, optionally substituted C₁-C₆ alkyl, optionally substituted C₁-C₆ haloalkyl, optionally substituted C₁-C₆ alkenyl, optionally substituted C₁-C₆ hydroxyalkyl, optionally substituted C₁-C₆ aminoalkyl, optionally substituted C₁-C₆ alkoxyl, optionally substituted saturated or unsaturated C₃-C₆ cycloalkyl, and optionally substituted saturated or unsaturated C₃-C₆ cycloalkoxyl, wherein
      the optionally substituted R⁶ moieties comprise 0-3 substituents independently selected from -D, halo, -OH, -COOH, -NH₂, =O, -CN, C₁-C₆ alkyl, C₁-C₆ alkenyl, C₁-C₆ hydroxyalkyl, C₁-C₆ hydroxy-deuterated alkyl, C₁-C₆ haloalkyl, C₁-C₆ aminoalkyl, and C₁-C₆ alkoxyl;
   R⁹ is selected from optionally substituted C₁-C₆ alkyl, C₁-C₆ haloalkyl, optionally substituted saturated or unsaturated C₃-C₆ cycloalkyl, ptionally substituted saturated or unsaturated C₃-C₆ cycloalkoxyl, wherein
      optionally substituted R⁹ moieties comprise 0-2 substituents independently selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, C₁-C₆ haloalkoxyl, saturated or unsaturated C₃-C₆ cycloalkyl, saturated or unsaturated C₃-C₆ cycloalkoxyl, -CHR^{7f}R^{8f}, -OR^{7f}, - OC(O)(R^{7f}), -C(O)(R^{7f}), -C(O)N(R^{7f}R^{8f}), -C(O)O(R^{7f}), -S(O)₂(R^{7f}), - S(O)ON(R^{7f}R^{8f}) and -N(R^{7f}R^{8f}) wherein
      each R^{7f} and R^{8f} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, C₁-C₆ haloalkoxyl, saturated or unsaturated C₃-C₆ cycloalkyl, and saturated or unsaturated C₃-C₆ cycloalkoxy;
R¹ is selected from H, optionally substituted C₁-C₆ alkyl, optionally substituted C₁-C₆ alkenyl, optionally substituted C₁-C₆ hydroxyalkyl, optionally substituted C₁-C₆ hydroxy deuterated alkyl, optionally substituted C₁-C₆ haloalkyl, optionally substituted C₁-C₆ haloalkoxyl, optionally substituted C₁-C₆ aminoalkyl, optionally substituted C₁-C₆ alkoxyl, optionally substituted saturated or unsaturated C₃-C₆ cycloalkyl, optionally substituted saturated or unsaturated C₃-C₆ cycloalkoxyl, optionally substituted mono or bicyclic aryl, optionally substituted 5-10 membered heteroaryl containing 1-4 heteroatom ring vertices selected from N, O, and S; optionally substituted saturated or unsaturated 3-7 membered heterocyclyl containing 1-2 heteroatom ring vertices selected from N, O, and S; optionally substituted saturated or unsaturated 7-8 membered bridged heterocyclyl containing 1-2 heteroatom ring vertices selected from N, O, and S; optionally substituted saturated or unsaturated 7-11 membered spiroheterocyclyl containing 1-2 heteroatom ring vertices selected from N, O, and S; and optionally substituted saturated or unsaturated 6-11 membered bicyclic heterocyclyl containing 1-2 heteroatom ring vertices selected from N, O, and S;
   wherein optionally substituted R¹ moieties comprise 0-4 substituents independently selected from -D, halo, -OH, -COOH, -NH₂, =O, -CN, C₁-C₆ alkyl, C₁-C₆ alkenyl, C₁-C₆ hydroxyalkyl, C₁-C₆ hydroxy-deuterated alkyl, C₁-C₆ haloalkyl, C₁-C₆ aminoalkyl, C₁-C₆ alkoxyl, C₁-C₆ haloalkoxyl, -R^{7a}, -X¹-R^{7a}, CHR^{7a} R^{8a}, -OR^{7a}, -O-X¹-R^{7a}, -X¹-O-X¹-R^{7a}, -OC(O)(R^{7a}), -O-X¹-C(O)(R^{7a}), -C(O)(R^{7a}), - C(O)N(R^{7a}R^{8a}), -NR^{7a}(CO)R^{8a}, -C(O)O(R^{7a}), S(O)₂R^{7a}, -S(O)₂N(R^{7a}R^{8a}), - N(R^{7a}R^{8a}), saturated or unsaturated C₃-C₆ cycloalkyl, saturated or unsaturated C₃-C₆ cycloalkoxyl, saturated or unsaturated 3-7 membered heterocyclyl containing 1-2 heteroatom ring vertices selected from N, O, and S, mono or bicyclic aryl, 5-10 membered heteroaryl containing 1-4 heteroatom ring vertices selected from N, O, and S, saturated or unsaturated 7-8 membered bridged heterocyclyl containing 1-2 heteroatom ring vertices selected from N, O, and S, saturated or unsaturated 7-11 membered spiroheterocyclyl containing 1-2 heteroatom ring vertices selected from N, O, and S, and 6-11 membered bicyclic heterocyclyl containing 1-2 heteroatom ring vertices selected from N, O, and S; wherein
   each X¹ is independently C₁₋₆ alkylene;
   each R^{7a} and R^{8a} are independently selected from H, C₁-C₆ alkyl, saturated or unsaturated C₃-C₆ cycloalkyl, saturated or unsaturated C₃-C₆ cycloalkoxyl, C₁-C₆ alkenyl, C₁-C₆ hydroxyalkyl, C₁-C₆ haloalkyl, C₁-C₆ aminoalkyl, C₁-C₆ alkoxyl, C₁-C₆ haloalkoxyl, saturated or unsaturated C₃-C₆ cycloalkyl, saturated or unsaturated C₃-C₆ cycloalkoxyl, aryl, saturated or unsaturated C₃-C₆ cycloalkyl, saturated or unsaturated C₃-C₆ cycloalkoxyl, saturated or unsaturated 3-7 membered heterocyclyl containing 1-2 heteroatom ring vertices selected from N, O, and S, wherein the aryl and 3-7 membered heterocyclyl groups are substituted with 0-3 substituents selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl; and
   the C₃-C₆ cycloalkyl, C₃-C₆ cycloalkoxyl, 3-7 membered heterocyclyl, the mono or bicyclic aryl, the 5-10 membered heteroaryl, the saturated or unsaturated 7-8 membered bridged heterocyclyl, the saturated or unsaturated 7-11 membered spiroheterocycly, and the 6-11 membered bicyclic heterocyclyl are each independently substituted with 0 to 3 moieties selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl, saturated or unsaturated 3-7 membered heterocyclyl containing 1-2 heteroatom ring vertices selected from N, O, and S, -CHR^{7b}R^{8b}, -OR^{7b}, -OC(O)( R^{7b}), -C(O)( R^{7b}), -C(O)N(R^{7b}R^{8b}), -NR^{7b}(CO)R^{8b}, -C(O)O(R^{7b}), -S(O)₂N(R^{7b}R^{8b}) and -N(R^{7b}R^{8b}), wherein
      each R^{7b} and R^{8b} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl; or
R¹ and R⁶ combine to form a 3-6 membered heterocycloalkyl substituted with 0-3 moieties independently selected from the group consisting of halo, -OH,-COOH, -NH₂, =O, - CN, C₁-C₆ alkyl, C₁-C₆ alkenyl, C₁-C₆ hydroxyalkyl, C₁-C₆ haloalkyl, C₁-C₆ aminoalkyl, and C₁-C₆ alkoxyl;
R⁵ is selected from H, deuterium, halo, C₁-C₆ alkyl, C₁-C₆ deuteroalkyl, and C₁-C₆ haloalkyl;
R² and R³ are each independently selected from H, OH, C₁-C₆ alkyl,C₂-C₆ alkynyl, C₃-C₆ cycloalkyl, and the mono or bicyclic aryl, wherein C₁-C₆ alkyl,C₂-C₆ alkynyl, C₃-C₆ cycloalkyl, and the mono or bicyclic aryl are each substituted with 0-3 moieties independently selected from halo, -OH, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl, - OC(O)(R^{7c}), -C(O)( R^{7c}), C(O)O(R^{7c}), S(O)₂N(R^{7c}R^{8c}), and N(R^{7c}R^{8c}), wherein
   each R^{7c} and R^{8c} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, C₁-C₆ haloalkoxy, saturated or unsaturated C₃-C₆ cycloalkyl, and saturated or unsaturated C₃-C₆ cycloalkoxyl;
   provided that R² and R³ are not both H; or
R² and R³ combine to form a C₃-C₆ cycloalkyl ring or a 3-7 membered heterocyclyl containing 1-2 heteroatom ring vertices independently selected from N, O, and S, wherein the ring formed can be optionally substituted with 1-2 substituents independently selected from C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, halo, -OH , =O, -CN, OC(O)( R^{7d}), - C(O)( R^{7d}), C(O)O(R^{7d}), S(O)₂N(R^{7d}R^{8d}) and N(R^{7d}R^{8d}), wherein
   each R^{7d} and R^{8d} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl;
each R⁴ is independently selected from halo, -OH, -NH₂, CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl, CHR^{7e}R^{8e}, OR^{7e}, OC(O)( R^{7e}), C(O)( R^{7e}), C(O)N(R^{7e}R^{8e}), C(O)O(R^{7e}), S(O)₂N(R^{7e}R^{8e}) and N(R^{7e}R^{8e}) wherein
   each R^{7e} and R^{8e} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl, and
the subscript p is 0,1, 2 or 3.

In some embodiments, X in Formula XI is S.

In some embodiments, X in Formula XI is O.

In some embodiments, X in Formula XI is NH.

In some embodiments, A in Formula XI is a bond.

In some embodiments, A in Formula XI is azetidinyl.

In some embodiments, A in Formula XI is -O-.

In some embodiments, A in Formula XI is -N(R⁶)-.

In some embodiments, A in Formula XI is -CH₂-N(R⁶)-.

In some embodiments, A in Formula XI I is -CHR⁹-N(R⁶)-.

In some embodiments, the compound of Formula XI is represented by the compound of formula XI-A, formula XI-A-1, formula XI-A-2 and/or a stereoisomer, a stable isotope, or a pharmaceutically acceptable salt thereof.

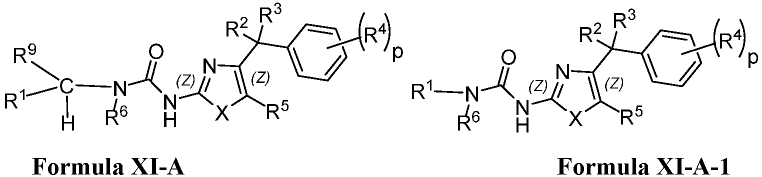

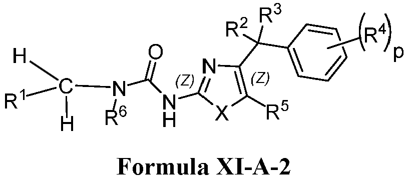

In some embodiments, the compound of formula XI is represented by the compound of formula XI-A-1-a, and/or a stereoisomer, a stable isotope, or a pharmaceutically acceptable salt thereof 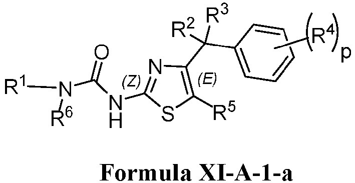 X, p, R¹, R², R³, R⁴,R⁵, R⁶, and R⁹ are as defined above.

In some embodiments Xin formula XI, XI-A, XI-A-1, XI-A-2, or XI-A-1-a is S, O or NH.

In some embodiments R⁶ in formula XI, XI-A, XI-A-1, XI-A-2, or XI-A-1-a is H, C₁-C₆ alkyl or C₁-C₆ hydroxyalkyl.

In some embodiments R⁹ in formula XI and XI-A is CH₃ or CH₂OH.

In some embodiments R⁹ in formula XI and XI-A is saturated C₃-C₆ cycloalkyl.

In some embodiments R¹ in formula XI, XI-A, XI-A-1, XI-A-2, or XI-A-1-a is selected from H and optionally substituted C₁-C₆ alkyl, wherein
optionally substituted C₁-C₆ alkyl comprises 0-4 substituents independently selected from halo, -OH,-COOH, -NH₂, =O, -CN, C₁-C₆ alkenyl, C₁-C₆ hydroxyalkyl, C₁-C₆ alkoxyl, C₁-C₆ haloalkoxyl, saturated or unsaturated C₃-C₆ cycloalkyl, saturated or unsaturated C₃-C₆ cycloalkoxyl, -CHR^{7a}R^{8a}, -OR^{7a}, -OC(O)(R^{7a}), -C(O)(R^{7a}), -C(O)N(R^{7a}R^{8a}), -C(O)O(R^{7a}), -S(O)₂R^{7a}, -S(O)₂N(R^{7a}R^{8a}) and - N(R^{7a}R^{8a}), wherein
each R^{7a} and R^{8a} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, C₁-C₆ haloalkoxyl, saturated or unsaturated C₃-C₆ cycloalkyl, and saturated or unsaturated C₃-C₆ cycloalkoxyl.

In some embodiments R¹ in formula XI, XI-A, XI-A-1, XI-A-2, or XI-A-1-a is optionally substituted saturated or unsaturated C₃-C₆ cycloalkyl, wherein
optionally substituted C₃-C₆ cycloalkyl comprises 0-4 substituents independently selected from halo, -OH,-COOH, -NH₂, =O, -CN, C₁-C₆ alkenyl, C₁-C₆ hydroxyalkyl, C₁-C₆ alkoxyl, and C₁-C₆ haloalkoxyl.

In some embodiments R¹ in formula XI, XI-A, XI-A-1, XI-A-2, or XI-A-1-a combines with R⁶ to form a 3-6 membered heterocycloalkyl substituted with 0-3 moieties independently selected from the group consisting of halo, -OH,-COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, and C₁-C₆ alkoxyl.

In some embodiments R¹ in formula XI, XI-A, XI-A-1, XI-A-2, or XI-A-1-a is C₁-C₆ alkyl substituted with 0-4 substituents independently selected from -OH, C₁-C₆ hydroxyalkyl, C₁-C₆ alkoxyl, -OC(O)( R^{7a}), -S(O)₂N(R^{7a}R^{8a}) and -N(R^{7a}R^{8a}), wherein
each R^{7a} and R^{8a} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl.

In some embodiments R¹ in formula XI, XI-A, XI-A-1, XI-A-2, or XI-A-1-a is C₁-C₆ alkyl substituted with 0-2 substituents independently selected from -OH, C₁-C₆ hydroxyalkyl, and -S(O)₂N(R^{7a}R^{8a}), wherein
each R^{7a} and R^{8a} are independently selected from H, and C₁-C₆ alkyl.

In some embodiments R¹ in formula XI, XI-A, XI-A-1, XI-A-2, or XI-A-1-a is optionally substituted C₁-C₆ hydroxyalkyl.

In some embodiments R¹ in formula XI, XI-A, XI-A-1, XI-A-2, or XI-A-1-a is a 5-10 membered heteroaryl containing 1-4 heteroatom ring vertices selected from N, O, and S,
the 5-10 membered bicyclic heteroaryl is substituted with 0 to 3 moieties selected from halo, -OH,-COOH, -NH₂, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, 3-7 membered heterocyclyl containing 1-2 heteroatom ring vertices selected from N, O, and S, saturated or unsaturated C₃-C₆ cycloalkyl, saturated or unsaturated C₃-C₆ cycloalkoxyl, -CHR^{7b}R^{8b}, -OR^{7b}, -OC(O)(R^{7b}), -C(O)( R^{7b}), -C(O)N(R^{7b}R^{8b}), -C(O)O(R^{7b}), -S(O)₂N(R^{7b}R^{8b}) and -N(R^{7b}R^{8b}), wherein each R^{7b} and R^{8b} are independently selected from H, C₁-C₆ alkyl, C₁-C₆
   alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl.

In some embodiments R¹ in formula XI, XI-A, XI-A-1, XI-A-2, or XI-A-1-a is pyridiyl substituted with 0 to 3 moieties selected from halo, -OH,-COOH, -NH₂, -CN, C₁-C₆ alkyl, C₁-C₆ alkenyl, 3-7 membered heterocyclyl containing 1-2 heteroatom ring vertices selected from N, O, and S, wherein
the 3-7 membered heterocyclyl is substituted with 0-3 substituents selected from halo, -OH, -COOH, -NH₂, -CN, C₁-C₆ alkyl, C₁-C₆ alkenyl, C₁-C₆ haloalkyl.

In some embodiments R¹ in formula XI, XI-A, XI-A-1, XI-A-2, or XI-A-1-a is a saturated or unsaturated 7-8 membered bridged heterocyclyl containing 1-2 heteroatom ring vertices selected from N, O, and S, wherein
the 7-8 membered bridged heterocyclyl is substituted with 0-3 moieties selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl, - CHR^{7b}R^{8b}, -OR^{7b}, -OC(O)( R^{7b}), -C(O)( R^{7b}), -C(O)N(R^{7b}R^{8b}), -C(O)O(R^{7b}), - S(O)₂N(R^{7b}R^{8b}) and -N(R^{7b}R^{8b}), wherein
each R^{7b} and R^{8b} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl.

In some embodiments R¹ in formula XI, XI-A, XI-A-1, XI-A-2, or XI-A-1-a is a saturated or unsaturated 7-11 membered spiroheterocyclyl containing 1-2 heteroatom ring vertices selected from N, O, and S, wherein
the 7-11 membered spiroheterocyclyl is substituted with 0-3 moieties selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl, - CHR^{7b}R^{8b}, OR^{7b}, -OC(O)( R^{7b}), -C(O)( R^{7b}), -C(O)N(R^{7b}R^{8b}), -C(O)O(R^{7b}), - S(O)₂N(R^{7b}R^{8b}) and -N(R^{7b}R^{8b}), wherein
each R^{7b} and R^{8b} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl.

In some embodiments, R¹ in formula XI, XI-A, XI-A-1, XI-A-2, or XI-A-1-a is aryl substituted with 0-3 substituents selected from halo, a 3-7 membered heterocyclyl containing 1-2 heteroatom ring vertices selected from N, O, and S; a 7-8 membered bridged heterocyclyl containing 1-2 heteroatom ring vertices selected from N, O, and S; and a saturated or unsaturated 7-11 membered spiroheterocyclyl containing 1-2 heteroatom ring vertices selected from N, O, and S, wherein
the 3-7 membered heterocyclyl, the 7-8 membered bridged heterocyclyl, and the 7-11 membered spiroheterocyclyl are substituted with from 0 to 3 moieties selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl, - CHR^{7b}R^{8b}, -OR^{7b}, -OC(O)(R^{7b}), -C(O)(R^{7b}), -C(O)N(R^{7b}R^{8b}), ^{_}C(O)O(R^{7b}), - S(O)₂R^{7b}, -S(O)₂N(R^{7b}R^{8b}) and -N(R^{7b}R^{8b}), wherein
each R^{7b} and R^{8b} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl

In some embodiments R¹ in formula XI, XI-A, XI-A-1, XI-A-2, or XI-A-1-a is aryl substituted with 0-3 moieties selected from halo -OH,-COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, and a 3-7 membered heterocyclyl containing 1-2 heteroatom ring vertices selected from N, O, and S,
the 3-7 membered heterocyclyl is substituted with 0-3 moieties selected from halo, - OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl, -CHR^{7b}R^{8b}, -OR^{7b}, -OC(O)(R^{7b}), -C(O)(R^{7b}), -C(O)N(R^{7b}R^{8b}), ^{_}C(O)O(R^{7b}), -S(O)₂N(R^{7b}R^{8b}) and -N(R^{7b}R^{8b}), wherein
each R^{7b} and R^{8b} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl.

In some embodiments R¹ in formula XI, XI-A, XI-A-1, XI-A-2, or XI-A-1-a is aryl substituted with 0-3 moieties selected from halo and a 3-7 membered heterocyclyl containing 1-2 heteroatom ring vertices selected from N, O, and S, wherein
the 3-7 membered heterocyclyl is further substituted with 0-3 moieties selected from - OH,-COOH, -NH₂, =O, -CN, and -C₁-C₆ alkyl.

In some embodiments, the compound of formula XI is represented by the compound of Formula XI-B and/or a stereoisomer, a stable isotope, or a pharmaceutically acceptable salt thereof, 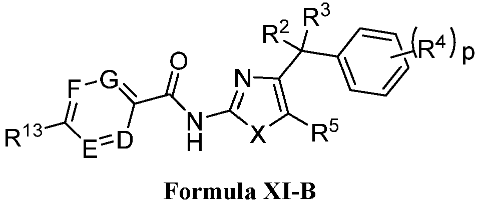
wherein X, p, R², R³, R⁴ and R⁵ are as defined above; and
D is CR¹⁰ or N;
E is CR¹⁴ or N;
F is CR¹² or N;
G is CR¹¹ or N;
provided that no more than three of D, E, F, and G are N;
R¹⁰, R¹¹ , R¹² , R¹³ and R¹⁴, when present, are each independently selected from H, halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, C₁-C₆ haloalkoxyl, -R^{7a}, -X¹-R^{7a}, X¹-OX¹-R^{7a}, -CHR^{7a}R^{8a}, -OR^{7a}, -O-X¹-R^{7a}, -OC(O)( R^{7a}), -O-X¹-C(O)(R^{7a}), -C(O)(R^{7a}), - C(O)N(R^{7a}R^{8a}), -C(O)O(R^{7a}), S(O)₂R^{7a}, -S(O)₂N(R^{7a}R^{8a}), -N(R^{7a}R^{8a}), saturated or unsaturated C₃-C₆ cycloalkyl, saturated or unsaturated C₃-C₆ cycloalkoxyl, saturated or unsaturated 3-7 membered heterocyclyl containing 1-2 heteroatom ring vertices selected from N, O, and S; mono or bicyclic aryl, a 9-10 membered bicyclic heteroaryl containing 1-4 heteroatom ring vertices selected from N, O, and S; saturated or unsaturated 7-8 membered bridged heterocyclyl containing 1-2 heteroatom ring vertices selected from N, O, and S; and saturated or unsaturated 7-11 membered spiroheterocyclyl containing 1-2 heteroatom ring vertices selected from N, O, and S; 6-11 membered bicyclic heterocyclyl containing 1-2 heteroatom ring vertices selected from N, O, and S; wherein
each X¹ is independently C₁₋₆ alkylene;
each R^{7a} and R^{8a} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl; and
the 3-7 membered heterocyclyl, the mono or bicyclic aryl, the 9-10 membered bicyclic heteroaryl, the 7-8 membered bridged heterocyclyl, the 7-11 membered spiroheterocycly, and the 6-11 membered bicyclic heterocyclyl are each independently substituted with 0 to 2 moieties selected from halo, -OH, - COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl, -CHR^{7g}R^{8g}, -OR^{7g}, - OC(O)( R^{7g}), -C(O)( R^{7g}), -C(O)N(R^{7g}R^{8g}), -NR^{7g}(CO)R^{8g}, -C(O)O(R^{7g}), - S(O)₂N(R^{7g}R^{8g}) and -N(R^{7g}R^{8g}), wherein
   each R^{7g} and R^{8g} are each independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl;

In some embodiments, D, E, F and G in Formula XI-B are CR¹⁰, CR¹⁴, CR¹², and CR¹¹, respectively.

In some embodiments, F and G in Formula XI-B are CR¹⁴ and CR¹¹, respectively, E is N or CR¹⁴ and D is N or CR¹⁰.

In some embodiments, R¹⁰ and R¹¹ in Formula XI-B are each H, R¹² and R¹⁴ are each independently selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl, -CHR^{7b}R^{8b}, - OR^{7b}, -OC(O)( R^{7b}), -C(O)( R^{7b}), -C(O)N(R^{7b}R^{8b}), -C(O)O(R^{7b}), -S(O)₂N(R^{7b}R^{8b}) and - N(R^{7b}R^{8b}), wherein R^{7b} and R^{8b} are each independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl; R¹³ is 3-7 membered heterocyclyl containing 1-2 heteroatom ring vertices selected from N, O, and S, saturated or unsaturated 7-8 membered bridged heterocyclyl containing 1-2 heteroatom ring vertices selected from N, O, and S, saturated or unsaturated 7-11 membered spiroheterocyclyl containing 1-2 heteroatom ring vertices selected from N, O, and S, wherein the 3-7 membered heterocyclyl, the 7-8 membered bridged heterocyclyl, and the 7-11 membered spiroheterocyclyl are optionally substituted with 0-2 moieties independently selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl.

In some embodiments, R¹² and R¹⁴ in Formula XI-B are H, R¹⁰ and R¹¹ are each independently selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl, -CHR^{7b}R^{8b}, - OR^{7b}, -OC(O)( R^{7b}), -C(O)( R^{7b}), -C(O)N(R^{7b}R^{8b}), -C(O)O(R^{7b}), -S(O)₂N(R^{7b}R^{8b}) and - N(R^{7b}R^{8b}), wherein R^{7b} and R^{8b} are each independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl; R¹³ is 3-7 membered heterocyclyl containing 1-2 heteroatom ring vertices selected from N, O, and S, saturated or unsaturated 7-8 membered bridged heterocyclyl containing 1-2 heteroatom ring vertices selected from N, O, and S, saturated or unsaturated 7-11 membered spiroheterocyclyl containing 1-2 heteroatom ring vertices selected from N, O, and S, wherein the 3-7 membered heterocyclyl, the 7-8 membered bridged heterocyclyl, and the 7-11 membered spiroheterocyclyl are optionally substituted with 0-2 moieties independently selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl.

In some embodiments, R¹⁰, R¹¹, R¹² and R¹⁴ in Formula XI-B are all H; R¹³ is saturated or unsaturated C₃-C₆ cycloalkyl, 3-7 membered heterocyclyl containing 1-2 heteroatom ring vertices selected from N, O, and S, saturated or unsaturated 7-8 membered bridged heterocyclyl containing 1-2 heteroatom ring vertices selected from N, O, and S, saturated or unsaturated 7-11 membered spiroheterocyclyl containing 1-2 heteroatom ring vertices selected from N, O, and S, wherein the 3-7 membered heterocyclyl, the 7-8 membered bridged heterocyclyl, and the 7-11 membered spiroheterocyclyl are optionally substituted with 0-2 moieties independently selected from halo, -OH, -COOH, -NH₂, =O, - CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl.

In some embodiments, R¹⁰, R¹¹, R¹² and R¹⁴ in Formula XI-B are each H; R¹³ is 3-7 membered heterocyclyl containing 1-2 heteroatom ring vertices selected from N, O, and S w substituted with 0-2 moieties independently selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl.

In some embodiments, R¹⁰, R¹¹, R¹² and R¹⁴ in Formula XI-B are each H; R¹³ is optionally substituted saturated or unsaturated 7-8 membered bridged heterocyclyl containing 1-2 heteroatom ring vertices selected from N, O, and S substituted with 0-2 substituents selected from -OH,-COOH, -NH₂, =O, -CN, and-C₁-C₆ alkyl.

In some embodiments, the compound of Formula XI-B is represented by the compound of Formula XI-B-1 or XI-B-2, and/or a stereoisomer, a stable isotope, or a pharmaceutically acceptable salt thereof

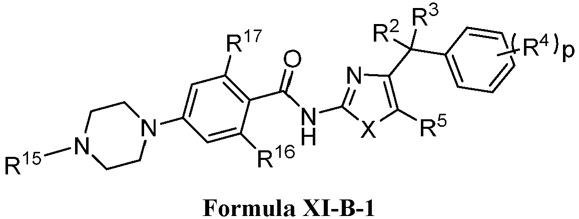

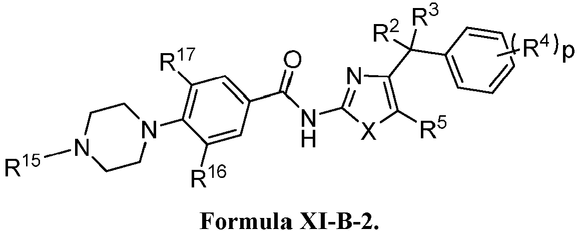

In some embodiments, the compound of Formula XI-B-1or XI-B-2 is represented by the compound of formula XI-B-1-a, XI-B-2-a and/or a stereoisomer, a stable isotope, or a pharmaceutically acceptable salt thereof, 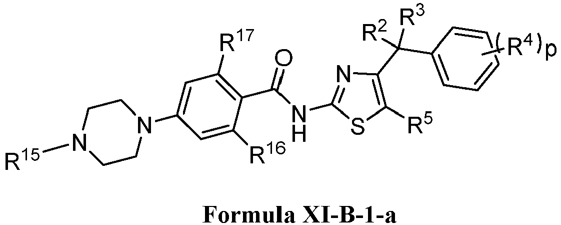 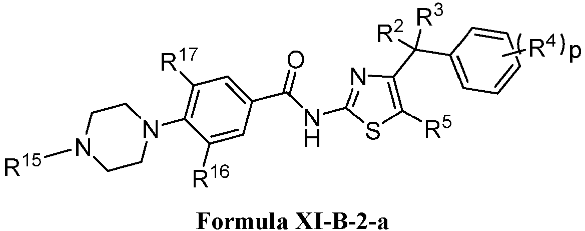
wherein p, R², R³, R⁴ and R⁵ are as defined above; and
R¹⁶ and R¹⁷ are each independently selected from halo and C₁-C₆ alkyl;
R¹⁵ is selected from -OH, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, C₁-C₆ haloalkoxyl, saturated or unsaturated C₃-C₆ cycloalkyl, saturated or unsaturated C₃-C₆ cycloalkoxyl, -CHR^{7b}R^{8b}, -C(O)( R^{7b}), - C(O)N(R^{7b}R^{1b}), ^{_}C(O)O(R^{7b}), -S(O)₂R^{7b} and -S(O)₂N(R^{7b}R^{8b}), wherein
   each R^{7b} and R^{8b} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl.

In some embodiments, R¹⁵ in Formula XI-B-1 or XI-B-2 is selected from C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, C₁-C₆ haloalkoxyl; saturated or unsaturated C₃-C₆ cycloalkyl, saturated or unsaturated C₃-C₆ cycloalkoxyl, -CHR^{7b}R^{8b}, wherein
each R^{7b} and R^{8b} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl.

In some embodiments, R¹⁵ in formula XI-B-1 or XI-B-2 is C₁-C₆ alkyl.

In some embodiments, both R² and R³ in formula XI-B-1 or XI-B-2 are methyl groups.

In some embodiments, R² and R³ in formula XI-B-1 or XI-B-2are each independently a methyl or an ethynyl group.

In some embodiments, XI-B-1-a is represented by Formula XI-B-1-a-I, and XI-B-2-a is represented by Formula XI-B-2-a-I 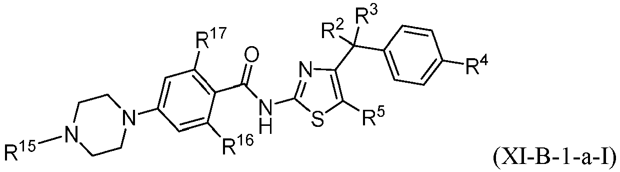 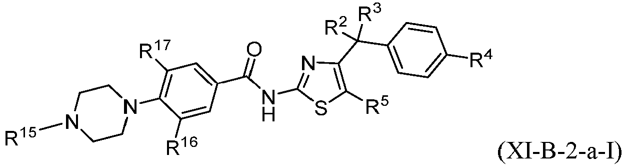 or a pharmaceutically acceptable salt thereof, wherein R⁴ is halo.

In some embodiments, XI-B-1-a is represented by Formula XI-B-1-a-II, and XI-B-2-a is represented by Formula XI-B-2-a-II 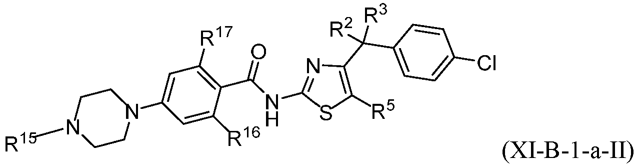 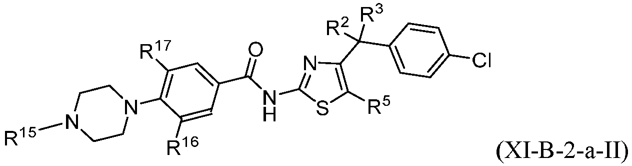 or a pharmaceutically acceptable salt thereof.

In some embodiments, XI-B-1-a is represented by Formula XI-B-1-a-III, and XI-B-2-a is represented by Formula XI-B-2-a-III 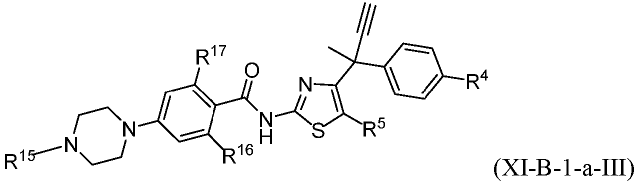 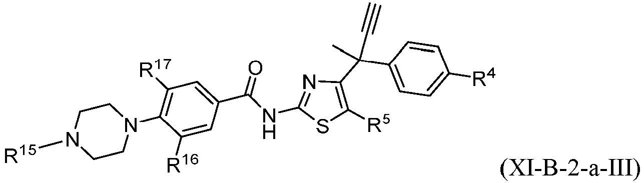 or a pharmaceutically acceptable salt thereof.

In some embodiments, XI-B-1-a is represented by Formula XI-B-1-a-IV, and XI-B-2-a is represented by Formula XI-B-2-a-IV 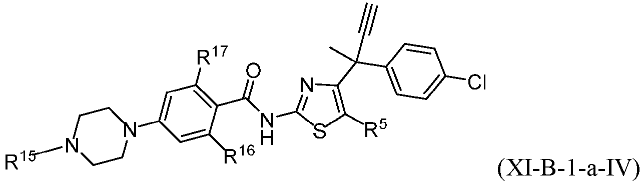 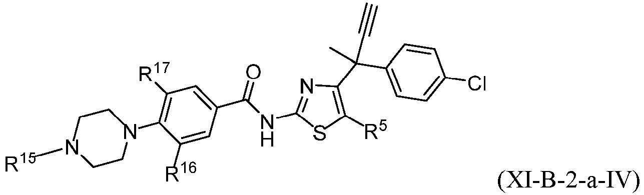 or a pharmaceutically acceptable salt thereof.

In some embodiments, R⁵ in formula XI-B-1 or XI-B-2 is H or methyl.

The present invention discloses novel heterocyclic compounds as inhibitors of ALPK1. The compounds are represented by formula XI-C 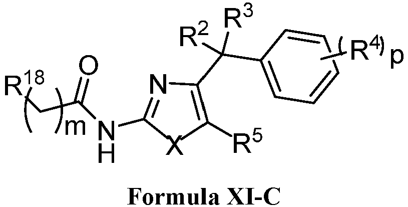
Wherein X, R², R³, R⁴ and R⁵ are as defined above formula XI; and
m is an integer from 0-6;
   R¹⁸ is selected from H, halo, -OH, -COOH, -NH₂, -CN, C₁-C₆ alkenyl, C₁-C₆ hydroxyalkyl, C₁-C₆ haloalkyl, C₁-C₆ aminoalkyl, C₁-C₆ alkoxyl, C₁-C₆ haloalkoxyl, -R^{7a}, -X¹-R^{7a}, CHR^{7a} R^{8a}, -OR^{7a}, -O-X¹-R^{7a}, X¹-O-X¹-R^{7a}, - OC(O)(R^{7a}), -O-X¹-C(O)(R^{7a}), -C(O)(R^{7a}), -C(O)N(R^{7a}R^{8a}), -NR^{7a}(CO)R^{8a}, - C(O)O(R^{7a}), S(O)₂R^{7a}, -S(O)₂N(R^{7a}R^{8a}), -N(R^{7a}R^{8a}), saturated or unsaturated C₃-C₆ cycloalkyl, saturated or unsaturated C₃-C₆ cycloalkoxyl, saturated or unsaturated 3-7 membered heterocyclyl containing 1-2 heteroatom ring vertices selected from N, O, and S, mono or bicyclic aryl, 9-10 membered bicyclic heteroaryl containing 1-4 heteroatom ring vertices selected from N, O, and S, saturated or unsaturated 7-8 membered bridged heterocyclyl containing 1-2 heteroatom ring vertices selected from N, O, and S, saturated or unsaturated 7-11 membered spiroheterocyclyl containing 1-2 heteroatom ring vertices selected from N, O, and S, and 6-11 membered bicyclic heterocyclyl containing 1-2 heteroatom ring vertices selected from N, O, and S; wherein
   each X¹ is independently C₁₋₆ alkylene;
   each R^{7a} and R^{8a} are independently selected from H, C₁-C₆ alkyl, saturated or unsaturated C₃-C₆ cycloalkyl, saturated or unsaturated C₃-C₆ cycloalkoxyl, C₁-C₆ alkenyl, C₁-C₆ hydroxyalkyl, C₁-C₆ haloalkyl, C₁-C₆ aminoalkyl, C₁-C₆ alkoxyl, C₁-C₆ haloalkoxyl, saturated or unsaturated C₃-C₆ cycloalkyl, saturated or unsaturated C₃-C₆ cycloalkoxyl, aryl, saturated or unsaturated C₃-C₆ cycloalkyl, saturated or unsaturated C₃-C₆ cycloalkoxyl, saturated or unsaturated 3-7 membered heterocyclyl containing 1-2 heteroatom ring vertices selected from N, O, and S, wherein the aryl and 3-7 membered heterocyclyl groups are substituted with 0-3 substituents selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl; and
   the C₃-C₆ cycloalkyl, C₃-C₆ cycloalkoxyl, 3-7 membered heterocyclyl, the mono or bicyclic aryl, the 9-10 membered bicyclic heteroaryl, the saturated or unsaturated 7-8 membered bridged heterocyclyl, the saturated or unsaturated 7-11 membered spiroheterocycly, and the 6-11 membered bicyclic heterocyclyl are each independently substituted with 0 to 3 moieties selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl, saturated or unsaturated 3-7 membered heterocyclyl containing 1-2 heteroatom ring vertices selected from N, O, and S, -CHR^{7b}R^{8b}, -OR^{7b}, -OC(O)( R^{7b}), - C(O)( R^{7b}), -C(O)N(R^{7b}R^{8b}), -NR^{7b}(CO)R^{8b}, -C(O)O(R^{7b}), -S(O)₂ N(R^{7b}R^{8b}) and -N(R^{7b}R^{8b}), wherein each R^{7b} and R^{8b} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl.

In some embodiments, the compound of formula XI-C is represented by the compound of formula XI-C-1, and/or a stereoisomer, a stable isotope, or a pharmaceutically acceptable salt thereof, 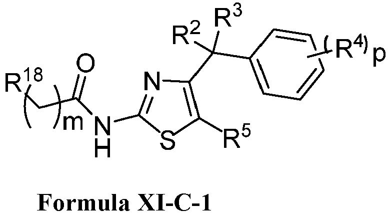 wherein R², R³, R⁴ and R⁵ are as defined above Formula XI; and
m is an integer from 0-6;
R¹⁸ is selected from H, halo, -OH, -COOH, -NH₂, -CN, C₁-C₆ alkenyl, C₁-C₆ hydroxyalkyl, C₁-C₆ haloalkyl, C₁-C₆ aminoalkyl, C₁-C₆ alkoxyl, C₁-C₆ haloalkoxyl, -R^{7a}, -X¹-R^{7a}, CHR^{7a} R^{8a}, -OR^{7a}, -O-X¹-R^{7a}, X¹-O-X¹-R^{7a}, - OC(O)(R^{7a}), -O-X¹-C(O)(R^{7a}), -C(O)(R^{7a}), -C(O)N(R^{7a}R^{8a}), -NR^{7a}(CO)R^{8a}, - C(O)O(R^{7a}), S(O)₂R^{7a}, -S(O)₂N(R^{7a}R^{8a}), -N(R^{7a}R^{8a}), saturated or unsaturated C₃-C₆ cycloalkyl, saturated or unsaturated C₃-C₆ cycloalkoxyl, saturated or unsaturated 3-7 membered heterocyclyl containing 1-2 heteroatom ring vertices selected from N, O, and S, mono or bicyclic aryl, 9-10 membered bicyclic heteroaryl containing 1-4 heteroatom ring vertices selected from N, O, and S, saturated or unsaturated 7-8 membered bridged heterocyclyl containing 1-2 heteroatom ring vertices selected from N, O, and S, saturated or unsaturated 7-11 membered spiroheterocyclyl containing 1-2 heteroatom ring vertices selected from N, O, and S, and 6-11 membered bicyclic heterocyclyl containing 1-2 heteroatom ring vertices selected from N, O, and S; wherein
   each X¹ is independently C₁₋₆ alkylene;
   each R^{7a} and R^{8a} are independently selected from H, C₁-C₆ alkyl, saturated or unsaturated C₃-C₆ cycloalkyl, saturated or unsaturated C₃-C₆ cycloalkoxyl, C₁-C₆ alkenyl, C₁-C₆ hydroxyalkyl, C₁-C₆ haloalkyl, C₁-C₆ aminoalkyl, C₁-C₆ alkoxyl, C₁-C₆ haloalkoxyl, saturated or unsaturated C₃-C₆ cycloalkyl, saturated or unsaturated C₃-C₆ cycloalkoxyl, aryl, saturated or unsaturated C₃-C₆ cycloalkyl, saturated or unsaturated C₃-C₆ cycloalkoxyl, saturated or unsaturated 3-7 membered heterocyclyl containing 1-2 heteroatom ring vertices selected from N, O, and S, wherein the aryl and 3-7 membered heterocyclyl groups are substituted with 0-3 substituents selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl; and
   the C₃-C₆ cycloalkyl, C₃-C₆ cycloalkoxyl, 3-7 membered heterocyclyl, the mono or bicyclic aryl, the 9-10 membered bicyclic heteroaryl, the saturated or unsaturated 7-8 membered bridged heterocyclyl, the saturated or unsaturated 7-11 membered spiroheterocycly, and the 6-11 membered bicyclic heterocyclyl are each independently substituted with 0 to 3 moieties selected from halo, -OH, -COOH, -NH₂, =O, -CN, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, saturated or unsaturated C₃-C₆ cycloalkoxyl, saturated or unsaturated 3-7 membered heterocyclyl containing 1-2 heteroatom ring vertices selected from N, O, and S, -CHR^{7b}R^{8b}, -OR^{7b}, -OC(O)( R^{7b}), - C(O)( R^{7b}), -C(O)N(R^{7b}R^{8b}), -NR^{7b}(CO)R^{8b}, -C(O)O(R^{7b}), -S(O)₂ N(R^{7b}R^{8b}) and -N(R^{7b}R^{8b}), wherein each R^{7b} and R^{8b} are independently selected from H, C₁-C₆ alkyl, C₁-C₆ alkenyl, C₁-C₆ hydroxyalkyl, C₁-C₆ haloalkyl, C₁-C₆ aminoalkyl, C₁-C₆ alkoxyl, saturated or unsaturated C₃-C₆ cycloalkyl, and saturated or unsaturated C₃-C₆ cycloalkoxyl;

In some embodiments, m in formula XI-C or XI-C-1 is 1.

In some embodiments, R¹⁸ in formula XI-C or XI-C-1 is H.

In some embodiments, R² and R³ in each of the formulas described herein are both C₁-C₆ alkyl groups;

In some embodiments, R² is methyl and R³ is CH₂OMe in each of the formulas described herein.

In some embodiments, R² and R³ are each methyl in each of the formulas described herein.

In some embodiments, R² is methyl and R³ is ethynyl in each of the formulas described herein.

In some embodiments, R² is methyl and R³ is C₃-C₆ cycloalkyl.

In some embodiments, R² is methyl, and R³ is phenyl.

In some embodiments, in each of the formulas described herein, the subscript p is 1, and R⁴ is attached to the phenyl ring as shown below: 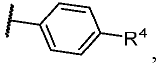 wherein the wavy line represents the point of attachment to the remainder of the formula.

In some embodiments, in each of the formulas described herein, the subscript p is 1, and R⁴ is halo attached to the phenyl ring as shown below: 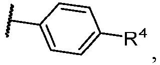 wherein the wavy line represents the point of attachment to the remainder of the formula.

In some embodiments, in each of the formulas described herein, the subscript p is 1, and R⁴ is chloro attached to the phenyl ring as shown below: 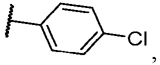 wherein the wavy line represents the point of attachment to the remainder of the formula.

In some embodiments, in each of the formulas described herein, the subscript p is 1, and R⁴ is methoxy attached to the phenyl ring as shown below: 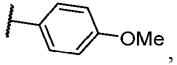 wherein the wavy line represents the point of attachment to the remainder of the formula.

In some embodiments, R⁵ in each of the formulas described herein is H.

In some embodiments, R⁵ in each of the formulas described herein is deuterium.

In some embodiments, R⁵ in each of the formulas described herein is C₁-C₆ deuteroalkyl. In some embodiments, R⁵ in each of the formulas described herein is selected from the group consisintg of -CH₂D, -CHD₂, and -CD₃.

In some embodiments, the carbon atom attached to R² and R³ in each of the formulas described herein is chiral. In such embodiments, it is understood that R² and R³ are not the same. In some embodiments, the carbon atom attached to R² and R³ in each of the formulas described herein is the S isomer, referring to the absolute stereochemistry at this carbon atom. In some embodiments, the carbon atom attached to R² and R³ in each of the formulas described herein is the R isomer, referring to the absolute stereochemistry at this carbon atom. In some embodiments, R² is methyl and R³ is ethynyl. In some embodiments, R² is methyl and R³ is C₃-C₆ cycloalkyl. In some embodiments, R² is methyl, and R³ is phenyl. In some embodiments, R³ is methyl and R² is ethynyl. In some embodiments, R³ is methyl and R² is C₃-C₆ cycloalkyl. In some embodiments, R³ is methyl, and R² is phenyl.

In some embodiments, the compound of Formula XI is selected from

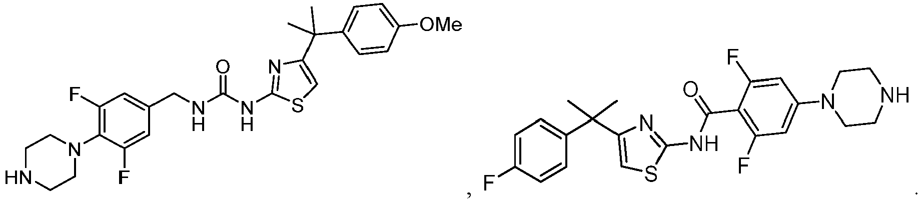

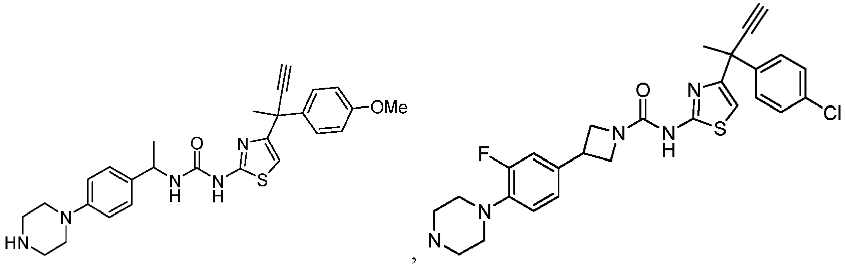

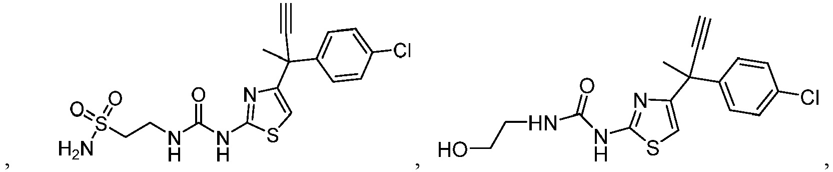

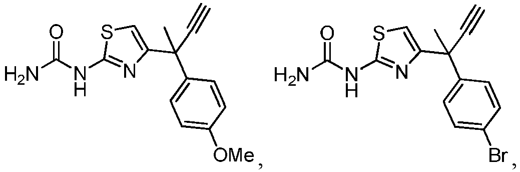

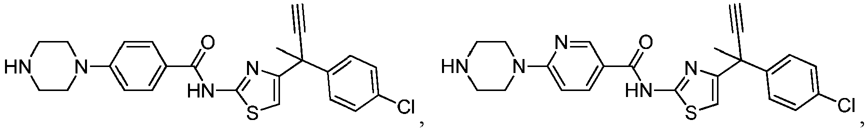

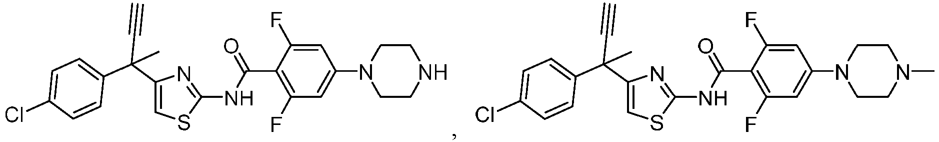

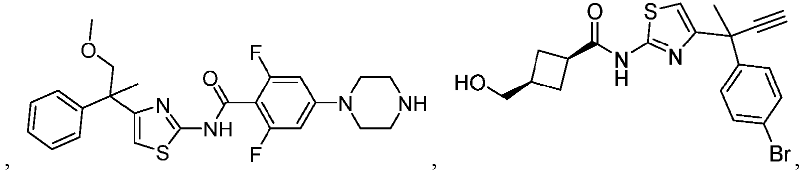

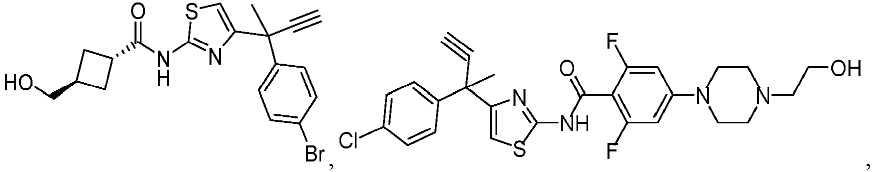

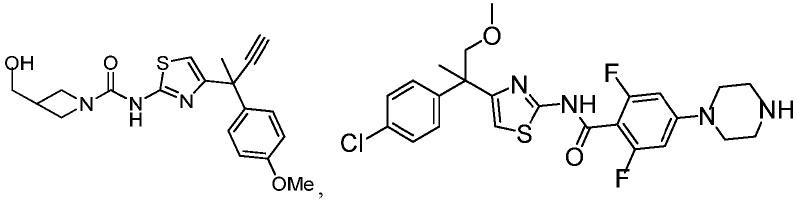

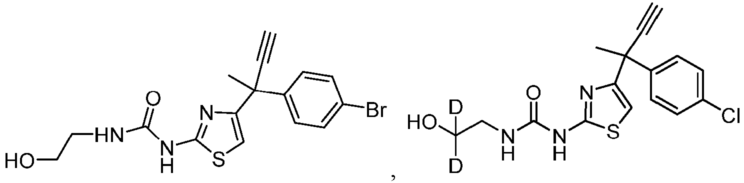

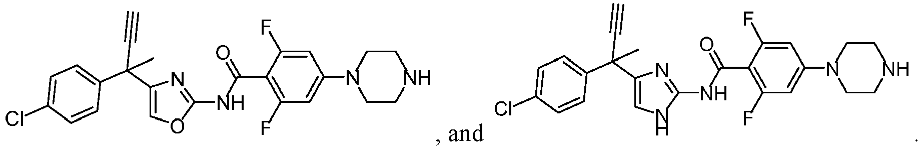

In some embodiments, the compound of Formula XI is selected from 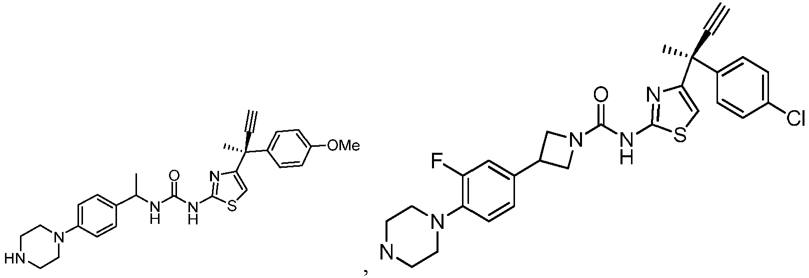 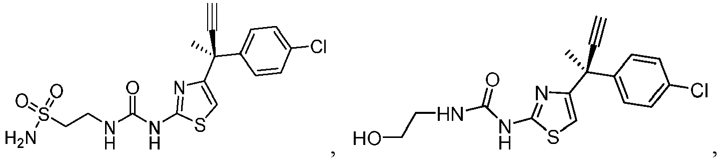 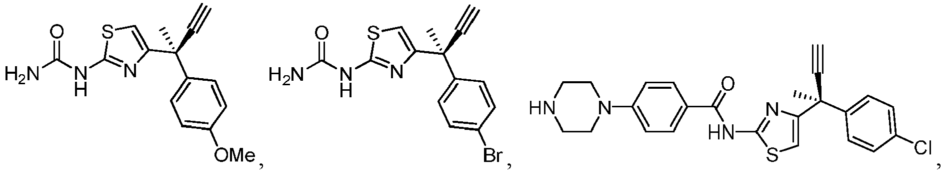 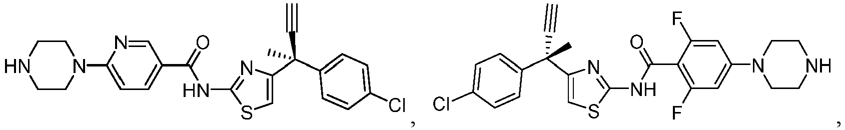 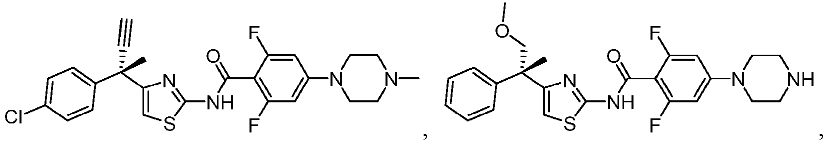 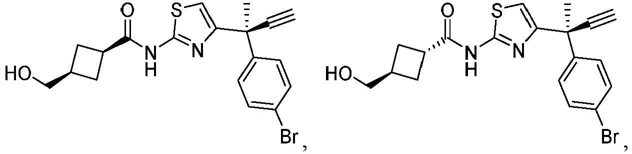 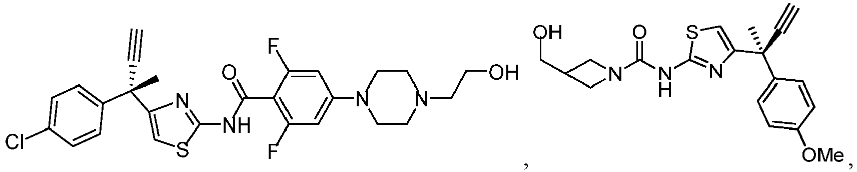 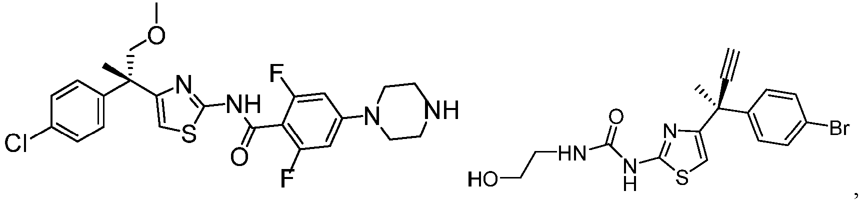 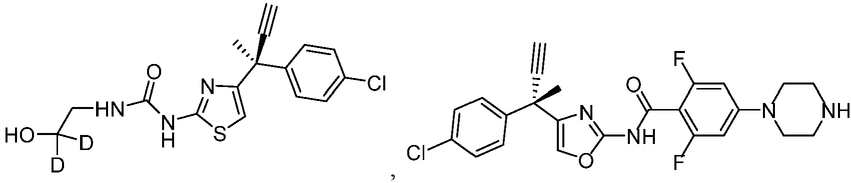 and

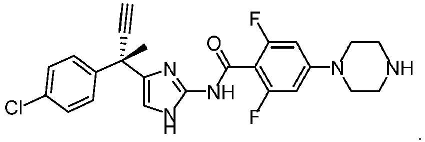

In some embodiments, the compound is selected from the examples provided herein.

### Preparation of Compounds of Formula XI and Exemplary Compounds

### ANALYTICAL DETAILS

The following examples relating to compounds A011-014, A016, A019, A026, A032-035, A037, A042-43, A055, A063, A068-69, A122, A123, D005-007, A260, A280-281, A233, A236, A245, A246, A259 and other compounds that are no longer covered by the claims are present for illustration purposes only.

**NMR:** Measurements were performed on a Bruker Ultrashield TM 400 (400 MHz) spectrometer using or not tetramethylsilane (TMS) as an internal standard. Chemical shifts (δ) are reported ppm downfield from TMS, spectra splitting pattern are designated as single (s), doublet (d), triplet(t), quartet (q), multiplet, unresolved or overlapping signals (m), broad signal (br). Deuterated solvent are given in parentheses and have a chemical shifts of dimethyl sulfoxide (δ2.50 ppm), chloroform (δ 7.26 ppm), methanol (δ3.31 ppm), or other solvent as indicated in NMR spectral data.

**LC-MS:** Shimadzu20A-2010MS
Detection: SPD-M20A
Column: MERCK, RP-18e 25-2mm;
Wavelength: UV 220nm, 254nm ;
Column temperature: 50°C; MS ionization: ESI
Mobile Phase: 1.5ML/4LTFA in water (solvent A) and 0.75ML/4LTFA in acetonitrile (solvent B),using the elution gradient 5%-95% (solvent B) over 0.7 minutes and holding at 95% for 0.4 minutes at a flow rate of 1.5 ml/min;

### Flash Column Chromatography System

System: CombiFlash Rf+
Column: Santai Technologies, Inc, SEPAFLASH ^{®}
Samples were typically adsorbed on isolute

### HPLC separation conditions

System : TRILUTION LC 4.0
Detection: Gilson 159 UV-VIS
Condition 1: Column: Phenomenex Gemini-NX 80*40mm*3um
Eluent A: water (0.05%NH3H2O+10mM NH4HCO3)
Eluent B: CH3CN
Begin B: 20-45%, End B: 80-20%, Gradient Time (min): 8
Condition 2: Column: Xtimate C18 10µ 250 mm *50mm;
Eluent A: water (0.04%NH3H2O+10mM NH4HCO3).
Eluent B: CH3CN 50%-80%; Gradient Time (min): 8

### SFC Chiral Seperation Conditions

Mobile phase: [0.1%NH₃H₂O ETOH]; B%: 30%-30%, 35%-35% or 45-45%
Column: DAICEL CHIRALCEL OJ-H(250mm*30mm,5um);
Mobile phase: [0.1%NH₃H₂O ETOH];B%: 30%-30%, 40%-40%;
Column: DAICEL CHIRALPAK AD (250mm*30mm,10um);
Mobile phase: [0.1%NH₃H₂O ETOH]; B%: 35%-35%;
Column: DAICEL CHIRALPAK AS (250mm*30mm,10um);
Mobile phase: [0.1%NH3H2O ETOH]; B%: 35%-35%

All starting materials, building blocks, reagents, acids, bases, dehydrating agents, solvents, and catalysts utilized to synthesis the compounds of the present invention are either commercially available or can be produced by organic synthesis methods known to one of ordinary skill in the art.

Below is the abbrivation table for chemistry:

| | |
|---|---|
| Ac | Acetyl |
| ACN | Acetonitrile |
| Cbz | Benzoxycarbonyl |
| CDI | N,N-Carbonyldiimidazole |
| Com. | Compound |
| DCM | Dichloromethane |
| DIEA | N,N-Diisopropylethylamine |
| DMAP | 4-Dimethylaminopyridine |
| DMF | N,N-Dimethylformamide |
| DMP | Dess-Martin Periodinane |
| DMSO | Dimethyl sulfoxide |
| EA | Ethyl Acetate |
| EDCI | 1-Ethyl-3-(3-dimethylaminopropyl)carbodiimide |
| ESI | Electron Spray Ionization |
| Et | Ethyl |
| HATU | 2-(7-Azabenzotriazol-1-yl)-N,N,N',N'-tetramethyluronium hexafluorophosphate |
| HBTU | O-Benzotriazole-N,N,N',N'-tetramethyl-uronium-hexafluorophosphate |
| HOBt | 1-Hydroxybenzotriazole |
| HPLC | High Performance Liquid Chromatography |
| Int | Intermediate |
| LCMS | Liquid Chromatography-Mass Spectrometry |
| LiHMDS | Bis(trimethylsilyl)amine lithium salt |
| Me | Methyl |
| MS | Mass Spectrometry |
| Ms | Methanesulfonyl |
| NMR | Nuclear Magnetic Resonance |
| Pd₂(dba)₃ | Tris(dibenzylideneacetone)dipalladium |
| PE | Petroleum Ether |
| PyBOP | Benzotriazol-1-yl-oxytripyrrolidinophosphonium hexafluorophosphate |
| RT | Room Temperature |
| TBAF | Tetrabutylammonium fluoride |
| TBDPS | t-butyldiphenylsilyl |
| TBME | tert-Butyl Methyl Ether |
| t-Bu | tert-butyl |
| TEA | Triethylamine |
| TFA | Trifluoroacetic Acid |
| THF | Tetrahydrofuran |
| TLC | Thin Layer Chromatography |
| TMS | Tetramethylsilane |
| Ts | p-Toluenesulfonyl |
| X-phos | (2-(2,4,6-triisopropylphenethyl)phenyl)dicyclohexylphosphine |

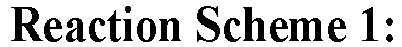

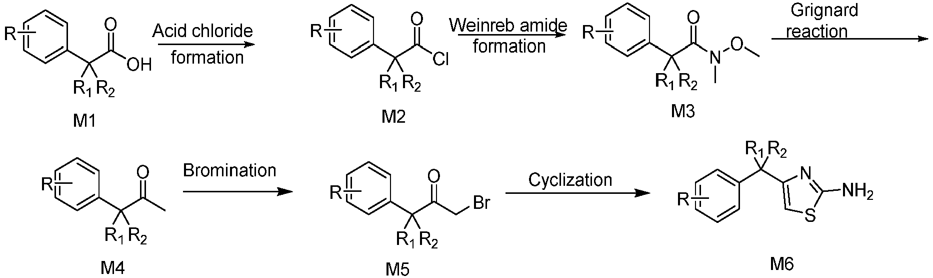

Appropriately substituted compound M1 wherein R are suitable 1-3 groups like halo or C₁-C₆ alkyl, etc, and R₁ and R₂ are suitable groups like independently selected from H, C₁-C₆ alkyl and C₂-C₆ alkynyl, converted to acid chloride with SOCl₂ or (COCl)₂ under heating or room temperature. Weinreb amide was formed by the reaction of N,O-dimethylhydroxylamine hydrochloride with the acid chloride at 0°C. Grignard reagent in THF was added to the Weinreb amide at 0°C to give the ketone, which was converted to M5 by bromination. The cyclization with thiourea under basic condition gave the intermediate M6.

### Example 1: Preparation of 4-(2-(4-bromophenyl)propan-2-yl)thiazol-2-amine (Intermediate 1)

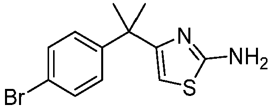

### Step 1. Preparation of compound 2-(4-bromophenyl)-2-methylpropanoyl chloride

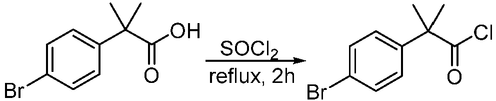

Compound 2-(4-bromophenyl)-2-methylpropanoic acid (100 g, 411 mmol, 1.0 eq) in SOCl₂ (175 mL, 6 eq) was warmed to reflux for 2 h. Then the solution was cooled to RT, the mixture was concentrated under reduced pressure to get dry acid chloride (yellow oil) which was used in next step without further purification.

### Step 2. Preparation of compound 2-(4-bromophenyl)-N-methoxy-N,2-dimethylpropanamide

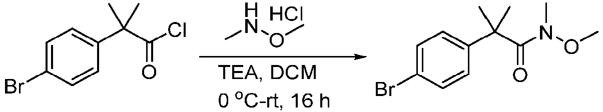

The solution of compound N,O-dimethylhydroxylamine HCl salt (48.2 g, 49 mmol, 1.2 eq) in DCM (300 mL) was cooled to 0 °C. Then to the mixture was added crude acid chloride obtained from step 1 above (1.0 eq) in DCM (200 mL) and TEA (114 mL, 2 eq), and the mixture was stirred at RT overnight. The reaction mixture was quenched with H₂O (200 mL). The mixture was extracted with DCM (200 mL x 3), the combined organic layers were washed with water (200 mL x 3), brine (200 mL x 3), dried over Na₂SO₄, filtered and concentrated to give a residue. The desired compound (108 g, pure) was obtained as a pale yellow oil which was used in next step without further purification.

¹H NMR (400 MHz, CDCl₃) *δ* 7.42 (d, *J* = 8.8 Hz, 2H), 7.12 (d, *J* = 8.8 Hz, 2H), 3.08 (s, 3H), 2.71 (s, 3H), 1.49 (s, 6H).

### Step 3. Preparation of compound 3-(4-bromophenyl)-3-methylbutan-2-one

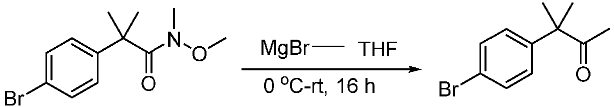

The solution of compound obtained from step 2 above (54 g, 189 mmol, 1 eq) in dry THF (500 mL) was cooled to 0°C. CH₃MgBr (3 M in THF, 253 mL, 757.8 mmol, 4 eq) was added dropwise. The mixture was stirred at RT overnight. The reaction mixture was quenched with *sat.* NH₄Cl (200 mL) and extracted with EA (300 mL x 2). The combined organic layers were washed with brine (300 mL x 2), dried over Na₂SO₄, filtered and concentrated to give a residue. The desired compound (90.4 g, pure) was obtained as a pale yellow oil which was used into the next step without further purification.

¹H NMR (400 MHz, CDCl₃) *δ* 7.45 (d, *J* = 8.4 Hz, 2H), 7.11 (d, *J* = 8.4 Hz, 2H), 1.90 (s, 3H), 1.44 (s, 6H).

### Step 4. Preparation of compound 1-bromo-3-(4-bromophenyl)-3-methylbutan-2-one

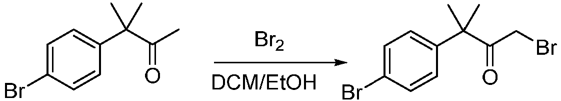

To the solution of compound obtained from step 3 above (46 g, 191 mmol, 1 eq) in DCM/EtOH (250 mL/250 mL) was added Br₂ (14.7 mL, 286 mmol, 1.5 eq) dropwise. The mixture was stirred at RT for 3.5 h. The reaction mixture was quenched with *sat*.Na₂SO₃ (150 mL). The mixture was extracted with DCM (300 mL x 2) and the combined organic layers were washed with brine (300 mL x 2), dried over Na₂SO₄, filtered and concentrated to give a residue. The desired compound (118.8 g, crude) was obtained as a white solid which was used into the next step without further purification.

¹H NMR (400 MHz, CDCl₃) *δ* 7.48 (d, *J* = 8.4 Hz, 2H), 7.11 (d, *J* = 8.4 Hz, 2H), 3.82 (s, 2H), 1.52 (s, 6H).

### Step 5. Preparation of 4-(2-(4-bromophenyl)propan-2-yl)thiazol-2-amine

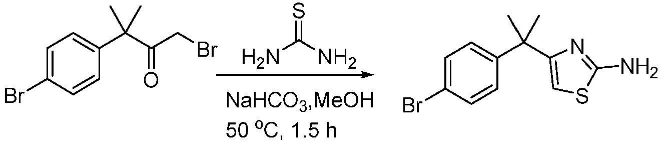

To the solution of compound obtained from step 4 above (50 g, 156 mmol, 1 eq) in MeOH (500 mL) was added thiourea (14.3 g, 188 mmol, 1.2 eq). The mixture was stirred at 50°C for 1.5 h. The mixture was concentrated under reduced pressure. The mixture was extracted with EA (300 mL x 2), the combined organic layers were washed with brine (300 mL x 2), dried over Na₂SO₄, filtered and concentrated to give a residue, the residue was purified by PE/EA=10: 1 on silica gel chromatography to give pure desired compound (34 g, white solid).

¹H NMR (400 MHz, DMSO-*d*₆) δ 7.39 (d, J=8.0 Hz, 2H), 7.14 (d, J=8.0 Hz, 2H), 6.78 (s, 2H), 6.22 (s, 1H), 1.50 (s, 6H). MS (ESI) *m*/*z* (M+H)⁺=297.0.

### Example 2:

### 4-(1-(4-bromophenyl)cyclopentyl)thiazol-2-amine (Intermediate 2)

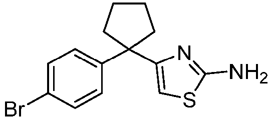

### Step 1. Preparation of compound ethyl 1-(4-bromophenyl)cyclopentane-1-carboxylate

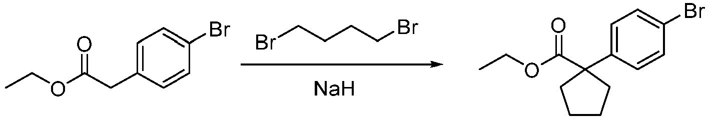

To a solution of compound ethyl 2-(4-bromophenyl)acetate (10 g, 41.3 mmol) in DMF (50 mL) , NaH (8.3 g, 207 mmol) was added slowly at 0°C and then the reaction was stirred at RT for 30 min. 1,4-dibromobutane (8.8 g, 41.3 mmol) was added slowly at RT. The mixture was stirred at RT overnight. The reaction mixture was concentrated to give a residue. The residue was purified by flash silica gel chromatography (PE: EA = 1: 0 to 5: 1). The title compound (7.8 g, yield: 63.8%) was obtained.

MS (ESI) m/z (M+H)⁺=297.0

### Step 2. Preparation of compound 1-(4-bromophenyl)cyclopentane-1-carboxylic acid

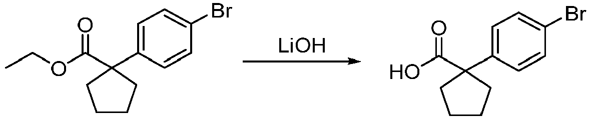

To a solution of compound ethyl 1-(4-bromophenyl)cyclopentane-1-carboxylate (7.8 g, 26.3 mmol) in THF (25 mL) were added NaOH (3.2 g,79 mmol) and H₂O (5 mL) and the reaction was stirred at 40 °C overnight. After cooling down, the PH value of the reaction solution was adjusted to 6. The reaction mixture was concentrated to give a residue. The residue was purified by flash silica gel chromatography (PE: EA = 1: 0 to 1: 2). The desired compound (5.6 g, yield: 79.4%) was obtained.

MS (ESI) m/z (M+H)⁺=269.0
The synthesis of following steps was similar as described in intermediate 1.

### Example 3:

### 4-(2-(5-bromopyridin-2-yl)propan-2-yl)thiazol-2-amine (Intermediate 3)

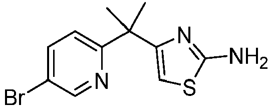

### Step 1. Preparation of compound methyl 2-(5-bromopyridin-2-yl)-2-methylpropanote

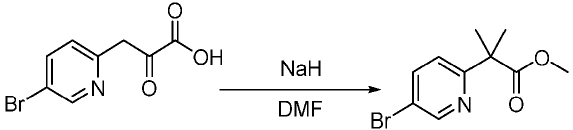

To a solution of 3-(5-bromopyridin-2-yl)-2-oxopropanoic acid (2 g, 9.26 mmol, 1.0 eq) in DMF (20 mL) was added NaH (1.3 g, 32.4 mmol, 3.5eq) at 0°C. The resulting mixture was stirred for 20 min at 0°C. The mixture was added CH₃I (2 mL, 3.5 eq) at 0°C and stirred for 6 h. The reaction mixture was quenched with water (50 mL), extracted with EA (25 mL x 2) and washed with brine (10 mL x 2), then dried over Na₂SO₄, filtered and evaporated to dryness. The resulting residue was purified by column chromatography on a silica gel to obtain the desired compound (1.95 g, yield: 93 %).

### Step 2. Preparation of compound 2-(5-bromopyridin-2-yl)-2-methylpropanoic acid

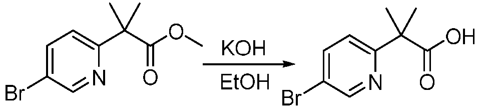

A mixture of 2-(5-bromopyridin-2-yl)-2-methylpropanoate (1.95 g, 7.56 mmol, 1.0eq) and KOH (1.9 mL, 2M in H₂O, 3.0eq) was heated to reflux for 1 h. The reaction was cooled to RT and quenched with 0.1M HCl ,extracted with EA, washed by brine, dried over Na₂SO₄, filtered and evaporated to dryness to obtain the desired compound (1.82 g, yield: 98 %).

The next few steps are similar as described for intermediate 1.

The following examples were synthesized analogous to the procedure of intermediate 1 using the appropriate starting materials and thiourea:

**Table 1**

| Com. ID | Structure | Name | HNMR | LCMS ([M+H]⁺=) |
|---|---|---|---|---|
| Intermediate 4 | 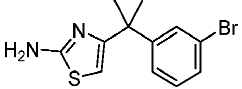 | 4-(2-(3-bromophenyl)propan-2-yl)thiazol-2-amine | ¹H NMR (400MHz, CDCl₃) *δ* 7.67 (t, J = 1.88 Hz, 1 H), 7.43 (d, J = 7.78 Hz, 1 H), 7.33 - 7.39 (m, 1 H), 7.14 - 7.22 (m, 1 H), 6.48 (s, 1 H), 4.87 (br s, 2 H), 2.57 (s, 1 H), 1.91 (s, 3 H). | 297.0 |
| Intermediate 5 | 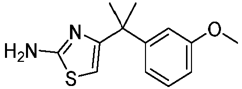 | 4-(2-(3-methoxyphenyl)propan-2-yl)thiazol-2-amine | | 249.1 |
| Intermediate 6 | 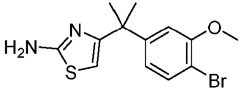 | 4-(2-(4-bromo-3-methoxyphenyl)propan-2-yl)thiazol-2-amine | | 327.0 |
| Intermediate 7 | 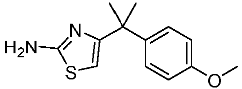 | 4-(2-(4-methoxyphenyl)propan-2-yl)thiazol-2-amine | | 249.1 |
| Intermediate 8 | 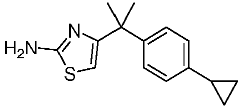 | 4-(2-(4-cyclopropylphenyl)propan-2-yl)thiazol-2-amine | | 259.1 |
| Intermediate 9 | 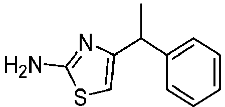 | 4-(1-phenylethyl)thiazol-2-amine | | 205.0 |
| Intermediate 10 | 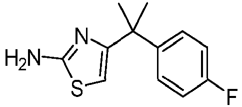 | 4-(2-(4-fluorophenyl)propan-2-yl)thiazol-2-amine | | 237.0 |
| Intermediate 11 | 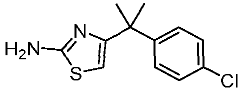 | 4-(2-(4-chlorophenyl)propan-2-yl)thiazol-2-amine | | 253.0 |
| Intermediate 12 | 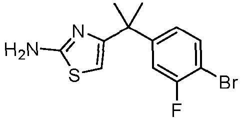 | 4-(2-(4-bromo-3-fluorophenyl)propan-2-yl)thiazol-2-amine | | 314.9 |
| Intermediate 13 | 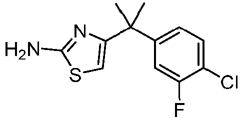 | 4-(2-(4-chloro-3-fluorophenyl)propan-2-yl)thiazol-2-amine | | 271.0 |
| Intermediate 14 | 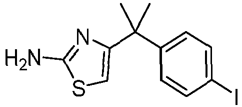 | 4-(2-(4-iodophenyl)propan-2-yl)thiazol-2-amine | | 344.9 |
| Intermediate 15 | 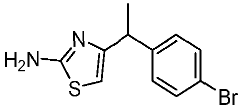 | 4-(1-(4-bromophenyl)ethyl)thiazol-2-amine | | 282.9 |
| Intermediate 16 | 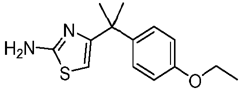 | 4-(2-(4-ethoxyphenyl)propan-2-yl)thiazol-2-amine | | 263.1 |
| Intermediate 17 | 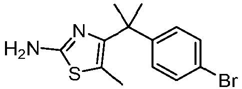 | 4-(2-(4-bromophenyl)propan-2-yl)-5-methylthiazol-2-amine | | 311.0 |
| Intermediate 18 | 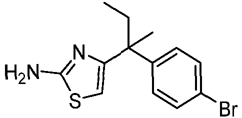 | 4-(2-(4-bromophenyl)butan-2-yl)thiazol-2-amine | | 311.0 |
| Intermediate 19 | 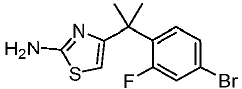 | 4-(2-(4-bromo-2-fluorophenyl)propan-2-yl)thiazol-2-amine | | 315.0 |
| Intermediate 20 | 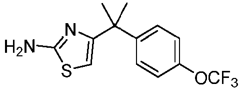 | 4-(2-(4-(trifluoromethoxy)phenyl)propan-2-yl)thiazol-2-amine | | 303.0 |
| Intermediate 21 | 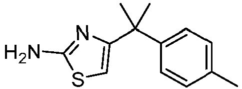 | 4-(2-(p-tolyl)propan-2-yl)thiazol-2-amine | | 233.1 |
| Intermediate 22 | 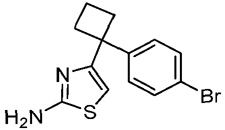 | 4-(1-(4-bromophenyl)cyclobutyl)thiazol-2-amine | ¹H NMR (400MHz, CDCl₃) *δ* 7.45 - 7.41 (m, 2H), 7.22 - 7.17 (m, 2H), 6.05 (s, 1H), 4.88 (s, 2H), 2.74 - 2.65 (m, 2H), 2.62 - 2.51 (m, 2H), 2.20 - 2.04 (m, 1H), 1.97 - 1.85 (m, 1H). | 309.0 |
| Intermediate 23 | 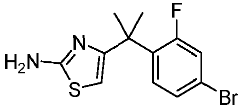 | 4-(2-(4-bromo-2-fluorophenyl)propan-2-yl)thiazol-2-amine | | 314.9 |
| Intermediate 24 | 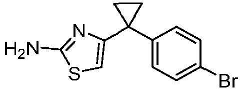 | 4-(1-(4-bromophenyl)cyclopropyl)thiazol-2-amine | | 294.9 |
| Intermediate 25 | 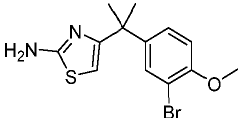 | 4-(2-(3-bromo-4-methoxyphenyl)propan-2-yl)thiazol-2-amine | | 327.0 |
| Intermediate 26 | 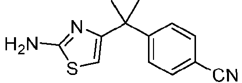 | 4-(2-(2-aminothiazol-4-yl)propan-2-yl)benzonitrile | | 244.1 |

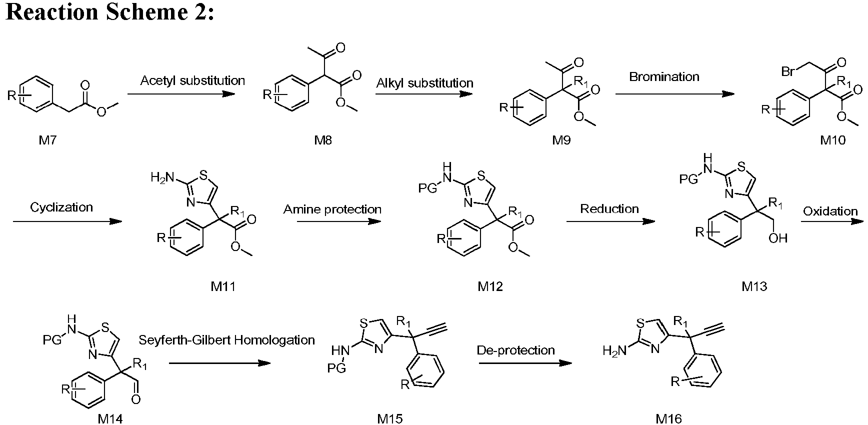

Appropriately substituted compound M7 wherein R was suitable 1-3 groups like halo or C₁-C₆ alkyl, etc, was acetylated with lithium base at lower than -60°C condition. M9 was obtained by alkyl substitution like C1-C6 alkyl group, of M8 under base condition at 50-70°C. After bromination, M10 was obtained. The cyclization of M10 with thiourea under base condition gave the thiazole intermediate M11. An appropriate protection group was introduced to protect amine. The reduction of ester into alcohol was performed by LiBH₄ at 0°C yielding M13, which was oxidized to the corresponding aldehyde by using Dess-Martin Periodinane (DMP) reagent. The alkynylthiazole amine intermediate M15 was obtained by Seyferth-Gilbert Homologation with treating M14 with 1-diazo-1-dimethoxyphosphoryl-propan-2-one under base condition at RT. The final de-protection gave the intermediate M16.

### Example 4:

### Preparation of 4-(2-(4-chlorophenyl)but-3-yn-2-yl)thiazol-2-amine (Intermediate 27)

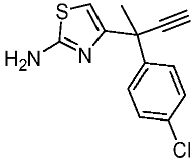

### Step 1. Preparation of compound methyl 2-(4-chlorophenyl)-3-oxobutanoate

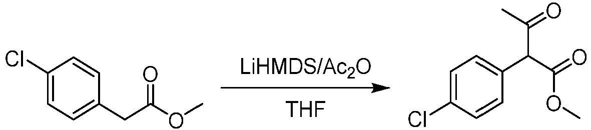

To a solution of compound methyl 2-(4-chlorophenyl)acetate (10g, 54.2 mmol, 8.77 mL) in THF (80 mL) was added dropwise LiHMDS (1M, 65.0 mL) at -78°C. The mixture was stirred at -78°C for 20 min. Then acetyl acetate (5.53 g, 54.17 mmol, 5.07 mL) was added at -78°C. The mixture was warmed to 0°C and stirred for 2 h at 0°C. The mixture was quenched with *sat.* NH₄Cl (200 mL) and extracted with EA (100 mL x 3). The combined organic layers were washed with brine (200 mL), dried over anhydrous Na₂SO₄, filtered and concentrated to give a residue. The residue was purified by flash silica gel chromatography (PE: EA = 1: 0 to 5: 1). The desired compound (7.47 g, yield: 60.9%) was obtained as a pale yellow oil.

MS (ESI) *m*/*z* (M+H)⁺=227.1.

### Step 2. Preparation of compound methyl 2-(4-chlorophenyl)-2-methyl-3-oxobutanoate

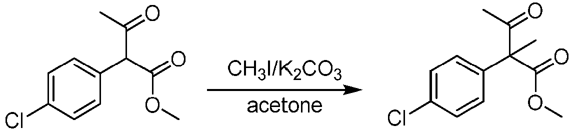

To a solution of compound obtained from step 1 above (7.47 g, 33.0 mmol) and K₂CO₃ (22.8 g, 165 mmol) in acetone (60 mL) was added iodomethane (13.10 g, 92.28 mmol, 5.74 mL). The mixture was stirred at 70 °C for 16 h. The mixture was filtered and the filtrate was concentrated to give a residue. The desired compound (7.79 g, yield: 98.2%) was obtained as a pale yellow oil which was used into the next step without further purification.

MS (ESI) *m*/*z* (M+H)⁺=241.1.

### Step 3. Preparation of compound methyl 4-bromo-2-(4-chlorophenyl)-2-methyl-3-oxobutanoate

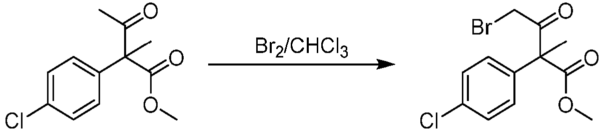

To a solution of compound obtained from step 2 above (7.79 g, 32.4 mmol) in CHCl₃ (80 mL) was added Br₂ (4.66 g, 29.1 mmol, 1.50 mL). The mixture was stirred at 75 °C for 16 h. The reaction mixture was adjust to PH = 6-7 with NaOH (1 N), and then washed with H₂O (100 mL), brined (100 mL), dried over anhydrous Na₂SO₄, filtered and concentrated to give a residue. The desired compound (9.91 g, yield: 95.8%) was obtained as a pale brown oil, which was used into the next step without further purification.

MS (ESI) *m*/*z* (M+H)⁺=319.0.

### Step 4. Preparation of compound methyl 2-(2-aminothiazol-4-yl)-2-(4-chlorophenyl)propanoate

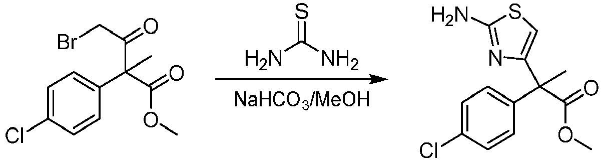

To a solution of compound obtained from step 3 above (9.91 g, 31.0 mmol) and thiourea (2.83 g, 37.2 mmol) in MeOH (60 mL) was added NaHCO₃ (3.13 g, 37.2 mmol, 1.45 mL). The mixture was stirred at 50 °C for 1 h. The reaction mixture was concentrated to give a residue. The precipitate was triturated in H₂O (100 mL) and collected by filtration. The desired compound (8.49 g, yield: 92.3%) was obtained as a brown solid.

MS (ESI) *m*/*z* (M+H)⁺=297.0.

### Step 5. Preparation of compound methyl 2-(2-acetamidothiazol-4-yl)-2-(4-chlorophenyl)propanoate

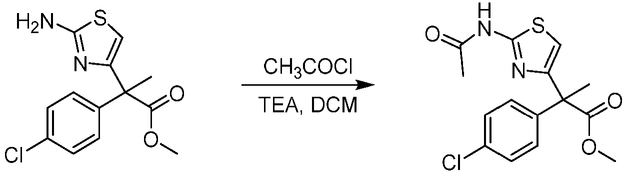

To a solution of compound obtained from step 4 above (3 g, 10.1 mmol) and TEA (1.53 g, 15.2 mmol, 2.11 mL) in DCM (60 mL) was added acetyl chloride (794 mg, 10.11 mmol, 721 uL) at 0 °C. The mixture was stirred at 25 °C for 1.5 h. The second batch of acetyl chloride (794 mg, 10.1 mmol, 721 uL) and TEA (1.53 g, 15.2 mmol, 2.11 mL) was added at 0°C, the mixture was stirred at 25 °C for 1 h. The third batch of acetyl chloride (793.5 mg, 10.11 mmol, 721.38 uL) and TEA (1.53 g, 15.16 mmol, 2.11 mL) was added at 0°C, the mixture was stirred at 25 °C for 1.5 h. The reaction mixture was quenched with H₂O (3 mL) and then added anhydrous Na₂SO₄, filtered and concentrated to give a residue. The residue was purified by flash silica gel chromatography (PE: EA = 1: 0 to 2: 1). The desired compound (1.4 g, yield: 32.6%) was obtained as a pale yellow solid.

MS (ESI) *m*/*z* (M+H)⁺=339.1.

### Step 6. Preparation of compound N-(4-(2-(4-chlorophenyl)-1-hydroxypropan-2-yl)thiazol-2-yl)acetamide

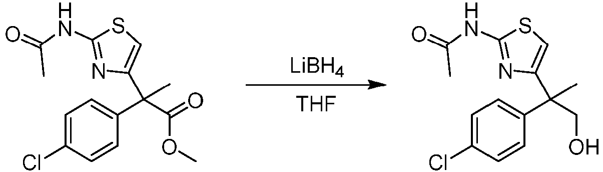

To a solution of compound obtained from step 5 above (1.4 g, 4.13 mmol) in THF (50 mL) was added partly LiBH₄ (450 mg, 20.66 mmol). The mixture was stirred at 25 °C for 16 h. The reaction mixture was quenched with *sat.* NH₄Cl (40 mL) and then extracted with EA (30 mL x 3), the combined organic layer was washed with brine (60 mL), dried over anhydrous Na₂SO₄, filtered and concentrated to give a residue. The residue was purified by flash silica gel chromatography (PE: EA = 1: 0 to 2: 3). The desired compound (970 mg, yield: 73.4%) was obtained as a pale yellow solid.

MS (ESI) *m*/*z* (M+H)⁺=311.1.

### Step 7. Preparation of compound N-(4-(2-(4-chlorophenyl)-1-oxopropan-2-yl)thiazol-2-yl)acetamide

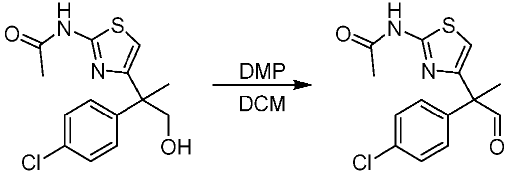

To a solution of compound obtained from step 6 above (970 mg, 3.12 mmol) in DCM (30 mL) was added partly DMP (1.72 g, 4.06 mmol) in DCM (20 mL). The mixture was stirred at 25 °C for 2 h. DMP (1.72 g, 4.06 mmol) in DCM (20 mL) was added and the mixture was stirred at 25 °C for 1 h. DMP (1.06 g, 2.50 mmol) in DCM (20 mL) was added and the mixture was stirred at 25 °C for 2 h. The reaction mixture was diluted with DCM (40 mL), quenched with *sat.* Na₂S₂O₃/ *sat.* NaHCO₃ (1/ 1, 200 mL), the organic layer was separated and the aqueous layer was extracted with DCM (60 mL), the combined organic layers were washed with *sat.* Na₂S₂O₃/ *sat.* NaHCO₃ (1/ 1, 100 mL), water (200 mL x 2), brine (200 mL x 2), dried over anhydrous Na₂SO₄, filtered and concentrated to give a residue. The desired compound (1.03 g, crude) was obtained as a yellow solid which was used into the next step without further purification.

### Step 8. Preparation of compound N-(4-(2-(4-chlorophenyl)but-3-yn-2-yl)thiazol-2-yl)acetamide

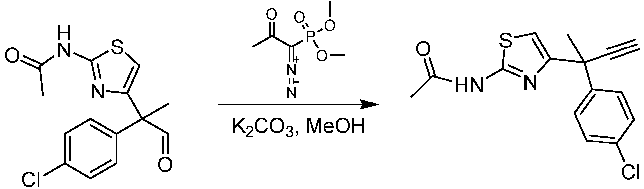

To a solution of compound obtained from step 7 above (1.03 g, 3.34 mmol) and 1-diazo-1-dimethoxyphosphoryl-propan-2-one (961 mg, 5.00 mmol) in MeOH (40 mL) was added K₂CO₃ (922 mg, 6.67 mmol). The mixture was stirred at 25 °C for 12 h. The reaction mixture was concentrated to give a residue. The residue was purified by flash silica gel chromatography (PE: EA = 1: 0 to 1: 1). The residue was purified by prep-HPLC (column: Venusil ASB Phenyl 150 x 30 mm x 5 um; mobile phase: [water (0.05% HCl)-ACN]; B%: 55%-85%, 9 min). The desired compound (219 mg, yield: 21.54%) was obtained as a white solid.

¹H NMR (400MHz, CDCl₃) *δ* 9.98 (br s, 1H), 7.45 (d, *J =* 8.5 Hz, 2H), 7.30 (d, *J* = 8.5 Hz, 2H), 6.88 (s, 1H), 2.63 (s, 1H), 2.25 (s, 3H), 1.99 (s, 3H). MS (ESI) *m*/*z* (M+H)⁺=305.1.

### Step 9. Preparation of compound 4-(2-(4-chlorophenyl)but-3-yn-2-yl)thiazol-2-amine

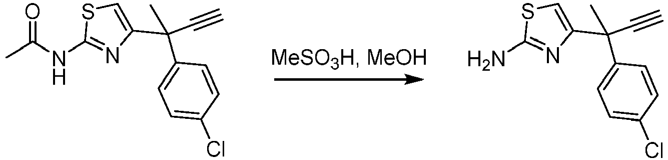

To a solution of compound obtained from step 8 above (180 mg, 591 umol) in MeOH (10 mL) was added methanesulfonic acid (284 mg, 2.95 mmol, 210 µL). The mixture was stirred at 80 °C for 16 h. The reaction mixture was adjusted pH = 9-10 with solid NaHCO₃ and concentrated to give a residue. The residue was purified by flash silica gel chromatography (PE: EA = 1: 0 to 2: 1). The desired compound (137 mg, yield: 88.3%) was obtained as a pale yellow solid.

¹H NMR (400MHz, CDCl₃) *δ* 7.39 - 7.32 (m, 2H), 7.20 - 7.16 (m, 2H), 6.35 (s, 1H), 4.90 (br s, 2H), 2.46 (s, 1H), 1.82 (s, 3H). MS (ESI) *m*/*z* (M+H)⁺=263.0.

The following examples were synthesized analogous to the procedure of example 4 (intermediate 27) using the appropriate starting materials and thiourea:

**Table 2**

| Com. ID | Structure | Name | HNMR | LCMS ([M+H]⁺=) |
|---|---|---|---|---|
| Intermediate 28 | 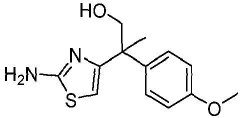 | 2-(2-aminothiazol-4-yl)-2-(4-methoxyphenyl)propan-1-ol | | 265.1 |
| Intermediate 29 | 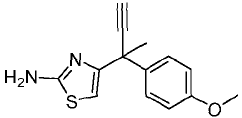 | 4-(2-(4-methoxyphenyl)but-3-yn-2-yl)thiazol-2-amine | | 259.1 |
| Intermediate 30 | 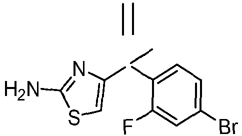 | 4-(2-(4-bromo-2-fluorophenyl)but-3-yn-2-yl)thiazol-2-amine | ¹H NMR (400 MHz, DMSO-*d*₆) *δ* 7.52 - 7.46 (m, 1H), 7.45 - 7.39 (m, 2H), 6.93 (s, 2H), 6.42 (s, 1H), 3.44 (s, 1H), 1.84 (s, 3H). 19F NMR (376 MHz, DMSO-d6) δ - 107.657 | 324.9 |
| Intermediate 31 | 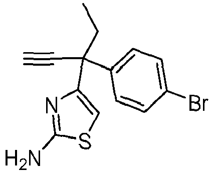 | 4-(3-(4-bromophenyl)pent-1-yn-3-yl)thiazol-2-amine | ¹H NMR (400MHz, DMSO-*d*₆) *δ* 7.60 - 7.58 (m, 2 H), 7.43 - 7.39 (m, 2 H), 6.74 (s, 1 H), 3.77 (s, 1 H), 2.32 - 2.23 (m, 1 H), 2.18 - 2.11 (m, 1 H), 0.83 (t, J =7.2 Hz, 3 H) | 321.0 |
| Intermediate 32 | 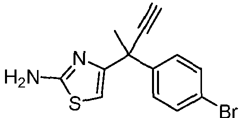 | 4-(2-(4-bromophenyl)but-3-yn-2-yl)thiazol-2-amine | | 306.9 |

### Example 5:

### 4-(2-(4-bromophenyl)-1-methoxypropan-2-yl) thiazol-2-amine (Intermediate 33)

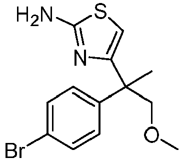

### Step 1. Preparation of compound N-(4-(2-(4-bromophenyl)-1-methoxypropan -2-yl)thiazol-2-yl)acetamide

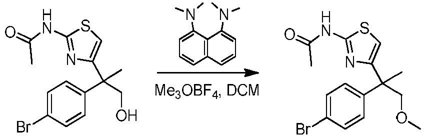

To a solution of N-(4-(2-(4-bromophenyl)-1-hydroxypropan-2-yl)thiazol-2-yl) acetamide (200 mg, 563 µmol, synthesized in the similar method described in intermediate 46) and N1,N1,N8,N8-tetramethylnaphthalene-1,8 -diamine (603 mg, 2.81 mmol) in DCM (10 mL) was added trimethyloxonium;tetrafluoroborate (416 mg, 2.8 mmol) at 0°C. The mixture was stirred at 25 °C for 16 h. The reaction mixture was diluted with DCM (10 mL), quenched with NH₃.H₂O (10 mL), washed with H₂O (30 mL), HCl (1 N, 20 mL), *sat.* NaHCO₃ (20 mL) and brine (40 mL), dried over anhydrous Na₂SO₄, filtered and concentrated to give a residue. The residue was purified by flash silica gel chromatography (PE: EA = 1: 0 to 1: 1). The desired compound (41 mg, yield: 19.72%) was obtained as a white solid.

¹H NMR (400 MHz, CDCl₃) *δ* 8.69 (br s, 1H), 7.39 (d, *J* = 8.5 Hz, 2H), 7.10 (d, *J =* 8.5 Hz, 2H), 6.69 (s, 1H), 3.80 (s, 2H), 3.34 (s, 3H), 2.20 (s, 3H), 1.68 (s, 3H). MS (ESI) *m*/*z* (M+H)⁺=371.0.

### Step 2. Preparation of compound 4-(2-(4-bromophenyl)-1-methoxypropan-2-yl) thiazol-2-amine

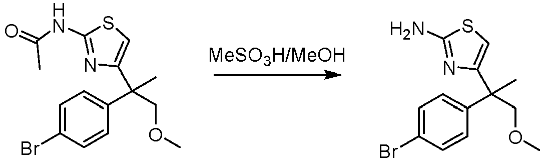

The synthesis is similar as described in intermediate 44. The desired compound (20 mg, yield: 90.3%) was obtained as a white solid.

¹H NMR (400 MHz, CDCl₃) *δ* 7.42 - 7.36 (m, 2H), 7.18 - 7.13 (m, 2H), 6.22 (s, 1H), 4.83 (br s, 2H), 3.84 - 3.73 (m, 2H), 3.34 (s, 3H), 1.65 (s, 3H). MS (ESI) *m*/*z* (M+H)⁺= 327.0.

The following intermediates were synthesized analogous to the procedure of example 5 (intermediate 33) using the appropriate starting materials and thiourea:

**Table 3**

| Com. ID | Structure | Name | HNMR | LCMS ([M+H]⁺=) |
|---|---|---|---|---|
| Intermediate 34 | 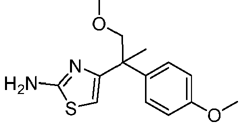 | 4-(1-methoxy-2-(4-methoxyphenyl)propan-2-yl)thiazol-2-amine | | 279.1 |
| Intermediate 35 | 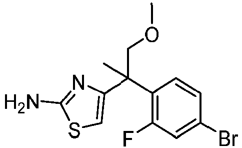 | 4-(2-(4-bromo-2-fluorophenyl)-1-methoxypropan-2-yl)thiazol-2-amine | | 345.0 |
| Intermediate 36 | 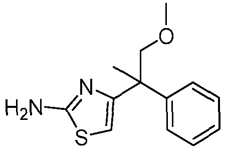 | 4-(1-methoxy-2-phenylpropan-2-yl)thiazol-2-amine | | 248.1 |
| Intermediate 37 | 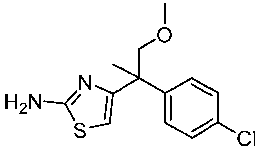 | 4-(2-(4-chlorophenyl)-1-methoxypropan-2-yl)thiazol-2-amine | | 282.1 |

### Example 6:

### 1-(2-aminothiazol-4-yl)-1-(4-bromophenyl)ethan-1-ol (Intermediate 38)

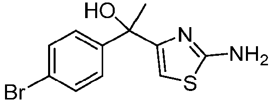

### Step 1. Preparation of compound 1-(4-bromophenyl)propane-1,2-dione

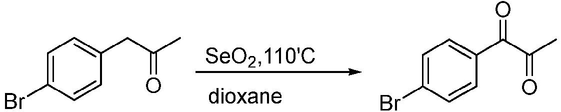

To a solution of compound 1-(4-bromophenyl)propan-2-one (2.0 g, 9.4 mmol, 1.0 eq) in dioxane (20 mL) was added SeO₂ (3.12 g, 28.1 mmol, 3.0 eq). The mixture was stirred at 110 °C for 4 h. After cooling down, the reaction mixture was concentrated to give a residue. The residue was purified by flash silica gel chromatography (PE: EA =96%: 4%). The desired compound (960 mg, yield: 45%) was obtained as a yellow oil.

### Step 2. Preparation of compound 3-bromo-1-(4-bromophenyl)propane-1,2-dione

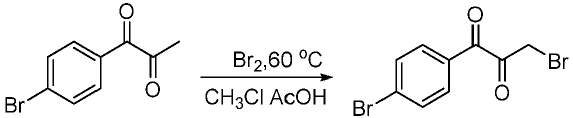

To a solution of compound obtained from step 1 above (960 mg, 4.23 mmol,1.0 eq) in CH₃Cl (20 mL) was added Br₂ (1.05 g, 6.34 mmol,1.5 eq) and AcOH (3 drops). The mixture was stirred at 60°C for 16 h. The reaction mixture was quenched by *sat*.Na₂SO₃ (aq) (20 mL), extracted with DCM (20 mL x 2) and washed with brine (15 mL), then dried over Na₂SO₄, filtered and evaporated to dryness. The residue was purified by flash silica gel chromatography (PE: EA =94%: 6%). The desired compound (800 mg, yield: 74%) was obtained as a yellow oil.

### Step 3. Preparation of compound (2-aminothiazol-4-yl)(4-bromophenyl)methanone

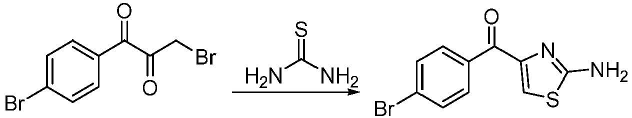

To a solution of compound obtained from step 2 above (800 mg, 2.62 mmol,1.0 eq) in MeOH (8 mL) was added thiourea (200 mg, 2.62 mmol, 1.0 eq) and NaHCO₃. The mixture was stirred at 50 °C for 1.5 h. The mixture was concentrated under reduced pressure, extracted with EA (15 mL x 2), the combined organic layers were washed with brine (10 mL x 2), dried over Na₂SO₄, filtered and concentrated to give a residue, which was purified by flash silica gel chromatography (PE: EA=3: 1) to get the desired group (680 mg, yield: 90%).

### Step 4 Preparation of compound 1-(2-aminothiazol-4-yl)-1-(4-bromophenyl)ethan-1-ol

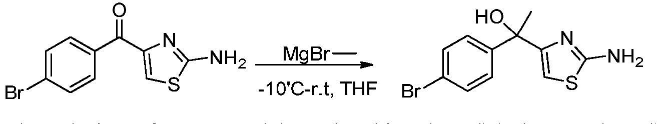

The solution of compound (2-aminothiazol-4-yl)(4-bromophenyl)methanone (200 mg, 0.71 mmol, 1.0 eq) in dry THF (4 mL) was cooled to 0°C, and was added CH₃MgBr (3 M in THF, 1.6 mL, 4.9 mmol, 7.0 eq) dropwise. The mixture was stirred at RT overnight. The reaction mixture was quenched with *sat.* NH₄Cl (200 mL), The mixture was extracted with EA (20 mL x 2), the combined organic layers were washed with brine (10 mL x 2), dried over Na₂SO₄, filtered and concentrated to give a residue. The resulting residue was purified by Prep-TLC to give the desired compound (40 mg, yield: 20%).

¹H NMR (400 MHz, DMSO) *δ* 7.45 - 7.38 (m, 2H), 7.22 (t, J = 7.5 Hz, 2H), 7.12 (t, J = 7.3 Hz, 1H), 6.77 (s, 2H), 6.30 (s, 1H), 5.37 (s, 1H), 1.67 (s, 3H).

MS (ESI) m/z (M+H)⁺=221.0

### Example 7:4-(2-(4-chlorophenyl)but-3-yn-2-yl)thiazol-5-d-2-amine

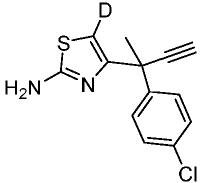

### Step 1. Preparation of compound N-(5-bromo-4-(2-(4-chlorophenyl)but-3-yn-2-yl)thiazol-2-yl)acetamide

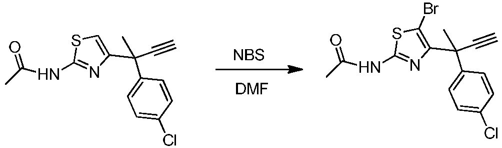

The mixture of N-[4-[1-(4-chlorophenyl)-1-methyl-prop-2-ynyl]thiazol-2-yl]acetamide (1 g, 3.28 mmol ) and NBS (700.74 mg, 3.94 mmol) in DMF (10 mL) was stirred at 50 °C for 2 h. The reaction was cooled to room temperature and then diluted with H₂O (50 mL), extracted with EtOAc (30 mL x 3), the organic phase was combined and washed with brine (50 mL x 3), concentrated to give a residue. The residue was purified by flash silica gel chromatography (PE: EA = 1: 0 to 3:1). The desired compound (800 mg, yield: 52.6 %) was obtained as a yellow solid.

¹H NMR (400MHz, CDCl₃) *δ* 8.89 (br. s, 1H), 7.33-7.41 (m, 2H), 7.24-7.32 (m, 2H), 2.61 (s, 1H), 2.29 (s, 3H), 2.00 (s, 3H). MS (ESI) *m*/*z* (M+H)⁺= 384.8.

### Step 2. Preparation of compound 4-[1-(4-chlorophenyl)-1-methyl-prop-2-ynyl]-5-deuterio-thiazol-2-amine

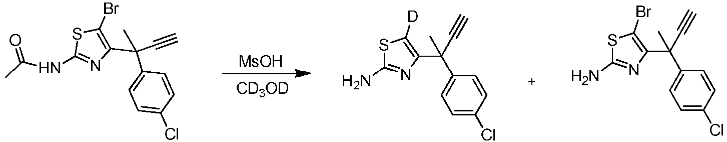

The mixture of compound obtained from step 1 above (600 mg, 1.56 mmol) and MsOH (751.43 mg, 7.82 mmol) in CD₃OD (8 mL) was stirred at 80 °C for 16 h. The reaction was adjusted to pH = 8-9 with sat. NaHCO₃ aqueous, and then extracted with EtOAc (30 mL x 3), the organic phase was combined and washed with brine (30 mL), concentrated to give a residue. The residue was purified by silica gel chromatography (PE: EA=1:0 to 3:1) to give the products, which was re-purified by Pre-TLC (PE: EA=3:1). The desired compound (100 mg, yield: 20.8 %) was obtained as a yellow oil.

¹H NMR (400MHz, CDCl₃) *δ* 8.89 (br. s, 1H), 7.33-7.41 (m, 2H), 7.24-7.32 (m, 2H), 2.61 (s, 1H), 2.29 (s, 3H), 2.00 (s, 3H). MS (ESI) *m*/*z* (M+H)⁺= 263.8.

At the same time, the byproduct 5-bromo-4-[1-(4-chlorophenyl)-1-methyl-prop-2-ynyl]thiazol-2-amine (300 mg, yield: 52.2%) was obtained as a yellow solid.

MS (ESI) *m*/*z* (M+H)⁺= 343.1.

### General Method I

To a solution of thiazole amines (1 eq) and in appropriate organic solvent like DMF was added NaH (1.2-1.5 eqiv.) at 0-10°C, the resulting mixture was stirred for 5-30 mins. The mixture was added activated amine by CDI and stirred for 4-16 hours. Once the reaction was completed, the resulting suspension was diluted with organic solvent and washed with brine and then dried. After filtration and evaporation, the resulting residue was purified by trituration/Prep-TLC/chromatography/Prep-HPLC to give the product.

### Example 8: Preparation of tert-butyl 4-(4-((3-(4-(2-(4-chloro-3-fluorophenyl)propan-2-yl)thiazol-2-yl)ureido)methyl)phenyl)piperazine-1-carboxylate

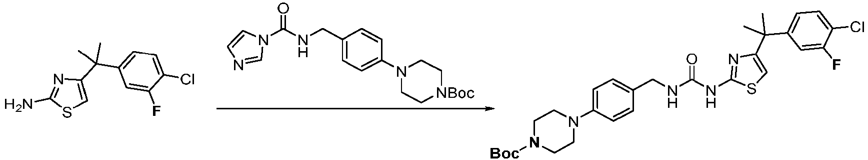

To a solution of 4-(2-(4-chloro-3-fluorophenyl)propan-2-yl)thiazol-2-amine (40 mg, 0.15mmol, 1 eq) and in DMF (5 mL) was added NaH (7 mg, 0.3mmol, 2 eq) at 10°C. The resulting mixture was stirred for 5min. The mixture was added tert-butyl 4-(4-((1H-imidazole-1-carboxamido)methyl)phenyl)piperazine-1-carboxylate (58 mg, 0.15 mmol, 1eq), and stirred overnight. The reaction was quenched with water, extracted with EA and combined organic layers were washed with brine then dried (Na₂SO₄), filtered and evaporated to dryness. The resulting residue was purified by Prep-TLC (PE: EA=3: 1) to give the title compound 35 mg (0.06 mmol) with the yield 40%. MS (ESI) *m*/*z* (M+H)⁺=588.2

### General Method II

To a solution of amine fragment (1 eq) and pyridine in appropriate solvent like dry DCM was added phenyl carbonochloridate (2 eq) below 20°C slowly. The mixture was stirred at RT for 4-6 h. Once the reaction was completed, the resulting reaction was diluted with organic solvent and washed with brine and then dried. After filtration and evaporation, the resulting residue was purified by trituration/Prep-TLC/chromatography/Prep-HPLC to give the product.

### Example 9: Preparation of tert-butyl 4-(5-((3-(4-(2-(4-bromophenyl)propan-2-yl)thiazol-2-yl) ureido)methyl)pyrimidin-2-yl)piperazine-1-carboxylate

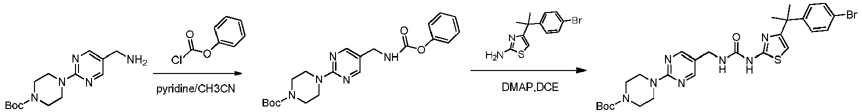

Phenyl carbonochloridate (336mg, 2.2 mmol, 269.0 µL) was added to the mixture of tert-butyl 4-(5-(aminomethyl)pyrimidin-2-yl)piperazine-1-carboxylate (600 mg, 2.1 mmol), pyridine (194 mg, 2.5 mmol, 198µL) in CH₃CN (15 mL) at - 20°C. After addition, the mixture was allowed to warm to 25 °C and stirred at 25 °C for 0.25 h. The solvent was removed under vacuum. The residue was triturated with ice water (15 mL). White solid was precipitated from the mixture. The mixture was filtered and the solid was collected, dried under vacuum. Tert-butyl 4-(5-(((phenoxycarbonyl)amino)methyl)pyrimidin-2-yl)piperazine-1-carboxylate (420 mg, yield: 38.2%) was obtained as a white solid. MS (ESI) m/z (M+H)⁺ = 414.2.

To the mixture of tert-butyl 4-(5-(((phenoxycarbonyl)amino)methyl)pyrimidin-2-yl) piperazine-1-carboxylate (139 mg, 336 µmol) and 4-(2-(4-bromophenyl) propan-2-yl)thiazol-2-amine (50 mg, 168 µmol) in DCE (10 mL) was added DMAP (41.0 mg, 337.0 µmol, 2 eq). The mixture was stirred at 85°C for 16 h. The mixture was concentrated under vacuum. The residue was purified by prep-TLC (SiO₂, DCM: MeOH = 13: 1) and further purified by prep-TLC (SiO2, DCM: MeOH = 12: 1). The desired compound (60 mg, yield: 57.7%) was obtained as a white solid.

MS (ESI) *m*/*z* (M+H)⁺=616.2.

### General Method III

To a solution of substituted thiazol-2-amine and hunig base or pyridine in appropriate solvent like DCM or CH₃CN, or DCM/water was added phenyl carbonochloridate (2 eq) at 0°C-RT slowly. The mixture was stirred 2-4 h at RT and the resulting reaction was diluted with organic solvent and washed with brine and then dried. After filtration and evaporation, the resulting residue was purified by chromatography to give the substituted thiazol-2-amine carbamate.

The mixture of the substituted thiazol-2-amine carbamate, amine and DMAP in appropriate solvent like THF was heated to reflux for 1-2 h. After cooling down, the resulting reaction evaporated and diluted with appropriate organic solvent like EA and washed with brine and then dried. After filtration and evaporation, the resulting residue was purified by trituration/Prep-TLC/chromatography/Prep-HPLC to give the product.

### Example 10: Preparation of 1-(4-(4-((tert-butyldimethylsilyl)oxy)piperidin-1-yl)benzyl)-3 -(4-(2-(4-methoxyphenyl)propan-2-yl)thiazol-2-yl)urea

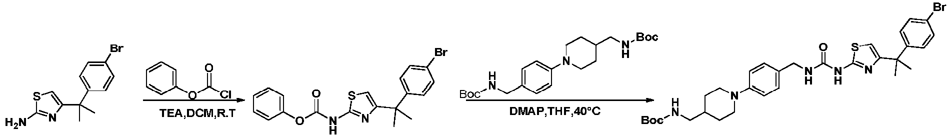

To a solution of 4-(2-(4-bromophenyl)propan-2-yl)thiazol-2-amine (100 mg, 0.34 mmol, 1 eq) and triethylamine in dry DCM (5 mL) was added phenyl carbonochloridate (106 mg, 0.68 mmol, 2 eq) at 0°C-RT slowly and the mixture was stirred for 4 h at RT. Quenched by brine, extracted with EA, the combined organic layers were washed with brine, dried over Na₂SO₄, filtered and concentrated to give a residue, which was purified by column chromatography on a silica gel to afford phenyl (4-(2-(4-bromophenyl)propan-2-yl)thiazol-2-yl)carbamate (112 mg).

The mixture of phenyl (4-(2-(4-bromophenyl)propan-2-yl)thiazol-2-yl)carbamate (112 mg, 0.27 mmol, 1 eq), tert-butyl ((1-(4-(aminomethyl)phenyl)piperidin-4-yl)methyl)carbamate (24 mg, 0.27 mmol, 1 eq) and DMAP (52 mg, 0.4 mmol, 1.5 eq) in THF (5 mL) was heated to reflux for 1 hour. Cooled down to RT, the reaction mixture was participated between H₂O (15 mL) and EA (10 mL x 2), the combined organic layers were washed with brine (10 mL), dried over Na₂SO₄, filtered and concentrated. The residue was purified by column chromatography on a silica gel to afford tert-butyl ((1-(4-((3-(4-(2-(4-bromophenyl)propan-2-yl)thiazol-2-yl)ureido)methyl)phenyl)piperidin-4-yl)methyl)carbamate (42 mg) as a white powder.

### General Method IV

The mixture of amine and isocyanate-alkanes in THF was stirred at RT overnight. Once the reaction was completed, the resulting suspension was diluted with organic solvent and washed with brine and then dried. After filtration and evaporation, the resulting residue was purified by trituration/Prep-TLC/Prep-HPLC to give the product.

### Example 11: Preparation of 1-ethyl-3-(4-(2-(4-methoxyphenyl)propan-2-yl)thiazol-2-yl)urea

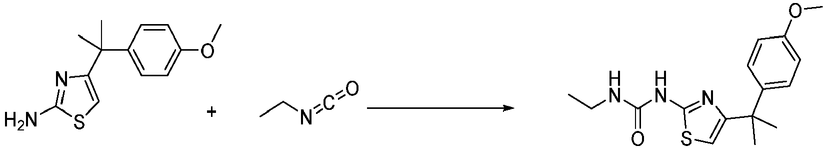

To a solution of 4-(2-(4-methoxyphenyl)propan-2-yl)thiophen-2-amine (200 mg, 0.67 mmol) in THF (5 mL), was added isocyanatoethane (48 mg, 0.67 mmol) and TEA (136 mg, 1.34 mmol). The resulting mixture was stirred at RT overnight. The mixture was concentrated at 45°C with reduce pressure to remove THF. The resulting suspension was diluted with EtOAc and washed with brine and then dried (Na₂SO₄), filtered and evaporated to dryness. The resulting residue was purified by Prep-TLC to give the desired compound (164 mg, yield: 65.4%) as a pale yellow solid. MS (ESI) m/z (M+H) ⁺ = 367.1.

### De-BOC General Method

The Boc compounds were dissolved in HCl/MeOH, the reaction mixture was stirred for 1-2 h at RT. The solution was concentrated to dryness to give the final compound.

### Example 12: Preparation of compound 1-(4-(2-(4-bromophenyl)propan-2-yl)thiazol-2-yl)-3-((6-(piperazin-1-yl)pyridin-3-yl)methyl)urea hydrochloride

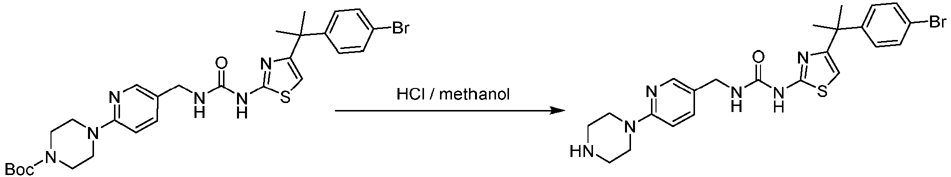

To a solution of tert-butyl 4-(5-((3-(4-(2-(4-bromophenyl)propan-2-yl)thiazol-2-yl)ureido)methyl)pyridin-2-yl)piperazine-1-carboxylate (70.0 mg, 113.71 µmol) in MeOH (2 mL) was added HCl/MeOH (4 M, 2 mL). The mixture was stirred at 25 °C for 1 hr. The mixture was concentrated in vacuum. The desired compound (47.0 mg, yield: 74.1%, HCl) was obtained as a white solid.

¹H NMR (400MHz, DMSO-*d*₆) *δ* 10.90 (br s, 1H), 9.66 (br s, 2H), 8.05 - 7.92 (m, 2H), 7.48 - 7.28 (m, 4H), 7.21 - 7.10 (m, 2H), 6.75 (s, 1H), 4.30 - 4.20 (m, 2H), 4.04 - 3.92 (m, 4H), 3.24 (br s, 4H), 1.57 (s, 6H). MS (ESI) *m*/*z* (M+H)⁺= 517.2.

The following examples were synthesized analogous to the procedure of example 8, 9, 10, 11 and 12 using the appropriate intermediates and the corresponding fragments:

**Table 4**

| Com. ID | Structure | Name | HNMR | LCMS ([M+H]⁺=) | Kinase assay IC50 nM | NFkB assay IC50µM |
|---|---|---|---|---|---|---|
| A001 | 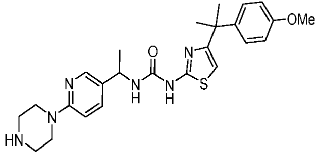 | 1-(4-(2-(4-methoxyphenyl)propan-2-yl)thiazol-2-yl)-3-(1-(6-(piperazin-1-yl)pyridin-3-yl)ethyl)urea | | 481.0 | 58 | 0.67 |
| A002 | 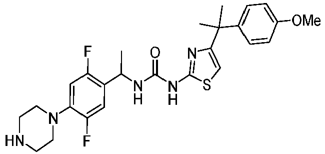 | 1-(1-(2,5-difluoro-4-(piperazin-1-yl)phenyl)ethyl)-3-(4-(2-(4-methoxyphenyl)propan-2-yl)thiazol-2-yl)urea | | 516.1 | 63 | 0.70 |
| A003 | 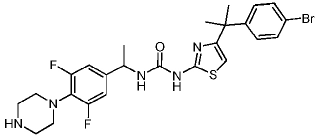 | 1-(4-(2-(4-bromophenyl)propan-2-yl)thiazol-2-yl)-3-(1-(3,5-difluoro-4-(piperazin-1-yl)phenyl)ethyl)urea | | 564.0 | 45 | 1.20 |
| A004 | 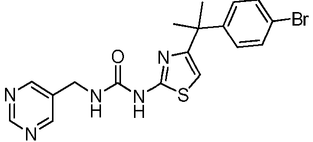 | 1-(4-(2-(4-bromophenyl)propan-2-yl)thiazol-2-yl)-3-(pyrimidin-5-ylmethyl)urea | | 432.1 | 240 | 2.03 |
| A005 | 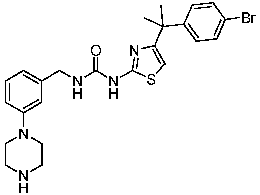 | 1-(4-(2-(4-bromophenyl)propan-2-yl)thiazol-2-yl)-3-(3-(piperazin-1-yl)benzyl)urea | | 514.0 | 97 | 3.15 |
| A006 | 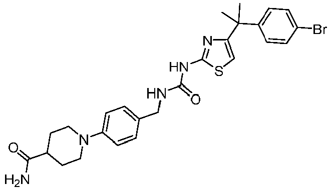 | 1-(4-((3-(4-(2-(4-bromophenyl)propan-2-yl)thiazol-2-yl)ureido)methyl)phenyl )piperidine-4-carboxamide | | 556.1 | 104 | |
| A007 | 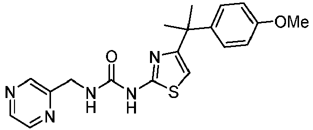 | 1-(4-(2-(4-methoxyphenyl)propan-2-yl)thiazol-2-yl)-3-(pyrazin-2-ylmethyl)urea | | 384.0 | 91 | 5.58 |
| A008 | 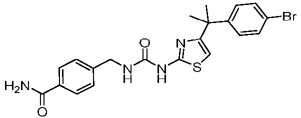 | 4-((3-(4-(2-(4-bromophenyl)propan-2-yl)thiazol-2-yl)ureido)methyl)benza mide | | 473.1 | 8 | 0.70 |
| A009 | 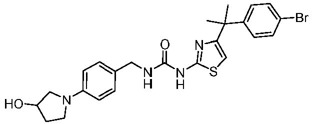 | 1-(4-(2-(4-bromophenyl)propan-2-yl)thiazol-2-yl)-3-(4-(3-hydroxypyrrolidin-1-yl)benzyl)urea | | 515.1 | 154 | 3.15 |
| A010 | 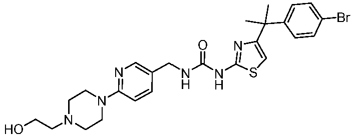 | 1-(4-(2-(4-bromophenyl)propan-2-yl)thiazol-2-yl)-3-((6-(4-(2-hydroxyethyl)piperazin-1-yl)pyridin-3-yl)methyl)urea | | 559.0 | 200 | 3.95 |
| A011 | 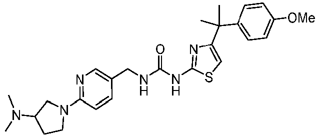 | 1-((6-(3-(dimethylamino)pyrrolid in-1-yl)pyridin-3-yl)methyl)-3-(4-(2-(4-methoxyphenyl)propan-2-yl)thiazol-2-yl)urea | | 495.1 | 49 | 1.37 |
| A012 | 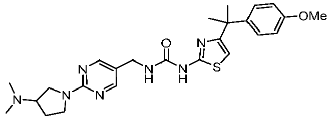 | 1-((2-(3-(dimethylamino)pyrrolid in-1-yl)pyrimidin-5-yl)methyl)-3-(4-(2-(4-methoxyphenyl)propan-2-yl)thiazol-2-yl)urea | | 496.0 | 40 | 2.21 |
| A013 | 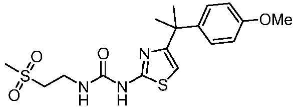 | 1-(4-(2-(4-methoxyphenyl)propan-2-yl)thiazol-2-yl)-3-(2-(methylsulfonyl)ethyl)ur ea | | 398.1 | 316 | 2.97 |
| A014 | 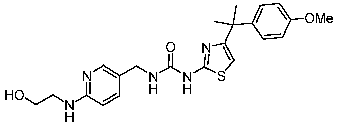 | 1-((6-((2-hydroxyethyl)amino)pyr idin-3-yl)methyl)-3-(4-(2-(4-methoxyphenyl)propan-2-yl)thiazol-2-yl)urea | | 442.0 | 160 | 0.88 |
| A015 | 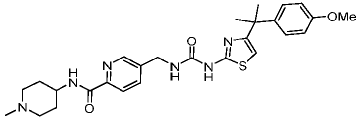 | 5-((3-(4-(2-(4-methoxyphenyl)propan-2-yl)thiazol-2-yl)ureido)methyl)-N-(1-methylpiperidin-4-yl)picolinamide | | 523.0 | 24 | 0.88 |
| A016 | 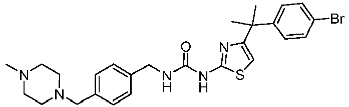 | 1-(4-(2-(4-bromophenyl)propan-2-yl)thiazol-2-yl)-3-(4-((4-methylpiperazin-1-yl)methyl)benzyl)urea | | 542.1 | 27 | 2.34 |
| A017 | 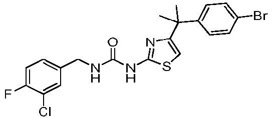 | 1-(4-(2-(4-bromophenyl)propan-2-yl)thiazol-2-yl)-3-(3-chloro-4-fluorobenzyl)urea | | 482.1 | 204 | 0.97 |
| A018 | 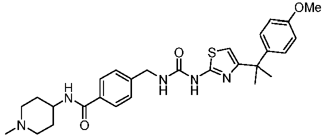 | 4-((3-(4-(2-(4-methoxyphenyl)propan-2-yl)thiazol-2-yl)ureido)methyl)-N-(1-methylpiperidin-4-yl)benzamide | | 522.1 | 29 | 1.28 |
| A019 | 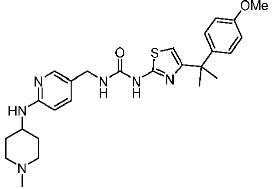 | 1-(4-(2-(4-methoxyphenyl)propan-2-yl)thiazol-2-yl)-3-((6-((1-methylpiperidin-4-yl)amino)pyridin-3-yl)methyl)urea | | 495.1 | 32 | 1.29 |
| A020 | 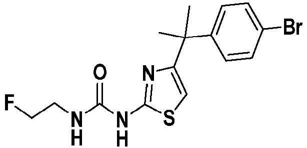 | 1-(4-(2-(4-bromophenyl)propan-2-yl)thiazol-2-yl)-3-(2-fluoroethyl)urea | ¹H NMR (400 MHz, CDCl₃) *δ* 7.40 - 7.35 (m, 2H), 7.15 - 7.10 (m, 2H), 6.42 (s, 1H), 4.54 - 4.49 (m, 1H), 4.41 - 4.37 (m, 1H), 3.55 (ddd, J = 28.1, 10.0, 5.2 Hz, 2H), 1.63 (s, 6H). | 386.0 | 144 | 6.79 |
| A021 | 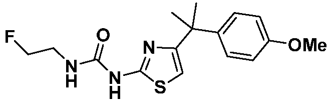 | 1-(2-fluoroethyl)-3-(4-(2-(4-methoxyphenyl)propan-2-yl)thiazol-2-yl)urea | ¹H NMR (400 MHz, DMSO-*d*₆) *δ* 10.53 (s, 1H), 7.12 - 7.00 (m, 2H), 6.83 - 6.68 (m, 2H), 6.60 (s, 1H), 6.48 (s, 1H), 4.40 (dt, J = 47.5, 5.0 Hz, 2H), 3.72 - 3.58 (m, 3H), 3.37 (ddd, J = 26.8, 10.6, 5.2 Hz, 2H), 1.60 - 1.46 (m, 6H). | 338.1 | 104 | 1.39 |
| A022 | 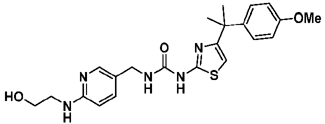 | 1-((6-((2-hydroxyethyl)amino)pyr idin-3-yl)methyl)-3-(4-(2-(4-methoxyphenyl)propan-2-yl)thiazol-2-yl)urea | ¹H NMR (400 MHz, CD3OD) *δ* 7.85 (dd, J = 9.3, 1.9 Hz, 1H), 7.75 (s, 1H), 7.20 - 7.15 (m, 2H), 7.08 (d, J = 9.3 Hz, 1H), 6.87 - 6.81 (m, 2H), 6.78 (s, 1H), 4.31 (s, 2H), 3.85 - 3.78 (m, 2H), 3.78 - 3.73 (m, 3H), 3.55 - 3.48 (m, 2H), 1.67 (s, 6H). | 442.1 | 232 | 0.96 |
| A023 | 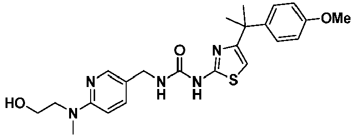 | 1-((6-((2-hydroxyethyl)(methyl)a mino)pyridin-3-yl)methyl)-3-(4-(2-(4-methoxyphenyl)propan-2-yl)thiazol-2-yl)urea | ¹H NMR (400 MHz, CD3OD) *δ* 7.94 (dd, J = 9.5, 2.1 Hz, 1H), 7.84 (d, J = 1.6 Hz, 1H), 7.31 (d, J = 9.5 Hz, 1H), 7.21 - 7.16 (m, 2H), 6.88 - 6.85 (m, 1H), 6.85 (d, J = 2.1 Hz, 1H), 6.84 (s, 1H), 4.35 (s, 2H), 3.85 (t, J = 4.8 Hz, 2H), 3.80 - 3.77 (m, 2H), 3.77 (s, 3H), 3.29 (s, 3H), 1.68 (s, 6H). | 456.0 | 229 | 1.32 |
| A024 | 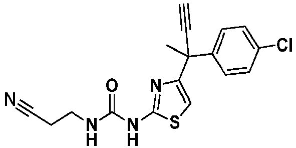 | 1-(4-(2-(4-chlorophenyl)but-3-yn-2-yl)thiazol-2-yl)-3-(2-cyanoethyl)urea | ¹H NMR (400MHz, CDCl₃) *δ* 7.44 - 7.37 (m, 2H), 7.30 - 7.24 (m, 2H), 6.75 (s, 1H), 3.55 - 3.40 (m, 2H), 2.58 - 2.51 (m, 3H), 1.92 (s, 3H). | 359.1 | 13 | 1.25 |
| A025 | 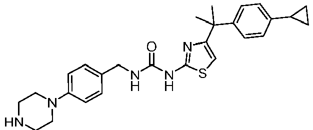 | 1-(4-(2-(4-cyclopropylphenyl)prop an-2-yl)thiazol-2-yl)-3-(4-(piperazin-1-yl)benzyl)urea | | 476.0 | 334 | 8.58 |
| A026 | 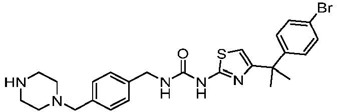 | 1-(4-(2-(4-bromophenyl)propan-2-yl)thiazol-2-yl)-3-(4-(piperazin-1-ylmethyl)benzyl)urea | | 528.0 | 117 | 1.25 |
| A027 | 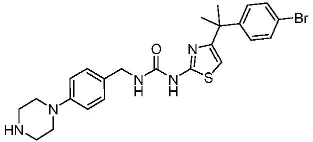 | 1-(4-(2-(4-bromophenyl)propan-2-yl)thiazol-2-yl)-3-(4-(piperazin-1-yl)benzyl)urea | | 514.0 | 46 | 1.45 |
| A028 | 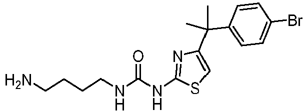 | 1-(4-aminobutyl)-3-(4-(2-(4-bromophenyl)propan-2-yl)thiazol-2-yl)urea | | 411.1 | 53 | 2.56 |
| A029 | 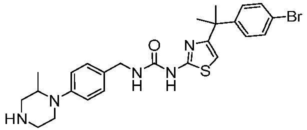 | 1-(4-(2-(4-bromophenyl)propan-2-yl)thiazol-2-yl)-3-(4-(2-methylpiperazin-1-yl)benzyl)urea | | 528.1 | 73 | |
| A030 | 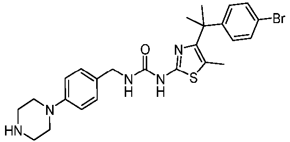 | 1-(4-(2-(4-bromophenyl)propan-2-yl)-5-methylthiazol-2-yl)-3-(4-(piperazin-1-yl)benzyl)urea | | 528.1 | 232 | 4.11 |
| A031 | 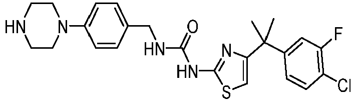 | 1-(4-(2-(4-chloro-3-fluorophenyl)propan-2-yl)thiazol-2-yl)-3-(4-(piperazin-1-yl)benzyl)urea | | 488.0 | 345 | 1.45 |
| A032 | 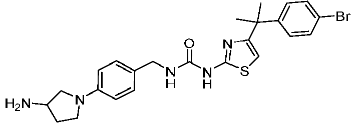 | 1-(4-(3-aminopyrrolidin-1-yl)benzyl)-3-(4-(2-(4-bromophenyl)propan-2-yl)thiazol-2-yl)urea | | 514.0 | 291 | 0.58 |
| A033 | 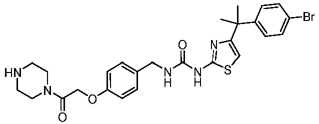 | 1-(4-(2-(4-bromophenyl)propan-2-yl)thiazol-2-yl)-3-(4-(2-oxo-2-(piperazin-1-yl)ethoxy)benzyl)urea | | 572.0 | 80 | 3.19 |
| A034 | 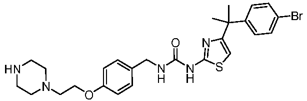 | 1-(4-(2-(4-bromophenyl)propan-2-yl)thiazol-2-yl)-3-(4-(2-(piperazin-1-yl)ethoxy)benzyl)urea | | 558.0 | 114 | 0.73 |
| A035 | 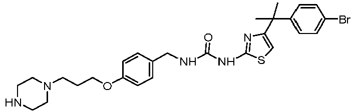 | 1-(4-(2-(4-bromophenyl)propan-2-yl)thiazol-2-yl)-3-(4-(3-(piperazin-1-yl)propoxy)benzyl)urea | | 572.0 | 52 | 2.03 |
| A036 | 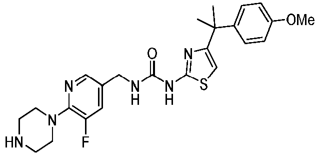 | 1-((5-fluoro-6-(piperazin-1-yl)pyridin-3-yl)methyl)-3-(4-(2-(4-methoxyphenyl)propan-2-yl)thiazol-2-yl)urea | | 485.1 | 24 | 0.62 |
| A037 | 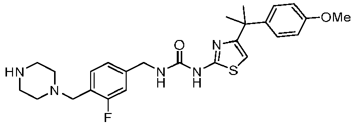 | 1-(3-fluoro-4-(piperazin-1-ylmethyl)benzyl)-3-(4-(2-(4-methoxyphenyl)propan-2-yl)thiazol-2-yl)urea | | 498.1 | 57 | 2.51 |
| A038 | 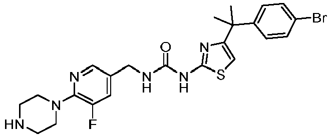 | 1-(4-(2-(4-bromophenyl)propan-2-yl)thiazol-2-yl)-3-((5-fluoro-6-(piperazin-1-yl)pyridin-3-yl)methyl)urea | | 533.1 | 30 | 1.66 |
| A039 | 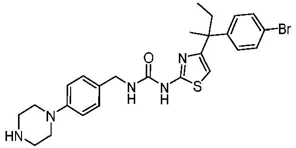 | 1-(4-(2-(4-bromophenyl)butan-2-yl)thiazol-2-yl)-3-(4-(piperazin-1-yl)benzyl)urea | | 528.1 | 189 | 0.711 |
| A040 | 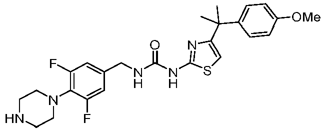 | 1-(3,5-difluoro-4-(piperazin-1-yl)benzyl)-3-(4-(2-(4-methoxyphenyl)propan-2-yl)thiazol-2-yl)urea | | 502.1 | 45 | 0.50 |
| A041 | 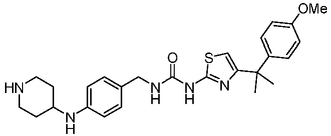 | 1-(4-(2-(4-methoxyphenyl)propan-2-yl)thiazol-2-yl)-3-(4-(piperidin-4-ylamino)benzyl)urea | | 480.0 | 23 | 1.86 |
| A042 | 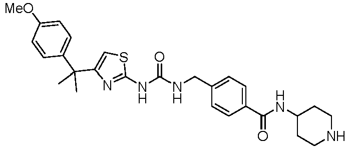 | 4-((3-(4-(2-(4-methoxyphenyl)propan-2-yl)thiazol-2-yl)ureido)methyl)-N-(piperidin-4-yl)benzamide | | 508.0 | 15 | |
| A043 | 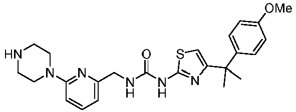 | 1-(4-(2-(4-methoxyphenyl)propan-2-yl)thiazol-2-yl)-3-((6-(piperazin-1-yl)pyridin-2-yl)methyl)urea | | 467.0 | 165 | |
| A044 | 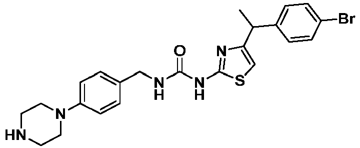 | 1-(4-(1-(4-bromophenyl)ethyl)thiaz ol-2-yl)-3-(4-(piperazin-1-yl)benzyl)urea | ¹H NMR (400 MHz, CD3OD) *δ* 7.45 (d, J = 6.9 Hz, 2H), 7.22 (s, 2H), 7.14 (s, 2H), 6.97 (s, 3H), 4.32 (s, 2H), 4.18 (s, 1H), 3.37 (s, 4H), 3.33 (s, 4H), 1.59 (s, 3H). | 500.0 | 147 | 1.11 |
| A045 | 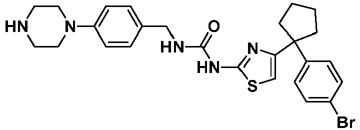 | 1-(4-(1-(4-bromophenyl)cyclopent yl)thiazol-2-yl)-3-(4-(piperazin-1-yl)benzyl)urea | ¹H NMR (400 MHz, DMSO-*d*₆) *δ* 9.28 (s, 1H), 7.39 (d, J = 8.3 Hz, 2H), 7.19 (d, J = 8.5 Hz, 2H), 7.13 (d, J = 8.3 Hz, 2H), 6.93 (d, J = 8.5 Hz, 2H), 6.76 (s, 1H), 4.18 (d, J = 4.4 Hz, 2H), 3.31 (s, 4H), 3.16 (s, 4H), 2.41 (d, J = 12.9 Hz, 2H), 1.95 (s, 2H), 1.59 (d, J = 9.0 Hz, 4H). | 540.1 | 24 | 1.07 |
| A046 | 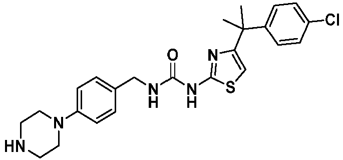 | 1-(4-(2-(4-chlorophenyl)propan-2-yl)thiazol-2-yl)-3-(4-(piperazin-1-yl)benzyl)urea | ¹H NMR (400 MHz, DMSO-*d*₆) *δ* 8.99 (s, 1H), 7.26 (d, J = 8.5 Hz, 2H), 7.17 (d, J = 8.4 Hz, 2H), 7.12 (d, J = 8.2 Hz, 2H), 6.91 (d, J = 8.7 Hz, 3H), 6.70 (s, 1H), 4.17 (d, J = 5.8 Hz, 2H), 3.28 (s, 4H), 3.17 (s, 4H), 1.54 (s, 6H). | 470.0 | 79 | 1.06 |
| A047 | 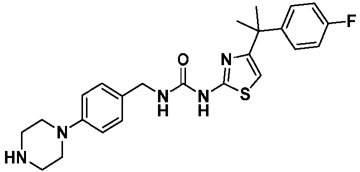 | 1-(4-(2-(4-fluorophenyl)propan-2-yl)thiazol-2-yl)-3-(4-(piperazin-1-yl)benzyl)urea | ¹H NMR (400 MHz, DMSO-*d*₆) *δ* 7.18 (dd, J = 8.1, 5.7 Hz, 2H), 7.12 (d, J = 8.2 Hz, 2H), 7.01 (t, J = 8.6 Hz, 2H), 6.89 (d, J = 8.1 Hz, 2H), 6.84 (s, 1H), 6.66 (s, 1H), 4.17 (d, J = 5.6 Hz, 2H), 3.24 (d, J = 5.3 Hz, 4H), 3.13 (d, J = 5.0 Hz, 4H), 1.54 (s, 6H). | 454.0 | 167 | 2.10 |
| A048 | 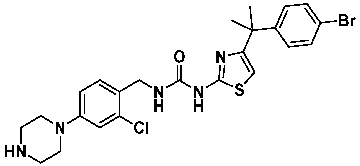 | 1-(4-(2-(4-bromophenyl)propan-2-yl)thiazol-2-yl)-3-(2-chloro-4-(piperazin-1-yl)benzyl)urea | ¹H NMR (400 MHz, DMSO-*d*₆) *δ* 9.24 (s, 1H), 7.39 (d, J = 8.6 Hz, 2H), 7.19 (d, J = 8.6 Hz, 1H), 7.12 (d, J = 8.6 Hz, 3H), 7.01 (d, J = 2.3 Hz, 1H), 6.90 (dd, J = 8.7, 2.3 Hz, 1H), 6.70 (s, 1H), 4.25 (d, J = 5.7 Hz, 2H), 3.43 - 3.28 (m, 4H), 3.13 (s, 4H), 1.54 (s, 6H). | 548.0 | 138 | 1.53 |
| A049 | 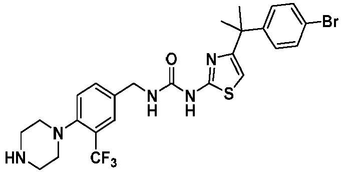 | 1-(4-(2-(4-bromophenyl)propan-2-yl)thiazol-2-yl)-3-(4-(piperazin-1-yl)-3-(trifluoromethyl)benzyl) urea | ¹H NMR (400 MHz, DMSO-*d*₆) *δ* 9.11 (s, 2H), 7.54 (d, J = 9.0 Hz, 2H), 7.46 (d, J = 8.1 Hz, 1H), 7.38 (d, J = 8.5 Hz, 2H), 7.19 (s, 1H), 7.11 (d, J = 8.5 Hz, 2H), 6.70 (s, 1H), 4.30 (d, J = 5.7 Hz, 2H), 3.10 (d, J = 17.0 Hz, 4H), 3.01 (d, J = 3.9 Hz, 4H), 1.56 (d, J = 22.5 Hz, 6H). | 582.0 | 119 | 0.8 |
| A050 | 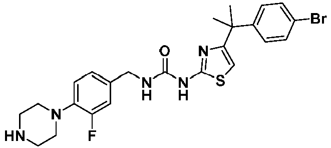 | 1-(4-(2-(4-bromophenyl)propan-2-yl)thiazol-2-yl)-3-(3-fluoro-4-(piperazin-1-yl)benzyl)urea | ¹H NMR (400 MHz, DMSO-*d*₆) *δ* 9.31 (s, 1H), 7.31 (d, J = 44.6 Hz, 3H), 7.04 (d, J = 44.4 Hz, 4H), 6.68 (s, 1H), 4.18 (s, 2H), 3.15 (s, 8H), 1.51 (s, 6H). | 532.1 | 26 | 1.21 |
| A051 | 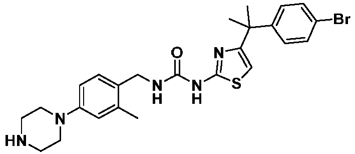 | 1-(4-(2-(4-bromophenyl)propan-2-yl)thiazol-2-yl)-3-(2-methyl-4-(piperazin-1-yl)benzyl)urea | ¹H NMR (400 MHz, DMSO-*d*₆) *δ* 10.54 - 10.45 (m, 1H), 9.02 (s, 1H), 7.39 (d, J = 8.6 Hz, 2H), 7.11 (d, J = 8.6 Hz, 2H), 7.05 (d, J = 8.5 Hz, 1H), 6.78 (s, 2H), 6.74 (d, J = 8.5 Hz, 1H), 6.70 (s, 1H), 4.17 (d, J = 5.4 Hz, 2H), 3.28 (d, J = 5.5 Hz, 4H), 3.16 (s, 4H), 2.20 (s, 3H), 1.54 (s, 6H). | 528.1 | 125 | 0.29 |
| A052 | 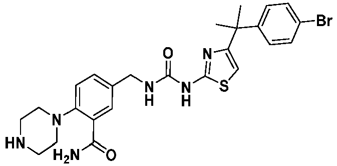 | 5-((3-(4-(2-(4-bromophenyl)propan-2-yl)thiazol-2-yl)ureido)methyl)-2-(piperazin-1-yl)benzamide | ¹H NMR (400 MHz, DMSO-*d*₆) *δ* 9.35 (s, 1H), 8.04 (s, 1H), 7.54 - 7.32 (m, 3H), 7.23 (d, J = 29.5 Hz, 2H), 7.09 (d, J = 19.3 Hz, 3H), 6.70 (s, 1H), 3.13 (d, J = 46.5 Hz, 8H), 2.66 (d, J = 17.4 Hz, 2H), 1.53 (s, 6H). | 557.1 | 24 | 1.96 |
| A053 | 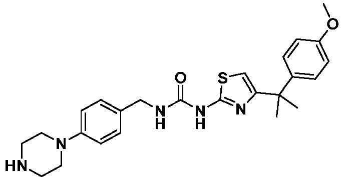 | 1-(4-(2-(4-methoxyphenyl)propan-2-yl)thiazol-2-yl)-3-(4-(piperazin-1-yl)benzyl)urea | ¹H NMR (400 MHz, DMSO-*d*₆) *δ* 9.15 (s, 1H), 7.09 (d, J = 18.4 Hz, 4H), 6.90 (s, 2H), 6.75 (s, 2H), 6.61 (s, 1H), 4.17 (s, 2H), 3.65 (s, 3H), 3.28 (s, 4H), 3.15 (s, 4H), 1.52 (s, 6H). | 466.1 | 43 | 0.76 |
| A054 | 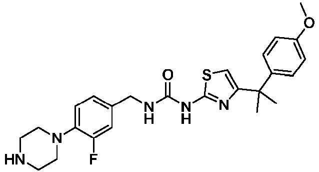 | 1-(3-fluoro-4-(piperazin-1-yl)benzyl)-3-(4-(2-(4-methoxyphenyl)propan-2-yl)thiazol-2-yl)urea | ¹H NMR (400 MHz, CD3OD) *δ* 10.12 (s, 2H), 8.12 (s, 1H), 7.81 (d, J = 23.1 Hz, 5H), 7.53 (s, 2H), 7.39 (s, 1H), 4.97 (s, 2H), 4.43 (s, 3H), 3.93 (s, 8H), 2.29 (s, 6H). | 484.1 | 30 | 0.88 |
| A055 | 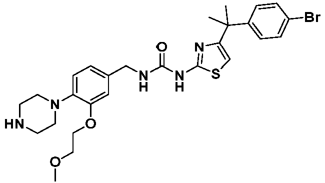 | 1-(4-(2-(4-bromophenyl)propan-2-yl)thiazol-2-yl)-3-(3-(2-methoxyethoxy)-4-(piperazin-1-yl)benzyl)urea | ¹H NMR (400 MHz, CD3OD) *δ* 7.48 (d, J = 8.4 Hz, 2H), 7.19 (d, J = 7.1 Hz, 2H), 7.11 (s, 1H), 7.00 (s, 2H), 6.93 (s, 1H), 4.38 (s, 2H), 4.19 (s, 4H), 3.77 (s, 4H), 3.43 (s, 4H), 3.40 (s, 3H), 1.69 (s, 6H). | 588.1 | 182 | 4.70 |
| A056 | 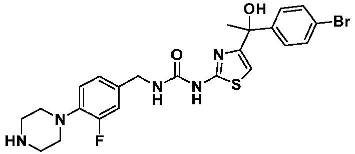 | 1-(4-(1-(4-bromophenyl)-1-hydroxyethyl)thiazol-2-yl)-3-(3-fluoro-4-(piperazin-1-yl)benzyl)urea | ¹H NMR (400 MHz, CD3OD) *δ* 7.46 (d, J = 8.4 Hz, 2H), 7.38 (d, J = 8.5 Hz, 2H), 7.04 (t, J = 9.9 Hz, 4H), 4.36 (d, J = 6.7 Hz, 2H), 3.29 (s, 8H), 1.84 (d, J = 13.6 Hz, 3H). | 534.1 | 176 | 1.06 |
| A057 | 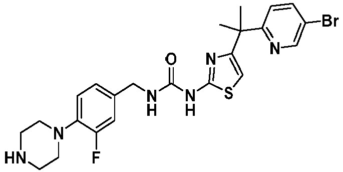 | 1-(4-(2-(5-bromopyridin-2-yl)propan-2-yl)thiazol-2-yl)-3-(3-fluoro-4-(piperazin-1-yl)benzyl)urea | ¹H NMR (400 MHz, CD3OD) *δ* 8.48 (d, J = 2.3 Hz, 1H), 7.80 - 7.73 (m, 1H), 7.08 (d, J = 8.4 Hz, 1H), 7.01 (d, J = 4.2 Hz, 2H), 6.99 (s, 1H), 6.73 (s, 1H), 4.30 (s, 2H), 3.25 (s, 4H), 3.23 (s, 4H), 1.64 (s, 6H). | 533.1 | 246 | |
| A058 | 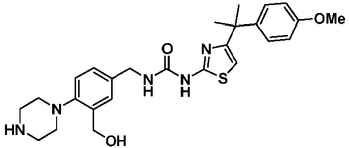 | 1-(3-(hydroxymethyl)-4-(piperazin-1-yl)benzyl)-3-(4-(2-(4-methoxyphenyl)propan-2-yl)thiazol-2-yl)urea | ¹H NMR (400 MHz, CD3OD) *δ* 7.41 (d, J = 4.2 Hz, 2H), 7.21 (s, 1H), 7.15 (d, J = 6.7 Hz, 1H), 7.12 (d, J = 5.9 Hz, 2H), 6.88 (s, 1H), 6.85 (s, 1H), 6.82 (d, J = 5.7 Hz, 2H), 4.67 (s, 2H), 4.38 (s, 2H), 3.74 (s, 3H), 3.25 - 3.22 (m, 4H), 3.17 - 3.11 (m, 4H), 1.63 (s, 6H). | 496.1 | 130 | 3.10 |
| A059 | 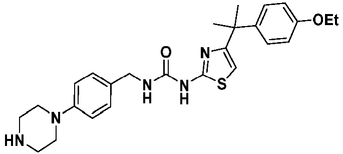 | 1-(4-(2-(4-ethoxyphenyl)propan-2-yl)thiazol-2-yl)-3-(4-(piperazin-1-yl)benzyl)urea | ¹H NMR (400 MHz, CD3OD) *δ* 7.17 (t, J = 5.9 Hz, 2H), 7.09 (dd, J = 9.4, 2.7 Hz, 2H), 6.96 (d, J = 8.7 Hz, 2H), 6.78 - 6.73 (m, 2H), 6.58 (s, 1H), 4.28 (s, 2H), 3.96 (q, J = 7.0 Hz, 2H), 3.34 (d, J = 3.6 Hz, 4H), 3.33 (s, 4H), 1.57 (s, 6H), 1.37 - 1.29 (m, 3H). | 480.0 | 269 | |
| A060 | 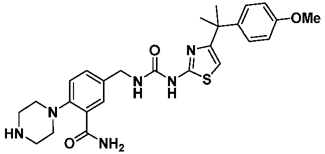 | 5-((3-(4-(2-(4-methoxyphenyl)propan-2-yl)thiazol-2-yl)ureido)methyl)-2-(piperazin-1-yl)benzamide | ¹H NMR (400 MHz, DMSO-*d*₆) *δ* 9.35 (s, 2H), 8.05 (s, 1H), 7.53 (d, J = 2.0 Hz, 1H), 7.48 (s, 1H), 7.30 (d, J = 10.1 Hz, 2H), 7.08 (t, J = 8.0 Hz, 3H), 6.78 (d, J = 8.8 Hz, 2H), 6.65 (s, 1H), 4.24 (d, J = 5.2 Hz, 2H), 3.66 (s, 3H), 3.20 (s, 4H), 3.08 (s, 4H), 1.54 (s, 6H). | 509.0 | 8 | 4.80 |
| A061 | 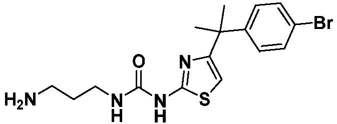 | 1-(3-aminopropyl)-3-(4-(2-(4-bromophenyl)propan-2-yl)thiazol-2-yl)urea | ¹H NMR (400 MHz, DMSO-*d*₆) *δ* 8.08 (s, 3H), 7.49 (s, 1H), 7.40 (d, J = 8.2 Hz, 2H), 7.11 (d, J = 8.2 Hz, 2H), 6.78 (s, 1H), 3.14 - 3.12 (s, 2H), 2.73 (s, 2H), 1.70 (s, 2H), 1.53 (s, 6H). | 397.0 | 195 | 1.99 |
| A062 | 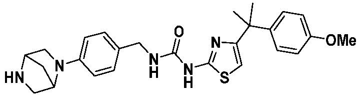 | 1-(4-(2,5-diazabicyclo[2.2.1]hepta n-2-yl)benzyl)-3-(4-(2-(4-methoxyphenyl)propan-2-yl)thiazol-2-yl)urea | ¹H NMR (400 MHz, CD3OD) *δ* 7.22 - 7.15 (m, 4H), 7.09 (s, 1H), 6.89 (d, J = 8.8 Hz, 2H), 6.64 (d, J = 8.5 Hz, 2H), 4.62 (s, 1H), 4.46 (s, 1H), 4.30 (s, 2H), 3.75 (s, 3H), 3.69 (dd, J = 10.7, 2.2 Hz, 1H), 3.31 - 3.27 (m, 3H), 2.25 (d, J = 11.0 Hz, 1H), 2.04 (d, J = 11.2 Hz, 1H), 1.68 (s, 6H). | 478.1 | 60 | 1.33 |
| A063 | 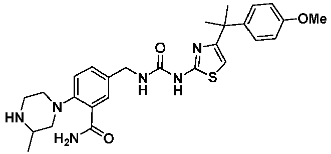 | 5-((3-(4-(2-(4-methoxyphenyl)propan-2-yl)thiazol-2-yl)ureido)methyl)-2-(3-methylpiperazin-1-yl)benzamide | ¹H NMR (400 MHz, CD3OD) *δ* 7.70 (d, J = 2.0 Hz, 1H), 7.50 (dd, J = 8.3, 2.0 Hz, 1H), 7.28 (d, J = 8.3 Hz, 1H), 7.23 (s, 1H), 7.20 (s, 1H), 7.14 (s, 1H), 6.93 (d, J = 8.8 Hz, 2H), 4.47 (s, 2H), 3.79 (s, 3H), 3.56 - 3.48 (m, 1H), 3.46 - 3.41 (m, 1H), 3.38 (d, J = 11.9 Hz, 1H), 3.17 (d, J = 12.7 Hz, 1H), 3.00 (d, J = 13.9 Hz, 1H), 2.97 - 2.91 (m, 1H), 2.90 - 2.84 (m, 1H), 1.72 (s, 6H), 1.40 (d, J = 6.6 Hz, 3H). | 523.0 | 29 | 2.91 |
| A064 | 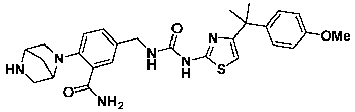 | 2-(2,5-diazabicyclo[2.2.1]hepta n-2-yl)-5-((3-(4-(2-(4-methoxyphenyl)propan-2-yl)thiazol-2-yl)ureido)methyl)benza mide | ¹H NMR (400 MHz, DMSO-*d*₆) *δ* 10.70 (s, 1H), 9.72 (s, 1H), 8.89 (s, 1H), 7.68 (s, 1H), 7.37 (s, 1H), 7.23 (d, J = 1.9 Hz, 1H), 7.18 (dd, J = 8.5, 1.9 Hz, 1H), 7.13 (d, J = 8.8 Hz, 2H), 6.80 (dd, J = 14.2, 8.7 Hz, 3H), 6.68 (d, J = 1.1 Hz, 1H), 5.44 (s, 5H), 4.48 (s, 1H), 4.35 (s, 1H), 4.22 (d, J = 5.1 Hz, 2H), 3.71 (s, 3H), 3.17 (s, 1H), 3.11 (d, J = 10.7 Hz, 1H), 2.04 (d, J = 10.5 Hz, 1H), 1.95 - 1.89 (m, 1H), 1.58 (s, 6H). | 521.0 | 41 | 19.73 |
| A065 | 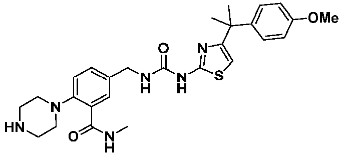 | 5-((3-(4-(2-(4-methoxyphenyl)propan-2-yl)thiazol-2-yl)ureido)methyl)-N-methyl-2-(piperazin-1-yl)benzamide | ¹HNMR (400 MHz, DMSO-*d*₆) *δ* 9.42 (s, 1H), 8.45 (s, 1H), 7.51 (d, J = 28.5 Hz, 2H), 7.28 (d, J = 8.0 Hz, 1H), 7.07 (t, J = 10.0 Hz, 3H), 6.78 (d, J = 8.4 Hz, 2H), 6.67 (s, 1H), 4.23 (s, 2H), 3.66 (s, 3H), 3.18 (s, 4H), 3.06 (s, 4H), 2.77 (d, J = 3.7 Hz, 3H), 2.46 (s, 1H), 1.54 (s, 6H). | 523.0 | 30 | 4.44 |
| A066 | 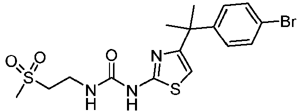 | 1-(4-(2-(4-bromophenyl)propan-2-yl)thiazol-2-yl)-3-(2-(methylsulfonyl)ethyl)ur ea | | 446.0 | 126 | 10.50 |
| A067 | 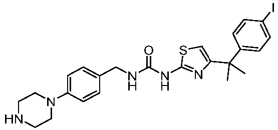 | 1-(4-(2-(4-iodophenyl)propan-2-yl)thiazol-2-yl)-3-(4-(piperazin-1-yl)benzyl)urea | | 562.0 | 28 | 1.11 |
| A068 | 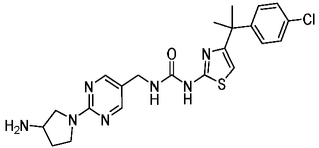 | 1-((2-(3-aminopyrrolidin-1-yl)pyrimidin-5-yl)methyl)-3-(4-(2-(4-chlorophenyl)propan-2-yl)thiazol-2-yl)urea | | 472.0 | 269 | |
| A069 | 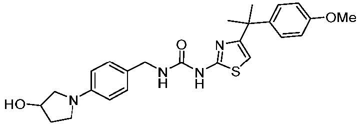 | 1-(4-(3-hydroxypyrrolidin-1-yl)benzyl)-3-(4-(2-(4-methoxyphenyl)propan-2-yl)thiazol-2-yl)urea | | 467.0 | 269 | |
| A070 | 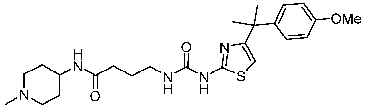 | 4-(3-(4-(2-(4-methoxyphenyl)propan-2-yl)thiazol-2-yl)ureido)-N-(1-methylpiperidin-4-yl)butanamide | | 474.0 | 140 | 16.91 |
| A071 | 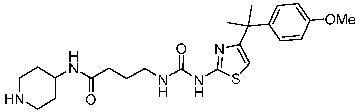 | 4-(3-(4-(2-(4-methoxyphenyl)propan-2-yl)thiazol-2-yl)ureido)-N-(piperidin-4-yl)butanamide | | 460.1 | 64 | |
| A072 | 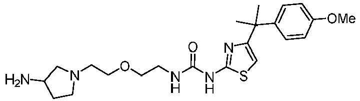 | 1-(2-(2-(3-aminopyrrolidin-1-yl)ethoxy)ethyl)-3-(4-(2-(4-methoxyphenyl)propan-2-yl)thiazol-2-yl)urea | | 448.1 | 143 | 1.45 |
| A073 | 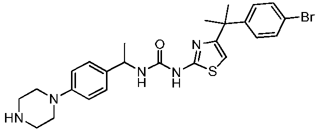 | 1-(4-(2-(4-bromophenyl)propan-2-yl)thiazol-2-yl)-3-(1-(4-(piperazin-1-yl)phenyl)ethyl)urea | | 528.1 | 85 | 2.79 |
| A074 | 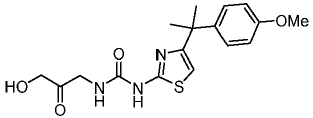 | 1-(3-hydroxy-2-oxopropyl)-3-(4-(2-(4-methoxyphenyl)propan-2-yl)thiazol-2-yl)urea | | 364.1 | 61 | |
| A075 | 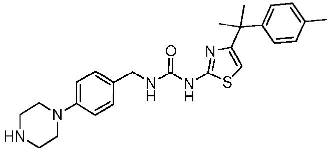 | 1-(4-(piperazin-1-yl)benzyl)-3-(4-(2-(p-tolyl)propan-2-yl)thiazol-2-yl)urea | | 450.1 | 25 | 0.267 |
| \A076 | 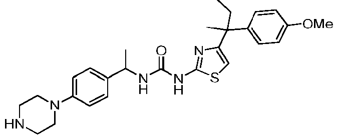 | 1-(4-(2-(4-methoxyphenyl)butan-2-yl)thiazol-2-yl)-3-(1-(4-(piperazin-1-yl)phenyl)ethyl)urea | | 494.1 | 147 | 4.48 |
| A077 | 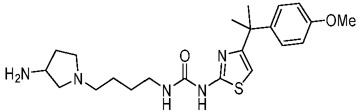 | 1-(4-(3-aminopyrrolidin-1-yl)butyl)-3-(4-(2-(4-methoxyphenyl)propan-2-yl)thiazol-2-yl)urea | | 432.1 | 180 | 2.97 |
| A078 | 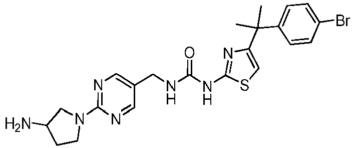 | 1-((2-(3-aminopyrrolidin-1-yl)pyrimidin-5-yl)methyl)-3-(4-(2-(4-bromophenyl)propan-2-yl)thiazol-2-yl)urea | ¹HNMR (400 MHz, DMSO-*d*₆) *δ* 8.46(br, 5H), 7.39(d, J = 8.8Hz, 2H), 7.32(s, 1H), 7.10(d, J = 8.8Hz, 2H), 6.73(s, 1H), 4.15(s, 2H), 3.70-3.59(m, 5H), 2.30-2.27(m, 1H), 2.16-2.13(m, 1H), 1.53(s, 6H). | 516.1 | 8 | |
| A079 | 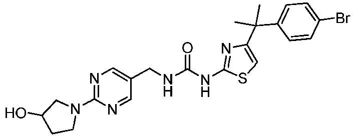 | 1-(4-(2-(4-bromophenyl)propan-2-yl)thiazol-2-yl)-3-((2-(3-hydroxypyrrolidin-1-yl)pyrimidin-5-yl)methyl)urea | ¹H NMR (400MHz, CDCl₃) *δ* 8.13 (s, 2H), 7.34 (d, J = 8.4 Hz, 2H), 7.05 (d, J = 8.4 Hz, 2H), 6.48 (s, 1H), 4.49 (br s, 1H), 4.07 (br d, J = 4.0 Hz, 2H), 3.85 - 3.50 (m, 4H), 2.22 - 1.92 (m, 3H), 1.59 (s, 6H), 1.26 (s, 1H). | 519.2 | 170 | 1.68 |
| A080 | 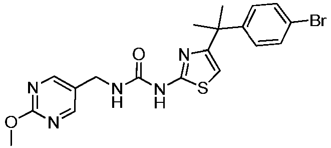 | 1-(4-(2-(4-bromophenyl)propan-2-yl)thiazol-2-yl)-3-((2-methoxypyrimidin-5-yl)methyl)urea | ¹HNMR (400 MHz, DMSO-*d*₆) *δ* 10.72 (s, 1 H), 8.49 (s, 2 H), 7.39 (d, J=8.56 Hz, 2 H), 7.14 - 7.07 (m, 2 H), 6.85 - 6.79 (m, 1 H), 6.70 (s, 1 H), 4.20 (s, 2 H), 3.82 - 3.87 (m, 3 H), 1.53 (s, 6 H). | 461.9 | 172 | 13.30 |
| A081 | 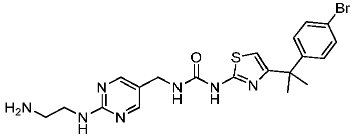 | 1-((2-((2-aminoethyl)amino)pyri midin-5-yl)methyl)-3-(4-(2-(4-bromophenyl)propan-2-yl)thiazol-2-yl)urea | ¹H NMR (400 MHz, DMSO-*d*₆) *δ* 8.44 (s, 2 H), 8.06 (br s, 3 H), 7.43 (d, J=8.56 Hz, 2 H), 7.24 (br s, 1 H), 7.14 (d, J=8.56 Hz, 2 H), 6.75 (s, 1 H), 4.17 (br d, J=5.38 Hz, 2 H), 3.52 - 3.65 (m, 2 H), 2.88 - 3.04 (m, 2 H), 1.57 (s, 6 H). | 492.1 | 17 | 1.84 |
| A082 | 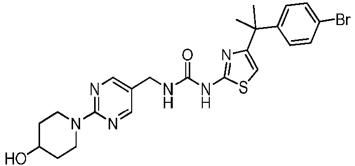 | 1-(4-(2-(4-bromophenyl)propan-2-yl)thiazol-2-yl)-3-((2-(4-hydroxypiperidin-1-yl)pyrimidin-5-yl)methyl)urea | ¹H NMR (400MHz, DMSO-*d*₆) *δ* 10.65 (s, 1H), 8.27 (s, 2H), 7.42 (d, *J* = 8.6 Hz, 2H), 7.14 (d, *J=* 8.8 Hz, 2H), 6.77 - 6.67 (m, 2H), 4.74 - 4.68 (m, 1H), 4.27 - 4.19 (m, 2H), 4.12 - 4.06 (m, 2H), 3.75 - 3.66 (m, 1H), 3.26 - 3.18 (m, 2H), 1.79 - 1.71 (m, 2H), 1.57 (s, 6H), 1.31 - 1.24 (m, 2H). | 533.0 | 125 | 6.15 |
| A083 | 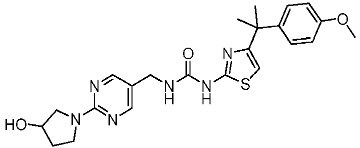 | 1-((2-(3-hydroxypyrrolidin-1-yl)pyrimidin-5-yl)methyl)-3-(4-(2-(4-methoxyphenyl)propan-2-yl)thiazol-2-yl)urea | ¹HNMR (400 MHz, CDCl₃) *δ* 8.16 (s, 2 H), 7.15 - 7.03 (m, 2 H), 6.79 - 6.77 (m, 1 H), 6.83 - 6.72 (m, 1 H), 6.46 - 6.38 (m, 1 H), 4.55 - 4.49 (m, 1 H), 4.15 - 4.06 (m, 2 H), 3.77 (s, 3 H), 3.71 - 3.59 (m, 4 H), 2.13 - 2.02 (m, 2 H), 1.58 (s, 6 H) | 469.2 | 111 | 0.68 |
| A084 | 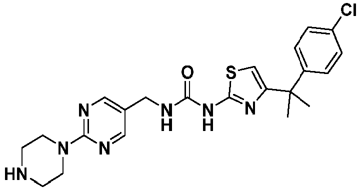 | 1-(4-(2-(4-chlorophenyl)propan-2-yl)thiazol-2-yl)-3-((2-(piperazin-1-yl)pyrimidin-5-yl)methyl)urea | ¹H NMR (400 MHz, DMSO-*d*₆) *δ* 9.17 (s, 1H), 8.34 (s, 2H), 7.25 (d, J = 8.0 Hz, 2H), 7.16 (d, J = 8.0 Hz, 2H), 7.02 (s, 1H), 6.69 (s, 1H), 4.11 (s, 2H), 3.90 (s, 4H), 3.09 (s, 4H), 1.53 (s, 6H). | 472.0 | 22 | 0.34 |
| A085 | 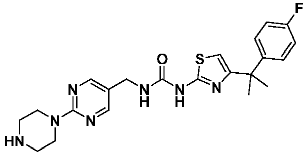 | 1-(4-(2-(4-fluorophenyl)propan-2-yl)thiazol-2-yl)-3-((2-(piperazin-1-yl)pyrimidin-5-yl)methyl)urea | ¹HNMR (400 MHz, DMSO-*d*₆) *δ* 9.34 (s, 1H), 8.35 (s, 1H), 7.22 (d, J = 40.8 Hz, 2H), 7.01 (s, 2H), 6.69 (s, 1H), 4.11 (s, 2H), 3.91 (s, 4H), 3.08 (s, 4H), 1.54 (s, 6H). | 456.0 | 85 | 0.49 |
| A086 | 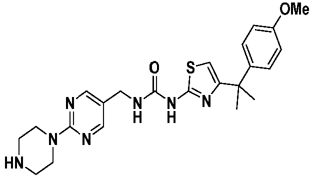 | 1-(4-(2-(4-methoxyphenyl)propan-2-yl)thiazol-2-yl)-3-((2-(piperazin-1-yl)pyrimidin-5-yl)methyl)urea | ¹HNMR (400 MHz, CD3OD) *δ* 8.47 (s, 2H), 7.17 (s, 2H), 7.09 (s, 1H), 6.88 (s, 2H), 4.32 (s, 2H), 4.09 (s, 3H), 3.74 (s, 4H), 3.32 (d, J = 5.5 Hz, 4H), 1.67 (s, 6H). | 468.0 | 16 | 0.39 |
| A087 | 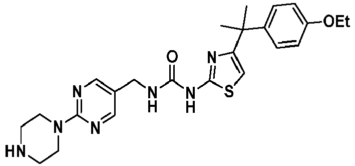 | 1-(4-(2-(4-ethoxyphenyl)propan-2-yl)thiazol-2-yl)-3-((2-(piperazin-1-yl)pyrimidin-5-yl)methyl)urea | ¹HNMR (400 MHz, CD3OD) *δ* 8.57 (s, 2H), 7.15 (d, J = 8.2 Hz, 2H), 7.11 (s, 1H), 6.87 (d, J = 8.2 Hz, 2H), 4.37 (s, 2H), 4.14 (s, 4H), 3.99 (q, J = 6.9 Hz, 2H), 3.34 (d, J = 12.6 Hz, 4H), 1.68 (s, 6H), 1.34 (t, J = 6.9 Hz, 3H). | 482.1 | 89 | 1.74 |
| A088 | 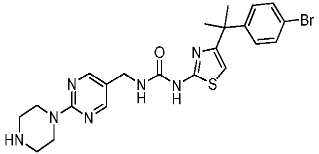 | 1-(4-(2-(4-bromophenyl)propan-2-yl)thiazol-2-yl)-3-((2-(piperazin-1-yl)pyrimidin-5-yl)methyl)urea compound with methane (1:1) | ¹H NMR (400 MHz, CD3OD) *δ* 8.51(s, 2H), 7.53-7.51(m, 2H), 7.24-7.22(m, 2H), 7.15(s, 1H), 4.36(s, 2H), 4.14-4.12(m, 4H), 3.34-3.33(m, 4H), 1.73 (s, 6H). | 518.0 | 13 | 1.45 |
| A089 | 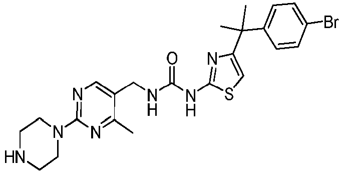 | 1-(4-(2-(4-bromophenyl)propan-2-yl)thiazol-2-yl)-3-((4-methyl-2-(piperazin-1-yl)pyrimidin-5-yl)methyl)urea | ¹H NMR (400MHz, DMSO-d₆) *δ* 10.70 (br s, 1H), 9.37 (br s, 2H), 8.21 (s, 1H), 7.42 (d, J = 8.3 Hz, 2H), 7.34 - 7.26 (m, 1H), 7.14 (d, J = 8.6 Hz, 2H), 6.75 (s, 1H), 4.20 (br d, J = 5.1 Hz, 2H), 4.01 - 3.91 (m, 4H), 3.12 (br s, 4H), 2.37 (s, 3H), 1.57 (s, 6H) | 532.0 | 26 | 1.19 |
| A090 | 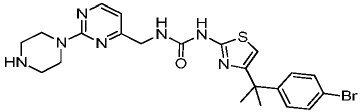 | 1-(4-(2-(4-bromophenyl)propan-2-yl)thiazol-2-yl)-3-((2-(piperazin-1-yl)pyrimidin-4-yl)methyl)urea | ¹H NMR (400MHz, DMSO-d₆) *δ* 9.45 (br s, 2H), 8.35 (d, J=5.1 Hz, 1H), 7.41-7.47 (m, 3H), 7.24-7.34 (m, 2H), 7.11-7.21 (m, 3H), 6.75 (s, 1H), 6.67 (d, J=5.1 Hz, 1H), 4.28 (br s, 1H), 4.27 (br s, 1H), 4.05 (s, 1H), 3.05 (s, 1H), 1.58 (s, 6H) | 516.1 | 178 | 1.11 |
| A091 | 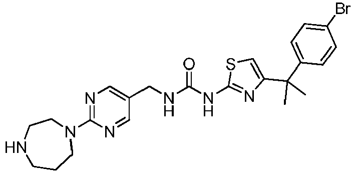 | 1-((2-(1,4-diazepan-1-yl)pyrimidin-5-yl)methyl)-3-(4-(2-(4-bromophenyl)propan-2-yl)thiazol-2-yl)urea | ¹H NMR (400MHz, DMSO-*d*₆) *δ* 9.24 (br s, 2H), 8.31 (d, J = 5.0 Hz, 1H), 7.45 (s, 1H), 7.42 (d, J = 3.3 Hz, 2H), 7.29 (s, 1H), 7.17 (d, J = 1.8 Hz, 2H), 7.15 - 7.14 (m, 1H), 6.74 (s, 1H), 6.64 (d, J = 5.0 Hz, 1H), 4.28 (br d, J = 5.3 Hz, 2H), 4.01 (br s, 2H), 3.20 (br s, 2H), 3.16 - 3.08 (m, 2H), 2.09 - 2.01 (m, 2H), 1.58 (s, 6H), 1.26 - 1.19 (m, 2H). | 530.0 | 135 | 3.45 |
| A092 | 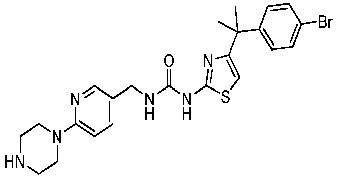 | 1-(4-(2-(4-bromophenyl)propan-2-yl)thiazol-2-yl)-3-((6-(piperazin-1-yl)pyridin-3-yl)methyl)urea | ¹H NMR (400MHz, DMSO-d₆) *δ* 10.90 (br s, 1H), 9.66 (br s, 2H), 8.05 - 7.92 (m, 2H), 7.48 - 7.28 (m, 4H), 7.21 - 7.10 (m, 2H), 6.75 (s, 1H), 4.30 - 4.20 (m, 2H), 4.04 - 3.92 (m, 4H), 3.24 (br s, 4H), 1.57 (s, 6H). | 517.2 | 20 | 0.57 |
| A093 | 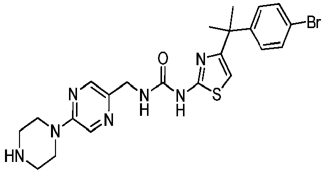 | 1-(4-(2-(4-bromophenyl)propan-2-yl)thiazol-2-yl)-3-((5-(piperazin-1-yl)pyrazin-2-yl)methyl)urea | ¹H NMR (400MHz, DMSO-d₆) *δ* 9.26 (s, 2H), 8.30 (s, 1H), 8.07 (s, 2H), 7.41 - 7.36 (m, 2H), 7.13 - 7.11 (m, 3H), 6.72 (s, 1H), 4.29 - 4.28 (m, 2H), 3.76 - 3.74 (m, 4H), 3.16 - 3.14 (m, 4H),1.54 (s, 6H). | 516.1 | 9 | 0.32 |
| A094 | 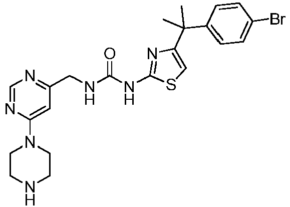 | 1-(4-(2-(4-bromophenyl)propan-2-yl)thiazol-2-yl)-3-((6-(piperazin-1-yl)pyrimidin-4-yl)methyl)urea | ¹H NMR (400MHz, DMSO-d₆) *δ* 11.05 (br s, 1H), 9.70 (br s, 2H), 8.86 (s, 1H), 7.48 - 7.43 (m, 2H), 7.39 - 7.33 (m, 1H), 7.21 - 7.14 (m, 3H), 6.78 (s, 1H), 4.45 (br d, *J* = 6.0 Hz, 2H), 4.11 (br s, 4H), 3.25 (br s, 4H), 1.59 (s, 6H). | 518.2 | 73 | 1.29 |
| A095 | 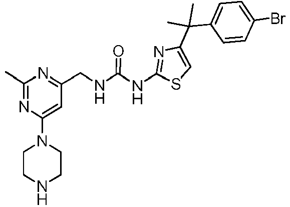 | 1-(4-(2-(4-bromophenyl)propan-2-yl)thiazol-2-yl)-3-((2-methyl-6-(piperazin-1-yl)pyrimidin-4-yl)methyl)urea | ¹HNMR (400 MHz, DMSO-*d*₆) *δ* 7.40 (d, J = 8.4Hz, 2H), 7.13 (d, J = 8.8Hz, 2H), 6.87(s, 1H), 6.70(s, 1H), 6.43( s, 1H), 4.12(s, 2H), 3.46-3.39(m, 4H), 2.69-2.67(m, 4H), 2.30(s, 3H), 1.55 (s, 6H). | 532.1 | 32 | |
| A096 | 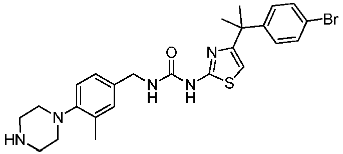 | 1-(4-(2-(4-bromophenyl)propan-2-yl)thiazol-2-yl)-3-(3-methyl-4-(piperazin-1-yl)benzyl)urea | ¹H NMR (400MHz, DMSO-*d₆*) *δ* 10.61 (br s, 1H), 9.04 (br s, 2H), 7.40 (d, *J* = 8.8 Hz, 2H), 7.12 (d, *J* = 8.8 Hz, 2H), 7.07 - 6.99 (m, 2H), 6.95 (br d, *J* = 7.6 Hz, 2H), 6.71 (s, 1H), 4.18 (br d, *J*=5.6 Hz, 2H), 3.17 (br s, 4H), 2.97 (br d, *J*=4.4 Hz, 4H), 2.19 (s, 3H), 1.54 (s, 6H). | [M+Na]⁺=55 2.0 | 69 | 1.43 |
| A097 | 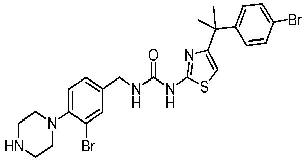 | 1-(3-bromo-4-(piperazin-1-yl)benzyl)-3-(4-(2-(4-bromophenyl)propan-2-yl)thiazol-2-yl)urea | ¹HNMR (400 MHz, CDCl₃) *δ* 9.18 (brs, 2H), 7.43 (d, *J* = 8.4 Hz, 2H), 7.57-7.29 (m, 1H), 7.18-7.13 (m, 4H), 6.75 (s, 1H), 4.24 (s, 2H), 3.23 - 3.14 (m, 8H), 1.58 (s, 6H). | 593.0 | 87 | 1.42 |
| A098 | 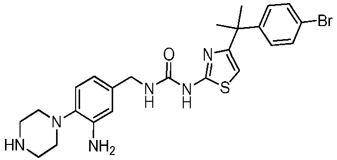 | 1-(3-amino-4-(piperazin-1-yl)benzyl)-3-(4-(2-(4-bromophenyl)propan-2-yl)thiazol-2-yl)urea | ¹HNMR (400 MHz, DMSO-*d*₆) *δ* 10.76(br, 1H), 9.22(br, 2H), 7.42-7.39(m, 2H), 7.26-7.12(m, 6H), 6.71(s, 1H), 4.26-4.25(m, 2H), 3.24-3.22(m, 4H), 3.03-3.02(m, 4H), 1.55(s, 6H). | 530.9 | 31 | 1.07 |
| A099 | 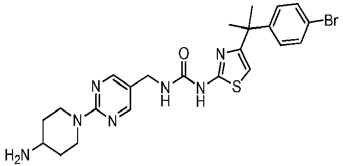 | 1-((2-(4-aminopiperidin-1-yl)pyrimidin-5-yl)methyl)-3-(4-(2-(4-bromophenyl)propan-2-yl)thiazol-2-yl)urea | ¹H NMR (400MHz, DMSO-*d*₆) *δ* 10.94 - 10.50 (m, 1H), 8.34 (s, 2H), 8.25 - 7.95 (m, 3H), 7.42 (d, J = 8.5 Hz, 2H), 7.19 - 6.92 (m, 3H), 6.74 (s, 1H), 4.71 - 4.54 (m, 2H), 4.19 - 4.05 (m, 2H), 3.38 - 3.24 (m, 1H), 3.04 - 2.87 (m, 2H), 2.03 - 1.90 (m, 2H), 1.57 (s, 6H), 1.49 - 1.35 (m, 2H). | 532.0 | 13 | 0.45 |
| A100 | 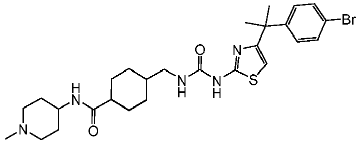 | 4-((3-(4-(2-(4-bromophenyl)propan-2-yl)thiazol-2-yl)ureido)methyl)-N-(1-methylpiperidin-4-yl)cyclohexane-1-carboxamide | | 576.0 | 72 | 1.37 |
| A101 | 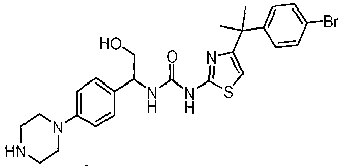 | 1-(4-(2-(4-bromophenyl)propan-2-yl)thiazol-2-yl)-3-(2-hydroxy-1-(4-(piperazin-1-yl)phenyl)ethyl)urea | | 544.1 | 50 | 1.65 |
| A102 | 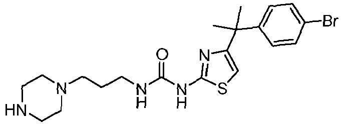 | 1-(4-(2-(4-bromophenyl)propan-2-yl)thiazol-2-yl)-3-(3-(piperazin-1-yl)propyl)urea | | 466.1 | 90 | 0.71 |
| A103 | 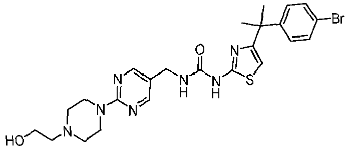 | 1-(4-(2-(4-bromophenyl)propan-2-yl)thiazol-2-yl)-3-((2-(4-(2-hydroxyethyl)piperazin-1-yl)pyrimidin-5-yl)methyl)urea | | 560.1 | 97 | 6.2 |
| A104 | 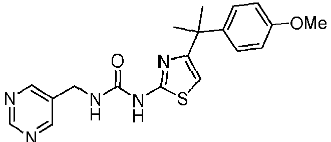 | 1-(4-(2-(4-methoxyphenyl)propan-2-yl)thiazol-2-yl)-3-(pyrimidin-5-ylmethyl)urea | | 384.1 | 223 | 1.69 |
| A105 | 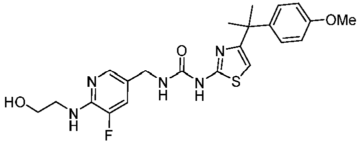 | 1-((5-fluoro-6-((2-hydroxyethyl)amino)pyr idin-3-yl)methyl)-3-(4-(2-(4-methoxyphenyl)propan-2-yl)thiazol-2-yl)urea | | 460.1 | 144 | 1.02 |
| A106 | 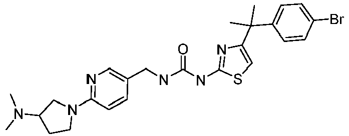 | 1-(4-(2-(4-bromophenyl)propan-2-yl)thiazol-2-yl)-3-((6-(3-(dimethylamino)pyrrolid in-1-yl)pyridin-3-yl)methyl)urea | | 543.1 | 48 | 6.72 |
| A107 | 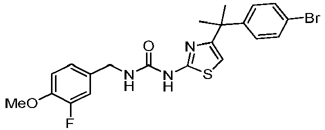 | 1-(4-(2-(4-bromophenyl)propan-2-yl)thiazol-2-yl)-3-(3-fluoro-4-methoxybenzyl)urea | | 478.1 | 216 | |
| A108 | 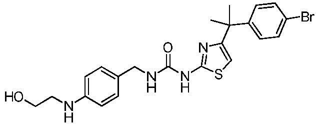 | 1-(4-(2-(4-bromophenyl)propan-2-yl)thiazol-2-yl)-3-(4-((2-hydroxyethyl)amino)ben zyl)urea | | 489.1 | 244 | 0.82 |
| A109 | 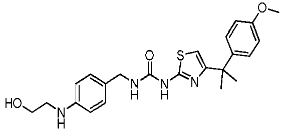 | 1-(4-((2-hydroxyethyl)amino)ben zyl)-3-(4-(2-(4-methoxyphenyl)propan-2-yl)thiazol-2-yl)urea | | 441.0 | 146 | |
| A110 | 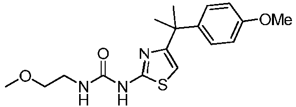 | 1-(2-methoxyethyl)-3-(4-(2-(4-methoxyphenyl)propan-2-yl)thiazol-2-yl)urea | | 350.0 | 193 | 0.94 |
| A111 | 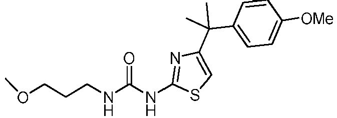 | 1-(4-(2-(4-methoxyphenyl)propan-2-yl)thiazol-2-yl)-3-(3-methoxypropyl)urea | | 364.0 | 127 | 1.24 |
| A112 | 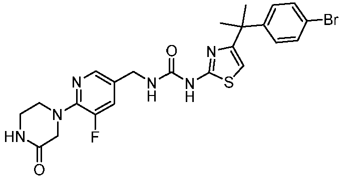 | 1-(4-(2-(4-bromophenyl)propan-2-yl)thiazol-2-yl)-3-((5-fluoro-6-(3-oxopiperazin-1-yl)pyridin-3-yl)methyl)urea | | 547.1 | 334 | |
| A113 | 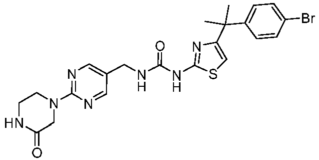 | 1-(4-(2-(4-bromophenyl)propan-2-yl)thiazol-2-yl)-3-((2-(3-oxopiperazin-1-yl)pyrimidin-5-yl)methyl)urea | | 530.1 | 126 | |
| A114 | 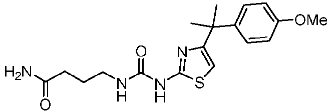 | 4-(3-(4-(2-(4-methoxyphenyl)propan-2-yl)thiazol-2-yl)ureido)butanamide | | 377.0 | 260 | |
| A115 | 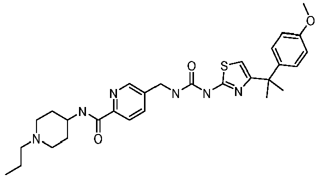 | 5-((3-(4-(2-(4-methoxyphenyl)propan-2-yl)thiazol-2-yl)ureido)methyl)-N-(1-propylpiperidin-4-yl)picolinamide | | 551.0 | 19 | 0.83 |
| A116 | 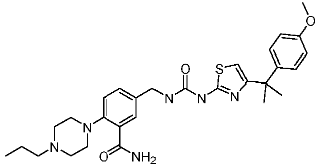 | 5-((3-(4-(2-(4-methoxyphenyl)propan-2-yl)thiazol-2-yl)ureido)methyl)-2-(4-propylpiperazin-1-yl)benzamide | | 551.0 | 72 | 0.72 |
| A117 | 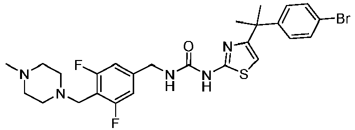 | 1-(4-(2-(4-bromophenyl)propan-2-yl)thiazol-2-yl)-3-(3,5-difluoro-4-((4-methylpiperazin-1-yl)methyl)benzyl)urea | | 578.1 | 67 | 5.03 |
| A118 | 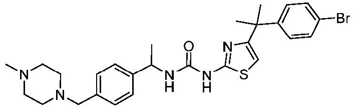 | 1-(4-(2-(4-bromophenyl)propan-2-yl)thiazol-2-yl)-3-(1-(4-((4-methylpiperazin-1-yl)methyl)phenyl)ethyl) urea | | 556.1 | 227 | 4.38 |
| A119 | 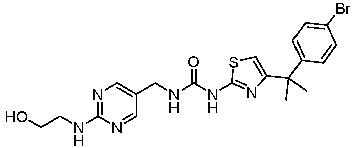 | 1-(4-(2-(4-bromophenyl)propan-2-yl)thiazol-2-yl)-3-((2-((2-hydroxyethyl)amino)pyr imidin-5-yl)methyl)urea | | 491.1 | 36 | 4.43 |
| A120 | 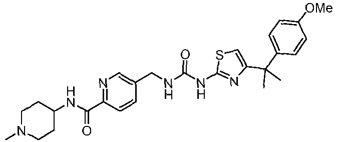 | 5-((3-(4-(2-(4-methoxyphenyl)propan-2-yl)thiazol-2-yl)ureido)methyl)-N-(1-methylpiperidin-4-yl)picolinamide | | *523.0* | 53 | 0.99 |
| A121 | 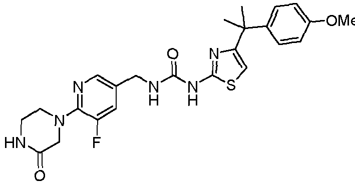 | 1-((5-fluoro-6-(3-oxopiperazin-1-yl)pyridin-3-yl)methyl)-3-(4-(2-(4-methoxyphenyl)propan-2-yl)thiazol-2-yl)urea | | 499.0 | 209 | 2.02 |
| A122 | 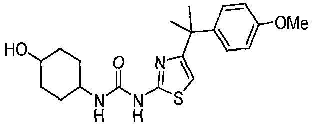 | 1-(4-hydroxycyclohexyl)-3-(4-(2-(4-methoxyphenyl)propan-2-yl)thiazol-2-yl)urea | | 390.0 | 219 | 1.70 |
| A123 | 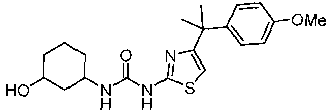 | 1-(3-hydroxycyclohexyl)-3-(4-(2-(4-methoxyphenyl)propan-2-yl)thiazol-2-yl)urea | | 390.0 | 354 | 2.40 |
| A124 | 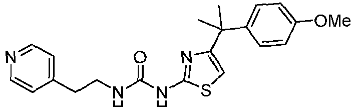 | 1-(4-(2-(4-methoxyphenyl)propan-2-yl)thiazol-2-yl)-3-(2-(pyridin-4-yl)ethyl)urea | | 397.0 | 165 | 1.05 |
| A125 | 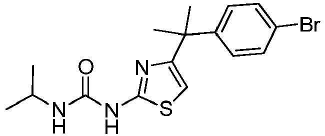 | 1-(4-(2-(4-bromophenyl)propan-2-yl)thiazol-2-yl)-3-isopropylurea | ¹H NMR (400 MHz, CDCl₃) *δ* 8.57 (br s, 1H), 7.39 (d, *J* = 8.8 Hz, 2H), 7.13 (d, *J* = 8.4 Hz, 2H), 6.43 (s, 1H), 3.96-3.82 (m, 1H), 1.64 (s, 6H), 1.10 (d, *J* = 6.4 Hz, 6H). | 382.1 | 28 | 2.80 |
| A126 | 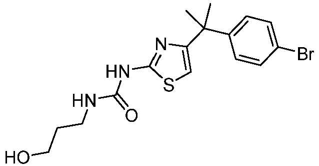 | 1-(4-(2-(4-bromophenyl)propan-2-yl)thiazol-2-yl)-3-(3-hydroxypropyl)urea | ¹HNMR (400 MHz, CDCl₃) *δ* 7.30 -7.26 (m, 2H), 7.15 - 7.10 (m, 2H), 6.47 (s, 1H), 3.60-3.55 (m, 2H), 3.45-3.30 (m, 2H), 1.63 (s, 6H), 1.40 - 1.30 (m, 2H). | 398.1 | 88 | 2.96 |
| A127 | 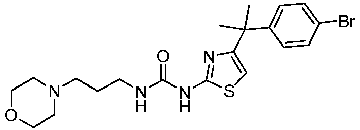 | 1-(4-(2-(4-bromophenyl)propan-2-yl)thiazol-2-yl)-3-(3-morpholinopropyl)urea | ¹HNMR (400 MHz, DMSO-*d*₆) *δ* 10.50 (br s, 1 H), 7.40 (d, J=8.56 Hz, 2 H), 7.12 (d, J=8.56 Hz, 2 H), 6.68 (s, 1 H), 6.29 (br s, 1 H), 3.53 (t, J=4.52 Hz, 4 H), 3.09 (q, J=6.60 Hz, 2 H), 2.27 (br s, 4 H), 2.22 (t, J=6.97 Hz, 2 H) 1.54 (s, 6 H), 1.53 - 1.47 (m, 2 H). | 467.1 | 113 | 0.94 |
| A128 | 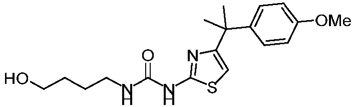 | 1-(4-hydroxybutyl)-3-(4-(2-(4-methoxyphenyl)propan-2-yl)thiazol-2-yl)urea | | 364.1 | 146 | 0.53 |
| A129 | 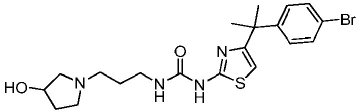 | 1-(4-(2-(4-bromophenyl)propan-2-yl)thiazol-2-yl)-3-(3-(3-hydroxypyrrolidin-1-yl)propyl)urea | ¹HNMR (400 MHz, CDCl₃) *δ* 7.40 - 7.34 (m, 2H), 7.40 - 7.34 (m, 2H), 7.08 (d, J=8.78 Hz, 2 H), 6.56 (br s, 1 H), 5.00 - 4.84 (m, 1H), 4.12 - 4.00 (m, 1H), 3.46 - 3.19 (m, 3H), 3.01 - 2.74 (m, 3H), 2.40 - 2.21 (m, 1H), 2.00 - 1.82 (m, 2H), 1.64 - 1.51 (m, 6H), 1.36 - 1.20 (m, 2H). | 467.2 | 40 | 0.51 |
| A130 | 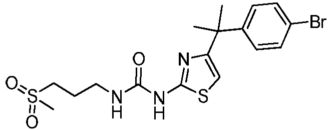 | 1-(4-(2-(4-bromophenyl)propan-2-yl)thiazol-2-yl)-3-(3-(methylsulfonyl)propyl) urea | ¹HNMR (400 MHz, CDCl₃) *δ* 7.38 (d, J=8.07 Hz, 2 H), 7.10 (d, J=8.31 Hz, 2 H), 6.47 (s, 1 H), 3.38 (q, J=6.11 Hz, 2 H), 2.99 (br t, J=7.58 Hz, 2 H), 2.86 (s, 3 H), 2.03 - 1.95 (m, 2 H),1.62 (s, 6 H). | 462.1 | 293 | 1.10 |
| A131 | 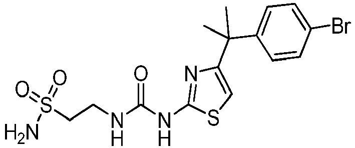 | 2-(3-(4-(2-(4-bromophenyl)propan-2-yl)thiazol-2-yl)ureido)ethanesulfona mide | ¹HNMR (400 MHz, CD3OD) *δ* 7.38 (d, J=8.31 Hz, 2 H), 7.16 (d, J=8.56 Hz, 2 H), 6.66 (s, 1 H), 3.67 (t, J=6.48 Hz, 2 H), 3.26 (t, J=6.36 Hz, 2 H), 1.63 (s, 6 H). | 447.0 | 219 | 0.94 |
| A132 | 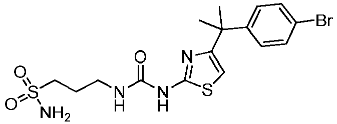 | 3-(3-(4-(2-(4-bromophenyl)propan-2-yl)thiazol-2-yl)ureido)propane-1-sulfonamide | ¹HNMR (400 MHz, CDCl₃) δ 7.43 - 7.35 (m, 2 H), 7.13 - 7.03 (m, 2 H), 6.53 (s, 1 H) 5.56 - 5.31 (m, 1 H), 3.34 (d, *J* =5.77 Hz, 2 H), 3.03 (s, 2 H), 1.99 - 1.89 (m, 2 H), 1.61 (s, 6 H). | 463.1 | 80 | 4.56 |
| A133 | 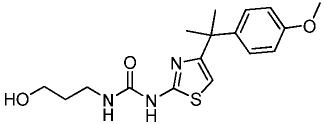 | 1-(3-hydroxypropyl)-3-(4-(2-(4-methoxyphenyl)propan-2-yl)thiazol-2-yl)urea | | 350.2 | 57 | 0.60 |
| A134 | 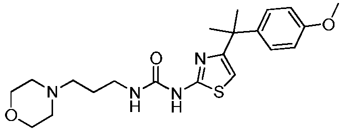 | 1-(4-(2-(4-methoxyphenyl)propan-2-yl)thiazol-2-yl)-3-(3-morpholinopropyl)urea | ¹H NMR (400 MHz, DMSO-*d*₆) *δ* 10.46 (br s, 1 H), 7.11 - 7.05 (m, 2 H), 6.77 (d, J=9.05 Hz, 2 H), 6.59 (s, 1 H), 6.32 (br s, 1 H), 3.66 (s, 3 H), 3.53 (br t, J=4.52 Hz, 4 H), 3.12 - 3.06 (m, 2 H), 2.27 (br s, 4 H), 2.22 (t, J=6.97 Hz, 2 H), 1.59 - 1.49 (m, 8 H), | 419.2 | 152 | 5.11 |
| A135 | 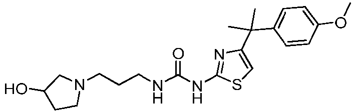 | 1-(3-(3-hydroxypyrrolidin-1-yl)propyl)-3-(4-(2-(4-methoxyphenyl)propan-2-yl)thiazol-2-yl)urea | ¹H NMR (400 MHz, CDCl₃) δ 7.08 (d, *J* = 8.8 Hz, 2H), 6.77 (d, *J* = 8.8 Hz, 2H), 6.69 (s, 1H), 6.60 (s, 1H), 5.40 (s, 1H), 4.34 (s, 1H), 3.66 (s, 3H), 3.11 (s, 3H), 3.03 (s, 3H), 1.77 (s, 2H), 1.53 (s, 6H), 1.35 - 1.15 (m, 2H), 0.95 - 0.85 (m, 2H). | 419.2 | 112 | 1.24 |
| A136 | 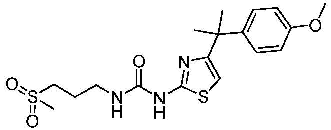 | 1-(4-(2-(4-methoxyphenyl)propan-2-yl)thiazol-2-yl)-3-(3-(methylsulfonyl)propyl) urea | ¹HNMR (400 MHz, CD3OD) *δ* 7.17 (d, J = 8.8Hz, 2H), 6.82 (d, J = 8.8Hz, 2H), 6.43(s, 3H), 3.78(s, 3H), 3.42-3.38(m, 2H), 3.00-2.97(m, 2H), 2.86(s, 3H), 2.05-1.98(m, 2H), 1.64(s, 3H) | 412.2 | 209 | 0.64 |
| A137 | 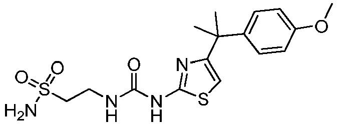 | 2-(3-(4-(2-(4-methoxyphenyl)propan-2-yl)thiazol-2-yl)ureido)ethanesulfona mide | ¹HNMR (400 MHz, CD3OD) *δ* 7.18 (d, *J =* 8.8 Hz, 2H), 7.25 (d, *J* = 8.4 Hz, 2H), 6.61 (s, 1H), 3.77 (s, 3H), 3.72-3.66 (m, 2H), 3.30-3.25 (m, 2H), 1.65 (s, 6H). | 399.2 | 42 | 1.96 |
| A138 | 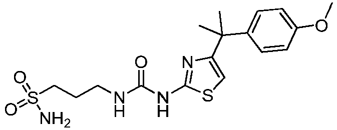 | 3-(3-(4-(2-(4-methoxyphenyl)propan-2-yl)thiazol-2-yl)ureido)propane-1-sulfonamide | ¹H NMR (400 MHz, CD3OD) *δ* 7.17 (d, *J* = 8.8 Hz, 2H), 6.83 (d, *J* = 8.0 Hz, 2H), 6.63 (s, 1H), 3.77 (s, 3H), 3.39-3.35 (m, 2H), 3.15-3.02 (m, 2H), 2.12-2.00 (m, 2H), 1.65 (s, 6H). | 413.2 | 97 | 2.38 |
| A139 | 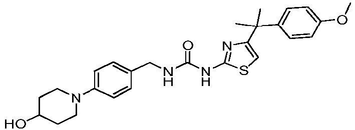 | 1-(4-(4-hydroxypiperidin-1-yl)benzyl)-3-(4-(2-(4-methoxyphenyl)propan-2-yl)thiazol-2-yl)urea | ¹HNMR (400 MHz, DMSO-*d*₆) *δ* 10.48 (s, 1 H), 7.09 (dd, J=12.59, 8.68 Hz, 4 H), 6.87 (d, J=8.56 Hz, 2 H), 6.79 (d, J=9.05 Hz, 2 H), 6.68 (br s, 1 H), 6.63 (s, 1 H), 4.67 (d, J=4.16 Hz, 1 H), 4.17 (d, J=5.87 Hz, 2 H), 3.69 (s, 3 H), 3.59 (td, J=8.80, 4.40 Hz, 1 H), 3.51 - 3.44 (m, 2 H), 2.83 - 2.72 (m, 2 H), 1.82 - 1.73 (m, 2 H), 1.56 (s, 6 H), 1.52 - 1.34 (m, 2 H). | 481.2 | 261 | 1.35 |
| A140 | 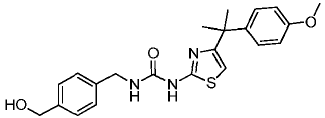 | 1-(4-(hydroxymethyl)benzyl) -3-(4-(2-(4-methoxyphenyl)propan-2-yl)thiazol-2-yl)urea | ¹H NMR (400 MHz, CDCl₃) *δ* 7.25 - 7.22 (m, 2H), 7.18-7.10 (m, 4H), 6.83-6.73 (m, 2H), 6.45 (s, 1H), 4.57 (s, 2H), 4.31 - 4.29 (m, 2H), 3.76 (s, 3H), 1.59 (s, 6H). | 412.2 | 131 | 5.52 |
| A141 | 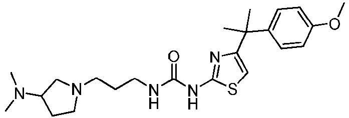 | 1-(3-(3-(dimethylamino)pyrrolid in-1-yl)propyl)-3-(4-(2-(4-methoxyphenyl)propan-2-yl)thiazol-2-yl)urea | ¹H NMR (400 MHz, DMSO-*d*₆) *δ* 10.43 (s, 1H) 7.08 (d, J=8.82 Hz, 2H) 6.77 (d, J=8.82 Hz, 2H) 6.58 (s, 1H) 6.36 (s, 1H) 3.66 (s, 3H) 3.08 (q, J=6.39 Hz, 2H) 2.57-2.70 (m, 2H) 2.50-2.56 (m, 1H) 2.23-2.40 (m, 3H) 2.19 (t, J=7.06 Hz, 1H) 2.05 (s, 6H) 1.73-1.84 (m, 1H) 1.44-1.60 (m, 9H). | 446.3 | 228 | 4.22 |
| A142 | 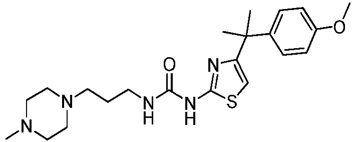 | 1-(4-(2-(4-methoxyphenyl)propan-2-yl)thiazol-2-yl)-3-(3-(4-methylpiperazin-1-yl)propyl)urea | ¹HNMR (400 MHz, DMSO-*d*₆) *δ* 10.49(br, 1H), 7.14-7.12(m, 2H), 6.83-6.80(m, 2H), 6.63(s, 1H), 6.34(br, 1H), 3.73(s, 3H), 3.14-3.09(m, 2H), 2.53(s, 3H), 2.40-2.29(m, 9H), 2.21(s, 3H), 1.57-1.52(m, 8H). | 432.3 | 180 | 1.33 |
| A143 | 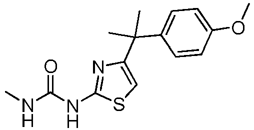 | 1-(4-(2-(4-methoxyphenyl)propan-2-yl)thiazol-2-yl)-3-methylurea | ¹H NMR (400 MHz, CD3OD) *δ* 7.17 (d, *J* = 9.2 Hz, 2H), 7.25 (d, *J* = 8.8 Hz, 2H), 6.60 (s, 1H), 3.77 (s, 3H), 2.78 (s, 3H), 1.64 (s, 6H). | 306.2 | 53 | |
| A144 | 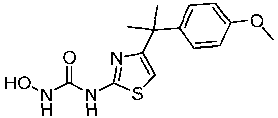 | 1-hydroxy-3-(4-(2-(4-methoxyphenyl)propan-2-yl)thiazol-2-yl)urea | ¹HNMR (400 MHz, DMSO-*d*₆) *δ* 9.26 - 8.43 (m, 2H), 7.12 - 7.04 (m, 2H), 6.80 - 6.74 (m, 2H), 6.67 (s, 1H), 3.66 (s, 3H), 1.54 (s, 6H). | 308.1 | 154 | 1.75 |
| A145 | 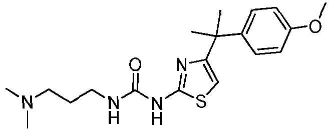 | 1-(3-(dimethylamino)propyl) -3-(4-(2-(4-methoxyphenyl)propan-2-yl)thiazol-2-yl)urea | ¹HNMR (400 MHz, CD3OD) *δ* 7.14 (d, J=8.82 Hz, 2 H), 6.80 (d, J=9.04 Hz, 2 H), 6.59 (s, 1 H), 3.74 (s, 3 H), 3.22 (t, J=6.84 Hz, 2 H), 2.35 - 2.30 (m, 2 H), 2.21 (s, 6 H), 1.71 - 1.64 (m, 2 H), 1.62 (s, 6 H). | 377.2 | 216 | 4.49 |
| A146 | 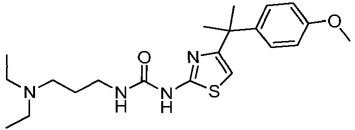 | 1-(3-(diethylamino)propyl)-3-(4-(2-(4-methoxyphenyl)propan-2-yl)thiazol-2-yl)urea | ¹H NMR (400MHz, CD3OD) *δ* 7.14 (d, *J* = 8.8 Hz, 2H), 6.80 (d, *J* = 8.8 Hz, 2H), 6.59 (s, 1H), 3.74 (s, 3H), 3.21 (br t, *J* = 6.7 Hz, 2H), 2.68 - 2.24 (m, 6H), 1.97 - 1.39 (m, 8H), 1.03 (t, *J* = 7.2 Hz, 6H). | 405.2 | 124 | 3.07 |
| A147 | 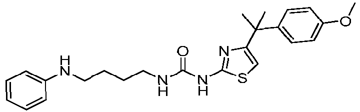 | 1-(4-(2-(4-methoxyphenyl)propan-2-yl)thiazol-2-yl)-3-(4-(phenylamino)butyl)ure a | ¹H NMR (400 MHz, CD3OD) *δ* 7.13 (d, J=8.82 Hz, 2 H), 7.09 - 7.04 (m, 3 H), 6.79 (d, J=8.82 Hz, 2 H), 6.62 - 6.55 (m, 4 H), 3.72 (s, 3 H), 3.25 - 3.19 (m, 2 H), 3.06 (br t, J=6.50 Hz, 2 H), 1.61 (s, 6 H), 1.59 - 1.54 (m, 4 H). | 439.3 | 235 | 1.91 |
| A148 | 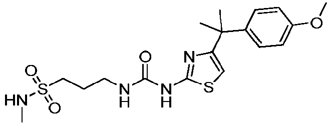 | 3-(3-(4-(2-(4-methoxyphenyl)propan-2-yl)thiazol-2-yl)ureido)-N-methylpropane-1-sulfonamide | ¹H NMR (400MHz, CDCl₃) *δ* 7.16 - 7.14 (m, 2H), 6.84 - 6.82 (m, 2H), 6.46 (s, 1H), 3.78 (s, 3H), 3.38 - 3.34 (m, 2H), 2.97 - 2.93 (m, 2H), 2.68 - 2.67 (m, 3H), 1.96 - 1.89 (m, 2H), 1.63 (s, 6H). | 427.0 | 45 | 0.85 |
| A149 | 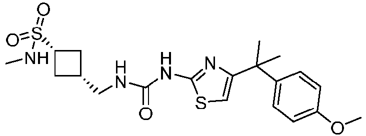 | rac-(1s,3s)-3-((3-(4-(2-(4-methoxyphenyl)propan-2-yl)thiazol-2-yl)ureido)methyl)-N-methylcyclobutane-1-sulfonamide | ¹H NMR (400MHz, CDCl₃) *δ* 8.67 (br s, 1H), 7.13 - 7.11 (m, 2H), 6.82 - 6.80 (m, 2H), 6.41 (s, 1H), 4.85 (br s, 1H), 3.77 (s, 3H), 3.67 - 3.60 (m, 1H), 3.27 - 3.24 (m, 2H), 2.64 - 2.63 (m, 3H), 2.41 - 2.37 (m, 1H), 2.25 - 2.17 (m, 2H), 2.10 - 2.05 (m, 2H), 1.61 (s, 6H). | 453.2 | 155 | 9.40 |
| A150 | 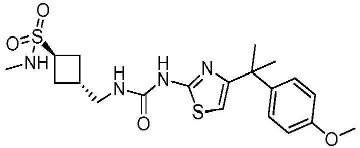 | rac-(1r,3r)-3-((3-(4-(2-(4-methoxyphenyl)propan-2-yl)thiazol-2-yl)ureido)methyl)-N-methylcyclobutane-1-sulfonamide | ¹H NMR (400MHz, CDCl₃) *δ* 9.06 (br s, 1H), 7.12 - 7.09 (m, 2H), 6.82 - 6.79 (m, 2H), 6.44 (s, 1H), 4.94 (br s, 1H), 3.77 (s, 3H), 3.75 - 3.64 (m, 1H), 3.31 - 3.28 (m, 2H), 2.68 - 2.67 (m, 3H), 2.51 - 2.47 (m, 1H), 2.43 - 2.36 (m, 2H), 1.99 - 1.91 (m, 2H), 1.60 (s, 6H). | 453.2 | 25 | 1.41 |
| A151 | 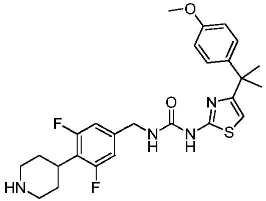 | 1-(3,5-difluoro-4-(piperidin-4-yl)benzyl)-3-(4-(2-(4-methoxyphenyl)propan-2-yl)thiazol-2-yl)urea hydrochloride | ¹H NMR (400MHz, DMSO-*d*₆) *δ* 10.72 (br, 1H), 9.10-8.70 (m, 2H), 7.09 - 7.07(m, 2H), 6.95 - 6.93 (m, 2H), 6.78-6.76 (m, 2H), 6.62 (s, 1H), 4.22 - 4.20 (m, 2H), 3.66 (s, 3H), 3.30 - 3.25 (m, 4H), 3.20 - 3.14 (m, 4H), 1.53 (s, 6H). | 502.1 | 43 | 0.95 |
| A152 | 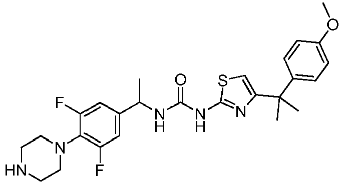 | 1-(1-(3,5-difluoro-4-(piperazin-1-yl)phenyl)ethyl)-3-(4-(2-(4-methoxyphenyl)propan-2-yl)thiazol-2-yl)urea | ¹H NMR (400MHz, DMSO-d₆) *δ* 10.74 (br s, 2H), 7.27 (s, 1H), 7.13 - 7.10 (m, 2H), 6.99 - 6.97 (m, 2H), 6.82 - 6.79 (m, 2H), 6.68 (s, 1H), 4.26 - 4.24 (m, 2H), 3.70 (s, 3H), 3.47 - 3.40 (m, 4H), 3.28 - 3.25 (m, 2H), 3.16 - 3.08 (m, 2H), 2.80 - 2.79 (m, 3H),1.57 (s, 6H). | 516.1 | 55 | 0.35 |
| A153 | 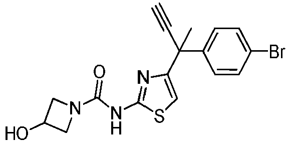 | N-(4-(2-(4-bromophenyl)but-3-yn-2-yl)thiazol-2-yl)-3-hydroxyazetidine-1-carboxamide | ¹H NMR (400MHz, CDCl₃) *δ* 7.84(br, 1H), 7.44(d, J = 8.4Hz, 2H), 7.34(d, J = 8.8Hz, 2H), 7.28(s, 1H), 6.80(s, 1H), 4.72-4.68(m, 1H), 4.29-4.25(m, 2H), 3.95-3.91(m, 2H), 2.58(s, 1H), 1.93(s, 3H). | 408.0 | 76 | 0.37 |
| A154 | 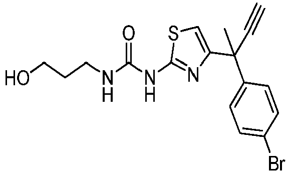 | 1-(4-(2-(4-bromophenyl)but-3-yn-2-yl)thiazol-2-yl)-3-(3-hydroxypropyl)urea | ¹H NMR (400MHz, CDCl₃) *δ* 7.46(d, J = 7.6Hz, 2H), 7.37(d, J = 8.4Hz, 2H), 6.76(s, 1H), 3.64(t, J = 5.2Hz, 2H), 3.43(t, J = 6.0Hz, 2H), 2.58(s, 1H), 1.95(s, 3H), 1.70-1.69(m, 2H). | 408.0 | 21 | 0.38 |
| A155 | 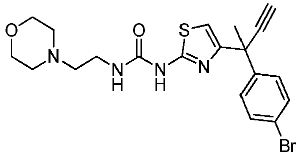 | 1-(4-(2-(4-bromophenyl)but-3-yn-2-yl)thiazol-2-yl)-3-(2-morpholinoethyl)urea | ¹H NMR (400MHz, CDCl₃) *δ* 7.48-7.42 (m, 4H), 7.00(s, 1H), 4.11-4.08 (m, 2H), 3.82-3.67(m, 2H), 3.66-3.63(m, 4H), 3.35(s, 2H), 3.33-3.21(m, 2H), 3.00(s, 1H), 1.94(s, 3H). | 463.1 | 30 | 0.90 |
| A156 | 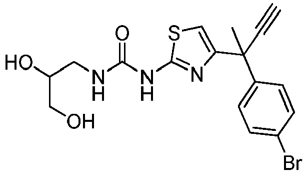 | 1-(4-(2-(4-bromophenyl)but-3-yn-2-yl)thiazol-2-yl)-3-(2,3-dihydroxypropyl)urea | ¹H NMR (400MHz, CD3OD) 7.44 (s, 4H), 6.91 (d, J=1.0 Hz, 1H), 3.66-3.75 (m, 1H), 3.51 (d, J=5.3 Hz, 2H), 3.38-3.47 (m, 1H), 3.21 (ddd, J=13.8, 6.9, 2.6 Hz, 1H), 2.95 (s, 1H), 1.92 ppm (s, 3H) | 424.0 | 22 | 0.38 |
| A157 | 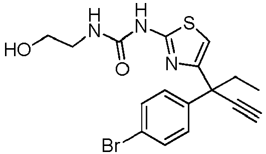 | 1-(4-(3-(4-bromophenyl)pent-1-yn-3-yl)thiazol-2-yl)-3-(2-hydroxyethyl)urea | ¹H NMR R (400 MHz, CDCl₃) *δ* 7.44 - 7.42 (m, 2 H), 7.37 - 7.35 (m, 2 H), 6.81 (s, 1 H), 3.53 - 3.50 (m, 2 H), 3.38- 3.35 (m, 2 H), 2.59 (s, 1 H), 2.38- 2.29 (m, 1 H), 2.21 - 2.12 (m, 1 H), 0.94 (t, J = 7.6 Hz, 3 H) | 408.0 | 22 | 1.36 |
| A158 | 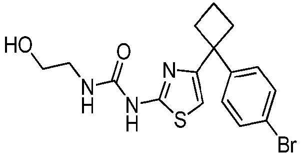 | 1-(4-(1-(4-bromophenyl)cyclobutyl )thiazol-2-yl)-3-(2-hydroxyethyl)urea | ¹H NMR (400 MHz, DMSO-*d*₆) *δ* 10.50 (s, 1 H), 7.47 (br d, J = 8.28 Hz, 2 H), 7.19 (br d, J = 8.28 Hz, 2 H), 6.66 (s, 1 H), 6.47 (br s, 1 H), 4.76 (br s, 1 H), 3.41 (br d, J = 5.27 Hz, 2 H), 3.16 (br d, J = 5.77 Hz, 2 H), 2.65 (br d, J = 13.30 Hz, 2 H), 2.33 (br s, 2 H), 1.91 - 2.03 (m, 1 H), 1.83 (br s, 1 H) | 398.0 | 67 | 2.86 |
| A159 | 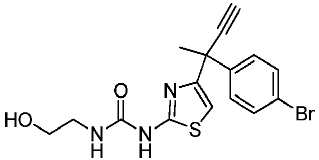 | 1-(4-(2-(4-bromophenyl)but-3-yn-2-yl)thiazol-2-yl)-3-(2-hydroxyethyl)urea | ¹H NMR(400MHz,CDCl₃) *δ* 7.46 (d, J=8.5 Hz, 2H), 7.36 (d, J=8.5 Hz, 2H), 6.72 (s, 1H), 3.62 (br d, J=4.3 Hz, 2H), 3.36 (br d, J=3.9 Hz, 2H), 2.67 (s, 1H), 1.96 (s, 3H) | 394.0 | 48 | 0.49 |
| A160 | 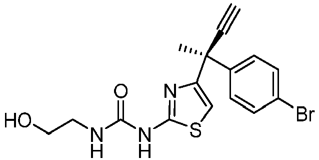 | (S)-1-(4-(2-(4-bromophenyl)but-3-yn-2-yl)thiazol-2-yl)-3-(2-hydroxyethyl)urea | ¹H NMR (400MHz, CDCl₃) *δ* 7.41-7.39(m, 2H), 7.31-7.29(m, 2H), 7.24(s, 1H), 3.45-3.43(m, 2H), 3.32-3.29(m, 2H), 2.52(s, 1H), 1.89(s, 3H). | 394.0 | 10 | 0.17 |
| A161 | 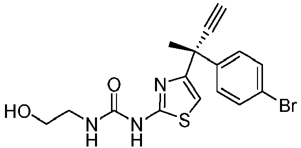 | (R)-1-(4-(2-(4-bromophenyl)but-3-yn-2-yl)thiazol-2-yl)-3-(2-hydroxyethyl)urea | ¹H NMR (400MHz, CDCl₃) *δ* 7.37-7.35(m, 2H), 7.26-7.24(m, 2H), 7.19(s, 1H), 6.69(s, 1H), 3.38-3.37(m, 2H), 3.25-3.24(m, 2H), 2.47(s, 1H), 1.84(s, 3H). | 394.0 | 68 | 0.55 |
| A162 | 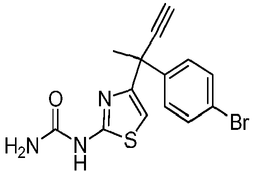 | 1-(4-(2-(4-bromophenyl)but-3-yn-2-yl)thiazol-2-yl)urea | ¹H NMR (400MHz, DMSO-*d*₆) *δ* 10.60(s, 1H), 7.53-7.50(m, 2H), 7.39-7.37(m, 2H), 6.90(s, 1H), 3.50(s, 1H), 1.85(s, 3H). | 352.0 | 38 | 0.18 |
| A163 | 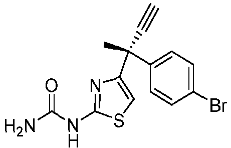 | (S)-1-(4-(2-(4-bromophenyl)but-3-yn-2-yl)thiazol-2-yl)urea | ¹H NMR (400MHz, DMSO-*d*₆) *δ* 10.61(s, 1H), 7.53-7.51(m, 2H), 7.39-7.34(m, 2H), 5.76(s, 3H), 3.50(s, 1H), 1.85(s, 3H). | 349.9 | 4 | 0.35 |
| A164 | 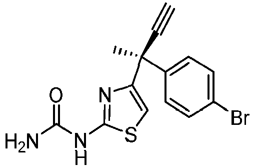 | (R)-1-(4-(2-(4-bromophenyl)but-3-yn-2-yl)thiazol-2-yl)urea | ¹H NMR (400MHz, DMSO-*d*₆) *δ* 10.64(s, 1H), 7.53-7.50(m, 2H), 7.39-7.37(m, 2H), 6.89(s, 1H), 3.50(s, 1H), 1.85(s, 3H). | 350.0 | 17 | 0.59 |
| A165 | 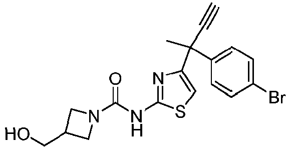 | N-(4-(2-(4-bromophenyl)but-3-yn-2-yl)thiazol-2-yl)-3-(hydroxymethyl)azetidin e-1-carboxamide | ¹H NMR (400MHz, CDCl₃) *δ* 7.79(br, 1H), 7.41-7.39(m, 2H), 7.31-7.29(m, 2H), 7.24(s, 1H), 6.75(s, 1H), 4.07(t, J = 8.0Hz, 2H), 3.83-3.80(m, 2H), 3.72-3.71(m, 2H), 2.81-2.77(m, 1H), 2.54(s, 1H), 1.89(s, 3H). | 420.0 | 138 | 0.73 |
| A166 | 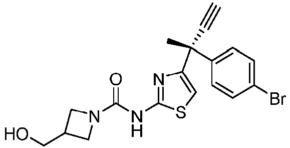 | (R)-N-(4-(2-(4-bromophenyl)but-3-yn-2-yl)thiazol-2-yl)-3-(hydroxymethyl)azetidin e-1-carboxamide | ¹H NMR (400MHz, CDCl₃) *δ* 7.85(br, 1H), 7.46 -7.43 (m, 2H), 7.36-7.33 (m, 2H), 6.79 (s, 1H), 4.11 - 4.08 (m, 2H), 3.88-3.85 (m, 2H), 3.76 -3.75 (m, 2H), 2.87-2.80 (m, 1H), 2.58 (s, 1H), 1.94 (s, 3H). | 420.0 | 25 | 5.71 |
| A167 | 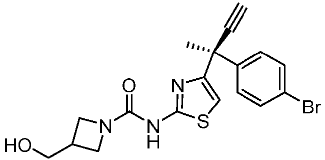 | (S)-N-(4-(2-(4-bromophenyl)but-3-yn-2-yl)thiazol-2-yl)-3-(hydroxymethyl)azetidin e-1-carboxamide | ¹H NMR (400MHz, CDCl₃) *δ* 7.85(br, 1H), 7.45 -7.43 (m, 2H), 7.36-7.33 (m, 2H), 6.79 (s, 1H), 4.11 - 4.09 (m, 2H), 3.87-3.85 (m, 2H), 3.77 -3.75 (m, 2H), 2.85-2.81 (m, 1H), 2.58 (s, 1H), 1.93(s, 3H). | 420.0 | 7 | 0.74 |
| A168 | 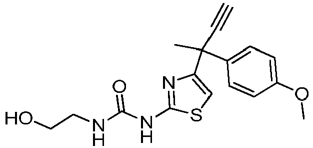 | 1-(2-hydroxyethyl)-3-(4-(2-(4-methoxyphenyl)but-3-yn-2-yl)thiazol-2-yl)urea | ¹H NMR (400MHz, CDCl₃) *δ* 7.38(d, J = 8.8Hz, 2H), 6.87-6.84(m, 2H), 6.73(s, 1H), 3.80(s, 3H), 3.45(s, 2H), 3.35-3.33(m, 2H), 2.54(s, 1H), 1.94(s, 3H). | 346.1 | 27 | 0.40 |
| A169 | 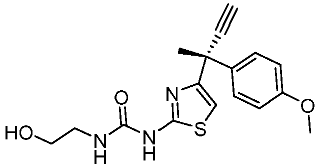 | (R)-1-(2-hydroxyethyl)-3-(4-(2-(4-methoxyphenyl)but-3-yn-2-yl)thiazol-2-yl)urea | ¹H NMR (400MHz, CDCl₃) *δ* 7.36 (d, J = 8.8 Hz, 2H), 6.83 (d, J = 8.8 Hz, 2H), 6.70 (s, 1H), 3.78 (s, 3H), 3.46 (br d, J = 4.8 Hz, 2H), 3.32 (br d, J = 4.8 Hz, 2H), 2.52 (s, 1H), 1.92 (s, 3H) | 346.1 | 23 | 1.67 |
| A170 | 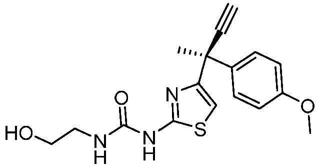 | (S)-1-(2-hydroxyethyl)-3-(4-(2-(4-methoxyphenyl)but-3-yn-2-yl)thiazol-2-yl)urea | ¹H NMR (400MHz, CDCl₃) *δ* 7.37 (d, J = 8.8 Hz, 2H), 6.84 (d, J = 8.8 Hz, 2H), 6.70 (s, 1H), 3.78 (s, 3H), 3.49 (br d, J = 5.3 Hz, 2H), 3.34 (br d, J = 5.3 Hz, 2H), 2.52 (s, 1H), 1.92 (s, 3H) | 346.1 | 8 | 0.46 |
| A171 | 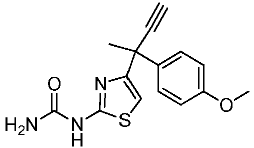 | 1-(4-(2-(4-methoxyphenyl)but-3-yn-2-yl)thiazol-2-yl)urea | ¹H NMR (400MHz, DMSO-*d*₆) *δ* 10.58 (s, 1H), 7.33 (d, J = 8.8 Hz, 2H), 6.86 (d, J = 8.8 Hz, 2H), 6.81 (s, 1H), 6.22 (br s, 2H), 3.72 (s, 3H), 3.40 (s, 1H), 1.83 (s, 3H) | 302.0 | 16 | 0.73 |
| A172 | 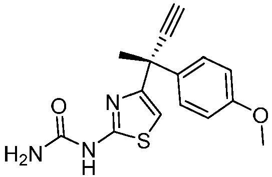 | (R)-1-(4-(2-(4-methoxyphenyl)but-3-yn-2-yl)thiazol-2-yl)urea | ¹H NMR (400MHz, DMSO-*d*₆) *δ* 10.58 (br s, 1H), 7.33 (d, J = 8.8 Hz, 2H), 6.86 (d, J = 8.8 Hz, 2H), 6.81 (s, 1H), 6.24 (br s, 1H), 3.72 (s, 3H), 3.41 (s, 1H), 1.83 (s, 3H) | 302.0 | 36 | 1.28 |
| A173 | 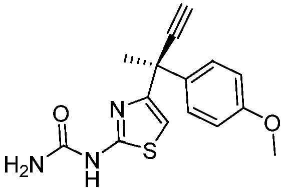 | (S)-1-(4-(2-(4-methoxyphenyl)but-3-yn-2-yl)thiazol-2-yl)urea | ¹H NMR (400MHz, DMSO-*d*₆) *δ* 10.60 (br s, 1H), 7.33 (d, J = 8.8 Hz, 2H), 6.86 (d, J = 8.8 Hz, 2H), 6.80 (s, 1H), 6.23 (br s, 1H), 3.72 (s, 3H), 3.40 (s, 1H), 1.83 (s, 3H) | 302.0 | 8 | 0.58 |
| A174 | 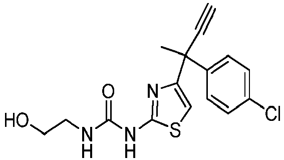 | 1-(4-(2-(4-chlorophenyl)but-3-yn-2-yl)thiazol-2-yl)-3-(2-hydroxyethyl)urea | ¹H NMR (400 MHz, DMSO-*d*₆) *δ* 10.64 (s, 1 H), 7.33 - 7.46 (m, 4 H), 6.90 (s, 1 H),6.34 (br s, 1 H), 4.75 (t, J = 5.14 Hz, 1 H), 3.50 (s, 1 H), 3.41 (q, J = 5.52 Hz, 2 H),3.16 (q, J = 5.52 Hz, 2 H), 1.85 (s, 3 H) | 350.0 | 34 | 0.31 |
| A175 | 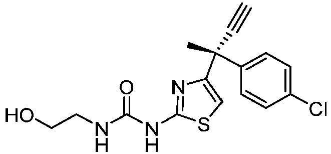 | (R)-1-(4-(2-(4-chlorophenyl)but-3-yn-2-yl)thiazol-2-yl)-3-(2-hydroxyethyl)urea | ¹HNMR (400 MHz, DMSO-*d*₆) *δ* 10.63 (br s, 1 H), 7.33 - 7.48 (m, 4 H), 6.90 (s, 1H), 6.35 (br s, 1 H), 4.75 (t, J = 5.27 Hz, 1 H), 3.50 (s, 1 H), 3.41 (q, J = 5.35 Hz, 2H), 3.16 (q, J = 5.52 Hz, 2 H), 1.85 (s, 3 H) | 350.0 | 32 | 1.31 |
| A176 | 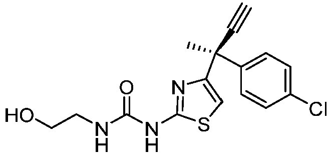 | (S)-1-(4-(2-(4-chlorophenyl)but-3-yn-2-yl)thiazol-2-yl)-3-(2-hydroxyethyl)urea | ¹HNMR (400 MHz, DMSO-*d*₆) *δ* 10.64 (br s, 1 H), 7.34 - 7.49 (m, 4 H), 6.90 (s, 1H), 6.35 (br s, 1 H), 4.76 (t, J = 5.14 Hz, 1 H), 3.51 (s, 1 H), 3.42 (q, J = 5.52 Hz, 2H), 3.16 (q, J = 5.35 Hz, 2 H), 1.85 (s, 3 H) | 350.0 | 5.4 | 0.28 |
| A177 | 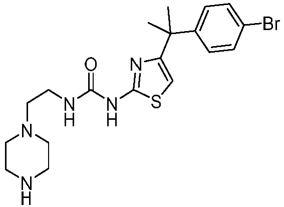 | 1-(4-(2-(4-bromophenyl)propan-2-yl)thiazol-2-yl)-3-(2-(piperazin-1-yl)ethyl)urea | | 452.0 | 249 | 1.66 |
| A178 | 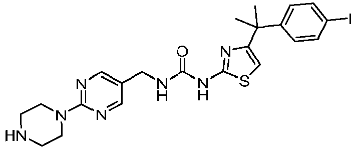 | 1-(4-(2-(4-iodophenyl)propan-2-yl)thiazol-2-yl)-3-((2-(piperazin-1-yl)pyrimidin-5-yl)methyl)urea | | 564.0 | 42 | 0.92 |
| A179 | 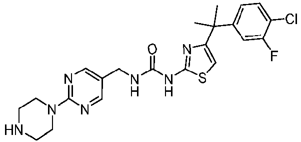 | 1-(4-(2-(4-chloro-3-fluorophenyl)propan-2-yl)thiazol-2-yl)-3-((2-(piperazin-1-yl)pyrimidin-5-yl)methyl)urea | | 490.0 | 95 | 1.36 |
| A180 | 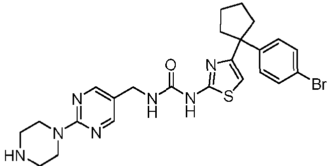 | 1-(4-(1-(4-bromophenyl)cyclopent yl)thiazol-2-yl)-3-((2-(piperazin-1-yl)pyrimidin-5-yl)methyl)urea | | 542.0 | 45 | |
| A181 | 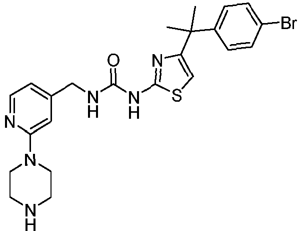 | 1-(4-(2-(4-bromophenyl)propan-2-yl)thiazol-2-yl)-3-((2-(piperazin-1-yl)pyridin-4-yl)methyl)urea | | 515.0 | 131 | 1.52 |
| A182 | 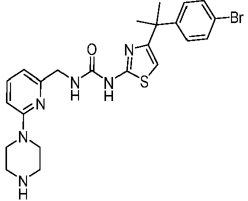 | 1-(4-(2-(4-bromophenyl)propan-2-yl)thiazol-2-yl)-3-((6-(piperazin-1-yl)pyridin-2-yl)methyl)urea | | 515.0 | 163 | 4.30 |
| A183 | 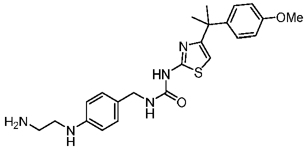 | 1-(4-((2-aminoethyl)amino)benz yl)-3-(4-(2-(4-methoxyphenyl)propan-2-yl)thiazol-2-yl)urea | | 440.0 | 52 | 2.31 |
| A184 | 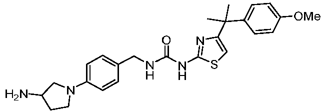 | 1-(4-(3-aminopyrrolidin-1-yl)benzyl)-3-(4-(2-(4-methoxyphenyl)propan-2-yl)thiazol-2-yl)urea | | 466.0 | 242 | |
| A185 | 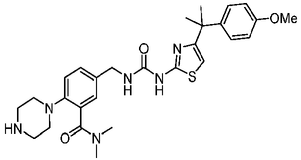 | 5-((3-(4-(2-(4-methoxyphenyl)propan-2-yl)thiazol-2-yl)ureido)methyl)-N,N-dimethyl-2-(piperazin-1-yl)benzamide | | 537.0 | 56 | |
| A186 | 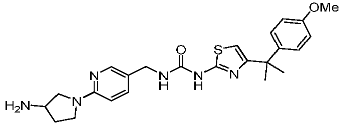 | 1-((6-(3-aminopyrrolidin-1-yl)pyridin-3-yl)methyl)-3-(4-(2-(4-methoxyphenyl)propan-2-yl)thiazol-2-yl)urea | | 467.0 | 19 | 1.55 |
| A187 | 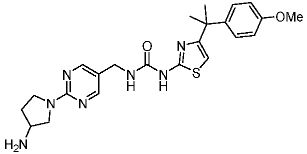 | 1-((2-(3-aminopyrrolidin-1-yl)pyrimidin-5-yl)methyl)-3-(4-(2-(4-methoxyphenyl)propan-2-yl)thiazol-2-yl)urea | | 468.0 | 7 | 0.7 |
| A188 | 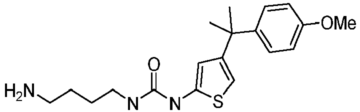 | 1-(4-aminobutyl)-3-(4-(2-(4-methoxyphenyl)propan-2-yl)thiophen-2-yl)urea | | 362.0 | 59 | |
| A189 | 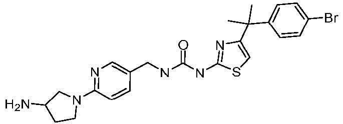 | 1-((6-(3-aminopyrrolidin-1-yl)pyridin-3-yl)methyl)-3-(4-(2-(4-bromophenyl)propan-2-yl)thiazol-2-yl)urea | | 515.0 | 41 | 2.31 |
| A190 | 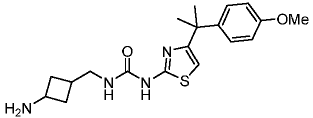 | 1-((3-aminocyclobutyl)methyl )-3-(4-(2-(4-methoxyphenyl)propan-2-yl)thiazol-2-yl)urea | | 375.0 | 458 | 3.02 |
| A191 | 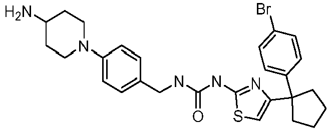 | 1-(4-(4-aminopiperidin-1-yl)benzyl)-3-(4-(1-(4-bromophenyl)cyclopent yl)thiazol-2-yl)urea | | 554.0 | 80 | 6.91 |
| A192 | 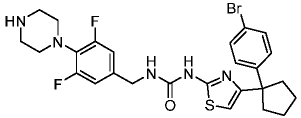 | 1-(4-(1-(4-bromophenyl)cyclopent yl)thiazol-2-yl)-3-(3,5-difluoro-4-(piperazin-1-yl)benzyl)urea | | 576.0 | 106 | 4.34 |
| A193 | 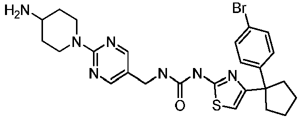 | 1-(4-(4-aminopiperidin-1-yl)benzyl)-3-(4-(1-(4-bromophenyl)cyclopent yl)thiazol-2-yl)urea | | 556.0 | 15 | 6.93 |
| A194 | 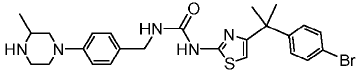 | 1-(4-(2-(4-bromophenyl)propan-2-yl)thiazol-2-yl)-3-(4-(3-methylpiperazin-1-yl)benzyl)urea | | 528.0 | 172 | 3.71 |
| A195 | 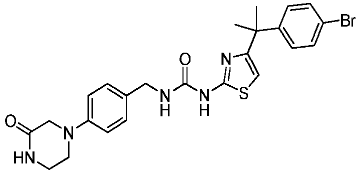 | 1-(4-(2-(4-bromophenyl)propan-2-yl)thiazol-2-yl)-3-(4-(3-oxopiperazin-1-yl)benzyl)urea | | 528.0 | 132 | 9.94 |
| A196 | 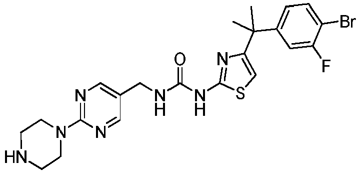 | 1-(4-(2-(4-bromo-3-fluorophenyl)propan-2-yl)thiazol-2-yl)-3-((2-(piperazin-1-yl)pyrimidin-5-yl)methyl)urea | | 534.0 | 76 | 3.34 |
| A197 | 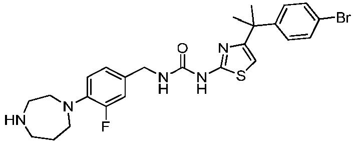 | 1-(4-(1,4-diazepan-1-yl)-3-fluorobenzyl)-3-(4-(2-(4-bromophenyl)propan-2-yl)thiazol-2-yl)urea | | 546.0 | 67 | |
| A198 | 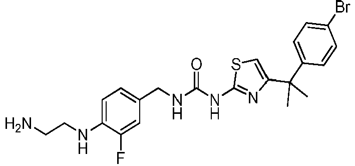 | 1-(4-((2-aminoethyl)amino)-3-fluorobenzyl)-3-(4-(2-(4-bromophenyl)propan-2-yl)thiazol-2-yl)urea | | 506.0 | 18 | 1.32 |
| A199 | 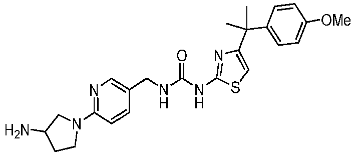 | 1-((6-(3-aminopyrrolidin-1-yl)pyridin-3-yl)methyl)-3-(4-(2-(4-methoxyphenyl)propan-2-yl)thiazol-2-yl)urea | | 467.0 | 34 | 0.46 |
| A200 | 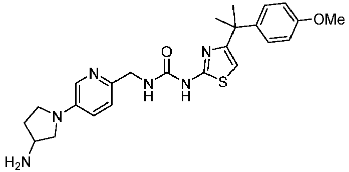 | 1-((5-(3-aminopyrrolidin-1-yl)pyridin-2-yl)methyl)-3-(4-(2-(4-methoxyphenyl)propan-2-yl)thiazol-2-yl)urea | | 467.0 | 43 | 0.52 |
| A201 | 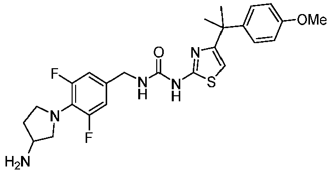 | 1-(4-(3-aminopyrrolidin-1-yl)-3,5-difluorobenzyl)-3-(4-(2-(4-methoxyphenyl)propan-2-yl)thiazol-2-yl)urea | | 502.0 | 25 | 0.53 |
| A202 | 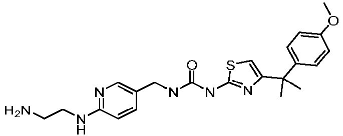 | 1-((6-((2-aminoethyl)amino)pyrid in-3-yl)methyl)-3-(4-(2-(4-methoxyphenyl)propan-2-yl)thiazol-2-yl)urea | | 441.0 | 34 | 5.71 |
| A203 | 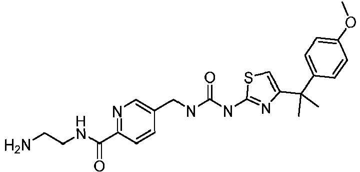 | N-(2-aminoethyl)-5-((3-(4-(2-(4-methoxyphenyl)propan-2-yl)thiazol-2-yl)ureido)methyl)picolin amide | | 469.0 | 94 | 5.70 |
| A204 | 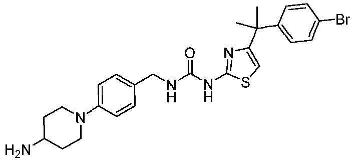 | 1-(4-(4-aminopiperidin-1-yl)benzyl)-3-(4-(2-(4-bromophenyl)propan-2-yl)thiazol-2-yl)urea | | 528.0 | 9 | 1.89 |
| A205 | 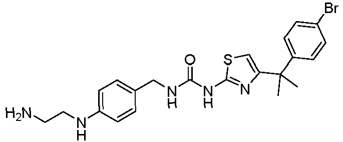 | 1-(4-((2-aminoethyl)amino)benz yl)-3-(4-(2-(4-bromophenyl)propan-2-yl)thiazol-2-yl)urea | | 488.0 | 59 | 1.48 |
| A206 | 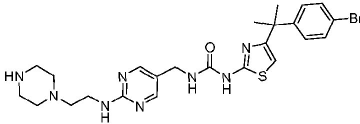 | 1-(4-(2-(4-bromophenyl)propan-2-yl)thiazol-2-yl)-3-((2-((2-(piperazin-1-yl)ethyl)amino)pyrimidi n-5-yl)methyl)urea | | 559.0 | 68 | |
| A207 | 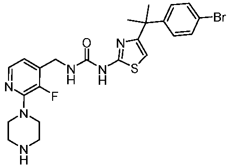 | 1-(4-(2-(4-bromophenyl)propan-2-yl)thiazol-2-yl)-3-((3-fluoro-2-(piperazin-1-yl)pyridin-4-yl)methyl)urea | | 533.0 | 99 | 2.18 |
| A208 | 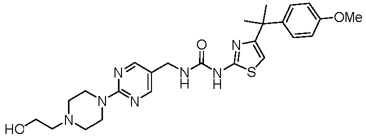 | 1-((2-(4-(2-hydroxyethyl)piperazin-1-yl)pyrimidin-5-yl)methyl)-3-(4-(2-(4-methoxyphenyl)propan-2-yl)thiazol-2-yl)urea | | 512.0 | 78 | 0.76 |
| A209 | 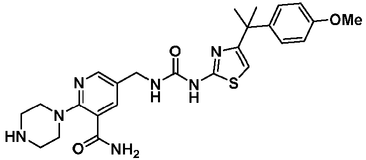 | 5-((3-(4-(2-(4-methoxyphenyl)propan-2-yl)thiazol-2-yl)ureido)methyl)-2-(piperazin-1-yl)nicotinamide | | 510.0 | 43 | |
| A210 | 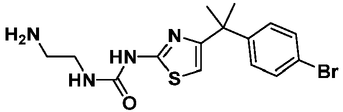 | 1-(2-aminoethyl)-3-(4-(2-(4-bromophenyl)propan-2-yl)thiazol-2-yl)urea | | 383.0 | 95 | 4.16 |
| A211 | 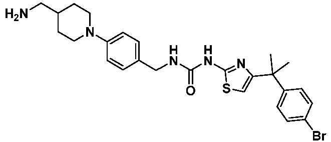 | 1-(4-(4-(aminomethyl)piperidin-1-yl)benzyl)-3-(4-(2-(4-bromophenyl)propan-2-yl)thiazol-2-yl)urea | | 542.0 | 94 | 2.59 |
| A212 | 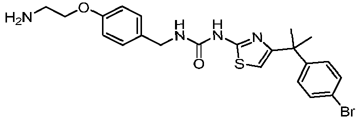 | 1-(4-(2-aminoethoxy)benzyl)-3-(4-(2-(4-bromophenyl)propan-2-yl)thiazol-2-yl)urea | | 489.0 | 289 | 2.75 |
| A213 | 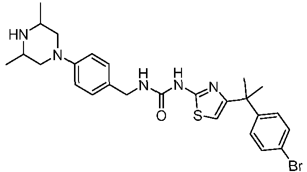 | 1-(4-(2-(4-bromophenyl)propan-2-yl)thiazol-2-yl)-3-(4-(3,5-dimethylpiperazin-1-yl)benzyl)urea | | 542.0 | 9 | 6.38 |
| A214 | 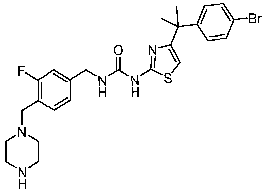 | 1-(4-(2-(4-bromophenyl)propan-2-yl)thiazol-2-yl)-3-(3-fluoro-4-(piperazin-1-ylmethyl)benzyl)urea | | 546.0 | 40 | 1.56 |
| A215 | 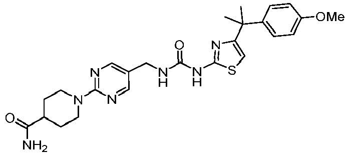 | 1-(5-((3-(4-(2-(4-methoxyphenyl)propan-2-yl)thiazol-2-yl)ureido)methyl)pyrimi din-2-yl)piperidine-4-carboxamide | | 510.0 | 150 | 2.21 |
| A216 | 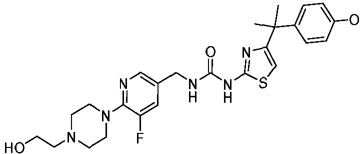 | . 1-((5-fluoro-6-(4-(2-hydroxyethyl)piperazin-1-yl)pyridin-3-yl)methyl)-3-(4-(2-(4-methoxyphenyl)propan-2-yl)thiazol-2-yl)urea | | 529.0 | 61 | 1.43 |
| A217 | 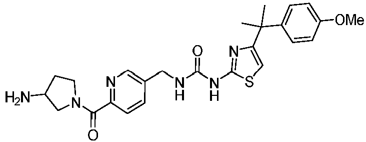 | 1-((6-(3-aminopyrrolidine-1-carbonyl)pyridin-3-yl)methyl)-3-(4-(2-(4-methoxyphenyl)propan-2-yl)thiazol-2-yl)urea | | 495.0 | 56 | 32.58 |
| A218 | 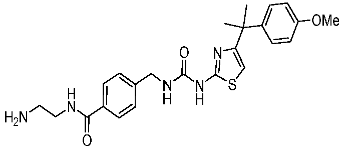 | N-(2-aminoethyl)-4-((3-(4-(2-(4-methoxyphenyl)propan-2-yl)thiazol-2-yl)ureido)methyl)benza mide | | 468.0 | 29 | 6.71 |
| A219 | 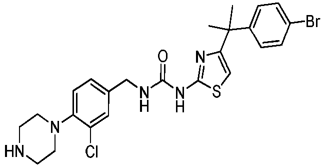 | 1-(4-(2-(4-bromophenyl)propan-2-yl)thiazol-2-yl)-3-(3-chloro-4-(piperazin-1-yl)benzyl)urea | ¹HNMR (400 MHz, DMSO-*d*₆) *δ* 10.76 (br s, 1 H), 9.25 (br s, 2 H), 7.43 (d, J=8.56 Hz, 2 H), 7.34 (d, J=1.96 Hz, 1H), 7.24 - 7.20 (m, 1 H), 7.15 (d, J=8.56 Hz, 4 H), 6.74 (s, 1 H), 4.25 (br d, J=5.62 Hz, 2 H), 3.27 - 3.08 (m, 8 H), 1.57 (s, 6 H). | 550.0 | 46 | 2.73 |
| A220 | 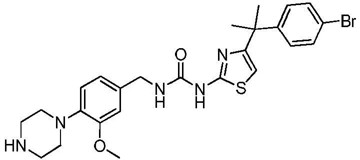 | 1-(4-(2-(4-bromophenyl)propan-2-yl)thiazol-2-yl)-3-(3-methoxy-4-(piperazin-1-yl)benzyl)urea | ¹HNMR (400 MHz, DMSO-*d*₆) *δ* 10.67 (br s, 1 H) 9.14 (br s, 2 H), 7.43 (d, J=8.56 Hz, 2 H), 7.15 (d, J=8.56 Hz, 2 H), 7.12 (br s, 1 H), 6.93 - 6.88 (m, 2 H), 6.80 (br d, J=8.07 Hz, 1 H), 6.74 (s, 1 H), 4.25 (br d, J=5.38 Hz, 2 H), 3.77 (s, 3 H), 3.18 (br d, J=14.18 Hz, 8 H), 1.57 (s, 6 H). | 544.2 | 44 | 1.32 |
| A221 | 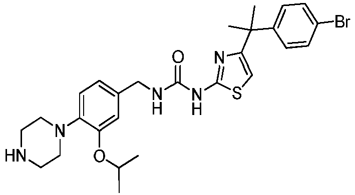 | 1-(4-(2-(4-bromophenyl)propan-2-yl)thiazol-2-yl)-3-(3-isopropoxy-4-(piperazin-1-yl)benzyl)urea | ¹HNMR (400 MHz, DMSO-*d*₆) *δ* 10.79 (br s, 1 H), 9.48 (br s, 2 H), 7.49 (br d, J=8.80 Hz, 1 H), 7.43 (d, J=8.56 Hz, 2 H), 7.15 (d, J=8.56 Hz, 2 H), 7.03 (br d, J=8.07 Hz, 1 H), 6.94 (s, 1 H), 6.81 (br d, J=7.83 Hz, 1 H), 6.77 (s, 1 H), 6.01 (br s, 8 H), 4.61 (dt, J=11.98, 5.99 Hz, 1 H), 4.24 (br d, J=4.40 Hz, 2 H), 1.58 (s, 6 H), 1.28 (d, J=6.11 Hz, 6 H). | 574.1 | 144 | 2.38 |
| A222 | 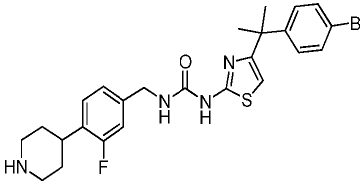 | 1-(4-(2-(4-bromophenyl)propan-2-yl)thiazol-2-yl)-3-(3-fluoro-4-(piperidin-4-yl)benzyl)urea | ¹H NMR (400 MHz, DMSO-*d*₆) *δ* 10.76 (br s, 1 H), 9.06 (br s, 1 H), 8.88 (br d, J=9.78 Hz, 1 H), 7.43 (d, J=8.56 Hz, 2 H), 7.29 - 7.19 (m, 2 H), 7.15 (d, J=8.56 Hz, 2 H), 7.10 - 7.04 (m, 2 H), 6.74 (s, 1 H), 4.28 (br d, J=5.62 Hz, 2 H),3.33 (br d, J=12.47 Hz, 2 H), 3.14 - 2.93 (m, 2 H), 1.93 - 1.81 (m, 3 H), 1.57 (s, 6 H), 1.23 (br s, 2 H). | 532.2 | 56 | 0.93 |
| A223 | 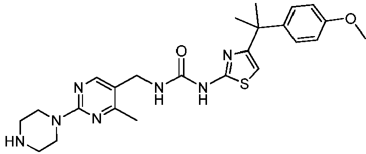 | 1-(4-(2-(4-methoxyphenyl)propan-2-yl)thiazol-2-yl)-3-((4-methyl-2-(piperazin-1-yl)pyrimidin-5-yl)methyl)urea | ¹HNMR (400 MHz, DMSO-*d*₆) *δ* 9.50 (br s, 2 H), 8.23 (s, 1 H), 7.76 - 7.57 (m, 1H), 7.17 - 7.06 (m, 2 H), 6.86 - 6.79 (m, 2 H), 6.72 (s, 1 H), 4.26 - 4.18 (m, 2 H), 4.0 - 3.92 (m, 4 H), 3.70 (s, 3 H), 3.13 ( s, 4 H), 2.40 (s, 3 H), 1.57 (s, 6 H). | 482.4 | 49 | 0.76 |
| A224 | 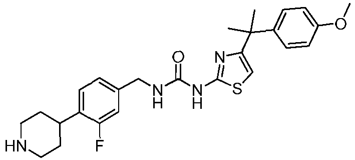 | 1-(3-fluoro-4-(piperidin-4-yl)benzyl)-3-(4-(2-(4-methoxyphenyl)propan-2-yl)thiazol-2-yl)urea | ¹H NMR (400 MHz, DMSO-*d*₆) *δ* 10.73 (br s, 1 H), 8.93 (br s, 1 H), 8.72 (br s, 1 H), 7.26 - 7.20 (m, 1 H), 7.16 - 7.02 (m, 5 H), 6.81 (d, J=8.80 Hz, 2 H), 6.66 (s, 1 H), 4.29 (br d, J=5.87 Hz, 2 H), 3.70 (s, 3 H), 3.34 (br d, J=12.23 Hz, 2 H), 3.15 - 3.07 (m, 1 H), 3.07 - 2.94 (m, 2 H), 1.87 (br s, 4 H), 1.57 (s, 6 H). | 483.2 | 21 | 0.73 |
| A225 | 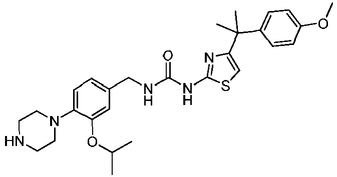 | 1-(3-isopropoxy-4-(piperazin-1-yl)benzyl)-3-(4-(2-(4-methoxyphenyl)propan-2-yl)thiazol-2-yl)urea | ¹HNMR (400 MHz, CD3OD) *δ* 7.23 - 7.16 (m, 3 H), 7.11 (s, 1 H), 7.07 (s, 1 H), 6.97 - 6.87 (m, 3 H), 4.75 (dt, J=11.49, 5.75 Hz, 1 H), 4.40 (s, 2 H), 3.76 (s, 3 H), 3.51 (s, 8 H), 1.69 (s, 6 H), 1.38 (d, J=5.87 Hz, 6 H). | 524.3 | 108 | 1.05 |
| A226 | 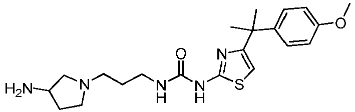 | 1-(3-(3-aminopyrrolidin-1-yl)propyl)-3-(4-(2-(4-methoxyphenyl)propan-2-yl)thiazol-2-yl)urea | ¹HNMR (400 MHz, DMSO-*d*₆) *δ* 11.30 (s, 1H) 10.94 (s, 1H) 8.64 (s, 1H) 8.55 (s, 1H) 7.09 (d, J=8.82 Hz, 2H) 6.95 (s, 1H) 6.78 (d, J=8.82 Hz, 2H) 6.64 (s, 1H) 4.00 (d, J=13.89Hz, 1H) 3.67 (s, 3H) 3.37-3.49 (m, 1H) 3.35 (s,2H) 3.01-3.23 (m, 5H) 2.06-2.28 (m, 1H) 1.92-2.06 (m, 1H) 1.75-1.86 (m, 2H) 1.54 (s, 6H) | 418.3 | 100 | 4.82 |
| A227 | 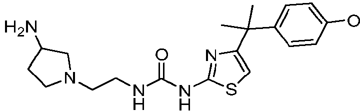 | 1-(2-(3-aminopyrrolidin-1-yl)ethyl)-3-(4-(2-(4-methoxyphenyl)propan-2-yl)thiazol-2-yl)urea | ¹H NMR (400 MHz, D2O) *δ* 7.20 - 7.13 (m, 2H), 6.95 (s, 1H), 6.89 - 6.83 (m, 2H), 4.20 - 3.74 (m, 3H), 3.71 (s, 3H), 3.68 - 3.47 (m, 4H), 3.46 - 3.32 (m, 2H), 2.65 - 2.48 (m, 1H), 2.24 - 2.07 (m, 1H), 1.56 (s, 6H). | 404.3 | 138 | 3.12 |
| A228 | 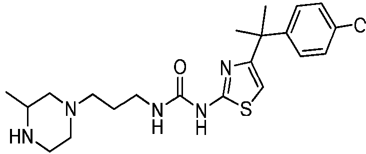 | 1-(4-(2-(4-methoxyphenyl)propan-2-yl)thiazol-2-yl)-3-(3-(3-methylpiperazin-1-yl)propyl)urea | ¹H NMR (400MHz, DMSO-*d*₆) *δ* 11.79 (br s, 1H), 10.68 (br s, 1H), 9.97 (br s, 2H), 7.12 (d, *J* = 8.6 Hz, 2H), 6.88 (s, 1H), 6.81 (d, *J* = 8.8 Hz, 2H), 6.67 (s, 1H), 3.75 - 3.62 (m, 6H), 3.58 - 3.49 (m, 1H), 3.48 - 3.36 (m, 1H), 3.29 - 3.16 (m, 3H), 3.15 - 2.99 (m, 3H), 1.96 - 1.82 (m, 2H), 1.57 (s, 6H), 1.30 (d, *J* = 6.4 Hz, 2H). | 432.3 | 100 | 3.86 |
| A229 | 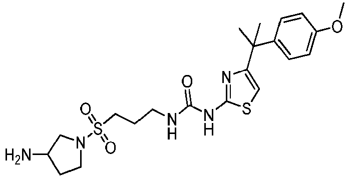 | 1-(3-((3-aminopyrrolidin-1-yl)sulfonyl)propyl)-3-(4-(2-(4-methoxyphenyl)propan-2-yl)thiazol-2-yl)urea | ¹HNMR (400 MHz, DMSO-*d*₆) *δ* 8.47 (br s, 3 H), 7.24 (br s, 1 H), 7.12 (d, J=8.82 Hz, 2 H), 6.82 (d, J=8.60 Hz, 2 H), 6.70 (s, 1 H), 3.83 (br d, J=5.07 Hz, 1 H), 3.70 (s, 3 H), 3.58 (br dd, J=10.80, 6.62 Hz, 1 H), 3.52 - 3.43 (m, 1 H), 3.41 - 3.28 (m, 3 H), 3.26 - 3.13 (m, 4 H), 2.22 (dq, J=13.70, 6.90 Hz, 1H), 2.05 - 1.92 (m, 1H), 1.84 (dt, J = 14.55, 7.06 Hz, 2 H), 1.57 (s, 6 H). | 482.2 | 169 | 2.27 |
| A230 | 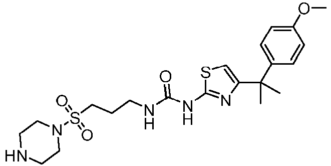 | 1-(4-(2-(4-methoxyphenyl)propan-2-yl)thiazol-2-yl)-3-(3-(piperazin-1-ylsulfonyl)propyl)urea | ¹HNMR (400 MHz, DMSO-*d*₆) *δ* 7.10 - 7.07 (m, 2 H), 6.78 - 6.75 (m, 2 H), 6.59 (s, 1 H), 3.67 (s, 3 H), 3.30 - 3.15 (m, 2 H), 3.02 - 2.95 (m, 6 H), 2.68 - 2.66 (m, 4 H), 1.79 - 1.72 (m, 2 H), 1.52 (s, 6 H). | 482.2 | 127 | 3.01 |
| A231 | 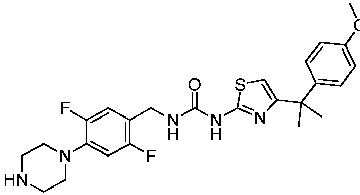 | 1-(2,5-difluoro-4-(piperazin-1-yl)benzyl)-3-(4-(2-(4-methoxyphenyl)propan-2-yl)thiazol-2-yl)urea hydrochloride | ¹HNMR (400 MHz, DMSO-*d*₆) *δ* 10.75 (br s, 1 H), 9.33 (br s, 2 H), 7.39 (br s, 1 H), 7.16 - 7.10 (m, 3 H), 6.98 (dd, J=11.36, 7.39 Hz, 1 H), 6.80 (d, J=8.82 Hz, 2 H), 6.68 (s, 1 H), 4.27 (br d, J=5.51 Hz, 2 H), 3.70 (s, 3 H), 3.23 (br s, 8 H), 1.57 (s, 6 H), | 502.2 | 27 | 3.36 |
| A232 | 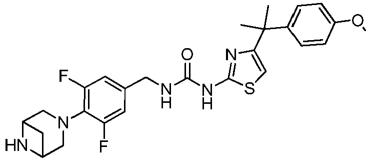 | 1-(4-(3,6-diazabicyclo[3.1.1]hepta n-3-yl)-3,5-difluorobenzyl)-3-(4-(2-(4-methoxyphenyl)propan-2-yl)thiazol-2-yl)urea | ¹H NMR (400MHz, DMSO-*d*₆) *δ* 10.85 - 10.72 (m, 1H), 9.80 - 9.68 (m, 1H), 8.16 - 8.08 (m, 1H), 7.12 (br d, *J* = 8.8 Hz, 2H), 7.05 - 6.93 (m, 2H), 6.86 - 6.76 (m, 2H), 6.71 - 6.64 (m, 1H), 4.27 (br d, *J* = 6.1 Hz, 4H), 3.88 (s, 2H), 3.70 (s, 3H), 3.54 - 3.49 (m, 2H), 2.86 - 2.76 (m, 1H), 2.14 - 2.06 (m, 1H), 1.57 (s, 6H). | 514.3 | 88 | 0.59 |
| A233 | 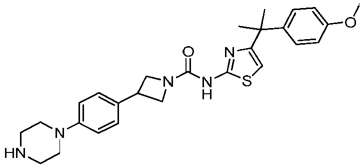 | N-(4-(2-(4-methoxyphenyl)propan-2-yl)thiazol-2-yl)-3-(4-(piperazin-1-yl)phenyl)azetidine-1-carboxamide | ¹H NMR (400MHz, DMSO-*d*₆) *δ* 9.25 (br s, 2H), 7.33 (s, 0.4H), 7.25 - 7.23 (m, 2H), 7.20 - 7.18 (m, 2H), 7.14 - 7.12 (m, 0.4 H), 6.98 - 6.96 (m, 2H),6.82 - 6.80 (m, 2H), 6.66 (s, 1H), 4.38 - 4.36 (m, 2H), 3.94 - 3.91 (m, 2H), 3.76 - 3.72 (m, 1H), 3.72 (s, 3H), 3.39 - 3.38 (m, 4H), 3.37 - 3.20 (m, 4H), 1.60 (s, 6H) . | 492.2 | 43 | 0.33 |
| A234 | 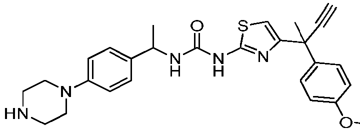 | 1-(4-(2-(4-methoxyphenyl)but-3-yn-2-yl)thiazol-2-yl)-3-(1-(4-(piperazin-1-yl)phenyl)ethyl)urea | 1H NMR (400 MHz, DMSO-*d*₆) *δ* 10.50 (br s, 1 H), 9.22 (br s, 2 H), 7.31 (d, J = 8.8 Hz, 2 H), 7.19 (d, J = 8.4 Hz, 3 H), 6.95 (d, J = 8.4 Hz, 2 H), 6.85 (dd, J=8.4, 1.2 Hz, 2 H), 6.81-6.79 (m, 1 H), 4.76 - 4.73 (m, 1 H), 3.71 (s, 3 H), 3.39 (s, 1 H), 3.35 - 3.32(m, 4 H) 3.24 - 3.16 (m, 4 H), 1.82 (d, J = 2.4 Hz, 3 H), 1.33 (d, J=6.8 Hz, 3 H) | [M+Na]⁺=51 2.3 | 36 | 2.67 |
| A235 | 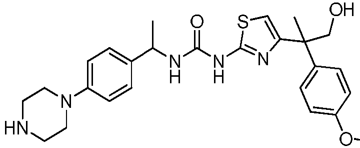 | 1-(4-(1-hydroxy-2-(4-methoxyphenyl)propan-2-yl)thiazol-2-yl)-3-(1-(4-(piperazin-1-yl)phenyl)ethyl)urea | 1H NMR (400 MHz, DMSO-*d*₆) *δ* 10.47 (br s, 1 H), 9.11 (br s, 2 H), 7.36 - 7.23 (m, 1 H), 7.19 (d, J = 8.8 Hz, 2 H), 7.10 (d, J = 8.8 Hz, 2 H), 6.95 (d, J = 8.8 Hz, 2 H), 6.75 (d, J = 8.0 Hz, 2 H), 6.69 (s, 1 H), 4.77 - 4.73 (m, 1 H), 3.80 - 3.76 (m, 1 H), 3.70 (s, 3 H) 3.34 - 3.31 (m, 4 H), 3.24 - 3.16 (m, 4 H), 2.07 (s, 1 H), 1.55 (s, 3 H), 1.33 (d, J=6.8 Hz, 3 H) | 496.2 | 61 | 27.01 |
| A236 | 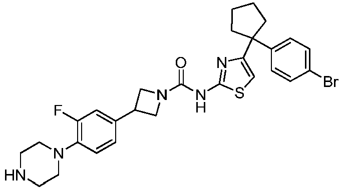 | N-(4-(1-(4-bromophenyl)cyclopent yl)thiazol-2-yl)-3-(3-fluoro-4-(piperazin-1-yl)phenyl)azetidine-1-carboxamide | ¹H NMR (400 MHz, CD3OD) *δ* 7.34 (d, J=8.56 Hz, 2 H) 7.21 (d, J=8.56 Hz, 2 H) 7.08 - 7.00 (m, 3 H) 6.68 (s, 1H) 4.59 (br d, J=16.63 Hz, 1 H) 4.40 (t, J=8.56 Hz, 2 H) 3.98 (dd, J=8.31, 6.11 Hz, 2 H) 3.77 - 3.87 (m, 1 H) 3.02 (br d, J=6.11Hz, 8 H) 2.43 - 2.53 (m, 2 H) 2.01 - 2.13 (m, 2 H) 1.62 - 1.77 (m, 4 H) | 584.3 | 13 | 4.50 |
| A237 | 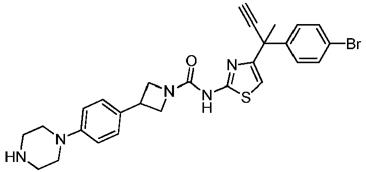 | N-(4-(2-(4-bromophenyl)but-3-yn-2-yl)thiazol-2-yl)-3-(4-(piperazin-1-yl)phenyl)azetidine-1-carboxamide | ¹H NMR (400 MHz, DMSO-*d*₆) *δ* 10.93 (s, 1 H) 9.22 (s, 2 H) 7.47 (d, J = 8.4 Hz, 2 H) 7.30 (d, J = 8.4 Hz, 2 H) 7.21 (d, J = 8.4Hz, 2 H), 6.90 - 6.98 (m, 3 H), 4.30 (br s, 1 H), 3.87 - 3.84 (m, 2 H), 3.66 - 3.74 (m, 1 H), 3.46 (s, 1 H), 3.31 - 3.30 (m, 4 H),3.22 - 3.10 (m, 4 H), 1.82 (s, 3 H) | 550.1 | 7 | 0.61 |
| A238 | 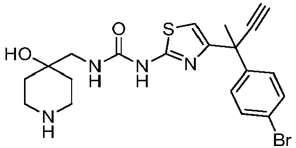 | 1-(4-(2-(4-bromophenyl)but-3-yn-2-yl)thiazol-2-yl)-3-((4-hydroxypiperidin-4-yl)methyl)urea | ¹H NMR (400 MHz, CD3OD) *δ* 7.53 -7.20 (m, 2 H), 7.44 - 7.42 (m, 2 H), 7.14 (s, 1 H), 3.33 - 3.27 (m, 2 H), 3.26 - 3.25 (m, 4 H), 3.16 (s, 1 H), 1.95 (s, 3 H), 1.79 -1.77 (m, 4 H) | 465.1 | 19 | 27.00 |
| A239 | 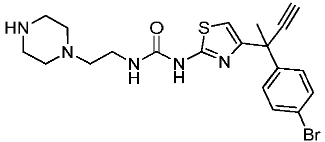 | 1-(4-(2-(4-bromophenyl)but-3-yn-2-yl)thiazol-2-yl)-3-(2-(piperazin-1-yl)ethyl)urea | ¹H NMR (400MHz, CD3OD) *δ* 7.47-7.54 (m, 2H), 7.38-7.46 (m, 2H), 7.14 (s, 1H), 3.62-3.82 (m, 8H), 3.47 (br t, J=5.6 Hz, 2H), 3.14 (s, 1H), 1.95 (s, 3H) | 462.0 | 20 | 0.85 |
| A240 | 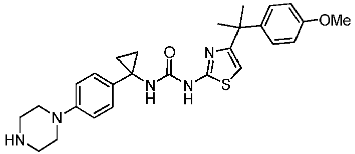 | 1-(4-(2-(4-methoxyphenyl)propan-2-yl)thiazol-2-yl)-3-(1-(4-(piperazin-1-yl)phenyl)cyclopropyl)u rea | | 492.0 | 97 | 1.88 |
| A241 | 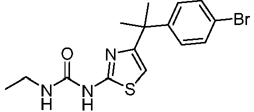 | 1-(4-(2-(4-bromophenyl)propan-2-yl)thiazol-2-yl)-3-ethylurea | | 368.1 | 98 | 3.41 |
| A242 | 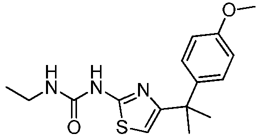 | 1-ethyl-3-(4-(2-(4-methoxyphenyl)propan-2-yl)thiazol-2-yl)urea | | 320.1 | 42 | 0.98 |
| A243 | 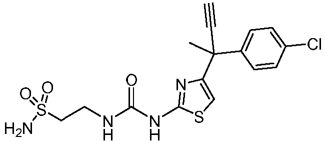 | 2-(3-(4-(2-(4-chlorophenyl)but-3-yn-2-yl)thiazol-2-yl)ureido)ethane-1-sulfonamide | ¹HNMR (400 MHz, CDCl₃) *δ* 7.44-7.26 (m, 4 H), 6.75 ( s, 2 H), 5.82 (s, 2 H), 3.80 - 3.65 (m, 2 H), 3.25 - 3.13 (m, 2 H), 2.63 (s, 1 H), 1.92 (s, 3 H). | 413.1 | 23 | 0.59 |
| A244 | 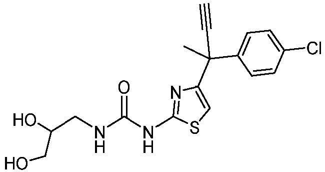 | 1-(4-(2-(4-chlorophenyl)but-3-yn-2-yl)thiazol-2-yl)-3-(2,3-dihydroxypropyl)urea | ¹H NMR (400 MHz, CDCl₃) *δ* 9.63 (br. s, 1 H), 7.39 - 7.35 (m, 2 H), 7.25 - 7.19 (m, 2 H), 6.65 ( s, 2 H), 3.72 - 3.62 (m, 1 H), 3.52 - 3.33 (m, 2 H), 3.29 - 3.09 (m, 2 H), 2.56 (s, 1 H), 1.86 (s, 3 H). | 380.1 | 72 | 1.03 |
| A245 | 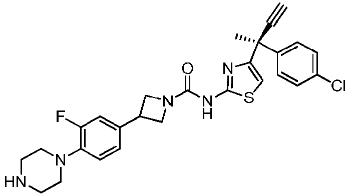 | S)-N-(4-(2-(4-chlorophenyl)but-3-yn-2-yl)thiazol-2-yl)-3-(3-fluoro-4-(piperazin-1-yl)phenyl)azetidine-1-carboxamide | ¹H NMR (400 MHz, DMSO-*d*₆) *δ* 10.95 (s, 1 H), 8.97 (br s, 2 H), 7.35 - 7.42 (m, 4 H), 7.20 - 7.30 (m, 1 H), 7.11 - 7.18 (m, 1 H), 7.04 - 7.09 (m, 1 H), 6.97 (s, 1 H), 4.33 (br t, *J* = 8.11 Hz, 2 H), 3.91 (br t, *J* = 7.03 Hz, 2 H), 3.75 - 3.81 (m, 1 H), 3.48 (s, 1 H), 3.21 (br d, *J* = 8.94 Hz, 8 H), 1.85 (s, 3 H) | 524.1 | 16 | 0.68 |
| A246 | 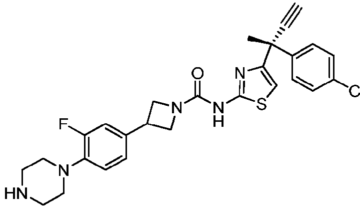 | (R)-N-(4-(2-(4-chlorophenyl)but-3-yn-2-yl)thiazol-2-yl)-3-(3-fluoro-4-(piperazin-1-yl)phenyl)azetidine-1-carboxamide | ¹HNMR (400 MHz, DMSO-*d*₆) *δ* 10.95 (s, 1 H), 8.95 (br s, 2 H), 7.35 - 7.42 (m, 4 H), 7.18 - 7.30 (m, 1 H), 7.10 - 7.17 (m, 1 H), 7.02 - 7.09 (m, 1 H), 6.97 (s, 1 H), 4.33 (br t, *J* = 8.17 Hz, 2 H), 3.86 - 3.97 (m, 2 H), 3.75 - 3.81 (m, 1 H), 3.48 (s, 1 H), 3.23 (br s, 4 H), 3.20 (br s, 4 H), 1.86 (s, 3H) | 524.1 | 29 | 1.59 |
| A247 | 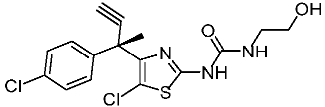 | (S)-1-(5-chloro-4-(2-(4-chlorophenyl)but-3-yn-2-yl)thiazol-2-yl)-3-(2-hydroxyethyl)urea | ¹HNMR (400 MHz, DMSO-*d*₆) *δ* 10.92 (1, 1H), 7.38 - 7.36 (m, 4H), 6.49 (s, 1H), 4.80 (s, 1H), 3.58 (s, 1H), 3.45 -3.35 (m, 2H), 3.22 -3.18 (m, 2H), 1.85 (s, 3H) | 429.7 | 408 | 1.85 |
| A248 | 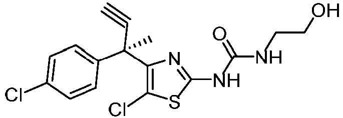 | (R)-1-(5-chloro-4-(2-(4-chlorophenyl)but-3-yn-2-yl)thiazol-2-yl)-3-(2-hydroxyethyl)urea | ¹HNMR (400 MHz, DMSO-*d*₆) *δ* 10.92 (1, 1H), 7.40 - 7.30 (m, 4H), 6.50 (s, 1H), 4.77 (s, 1H), 3.55 (s, 1H), 3.43 -3.35 (m, 2H), 3.20 -3.15 (m, 2H), 1.87 (s, 3H) | 429.9 | 1294 | 2.73 |
| A249 | 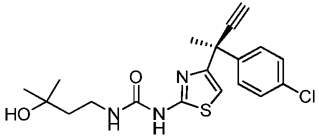 | (S)-1-(4-(2-(4-chlorophenyl)but-3-yn-2-yl)thiazol-2-yl)-3-(3-hydroxy-3-methylbutyl)urea | ¹HNMR (400 MHz, DMSO-*d*₆) *δ* 10.66 (s, 1H), 7.40-7.46 (m, 2H), 7.33-7.39 (m, 2H), 6.88 (s, 1H), 6.27 (s, 1H), 4.32 (s, 1H), 3.48 (s, 1H), 3.13-3.23 (m, 2H), 1.85 (s, 3H), 1.45-1.57 (m, 2H), 1.09 (s, 6H) | 392.0 | 128 | 0.67 |
| A250 | 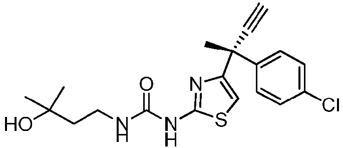 | (R)-1-(4-(2-(4-chlorophenyl)but-3-yn-2-yl)thiazol-2-yl)-3-(3-hydroxy-3-methylbutyl)urea | ¹H NMR (400 MHz, DMSO-*d*₆) *δ* 10.65 (s, 1H), 7.41-7.46 (m, 2H), 7.34-7.40 (m, 2H), 6.88 (s, 1H), 6.26 (s, 1H), 4.31 (s, 1H), 3.49 (s, 1H), 3.13-3.22 (m, 2H), 1.85 (s, 3H), 1.46-1.56 (m, 2H), 1.09 (s, 6H) | 392.0 | 417 | 1.18 |
| A251 | 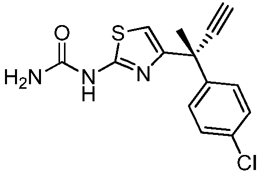 | (R)-1-(4-(2-(4-chlorophenyl)but-3-yn-2-yl)thiazol-2-yl)urea | ¹HNMR (400 MHz, DMSO-*d*₆) *δ* 10.59 (1, 1H), 7.44 - 7.42 (m, 2H), 7.38 - 7.36 (m, 2H), 6.89 (s, 1H), 6.22 (s, 1H), 3.49 (s, 1H), 1.85 (s, 3H) | 305.9 | 81 | 1.67 |
| A252 | 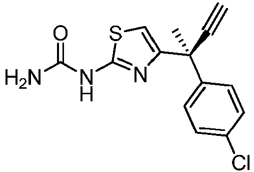 | (S)-1-(4-(2-(4-chlorophenyl)but-3-yn-2-yl)thiazol-2-yl)urea | ¹H NMR (400 MHz, DMSO-*d*₆) *δ* 10.60 (1, 1H), 7.44 - 7.42 (m, 2H), 7.39 - 7.36 (m, 2H), 6.90 (s, 1H), 6.22 (s, 1H), 3.49 (s, 1H), 1.85 (s, 3H) | 305.9 | 30 | 0.53 |
| A253 | 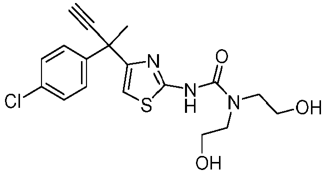 | 3-(4-(2-(4-chlorophenyl)but-3-yn-2-yl)thiazol-2-yl)-1,1-bis(2-hydroxyethyl)urea | ¹HNMR (400 MHz, DMSO-*d*₆)δ 7.36 (4H, J = 19.6 Hz, t), 6.91 (1H, s), 3.51 (4H, s), 3.45 (1H, s), 3.40 (4H, s), 1.83 (3H, s) | 394.0 | 172 | 3.05 |
| A254 | 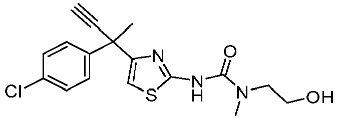 | 3-(4-(2-(4-chlorophenyl)but-3-yn-2-yl)thiazol-2-yl)-1-(2-hydroxyethyl)-1-methylurea | ¹H NMR (400 MHz, DMSO-*d*₆) *δ* 7.38-7.33 (4H, m), 6.90 (1H, s), 3.47 (2H, J=10.4 Hz, t), 3.44 (1H, s), 3.37 (2H, J=10.4 Hz, t), 2.91 (3H, s), 1.82 (3H, s) | 364.0 | 548 | 2.07 |
| A255 | 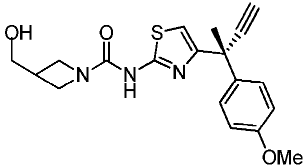 | (R)-3-(hydroxymethyl)-N-(4-(2-(4-methoxyphenyl)but-3-yn-2-yl)thiazol-2-yl)azetidine-1-carboxamide | ¹H NMR (400 MHz, DMSO-*d*₆) *δ* 10.83 (s, 1 H), 7.29 (d, *J* = 8.78 Hz, 2 H), 6.87 (s, 1 H), 6.85 (s, 2 H), 4.78 (t, *J=* 5.27 Hz, 1 H), 3.94 (br s, 2 H), 3.71 (s, 3 H), 3.68 (br s, 2 H), 3.48 (t, *J =* 5.65 Hz, 2 H), 3.38 (s, 1 H), 2.56 - 2.70 (m, 1 H), 1.83 (s, 3 H). | 372.0 | 688 | |
| A256 | 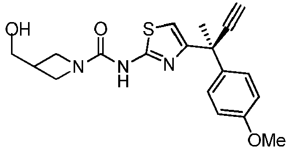 | (S)-3-(hydroxymethyl)-N-(4-(2-(4-methoxyphenyl)but-3-yn-2-yl)thiazol-2-yl)azetidine-1-carboxamide | ¹H NMR (400 MHz, DMSO-*d*₆) *δ* 10.85 (s, 1 H), 7.30 (br d, *J* = 8.78 Hz, 2 H), 6.87 (s, 1 H), 6.86 (s, 2 H), 4.79 (t, *J* = 5.14 Hz, 1 H), 3.86 - 4.07 (m, 2 H), 3.72 (s, 3 H), 3.69 (br s, 2 H), 3.49 (br t, *J =* 5.52 Hz, 2 H), 3.39 (s, 1 H), 2.56 - 2.72 (m, 1 H), 1.84 (s, 3 H). | 372.1 | 273 | 14.3 |
| A257 | 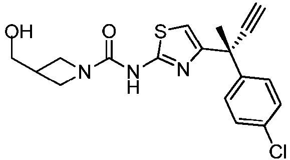 | (R)-N-(4-(2-(4-chlorophenyl)but-3-yn-2-yl)thiazol-2-yl)-3-(hydroxymethyl)azetidin e-1-carboxamide | ¹H NMR (400 MHz, CDCl3) *δ* 7.79 (s, 1 H), 7.45-7.40 (m, 2 H), 7.30-7.20 (m, 2 H), 6.78 (s, 1 H), 4.20 - 4.08 (m, 2 H), 3.90 - 3.84 (m, 2 H), 3.80 - 3.75 (m, 2 H), 2.88 -2.75 (m, 1 H), 2.57 (s, 1 H), 2.10 - 2.00 (m, 1 H), 1.92 (s, 3 H). | 376.1 | 1554 | 2.01 |
| A258 | 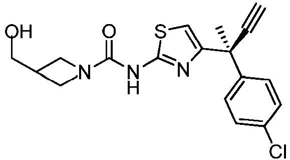 | (S)-N-(4-(2-(4-chlorophenyl)but-3-yn-2-yl)thiazol-2-yl)-3-(hydroxymethyl)azetidin e-1-carboxamide | ¹H NMR (400 MHz, CDCl₃) *δ* 7.78 (s, 1 H), 7.45 - 7.36 (m, 2 H), 7.30 - 7.20 (m, 2 H), 6.78 (s, 1 H), 4.20 - 4.08 (m, 2 H), 3.90 - 3.84 (m, 2 H), 3.80 - 3.75 (m, 2 H), 2.90 -2.80 (m, 1 H), 2.57 (s, 1 H), 2.05 - 1.95 (m, 1 H), 1.93 (s, 3 H). | 376.1 | 357 | 1.33 |
| A259 | 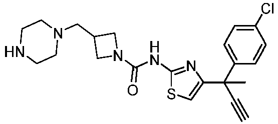 | N-(4-(2-(4-chlorophenyl)but-3-yn-2-yl)thiazol-2-yl)-3-(piperazin-1-ylmethyl)azetidine-1-carboxamide | | 444.2 | 48 | 0.44 |
| A260 | 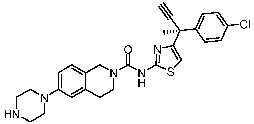 | (S)-N-(4-(2-(4-chlorophenyl)but-3-yn-2-yl)thiazol-2-yl)-6-(piperazin-1-yl)-3,4-dihydroisoquinoline-2(1H)-carboxamide | ¹HNMR (400 MHz, DMSO-*d*₆) δ 8.23 (s, 1H), 7.41 -7.35 (m, 4H), 6.99 - 6.91 (m, 2H), 6.75 (d, *J* = 7.7 Hz, 1H), 6.67 (s, 1H), 4.47 (s, 2H), 3.63 (s, 2H), 3.05(s, 4H), 2.90 (s, 4H), 2.70 (s, 2H), 1.81 (s, 3H). | 506.2 | 74 | 1.08 |
| A261 | 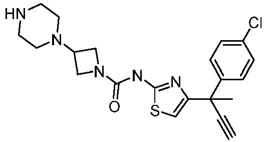 | N-(4-(2-(4-chlorophenyl)but-3-yn-2-yl)thiazol-2-yl)-3-(piperazin-1-yl)azetidine-1-carboxamide | | 430.1 | 83 | 0.51 |
| A262 | 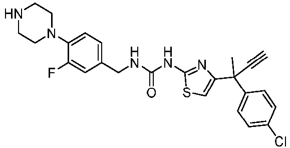 | 1-(4-(2-(4-chlorophenyl)but-3-yn-2-yl)thiazol-2-yl)-3-(3-fluoro-4-(piperazin-1-yl)benzyl)urea | | 498.1 | 13 | 0.67 |
| A263 | 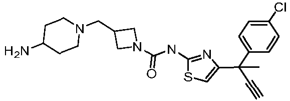 | 3-((4-aminopiperidin-1-yl)methyl)-N-(4-(2-(4-chlorophenyl)but-3-yn-2-yl)thiazol-2-yl)azetidine-1-carboxamide | | 458.1 | 39 | 1.44 |
| A264 | 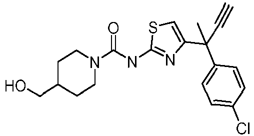 | N-(4-(2-(4-chlorophenyl)but-3-yn-2-yl)thiazol-2-yl)-4-(hydroxymethyl)piperidi ne-1-carboxamide | | 404.1 | 1374 | 2.81 |
| A265 | 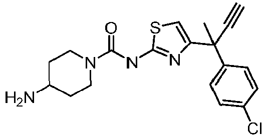 | 4-amino-N-(4-(2-(4-chlorophenyl)but-3-yn-2-yl)thiazol-2-yl)piperidine-1-carboxamide | | 389.1 | 4974 | 2.90 |
| A266 | 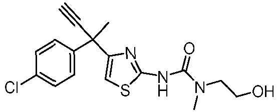 | 3-(4-(2-(4-chlorophenyl)but-3-yn-2-yl)thiazol-2-yl)-1-(2-hydroxyethyl)-1-methylurea | ¹H NMR (400 MHz, DMSO-*d*₆) δ 7.34 (t, *J* = 20.0 Hz, 4H), 6.87 (s, 1H), 3.44-3.40 (m, *J* = 16.0, 5H), 2.88 (s, 3H), 1.79 (s, 3H). | 364.0 | 546 | 2.07 |
| A267 | 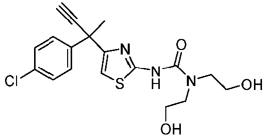 | 3-(4-(2-(4-chlorophenyl)but-3-yn-2-yl)thiazol-2-yl)-1,1-bis(2-hydroxyethyl)urea | ¹H NMR (400 MHz, DMSO-*d*₆) *δ* 7.34 (t, *J =* 20.0 Hz, 4H), 6.86 (s, 1H), 3.46-3.40 (m, *J =* 16.0, 9H), 1.78 (s, 3H). | 394.0 | 171 | 3.05 |
| A268 | 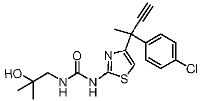 | 1-(4-(2-(4-chlorophenyl)but-3-yn-2-yl)thiazol-2-yl)-3-(2-hydroxy-2-methylpropyl)urea | ¹H NMR (400 MHz, DMSO-*d*₆) *δ* 7.43-7.34 (dd, J = 27.8, 7.5 Hz, 4H), 6.87 (s, 1H), 6.38 (s, 1H), 4.56 (s, 1H), 3.02 (d, *J =* 4.0 Hz, 2H), 1.83 (s, 3H), 1.03 (s, 6H). | 378.1 | 218 | 1.31 |
| A269 | 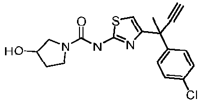 | N-(4-(2-(4-chlorophenyl)but-3-yn-2-yl)thiazol-2-yl)-3-hydroxypyrrolidine-1-carboxamide | ¹H NMR (400 MHz, DMSO-*d*₆) δ 7.36 (s, 4H), 6.90 (s, 1H), 4.93 (s, 1H), 4.22 (s, 1H), 3.44 (s, 4H), 1.83 (s, 3H), 1.74 (s, 2H). | 376.0 | 881 | 1.26 |
| A270 | 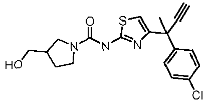 | N-(4-(2-(4-chlorophenyl)but-3-yn-2-yl)thiazol-2-yl)-3-(hydroxymethyl)pyrroli dine-1-carboxamide | ¹H NMR (400 MHz, DMSO-*d*₆) δ 7.36 (s, 4H), 6.90 (s, 1H), 4.68 (s, 1H), 3.44 (s, 4H), 3.11 (s, 2H), 2.24 (s, 1H), 1.83 (s, 3H), 1.60 (s, 2H). | 390.1 | 904 | 1.97 |
| A271 | 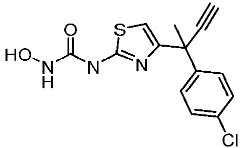 | 1-(4-(2-(4-chlorophenyl)but-3-yn-2-yl)thiazol-2-yl)-3-hydroxyurea | ¹H NMR (400 MHz, DMSO-*d*₆) *δ* 7.40-7.36 (m, *J* = 16.0 Hz, 4H), 6.94 (s, 1H), 3.47 (s, 1H), 1.84 (s, 3H). | 322.0 | 493 | 0.73 |
| A272 | 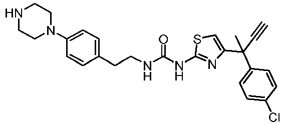 | 1-(4-(2-(4-chlorophenyl)but-3-yn-2-yl)thiazol-2-yl)-3-(4-(piperazin-1-yl)phenethyl)urea | | 494.1 | 135 | 0.89 |
| A273 | 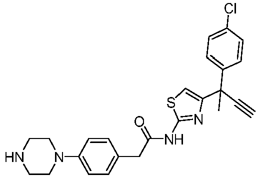 | N-(4-(2-(4-chlorophenyl)but-3-yn-2-yl)thiazol-2-yl)-2-(4-(piperazin-1-yl)phenyl)acetamide | ¹H NMR (400 MHz, DMSO-*d*₆) δ 7.31 (s, 4H), 7.13-7.09 (m, 4H), 6.83 (d, J = 8.0 Hz, 1H), 3.54 (s, , 2H), 3.50 (m, 4H), 2.78 (s, 4H), 1.84 (s, 3H). | 465.1 | 60 | 2.68 |
| A274 | 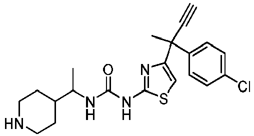 | 1-(4-(2-(4-chlorophenyl)but-3-yn-2-yl)thiazol-2-yl)-3-(1-(piperidin-4-yl)ethyl)urea | ¹H NMR (400 MHz, DMSO-*d*₆) δ 7.41-7.34 (m, 4H), 6.86 (s, 1H), 3.16 (s, 4H), 2.67 (s, 1H), 1.82 (s, 3H), 1.66 (s, 2H), 1.49 (s,1H), 1.25 (s, 2H), 1.01 (s, 3H). | 417.1 | 467 | 2.55 |
| A275 | 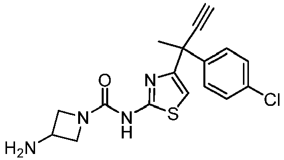 | 3-amino-N-(4-(2-(4-chlorophenyl)but-3-yn-2-yl)thiazol-2-yl)azetidine-1-carboxamide | | 361.0 | 503 | 1.59 |
| A276 | 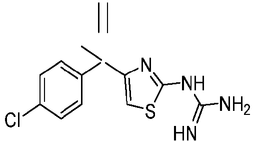 | 1-(4-(2-(4-chlorophenyl)but-3-yn-2-yl)thiazol-2-yl)guanidine | | 305.0 | 475 | 1.58 |
| A272 | 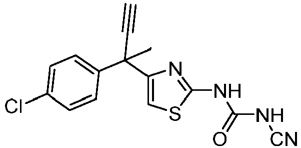 | | | [M+H]⁺=331 .0 | 666 | NA |
| A273 | 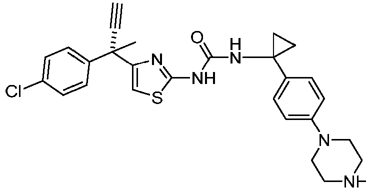 | 1-(4-(2-(4-chlorophenyl)but-3-yn-2-yl)thiazol-2-yl)-3-(1-(4-(piperazin-1-yl)phenyl)cyclopropyl)u rea | ¹H NMR (400 MHz, DMSO-*d*₆) *δ* 10.77 - 11.73 (m, 1H), 8.01 (br s, 1 H), 7.39 - 7.44 (m, 2 H), 7.34 - 7.39 (m, 2 H), 7.02 (d, *J=* 8.70 Hz, 2 H), 6.86 (s, 1 H), 6.80 (br d, *J=* 8.82 Hz, 2 H), 3.48 (s, 1 H), 2.92 - 2.99 (m, 4 H), 2.75 - 2.83 (m, 4 H), 1.84 (s, 3 H), 1.06 (br d, *J=* 9.06 Hz, 4 H). | 506.1 | 237 | 3.20 |
| A274 | 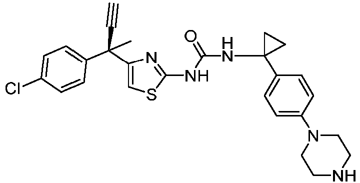 | 1-(4-(2-(4-chlorophenyl)but-3-yn-2-yl)thiazol-2-yl)-3-(1-(4-(piperazin-1-yl)phenyl)cyclopropyl)u rea | ¹H NMR (400 MHz, DMSO-*d*₆) *δ* 10.50 (br s, 1 H), 7.40 - 7.47 (m, 2 H), 7.34 - 7.40 (m, 2 H), 7.02 (br d, *J =* 8.70 Hz, 3 H), 6.91 (s, 1 H), 6.82 (br d, *J =* 8.82 Hz, 2 H), 3.50 (s, 1 H), 2.92 - 3.04 (m, 4 H), 2.75 - 2.88 (m, 4 H), 1.84 (s, 3 H), 1.09 (br d, *J =* 3.58 Hz, 4 H). | 506.1 | 327 | 3.8 |
| A275 | 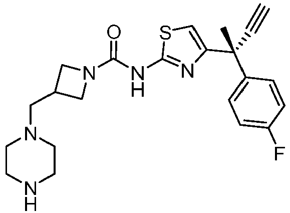 | N-(4-(2-(4-fluorophenyl)but-3-yn-2-yl)thiazol-2-yl)-3-(piperazin-1-ylmethyl)azetidine-1-carboxamide | ¹H NMR (400 MHz, DMSO-*d*₆) *δ* 12.00 (br s, 1 H), 11.01 (br s, 1 H), 9.79 (br s, 2 H), 7.40 (dd,*J* = 8.66, 5.40 Hz, 2 H), 7.13 (t, *J* = 8.66 Hz, 2 H), 6.93 (s, 1 H), 4.09 (br s, 2 H), 3.76 - 3.85 (m, 2 H), 3.57 (br d, *J* = 4.77 Hz, 2 H), 3.39 - 3.53 (m, 7 H), 3.26 (br s, 2 H), 3.11 (br d, *J* = 6.53 Hz, 1 H), 1.85 (s, 3 H). | 428.0 | 246 | 3.38 |
| A276 | 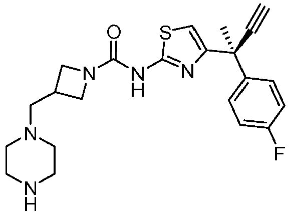 | N-(4-(2-(4-fluorophenyl)but-3-yn-2-yl)thiazol-2-yl)-3-(piperazin-1-ylmethyl)azetidine-1-carboxamide | ¹HNMR (400 MHz, DMSO-*d*₆) *δ* 12.01 (br s, 1 H), 11.01 (br s, 1 H), 9.79 (br s, 2 H), 7.40 (dd,*J =* 8.41, 5.40 Hz, 2 H), 7.13 (t, *J* = 8.78 Hz, 2 H), 6.93 (s, 1 H), 4.01 - 4.12 (m, 2 H), 3.75 - 3.85 (m, 2 H), 3.58 (br s, 2 H), 3.37 - 3.52 (m, 7 H), 3.27 (br s, 2 H), 3.10 (br s, 1 H), 1.85 (s, 3 H). | 428.1 | 345 | 0.56 |
| A277 | 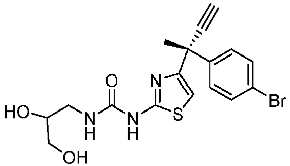 | 1-(4-(2-(4-bromophenyl)but-3-yn-2-yl)thiazol-2-yl)-3-(2,3-dihydroxypropyl)urea | ¹H NMR (400 MHz, DMSO-*d*₆) *δ* 10.60 (br s, 1 H), 7.51 (d, *J =* 8.53 Hz, 2 H), 7.37 (d, *J =* 8.53 Hz, 2 H), 6.90 (s, 1 H), 6.33 (br s, 1 H), 4.86 (br d, *J =* 4.77 Hz, 1 H), 4.59 (t, *J* = 5.65 Hz, 1 H), 3.50 (s, 1 H), 3.42 - 3.49 (m, 1 H), 3.34 - 3.37 (m, 0.5 H), 3.20 - 3.31 (m, 2.5 H), 2.90 - 3.04 (m, 1 H), 1.85 (s, 3 H). | 424.0 | 2667 | 1.23 |
| A278 | 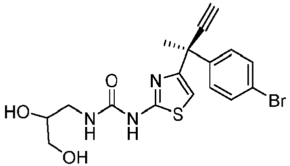 | 1-(4-(2-(4-bromophenyl)but-3-yn-2-yl)thiazol-2-yl)-3-(2,3-dihydroxypropyl)urea | ¹H NMR (400 MHz, DMSO-*d*₆) *δ* 10.62 (br s, 1 H), 7.51 (d, *J =* 8.53 Hz, 2 H), 7.37 (d, *J =* 8.53 Hz, 2 H), 6.89 (s, 1 H), 6.34 (br s, 1 H), 4.86 (br d, *J =* 4.52 Hz, 1 H), 4.60 (t, *J =* 5.40 Hz, 1 H), 3.50 (s, 1 H), 3.47 (br d, *J =* 4.77 Hz, 1 H), 3.35 (br s, 0.5 H), 3.20 - 3.31 (m, 2.5 H), 2.90 - 3.03 (m, 1 H), 1.84 (s, 3 H). | 424.0 | 624 | 0.52 |
| A279 | 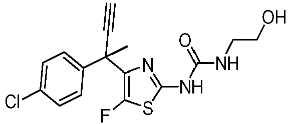 | 1-(4-(2-(4-chlorophenyl)but-3-yn-2-yl)-5-fluorothiazol-2-yl)-3-(2-hydroxyethyl)urea | ¹HNMR (400 MHz, DMSO-*d*₆) *δ* 10.65 (s, 1 H), 7.50 - 7.35 (m, 4H), 6.34 (br. s, 1H), 4.76 (t, J = 4.0 Hz, 1H), 3.53 (s, 1 H), 3.41 (q, J = 4.0 Hz, 1H), 3.15 (q, J = 4.0 Hz, 1H), 1.86 (s, 3 H). | 368.0 | 222 | 4.23 |
| A280 | 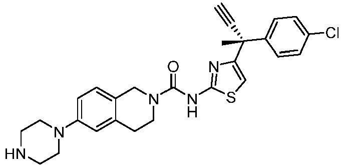 | N-(4-(2-(4-chlorophenyl)but-3-yn-2-yl)thiazol-2-yl)-6-(piperazin-1-yl)-3,4-dihydroisoquinoline-2(1H)-carboxamide | ¹H NMR (400 MHz, DMSO-*d*₆) *δ* 7.44 - 7.35 (m, 4 H), 7.01 - 6.94 (m, 2 H), 6.81 - 6.75 (m, 1 H), 6.72 - 6.68 (m, 1 H), 4.53 (s, 2H), 3.66 (t, J =8 Hz, 2H), 3.50 (s, 1H), 3.05 - 2.99 (m, 4H), 2.88 - 2.82 (m, 4H), 2.75 (t, J =8 Hz, 2H ), 1.87 (s, 3H). | 506.5 | 21 | 2.18 |
| A281 | 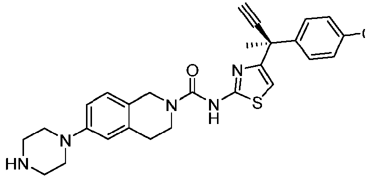 | N-(4-(2-(4-chlorophenyl)but-3-yn-2-yl)thiazol-2-yl)-6-(piperazin-1-yl)-3,4-dihydroisoquinoline-2(1H)-carboxamide | ¹HNMR (400 MHz, DMSO-*d*₆) *δ* 7.44 - 7.36 (m, 4 H), 7.01 - 6.94 (m, 2 H), 6.80 - 6.75 (m, 1 H), 6.71 - 6.68 (m, 1 H), 4.53 (s, 2H), 3.66 (t, J =8 Hz, 2H), 3.50 (s, 1H), 3.04 - 2.98 (m, 4H), 2.86 - 2.80 (m, 4H), 2.75 (t, J =8 Hz, 2H ), 1.87 (s, 3H). | 506.5 | 38 | 0.86 |
| A282 | 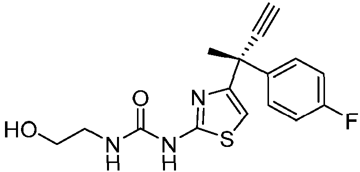 | 1-(4-(2-(4-fluorophenyl)but-3-yn-2-yl)thiazol-2-yl)-3-(2-hydroxyethyl)urea | ¹H NMR (400 MHz, DMSO-*d*₆) *δ* 10.63 (s, 1 H), 7.44 (dd, *J =* 8.78, 5.52 Hz, 2 H), 7.13 (t, *J* = 8.91 Hz, 2 H), 6.87 (s, 1 H), 6.34 (br s, 1 H), 4.75 (t, *J* = 5.02 Hz, 1 H), 3.48 (s, 1 H), 3.41 (q, *J =* 5.52 Hz, 2 H), 3.16 (q, *J* = 5.60 Hz, 2 H), 1.85 (s, 3 H). | 334.1 | 2153 | 7.72 |
| A283 | 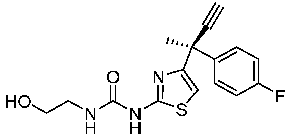 | 1-(4-(2-(4-fluorophenyl)but-3-yn-2-yl)thiazol-2-yl)-3-(2-hydroxyethyl)urea | ¹H NMR (400 MHz, DMSO-*d*₆) *δ* 10.63 (s, 1 H), 7.44 (dd, *J =* 8.78, 5.52 Hz, 2 H), 7.13 (t, *J =* 8.91 Hz, 2 H), 6.88 (s, 1 H), 6.33 (br s, 1 H), 4.75 (t, *J* = 5.14 Hz, 1 H), 3.48 (s, 1 H), 3.41 (q, *J* = 5.35 Hz, 2 H), 3.16 (q, *J* = 5.52 Hz, 2 H), 1.85 (s, 3 H). | 334.1 | 550 | 1.70 |
| A284 | 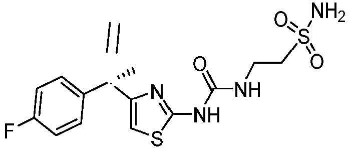 | 2-(3-(4-(2-(4-fluorophenyl)but-3-yn-2-yl)thiazol-2-yl)ureido)ethanesulfona mide | ¹H NMR (400 MHz, DMSO-*d*₆) *δ* 10.94 (br s, 1 H), 7.44 (dd, *J =* 8.78, 5.52 Hz, 2 H), 7.13 (t, *J*= 8.91 Hz, 2 H), 6.92 (s, 2 H), 6.90 (s, 1 H), 6.48 (br t, *J* = 5.65 Hz, 1 H), 3.49 - 3.56 (m, 2 H), 3.48 (s, 1 H), 3.13 (t, *J =* 6.65 Hz, 2 H), 1.85 (s, 3 H). | 397.0 | 666 | 2.60 |
| A285 | 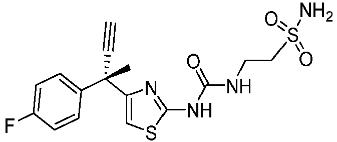 | 2-(3-(4-(2-(4-fluorophenyl)but-3-yn-2-yl)thiazol-2-yl)ureido)ethanesulfona mide | ¹H NMR (400 MHz, DMSO-*d*₆) *δ* 10.95 (br s, 1 H), 7.37 - 7.50 (m, 2 H), 7.13 (t, *J* = 8.78 Hz, 2 H), 6.92 (s, 2 H), 6.90 (s, 1 H), 6.48 (br t, *J =* 5.90 Hz, 1 H), 3.49 - 3.56 (m, 2 H), 3.48 (s, 1 H), 3.14 (t, *J =* 6.65 Hz, 2 H), 1.85 (s, 3 H). | 397.1 | 350 | 1.36 |
| A286 | 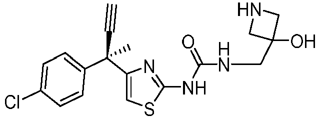 | 1-(4-(2-(4-chlorophenyl)but-3-yn-2-yl)thiazol-2-yl)-3-((3-hydroxyazetidin-3-yl)methyl)urea | ¹H NMR (400 MHz, DMSO-*d*₆) *δ* 10.75 (br s, 1 H), 9.17 (br s, 1 H), 8.99 (br s, 1 H), 7.46 - 7.41 (m, 2 H), 7.40 -7.36 (m, 2 H), 6.93 (s, 1 H), 6.82 (br s, 1 H), 3.75 - 3.90 (m, 5 H), 3.52 (s, 1 H), 3.43 (d, *J =* 6.0 Hz, 2 H), 1.85 (s, 3 H) | 391.1 | 495 | 15.40 |
| A287 | 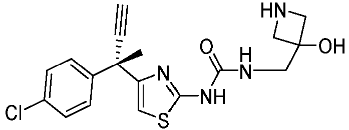 | 1-(4-(2-(4-chlorophenyl)but-3-yn-2-yl)thiazol-2-yl)-3-((3-hydroxyazetidin-3-yl)methyl)urea | ¹H NMR (400 MHz, DMSO-*d*₆) *δ* 10.75 (br s, 1 H), 9.10 (br s, 1 H), 8.95 (br s, 1 H), 7.45 - 7.41 (m, 2 H), 7.40- 7.35 (m, 2 H), 6.93 (s, 1 H), 6.77 (br s, 1 H), 3.86 (br s, 5 H), 3.51 (s, 1 H) 3.43 (br d, *J* = 6.0 Hz, 2 H), 1.85 (s, 3 H) | 391.1 | 130 | 4.40 |
| A288 | 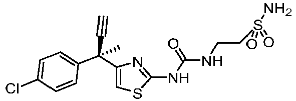 | 2-(3-(4-(2-(4-chlorophenyl)but-3-yn-2-yl)thiazol-2-yl)ureido)ethanesulfona mide | H NMR (400 MHz, CDCl₃) *δ* 9.48 (br s, 1 H), 7.30 (s, 4 H), 6.76 (s, 1 H), 6.05 (br s, 3 H), 3.75 - 3.65 (m, 2 H), 3.09 - 2.99 (m, 2 H), 2.53 (s, 1 H), 1.87 (s, 3 H) | 413.1 | 270 | 1.21 |
| A289 | 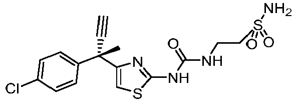 | 2-(3-(4-(2-(4-chlorophenyl)but-3-yn-2-yl)thiazol-2-yl)ureido)ethanesulfona mide | H NMR (400 MHz, CDCl₃) *δ* 9.48 (br s, 1 H), 7.30 (s, 4 H), 6.76 (s, 1 H), 6.06 (br s, 3 H), 3.75 - 3.65 (m, 2 H), 3.10 - 2.98 (m, 2 H), 2.53 (s, 1 H) 1.87 (s, 3 H) | 413.1 | 54 | 0.47 |
| A290 | 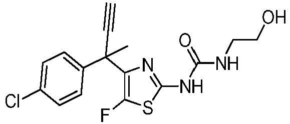 | 1-(4-(2-(4-chlorophenyl)but-3-yn-2-yl)-5-fluorothiazol-2-yl)-3-(2-hydroxyethyl)urea | ¹H NMR (400 MHz, DMSO-*d*₆) *δ* 10.65 (s, 1 H), 7.50 - 7.35 (m, 4H), 6.34 (br. s, 1H), 4.76 (t, J = 4.0 Hz, 1H), 3.53 (s, 1 H), 3.41 (q, J = 4.0 Hz, 1H), 3.15 (q, J = 4.0 Hz, 1H), 1.86 (s, 3 H). | 368.0 | 222 | 4.23 |
| A291 | 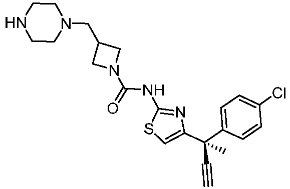 | N-(4-(2-(4-chlorophenyl)but-3-yn-2-yl)thiazol-2-yl)-3-(piperazin-1-ylmethyl)azetidine-1-carboxamide | ¹H NMR (400 MHz, DMSO-*d*₆) *δ* 7.46 - 7.34 (m, 4 H), 6.94 (s, 1H), 4.10 - 3.95 (m, 2 H), 4.0 (br. s, 2H), 3.58 (br. s, 2H), 3.49 (br. s, 1 H), 2.81 - 2.70 (m, 1H), 2.64 (br. s, 4 H), 2.46 - 2.40 (m, 3H), 2.24 (br. s, 4 H), 1.85 (s, 3 H). | 444.0 | 109 | 1.65 |
| A292 | 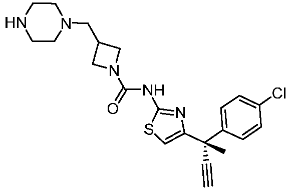 | N-(4-(2-(4-chlorophenyl)but-3-yn-2-yl)thiazol-2-yl)-3-(piperazin-1-ylmethyl)azetidine-1-carboxamide | ¹H NMR (400 MHz, DMSO-*d*₆) *δ* 10.91 (br. s, 1H), 8.78 (br. s, 1H), 7.42 - 7.35 (m, 4 H), 6.95 (s, 1H), 4.09 - 3.96 (m, 2 H), 3.65 - 3.55 (br. s, 2H), 3.50 (br. s, 1 H), 3.08 - 2.99 (m, 4H), 2.87 - 2.70 (m, 1H), 2.60 - 2.52 (m, 4 H), 1.85 (s, 3 H). | 444.1 | 43 | 0.65 |
| A293 | 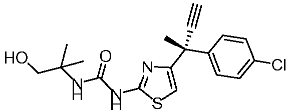 | 1-(4-(2-(4-chlorophenyl)but-3-yn-2-yl)thiazol-2-yl)-3-(1-hydroxy-2-methylpropan-2-yl)urea | ¹H NMR (400 MHz, DMSO-*d*₆) *δ* 10.47 (s, 1 H), 7.29 - 7.49 (m, 4 H), 6.88 (s, 1 H), 6.15 (br. s, 1 H), 4.93 (t, *J=* 5.40 Hz, 1H), 3.49 (s, 1 H), 3.33 (br s, 2 H), 1.84 (s, 3 H), 1.19 (s, 6 H). | 378.1 | 9361 | 8.11 |
| A294 | 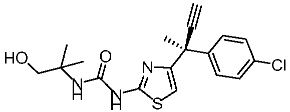 | 1-(4-(2-(4-chlorophenyl)but-3-yn-2-yl)thiazol-2-yl)-3-(1-hydroxy-2-methylpropan-2-yl)urea | ¹H NMR (400 MHz, DMSO-*d*₆) *δ* 10.47 (s, 1 H), 7.31 - 7.50 (m, 4 H), 6.88 (s, 1 H), 6.15 (br s, 1 H), 4.94 (t, *J=* 5.52 Hz, 1 H), 3.49 (s, 1 H), 3.33 (br s, 2 H), 1.84 (s, 3 H), 1.19 (s, 6 H). | 378.1 | 285 | 3.04 |
| A295 | 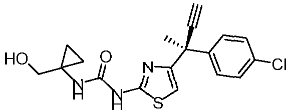 | 1-(4-(2-(4-chlorophenyl)but-3-yn-2-yl)thiazol-2-yl)-3-(1-(hydroxymethyl)cyclopr opyl)urea | ¹H NMR (400 MHz, DMSO-*d*₆) *δ* 10.46 (br s, 1 H), 7.33 - 7.47 (m, 4 H), 6.91 (s, 1 H), 6.57 (br s, 1 H), 4.58 - 4.99 (m, 1 H), 3.49 (s, 1 H), 3.37 (br s, 2 H), 1.84 (s, 3 H), 0.54 - 0.75 (m, 4 H). | 376.1 | 522 | 1.79 |
| A296 | 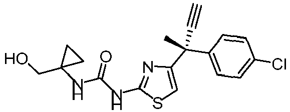 | 1-(4-(2-(4-chlorophenyl)but-3-yn-2-yl)thiazol-2-yl)-3-(1-(hydroxymethyl)cyclopr opyl)urea | ¹H NMR (400 MHz, DMSO-*d*₆) *δ* 10.45 (br s, 1 H), 7.31 - 7.45 (m, 4 H), 6.92 (s, 1 H), 6.55 (br s, 1 H), 4.77 (br s, 1 H), 3.49 (s, 1 H), 3.36 - 3.37 (m, 2 H), 1.84 (s, 3 H), 0.53 - 0.77 (m, 4 H). | 376.1 | 114 | 4.79 |
| A297 | 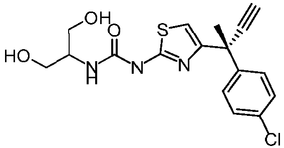 | 1-(4-(2-(4-chlorophenyl)but-3-yn-2-yl)thiazol-2-yl)-3-(1,3-dihydroxypropan-2-yl)urea | ¹H NMR (400MHz, CDCl₃) *δ* 7.42 (d, *J=* 8.3 Hz, 2H), 7.31 - 7.27 (m, 2H), 6.76 (s, 1H), 3.87 (s, 1H), 3.71 - 3.52 (m, 4H), 2.60 (s, 1H), 1.93 (s, 3H) | 380.1 | 935 | 4.39 |
| A298 | 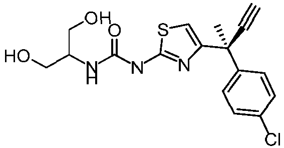 | 1-(4-(2-(4-chlorophenyl)but-3-yn-2-yl)thiazol-2-yl)-3-(1,3-dihydroxypropan-2-yl)urea | ¹H NMR (400MHz, CDCl₃) *δ* 7.41 (d, *J=* 8.3 Hz, 2H), 7.32 - 7.26 (m, 2H), 6.75 (s, 1H), 3.86 (s, 1H), 3.69 - 3.52 (m, 4H), 2.60 (s, 1H), 1.92 (s, 3H) | 380.1 | 246 | 1.15 |
| A299 | 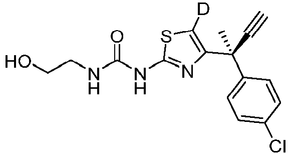 | 1-(4-(2-(4-chlorophenyl)but-3-yn-2-yl)thiazol-2-yl)-3-(2-hydroxyethyl)urea | ¹H NMR (400MHz, CDCl₃) *δ* 7.44 - 7.36 (m, 2H), 7.31 - 7.27 (m, 2H), 6.77 (s, 1H), 3.52 - 3.41 (m, 2H), 3.39 - 3.27 (m, 2H), 2.56 (s, 1H), 1.93 (s, 3H) | 351.1 | 58 | 0.46 |
| A300 | 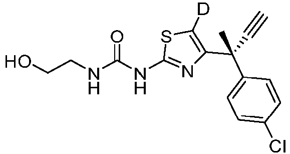 | 1-(4-(2-(4-chlorophenyl)but-3-yn-2-yl)thiazol-2-yl)-3-(2-hydroxyethyl)urea | ¹H NMR (400MHz, CDCl₃) *δ* 7.43 - 7.36 (m, 2H), 7.29 (d, *J* =7.3 Hz, 2H), 6.77 (s, 1H), 3.53 - 3.41 (m, 2H), 3.38 - 3.27 (m, 2H), 2.56 (s, 1H), 1.93 (s, 3H) | 351.1 | 341 | 3.01 |
| A301 | 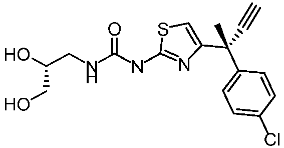 | 1-(4-(2-(4-chlorophenyl)but-3-yn-2-yl)thiazol-2-yl)-3-((R)-2,3-dihydroxypropyl)urea | ¹H NMR (400MHz, DMSO-*d*₆) *δ* 10.64 (br s, 1H), 7.47 - 7.40 (m, 2H), 7.40 - 7.34 (m, 2H), 6.89 (s, 1H), 6.41 (s, 1H), 3.54 - 3.42 (m, 2H), 3.37 - 3.20 (m, 3H), 3.04 - 2.91 (m, 1H), 1.85 (s, 3H) | 380.0 | 354 | 2.22 |
| A302 | 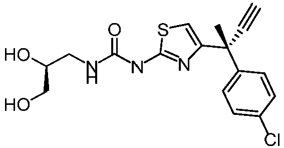 | 1-(4-(2-(4-chlorophenyl)but-3-yn-2-yl)thiazol-2-yl)-3-((S)-2,3-dihydroxypropyl)urea | ¹H NMR (400MHz, DMSO-*d*₆) *δ* 10.64 (br s, 1H), 7.46 - 7.40 (m, 2H), 7.40 - 7.34 (m, 2H), 6.89 (s, 1H), 6.43 (s, 1H), 3.54 - 3.43 (m, 2H), 3.37 - 3.20 (m, 3H), 3.03 - 2.91 (m, 1H), 1.85 (s, 3H) | 379.9 | 417 | 2.04 |
| A303 | 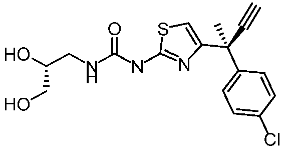 | 1-(4-(2-(4-chlorophenyl)but-3-yn-2-yl)thiazol-2-yl)-3-((R)-2,3-dihydroxypropyl)urea | ¹H NMR (400MHz, DMSO-*d*₆) *δ* 10.64 (br s, 1H), 7.48 - 7.40 (m, 2H), 7.40 - 7.33 (m, 2H), 6.89 (s, 1H), 6.43 (s, 1H), 3.56 - 3.41 (m, 2H), 3.37 - 3.19 (m, 3H), 3.04 - 2.88 (m, 1H), 1.85 (s, 3H) | 380.0 | 101 | 0.66 |
| A304 | 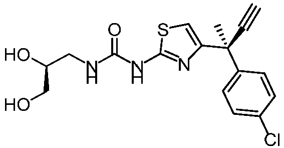 | 1-(4-(2-(4-chlorophenyl)but-3-yn-2-yl)thiazol-2-yl)-3-((S)-2,3-dihydroxypropyl)urea | ¹H NMR (400MHz, DMSO-*d*₆) *δ* 10.67 (br s, 1H), 7.48 - 7.40 (m, 2H), 7.40 - 7.33 (m, 2H), 6.90 (s, 1H), 6.54 (s, 1H), 3.57 - 3.42 (m, 2H), 3.38 - 3.20 (m, 3H), 3.07 - 2.90 (m, 1H), 1.85 (s, 3H) | 380.0 | 90 | 0.52 |
| A305 | 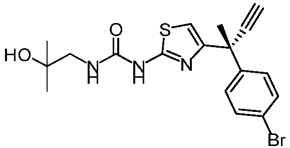 | 1-[4-[1-(4-bromophenyl)-1-methyl-prop-2-ynyl]thiazol-2-yl]-3-(2-hydroxy-2-methyl-propyl)urea | ¹H NMR (400MHz, DMSO-*d*₆) *δ* 10.57 (br. s, 1H), 7.51 (d, *J*=8.5 Hz, 2H), 7.38 (d, *J*=8.6 Hz, 2H), 6.89 (s, 1H), 6.37 (br. s, 1H), 4.54 (s, 1H), 3.50 (s, 1H), 3.04 (d, *J*=5.7 Hz, 2H), 1.85 (s, 3H), 1.05 (s, 6H). | 422.0 | 167 | 2.69 |
| A306 | 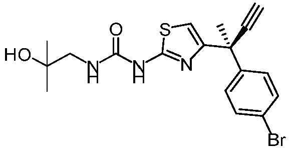 | 1-[4-[1-(4-bromophenyl)-1-methyl-prop-2-ynyl]thiazol-2-yl]-3-(2-hydroxy-2-methyl-propyl)urea | ¹H NMR (400MHz, DMSO-*d*₆) *δ* 10.57 (br. s, 1H), 7.51 (d, J=8.6 Hz, 2H), 7.38 (d, J=8.6 Hz, 2H), 6.89 (s, 1H), 6.37 (br. s, 1H), 4.54 (s, 1H), 3.50 (s, 1H), 3.04 (d, J=5.7 Hz, 2H), 1.85 (s, 3H), 1.05 (s, 6H). | 422.1 | 56 | 0.83 |
| A307 | 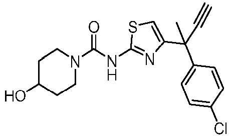 | N-(4-(2-(4-chlorophenyl)but-3-yn-2-yl)thiazol-2-yl)-4-hydroxypiperidine-1-carboxamide | | 390.1 | 2058 | 3.15 |
| A308 | 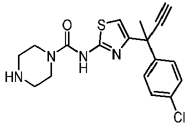 | N-(4-(2-(4-chlorophenyl)but-3-yn-2-yl)thiazol-2-yl)piperazine-1-carboxamide | | 375.1 | 1535 | 5.33 |
| A309 | 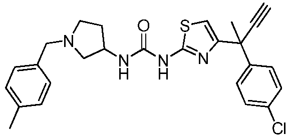 | 1-(4-(2-(4-chlorophenyl)but-3-yn-2-yl)thiazol-2-yl)-3-(1-(4-methylbenzyl)pyrrolidin -3-yl)urea | ¹H NMR (400 MHz, DMSO-*d*₆) *δ* 10.44 (br s, 1H), 7.48 - 7.33 (m, 4H), 7.23 - 7.06 (m, 4H), 6.90 (s, 1H), 6.51 (br s, 1H), 4.10 (br s, 1H), 3.60 - 3.43 (m, 3H), 2.74 - 2.59 (m, 1H), 2.56 - 2.51 (m, 1H), 2.38 - 2.21 (m, 5H), 2.20 - 2.08 (m, 1H), 1.84 (s, 3H), 1.54 - 1.40 (m, 1H). | 479.1 | 1414 | 4.12 |
| A310 | 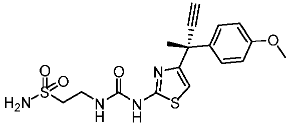 | 2-(3-(4-(2-(4-methoxyphenyl)but-3-yn-2-yl)thiazol-2-yl)ureido)ethanesulfona mide | ¹H NMR (400 MHz, CDCl₃) *δ* 7.32 - 7.27 (m, 2H), 6.89 - 6.79 (m, 2H), 6.74 (s, 1H), 6.02 (br s, 3H), 3.80 (s, 3H), 3.74 - 3.59 (m, 2H), 3.15 - 2.95 (m, 2H), 2.54 (s, 1H), 1.87 (s, 3H). | 409.0 | 223 | 0.98 |
| A311 | 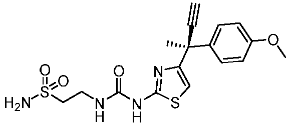 | 2-(3-(4-(2-(4-methoxyphenyl)but-3-yn-2-yl)thiazol-2-yl)ureido)ethanesulfona mide | ¹H NMR (400 MHz, CDCl₃) *δ* 7.27 - 7.23 (m, 2H), 6.88 - 6.80 (m, 2H), 6.75 (s, 1H), 6.07 (br s, 3H), 3.80 (s, 3H), 3.74 - 3.62 (m, 2H), 3.10 - 2.93 (m, 2H), 2.51 (s, 1H), 1.86 (s, 3H). | 409.0 | 95 | 0.66 |
| A312 | 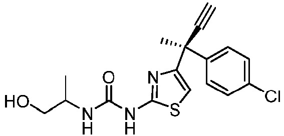 | 1-(4-(2-(4-chlorophenyl)but-3-yn-2-yl)thiazol-2-yl)-3-(1-hydroxypropan-2-yl)urea | ¹H NMR (400 MHz, CD₃OD) *δ* 7.54 - 7.46 (m, 2H), 7.32 - 7.25 (m, 2H), 6.91 (s, 1H), 3.93 - 3.76 (m, 1H), 3.60 - 3.41 (m, 2H), 2.96 (s, 1H), 1.92 (s, 3H), 1.16 (d, *J =* 6.4 Hz, 3H). | 365.6 | 545 | 2.12 |
| A313 | 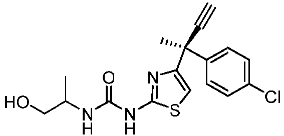 | 1-(4-(2-(4-chlorophenyl)but-3-yn-2-yl)thiazol-2-yl)-3-(1-hydroxypropan-2-yl)urea | ¹H NMR (400 MHz, CD₃OD) *δ* 7.55 - 7.43 (m, 2H), 7.37 - 7.24 (m, 2H), 6.90 (s, 1H), 3.91 - 3.76 (m, 1H), 3.56 - 3.44 (m, 2H), 2.96 (s, 1H), 1.92 (s, 3H), 1.16 (d, *J =* 6.4 Hz, 3H). | 365.5 | 614 | 2.82 |
| A314 | 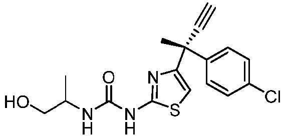 | 1-(4-(2-(4-chlorophenyl)but-3-yn-2-yl)thiazol-2-yl)-3-(1-hydroxypropan-2-yl)urea | ¹H NMR (400 MHz, CD₃OD) *δ* 7.58 - 7.39 (m, 2H), 7.36 - 7.18 (m, 2H), 6.90 (s, 1H), 3.97 - 3.75 (m, 1H), 3.62 - 3.37 (m, 2H), 2.96 (s, 1H), 1.92 (s, 3H), 1.16 (d, *J =* 6.4 Hz, 3H). | 365.5 | 261 | 0.80 |
| A315 | 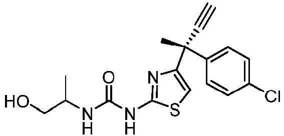 | 1-(4-(2-(4-chlorophenyl)but-3-yn-2-yl)thiazol-2-yl)-3-(1-hydroxypropan-2-yl)urea | ¹H NMR (400 MHz, CD₃OD) *δ* 7.57 - 7.46 (m, 2H), 7.35 - 7.21 (m, 2H), 6.91 (s, 1H), 4.00 - 3.73 (m, 1H), 3.56 - 3.41 (m, 2H), 2.96 (m, 1H), 1.92 (s, 3H), 1.16 (d, *J =* 6.4 Hz, 3H). | 365.6 | 81 | 0.65 |
| A316 | 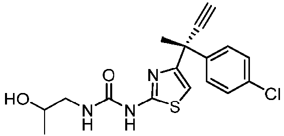 | 1-(4-(2-(4-chlorophenyl)but-3-yn-2-yl)thiazol-2-yl)-3-(2-hydroxypropyl)urea | ¹H NMR (400 MHz, CD₃OD) *δ* 7.54 - 7.44 (m, 2H), 7.32 - 7.25 (m, 2H), 6.91 (s, 1H), 3.90 - 3.76 (m, 1H), 3.28 (s, 1H), 3.16 - 3.04 (m, 1H), 2.95 (s, 1H), 1.92 (s, 3H), 1.15 (d, *J* = 6.4 Hz, 3H). | 365.6 | 421 | 1.81 |
| A317 | 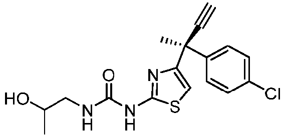 | 1-(4-(2-(4-chlorophenyl)but-3-yn-2-yl)thiazol-2-yl)-3-(2-hydroxypropyl)urea | ¹H NMR (400 MHz, CD₃OD) *δ* 7.52 - 7.44 (m, 2H), 7.32 - 7.26 (m, 2H), 6.91 (s, 1H), 3.89 - 3.78 (m, 1H), 3.30 - 3.25 (m, 1H), 3.15 - 3.06 (m, 1H), 2.95 (s, 1H), 1.92 (s, 3H), 1.15 (d, *J =* 6.4 Hz, 3H). | 365.6 | 85 | 0.97 |
| A318 | 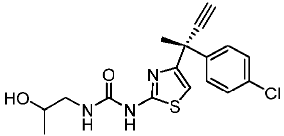 | 1-(4-(2-(4-chlorophenyl)but-3-yn-2-yl)thiazol-2-yl)-3-(2-hydroxypropyl)urea | ¹H NMR (400 MHz, CD₃OD) *δ* 7.56 - 7.43 (m, 2H), 7.34 - 7.22 (m, 2H), 6.91 (s, 1H), 3.92 - 3.70 (m, 1H), 3.30 - 3.25 (m, 1H), 3.15 - 3.06 (m, 1H), 2.95 (s, 1H), 1.92 (s, 3H), 1.15 (d, *J =* 6.4 Hz, 3H). | 365.6 | 534 | 2.24 |
| A319 | 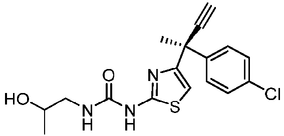 | 1-(4-(2-(4-chlorophenyl)but-3-yn-2-yl)thiazol-2-yl)-3-(2-hydroxypropyl)urea | ¹H NMR (400 MHz, CD₃OD) *δ* 7.59 - 7.43 (m, 2H), 7.35 - 7.14 (m, 2H), 6.91 (s, 1H), 3.89 - 3.75 (m, 1H), 3.30 - 3.24 (m, 1H), 3.14 - 3.05 (m, 1H), 2.95 (s, 1H), 1.92 (s, 3H), 1.15 (d, *J =* 6.4 Hz, 3H). | 365.6 | 156 | 0.67 |
| A320 | 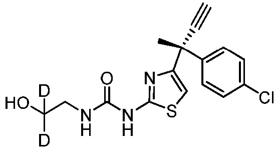 | 1-[4-[1-(4-chlorophenyl)-1-methyl-prop-2-ynyl]thiazol-2-yl]-3-(2,2-dideuterio-2-hydroxy-ethyl)urea | ¹H NMR (400MHz, CDCl₃) *δ* 7.34-7.28 (m, 2H), 7.22 - 7.19 (m, 2H), 6.69 (s, 1H), 3.23 (d, *J =* 5.5 Hz, 2H), 2.48 (s, 1H), 1.84 (s, 3H) | 352.1 | 44 | |
| A321 | 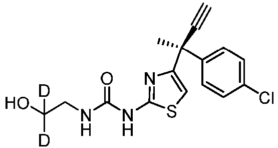 | 1-[4-[1-(4-chlorophenyl)-1-methyl-prop-2-ynyl]thiazol-2-yl]-3-(2,2-dideuterio-2-hydroxy-ethyl)urea | ¹H NMR (400MHz, CDCl₃) *δ* 7.43 - 7.35 (m, 2H), 7.31 - 7.27 (m, 2H), 6.76 (s, 1H), 3.31 (d, *J =* 5.5 Hz, 2H), 2.55 (s, 1H), 1.92 (s, 3H) | 352.1 | 28 | 0.30 |

### Example 13: Preparation of tert-butyl 4-(4-((3-(4-(1-(4-bromophenyl)ethyl)thiazol-2-yl)ureido)methyl)phenyl)piperazine-1-carboxylate

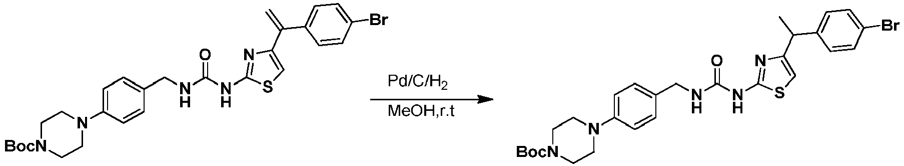

To a solution of tert-butyl 4-(4-((3-(4-(1-(4-bromophenyl)vinyl)thiazol-2-yl)ureido)methyl)phenyl)piperazine-1-carboxylate (120 mg) in MeOH (5 mL) was added Pd/C (12 mg), the mixture was stirred overnight at RT under hydrogen pressure. After filtration and evaporation, the obtained residue was purified by column chromatography on a silica gel to afford tert-butyl 4-(4-((3-(4-(1-(4-bromophenyl)ethyl)thiazol-2-yl)ureido)methyl)phenyl)piperazine-1-carboxylate (73 mg).

### Example 14: Preparation of tert-butyl 4-(5-((3-(4-(2-(4-bromophenyl)propan-2-yl)thiazol-2-yl)ureido)methyl)-3-fluoropyridin-2-yl)piperazine-1-carboxylate

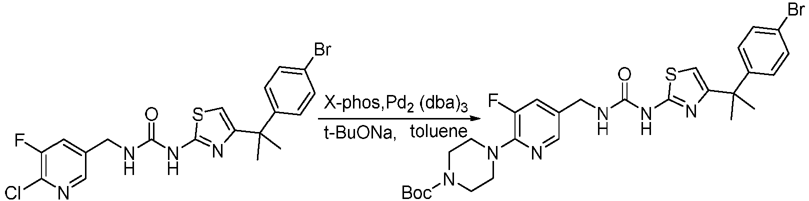

A suspension of 1-(4-(2-(4-bromophenyl)propan-2-yl)thiazol-2-yl)-3-((6-chloro-5-fluoropyridin-3-yl)methyl)urea (174 mg, 0.4 mmol), tert-butyl piperazine-1-carboxylate (82 mg, 0.44 mmol), X-phos (39 mg, 0.08 mmol), Pd₂ (dba) ₃ (36.6 mg, 0.04 mmol) and t-BuONa (46.1 mg, 0.48 mmol) in toluene (5 mL) was stirred at 90°C under N₂ atmosphere overnight. The reaction mixture was cooled to RT and filtered off the solid, the residue was dissolved in ethyl acetate (100 mL) and washed with brine. The organic phase was dried over MgSO₄, filtered, concentrated in vacuum to give the crude product, which was purified by flashed column to give the desired product (67 mg, yield 25%).

### Example 15: Preparation of 5-((3-(4-(2-(4-methoxyphenyl)propan-2-yl)thiazol-2-yl)ureido)methyl)-2-(3-methylpiperazin-1-yl)benzamide

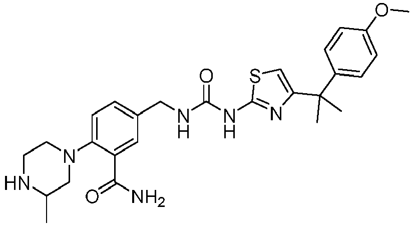

### Step 1 Preparation of 2-(4-(tert-butoxycarbonyl)-3-methylpiperazin-1-yl)-5-((3-(4-(2-(4-methoxyphenyl)propan-2-yl)thiazol-2-yl)ureido)methyl)benzoic acid

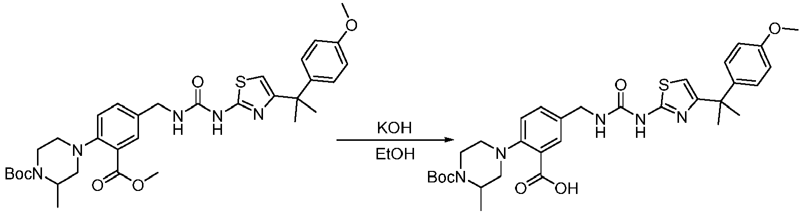

A mixture of tert-butyl 4-(2-(methoxycarbonyl)-4-((3-(4-(2-(4-methoxyphenyl)propan-2-yl)thiazol-2-yl)ureido)methyl)phenyl)-2-methylpiperazine-1-carboxylate (270 mg, 0.42 mmol, 1 eq) and KOH (23.5 mg, 0.42 mmol, 1 eq), was heated to reflux for 0.5 h. After cooling, the reaction was quenched with sat. NH₄Cl (aq), extracted with EA, washed with brine, dried over Na₂SO₄, filtered and evaporated to dryness. The resulting residue was purified by Prep-TLC to give the desired compound (215 mg).

### Step 2: Preparation of tert-butyl 4-(2-carbamoyl-4-((3-(4-(2-(4-methoxyphenyl)propan-2-yl)thiazol-2-yl)ureido)methyl)phenyl)-2-methylpiperazine-1-carboxylate

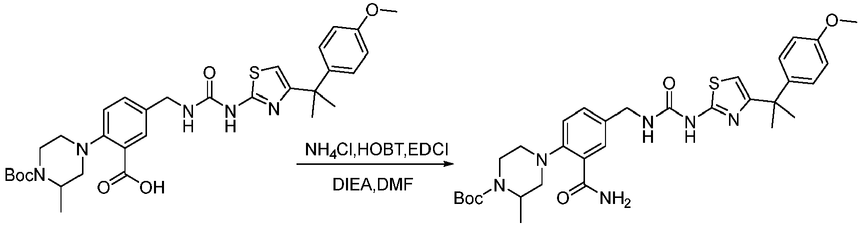

A mixture of 2-(4-(tert-butoxycarbonyl)-3-methylpiperazin-1-yl)-5-((3-(4-(2-(4-methoxyphenyl)propan-2-yl)thiazol-2-yl)ureido)methyl)benzoic acid (215 mg, 0.34 mmol, 1 eq), EDCI (132 mg, 0.69 mmol, 2 eq), HOBt (93 mg, 0.69 mmol, 2 eq) and DIEA (133 mg, 1.03 mmol, 3 eq) were dissolved in THF (0.1 M) and stirred for 15 min at RT. NH₄Cl (36.9 mg, 0.69 mmol, 2 eq) was then added in one portion and the reaction was stirred at RT. Once judged complete by TLC analysis, the resulting suspension was diluted with EtOAc and washed with brine and then dried (Na₂SO₄), filtered and evaporated to dryness. The resulting residue was purified by trituration or Prep-TLC to give the desired product (201 mg).

### Example 16: Preparation of 1-((6-((2-hydroxyethyl)amino)pyridin-3-yl)methyl)-3-(4-(2-(4-methoxyphenyl)propan-2-yl)thiazol-2-yl)urea

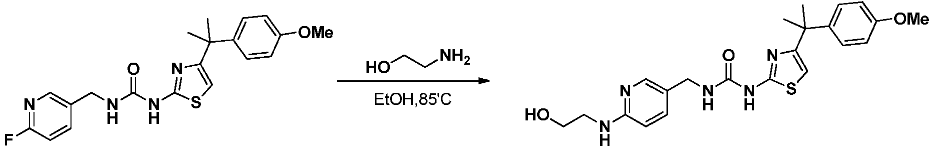

A mixture of 1-((6-fluoropyridin-3-yl)methyl)-3-(4-(2-(4-methoxyphenyl)propan-2-yl)thiazol-2-yl)urea (50 g, 0.13 mmol, 1.0 eq) and 2-aminoethanol (11.9 mg, 0.19 mmol, 1.5 eq) in EtOH was heated to 90°C for 14 h. After the reaction was cooled down to RT, concentrated to give a residue, which was purified by column chromatography on a silica gel to afford 1-((6-((2-hydroxyethyl)amino)pyridin-3-yl)methyl)-3-(4-(2-(4-methoxyphenyl)propan-2-yl)thiazol-2-yl)urea (21 mg).

### Example 17: Preparation of 1-(4-(2-(4-methoxyphenyl)but-3-yn-2-yl)thiazol-2-yl)-3-(1-(4-(piperazin-1-yl)phenyl)ethyl)urea

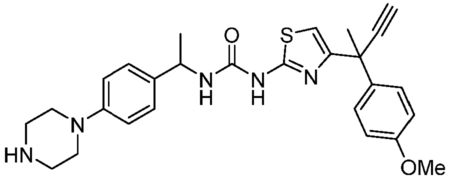

### Step 1. Preparation of methyl 2-(4-methoxyphenyl)acetate

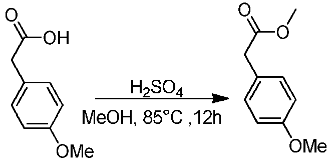

A mixture of 2-(4-methoxyphenyl)acetic acid (20.0 g, 120.4 mmol) in MeOH (100 mL) was added H₂SO₄ (1.2 g, 12.0 mmol, 642 µL) at 15 °C. The mixture was stirred for 12 h at 85°C. The mixture was diluted with EA (400 mL), washed with sat. NaHCO₃ aq (100 mL), brine (10 mL), dried over anhydrous Na₂SO₄, filtered and concentrated in vacuum to give a residue. The residue was purified by silica column (ethyl acetate in petroleum ether =0-15%). The desired product (21.6 g, yield: 99.7%) was obtained as yellow oil.

¹H NMR (400 MHz, CDCl₃) *δ* 7.21 (d, *J =* 8.8 Hz, 2 H) 6.87 (d, *J =* 8.8 Hz, 2 H), 3.80 (s, 3 H), 3.69 (s, 3 H), 3.58 (s, 2 H)

### Step 2. Preparation of compound methyl 2-(4-methoxyphenyl)-3-oxobutanoate

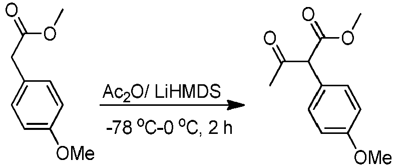

To a solution of compound obtained from step 1 above (23.8 g, 132.2 mmol) in THF (200 mL) was added LiHMDS (1 M, 159 mL) at -78°C. The mixture was stirred for 20 min at -78°C. Acetyl acetate (13.5 g, 132.2 mmol) was added to the solution. Then the mixture was warmed to 0°C and stirred for 2 h at 0°C. The mixture was quenched with sat NH₄Cl aq. (50 mL) and extracted with EA (3 x 50 mL). The combined organic layers were washed with brine (10 mL), dried over anhydrous Na₂SO₄, filtered and concentrated in vacuum. The residue was purified on silica gel chromatography (ethyl acetate in petroleum ether =0-15%) to give the desired compound (14.23 g, yield: 48.4%) as a yellow oil.

¹H NMR (400 MHz, CDCl₃) *δ* 12.97 (s, 1 H), 7.25 - 7.23 (m, 1.5 H), 7.07 - 7.03 (m, 2 H), 6.87 -6.85 (m, 2 H), 4.63 (s, 0.5 H), 3.80 (s, 3 H), 3.78 (s, 1.5 H), 3.73 (s, 1.5 H), 3.67 (s, 3 H), 2.15 (s, 1.5 H), 1.83 (s, 3 H). MS (ESI) *m*/*z* (M + H)⁺ =223.1

### Step 3. Preparation of compound methyl 2-(4-methoxyphenyl)-2-methyl-3-oxobutanoate

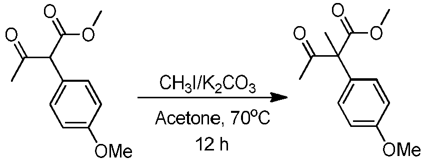

To a mixture of compound obtained from step 2 above (14.5 g, 65.4 mmol) and K₂CO₃ (45.2 g, 326.9 mmol) in ACETONE (100 mL) was added CH₃I (26.0 g, 183.3 mmol) at 15°C. The mixture was stirred at 70°C for 12 h. The mixture was filtered and the filtrate was concentrated in vacuum to give a residue. The residue was purified by silica column (ethyl acetate in petroleum ether =0-15%). The desired compound (9.76 g, yield: 63.2%) was obtained as a colorless oil.

¹H NMR (400 MHz, CDCl₃) *δ* 7.25 - 7.19 (m, 2 H), 6.95 - 6.86 (m, 2 H), 3.82 (s, 3 H), 3.79 (s, 3 H), 2.10 (s, 3 H), 1.77 (s, 3 H)

### Step 4. Preparation of compound methyl 4-bromo-2-(4-methoxyphenyl)-2-methyl-3-oxobutanoate

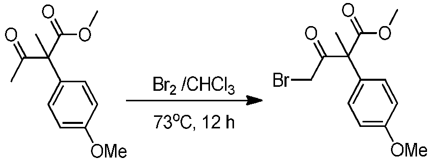

To a solution of compound obtained from step 3 above (1 g, 4.2 mmol) in CHCl₃ (20 mL) was added Br₂ (676 mg, 4.2 mmol) at 15 °C. The mixture was stirred at 73°C for 12 h. The mixture was washed with H₂O (20 mL), brine (10 mL), dried over anhydrous Na₂SO₄, filtered and concentrated in vacuum to give a residue. The desired product (1.03 g, crude) was obtained as a colorless oil. The crude product was directly used for the next step without further purification.

MS (ESI) *m*/*z* (M+H)⁺=315.1

### Step 5. Preparation of compound methyl 2-(2-aminothiazol-4-yl)-2-(4-methoxyphenyl)propanoate

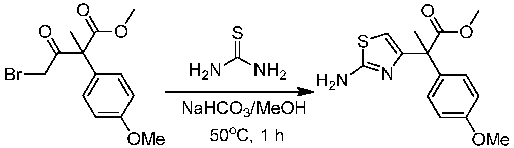

A mixture of compound obtained from step 4 above (1.03 g, 3.3 mmol), THIOUREA (299 mg, 3.9 mmol) and NaHCO₃ (329 mg, 3.9 mmol) in MeOH (15 m L) was stirred at 50 °C for 1 h. The mixture was concentrated in vacuum directly. The residue was triturated with H₂O (20 mL) at 15°C for 10 min., filtered and the cake was concentrated in vacuum to give a residue. The desired product (0.79 g, yield: 82.68%) was obtained as a yellow solid

¹H NMR (400 MHz, CDCl₃) δ 7.20 - 7.18 (m, 2 H), 6.97 - 6.92 (m, 2 H), 6.88 - 6.86 (m, 2 H), 5.95 (s, 1 H), 3.73 (s, 3 H), 3.61 (s, 3 H), 1.77 (s, 3 H).

### Step 6. Preparation of compound methyl 2-(4-methoxyphenyl)-2-(2-((phenoxycarbonyl)amino)thiazol-4-yl)propanoate

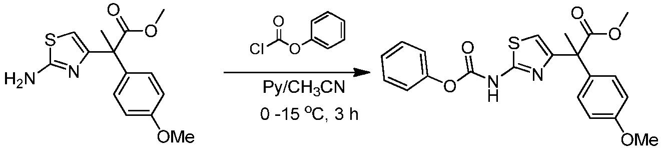

To a mixture of compound obtained from step 5 above (300 mg, 1.03 mmoL) and PYRIDINE (97.4 mg, 1.23 mmol) in CH₃CN (3 mL) was added phenyl carbonochloridate (169 mg, 1.08 mmol) at 0 °C. The mixture was stirred at 15°C for 3 h. The mixture was concentrated in vacuum directly. The residue was purified by silica column (ethyl acetate in petroleum ether =0-30%) to give the desired compound (330 mg, yield: 77.97%) which was obtained as a yellow oil.

MS (ESI) *m*/*z* (M+H)⁺=413.0

### Step 7. Preparation of compound tert-butyl 4-(4-(1-(3-(4-(1-methoxy-2-(4-methoxyphenyl)-1-oxopropan-2-yl)thiazol-2-yl)ureido)ethyl)phenyl)piperazine-1-carboxylate

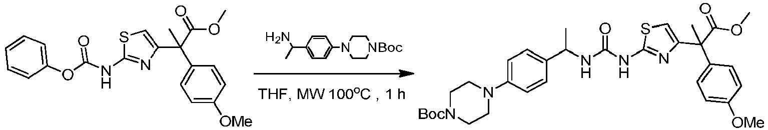

To a mixture of compound obtained from step 6 above (330 mg, 800 µmol) and tert-butyl 4-[4-(1-aminoethyl)phenyl]piperazine-1-carboxylate (269 mg, 880 µmol) in THF (2 mL) was stirred at 100°C for 1 h under Microwave. The mixture was directly concentrated in vacuum to give a residue. The residue was purified by silica column (ethyl acetate in petroleum ether =0-80%). The desired compound (441 mg, yield: 88.37%) was obtained as a yellow oil.

MS (ESI) *m*/*z* (M+H)⁺ = 646.2

### Step 8. Preparation of compound tert-butyl 4-(4-(1-(3-(4-(1-hydroxy-2-(4-methoxyphenyl)propan-2-yl)thiazol-2-yl)ureido)ethyl)phenyl)piperazine-1-carboxylate

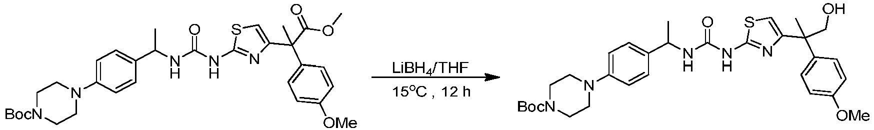

To a solution of compound obtained from step 7 above (370 mg, 593 µmol) in THF (10 mL) was added LiBH₄ (26 mg, 1.2 mmol) at 15°C. The mixture was stirred for 12 h at 15°C. The mixture was diluted with *sat.*NH₄Cl (15 mL) and extracted with EA (3 x 15 mL). The organic layers were washed with brine (10 mL), dried over anhydrous Na₂SO₄, filtered and concentrated in vacuum. The residue was purified by silica column (ethyl acetate in petroleum ether = 0-100%) to give the desired compound (307 mg, yield: 87.0%) which was obtained as a yellow solid.

¹H NMR (400 MHz, CDCl₃) *δ* 7.17 (d, *J* = 8.4 Hz, 2 H), 7.09 - 7.06 (m, 2 H), 6.86 - 6.80 (m, 4 H), 6.45 (s, 1 H), 4.94 - 4.91(m, 1 H), 4.05 - 4.00 (m, 1 H), 3.81 - 3.77 (m, 4 H), 3.56 - 3.54 (m, 4 H) 3.09 - 3.07 (m, 4 H), 1.56 (d, *J =* 1.6 Hz, 3 H), 1.49 (s, 9 H), 1.46 (d, *J =* 6.8 Hz, 3 H).

### Step 9. Preparation of compound 1-(4-(1-hydroxy-2-(4-methoxyphenyl)propan-2-yl)thiazol-2-yl)-3-(1-(4-(piperazin-1-yl)phenyl)ethyl)urea hydrochloride

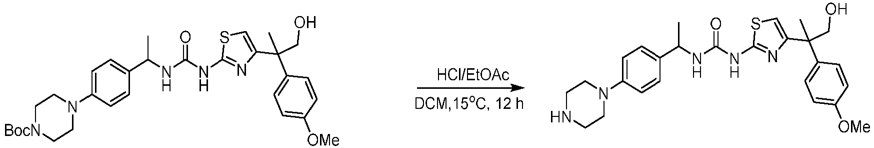

To a solution of compound obtained from step 8 above (50 mg, 83.93 µmol) in DCM (2 mL) was added HCl/EtOAc (4 M, 2 mL) at 15 °C. The mixture was stirred for 12 h at 15°C. The mixture was concentrated in vacuum to give the desired compound (34 mg, yield: 76.1%) was obtained as a yellow solid.

¹H NMR (400 MHz, DMSO) *δ* 10.47 (br s, 1 H), 9.11 (br s, 2 H), 7.36 - 7.23 (m, 1 H), 7.19 (d, *J =* 8.8 Hz, 2 H), 7.10 (d, *J =* 8.8 Hz, 2 H), 6.95 (d, *J =* 8.8 Hz, 2 H), 6.75 (d, *J =* 8.0 Hz, 2 H), 6.69 (s, 1 H), 4.77 - 4.73 (m, 1 H), 3.80 - 3.76 (m, 1 H), 3.70 (s, 3 H) 3.34 - 3.31 (m, 4 H), 3.24 - 3.16 (m, 4 H), 2.07 (s, 1 H), 1.55 (s, 3 H), 1.33 (d, *J* =6.8 Hz, 3 H). MS (ESI) *m*/*z* (M+H)⁺ = 496.2

### Step 10. Preparation of compound tert-butyl 4-(4-(1-(3-(4-(2-(4-methoxyphenyl)-1-oxopropan-2-yl)thiazol-2-yl)ureido)ethyl)phenyl)piperazine-1-carboxylate

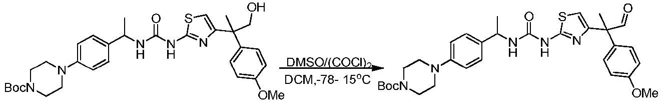

To a solution of oxalyl dichloride (68. 2 mg, 537.14 µmo) in DCM (2 mL) was added DMSO (66 mg, 839 µmol) at -78°C. After 10 min, compound obtained from step 9 above (100 mg, 168 µmol) in DCM (2 mL) was added and stirred for 1 h at -78°C. Et₃N (170 mg, 1.68 mmol) was added and stirred for 10 more min then warmed to 15°C and stirred for another 1 h. The mixture was diluted with H₂O (20 mL), extracted with DCM (3 x 20 mL). The organic layers were washed with brine (10 mL), dried over anhydrous Na₂SO₄, filtered and concentrated in vacuum to give a residue. The desired product (120 mg, crude) was obtained as a yellow oil. The crude product was directly used for the next step without further purification.

### Step 11. Preparation of compound tert-butyl 4-(4-(1-(3-(4-(2-(4-methoxyphenyl)but-3-yn-2-yl)thiazol-2-yl)ureido)ethyl)phenyl)piperazine-1-carboxylate

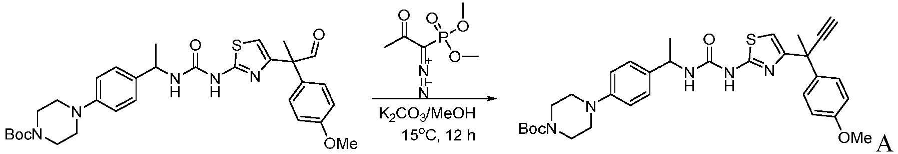

mixtrue of compound obtained from step 10 above (100 mg, 168 µmol) , dimethyl (1-diazo-2-oxopropyl)phosphonate (49 mg, 252.6 µmol) and K₂CO₃ (47 mg, 337 µmol in MeOH (5 mL) was stirred for 1 h at 15°C. The reaction was directly concentrated in vacuum. The residue was purified by prep. HPLC (column: Venusil ASB Phenyl 150*30mm*5um;mobile phase: [water(0.05%HCl)-ACN];B%: 65%-95%,10min) to give the desired compound (50 mg, yield: 50.34%) was obtained as a yellow oil.

MS (ESI) *m*/*z* (M+H)⁺=590.3

### Step 12. Preparation of compound 1-(4-(2-(4-methoxyphenyl)but-3-yn-2-yl)thiazol-2-yl)-3-(1-(4-(piperazin-1-yl)phenyl)ethyl)urea

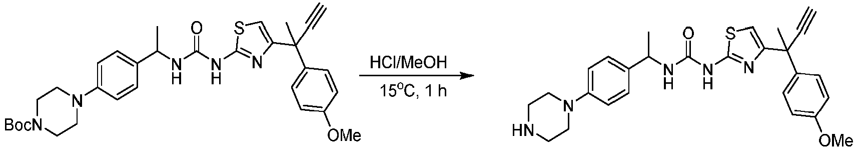

The desired compound (39 mg, yield: 87.4%) was obtained as a yellow solid using De-BOC method.

¹H NMR (400 MHz, DMSO-*d*₆) δ 10.50 (br s, 1 H), 9.22 (br s, 2 H), 7.31 (d, *J* = 8.8 Hz, 2 H), 7.19 (d, *J =* 8.4 Hz, 3 H), 6.95 (d, *J =* 8.4 Hz, 2 H), 6.85 (dd, J=8.4, 1.2 Hz, 2 H), 6.81- 6.79 (m, 1 H), 4.76 - 4.73 (m, 1 H), 3.71 (s, 3 H), 3.39 (s, 1 H), 3.35 - 3.32(m, 4 H) 3.24 - 3.16 (m, 4 H), 1.82 (d, *J =* 2.4 Hz, 3 H), 1.33 (d, *J*=6.8 Hz, 3 H).

MS (ESI) *m*/*z* (M+Na)⁺=512.3

### Example 18: Preparation of 1-(4-(2-(4-cyclopropylphenyl)propan-2-yl)thiazol-2-yl)-3-(4-(piperazin-1-yl)benzyl)urea

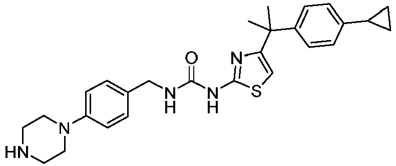

### Step 1: Preparation of tert-butyl 4-(4-((3-(4-(2-(4-cyclopropylphenyl)propan-2-yl)thiazol-2-yl)ureido)methyl)phenyl)piperazine-1-carboxylate

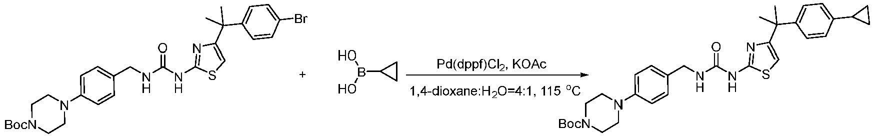

To a solution of compound obtained from step 1 above (81 mg, 0.13 mmol) in 1,4-dioxane (4 mL) and H₂O (1 mL) was added cyclopropylboronic acid (14 mg, 0.16 mmol), Pd(dppf)Cl₂ (10 mg, 0.013 mmol), KOAc (25 mg, 0.26 mmol). The reaction mixture was stirred at 115 °C overnight under N₂ atmosphere. The reaction progress was monitored by TLC. After the completion of the reaction, the mixture was filtered through a pad of celite, washed with EA .The filtrate was removed under reduced pressure and the residue was purified by column chromatography on silica gel (PE/EA = 2:1) to give the desired compound (45 mg, yield: 60.2%) as a white solid.

### Step 2.Preparation of compound 1-(4-(2-(4-cyclopropylphenyl)propan-2-yl)thiazol-2-yl)-3-(4-(piperazin-1-yl)benzyl)urea

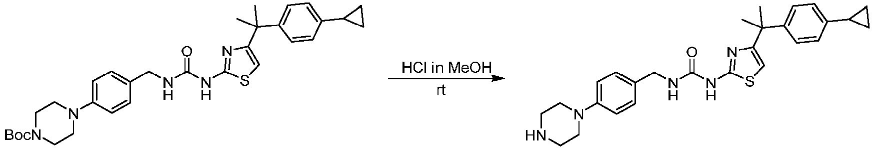

The desired compound was obtained as a white solid (40 mg, HCl salt, yield: 100%) with the procedure described in example 9. MS (ESI) m/z (M+H)⁺ = 476.2.

### Example 19: 1-(4-(2-(4-chlorophenyl)but-3-yn-2-yl)thiazol-2-yl)-3-(2-hydroxyethyl-2,2-d2)urea

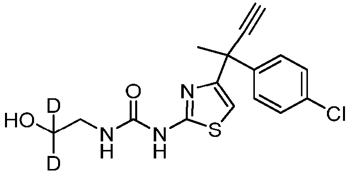

### Step 1. Preparation of compound tert-butyl N-(2,2-dideuterio-2-hydroxyethyl)carbamate

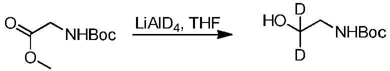

To a solution of methyl 2-((tert-butoxycarbonyl)amino)acetate (1 g, 5.29 mmol) in THF (20 mL) was added LiAlD₄ (364.8 mg, 7.93 mmol) at 0 °C and then the mixture was stirred at 80 °C for 3 h. EA (20 mL) was added dropwise and the H₂O (5 mL), and then extracted with EA (100 mL × 3). The combined organic phase was washed with brine (20 mL × 3), dried over anhydrous Na₂SO₄, filtered and concentrated to give a residue. The desired compound (610 mg, yield: 70.7%) was obtained as yellow oil, which was used into the next step without further purification.

¹H NMR (400MHz, CDCl₃) *δ* 5.17 (br s, 1H), 3.24 (d, *J =* 5.6 Hz, 2H), 3.08 (br s, 1H), 1.42 (s, 9H).

### Step 2. Preparation of compound 2-amino-1,1-dideuterio-ethanol

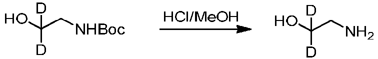

A mixture of compound obtained from step 1 above (610 mg, 3.74 mmol) in HCl/MeOH (4 M, 5 mL) was stirred at 25 °C for 3 h. The reaction mixture was concentrated. The desired compound (520 mg, crude, HCl) was obtained as yellow oil, which was used into the next step without further purification.

¹H NMR (400MHz, DMSO-*d*₆) *δ* 2.80 (q, *J=* 5.7 Hz, 2H).

### Step 3. Preparation of compound phenyl N-[4-[1-(4-chlorophenyl)-1-methyl-prop-2-ynyl]thiazol-2-yl]carbamate

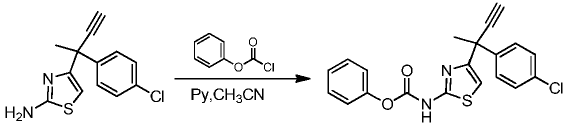

To a solution of 4-[1-(4-chlorophenyl)-1-methyl-prop-2-ynyl]thiazol-2-amine (500 mg, 1.90 mmol) and pyridine (752.60 mg, 9.51 mmol) in MeCN (20 mL) was added phenyl carbonochloridate (327.7 mg, 2.09 mmol) at 0 °C and then the mixture was stirred at 0 °C for 1 h. The residue was poured into water (30 mL). The aqueous phase was extracted with ethyl acetate (80 mL × 3). The combined organic phase was washed with brine (10 mL × 2), dried with anhydrous Na₂SO₄, filtered and concentrated in vacuum. The desired compound (830 mg, crude) was obtained as yellow oil, which was used into the next step without further purification.

MS (ESI) *m*/*z* (M+H)⁺=383.0

### Step 4. Preparation of compound 1-[4-[1-(4-chlorophenyl)-1-methyl-prop-2-ynyl]thiazol-2-yl]-3-(2,2-dideuterio-2-hydroxy-ethyl)urea

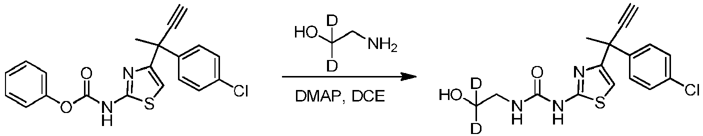

A mixture of compound obtained from step 3 above (400 mg, 1.04 mmol), compound obtained from step 2 above (98.9 mg, 1.57 mmol) and DMAP (12.8 mg, 104.48 umol) in DCE (20 mL) was stirred at 80 °C for 5 h. The reaction mixture was concentrated. The residue was purified by prep-HPLC (column: Xtimate C18 150*40mm*5um; mobile phase: [water (HCl)-ACN]; B%: 28%-58%,10min). The desired compound (90 mg, yield: 24.5%) was obtained as a white solid.

MS (ESI) *m*/*z* (M+H)⁺=352.1.

SFC: Column: ChiralPak IG-3 100×4.6mm I.D., 3um Mobile phase: A: CO₂ B:Ethanol (0.05% DEA) Gradient: from 5% to 40% of B in 5.5min and hold 40% for 3 min, then 5% of B for 1.5 min Flow rate: 2.5mL/min Column temperature:40 C, (P1: Rf = 4.159 min, P2: Rf = 4.831 min).

### Step 5. Preparation of compound 1-[4-[1-(4-chlorophenyl)-1-methyl-prop-2-ynyl]thiazol-2-yl]-3-(2,2-dideuterio-2-hydroxy-ethyl)urea

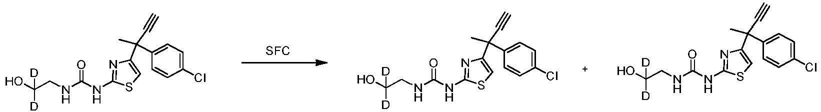

The compound obtained from step 4 above ((90 mg, 255.79 umol) was separated by SFC (column: DAICEL CHIRALPAK IG (250mm*30mm, 10um); mobile phase: [0.1%NH3H2O ETOH];B%: 40%-40%,min). Chiral isomers 1 (26.85 mg, yield: 29.8%) was obtained as a white solid.

¹H NMR (400MHz, CDCl₃) *δ* 7.34-7.28 (m, 2H), 7.22 - 7.19 (m, 2H), 6.69 (s, 1H), 3.23 (d, *J =* 5.5 Hz, 2H), 2.48 (s, 1H), 1.84 (s, 3H). MS (ESI) *m*/*z* (M+H)⁺=351.9. SFC Rf = 4.151 min.

Chiral isomers 2 (27.90 mg, yield: 31.0%) was obtained as a white solid.

¹H NMR (400MHz, CDCl₃) *δ* 7.43 - 7.35 (m, 2H), 7.31 - 7.27 (m, 2H), 6.76 (s, 1H), 3.31 (d, *J =* 5.5 Hz, 2H), 2.55 (s, 1H), 1.92 (s, 3H). MS (ESI) *m*/*z* (M+H)⁺=351.9. SFC: Rf = 4.815 min.

### General Method A

Carboxylic acids (1 equiv), EDCI (2-2.5 equiv), with or without HOBt (2 equiv) and DIEA (3 equiv)/pyridine/DMAP were dissolved in THF/DMF and stirred for 15-30 min at RT. Amine (1 equiv) was then added in one portion and the reaction was stirred at RT to 70°C for 2-16 hours. Once the reaction was completed, the resulting suspension was diluted with organic solvent and washed with brine and then dried. After filtration and evaporation, the resulting residue was purified by trituration/Prep-TLC/chromatography/Prep-HPLC to give the product.

### Example 20: Preparation of compound 4-((2-hydroxyethyl)amino)-N-(4-(2-(4-methoxyphenyl)propan-2-yl)thiazol-2-yl)benzamide

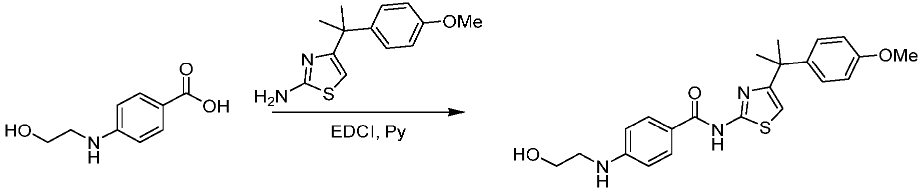

To a solution of 4-((2-hydroxyethyl)amino)benzoic acid (200 mg, 1.10 mmol) and 4-[1-(4-methoxyphenyl)-1-methyl-ethyl]thiazol-2-amine (261.98 mg, 919.85 umol, HCl) in Py (8 mL) was added EDCI (440.84 mg, 2.30 mmol). The mixture was stirred at 70 °C for 16 hr. The reaction mixture was concentrated to give a residue. The residue was purified by prep-HPLC (column: Agela ASB 150 x 25mm x 5um; mobile phase: [water (0.05% HCl)-ACN]; B%: 48%-78%, 10min). The desired compound (52 mg, yield: 13.57%) was obtained as a pale yellow solid.

¹H NMR (400MHz, DMSO-*d*₆) *δ* 12.06 (br s, 1H), 7.87 (d, *J =* 8.8 Hz, 2H), 7.12 (d, *J =* 8.8 Hz, 2H), 6.86 (s, 1H), 6.82 (d, *J =* 8.8 Hz, 2H), 6.62 (d, *J =* 8.8 Hz, 2H), 3.70 (s, 3H), 3.54 (t, *J* = 5.9 Hz, 2H), 3.16 (t, *J =* 5.9 Hz, 2H), 1.62 (s, 6H). MS (ESI) *m*/*z* (M+H)⁺=412.5.

### General Method B

The acid chloride was obtained by using SOCl₂ in appropriate solvent like DCM. To the acis chloride solution TEA or pyridine (3 equiv) a and mine (1 equiv) in DCM were added slowly at 0 °C under N₂, and further stirred for 0.5-2 h at RT. Once the reaction was completed, it was quenched with H₂O, extracted by EA and washed with brine then dried (Na₂SO₄), filtered and evaporated to dryness. The resulting residue was purified by trituration/Prep-TLC/chromatography/Prep-HPLC to give the product.

### Example 21

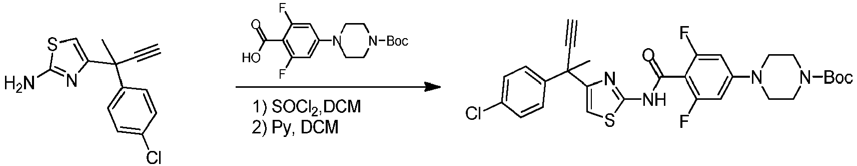

To a solution of 4-(4-tert-butoxycarbonylpiperazin-1-yl)-2,6-difluoro-benzoic acid (150 mg, 438.16 umol) in DCM (6 mL) was added SOCl₂ (31.8 uL, 438.16 umol). The mixture was stirred at 25 °C for 1 hr. The Py (176.74 uL, 2.19 mmol) was added and the reaction was stirred at 25 °C for 5 min , then 4-[1-(4-chlorophenyl)-1-methyl-prop-2-ynyl]thiazol-2-amine (115.07 mg, 437.94 umol) was added and the mixture was stirred at 25 °C for 16 hr. The reaction mixture was concentrated to give a residue. The residue was purified by flash silica gel chromatography (PE: EA=1:0 to 1:1). The desired compound (152 mg, yield: 54.4%) was obtained as a colorless oil.

MS (ESI) *m*/*z* (M+H)⁺ = 587.1.

### Example 22:Preparation of compound N-(4-(2-(4-chlorophenyl)but-3-yn-2-yl)-1H-imidazol-2-yl)-2,6-difluoro-4-(piperazin-1-yl)benzamide

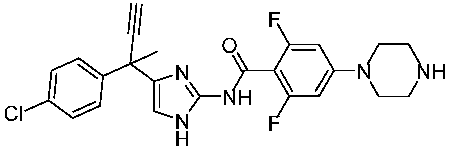

tep 1. Preparation of compound methyl 2-(4-chlorophenyl)-2-(imidazo[1,2-a]pyrimidin-2-yl)propanoate

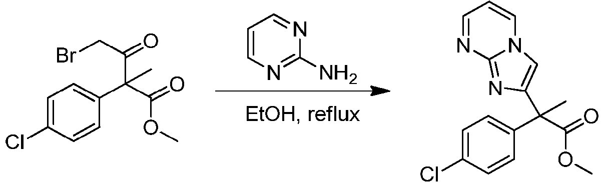

A mixture of pyrimidin-2-amine (1.0 g, 10.5 mmol) and methyl 4-bromo-2-(4-chlorophenyl)-2-methyl-3-oxo-butanoate (3.36 g, 10.5 mmol) in EtOH (20 mL) was stirred at 80 °C for 16h. The reaction was concentrated under reduced pressure and diluted with CH₂Cl₂ (40 mL) and sat. aq NaHCO₃ (20 mL), and the water phase was extracted with CH₂Cl₂ (3 x 30 mL). The combined organic layers were washed with sat. aq NaHCO₃ (2 x 20 mL), dried over Na₂SO₄, filtered and concentrated under reduced pressure. The residue was purified by column chromatography (SiO₂, **Petroleum ether/Ethyl acetate=1/0** to 0/1) to afford methyl 2-(4-chlorophenyl)-2-imidazo[1,2-a]pyrimidin-2-yl-propanoate (1.48 g, yield: 40.1%) as a white solid.

MS (ESI) *m*/*z* (M+H)⁺=316.0.

Step 2. Preparation of compound methyl 2-(2-amino-1H-imidazol-4-yl)-2-(4-chlorophenyl)propanoate

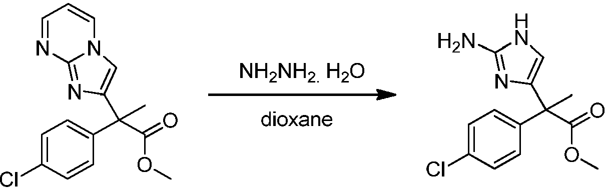

To a solution of methyl 2-(4-chlorophenyl)-2-imidazo[1,2-a]pyrimidin-2-yl-propanoate (600 mg, 1.90 mmol) in dioxane (5 mL) was added NH₂NH₂·H₂O (650 mg, 11.04 mmol, 85% purity). After addition, the reaction mixture was stirred at 80 °C for 16h. The reaction mixture was concentrated in vacuum. The residue was purified by column chromatography (SiO₂, **DCM: MeOH** = 100/**1-10/1**) to afford methyl 2-(2-amino-1H-imidazol-4-yl)-2-(4-chlorophenyl)propanoate (60 mg, yield: 33.9%) as white solid.

MS (ESI) *m*/*z* (M+H)⁺ = 280.1.

¹H NMR (400MHz, CD₃OD) *δ* 7.29 (d, *J =* 8.4 Hz, 2H), 7.20 (d, *J =* 8.4 Hz, 2H), 6.32 (s, 1H), 3.72 (s, 3H), 1.79 (s, 3H).

Step 3. Preparation of compound tert-butyl 4-(4-((4-(2-(4-chlorophenyl)-1-methoxy-1-oxopropan-2-yl)-1H-imidazol-2-yl)carbamoyl)-3,5-difluorophenyl)piperazine-1-carboxylate

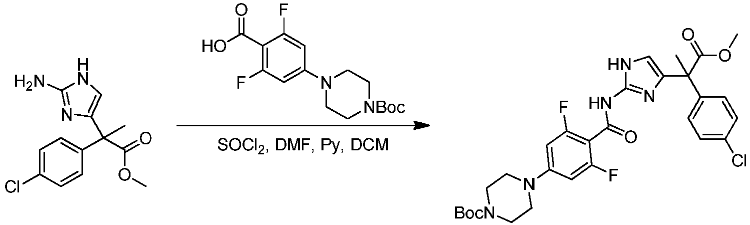

SOCl₂ (92 mg, 772 umol) was added to a solution of 4-(4-tert-butoxycarbonylpiperazin-1-yl)-2,6-difluoro-benzoic acid (220 mg, 644 umol) in DCM (10 mL), then DMF (13 mg, 172 umol) was added and the reaction mixture was stirred at 25 °C for 1 h, followed by Py (204 mg, 2.57 mmol) was added into the reaction mixture and stirred at 25 °C for 10 min, then methyl 2-(2-amino-1H-imidazol-4-yl)-2-(4-chlorophenyl)propanoate (120 mg, 429 umol) was added into the reaction mixture and stirred at 25 °C for 16 h. The reaction mixture was washed with sat. NaHCO₃ (5 mL), brine (5 mL), dried over Na₂SO₄, filtered and concentrated in vacuum. The residue was purified by column chromatography (SiO₂, **Petroleum ether/Ethyl acetate=10/1** to **2/1**) to afford tert-butyl 4-[4-[[4-[1-(4-chlorophenyl)-2-methoxy-1-methyl-2-oxo-ethyl]-1H-imidazol-2-yl]carbamoyl]-3,5-difluorophenyl]piperazine-1-carboxylate (120 mg, yield: 38.9% yield, 84% purity) as colorless gum.

MS (ESI) *m*/*z* (M+H)⁺ = 604.1.

Step 4. Preparation of compound tert-butyl 4-(4-((4-(2-(4-chlorophenyl)-1-hydroxypropan-2-yl)-1H-imidazol-2-yl)carbamoyl)-3,5-difluorophenyl)piperazine-1-carboxylate

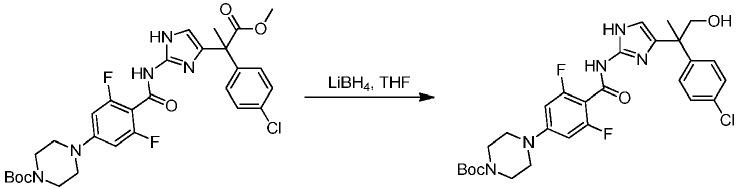

To a solution of tert-butyl 4-[4-[[4-[1-(4-chlorophenyl)-2-methoxy-1-methyl-2-oxoethyl]-1H-imidazol-2-yl]carbamoyl]-3,5-difluoro-phenyl]piperazine-1-carboxylate (120 mg, 199 umol) in THF (8 mL) was added LiBH₄ (4 M, 248 uL) at 0 °C. After addition, the reaction mixture was stirred at 25 °C for 16h. The reaction mixture was poured into 5 mL of sat. NH₄Cl and extracted with EtOAc (8 mL x 2), the extracts was washed with water (8 mL x 3), brine (8 mL), dried over Na₂SO₄, filtered and concentrated in vacuum to afford tert-butyl 4-[4-[[4-[1-(4-chlorophenyl)-2-hydroxy-1-methyl-ethyl]-1H-imidazol-2-yl]carbamoyl]-3,5-difluorophenyl]piperazine-1-carboxylate (110 mg, crude) as light brown gum which was used in next step without any purification.

MS (ESI) *m*/*z* (M+H)⁺ = 576.1.

### Step 5. Preparation of compound tert-butyl 4-(4-((4-(2-(4-chlorophenyl)-1-oxopropan-2-yl)-1H-imidazol-2-yl)carbamoyl)-3,5-difluorophenyl)piperazine-1-carboxylate

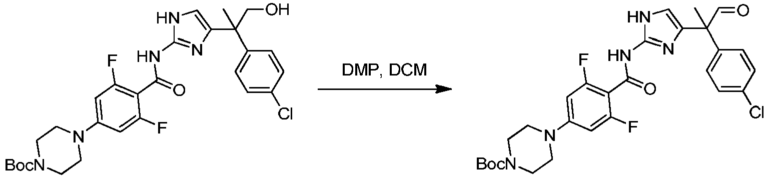

To a solution of tert-butyl 4-[4-[[4-[1-(4-chlorophenyl)-2-hydroxy-1-methyl-ethyl]-1H-imidazol-2-yl]carbamoyl]-3,5-difluoro-phenyl]piperazine-1-carboxylate (170 mg, 295 umol) in DCM (10 mL) was added DMP (500 mg, 1.18 mmol), after addition, the reaction mixture was stirred at 25 °C for 4h. The reaction mixture was diluted with DCM (10 mL), washed with sat. NaHCO₃ / sat. Na₂S₂O₃ (10 mL / 10 mL) for 3 times, then washed with brine (10 mL), dried over Na₂SO₄, filtered and concentrated in vacuum to afford tert-butyl 4-[4-[[4-[1-(4-chlorophenyl)-1-methyl-2-oxo-ethyl]-1H-imidazol-2-yl]carbamoyl]-3,5-difluorophenyl]piperazine-1-carboxylate (150 mg, crude) as light brown gum which was used in next step without any purification.

### Step 6. Preparation of compound tert-butyl 4-(4-((4-(2-(4-chlorophenyl)but-3-yn-2-yl)-1H-imidazol-2-yl)carbamoyl)-3,5-difluorophenyl)piperazine-1-carboxylate

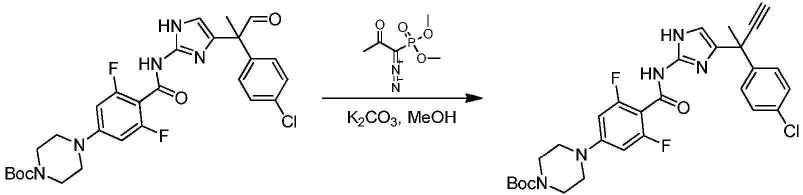

To a solution of tert-butyl 4-[4-[[4-[1-(4-chlorophenyl)-1-methyl-2-oxo-ethyl]-1H-imidazol-2-yl]carbamoyl]-3,5-difluoro-phenyl]piperazine-1-carboxylate (150 mg, 261 umol) and 1-diazo-1-dimethoxyphosphoryl-propan-2-one (75.3 mg, 392 umol) in MeOH (8 mL) was added K₂CO₃ (72.2 mg, 522.63 umol). After addition, the reaction mixture was stirred 25 °C for 16h. The reaction mixture was concentrated in vacuum and the residue was diluted with 10 mL of water and extracted with EtOAc (10 mL x 2), the combined extracts was washed with brine (10 mL), dried over Na₂SO₄, filtered and concentrated in vacuum. The residue was purified by prep-HPLC (**FA condition**) to afford tert-butyl 4-[4-[[4-[1-(4-chlorophenyl)-1-methyl-prop-2-ynyl]-1H-imidazol-2-yl]carbamoyl]-3,5-difluorophenyl]piperazine-1-carboxylate (12 mg, yield: 8.1%) as off-white solid.

### Step 7. Preparation of compound N-(4-(2-(4-chlorophenyl)but-3-yn-2-yl)-1H-imidazol-2-yl)-2,6-difluoro-4-(piperazin-1-yl)benzamide

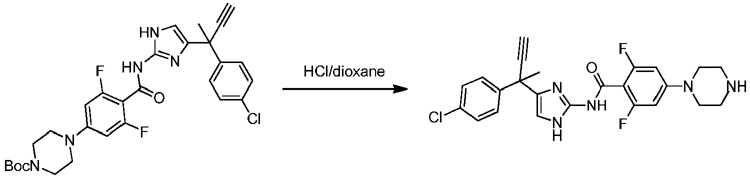

To a solution of tert-butyl 4-[4-[[4-[1-(4-chlorophenyl)-1-methyl-prop-2-ynyl]-1H-imidazol-2-yl]carbamoyl]-3,5-difluoro-phenyl]piperazine-1-carboxylate (12 mg, 21.05 umol) in MeOH (0.3 mL) was added HCl/dioxane (4 M, 900 uL). After addition, the reaction mixture was stirred at 25°C for 1h. The reaction mixture was concentrated in vacuum. The residue was purified by prep-HPLC (**TFA condition**) to afford N-[4-[1-(4-chlorophenyl)-1-methyl-prop-2-ynyl]-1H-imidazol-2-yl]-2,6-difluoro-4-piperazin-1-yl-benzamide (5 mg, yield: 40.2% yield, 2HCl salt) as light brown solid.

MS (ESI) *m*/*z* (M+Na)⁺= 492.3.

¹H NMR (400MHz, CD₃OD) *δ* 7.52 (br d, *J =* 8.4 Hz, 2H), 7.40 (br d, *J =* 8.4 Hz, 2H), 7.22 (s, 1H), 6.76 (br d, *J =* 12.4 Hz, 2H), 3.65 - 3.59 (m, 4H), 3.39 - 3.32 (m, 4H), 3.20 (s, 1H), 1.97 (s, 3H).

### Example 23: Preparation of N-(4-(2-(4-chlorophenyl)but-3-yn-2-yl)oxazol-2-yl)-2,6-difluoro-4-(piperazin-1-yl)benzamide

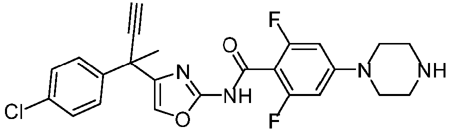

Step 1. Preparation of compound methyl 2-(2-aminooxazol-4-yl)-2-(4-chlorophenyl)propanoate

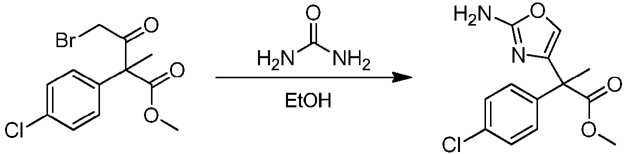

To a solution of methyl 4-bromo-2-(4-chlorophenyl)-2-methyl-3-oxobutanoate (1.00 g, 3.13 mmol) in EtOH (30 mL) was added urea (282 mg, 4.69 mmol) and the mixture was stirred at 80 °C for 20 h. The reaction mixture was concentrated under reduced pressure. The residue was purified by column chromatography (SiO₂, **Petroleum ether/Ethyl acetate=1/0** to 3/1) to afford methyl 2-(2-aminooxazol-4-yl)-2-(4-chlorophenyl)propanoate (50.0 mg, yield: 4.2%) as a yellow solid.

MS (ESI) *m*/*z* (M+H)⁺=281.1.

¹H NMR (400MHz, CD₃OD) *δ* 7.31 (s, 4H), 6.98 (s, 1H), 3.71 (s, 3H), 1.85 - 1.79 (m, 3H).

### Step 2. Preparation of compound tert-butyl 4-(4-((4-(2-(4-chlorophenyl)-1-methoxy-1-oxopropan-2-yl)oxazol-2-yl)carbamoyl)-3,5-difluorophenyl)piperazine-1-carboxylate

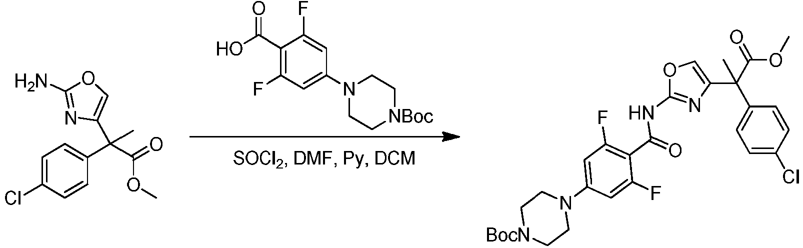

SOCl₂ (38.0 mg, 321 umol) was added to a solution of 4-(4-tert-butoxycarbonylpiperazin-1-yl)-2,6-difluoro-benzoic acid (91.0 mg, 267 umol) in DCM (5 mL). DMF (5.0 mg, 71.3 umol) was added and the reaction mixture was stirred at 25 °C for 1 h. Then Py (85.0 mg, 1.07 mmol) was added to the above reaction mixture and stirred at 25 °C for 10 min, then methyl 2-(2-aminooxazol-4-yl)-2-(4-chlorophenyl)propanoate (50.0 mg, 178 umol) was added into the reaction mixture and stirred at 25 °C for 16 h. The reaction mixture was washed with sat.NaHCO₃ (3 mL), brine (3 mL), dried over Na₂SO₄, filtered and concentrated in vacuum. The residue was purified by column chromatography (SiO₂, **Petroleum ether/Ethyl acetate=10/1** to **2/1**) to afford tert-butyl 4-[4-[[4-[1-(4-chlorophenyl)-2-methoxy-1-methyl-2-oxo-ethyl]oxazol-2-yl]carbamoyl]-3,5-difluoro-phenyl]piperazine-1-carboxylate (42.0 mg, yield: 30%) as colorless gum.

MS (ESI) *m*/*z* (M+H)⁺=605.1.

### Step 3. Preparation of compound tert-butyl 4-(4-((4-(2-(4-chlorophenyl)-1-hydroxypropan-2-yl)oxazol-2-yl)carbamoyl)-3,5-difluorophenyl)piperazine-1-carboxylate

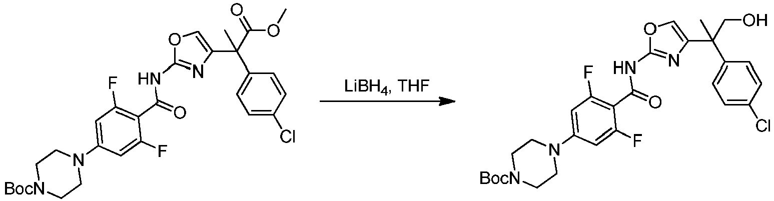

To a solution of LiBH₄ (4 M, 83 uL) in THF (3 mL) was added a solution of tert-butyl 4-[4-[[4-[1-(4-chlorophenyl)-2-methoxy-1-methyl-2-oxo-ethyl]oxazol-2-yl]carbamoyl]-3,5-difluoro-phenyl]piperazine-1-carboxylate (40.0 mg) in THF (2 mL) at 0 °C under N₂ atmosphere. After addition, the reaction mixture was stirred at 25 °C for 2h. The reaction mixture was poured into 5 mL of sat. NH₄Cl and extracted with EtOAc (8 mL x 2), the extracts was washed with water (8 mL x 3), brine (8 mL), dried over Na₂SO₄, filtered and concentrated in vacuum to afford tert-butyl 4-[4-[[4-[1-(4-chlorophenyl)-2-hydroxy-1-methyl-ethyl]oxazol-2-yl]carbamoyl]-3,5-difluoro-phenyl]piperazine-1-carboxylate (28.0 mg, crude) as white solid which was used in next step without any purification.

### Step 4. Preparation of compound tert-butyl 4-(4-((4-(2-(4-chlorophenyl)-1-oxopropan-2-yl)oxazol-2-yl)carbamoyl)-3,5-difluorophenyl)piperazine-1-carboxylate

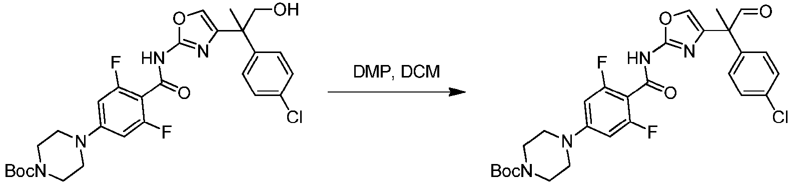

To a solution of tert-butyl 4-[4-[[4-[1-(4-chlorophenyl)-2-hydroxy-1-methylethyl]oxazol-2-yl]carbamoyl]-3,5-difluoro-phenyl]piperazine-1-carboxylate (28.0 mg, crude) in DCM (5 mL) was added DMP (41.0 mg, 97.05 umol), after addition, the reaction mixture was stirred at 25 °C for 3h. The reaction mixture was diluted with DCM (10 mL), washed with sat.NaHCO₃ / Na₂S₂O₃ (10 mL / 10 mL) for 3 times, then brine (10 mL), dried over Na₂SO₄, filtered and concentrated in vacuum to afford tert-butyl 4-[4-[[4-[1-(4-chlorophenyl)-1-methyl-2-oxo-ethyl]oxazol-2-yl]carbamoyl]-3,5-difluoro-phenyl]piperazine-1-carboxylate (30.0 mg, crude) as light brown solid which was used in next step without any purification.

MS (ESI) *m*/*z* (M+H)⁺=575.1.

### Step 5. Preparation of compound tert-butyl 4-(4-((4-(2-(4-chlorophenyl)but-3-yn-2-yl)oxazol-2-yl)carbamoyl)-3,5-difluorophenyl)piperazine-1-carboxylate

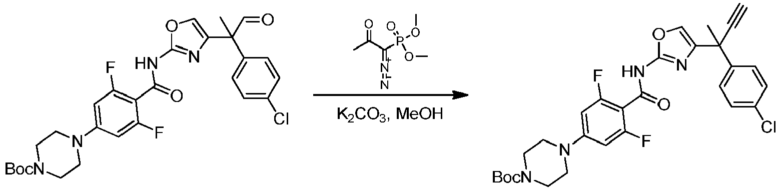

To a solution of tert-butyl 4-[4-[[4-[1-(4-chlorophenyl)-1-methyl-2-oxo-ethyl]oxazol-2-yl]carbamoyl]-3,5-difluoro-phenyl]piperazine-1-carboxylate (30.0 mg, crude) and 1-diazo-1-dimethoxyphosphoryl-propan-2-one (15.0 mg, 78.3 umol) in MeOH (4 mL) was added K₂CO₃ (14.0 mg, 104 umol). After addition, the reaction mixture was stirred 25 °C for 16h. The reaction mixture was concentrated in vacuum, the residue was diluted with H₂O (5 mL), extracted with EtOAc (5 mL x 3), the combined extracts was washed with brine (5 mL), dried over Na₂SO₄, filtered and concentrated in vacuum to afford tert-butyl 4-[4-[[4-[1-(4-chlorophenyl)-1-methyl-prop-2-ynyl]oxazol-2-yl]carbamoyl]-3,5-difluoro-phenyl]piperazine-1-carboxylate (28.0 mg, crude) as light brown gum.

MS (ESI) *m*/*z* (M+H)⁺=571.1.

### Step 6. Preparation of compound N-(4-(2-(4-chlorophenyl)but-3-yn-2-yl)oxazol-2-yl)-2,6-difluoro-4-(piperazin-1-yl)benzamide

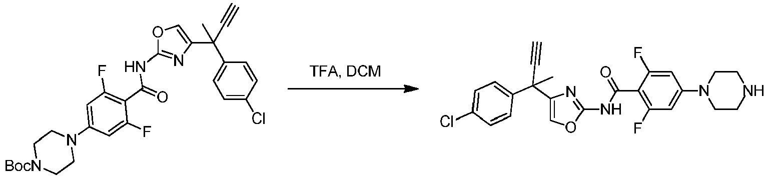

To a solution of tert-butyl 4-[4-[[4-[1-(4-chlorophenyl)-1-methyl-prop-2-ynyl]oxazol-2-yl]carbamoyl]-3,5-difluoro-phenyl]piperazine-1-carboxylate (25.0 mg, crude) in DCM (2 mL) was added TFA (2 mL). After addition, the reaction mixture was stirred at 25 °C for 1h. The reaction mixture was concentrated in vacuum. The residue was purified by prep-HPLC (FA **condition**) to afford N-[4-[1-(4-chlorophenyl)-1-methyl-prop-2-ynyl]oxazol-2-yl]-2,6-difluoro-4-piperazin-1-yl-benzamide (7.5 mg, yield: 28.7%, TFA salt) as light brown solid.

MS (ESI) *m*/*z* (M+H)⁺=471.3.

¹H NMR (400MHz, CD₃OD) δ 7.64 (s, 1H), 7.58 (br d, *J* = 8.0 Hz, 2H), 7.39 - 7.29 (m, 2H), 6.72 (br d, *J =* 11.8 Hz, 2H), 3.65 - 3.54 (m, 4H), 3.42 - 3.35 (m, 4H), 2.98 (s, 1H), 1.90 (s, 3H).

### General Method C

Carboxylic acids (1 equiv), HATU (1.2 equiv) or HBTU or PyBOP, and TEA or DIEA (3 equiv.) were dissolved in appropriate organic solvent, like THF or DMF and stirred for 15-30 min at RT. Amine (1-1.5 equiv.) was then added in one portion and the reaction was stirred at RT-100°C for 4-16 hours. Once the reaction was completed, the resulting suspension was diluted with organic solvent and washed with brine and then dried. After filtration and evaporation, the resulting residue was purified by trituration/Prep-TLC/chromatography/Prep-HPLC to give the product.

### Example 24:Preparation of compound methyl N-(4-(2-(4-bromophenyl)but-3-yn-2-yl)thiazol-2-yl)-3-((tert-butyldiphenylsilyl)oxy)cyclobutane-1-carboxamide

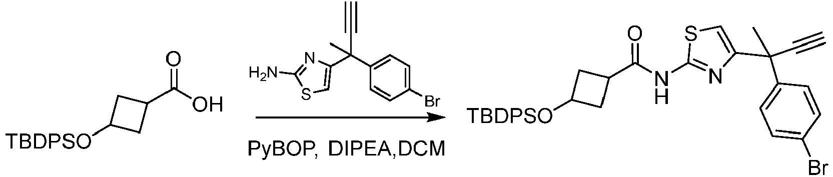

To a solution of 3-[tert-butyl(diphenyl)silyl]oxycyclobutanecarboxylic acid (1.36 g, 3.84 mmol), in DCM (10 mL) was added PyBOP (2.00 g, 3.84 mmol) at 25°C, After stirred for 10 min, methyl 2-(2-aminothiazol-4-yl)-2-(4-bromophenyl)propanoate (523.61 mg, 1.53 mmol) and DIPEA (594.97 mg, 4.60 mmol) was added at 25°C and the mixture was stirred for 12 h at 25 °C. The mixture was diluted with DCM (30 mL), washed with H2O (10 mL), brine (10 mL), dired over anhydrous Na2SO4, filtered and concentrated in vacuum. The obtained residue was purified by silica column (ethyl acetate in petroleum ether =0-25%). The desired compound (1.4 g, crude) was obtained as yellow oil. MS (ESI) *m*/*z* (M+H)⁺=643.1

**Table 5. The following examples were synthesized analogous to the procedure of example 20, 21 and 24 using the appropriate intermediates and the corresponding fragment**

| Com. ID | Structure | Name | HNMR | LCMS ([M+H]⁺ =) | Kinase assay IC50 nM | NFkB assay IC50 nM |
|---|---|---|---|---|---|---|
| B001 | 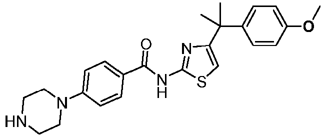 | N-(4-(2-(4-methoxyphenyl)propan-2-yl)thiazol-2-yl)-4-(piperazin-1-yl)benzamide | ¹H NMR (400 MHz, CD₃OD) *δ* 8.75 -8.74 (m, 1 H), 8.43 - 8.40 (m, 1 H), 7.44 - 7.42 (m, 2 H), 7.33 - 7.31 (m, 1 H), 7.22 - 7.20 (m, 2 H), 6.98 (s, 1 H), 4.08 - 4.06 (m, 4 H), 3.44 - 3.42 (m, 4 H), 1.72 (s, 6 H). | 437.0 | 123 | 0.75 |
| B002 | 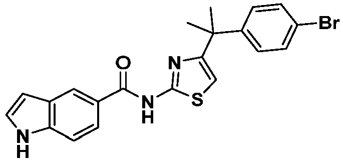 | N-(4-(2-(4-bromophenyl)propan-2-yl)thiazol-2-yl)-1H-indole-5-carboxamide | ¹H NMR (400 MHz, CDCl₃) *δ* 8.42 (s, 1H), 8.25 (s, 1H), 7.75 (dd, J = 8.6, 1.8 Hz, 1H), 7.44 (d, J = 8.6 Hz, 1H), 7.41 - 7.36 (m, 2H), 7.31 - 7.27 (m, 1H), 7.15 - 7.11 (m, 2H), 6.68 (s, 1H), 6.65 (s, 1H), 1.67 (d, J = 6.9 Hz, 6H). | 442.0 | 206 | 2.33 |
| B003 | 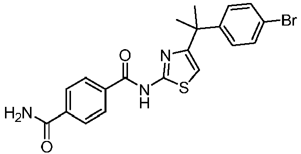 | N-(4-(2-(4-bromophenyl)propan-2-yl)thiazol-2-yl)terephthalamide | | 444.0 | 192 | |
| B004 | 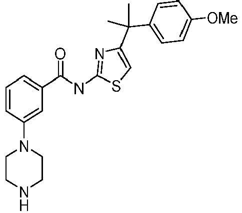 | N-(4-(2-(4-methoxyphenyl)propan-2-yl)thiazol-2-yl)-3-(piperazin-1-yl)benzamide | | 437.0 | 211 | 2.84 |
| B005 | 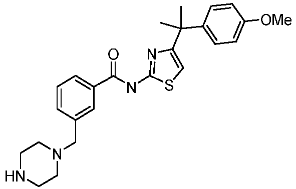 | N-(4-(2-(4-methoxyphenyl)propan-2-yl)thiazol-2-yl)-3-(piperazin-1-ylmethyl)benzamide | | 451.0 | 215 | 1.61 |
| B006 | 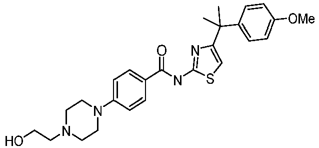 | 4-(4-(2-hydroxyethyl)piperazin-1-yl)-N-(4-(2-(4-methoxyphenyl)propan-2-yl)thiazol-2-yl)benzamide | | 481 | 595 | 2.71 |
| B007 | 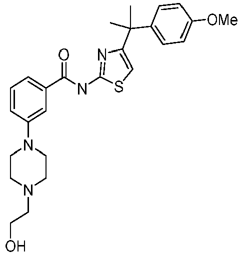 | 3-(4-(2-hydroxyethyl)piperazin-1-yl)-N-(4-(2-(4-methoxyphenyl)propan-2-yl)thiazol-2-yl)benzamide | | 481.0 | 230 | 1.17 |
| B008 | 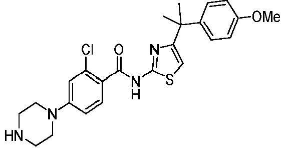 | 2-chloro-N-(4-(2-(4-methoxyphenyl)propan-2-yl)thiazol-2-yl)-4-(piperazin-1-yl)benzamide | | 471.0 | 37 | 0.37 |
| B009 | 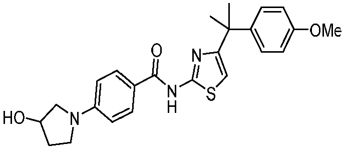 | 4-(3-hydroxypyrrolidin-1-yl)-N-(4-(2-(4-methoxyphenyl)propan-2-yl)thiazol-2-yl)benzamide | | 438.0 | 951 | 0.78 |
| B010 | 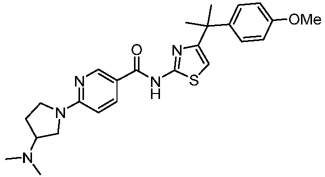 | 6-(3-(dimethylamino)pyrrolidin-1-yl)-N-(4-(2-(4-methoxyphenyl)propan-2-yl)thiazol-2-yl)nicotinamide | | 466.0 | 187 | 0.84 |
| B011 | 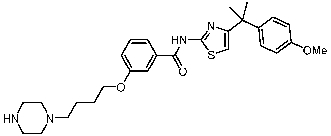 | N-(4-(2-(4-methoxyphenyl)propan-2-yl)thiazol-2-yl)-3-(4-(piperazin-1-yl)butoxy)benzamide | | 509.0 | 230 | 1.64 |
| B012 | 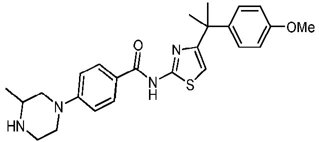 | N-(4-(2-(4-methoxyphenyl)propan-2-yl)thiazol-2-yl)-4-(3-methylpiperazin-1-yl)benzamide | | 451.0 | 118 | 0.76 |
| B013 | 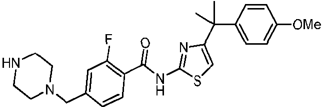 | 2-fluoro-N-(4-(2-(4-methoxyphenyl)propan-2-yl)thiazol-2-yl)-4-(piperazin-1-ylmethyl)benzamide | | 469.0 | 111 | 1.02 |
| B014 | 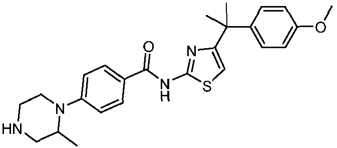 | tert-butyl 4-(4-((4-(2-(4-methoxyphenyl)propan-2-yl)thiazol-2-yl)carbamoyl)phenyl)-3-methylpiperazine-1-carboxylate | | 451.0 | 300 | 1.17 |
| B015 | 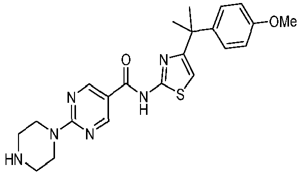 | N-(4-(2-(4-methoxyphenyl)propan-2-yl)thiazol-2-yl)-2-(piperazin-1-yl)pyrimidine-5-carboxamide | | 439.1 | 161 | 4.67 |
| B016 | 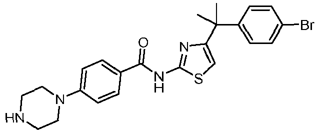 | N-(4-(2-(4-bromophenyl)propan-2-yl)thiazol-2-yl)-4-(piperazin-1-yl)benzamide | | 485.1 | 60 | |
| B017 | 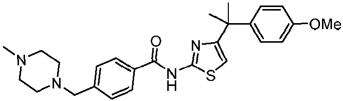 | N-(4-(2-(4-methoxyphenyl)propan-2-yl)thiazol-2-yl)-4-((4-methylpiperazin-1-yl)methyl)benzamide | | 465.1 | 148 | 3.76 |
| B018 | 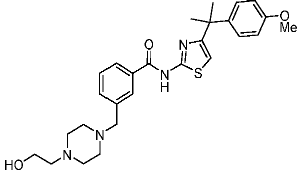 | 3-((4-(2-hydroxyethyl)piperazin-1-yl)methyl)-N-(4-(2-(4-methoxyphenyl)propan-2-yl)thiazol-2-yl)benzamide | | 495.1 | 187 | 1.64 |
| B019 | 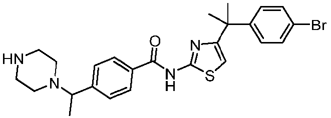 | N-(4-(2-(4-bromophenyl)propan-2-yl)thiazol-2-yl)-4-(1-(piperazin-1-yl)ethyl)benzamide | | 513.1 | 98 | 1.27 |
| B020 | 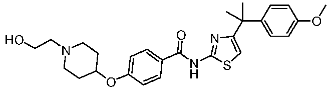 | 4-((1-(2-hydroxyethyl)piperidin-4-yl)oxy)-N-(4-(2-(4-methoxyphenyl)propan-2-yl)thiazol-2-yl)benzamide | | 496.1 | 170 | |
| B021 | 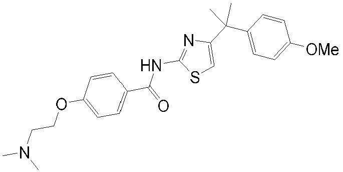 | 4-(2-(dimethylamino)ethoxy)-N-(4-(2-(4-methoxyphenyl)propan-2-yl)thiazol-2-yl)benzamide | | 440.1 | 83 | 1.48 |
| B022 | 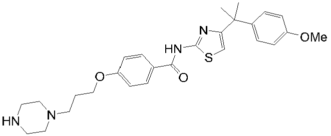 | N-(4-(2-(4-methoxyphenyl)propan-2-yl)thiazol-2-yl)-4-(3-(piperazin-1-yl)propoxy)benzamide | | 495.0 | 294 | 0.55 |
| B023 | 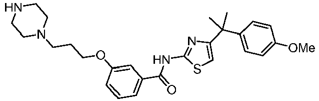 | N-(4-(2-(4-methoxyphenyl)propan-2-yl)thiazol-2-yl)-3-(3-(piperazin-1-yl)propoxy)benzamide | | 495.0 | 150 | 6.15 |
| B024 | 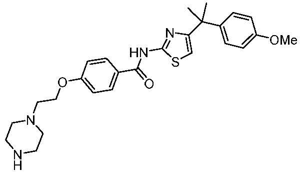 | N-(4-(2-(4-methoxyphenyl)propan-2-yl)thiazol-2-yl)-4-(2-(piperazin-1-yl)ethoxy)benzamide | | 481.1 | 116 | 4.29 |
| B025 | 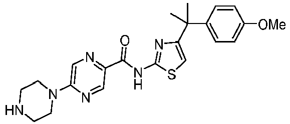 | N-(4-(2-(4-methoxyphenyl)propan-2-yl)thiazol-2-yl)-5-(piperazin-1-yl)pyrazine-2-carboxamide | | 439.1 | 298 | 3.59 |
| B026 | 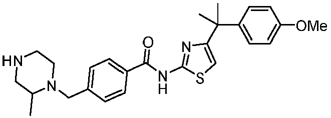 | N-(4-(2-(4-methoxyphenyl)propan-2-yl)thiazol-2-yl)-4-((2-methylpiperazin-1-yl)methyl)benzamide | | 465.0 | 127 | 0.61 |
| B027 | 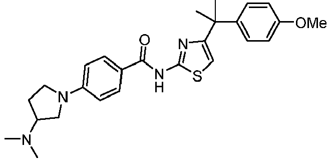 | 4-(3-(dimethylamino)pyrrolidin-1-yl)-N-(4-(2-(4-methoxyphenyl)propan-2-yl)thiazol-2-yl)benzamide | | 465.0 | 219 | 3.38 |
| B028 | 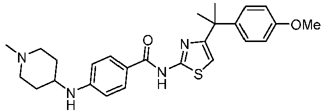 | N-(4-(2-(4-methoxyphenyl)propan-2-yl)thiazol-2-yl)-4-((1-methylpiperidin-4-yl)amino)benzamide | | 465.0 | 271 | 2.36 |
| B029 | 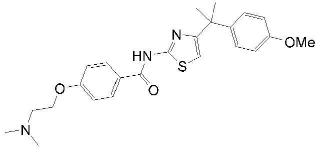 | 4-(2-(dimethylamino)ethoxy)-N-(4-(2-(4-methoxyphenyl)propan-2-yl)thiazol-2-yl)benzamide | | 440.0 | 83 | 1.48 |
| B030 | 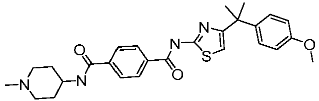 | N 1-(4-(2-(4-methoxyphenyl)propan-2-yl)thiazol-2-yl)-N4-(1-methylpiperidin-4-yl)terephthalamide | | 493.0 | 155 | 1.98 |
| B031 | 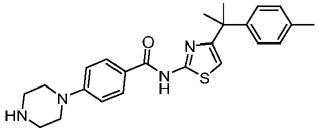 | 4-(piperazin-1-yl)-N-(4-(2-(p-tolyl)propan-2-yl)thiazol-2-yl)benzamide | | 421.0 | 37 | 0.51 |
| B032 | 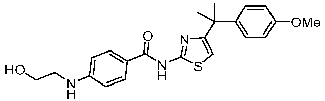 | 4-((2-hydroxyethyl)amino)-N-(4-(2-(4-methoxyphenyl)propan-2-yl)thiazol-2-yl)benzamide | | 412.0 | 280 | 3.81 |
| B033 | 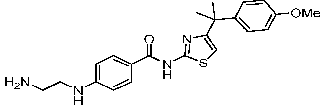 | 4-((2-aminoethyl)amino)-N-(4-(2-(4-methoxyphenyl)propan-2-yl)thiazol-2-yl)benzamide | | 411.0 | 247 | 6.45 |
| B034 | 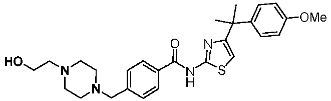 | 4-((4-(2-hydroxyethyl)piperazin-1-yl)methyl)-N-(4-(2-(4-methoxyphenyl)propan-2-yl)thiazol-2-yl)benzamide | | 495.0 | 44 | 0.95 |
| B035 | 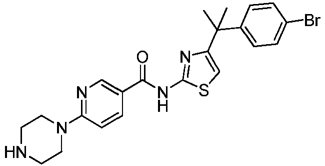 | N-(4-(2-(4-bromophenyl)propan-2-yl)thiazol-2-yl)-6-(piperazin-1-yl)nicotinamide | ¹H NMR (400 MHz, DMSO-*d*₆) *δ* 12.29 (br s, 1 H), 9.42 (br s, 2 H), 8.02 - 8.00 (m, 2 H), 7.13 - 7.11 (m, 1 H), 7.05 - 7.03 (m, 2 H), 6.90 (s, 1 H), 6.83 - 6.80 (m, 2 H), 3.69 (s, 3 H), 3.58 - 3.56 (m, 4 H), 3.17 - 3.16 (m, 4 H), 1.63 (s, 6 H). | 486.2 | 75 | 2.76 |
| B036 | 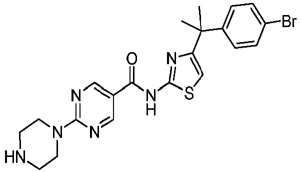 | N-(4-(2-(4-bromophenyl)propan-2-yl)thiazol-2-yl)-2-(piperazin-1-yl)pyrimidine-5-carboxamide | ¹H NMR (400MHz, DMSO-*d*₆) *δ* 12.51 (br s, 1H), 9.28 (br s, 2H), 9.00 (s, 2H), 7.49 - 7.41 (m, 2H), 7.19 - 7.12 (m, 2H), 7.04 (s, 1H), 4.13 - 3.99 (m, 4H), 3.21 - 3.15 (m, 4H), 1.64 (s, 6H). | 487.1 | 110 | 4.79 |
| B037 | 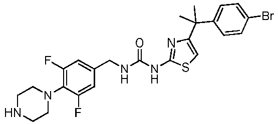 | 1-(4-(2-(4-bromophenyl)propan-2-yl)thiazol-2-yl)-3-(3,5-difluoro-4-(piperazin-1-yl)benzyl)urea | ¹H NMR (400MHz, CD3OD) *δ* 7.54 - 7.46 (m, 2H), 7.21 (d, *J =* 8.8 Hz, 2H), 7.12 (s, 1H), 6.97 (d, *J* = 9.8 Hz, 2H), 4.39 (s, 2H), 3.43 - 3.37 (m, 4H), 3.36 - 3.32 (m, 4H), 1.71 (s, 6H). | [M+Na]⁺ =574.1 | 23 | 1.68 |
| B038 | 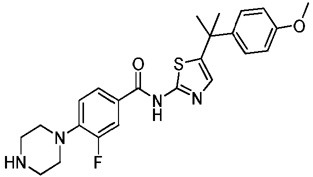 | 3-fluoro-N-(5-(2-(4-methoxyphenyl)propan-2-yl)thiazol-2-yl)-4-(piperazin-1-yl)benzamide | ¹H NMR (400 MHz, CD3OD) *δ* 7.86 (br d, J=8.56 Hz, 1 H), 7.78 (dd, J=13.57, 1.83 Hz, 1 H), 7.25 - 7.17 (m, 3 H), 7.07 (s, 1 H), 6.87 (d, J=8.80 Hz, 2 H), 3.76 (s, 3 H), 3.49 (br d, J=5.14 Hz, 4 H), 3.41 (br d, J=5.14 Hz, 4 H), 1.73 (s, 6 H). | 455.2 | 216 | 4.65 |
| B039 | 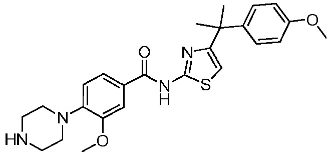 | 3-methoxy-N-(4-(2-(4-methoxyphenyl)propan-2-yl)thiazol-2-yl)-4-(piperazin-1-yl)benzamide | ¹H NMR (400MHz, DMSO-*d*₆) *δ* 7.65 (s, 2H), 7.12 (br d, *J =* 8.8 Hz, 2H), 6.93 - 6.87 (m, 2H), 6.82 (d, *J =* 8.8 Hz, 2H), 3.85 (s, 3H), 3.70 (s, 3H), 2.98 (br s, 4H), 2.82 (br s, 4H), 1.63 (s, 6H). | 467.1 | 123 | 2.97 |
| B040 | 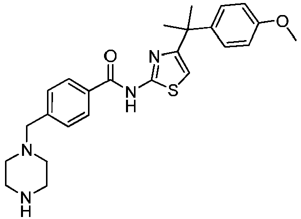 | N-(4-(2-(4-methoxyphenyl)propan-2-yl)thiazol-2-yl)-4-(piperazin-1-ylmethyl)benzamide | ¹H NMR (400MHz, DMSO-*d*₆) *δ* 12.67 (br s, 1H), 9.61 (br s, 1H), 8.59 (br d, *J =* 7.9 Hz, 1H), 8.13 (d, *J =* 8.2 Hz, 2H), 7.78 (br d, *J =* 8.2 Hz, 2H), 7.13 (d, *J =* 8.6 Hz, 2H), 6.98 (s, 1H), 6.82 (d, *J =* 8.6 Hz, 2H), 4.59 - 4.34 (m, 2H), 3.95 (br s, 8H), 3.70 (s, 3H), 3.49 - 3.43 (m, 2H), 1.64 (s, 6H) | 451.2 | 93 | 0.71 |
| B041 | 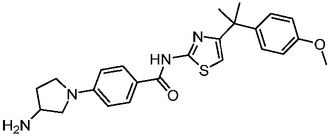 | 4-(3-aminopyrrolidin-1-yl)-N-(4-(2-(4-methoxyphenyl)propan-2-yl)thiazol-2-yl)benzamide | ¹H NMR (400MHz, DMSO-*d*₆) *δ* 11.79 (br s, 1H), 8.26 (br s, 3H), 8.01 (d, *J =* 9.0 Hz, 2H), 7.20 - 7.13 (m, 2H), 6.85 - 6.80 (m, 2H), 6.79 (s, 1H), 6.62 (d, *J =* 8.8 Hz, 2H), 4.05 - 3.90 (m, 1H), 3.73 (s, 3H), 3.62 - 3.54 (m, 2H), 3.48 - 3.38 (m, 2H), 2.42 - 2.35 (m, 1H), 2.20 - 2.13 (m, 1H), 1.66 (s, 6H). | 437.1 | 80 | 3.96 |
| B042 | 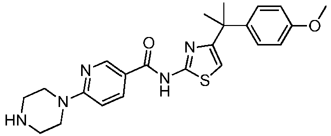 | N-(4-(2-(4-methoxyphenyl)propan-2-yl)thiazol-2-yl)-6-(piperazin-1-yl)nicotinamide | ¹H NMR (400 MHz, CD3OD) *δ* 8.78 (d, J=1.71 Hz, 1 H), 8.43 (br d, J=7.82 Hz, 1 H), 7.34 (br d, J=9.29 Hz, 1 H), 7.22 (d, J=8.80 Hz, 2 H), 7.06 (s, 1 H), 6.87 (d, J=8.80 Hz, 2 H), 4.09 (br s, 4 H), 3.76 (s, 3 H), 3.43 (br s, 4 H), 1.73 (s, 6 H). | 438.2 | 86 | 0.663 |
| B043 | 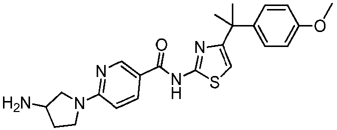 | 6-(3-aminopyrrolidin-1-yl)-N-(4-(2-(4-methoxyphenyl)propan-2-yl)thiazol-2-yl)nicotinamide | ¹H NMR (400MHz, DMSO-*d*₆) *δ* 12.66 (br s, 1H), 8.75 (s, 1H), 8.63 (br s, 3H), 8.40 (d, *J =* 10.3 Hz, 1H), 7.16 - 7.06 (m, 3H), 6.96 (s, 1H), 6.82 (d, *J =* 8.8 Hz, 2H), 4.08 - 3.98 (m, 1H), 3.92 - 3.80 (m, 3H), 3.70 (s, 4H), 2.41 - 2.19 (m, 2H), 1.63 (s, 6H) | 438.1 | 140 | 1.49 |
| B044 | 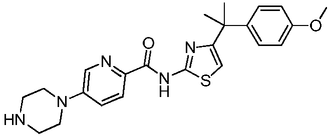 | N-(4-(2-(4-methoxyphenyl)propan-2-yl)thiazol-2-yl)-5-(piperazin-1-yl)picolinamide | ¹H NMR (400MHz, DMSO-*d*₆) *δ* 11.64 (br s, 1H), 9.39 (br s, 2H), 8.44 - 8.37 (m, 1H), 8.03 (d, *J* = 8.8 Hz, 1H), 7.56 (d, *J =* 8.6 Hz, 1H), 7.16 (d, *J =* 8.8 Hz, 2H), 6.94 (s, 1H), 6.83 (d, *J =* 8.8 Hz, 2H), 3.70 (s, 3H), 3.69 - 3.63 (m, 4H), 3.28 - 3.16 (m, 4H), 1.63 (s, 6H). | 438.1 | 213 | 1.86 |
| B045 | 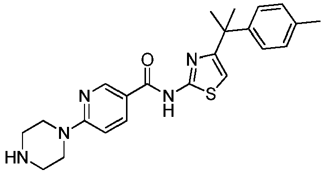 | 6-(piperazin-1-yl)-N-(4-(2-(p-tolyl)propan-2-yl)thiazol-2-yl)nicotinamide | ¹H NMR (400 MHz, DMSO-*d*₆) *δ* 12.37 (br s, 1 H), 9.28 (br s, 2 H), 8.83 (d, J=1.54 Hz, 1 H), 8.23 (br d, J=9.26 Hz, 1 H), 7.11 - 7.04 (m, 4 H), 6.99 (d, J=9.04 Hz, 1 H), 6.94 (s, 1 H), 3.89 (br s, 4 H), 3.16 (br s, 4 H), 2.23 (s, 3 H), 1.63 (s, 6 H). | 422.2 | 30 | 0.23 |
| B046 | 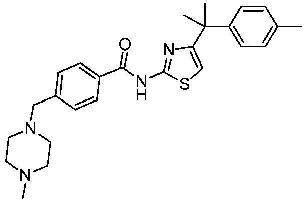 | 4-((4-methylpiperazin-1-yl)methyl)-N-(4-(2-(p-tolyl)propan-2-yl)thiazol-2-yl)benzamide | ¹H NMR (400 MHz, CD3OD) *δ* 8.12 (d, J=8.07 Hz, 2 H), 7.83 (d, J=8.31 Hz, 2 H), 7.20 - 7.16 (m, 2 H), 7.14 - 7.10 (m, 2 H), 7.08 (s, 1 H), 4.57 (s, 2 H), 3.64 (br s, 8 H), 3.01 (s, 3 H), 2.29 (s, 3 H), 1.73 (s, 6 H). | 449.3 | 28 | 0.21 |
| B047 | 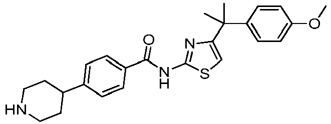 | N-(4-(2-(4-methoxyphenyl)propan-2-yl)thiazol-2-yl)-4-(piperidin-4-yl)benzamide hydrochloride | ¹H NMR (400 MHz, CD3OD) *δ* 8.08 - 7.96 (m, 2 H), 7.48 (br d, J=6.61 Hz, 2 H), 7.26 - 7.16 (m, 2 H), 7.13 - 7.03 (m, 1 H), 6.86 (br d, J=6.84 Hz, 2 H), 3.75 (s, 3 H), 3.56 - 3.45 (m, 2 H), 3.23 - 3.09 (m, 2 H), 3.03 (br t, J=12.13 Hz, 1 H), 2.15 - 2.03 (m, 2 H), 1.94 (q, J=13.38 Hz, 2 H), 1.72 (s, 6 H). | 436.2 | 128 | 0.54 |
| B048 | 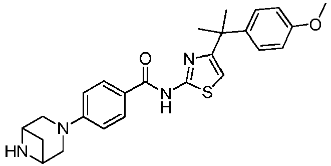 | 4-(3,6-diazabicyclo[3.1.1]heptan-3-yl)-N-(4-(2-(4-methoxyphenyl)propan-2-yl)thiazol-2-yl)benzamide | ¹H NMR (400MHz, DMSO-*d*₆) *δ* 12.18 (br s, 1H), 9.99 (br s, 1H), 8.39 (br s, 1H), 8.04 (d, J = 8.8 Hz, 2H), 7.15 - 7.07 (m, 2H), 6.87 (s, 1H), 6.80 (dd, J = 1.9, 8.9 Hz, 4H), 4.46 (br s, 2H), 3.89 - 3.72 (m, 4H), 3.68 (s, 3H), 2.95 - 2.82 (m, 1H), 1.83 (br dd, J = 5.8, 10.0 Hz, 1H), 1.61 (s, 6H) | 450.0 | 73 | 0.77 |
| B049 | 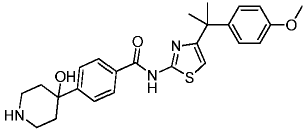 | 4-(4-hydroxypiperidin-4-yl)-N-(4-(2-(4-methoxyphenyl)propan-2-yl)thiazol-2-yl)benzamide hydrochloride | ¹H NMR (400 MHz, CD3OD) *δ* 8.06 (br d, J=8.38 Hz, 2 H), 7.73 (d, J=8.60 Hz, 2 H), 7.23 (d, J=8.82 Hz, 2 H), 7.15 - 7.05 (m, 1 H), 6.88 (d, J=8.82 Hz, 2 H), 3.76 (s, 3 H), 3.51 - 3.42 (m, 2 H), 3.40 - 3.33 (m, 2 H), 2.36 - 2.23 (m, 2 H), 1.93 (br d, J=13.89 Hz, 2 H), 1.74 (s, 6 H). | 452.2 | 459 | 0.37 |
| B050 | 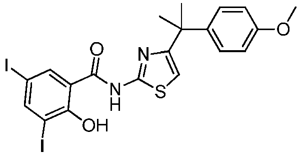 | 2-hydroxy-3,5-diiodo-N-(4-(2-(4-methoxyphenyl)propan-2-yl)thiazol-2-yl)benzamide | ¹H NMR (400 MHz, CDCl₃) *δ* 8.28 (br s, 1 H) 8.13 - 8.18 (m, 1 H) 7.21 (d, J=8.80 Hz, 2 H) 6.90 (d, J=8.80 Hz, 2 H) 6.66 (s, 1 H) 3.82 (s, 3 H) 1.74 (s, 6 H) | 620.9 | 77 | 0.71 |
| B051 | 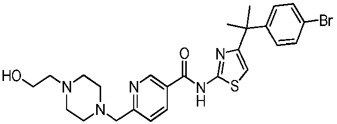 | N-(4-(2-(4-bromophenyl)propan-2-yl)thiazol-2-yl)-6-((4-(2-hydroxyethyl)piperazin-1-yl)methyl)nicotinamide | ¹H NMR (400 MHz, CD3OD) *δ* 9.05 (d, J=1.76 Hz, 1 H), 9.08 - 9.00 (m, 1 H), 8.33 (dd, J=8.16, 2.43 Hz, 1 H), 7.65 (d, J=8.16 Hz, 1 H), 7.39 (d, J=8.82 Hz, 2 H), 7.19 (d, J=8.60 Hz, 2 H), 6.88 (s, 1 H), 3.73 (s, 2 H), 3.67 (t, J=6.06 Hz, 2 H), 2.49 - 2.67 (m, 10 H), 1.69 (s, 6 H). | 546.2 | 61 | 0.54 |
| B052 | 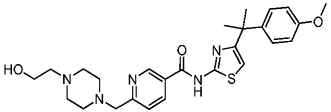 | 6-((4-(2-hydroxyethyl)piperazin-1-yl)methyl)-N-(4-(2-(4-methoxyphenyl)propan-2-yl)thiazol-2-yl)nicotinamide | ¹H NMR (400MHz, CDCl₃) *δ* 11.35 (br s, 1H), 8.69 - 8.56 (m, 1H), 8.16 (d, *J=* 8.1 Hz, 1H), 7.72 - 7.62 (m, 1H), 7.30 - 7.25 (m, 2H), 7.23 - 7.18 (m, 2H), 6.88 (d, *J=* 8.8 Hz, 2H), 6.61 (s, 1H), 3.79 (s, 3H), 1.80 (s, 6H). | 496.2 | 111 | 1.73 |
| B053 | 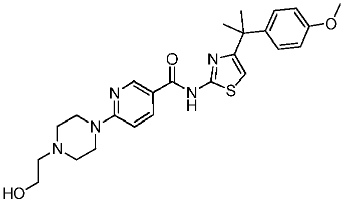 | 6-(4-(2-hydroxyethyl)piperazin-1-yl)-N-(4-(2-(4-methoxyphenyl)propan-2-yl)thiazol-2-yl)nicotinamide | ¹H NMR (400 MHz, CD3OD) *δ* 8.71 (d, J=2.45 Hz, 1 H), 8.04 (dd, J=9.17, 2.57 Hz, 1 H), 7.17 (d, J=8.80 Hz, 2 H), 6.85 - 6.78 (m, 3 H), 6.74 (s, 1 H), 3.74 (s, 3 H), 3.73 - 3.69 (m, 6 H), 2.63 - 2.59 (m, 4 H), 2.57 (t, J=5.87 Hz, 2 H), 1.67 (s, 6 H). | 482.2 | 104 | 1.97 |
| B054 | 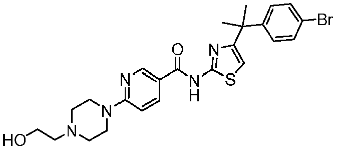 | N-(4-(2-(4-bromophenyl)propan-2-yl)thiazol-2-yl)-6-(4-(2-hydroxyethyl)piperazin-1-yl)nicotinamide | ¹H NMR (400MHz, DMSO-*d*₆) *δ* 12.31 - 12.23 (m, 1H), 8.79 (br s, 1H), 8.21 - 8.05 (m, 2H), 7.45 (br d, *J*=8.4 Hz, 2H), 7.16 (br d, *J*=7.9 Hz, 2H), 6.98 (br s, 1H), 6.89 - 6.83 (m, 1H), 4.54 - 4.38 (m, 2H), 3.62 (br s, 5H), 3.57 - 3.47 (m, 3H), 2.44 - 2.39 (m, 2H), 1.63 (br s, 6H) | 532.1 | 269 | 1.02 |
| B055 | 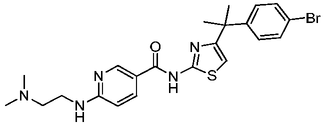 | N-(4-(2-(4-bromophenyl)propan-2-yl)thiazol-2-yl)-6-((2-(dimethylamino)ethyl)amino)n icotinamide | ¹H NMR (400MHz, DMSO-*d*₆) *δ* 12.66 (br s, 1H), 8.75 (s, 1H), 8.63 (br s, 3H), 8.40 (d, *J =* 10.3 Hz, 1H), 7.16 - 7.06 (m, 3H), 6.96 (s, 1H), 6.82 (d, *J* = 8.8 Hz, 2H), 4.08 - 3.98 (m, 1H), 3.92 - 3.80 (m, 3H), 3.70 (s, 4H), 2.41 - 2.19 (m, 2H), 1.63 (s, 6H) | 490.2 | 125 | 3.06 |
| B056 | 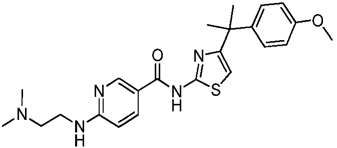 | 6-((2-(dimethylamino)ethyl)amino)-N-(4-(2-(4-methoxyphenyl)propan-2-yl)thiazol-2-yl)nicotinamide | ¹H NMR (400MHz, CDCl₃) *δ* 11.35 (br s, 1H), 8.69 - 8.56 (m, 1H), 8.16 (d, *J =* 8.1 Hz, 1H), 7.72 - 7.62 (m, 1H), 7.30 - 7.25 (m, 2H), 7.23 - 7.18 (m, 2H), 6.88 (d, *J* = 8.8 Hz, 2H), 6.61 (s, 1H), 3.79 (s, 3H), 1.80 (s, 6H) | 440.2 | 120 | 0.70 |
| B057 | 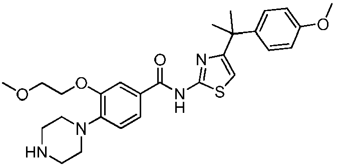 | 3-(2-methoxyethoxy)-N-(4-(2-(4-methoxyphenyl)propan-2-yl)thiazol-2-yl)-4-(piperazin-1-yl)benzamide hydrochloride | ¹H NMR (400 MHz, DMSO-*d*₆) *δ* 12.43 (br s, 1 H), 9.42 (br s, 2 H), 7.79 - 7.52 (m, 2 H), 7.12 (br d, J=8.56 Hz, 2 H), 7.00 (br d, J=8.31 Hz, 1 H), 6.92 (s, 1 H), 6.82 (br d, J=8.56 Hz, 2 H), 4.20 (br s, 1 H), 3.74 - 3.68 (m, 5 H), 3.33 (s, 7 H), 3.19 (br s, 4 H), 1.63 (s, 6 H). | 511.2 | 103 | 0.91 |
| B058 | 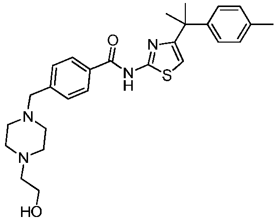 | 4-((4-(2-hydroxyethyl)piperazin-1-yl)methyl)-N-(4-(2-(p-tolyl)propan-2-yl)thiazol-2-yl)benzamide | ¹H NMR (400MHz, DMSO-*d*₆) *δ* 8.14- 8.12 (m, 2H), 7.85 - 7.83 (m, 2H), 7.20 -7.18 (m, 2H), 7.14 - 7.12 (m, 2H), 7.08 (s, 1H), 4.59 (s, 2H), 3.85 - 3.55 (m, 8H), 3.50 - 3.43 (m, 2H), 2.30 (s, 3H), 1.74 (s, 6H). | 479.6 | 68 | 0.61 |
| B059 | 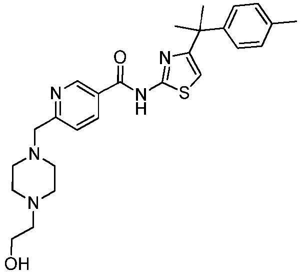 | 6-((4-(2-hydroxyethyl)piperazin-1-yl)methyl)-N-(4-(2-(p-tolyl)propan-2-yl)thiazol-2-yl)nicotinamide | ¹H NMR (400MHz, CDCl₃) *δ* 9.09 - 9.05 (m, 1H), 8.33 - 8.28 (m, 1H), 7.75 (br *d, J =* 8.8 Hz, 3H), 7.45 - 7.41 (m, 1H), 7.13 - 7.08 (m, 3H), 7.06 - 7.02 (m, 1H), 6.61 - 6.57 (m, 1H), 3.83 - 3.77 (m, 3H), 3.64 - 3.55 (m, 1H), 2.45 - 2.38 (m, 8H), 2.26 - 2.18 (m, 1H), 1.63 - 1.58 (m, 3H), 1.19 (s, 6H). | 480.3 | 159 | 1.18 |
| B060 | 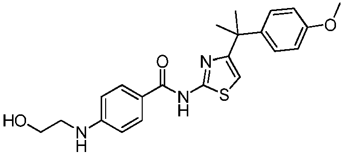 | 4-((2-hydroxyethyl)amino)-N-(4-(2-(4-methoxyphenyl)propan-2-yl)thiazol-2-yl)benzamide | ¹H NMR (400MHz, DMSO-*d*₆) *δ* 12.06 (br s, 1H), 7.87 (d, J = 8.8 Hz, 2H), 7.12 (d, J = 8.8 Hz, 2H), 6.86 (s, 1H), 6.82 (d, J = 8.8 Hz, 2H), 6.62 (d, J = 8.8 Hz, 2H), 3.70 (s, 3H), 3.54 (t, J = 5.9 Hz, 2H), 3.16 (t, J = 5.9 Hz, 2H), 1.62 (s, 6H) | 412.2 | 280 | 1.23 |
| B061 | 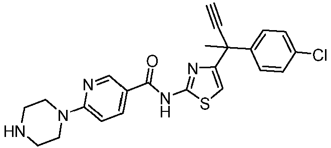 | N-(4-(2-(4-chlorophenyl)but-3-yn-2-yl)thiazol-2-yl)-6-(piperazin-1-yl)nicotinamide | ¹H NMR (400 MHz, DMSO-*d*₆) *δ* 12.56 (br s, 1 H), 9.61 (br s, 2 H), 8.83 (d, J = 2.8 Hz, 1 H), 8.28 (dd, J = 9.2, 2.4 Hz, 1 H), 7.45 - 7.38 (m, 4 H), 7.18 (s, 1 H), 7.09 (d, J = 8.8 Hz, 1 H), 3.99 - 3.93 (m, 4 H), 3.55 (s, 1 H), 3.18 (br s, 4 H), 1.91 (s, 3 H) | 452.1 | 167 | 0.78 |
| B062 | 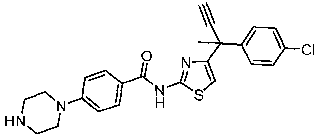 | N-(4-(2-(4-chlorophenyl)but-3-yn-2-yl)thiazol-2-yl)-4-(piperazin-1-yl)benzamide | ¹H NMR (400MHz, DMSO-*d*₆) *δ* 12.36 (s, 1H), 9.25 (br s, 2H), 8.01 (d, J=8.9 Hz, 2H), 7.45 - 7.37 (m, 4H), 7.14 (s, 1H), 7.04 (d, J=9.1 Hz, 2H), 3.58 - 3.55 (m, 4H), 3.18 (br s, 4H), 1.91 (s, 3H) | 451.1 | 12 | 1.01 |
| B063 | 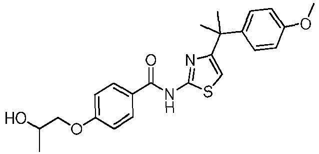 | 4-(2-hydroxypropoxy)-N-(4-(2-(4-methoxyphenyl)propan-2-yl)thiazol-2-yl)benzamide | ¹H NMR (400MHz, DMSO-*d*₆) *δ* 12.36 (br s, 1H), 8.05 (d, J = 9.0 Hz, 2H), 7.12 (d, J = 8.8 Hz, 2H), 7.02 (d, J = 9.0 Hz, 2H), 6.91 (s, 1H), 6.82 (d, J = 8.8 Hz, 2H), 3.99 - 3.93 (m, 1H), 3.88 (d, J = 4.0 Hz, 2H), 3.70 (s, 3H), 1.63 (s, 6H), 1.14 (d, J = 6.3 Hz, 3H) | 427.1 | 245 | 2.10 |
| B064 | 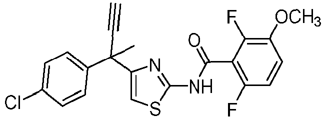 | N-(4-(2-(4-chlorophenyl)but-3-yn-2-yl)thiazol-2-yl)-2,6-difluoro-3-methoxybenzamide | | | 54 | 3.37 |
| B065 | 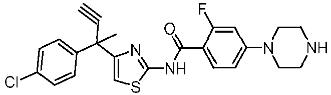 | N-(4-(2-(4-chlorophenyl)but-3-yn-2-yl)thiazol-2-yl)-2-fluoro-4-(piperazin-1-yl)benzamide | | 469.1 | 215 | 2.46 |
| B066 | 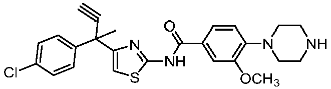 | N-(4-(2-(4-chlorophenyl)but-3-yn-2-yl)thiazol-2-yl)-3-methoxy-4-(piperazin-1-yl)benzamide | ¹H NMR (400 MHz, DMSO-*d*₆) *δ* 7.66 (1H, J=8.8 Hz, d), 7.41-7.35 (5H, m), 7.16 (1H, s), 6.91 (1H, J=7.2 Hz, d), 3.83 (3H, s), 3.51 (1H, s), 3.01 (4H, s), 2.87 (4H, S), 1.88(3H, s) | 481.1 | 184 | 1.20 |
| B067 | 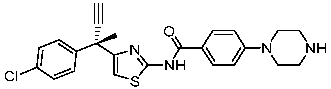 | N-(4-(2-(4-chlorophenyl)but-3-yn-2-yl)thiazol-2-yl)-4-(piperazin-1-yl)benzamide | ¹H NMR (400 MHz, DMSO-*d*₆) *δ* 12.36 (br s, 1 H), 9.31 (br s, 2 H), 8.02 (d, *J =* 8.78 Hz, 2 H), 7.35 - 7.47 (m, 4 H), 7.14 (s, 1 H), 7.04 (d, *J* = 8.78 Hz, 2 H), 3.49 - 3.65 (m, 5 H), 3.19 (br d, *J =* 4.77 Hz, 4 H), 1.91 (s, 3 H). | 451.0 | 90 | 1.43 |
| B068 | 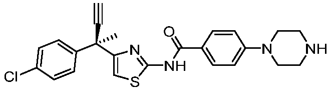 | N-(4-(2-(4-chlorophenyl)but-3-yn-2-yl)thiazol-2-yl)-4-(piperazin-1-yl)benzamide | ¹H NMR (400 MHz, DMSO-*d*₆) *δ* 12.36 (s, 1 H), 9.19 (br s, 2 H), 8.02 (d, *J* = 8.78 Hz, 2 H), 7.36 - 7.46 (m, 4 H), 7.15 (s, 1 H), 7.04 (d, *J* = 8.78 Hz, 2 H), 3.53 - 3.60 (m, 5 H), 3.19 (br s, 4 H), 1.91 (s, 3 H). | 451.0 | 530 | 4.16 |
| B069 | 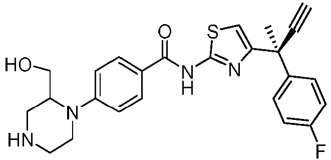 | N-[4-[1-(4-fluorophenyl)-1-methyl-prop-2-ynyl]thiazol-2-yl]-4-[2-(hydroxymethyl)piperazin-1-yl]benzamide | ¹H NMR (400MHz, CDCl₃) *δ* 9.26 (br s, 1H), 7.78 (d, *J*=8.9 Hz, 2H), 7.45 (dd, *J*=5.2, 8.8 Hz, 2H), 6.99 (t, *J*=8.7 Hz, 2H), 6.89 - 6.85 (m, 3H), 4.04 - 3.98 (m, 1H), 3.98 - 3.89 (m, 2H), 3.66 - 3.54 (m, 1H), 3.53 - 3.46 (m, 1H), 3.43 (br d, *J*=11.*7* Hz, 1H), 3.20 (br d, *J*=11.3 Hz, 1H), 3.14 (br d, *J*=11.9 Hz, 1H), 3.16 - 3.08 (m, 1H), 2.96 (dt, *J*=4.2, 11.7 Hz, 1H), 2.59 (s, 1H), 1.97 (s, 3H), 2.02 - 1.93 (m, 1H). | 465.0 | 20 | 0.96 |
| B070 | 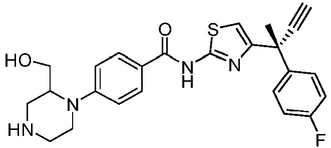 | N-[4-[1-(4-fluorophenyl)-1-methyl-prop-2-ynyl]thiazol-2-yl]-4-[2-(hydroxymethyl)piperazin-1-yl]benzamide | ¹H NMR (400MHz, CDCl₃) *δ* 9.23 (br s, 1H), 7.74 (d, *J*=8.9 Hz, 2H), 7.43 - 7.39 (m, 2H), 6.95 (t, *J*=8.6 Hz, 2H), 6.86 - 6.80 (m, 3H), 4.00 - 3.94 (m, 1H), 3.94 - 3.85 (m, 2H), 3.56 - 3.49 (m, 1H), 3.49 - 3.42 (m, 1H), 3.39 (br d, *J*=12.2 Hz, 1H), 3.16 (br d, *J*=9.5 Hz, 1H), 3.09 (br d, *J*=12.0 Hz, 1H), 2.91 (dt, *J*=4.4, 11.7 Hz, 1H), 2.54 (s, 1H), 1.93 (s, 3H). | 465.0 | 169 | 2.07 |
| B071 | 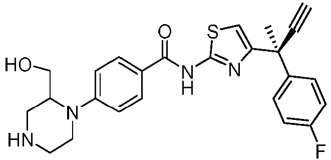 | N-[4-[1-(4-fluorophenyl)-1-methyl-prop-2-ynyl]thiazol-2-yl]-4-[2-(hydroxymethyl)piperazin-1-yl]benzamide | ¹H NMR (400MHz, CDCl₃) *δ* 9.62 (br s, 1H), 7.30 (br s, 2H), 7.07 (br s, 2H), 6.64 - 6.58 (m, 2H), 6.50 - 6.34 (m, 1H), 6.50 - 6.31 (m, 3H), 4.62 (br s, 1H), 3.78 (br s, 1H), 3.64 (br s, 1H), 3.50 (br s, 1H), 3.36 (br s, 1H), 3.23 (br s, 3H), 3.00 - 2.56 (m, 2H), 2.27 (br s, 1H), 1.70 (br s, 3H). | 465.0 | 60 | 1.30 |
| B072 | 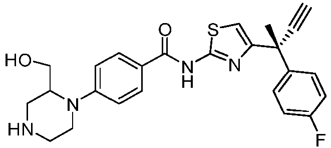 | N-[4-[1-(4-fluorophenyl)-1-methyl-prop-2-ynyl]thiazol-2-yl]-4-[2-(hydroxymethyl)piperazin-1-yl]benzamide | ¹H NMR (400MHz, CDCl₃) *δ* 9.26 (br s, 1H), 7.76 (d, *J*=8.9 Hz, 2H), 7.45 - 7.40 (m, 2H), 6.97 (t, *J*=8.8 Hz, 2H), 6.90 - 6.82 (m, 3H), 4.02 - 3.96 (m, 1H), 3.96 - 3.85 (m, 2H), 3.57 - 3.51 (m, 1H), 3.44 - 3.43 (m, 1H), 3.50 - 3.43 (m, 1H), 3.40 (br d, *J*=11.8 Hz, 1H), 3.17 (br d, *J*=11.4 Hz, 1H), 3.10 (br d, *J*=11.8 Hz, 1H), 2.93 (dt, *J*=4.3, 11.7 Hz, 1H), 2.56 (s, 1H), 1.95 (s, 3H). | 465.0 | 260 | 4.13 |
| B073 | 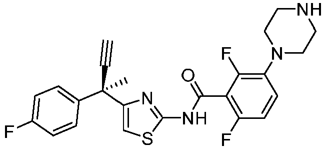 | 2,6-difluoro-N-(4-(2-(4-fluorophenyl)but-3-yn-2-yl)thiazol-2-yl)-3-(piperazin-1-yl)benzamide | ¹H NMR (400 MHz, DMSO-*d*₆) *δ* 13.06 (s, 1 H), 9.12 (br s, 2 H), 7.49 - 7.43 (m, 2 H), 7.32 - 7.24 (m, 2 H), 7.16 (t, *J* = 8.8 Hz, 3 H), 3.55 (s, 1 H) 3.22, (br s, 8 H), 1.92 (s, 3 H) | 471.1 | 851 | 2.39 |
| B074 | 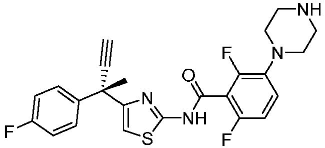 | 2,6-difluoro-N-(4-(2-(4-fluorophenyl)but-3-yn-2-yl)thiazol-2-yl)-3-(piperazin-1-yl)benzamide | ¹H NMR (400 MHz, DMSO-*d*₆) *δ* 13.07 (s, 1 H), 9.23 (br s, 2 H), 7.49 - 7.42 (m, 2 H), 7.31 - 7.24 (m, 2 H), 7.14 (t, *J = 8.8* Hz, 3 H), 3.55 (s, 1 H), 3.22 (s, 8 H), 1.92 (s, 3 H) | 471.1 | 96 | 0.36 |
| B075 | 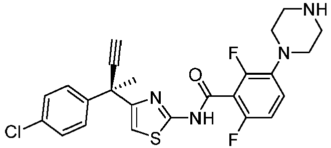 | N-(4-(2-(4-chlorophenyl)but-3-yn-2-yl)thiazol-2-yl)-2,6-difluoro-3-(piperazin-1-yl)benzamide | ¹H NMR (400 MHz, DMSO-*d*₆) *δ* 13.05 (s, 1 H), 9.36 (br s, 2 H), 7.48-7.37 (m, 4 H), 7.31 - 7.23 (m, 2 H), 7.18 - 7.15 (t, *J* = 8.8 Hz, 3 H), 3.56 (s, 1 H), 3.22 (s, 8 H), 1.90 (s, 3 H) | 487.1 | 308 | 2.13 |
| B076 | 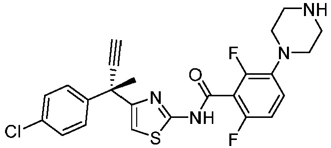 | N-(4-(2-(4-chlorophenyl)but-3-yn-2-yl)thiazol-2-yl)-2,6-difluoro-3-(piperazin-1-yl)benzamide | ¹H NMR (400 MHz, DMSO-*d*₆) *δ* 13.05 (s, 1 H), 9.33 (br s, 2 H), 7.46 - 7.37 (m, 4 H), 7.32 - 7.23 (m, 2 H), 7.12 - 7.18 (m, 1 H), 3.56 (s, 1 H), 3.22 (s, 8 H), 1.90 (s, 3 H) | 487.1 | 85 | 0.64 |
| B077 | 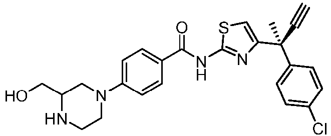 | N-(4-(2-(4-chlorophenyl)but-3-yn-2-yl)thiazol-2-yl)-4-(3-(hydroxymethyl)piperazin-1-yl)benzamide | ¹H NMR (400MHz, DMSO-*d*₆) *δ* 7.97 (d, J = 8.9 Hz, 2H), 7.45 - 7.37 (m, 4H), 7.12 (s, 1H), 6.94 (d, J = 9.2 Hz, 2H), 4.70 (br s, 1H), 3.85 - 3.70 (m, 2H), 3.53 (s, 1H), 3.41 - 3.36 (m, 1H), 3.01 - 2.95 (m, 1H), 2.76 - 2.67 (m, 3H), 2.47 - 2.38 (m, 1H), 1.91 (s, 3H) | 481.1 | 90 | 1.35 |
| B078 | 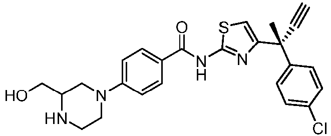 | N-(4-(2-(4-chlorophenyl)but-3-yn-2-yl)thiazol-2-yl)-4-(3-(hydroxymethyl)piperazin-1-yl)benzamide | ¹H NMR (400MHz, DMSO-*d*₆) *δ* 7.97 (d, J = 8.9 Hz, 2H), 7.45 - 7.37 (m, 4H), 7.11 (s, 1H), 6.93 (d, J = 8.9 Hz, 2H), 4.69 (br s, 1H), 3.79 (d, J = 11.2 Hz, 1H), 3.72 (d, J=8.2 Hz, 1H), 3.52 (s, 1H), 3.40 - 3.36 (m, 2H), 3.01 - 2.95 (m, 1H), 2.76 - 2.67 (m, 3H), 2.41 (t, J=11.0 Hz, 1H), 1.91 (s, 3H) | 481.1 | 169 | 2.13 |
| B079 | 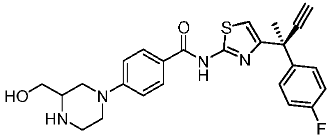 | N-(4-(2-(4-chlorophenyl)but-3-yn-2-yl)thiazol-2-yl)-4-(3-(hydroxymethyl)piperazin-1-yl)benzamide | ¹H NMR (400MHz, DMSO-*d*₆) *δ* 7.97 (d, *J =* 8.8 Hz, 2H), 7.51 - 7.40 (m, 2H), 7.15 (t, *J =* 8.9 Hz, 2H), 7.05 - 6.88 (m, 3H), 4.70 (br s, 1H), 3.82 - 3.65 (m, 2H), 3.49 (s, 1H), 3.41 - 3.37 (m, 2H), 3.04 - 2.93 (m, 1H), 2.80 - 2.63 (m, 3H), 2.44 - 2.32 (m, 1H), 1.91 (s, 3H) ¹⁹F NMR (376MHz, DMSO-*d*₆) *δ* -116.67 (s, 1F) | 465.2 | 174 | 2.99 |
| B080 | 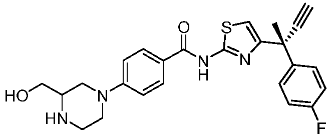 | N-(4-(2-(4-chlorophenyl)but-3-yn-2-yl)thiazol-2-yl)-4-(3-(hydroxymethyl)piperazin-1-yl)benzamide | ¹H NMR (400MHz, DMSO-*d*₆) *δ* 7.98 (d, *J =* 8.8 Hz, 2H), 7.49 - 7.36 (m, 2H), 7.15 (t, *J* = 8.8 Hz, 2H), 7.10 (s, 1H), 6.95 (d, *J =* 9.0 Hz, 2H), 4.83 (br s, 1H), 3.84 - 3.74 (m, 2H), 3.51 (s, 1H), 3.44 - 3.41 (m, 2H), 3.05 - 2.99 (m, 1H), 2.82 - 2.73 (m, 3H), 2.49 - 2.48 (m, 1H), 1.91 (s, 3H) | 465.2 | 286 | 3.30 |
| | | | ¹⁹F NMR (376MHz, DMSO-*d*₆) *δ* -116.51 (s, 1F) | | | |
| B081 | 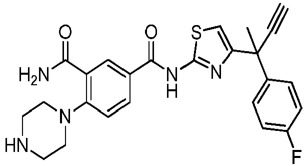 | N 1-(4-(2-(4-fluorophenyl)but-3-yn-2-yl)thiazol-2-yl)-4-(piperazin-1-yl)isophthalamide | ¹H NMR (400MHz, DMSO-*d*₆) *δ* 12.59 (br s, 1H), 9.42 (br s, 2H), 8.22 (d, *J =* 2.0 Hz, 1H), 8.15 - 8.07 (m, 1H), 7.88 (s, 1H), 7.61 (s, 1H), 7.50 - 7.42 (m, 2H), 7.20 - 7.13 (m, 4H), 3.53 (s, 1H), 3.29 (s, 4H), 3.23 (s, 4H), 1.92 (s, 3H) | 478.2 | 102 | 0.96 |
| | | | ¹⁹F NMR (376MHz, DMSO-*d*₆) *δ* -116.46 (s, 1F) | | | |
| B082 | 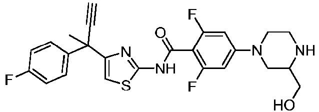 | 2,6-difluoro-N-(4-(2-(4-fluorophenyl)but-3-yn-2-yl)thiazol-2-yl)-4-(3-(hydroxymethyl)piperazin-1-yl)benzamide | ¹H NMR (400MHz, DMSO-*d*₆) *δ* 12.77 (s, 1H), 9.81 - 9.64 (m, 1H), 9.61 - 9.41 (m, 1H), 7.48 - 7.41 (m, 2H), 7.19 - 7.17 (m, 1H), 7.15 - 7.12 (m, 1H), 6.87 (s, 1H), 6.79 (d, *J =* 12.0 Hz, 2H), 3.98 (t, *J*=15.7 Hz, 2H), 3.77 - 3.63 (m, 2H), 3.54 (s, 1H), 3.30 - 3.20 (m, 3H), 3.16 - 2.97 (m, 2H), 1.90 (s, 3H) ¹⁹F NMR (376MHz, DMSO-*d*₆) *δ* -111.73 - - 111.77 (m, 2F), -116.39 (s, 1F) | 501.1 | 25 | 0.43 |
| B083 | 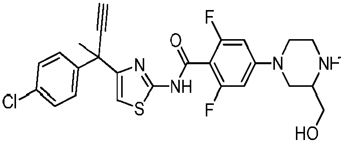 | N-(4-(2-(4-chlorophenyl)but-3-yn-2-yl)thiazol-2-yl)-2,6-difluoro-4-(3-(hydroxymethyl)piperazin-1-yl)benzamide | ¹H NMR (400MHz, DMSO-*d*₆) *δ* 7.52 - 7.36 (m, 4H), 7.16 (s, 1H), 6.63 (d, *J=* 12.6 Hz, 2H), 4.68 (t, *J=* 5.2 Hz, 1H), 3.77 - 3.63 (m, 2H), 3.54 (s, 1H), 3.00 - 2.93 (m, 1H), 2.80 - 2.63 (m, 3H), 2.47 - 2.29 (m, 3H), 1.90 (s, 3H) ¹⁹F NMR (376MHz, DMSO-*d*₆) *δ* -112.15 (s, 2F) | 517.1 | 78 | 1.27 |
| B084 | 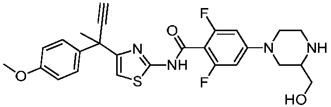 | 2,6-difluoro-4-(3-(hydroxymethyl)piperazin-1-yl)-N-(4-(2-(4-methoxyphenyl)but-3-yn-2-yl)thiazol-2-yl)benzamide | ¹H NMR (400MHz, DMSO-*d*₆) *δ* 12.74 (br s, 1H), 9.65 - 9.47 (m, 1H), 9.41 - 9.21 (m, *J*=10.0 Hz, 1H), 7.34 - 7.30 (m, 2H), 7.12 (s, 1H), 6.87 (d, *J*= 8.8 Hz, 2H), 6.79 (d, *J*=12.0 Hz, 2H), 3.97 (t, *J* = 15.1 Hz, 2H), 3.75 - 3.70 (m, 4H), 3.68 - 3.62 (m, 1H), 3.46 (s, 1H), 3.32 - 3.17 (m, 3H), 3.14 - 2.99 (m, 2H), 1.88 (s, 3H) ¹⁹F NMR (376MHz, DMSO-*d*₆) *δ* -111.79 (s, 2F) | 513.2 | 28 | 0.39 |
| B085 | 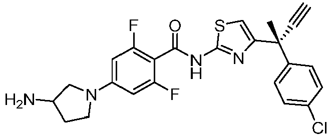 | 4-(3-aminopyrrolidin-1-yl)-N-(4-(2-(4-chlorophenyl)but-3-yn-2-yl)thiazol-2-yl)-2,6-difluorobenzamide | ¹H NMR (400 MHz, DMSO-*d*₆) *δ* 12.65 (s, 1H), 8.29 (br. s, 3H), 7.46 - 7.37 (m, 4 H), 7.19 (s, 1H), 6.37 - 6.29 (m, 2 H), 3.97 (br. s, 1H), 3.62 - 3.55 (m, 1 H), 3.55 (s, 1H), 3.52 - 3.47 (m, 1 H), 3.38 - 3.32 (m, 2 H), 2.37 - 2.26 (m, 1H), 2.16 - 2.05 (m, 1H), 1.90 (s, 3H). | 487.4 | 395 | 2.58 |
| B086 | 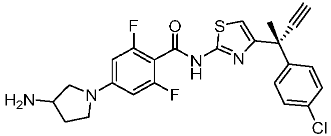 | 4-(3-aminopyrrolidin-1-yl)-N-(4-(2-(4-chlorophenyl)but-3-yn-2-yl)thiazol-2-yl)-2,6-difluorobenzamide | ¹H NMR (400 MHz, DMSO-*d*₆) *δ* 12.65 (s, 1H), 8.29 (br. s, 3H), 7.46 - 7.37 (m, 4 H), 7.19 (s, 1H), 6.35 - 6.31 (m, 2 H), 3.96 (br. s, 1H), 3.62 - 3.55 (m, 1 H), 3.55 (s, 1H), 3.52 - 3.47 (m, 1 H), 3.38 - 3.32 (m, 2 H), 2.37 - 2.26 (m, 1H), 2.16 - 2.05 (m, 1H), 1.90 (s, 3H). | 487.1 | 286 | 2.57 |
| B087 | 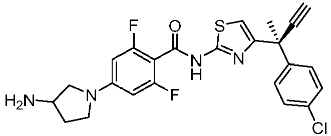 | 4-(3-aminopyrrolidin-1-yl)-N-(4-(2-(4-chlorophenyl)but-3-yn-2-yl)thiazol-2-yl)-2,6-difluorobenzamide | ¹H NMR (400 MHz, DMSO-*d*₆) *δ* 12.64 (s, 1H), 8.28 (br. s, 3H), 7.46 - 7.37 (m, 4 H), 7.19 (s, 1H), 6.35 - 6.31 (m, 2 H), 3.97 (br. s, 1H), 3.62 - 3.55 (m, 1 H), 3.55 (s, 1H), 3.52 - 3.47 (m, 1 H), 3.38 - 3.32 (m, 2 H), 2.37 - 2.26 (m, 1H), 2.16 - 2.05 (m, 1H), 1.90 (s, 3H). | 487.4 | 38 | 1.18 |
| B088 | 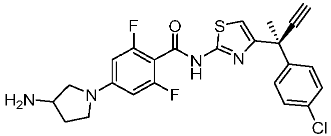 | 4-(3-aminopyrrolidin-1-yl)-N-(4-(2-(4-chlorophenyl)but-3-yn-2-yl)thiazol-2-yl)-2,6-difluorobenzamide | ¹H NMR (400 MHz, DMSO-*d*₆) *δ* 12.65 (s, 1H), 8.26 (br. s, 3H), 7.46 - 7.37 (m, 4 H), 7.19 (s, 1H), 6.35 - 6.31 (m, 2 H), 3.96 (br. s, 1H), 3.62 - 3.55 (m, 1 H), 3.55 (s, 1H), 3.52 - 3.47 (m, 1 H), 3.38 - 3.32 (m, 2 H), 2.37 - 2.26 (m, 1H), 2.16 - 2.05 (m, 1H), 1.90 (s, 3H). | 487.0 | 42 | 0.92 |
| B089 | 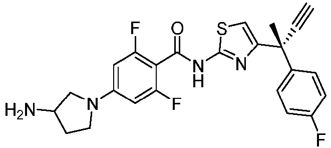 | 4-(3-aminopyrrolidin-1-yl)-2,6-difluoro-N-(4-(2-(4-fluorophenyl)but-3-yn-2-yl)thiazol-2-yl)benzamide | ¹H NMR (400 MHz, DMSO-*d*₆) *δ* 12.66 (s, 1H), 8.28 (br. s, 3H), 7.50- 7.40 (m, 2 H), 7.20 - 7.10 (m, 3H), 6.40 - 6.30 (m, 2 H), 3.96 (br. s, 1H), 3.61 - 3.55 (m, 1 H), 3.53 (s, 1H), 3.51 - 3.45 (m, 1 H), 3.41 - 3.33 (m, 2 H), 2.35 - 2.27 (m, 1H), 2.15 - 2.07 (m, 1H), 1.91 (s, 3H). | [M+Na⁺] =493.0 | 681 | 2.77 |
| B090 | 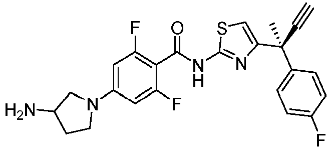 | 4-(3-aminopyrrolidin-1-yl)-2,6-difluoro-N-(4-(2-(4-fluorophenyl)but-3-yn-2-yl)thiazol-2-yl)benzamide | ¹H NMR (400 MHz, DMSO-*d*₆) *δ* 12.65 (s, 1H), 8.29 (br. s, 3H), 7.50- 7.40 (m, 2 H), 7.20 - 7.10 (m, 3H), 6.40 - 6.30 (m, 2 H), 3.96 (br. s, 1H), 3.61 - 3.55 (m, 1 H), 3.54 (s, 1H), 3.51 - 3.45 (m, 1 H), 3.41 - 3.33 (m, 2 H), 2.39 - 2.26 (m, 1H), 2.16 - 2.07 (m, 1H), 1.91 (s, 3H). | 471.0 | 107 | 1.52 |
| B091 | 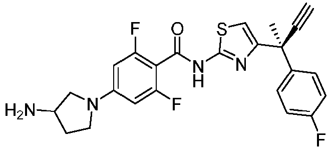 | 4-(3-aminopyrrolidin-1-yl)-2,6-difluoro-N-(4-(2-(4-fluorophenyl)but-3-yn-2-yl)thiazol-2-yl)benzamide | ¹H NMR (400 MHz, DMSO-*d*₆) *δ* 12.66 (s, 1H), 8.25 (br. s, 3H), 7.50- 7.40 (m, 2 H), 7.20 - 7.10 (m, 3H), 6.40 - 6.30 (m, 2 H), 3.96 (br. s, 1H), 3.61 - 3.55 (m, 1 H), 3.53 (s, 1H), 3.51 - 3.45 (m, 1 H), 3.39 - 3.31 (m, 2 H), 2.35 - 2.26 (m, 1H), 2.24 - 2.05 (m, 1H), 1.91 (s, 3H). | 471.0 | 65 | 1.05 |
| B092 | 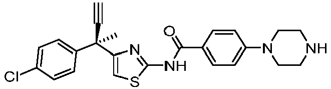 | N-(4-(2-(4-chlorophenyl)but-3-yn-2-yl)thiazol-2-yl)-4-(piperazin-1-yl)benzamide | ¹H NMR (400MHz, DMSO-*d*₆) *δ* 10.57 (br s, 1H), 7.50 (d, *J =* 8.6 Hz, 2H), 7.36 (d, *J*=8.6 Hz, 2H), 6.90 (s, 1H), 6.28 (br s, 1H), 4.75 (t, *J*=5.1 Hz, 2H), 3.60 (br. dd, *J*=4.8, 8.1 Hz, 1H), 3.50 (s, 1H), 3.42 - 3.35 (m, 4H), 1.84 (s, 3H). | 424.1 | 219 | 3.44 |
| B093 | 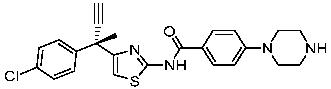 | N-(4-(2-(4-chlorophenyl)but-3-yn-2-yl)thiazol-2-yl)-4-(piperazin-1-yl)benzamide | ¹H NMR (400MHz, DMSO-*d*₆) *δ* 10.56 (br s, 1H), 7.50 (d, *J*=8.6 Hz, 2H), 7.36 (d, *J*=8.6 Hz, 2H), 6.90 (s, 1H), 6.28 (br s, 1H), 4.75 (t, *J*=5.1 Hz, 2H), 3.63 - 3.57 (m, 1H), 3.50 (s, 1H), 3.42 - 3.35 (m, 4H), 1.84 (s, 3H). | 424.0 | 43 | 0.58 |
| B094 | 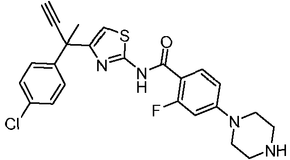 | N-(4-(2-(4-chlorophenyl)but-3-yn-2-yl)thiazol-2-yl)-2-fluoro-4-(piperazin-1-yl)benzamide | | 469.1 | 151 | 2.46 |
| B095 | 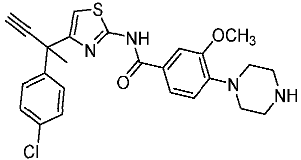 | N-(4-(2-(4-chlorophenyl)but-3-yn-2-yl)thiazol-2-yl)-3-methoxy-4-(piperazin-1-yl)benzamide | ¹H NMR (400 MHz, DMSO-*d*₆) δ 7.66 (d, *J =* 8.8 Hz, 2H), 7.41-7.36 (m, *J= 20.0* Hz, 4H), 7.15 (s, 1H), 6.92 (d, *J* = 8.0 Hz, 1H), 3.83 (s, 3H), 3.51 (s, 4H), 3.02 (s, 1H), 2.87 (s, 4H), 1.88 (s, 3H). | 481.1 | 184 | 1.20 |
| B096 | 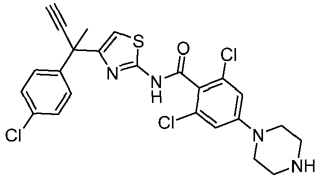 | 2,6-dichloro-N-(4-(2-(4-chlorophenyl)but-3-yn-2-yl)thiazol-2-yl)-4-(piperazin-1-yl)benzamide | | 519.0 | 312 | |
| B097 | 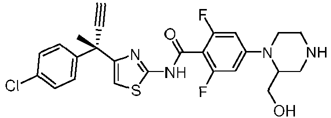 | N-[4-[1-(4-chlorophenyl)-1-methyl-prop-2-ynyl]thiazol-2-yl]-2,6-difluoro-4-[2-(hydroxymethyl)piperazin-1-yl]benzamide | ¹H NMR (400MHz, DMSO-*d*₆) *δ* 12.72 (s, 1H), 9.37 (br s, 1H), 8.72 (br s, 1H), 7.44 - 7.37 (m, 4H), 7.20 (s, 1H), 6.71 (d, *J*=12.5 Hz, 2H), 4.13 (br s, 1H), 3.75 (br s, 1H), 3.60 (br s, 1H), 3.58 (br s, 1H), 3.57 (br s, 1H), 3.49 (br s, 1H), 3.46 (br s, 1H), 3.33 - 3.23 (m, 2H), 3.15 (br d, *J*=8.8 Hz, 1H), 3.03 (br d, *J=*12.5 Hz, 1H), 1.90 (s, 3H). | 517.0 | 119 | 1.14 |
| B098 | 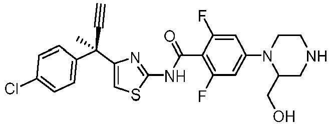 | N-[4-[1-(4-chlorophenyl)-1-methyl-prop-2-ynyl]thiazol-2-yl]-2,6-difluoro-4-[2-(hydroxymethyl)piperazin-1-yl]benzamide | ¹H NMR (400MHz, DMSO-*d*₆) *δ* 12.72 (s, 1H), 9.51 (br s, 1H), 8.85 (br s, 1H), 7.50 - 7.33 (m, 4H), 7.20 (s, 1H), 6.71 (br d, *J*=12.5 Hz, 2H), 4.13 (br s, 1H), 3.83 (br d, *J=*14.3 Hz, 2H), 3.74 (br dd, *J*=7.8, 11.3 Hz, 1H), 3.62 - 3.56 (m, 1H), 3.55 (s, 1H), 3.47 (br d, *J*=13.0 Hz, 1H), 3.34 - 3.22 (m, 2H), 3.20 - 3.08 (m, 1H), 3.02 (br d, *J*=10.3 Hz, 1H), 1.90 (s, 3H). | 517.0 | 20 | 0.26 |
| B099 | 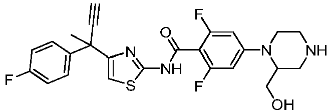 | 2,6-difluoro-N-[4-[1-(4-fluorophenyl)-1-methyl-prop-2-ynyl]thiazol-2-yl]-4-[2-(hydroxymethyl)piperazin-1-yl]benzamide | ¹H NMR (400MHz, DMSO-*d*₆) *δ* 7.48 - 7.41 (m, 1H), 7.44 (dd, *J*=5.5, 8.8 Hz, 2H), 7.20 - 7.10 (m, 3H), 6.57 (br d, *J*=13.1 Hz, 2H), 4.71 (br s, 1H), 3.76 - 3.65 (m, 2H), 3.52 (s, 1H), 3.48 (br d, *J*=10.4 Hz, 1H), 3.12 (br d, *J*=12.3 Hz, 1H), 2.96 - 2.90 (m, 1H), 2.89 - 2.81 (m, 1H), 2.68 (br d, *J*=3.2 Hz, 1H), 2.66 - 2.61 (m, 1H), 2.61 - 2.54 (m, 1H), 1.90 (s, 3H). | 501.0 | 30 | 0.64 |
| B100 | 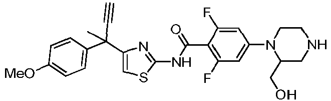 | 2,6-difluoro-4-[2-(hydroxymethyl)piperazin-1-yl]-N-[4-[1-(4-methoxyphenyl)-1-methyl-prop-2-ynyl]thiazol-2-yl]benzamide | ¹H NMR (400MHz, DMSO-*d*₆) *δ* 12.71 (s, 1H), 9.53 (br s, 1H), 8.87 (br s, 1H), 7.35 - 7.30 (m, 2H), 7.11 (s, 1H), 6.90 - 6.85 (m, 2H), 6.71 (br d, *J*=12.4 Hz, 2H), 3.72 (s, 4H), 3.61 - 3.56 (m, 1H), 3.49 - 3.41 (m, 2H), 3.32 - 3.23 (m, 2H), 3.13 (br s, 1H), 3.01 (br d, *J*=10.1 Hz, 1H), 1.88 (s, 3H). | 513.0 | 21 | 0.38 |
| B101 | 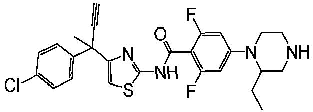 | N-(4-(2-(4-chlorophenyl)but-3-yn-2-yl)thiazol-2-yl)-4-(2-ethylpiperazin-1-yl)-2,6-difluorobenzamide | | 515.1 | 70 | 2.47 |
| B102 | 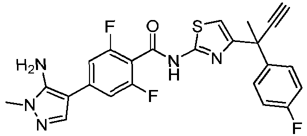 | 4-(5-amino-1-methyl-1H-pyrazol-4-yl)-2,6-difluoro-N-(4-(2-(4-fluorophenyl)but-3-yn-2-yl)thiazol-2-yl)benzamide | ¹H NMR (400 MHz, DMSO-*d*₆) *δ* 12.98 (br s, 1H), 7.67 (s, 1H), 7.51 - 7.40 (m, 2H), 7.33 - 7.09 (m, 5H), 5.80 (s, 2H), 3.59 (s, 3H), 3.54 (s, 1H), 1.92 (s, 3H). | 482.1 | 1269 | 2.25 |
| B103 | 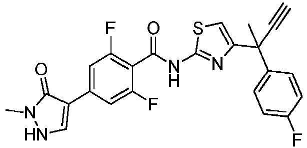 | 2,6-difluoro-N-(4-(2-(4-fluorophenyl)but-3-yn-2-yl)thiazol-2-yl)-4-(2-methyl-3-oxo-2,3-dihydro-1*H*-pyrazol-4-yl)benzamide | ¹H NMR (400 MHz, CD₃OD): *δ* 7.97 (br s, 1H), 7.60 - 7.51 (m, 4H), 7.11 (s, 1H), 7.01 (br t, *J=* 8.6 Hz, 2H), 3.53 (s, 3H), 2.97 (s, 1H), 1.97 (s, 3H). | 483.0 | 5139 | 5.21 |
| B104 | 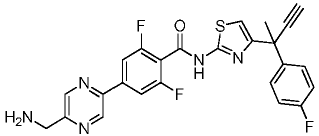 | 4-(5-(aminomethyl)pyrazin-2-yl)-2,6-difluoro-N-(4-(2-(4-fluorophenyl)but-3-yn-2-yl)thiazol-2-yl)benzamide | ¹H NMR (400 MHz, CD₃OD) *δ* 9.28 (s, 1H), 8.81 (s, 1H), 7.94 (d, *J* = 8.8 Hz, 2H), 7.64 - 7.48 (m, 2H), 7.16 (s, 1H), 7.06 - 6.95 (m, 2H), 4.45 (s, 2H), 2.99 (s, 1H), 2.04 - 1.92 (m, 3H). | 494.0 | 1474 | 2.98 |
| B105 | 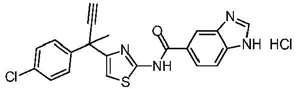 | *N* [4-[1-(4-chlorophenyl)-1-methyl-prop-2-ynyl]thiazol-2-yl]-1H-benzimidazole-5-carboxamide | ¹H NMR (400MHz, DMSO-*d*₆) *δ* 12.61 (br s, 2H), 8.50 - 8.30 (m, 2H), 7.94 (br s, 1H), 7.62 (br s, 1H), 7.47 - 7.44 (m, 2H), 7.42 - 7.38 (m, 2H), 7.17 (s, 1H), 3.55 (s, 1H), 1.92 (s, 3H) | 407.2 | | |
| B106 | 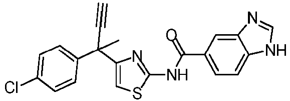 | *N*-[4-[1-(4-chlorophenyl)-1-methyl-prop-2-ynyl]thiazol-2-yl]-1H-benzimidazole-5-carboxamide | ¹H NMR (400MHz, DMSO-*d*₆) *δ* 12.90 - 12.70 (m, 1H), 12.62 (br d, *J*=9.3 Hz, 1H), 8.49 - 8.39 (m, 1H), 8.37 - 8.31 (m, 1H), 7.94 (ddd, *J*=1.4, 8.5, 14.6 Hz, 1H), 7.74 - 7.59 (m, 1H), 7.47 - 7.44 (m, 2H), 7.42 - 7.38 (m, 2H), 7.18 (d, *J*=2.0 Hz, 1H), 3.55 (s, 1H), 1.93 (s, 3H) | 407.2 | | |

**Table 6: The following examples were synthesized analogous to the procedure of example 20,21 22,23 and 24 using the appropriate intermediates and the corresponding fragment**

| Com. ID | Structure | Name | HNMR | LCMS ([M+H] ⁺=) | Kinase assay IC50 nM | NFkB assay IC50 nM |
|---|---|---|---|---|---|---|
| C001 | 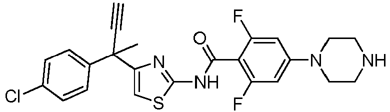 | N-(4-(2-(4-chlorophenyl)but-3-yn-2-yl)thiazol-2-yl)-2,6-difluoro-4-(piperazin-1-yl)benzamide | ¹H NMR (400 MHz, DMSO-*d*₆) *δ* 12.77 (s, 1 H), 9.25 (br s, 2 H), 7.33 - 7.49 (m, 4H), 7.21 (s, 1 H), 6.78 (br d, J = 12.05 Hz, 2 H), 3.52 - 3.58 (m, 5 H), 3.16 (br s, 4H), 1.90 (s, 3 H) | 487 | 3.7 | 0.59 |
| C002 | 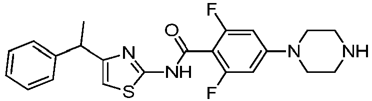 | 2,6-difluoro-N-(4-(1-phenylethyl)thiazol-2-yl)-4-(piperazin-1-yl)benzamide | ¹H NMR (400MHz, DMSO-*d*₆) *δ* 12.62 (br s, 1H), 9.37 (br s, 2H), 7.35 - 7.11 (m, 5H),6.95 (s, 1H), 6.79 (d, J=12.0 Hz, 2H), 4.16 (br s, 4H), 3.62 - 3.52 (m, 4H), 1.56 (d, J=7.3 Hz, 3H) | 429.1 | 47 | 0.83 |
| C003 | 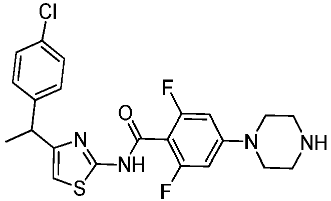 | N-(4-(1-(4-chlorophenyl)ethyl)thi azol-2-yl)-2,6-difluoro-4-(piperazin-1-yl)benzamide | ¹H NMR (400 MHz, DMSO-*d*₆) *δ* 12.62 (br s, 1 H), 9.43 (br s, 2 H), 7.37 - 7.31 (m, 2 H), 7.29 - 7.22 (m, 2 H), 6.97 (s, 1 H), 6.79 (br d, J = 12.0 Hz, 2 H), 4.19 (q, J = 7.2 Hz, 1 H), 3.64 - 3.54 (m, 4 H), 3.16 (br s, 4 H), 1.55 (d, J= 7.2 Hz, 3 H) | 463.1 | 11 | 1.34 |
| C004 | 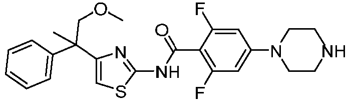 | 2,6-difluoro-N-(4-(1-methoxy-2-phenylpropan-2-yl)thiazol-2-yl)-4-(piperazin-1-yl)benzamide | ¹H NMR (400 MHz, DMSO-*d*₆) *δ* 12.64 (br. s,1H), 9.46 (s,1H), 7.28 - 7.20 (m. 2H), 7.18 - 7.16 (m. 2H), 6.98 (s,1H), 6.79 - 6.76 (m, 2H), 3.84 (s,2H), 3.59 - 3.56 (m, 4H), 3.26 (s, 3H), 3.16 - 3.14 (m, 4H), 1.67 (s, 3H). | 473.2 | 22 | 0.66 |
| C005 | 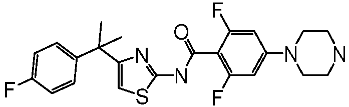 | 2,6-difluoro-N-(4-(2-(4-fluorophenyl)propan-2-yl)thiazol-2-yl)-4-(piperazin-1-yl)benzamide | ¹H NMR (400 MHz, DMSO-*d*₆) *δ* 12.65 (s, 1 H), 9.01 (s, 2 H) , 7.27 - 7.20 (m, 2 H), 7.11 - 7.04 (m, 2 H), 6.79 (s, 1 H), 6.76 (d, 1 H), 3.58 - 3.52 (m, 4 H), 3.20 - 3.12 (m, 4 H), 1.62 (s, 6H) | 461.2 | 6 | 0.68 |
| C006 | 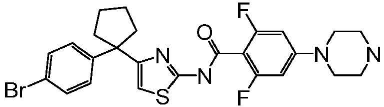 | N-(4-(1-(4-bromophenyl)cyclope ntyl)thiazol-2-yl)-2,6-difluoro-4-(piperazin-1-yl)benzamide | ¹H NMR (400 MHz, DMSO-*d*₆) *δ* 7.42 (d, 2 H),7.22 (d, 2 H), 7.04 (s, 1 H), 6.62 (s, 2 H), 3.18 (t, 4 H), 2.76 (t, 4 H), 2.45 (t, 4 H), 2.02 (t, 2 H), 1.62 (t, 2H) | 547.1 | 43 | 2.53 |
| C007 | 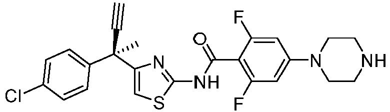 | (R)-N-(4-(2-(4-chlorophenyl)but-3-yn-2-yl)thiazol-2-yl)-2,6-difluoro-4-(piperazin-1-yl)benzamide | ¹H NMR (400 MHz, DMSO-*d*₆) *δ* 12.75 (s, 1 H), 9.42 (br. s, 2 H), 7.45 - 7.35 (m, 4 H), 7.20 (s, 1 H), 6.79 (s, 1 H), 6.76 (s, 1 H), 3.60 - 3.55 (m, 4 H), 3.54 (s, 1 H), 2.51 - 2.49 (m, 4H), 1.90 (s, 3 H) | 487.1 | 10 | 2.40 |
| C008 | 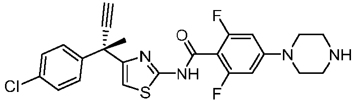 | (S)-N-(4-(2-(4-chlorophenyl)but-3-yn-2-yl)thiazol-2-yl)-2,6-difluoro-4-(piperazin-1-yl)benzamide | ¹H NMR (400 MHz, DMSO-*d*₆) *δ* 12.75 (s, 1 H), 9.47 (br. s, 2 H), 7.46 - 7.36 (m, 4 H), 7.20 (s, 1 H), 6.79 (s, 1 H), 6.76 (s, 1 H), 3.61 - 3.55 (m, 4 H), 3.54 (s, 1 H), 3.19 - 3.11 (m, 4H), 1.89 (s, 3 H) | 487.1 | 5 | 0.32 |
| C009 | 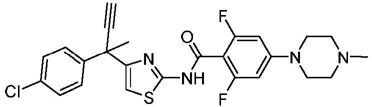 | N-(4-(2-(4-chlorophenyl)but-3-yn-2-yl)thiazol-2-yl)-2,6-difluoro-4-(4-methylpiperazin-1-yl)benzamide | ¹H NMR (400 MHz, DMSO-*d*₆) *δ* 2.76 (s, 1H), 7.34-7.47 (m, 4H), 7.20 (s, 1H), 6.75 (br d, J=12.2 Hz, 2H), 3.56-3.66 (m, 2H), 3.54 (s, 1H), 3.42-3.52 (m, 2H), 2.99-3.16 (m, 4H), 2.74 (s, 3H), 1.90 (s, 3H). | 501.1 | 7 | 0.70 |
| C010 | 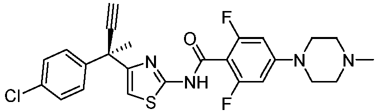 | (R)-N-(4-(2-(4-chlorophenyl)but-3-yn-2-yl)thiazol-2-yl)-2,6-difluoro-4-(4-methylpiperazin-1-yl)benzamide | ¹H NMR (400 MHz, DMSO-*d*₆) *δ* 12.79 (s, 1 H), 10.96 (br. s, 1 H), 7.45 - 7.35 (m, 4 H), 7.21 (s, 1 H), 6.82 (s, 1 H), 6.79 (s, 1 H), 4.09 - 4.00 (m, 2 H), 3.58 (s, 1 H), 3.49- 3.40 (m, 2 H), 3.30 - 3.20 (m, 2 H), 3.13 - 3.01 (m, 2H), 2.82 - 2.76 (m, 3 H), 1.90 (s, 3 H) | 501.1 | 56 | 1.49 |
| C011 | 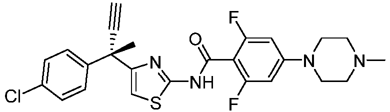 | (S)-N-(4-(2-(4-chlorophenyl)but-3-yn-2-yl)thiazol-2-yl)-2,6-difluoro-4-(4-methylpiperazin-1-yl)benzamide | ¹H NMR (400 MHz, DMSO-*d*₆) *δ* 12.79 (s, 1 H), 11.02 (br. s, 1 H), 7.47 - 7.35 (m, 4 H), 7.21 (s, 1 H), 6.82 (s, 1 H), 6.79 (s, 1 H), 4.09 - 4.00 (m, 2 H), 3.56 (s, 1 H), 3.49- 3.40 (m, 2 H), 3.32 - 3.20 (m, 2 H), 3.13 - 3.01 (m, 2H), 2.82 - 2.76 (m, 3 H), 1.90 (s, 3 H) | 501.1 | 18 | 0.44 |
| C012 | 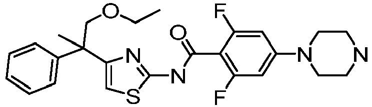 | N-(4-(1-ethoxy-2-phenylpropan-2-yl)thiazol-2-yl)-2,6-difluoro-4-(piperazin-1-yl)benzamide | ¹H NMR (400 MHz, DMSO-*d*₆) *δ* 7.22-7.18 (m, 5H), 6.89 (s, 1H), 6.61 (d, J = 12 Hz, 2H), 3.83 (s, 2H), 3.42-3.40 (m, 2H), 3.14 (t, 4H), 2.73 (t, 4H), 1.64 (s, 3H), 1.01 (s, 3H). | 487.1 | 78 | 1.45 |
| C013 | 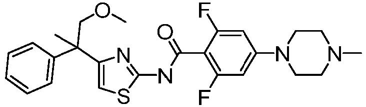 | N-(4-(1-ethoxy-2-phenylpropan-2-yl)thiazol-2-yl)-2,6-difluoro-4-(4-methylpiperazin-1-yl)benzamide | ¹H NMR (400 MHz, DMSO-*d*₆) *δ* 7.24-7.15 (m, 5H), 6.89 (s, 1H), 6.66 (d, J = 12 Hz, 2H), 3.80 (s, 2H), 3.25 (s, 4H), 3.22 (s, 3H), 2.35 (s, 4H), 2.17 (s, 3H), 1.63 (s, 3H). | 487.1 | 91 | 1.05 |
| C014 | 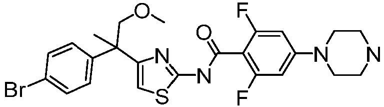 | N-(4-(2-(4-bromophenyl)-1-methoxypropan-2-yl)thiazol-2-yl)-2,6-difluoro-4-(piperazin-1-yl)benzamide | ¹H NMR (400 MHz, DMSO-*d*₆) *δ* 12.80 (s, 1 H), 7.81 (d, 2 H), 7.07 (d, 2 H), 7.03 (s, 2 H), 6.74 (s, 1 H), 3.81 (s, 2 H), 3.23 (s, 3 H), 3.18 (t, 4 H), 2.76 (t, 4 H), 1.72 (s, 3 H) | 551.1 | 13 | 1.19 |
| C015 | 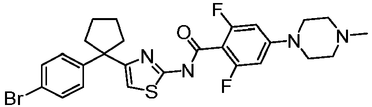 | N-(4-(1-(4-bromophenyl)cyclope ntyl)thiazol-2-yl)-2,6-difluoro-4-(4-methylpiperazin-1-yl)benzamide | ¹H NMR (400 MHz, DMSO-*d*₆) *δ 7.42* (d, J = 8 Hz, 2H), 7.20 (d, J = 4 Hz, 2H), 7.03 (s, 1H), 6.65 (d, J = 12 Hz, 2H), 3.38 (s, 4H), 2.35 (s, 4H), 2.17 (s, 3H), 2.01 (s, 4H), 1.62 (d, J = 8 Hz, 4H). | 561.1 | 266 | 17.01 |
| C016 | 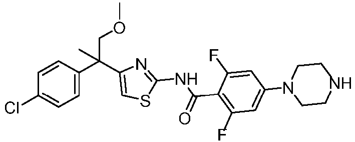 | N-(4-(2-(4-chlorophenyl)-1-methoxypropan-2-yl)thiazol-2-yl)-2,6-difluoro-4-(piperazin-1-yl)benzamide | ¹H NMR (400 MHz, DMSO-*d*₆) *δ* 12.64 (s, 1 H), 9.39 (s, 2 H), 7.34 - 7.29 (m, 2 H) 7.22 - 7.19 (m, 2 H), 6.99 (s, 1 H), 6.78 (d, *J* =12.0 Hz, 2 H), 3.80 - 3.77 (m, 2 H), 3.60 - 3.55 (m, 4 H) 3.1 (s, 3 H) 3.18 - 3.12 (m, 4 H), 1.65 (s, 3 H) | 507.1 | 16 | 1.11 |
| C017 | 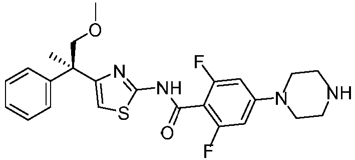 | (R)-2,6-difluoro-N-(4-(1-methoxy-2-phenylpropan-2-yl)thiazol-2-yl)-4-(piperazin-1-yl)benzamide | ¹H NMR (400 MHz, DMSO-*d*₆) *δ* 7.30 - 7.20 (m, 5H), 6.96 (s, 1H), 6.70 - 6.60 (m, 2H), 3.84 (s, 2H), 3.27 (s, 3H), 3.25 - 3.17 (m, 3H), 2.83 - 2.75 (m, 3H), 1.67 (s, 3H) | 473.1 | 67 | 1.38 |
| C018 | 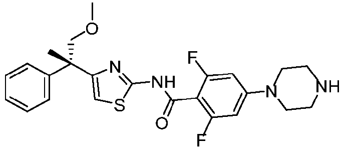 | (S)-2,6-difluoro-N-(4-(1-methoxy-2-phenylpropan-2-yl)thiazol-2-yl)-4-(piperazin-1-yl)benzamide | ¹H NMR (400 MHz, DMSO-*d*₆) *δ* 7.29 - 7.23 (m, 2H), 7.21 - 7.13 (m, 3H), 6.98 (s, 1H), 6.79 - 6.72 (m, 2H), 3.84 (s, 2H), 3.50 - 3.44 (m, 4H), 3.26 (s, 3H), 3.12 - 3.06 (m, 4H), 1.67 (s, 3H) | 473.1 | 36 | 0.97 |
| C019 | 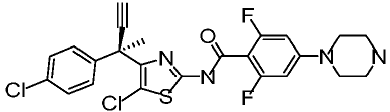 | (R)-N-(5-chloro-4-(2-(4-chlorophenyl)but-3-yn-2-yl)thiazol-2-yl)-2,6-difluoro-4-(piperazin-1-yl)benzamide | ¹H NMR (400 MHz, DMSO-*d*₆) *δ* 13.07 (s, 1 H), 9.16 (s, 2 H), 7.41 (s, 4 H), 6.83 (d, *J*=12.4 Hz, 2 H), 3.63 (s, 1 H), 3.62 - 3.57 (m, 4 H), 3.19 (br s, 4 H), 1.94 (s, 3 H). | 521.0 | | |
| C020 | 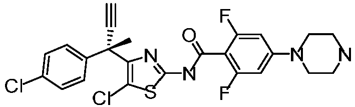 | (S)-N-(5-chloro-4-(2-(4-chlorophenyl)but-3-yn-2-yl)thiazol-2-yl)-2,6-difluoro-4-(piperazin-1-yl)benzamide | ¹H NMR (400 MHz, DMSO-*d*₆) *δ* 13.07 (s, 1 H), 9.23 (s, 2 H), 7.41 (s, 4 H), 6.83 (d, *J*=12.4 Hz, 2 H), 3.63 (s, 1 H), 3.62 - 3.57 (m, 4 H), 3.19 (br s, 4 H), 1.94 (s, 3 H) | 521.0 | 88 | |
| C021 | 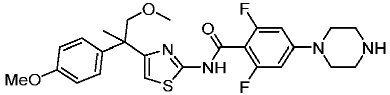 | 2,6-difluoro-N-(4-(1-methoxy-2-(4-methoxyphenyl)propa n-2-yl)thiazol-2-yl)-4-(piperazin-1-yl)benzamide | | | 44 | 0.95 |
| C022 | 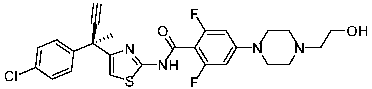 | (R)-N-(4-(2-(4-chlorophenyl)but-3-yn-2-yl)thiazol-2-yl)-2,6-difluoro-4-(4-(2-hydroxyethyl)piperazi n-1-yl)benzamide | ¹H NMR (400 MHz, DMSO-*d*₆) *δ* 12.68 (br., s, 1H), 7.42 -7.39 (m, 4 H), 7.18 (s, 1 H), 6.67 - 6.65 ( m, 2 H), 4.51 - 4.48 (m, 1 H), 3.53 (s, 1 H), 3.30 - 3.22 (m, 2 H), 2.44 - 2.39 (m, 2 H), 1.89 (s, 3 H). | 531.0 | 188 | |
| C023 | 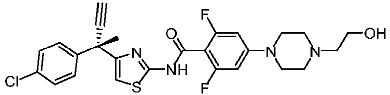 | (S)-N-(4-(2-(4-chlorophenyl)but-3-yn-2-yl)thiazol-2-yl)-2,6-difluoro-4-(4-(2-hydroxyethyl)piperazi n-1-yl)benzamide | ¹H NMR (400 MHz, DMSO-*d*₆) *δ* 12.68 (br., s, 1H), 7.50 -7.40 (m, 4 H), 7.19 (s, 1 H), 6.73 - 6.62 ( m, 2 H), 4.60 (br., s, 1H), 3.53 (s, 1 H), 3.35 - 3.25 (m, 4 H), 2.73 - 2.50 (m, 6 H), 1.90 (s, 3 H). | 531.0 | 60 | |
| C024 | 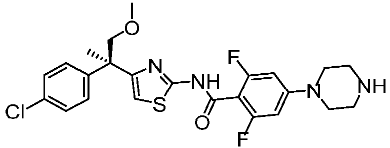 | (R)-N-(4-(2-(4-chlorophenyl)-1-methoxypropan-2-yl)thiazol-2-yl)-2,6-difluoro-4-(piperazin-1-yl)benzamide | ¹H NMR (400 MHz, DMSO-*d*₆) *δ* 12.64 (s, 1 H), 9.41 (s, 2 H), 7.32 (d, J = 8.4 Hz, 2 H) 7.22 (d, J = 8.8 Hz, 2 H), 6.99 (s, 1 H), 6.78 (d, J = 12.0 Hz, 2 H), 3.87 - 3.78 (m, 2 H) 3.62 - 3.55 (m, 4 H), 3.26 (s, 3 H), 3.20 - 3.13 (m, 4 H), 1.66 (s, 3 H) | 507.1 | 130 | |
| C025 | 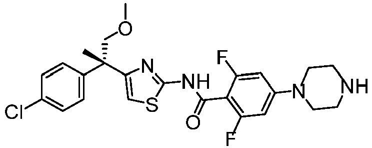 | (S)-N-(4-(2-(4-chlorophenyl)-1-methoxypropan-2-yl)thiazol-2-yl)-2,6-difluoro-4-(piperazin-1-yl)benzamide | ¹H NMR (400 MHz, DMSO-*d*₆) *δ* 12.64 (s, 1 H), 9.42 (s, 2 H), 7.32 (d, *J* = 8.4 Hz, 2 H) 7.21 (d, *J* = 8.8 Hz, 2 H), 6.99 (s, 1 H), 6.78 (d, *J =* 12.0 Hz, 2 H), 3.87 - 3.76 (m, 2 H) 3.58 - 3.56 (m, 4 H), 3.26 (s, 3 H) 3.20 - 3.13 (m, 4 H), 1.65 (s, 3 H) | 507.1 | 8 | |
| C026 | 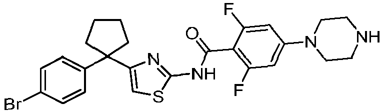 | N-(4-(1-(4-bromophenyl)cyclope ntyl)thiazol-2-yl)-2,6-difluoro-4-(piperazin-1-yl)benzamide | ¹H NMR (400 MHz, DMSO-*d*₆) *δ 7.42* (m, 2 H), 7.22 (m, 2 H), 7.04 (s, 1 H), 6.62 (d, *J =* 8.9 Hz, 2 H), 3.18 (m, 4 H), 2.76 (m, 4 H), 2.45 (s, 1 H), 2.02 (m, 2 H), 2.01 (s, 1 H), 1.62 (m, 4 H) | 547.2 | 437 | 2.53 |
| C027 | 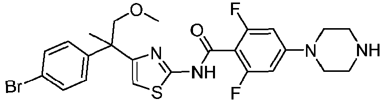 | N-(4-(2-(4-bromophenyl)-1-methoxypropan-2-yl)thiazol-2-yl)-2,6-difluoro-4-(piperazin-1-yl)benzamide | ¹H NMR (400 MHz, DMSO-*d*₆) δ 7.44 (d, *J =* 8.5 Hz, 2 H), 7.14 (d, *J =* 8.5 Hz, 2 H), 6.98 (s, 1 H), 6.74 (d, 2 H), 3.85 - 3.77 (m, 2 H), 3.51 (s, 4 H), 3.26 (s, 3 H), 3.11 (s, 4 H), 1.65 (s, 3 H) | 551.3 | 100 | 1.19 |
| C028 | 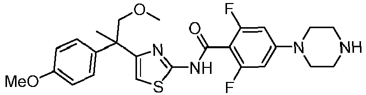 | 2,6-difluoro-N-(4-(1-methoxy-2-(4-methoxyphenyl)propa n-2-yl)thiazol-2-yl)-4-(piperazin-1-yl)benzamide | | 503.3 | 288 | 0.95 |
| C029 | 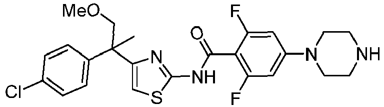 | N-(4-(2-(4-chlorophenyl)-1-methoxypropan-2-yl)thiazol-2-yl)-2,6-difluoro-4-(piperazin-1-yl)benzamide | | 507.2 | 128 | 5.05 |
| C030 | 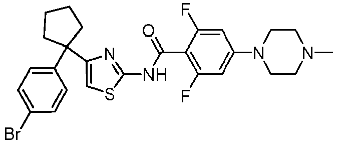 | N-(4-(1-(4-bromophenyl)cyclope ntyl)thiazol-2-yl)-2,6-difluoro-4-(4-methylpiperazin-1-yl)benzamide | ¹H NMR (400 MHz, DMSO-*d*₆) *δ 7.42* (d, *J=* 8.0 Hz, 2H), 7.22 (d, *J =* 8.0 Hz, 2H), 7.03 (s, 1H), 6.66 (d, *J =* 12.0 Hz, 2H), 3.26 (s, 4H), 2.36 (s, 4H), 2.17 (s, 3H), 2.02 (s, 4H), 1.61 (s, 4H). | 561.1 | 1770 | 17.00 |
| C031 | 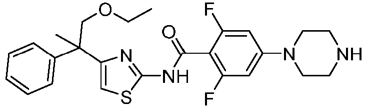 | N-(4-(1-ethoxy-2-phenylpropan-2-yl)thiazol-2-yl)-2,6-difluoro-4-(piperazin-1-yl)benzamide | ¹H NMR (400 MHz, DMSO-*d*₆) *δ* 7.24-7.12 (m, *J* = 48.0 Hz, 5H), 6.93 (s, 1H), 6.62 (d, *J =* 12.0 Hz, 2H), 3.84 (s, 2H), 3.42 (q, *J* = 16.0 Hz, 2H), 3.17 (s, 4H), 2.75 (s, 4H), 1.64 (s, 3H), 1.05 (t, *J =* 12.0 Hz, 3H). | 487.2 | 642 | 3.66 |
| C032 | 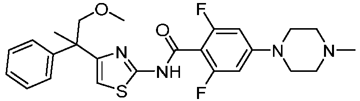 | 2,6-difluoro-N-(4-(1-methoxy-2-phenylpropan-2-yl)thiazol-2-yl)-4-(4-methylpiperazin-1-yl)benzamide | ¹H NMR (400 MHz, DMSO-*d*₆) δ 7.27-7.13 (m, *J =* 56.0 Hz, 5H), 6.89 (s, 1H), 6.66 (d, *J =* 12.0 Hz, 2H), 3.81 (s, 2H), 3.28 (s, 4H), 3.26 (s, 3H), 2.40-2.37 (m, 4H), 2.17 (s, 3H), 1.64 (s, 3H). | 487.2 | 593 | 1.05 |
| C033 | 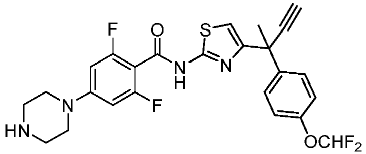 | N-(4-(2-(4-(difluoromethoxy)phe nyl)but-3-yn-2-yl)thiazol-2-yl)-2,6-difluoro-4-(piperazin-1-yl)benzamide | | 519.1 | 73 | 3.13 |
| C034 | 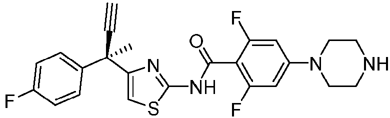 | 2,6-difluoro-N-(4-(2-(4-fluorophenyl)but-3-yn-2-yl)thiazol-2-yl)-4-(piperazin-1-yl)benzamide | ¹H NMR (400 MHz, DMSO-*d*₆) *δ* 12.76 (s, 1 H), 9.28 (br s, 2 H), 7.44 (dd, *J* = 8.91, 5.40 Hz, 2 H), 7.09 - 7.20 (m, 3 H), 6.78 (br d, *J =* 12.05 Hz, 2 H), 3.54 - 3.62 (m, 4 H), 3.53 (s, 1 H), 3.16 (br s, 4 H), 1.90 (s, 3 H). | 471.1 | 40 | 2.27 |
| C035 | 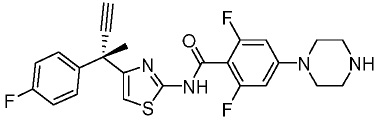 | 2,6-difluoro-N-(4-(2-(4-fluorophenyl)but-3-yn-2-yl)thiazol-2-yl)-4-(piperazin-1-yl)benzamide | ¹H NMR (400 MHz, DMSO-*d*₆) *δ* 12.76 (s, 1 H), 9.57 (br s, 2 H), 7.39 - 7.49 (m, 2 H), 7.09 - 7.20 (m, 3 H), 6.77 (br d, *J =* 12.30 Hz, 2 H), 3.54 - 3.65 (m, 4 H), 3.53 (s, 1 H), 3.15 (br s, 4 H), 1.90 (s, 3 H). | 471.1 | 19 | 0.39 |
| C036 | 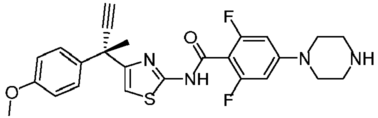 | 2,6-difluoro-N-[4-[1-(4-methoxyphenyl)-1-methyl-prop-2-ynyl]thiazol-2-yl]-4-piperazin-1-yl-benzamide | ¹H NMR (400MHz, DMSO-*d*₆) *δ* 12.76 (s, 1H), 8.89 (br s, 2H), 7.32 (d, *J*=8.8 Hz, 2H), 7.12 (s, 1H), 6.87 (d, *J*=9.0 Hz, 2H), 6.79 (d, *J*=12.1 Hz, 2H), 3.72 (s, 3H), 3.56 - 3.52 (m, 4H), 3.47 (s, 1H), 3.18 (br s, 4H), 1.88 (s, 3H). | 483.0 | 67 | 2.18 |
| C037 | 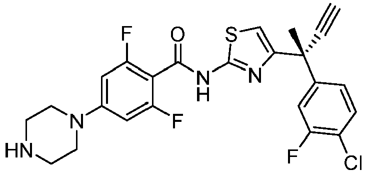 | N-(4-(2-(4-chloro-3-fluorophenyl)but-3-yn-2-yl)thiazol-2-yl)-2,6-difluoro-4-(piperazin-1-yl)benzamide | ¹H NMR (400 MHz, DMSO-*d*₆) *δ* 12.77 (s, 1 H), 9.25 (br s, 2 H), 7.56 (t, *J =* 8.17 Hz, 1 H), 7.42 (dd, *J* = 10.85, 2.15 Hz, 1 H), 7.28 (dd, *J* = 8.46, 1.91 Hz, 1 H), 7.23 (s, 1 H), 6.79 (br d, *J =* 12.16 Hz, 2 H), 3.62 (s, 1 H), 3.50 - 3.60 (m, 4 H), 3.17 (br s, 4 H), 1.91 (s, 3 H). | 505.0 | 151 | 2.30 |
| C038 | 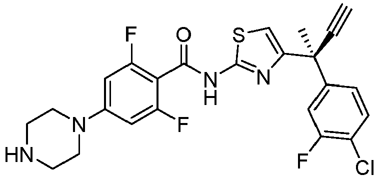 | N-(4-(2-(4-chloro-3-fluorophenyl)but-3-yn-2-yl)thiazol-2-yl)-2,6-difluoro-4-(piperazin-1-yl)benzamide | ¹H NMR (400 MHz, DMSO-*d*₆) *δ* 12.76 (s, 1 H), 9.12 (br s, 2 H), 7.56 (t, *J =* 8.16 Hz, 1 H), 7.42 (dd,*J =* 10.79, 2.01 Hz, 1 H), 7.28 (br d, *J* = 8.78 Hz, 1 H), 7.23 (s, 1 H), 6.79 (br d, *J* = 12.05 Hz, 2 H), 3.61 (s, 1 H), 3.53 - 3.59 (m, 4 H), 3.17 (br s, 4 H), 1.92 (s, 3 H). | 505.0 | 17 | 0.72 |
| C039 | 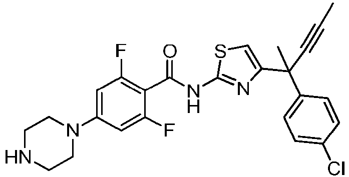 | N-(4-(2-(4-chlorophenyl)pent-3-yn-2-yl)thiazol-2-yl)-2,6-difluoro-4-(piperazin-1-yl)benzamide | ¹H NMR (400MHz, DMSO-*d*₆) *δ* 12.76 (br s, 1H), 9.42 (br s, 2H), 7.45 - 7.40 (m, 2H), 7.40 - 7.35 (m, 2H), 7.17 (s, 1H), 6.78 (d, *J*=12.3 Hz, 2H), 3.65 - 3.52 (m, 4H), 3.23 - 3.10 (m, 4H), 1.89 (s, 3H), 1.87 (s, 3H) | 501.2 | 36 | 2.28 |
| C040 | 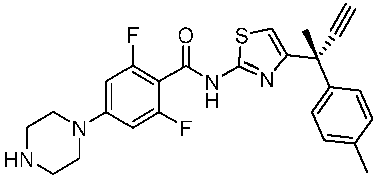 | 2,6-difluoro-N-[4-\|1-methyl-1-(p-tolyl)prop-2-ynyl]thiazol-2-yl]-4-piperazin-1-yl-benzamide | ¹H NMR (400MHz, DMSO-*d*₆) *δ* 7.30 (d, *J*=8.1 Hz, 2H), 7.16 - 7.07 (m, 3H), 6.71 (br d, *J*=12.4 Hz, 2H), 3.47 (s, 1H), 3.39 - 3.34 (m, 4H), 3.00 - 2.89 (m, 4H), 2.25 (s, 3H), 1.88 (s, 3H). | 467.0 | 29 | 1.27 |
| C041 | 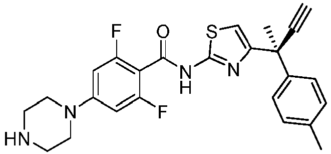 | 2,6-difluoro-N-[4-\|1-methyl-1-(p-tolyl)prop-2-ynyl]thiazol-2-yl]-4-piperazin-1-yl-benzamide | ¹H NMR (400MHz, DMSO-*d*₆) *δ* 7.30 (d, *J*=8.1 Hz, 2H), 7.13 - 7.10 (m, 3H), 6.64 (d, *J*=12.6 Hz, 2H), 3.45 (s, 1H), 3.24 - 3.13 (m, 6H), 2.78 - 2.75 (m, 4H), 2.26 (s, 3H), 1.88 (s, 3H). | 467.0 | 7 | 0.60 |
| C042 | 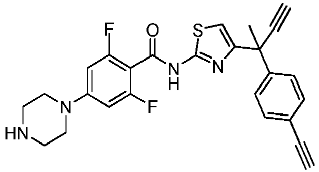 | N-[4-[1-(4-ethynylphenyl)-1-methyl-prop-2-ynyl]thiazol-2-yl]-2,6-difluoro-4-piperazin-1-yl-benzamide | ¹H NMR (400MHz, DMSO-*d*₆) *δ* 12.76 (br s, 1H), 9.52 (br s, 2H), 7.47 - 7.41 (m, 4H), 7.21 (s, 1H), 6.77 (br d, *J*=12.1 Hz, 2H), 4.16 (s, 1H), 3.58 (br s, 4H), 3.15 (br s, 4H), 1.90 (s, 3H). | 477.1 | 101 | 0.61 |
| C043 | 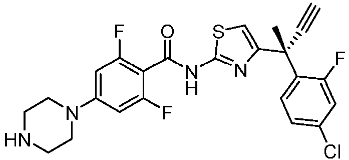 | N-(4-(2-(4-chloro-2-fluorophenyl)but-3-yn-2-yl)thiazol-2-yl)-2,6-difluoro-4-(piperazin-1-yl)benzamide | ¹H NMR (400 MHz, DMSO-*d*₆) *δ* 12.75 (s, 1 H), 9.18 (br s, 2 H), 7.67 (t, *J =* 8.8 Hz, 1 H), 7.39 - 7.29 (m, 2 H), 7.19 (s, 1 H), 6.78 (br d, *J*=12.0 Hz, 2 H), 3.59 (s, 1 H), 3.58 - 3.55 (m, 4 H), 3.17 (br s, 4 H), 1.94 (s, 3 H) | 505.1 | 285 | 3.47 |
| C044 | 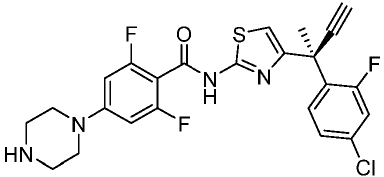 | N-(4-(2-(4-chloro-2-fluorophenyl)but-3-yn-2-yl)thiazol-2-yl)-2,6-difluoro-4-(piperazin-1-yl)benzamide | ¹H NMR (400 MHz, DMSO-*d*₆) *δ* 12.76 (s, 1 H), 9.14 (br s, 2 H), 7.67 (t, *J* =8.8 Hz, 1 H), 7.38 - 7.31 (m, 2 H), 7.20 (s, 1 H), 6.78 (d, *J*=12.4 Hz, 2 H), 3.60 (s, 1 H) 3.58 - 3.53(m, 4 H), 3.18 (br s, 4 H), 1.95 (s, 3 H) | 505.1 | 25 | 1.45 |
| C045 | 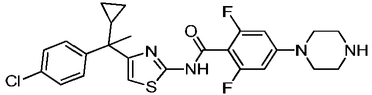 | N-(4-(1-(4-chlorophenyl)-1-cyclopropylethyl)thiaz ol-2-yl)-2,6-difluoro-4-(piperazin-1-yl)benzamide | ¹H NMR (400 MHz, DMSO-*d*₆) *δ* 12.61 (s, 1 H), 9.26 (br s, 2 H), 7.34 - 7.29 (m, 2 H), 7.20 (d, *J =* 8.8 Hz, 2 H), 7.14 (s, 1 H), 6.77 (d, *J =* 12.4 Hz, 2 H), 3.62 - 3.49 (m, 4 H), 3.16 (br s, 4 H), 1.53- 1.46 (m, 1 H), 1.42 (s, 3 H) 0.58 - 0.51 (m, 1 H), 0.49 - 0.42 (m, 1 H), 0.29 - 0.22 (m, 1 H), 0.21 - 0.14 (m, 1 H) | 503.1 | 25 | 1.63 |
| C046 | 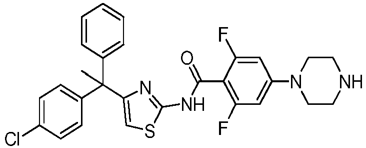 | N-(4-(1-(4-chlorophenyl)-1-phenylethyl)thiazol-2-yl)-2,6-difluoro-4-(piperazin-1-yl)benzamide | ¹H NMR (400 MHz, DMSO-*d*₆) *δ* 12.70 (s, 1 H), 9.21 (br s, 2 H), 7.37 - 7.28 (m, 4 H), 7.25 - 7.20 (m, 1 H), 7.15 - 7.08 (m, 4 H), 6.79 (d, *J*,=12.0 Hz, 2 H), 6.60 (s, 1 H) 3.60 - 3.56 (m, 4 H), 3.17 (br s, 4 H) 2.07 (s, 3 H) | 539.2 | 377 | 5.5 |
| C047 | 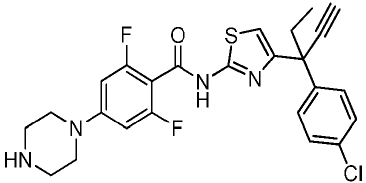 | N-(4-(3-(4-chlorophenyl)pent-1-yn-3-yl)thiazol-2-yl)-2,6-difluoro-4-(piperazin-1-yl)benzamide | ¹H NMR (400MHz, DMSO-*d*₆) *δ* 12.75 (s, 1H), 9.23 (br s, 2H), 7.49 - 7.43 (m, 2H), 7.41 - 7.36 (m, 2H), 7.21 (s, 1H), 6.78 (d, *J* = 12.0 Hz, 2H), 3.61 (s, 1H), 3.59 - 3.53 (m, 4H), 3.17 (s, 4H), 2.40 - 2.30 (m, 1H), 2.23 - 2.14 (m, 1H), 0.88 - 0.81 (m, 3H). ¹⁹F NMR (376MHz, DMSO-*d*₆) *δ* -111.77 (s, 2F) | 501.1 | 131 | 2.65 |
| C048 | 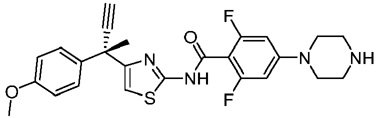 | 2,6-difluoro-N-(4-(2-(4-methoxyphenyl)but-3-yn-2-yl)thiazol-2-yl)-4-(piperazin-1-yl)benzamide | ¹H NMR (400MHz, DMSO-*d*₆) *δ* 12.75 (s, 1H), 9.55 (br s, 2H), 7.32 - 7.27 (m, 2H), 7.11 (s, 1H), 6.87 - 6.82 (m, 2H), 6.79 - 6.71 (m, 2H), 3.71 (s, 3H), 3.60 - 3.52 (m, 4H), 3.46 (s, 1H), 3.16 - 3.07 (br s, 4H), 1.88 (s, 3H). | 483 .1 | 16 | 0.41 |
| C049 | 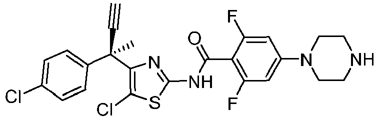 | N-(5-chloro-4-(2-(4-chlorophenyl)but-3-yn-2-yl)thiazol-2-yl)-2,6-difluoro-4-(piperazin-1-yl)benzamide | ¹H NMR (400 MHz, DMSO-*d*₆) *δ* 13.07 (s, 1 H), 9.16 (br s, 2 H), 7.41 (s, 4 H), 6.83 (d, *J =* 12.4 Hz, 2 H), 3.63 (s, 1 H), 3.62 - 3.57 (m, 4 H), 3.19 (br s, 4 H), 1.94 (s, 3 H) | 521.1 | 4524 | |
| C050 | 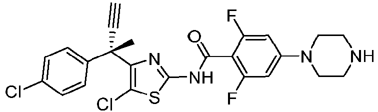 | N-(5-chloro-4-(2-(4-chlorophenyl)but-3-yn-2-yl)thiazol-2-yl)-2,6-difluoro-4-(piperazin-1-yl)benzamide | ¹H NMR (400 MHz, DMSO-*d*₆) *δ* 13.07 (br s, 1 H), 9.23 (br s, 2 H), 7.41 (s, 4 H), 6.83 (br d, *J* =12.4 Hz, 2 H), 3.63 (s, 1 H) 3.62 - 3.57 (m, 4 H), 3.19 (br s, 4 H), 1.94 (s, 3 H) | 521.1 | 923 | |
| C051 | 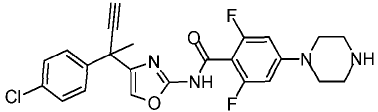 | N-(4-(2-(4-chlorophenyl)but-3-yn-2-yl)oxazol-2-yl)-2,6-difluoro-4-(piperazin-1-yl)benzamide | ¹H NMR (400 MHz, CD₃OD *-d*₆) *δ* 7.64 (s, 1H), 7.58 (br d, *J* = 8.0 Hz, 2H), 7.39 - 7.29 (m, 2H), 6.72 (br d, *J =* 11.8 Hz, 2H), 3.65 - 3.54 (m, 4H), 3.42 - 3.35 (m, 4H), 2.98 (s, 1H), 1.90 (s, 3H) | 471.3 | 26 | 0.39 |
| C052 | 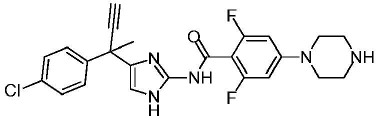 | *N*-(4-(2-(4-chlorophenyl)but-3-yn-2-yl)-1H-imidazol-2-yl)-2,6-difluoro-4-(piperazin-1-yl)benzamide | ¹H NMR (400 MHz, CD₃OD *-d₆) δ* 7.52 (br d, *J =* 8.4 Hz, 2H), 7.40 (br d, *J =* 8.4 Hz, 2H), 7.22 (s, 1H), 6.76 (br d, *J =* 12.4 Hz, 2H), 3.65 - 3.59 (m, 4H), 3.39 - 3.32 (m, 4H), 3.20 (s, 1H), 1.97 (s, 3H) | 470.3 | 44 | 1.03 |

**Table 7. The following examples were synthesized analogous to the procedure of example 21 and 24 using the appropriate intermediates and the corresponding fragment**

| Com. ID | Structure | Name | HNMR | LCMS ([M+H]⁺ =) | Kinase assay IC50 nM | NFkB assay IC50 nM |
|---|---|---|---|---|---|---|
| D001 | 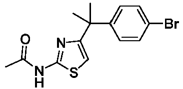 | N-(4-(2-(4-bromophenyl)propan-2-yl)thiazol-2-yl)acetamide | ¹H NMR (400 MHz, DMSO-*d*₆) *δ* 12.00 (s, 1H), 7.40 (d, J = 8.6 Hz, 2H), 7.11 (d, J = 8.5 Hz, 2H), 6.87 (s, 1H), 2.02 (s, 3H), 1.57 (s, 6H). | 339.0 | 100 | 2.01 |
| D002 | 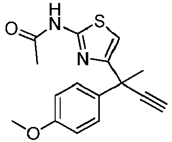 | N-(4-(2-(4-methoxyphenyl)but-3-yn-2-yl)thiazol-2-yl)acetamide | ¹H NMR (400 MHz, DMSO-*d*₆) *δ* 12.15 (s, 1 H), 7.30 (d, J = 8.8 Hz, 2 H), 6.99 (s, 1 H), 6.86 (d, J = 8.8 Hz, 2 H), 3.71 (s, 3 H), 3.42 (s, 1 H), 2.06 (s, 3 H), 1.85 (s, 3 H). | 301.1 | 196 | 1.14 |
| D003 | 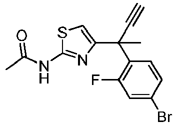 | N-(4-(2-(4-bromo-2-fluorophenyl)but-3-yn-2-yl)thiazol-2-yl)acetamide | ¹H NMR (400 MHz, DMSO-*d*₆) *δ* 12.14 (s, 1H), 7.59 (t, J = 8.4 Hz, 1H), 7.45 (d, J = 9.5 Hz, 2H), 7.09 - 7.06 (m, 1H), 3.58 - 3.54 (m, 1H), 2.07 (s, 3H), 1.92 (s, 3H) | 367.0 | 183 | 1.72 |
| D004 | 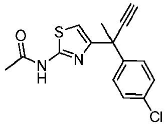 | N-(4-(2-(4-chlorophenyl)but-3-yn-2-yl)thiazol-2-yl)acetamide | ¹H NMR (400MHz, CDCl₃) *δ* 9.98 (br s, 1H), 7.45 (d, J = 8.5 Hz, 2H), 7.30 (d, J = 8.5 Hz, 2H), 6.88 (s, 1H), 2.63 (s, 1H), 2.25 (s, 3H), 1.99 (s, 3H) | 305.0 | 18 | 0.75 |
| D005 | 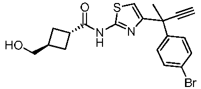 | rac-(1r,3r)-N-(4-(2-(4-bromophenyl)but-3-yn-2-yl)thiazol-2-yl)-3-(hydroxymethyl)cyclobutanec arboxamide | ¹H NMR (400 MHz, CDCl₃) *δ* 8.67 (br s, 1 H), 7.42 - 7.45 (m, 2 H), 7.34 - 7.38 (m, 2 H), 6.90 (s, 1 H),3.70 (br d, J=3.6 Hz, 2 H), 3.11 - 3.20 (m, 1 H), 2.56 - 2.64 (m, 2 H) 2.45 - 2.53 (m, 2 H), 2.09 - 2.17 (m, 2 H), 1.95 (s, 3 H), 1.43 (br s, 1 H) | 419.0 | 98 | 0.63 |
| D006 | 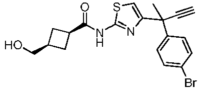 | rac-(1s,3s)-N-(4-(2-(4-bromophenyl)but-3-yn-2-yl)thiazol-2-yl)-3-(hydroxymethyl)cyclobutanec arboxamide | ¹H NMR (400 MHz, CDCl₃) *δ* 13.28 (s, 1 H), 7.48 - 7.56 (m, 4 H), 6.91 (s, 1 H), 3.65 (d, J = 4.4 Hz, 2 H), 3.32 (t, J=7.6 Hz, 1 H), 2.78 (s, 1 H), 2.65 - 2.55 (m, 1 H), 2.43 - 2.52 (m, 2 H), 2.34 - 2.32 (m, 2 H), 2.15 (s, 3 H), | 419.0 | 40 | 0.82 |
| D007 | 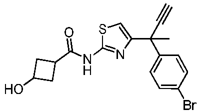 | N-(4-(2-(4-bromophenyl)but-3-yn-2-yl)thiazol-2-yl)-3-hydroxycyclobutanecarboxam ide | ¹H NMR (400 MHz, DMSO-*d*₆) *δ* 12.10 (s, 1 H) 7.48 - 7.54 (m, 2 H) 7.34 (d, J=8.53 Hz, 2 H) 7.09 (s, 1 H) 5.19 (d, J=7.03 Hz, 1 H) 3.90 - 4.01 (m, 1 H) 3.51 (s, 1 H) 3.33 (s, 3 H) 2.63 - 2.71 (m, 1 H) 2.29 - 2.37 (m, 2 H) 1.94 - 2.04 (m, 2 H) 1.86 (s, 3 H) | 405.0 | 55 | 0.97 |
| D008 | 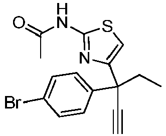 | N-(4-(3-(4-bromophenyl)pent-1-yn-3-yl)thiazol-2-yl)acetamide | ¹H NMR (400 MHz, CDCl₃) *δ* 8.77 (s, 1 H), 7.45 - 7.38, (m, 4 H), 6.95 (s, 1 H), 2.63 (s, 1 H), 2.43 - 2.34(m, 1 H), 2.24-2.14 (m, 4 H), 0.93 (t, J = 7.2 Hz, 3 H) | 363.0 | 143 | 2.11 |
| D009 | 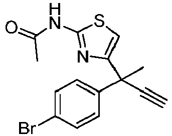 | N-(4-(2-(4-bromophenyl)but-3-yn-2-yl)thiazol-2-yl)acetamide | ¹H NMR (400MHz, CDCl₃) *δ* 8.79 (br s, 1H), 7.46 - 7.39 (m, 2H), 7.39 - 7.31 (m, 2H), 6.90 (s, 1H), 2.58 (s, 1H), 2.20 (s, 3H), 1.95 (s, 3H) | 350.9 | 16 | 0.96 |
| D010 | 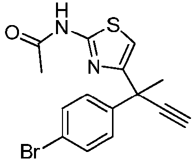 | (R)-N-(4-(2-(4-bromophenyl)but-3-yn-2-yl)thiazol-2-yl)acetamide | ¹H NMR (400MHz, CDCl₃) *δ* 8.74 (br s, 1H), 7.51 - 7.40 (m, 2H), 7.40 - 7.31 (m, 2H), 6.90 (s, 1H), 2.58 (s, 1H), 2.21 (s, 3H), 1.95 (s, 3H) | 350.9 | 102 | 6.38 |
| D011 | 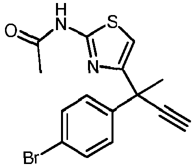 | (S)-N-(4-(2-(4-bromophenyl)but-3-yn-2-yl)thiazol-2-yl)acetamide | ¹H NMR (400MHz, CDCl₃) *δ* 8.78 (br s, 1H), 7.48 - 7.40 (m, 2H), 7.39 - 7.30 (m, 2H), 6.90 (s, 1H), 2.58 (s, 1H), 2.21 (s, 3H), 1.95 (s, 3H) | 350.8 | 14 | 0.39 |

### Example 25: 6-((2-(dimethylamino)ethyl)amino)-N-(4-(2-(4-methoxyphenyl)propan-2-yl)thiazol-2-yl)nicotinamide

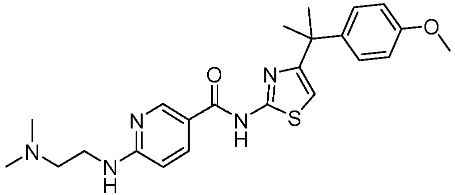

### Step 1. Preparation of compound 6-chloro-N-(4-(2-(4-methoxyphenyl)propan-2-yl)thiazol-2-yl)nicotinamide

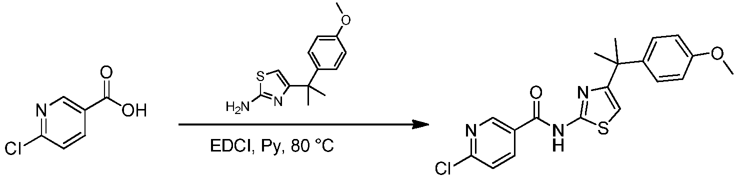

A mixture of compound 4-(2-(4-methoxyphenyl)propan-2-yl)thiazol-2-amine (100 mg, 0.35 mmol, HCl salt), compound 6-chloronicotinic acid (83.0 mg, 0.53 mmol) and EDCI (135 mg, 0.70 mmol) in pyridine (3 mL) was stirred at 80 °C for 2 h. The reaction mixture was concentrated. The residue was purified by silica gel chromatography (PE: EA=2:1). Desired compound (63 mg, 46.26% yield) was obtained as yellow oil.
MS (ESI) *m*/*z* (M+H)⁺=388.0

### Step 2. Preparation of compound 6-((2-(dimethylamino)ethyl)amino)-N-(4-(2-(4-methoxyphenyl)propan-2-yl)thiazol-2-yl)nicotinamide

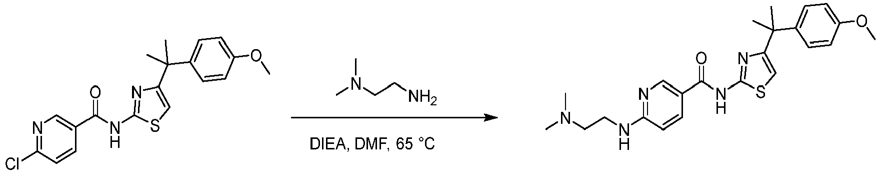

A mixture of compound obtained from step 1 above (63 mg, 0.16 mmol), N,N-dimethylethane-1,2-diamine (43.0 mg, 0.49 mmol) and DIEA (84.0 mg, 0.65 mmol) in DMF (5 mL) was stirred at 65°C for 16 h. The reaction mixture was concentrated. The residue was purified prep-HPLC (water (0.05%HCl)-ACN]). The desired compound (25.01 mg, 35.0% yield) was obtained as a yellow solid.

¹H NMR (400MHz, MeOD) *δ* 8.43 - 8.32 (m, 1H), 8.18 - 8.11 (m, 1H), 7.35 (s, 1H), 7.23 - 7.14 (m, 3H), 6.83 (d, *J =* 8.8 Hz, 2H), 6.79 (s, 1H), 3.76 (m, 4H), 1.70 (s, 6H).

MS (ESI) *m*/*z* (M+H)⁺=440.2

### Example 26: Preparation of compound 6-((4-(2-hydroxyethyl)piperazin-1-yl)methyl)-N-(4-(2-(p-tolyl)propan-2-yl)thiazol-2-yl)nicotinamide

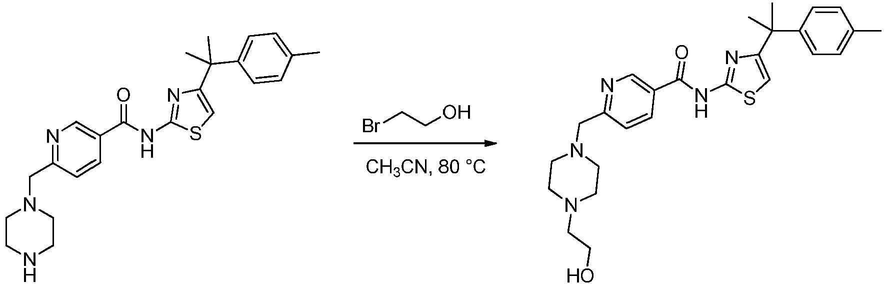

To a solution of compound 6-(piperazin-1-ylmethyl)-N-(4-(2-(p-tolyl)propan-2-yl)thiazol-2-yl)nicotinamide (0.03 g, 69 µmol, 1 *eq*) in CH₃CN (10 mL) was added 2-bromoethanol (9.47 mg, 76 µmol, 5 µL, 1.1 *eq*) and K₂CO₃ (19 mg, 137.8µmol, 2 *eq*). Then the reaction mixture was stirred at 80°C for 16 hr. The reaction was concentrated under reduced pressure to afford a residue. The residue was purified by prep-HPLC (water (0.225%FA)-ACN]; B%: 15%-45%, 7.5min). Compound (2.3 mg, yield: 6.9%) was obtained as a white solid.

¹H NMR (400MHz, CDCl₃) *δ* 9.09 - 9.05 (m, 1H), 8.33 - 8.28 (m, 1H), 7.75 (br d, *J* = 8.8 Hz, 3H), 7.45 - 7.41 (m, 1H), 7.13 - 7.08 (m, 3H), 7.06 - 7.02 (m, 1H), 6.61 - 6.57 (m, 1H), 3.83 - 3.77 (m, 3H), 3.64 - 3.55 (m, 1H), 2.45 - 2.38 (m, 8H), 2.26 - 2.18 (m, 1H), 1.63 - 1.58 (m, 3H), 1.19 (s, 6H). MS (ESI) *m*/*z* (M+H)⁺=480.3.

### Example 27: (1r,3r)-N-(4-(2-(4-bromophenyl)but-3-yn-2-yl)thiazol-2-yl)-3-(hydroxymethyl)cyclobutane-1-carboxamide

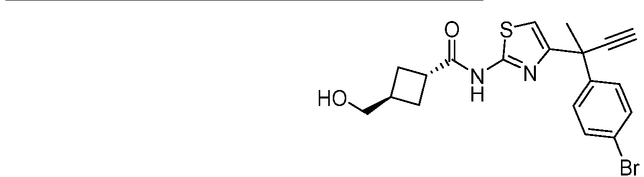

### Step 1. Preparation of compound methyl 3-(((tert-butyldiphenylsilyl)oxy)methyl) cyclobutanecarboxylate

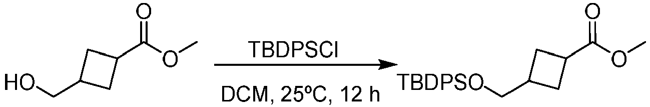

To a solution of methyl 3-(hydroxymethyl)cyclobutanecarboxylate (200 mg, 1.39 mmol) and imidazole (189 mg, 2.77 mmol) in DCM (5 mL) was added TBDPSCl (458 mg, 1.66 mmol, 427 µL) at 25°C. The solution was stirred for 12 h at 25°C. The mixture was diluted with DCM (30 mL), washed with H₂O (3 x 10 mL), brine (10 mL), dried over anhydrous Na₂SO₄, filtered and concentrated in vacuum to give a residue. The residue was purified by silica column (ethyl acetate in petroleum ether =0-20%). The desired compound (420 mg, yield: 79.1%) was obtained as a yellow oil.

MS (ESI) *m*/*z* (M+ H)⁺=383.1

### Step 2. Preparation of compound 3-(((tert-butyldiphenylsilyl)oxy)methyl) cyclobutanecarboxylic acid

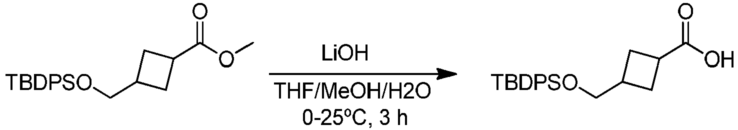

To mixture of compound obtained from step 1 above (412 mg, 1.08 mmol) in THF (1.5 mL)/MeOH (0.5 mL) /H₂O (0.5 mL) was added LiOH.H₂O (90.6 mg, 2.16 mmol) at 0°C. The mixture was stirred for 3 h at 25°C. The mixture was diluted with H₂O (15 mL), adjusted pH=6-7, extracted with EA (15 mL x 3). The combined organic layers were washed with brine (10 mL), dried over anhydrous Na₂SO₄, filtered and concentrated in vacuum to give a residue. The desired compound (413 mg, crude) was obtained as a yellow solid. The crude product was directly used for next step without further purification

MS (ESI) *m*/*z* (M+ Na)⁺=391.1

### Step 3. Preparation of compound methyl 2-(4-bromophenyl)-2-(2-((1R, 3R)-3-(((tert-butyldiphenylsilyl)oxy)methyl)cyclobutanecarboxamido)thiazol-4-yl)propanoate

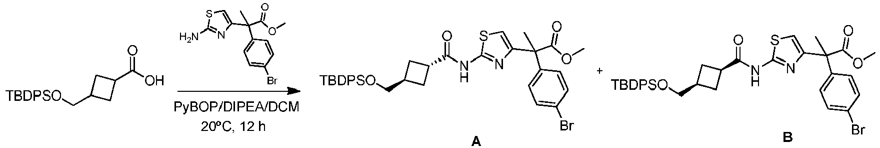

To solution of compound obtained from step 2 above (410 mg, 1.11 mmol) and DIPEA (173 mg, 1.34 mmol, 233 µL) in DCM (5 mL) was stirred for 10 min at 20°C. Methyl 2-(2-aminothiazol-4-yl)-2-(4-bromophenyl)propanoate (152 mg, 446 µmol) and PyBOP (580 mg, 1.11 mmol) was added at 20 °C. The mixture was stirred for 12 h at 20°C. The mixture was diluted with DCM (30 mL), washed with H₂O (10 mL), brine (10 mL), dried over anhydrous Na₂SO₄, filtered and concentrated in vacuum to give a residue. The residue was purified by silica column (ethyl acetate in petroleum ether=0-20%). The desired compound A (194 mg, crude) was obtained as a yellow oil. The other desired compound B (186 mg, crude) obtained as a yellow oil. The crude product was directly used for next step without further purification. The chiral of the products were confirmed in the final step.

### Step 4. Preparation of compound (1R, 3R)-N-(4-(2-(4-bromophenyl)-1-hydroxypropan-2-yl)thiazol-2-yl)-3-(((tert-butyldiphenylsilyl)oxy)methyl)cyclobutanecarboxamide

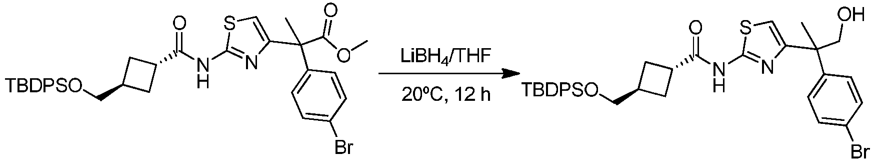

To a solution of compound obtained from step 3 above (194 mg, 280.4 µmol) in THF (5 mL) was added LiBH₄ (31 mg, 1.40 mmol) at 20 °C. The mixture was stirred at 20 °C for 12 h. The mixture was quenched with sat. NH₄Cl aq (10 mL), diluted with H₂O (20 mL) and extracted with EA (20 mL x 3). The organic layer was washed with brine (10 mL), dried over anhydrous Na₂SO₄, filtered and concentrated in vacuum to give a residue. The residue was purified by silica column (ethyl acetate in petroleum ether =0-30%). The desired compound (77 mg, yield: 41.4%) was obtained as a yellow oil.

MS (ESI) *m*/*z* (M+H)⁺=663.1

### Step 5. Preparation of compound (1R, 3R)-N-(4-(2-(4-bromophenyl)-1-oxopropan-2-yl)thiazol-2-yl)-3-(((tert-butyldiphenylsilyl)oxy)methyl)cyclobutanecarboxamide

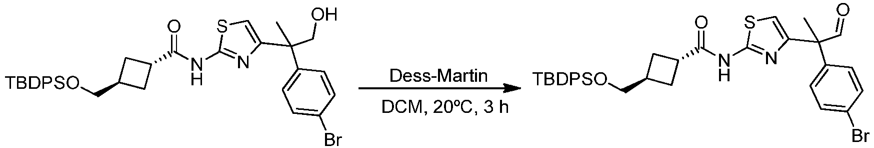

To a mixture of Dess-Martin (73 mg, 171.2 µmol, 53 µL) in DCM (2 mL) was added the solution of compound obtained from step 4 above (77 mg, 132 µmol) in DCM (2 mL) at 20 °C. The mixture was stirred for 3 h at 20 °C. The mixture was quenched with sat. NaHCO₃ (10 mL)/sat Na₂S₂O₄ (10 mL), extracted with DCM (15 mL x 3). The combined organic layers were washed with brine (10 mL), dried over anhydrous Na₂SO₄, filtered and concentrated in vacuum to give a residue. The desired compound (77 mg, crude) was obtained as a yellow solid. The crude product was directly used for next step without further purification.

### Step 6. Preparation of compound (1R, 3R)-N-(4-(2-(4-bromophenyl)but-3-yn-2-yl)thiazol-2-yl)-3-(((tert-butyldiphenylsilyl)oxy)methyl)cyclobutanecarboxamide

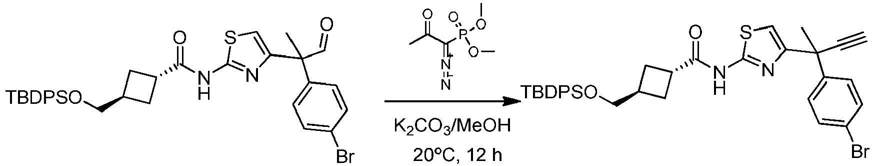

To a solution of compound obtained from step 5 above (77 mg, 116 µmol), 1-diazo-1-dimethoxyphosphoryl-propan-2-one (34 mg, 174.5 µmol) and K₂CO₃ (32 mg, 232.7 µmol) in MeOH (2 mL) was stirred for 12h at 20°C. The mixture was concentrated in vacuum to give a residue. The residue was purified by silica column (ethyl acetate in petroleum ether =0-15%). The desired compound (37 mg, yield: 48.3%) was obtained as a yellow oil.

MS (ESI) *m*/*z* (M+H)⁺=657.1

### Step 7. Preparation of compound (1R, 3R)-N-(4-(2-(4-bromophenyl)but-3-yn-2-yl)thiazol-2-yl)-3-(hydroxymethyl)cyclobutanecarboxamide

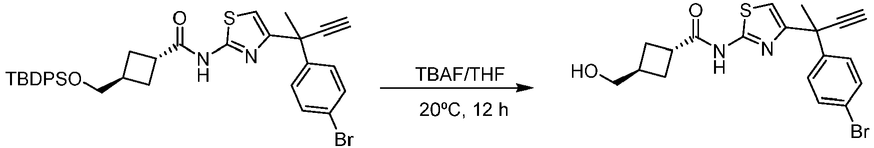

To a solution of compound obtained from step 6 above (37 mg, 56.3 µmol) in THF (2 mL) was added TBAF (1 M, 0.1 mL) at 20 °C. The mixture was stirred for 12 h at 20 °C. The mixture was diluted with EA (50 mL),washed with H₂O (10 mL X 3), brine (10 mL), dried over anhydrous Na₂SO₄, filtered and concentrated in vacuum to give a residue The residue was purified by silica column (ethyl acetate in petroleum ether =0-15%). The desired compound (12.61 mg, yield: 53.5%) was obtained as a yellow solid.

¹H NMR (400MHz, CDCl₃) δ 13.28 (br s, 1 H), 7.56 - 7.48 (m, 4 H), 6.91 (s, 1 H), 3.65 (d, *J =* 4.4 Hz, 2 H), 3.34 -3.30 (m, 1 H), 2.77 (s, 1 H), 2.63 - 2.61 (m, 1 H), 2.52 - 2.43 (m, 2 H), 2.34 - 2.32 (m, 2 H), 2.15 (s, 3 H). MS (ESI) *m*/*z* (M+H)⁺=419.0.

The other isomer was synthesized usign the similar procedure above.

### Example 28: 4-((4-(2-hydroxyethyl)piperazin-1-yl)methyl)-N-(4-(2-(4-methoxyphenyl)propan-2-yl)thiazol-2-yl)benzamide

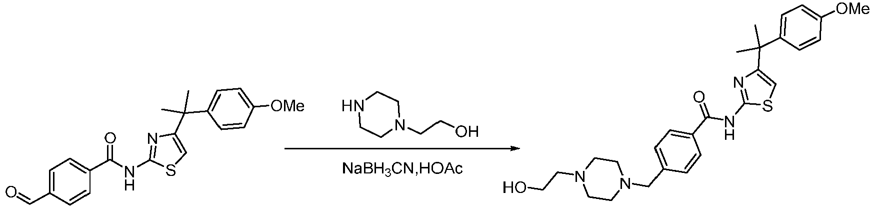

To a solution of compound 4-formyl-N-(4-(2-(4-methoxyphenyl)propan-2-yl)thiazol-2-yl)benzamide (120 mg, 0.32 mmol) and 2-(piperazin-1-yl)ethan-1-ol (42 mg, 0.32 mmol) in DCM (5 mL) was added NaBH₃CN (59 mg, 0.95 mmol) and HOAc (2 drops). The mixture was stirred at r.t overnight. The reaction mixture was concentrated to give a residue. The residue was purified by flash silica gel chromatography (DCM: MeOH = 1: 0 to 10: 1). The desired compound (80 mg, yield: 51.4%) was obtained as a white solid.

MS (ESI) m/z (M+H)⁺ = 495.2

### Methods of Use

The disclosure provides compound of Formula I, or a subembodiment thereof as described herein, for use in a method for inhibiting ALPK1 kinase activity in a cell or tissue of a subject in need of such therapy, the method comprising administering the compound to the subject.

The disclosure provides compound of Formula I, or a subembodiment thereof as described herein, for use in a method for inhibiting or reducing inflammation in a target tissue of a subject in need of such treatment, the method comprising administering the compound to the subject.
The disclosure provides compound of Formula I, or a subembodiment thereof as described herein, for use in a method for treating a disease, disorder, or condition characterized by excessive or inappropriate ALPK1-dependent proinflammatory signaling in a subject in need of such therapy, the method comprising administering the compound to the subject
   optionally wherein the disease, disorder, or condition is selected from sepsis, cancer, spiroandenoma, spiroandenocarcinoma, "Retinal dystrophy, Optic nerve edema, Splenomegaly, Anhidrosis and migraine Headache" ("ROSAH") syndrome, and "Periodic Fever, Aphthous Stomatitis, Pharyngitis, and Adenitis" ("PFAPA") syndrome.
optionally wherein the subject in need of such therapy is a subject carrying one or more genetic mutations in ALPK1.

### Pharmaceutical Compositions

In embodiments, the disclosure provides pharmaceutical compositions comprising a compound of Formula XI, or a subembodiment thereof, as described herein, and one or more carriers or excipients, preferably pharmaceutically acceptable carriers or excipients. As used herein, the phrase "pharmaceutically acceptable" refers to those compounds, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. Excipients for preparing a pharmaceutical composition are generally those that are known to be safe and non-toxic when administered to a human or animal body. Examples of pharmaceutically acceptable excipients include, without limitation, sterile liquids, water, buffered saline, ethanol, polyol (for example, glycerol, propylene glycol, liquid polyethylene glycol and the like), oils, detergents, suspending agents, carbohydrates (*e.g*., glucose, lactose, sucrose or dextran), antioxidants (*e.g*., ascorbic acid or glutathione), chelating agents, low molecular weight proteins, and suitable mixtures of any of the foregoing. The particular excipients utilized in a composition will depend upon various factors, including chemical stability and solubility of the compound being formulated and the intended route of administration.

A pharmaceutical composition can be provided in bulk or unit dosage form. It is especially advantageous to formulate pharmaceutical compositions in unit dosage form for ease of administration and uniformity of dosage. The term "unit dosage form" refers to physically discrete units suited as unitary dosages for the subject to be treated; each unit containing a predetermined quantity of an active compound calculated to produce the desired therapeutic effect in association with the required pharmaceutical carrier. A unit dosage form can be an ampoule, a vial, a suppository, a dragee, a tablet, a capsule, an IV bag, or a single pump on an aerosol inhaler.

In therapeutic applications, dose may vary depending on the chemical and physical properties of the active compound as well as clinical characteristics of the subject, including *e.g*., age, weight, and co-morbidities. Generally, the dose should be a therapeutically effective amount. An effective amount of a pharmaceutical composition is that which provides an objectively identifiable improvement as noted by the clinician or other qualified observer. For example, alleviating a symptom of a disorder, disease or condition.

A pharmaceutical composition as described herein may take any suitable form (*e.g*. liquids, aerosols, solutions, inhalants, mists, sprays; or solids, powders, ointments, pastes, creams, lotions, gels, patches and the like) for administration by any desired route (*e.g*. pulmonary, inhalation, intranasal, oral, buccal, sublingual, parenteral, subcutaneous, intravenous, intramuscular, intraperitoneal, intrapleural, intrathecal, transdermal, transmucosal, rectal, and the like). In embodiments, the pharmaceutical composition is in the form of an orally acceptable dosage form including, but not limited to, capsules, tablets, buccal forms, troches, lozenges, and oral liquids in the form of emulsions, aqueous suspensions, dispersions or solutions. Capsules may contain excipients such as inert fillers and/or diluents including starches (*e.g*., corn, potato or tapioca starch), sugars, artificial sweetening agents, powdered celluloses, such as crystalline and microcrystalline celluloses, flours, gelatins, gums, *etc.* In the case of tablets for oral use, carriers which are commonly used include lactose and corn starch. Lubricating agents, such as magnesium stearate, can also be added.

In embodiments, the pharmaceutical composition is in the form of a tablet. The tablet can comprise a unit dose of a compound described here together with an inert diluent or carrier such as a sugar or sugar alcohol, for example lactose, sucrose, sorbitol or mannitol. The tablet can further comprise a non-sugar derived diluent such as sodium carbonate, calcium phosphate, calcium carbonate, or a cellulose or derivative thereof such as methyl cellulose, ethyl cellulose, hydroxypropyl methyl cellulose, and starches such as corn starch. The tablet can further comprise binding and granulating agents such as polyvinylpyrrolidone, disintegrants (*e.g*. swellable crosslinked polymers such as crosslinked carboxymethylcellulose), lubricating agents (*e.g.* stearates), preservatives (*e.g.* parabens), antioxidants (*e.g.* butylated hydroxytoluene), buffering agents (*e.g.* phosphate or citrate buffers), and effervescent agents such as citrate/bicarbonate mixtures. The tablet may be a coated tablet. The coating can be a protective film coating (*e.g.* a wax or varnish) or a coating designed to control the release of the active compound, for example a delayed release (release of the active after a predetermined lag time following ingestion) or release at a particular location in the gastrointestinal tract. The latter can be achieved, for example, using enteric film coatings such as those sold under the brand name Eudragit^{®}.

Tablet formulations may be made by conventional compression, wet granulation or dry granulation methods and utilize pharmaceutically acceptable diluents, binding agents, lubricants, disintegrants, surface modifying agents (including surfactants), suspending or stabilizing agents, including, but not limited to, magnesium stearate, stearic acid, talc, sodium lauryl sulfate, microcrystalline cellulose, carboxymethylcellulose calcium, polyvinylpyrrolidone, gelatin, alginic acid, acacia gum, xanthan gum, sodium citrate, complex silicates, calcium carbonate, glycine, dextrin, sucrose, sorbitol, dicalcium phosphate, calcium sulfate, lactose, kaolin, mannitol, sodium chloride, talc, dry starches and powdered sugar. Preferred surface modifying agents include nonionic and anionic surface modifying agents. Representative examples of surface modifying agents include, but are not limited to, poloxamer 188, benzalkonium chloride, calcium stearate, cetostearyl alcohol, cetomacrogol emulsifying wax, sorbitan esters, colloidal silicon dioxide, phosphates, sodium dodecyl sulfate, magnesium aluminum silicate, and triethanolamine.

In embodiments, the pharmaceutical composition is in the form of a hard or soft gelatin capsule. In accordance with this formulation, the compound of the present invention may be in a solid, semi-solid, or liquid form.

In embodiments, the pharmaceutical composition is in the form of a sterile aqueous solution or dispersion suitable for parenteral administration. The term parenteral as used herein includes subcutaneous, intracutaneous, intravenous, intramuscular, intra-articular, intraarterial, intrasynovial, intrasternal, intrathecal, intralesional and intracranial injection or infusion techniques.

In embodiments, the pharmaceutical composition is in the form of a sterile aqueous solution or dispersion suitable for administration by either direct injection or by addition to sterile infusion fluids for intravenous infusion, and comprises a solvent or dispersion medium containing, water, ethanol, a polyol (*e.g.,* glycerol, propylene glycol and liquid polyethylene glycol), suitable mixtures thereof, or one or more vegetable oils. Solutions or suspensions can be prepared in water with the aid of co-solvent or a surfactant. Examples of suitable surfactants include polyethylene glycol (PEG)-fatty acids and PEG-fatty acid mono and diesters, PEG glycerol esters, alcohol-oil transesterification products, polyglyceryl fatty acids, propylene glycol fatty acid esters, sterol and sterol derivatives, polyethylene glycol sorbitan fatty acid esters, polyethylene glycol alkyl ethers, sugar and its derivatives, polyethylene glycol alkyl phenols, polyoxyethylene-polyoxypropylene (POE-POP) block copolymers, sorbitan fatty acid esters, ionic surfactants, fat-soluble vitamins and their salts, water-soluble vitamins and their amphiphilic derivatives, amino acids and their salts, and organic acids and their esters and anhydrides. Dispersions can also be prepared, for example, in glycerol, liquid polyethylene glycols and mixtures of the same in oils.

The present disclosure also provides packaging and kits comprising pharmaceutical compositions for use in the methods described here. The kit can comprise one or more containers selected from the group consisting of a bottle, a vial, an ampoule, a blister pack, and a syringe. The kit can further include one or more of instructions for use, one or more syringes, one or more applicators, or a sterile solution suitable for reconstituting a compound or composition described here.

All percentages and ratios used herein, unless otherwise indicated, are by weight.

The invention is further described and exemplified by the following nonlimiting examples.

### EXAMPLES

In embodiments, a compound of Formula XI, or a subembodiment described herein, is an inhibitor of ALPK1 as measured, for example, in an *in vitro* kinase assay, or an assay designed to measure the activation of downstream targets of ALPK1 pathway activation, for example NFkB transcriptional activation and the secretion of proinflammatory cytokines and chemokines, such as IL-8, which is also referred to as CXCL-8. In general, the computer program XL fit was used for data analysis, including non-linear regression analysis. The half maximal inhibitory concentration (IC50) was used as the measure of a compound's effectiveness in the assays. IC50 values were determined using the following logistic equation Y=min+ (max-min)/ (1+(X/IC50^-hillslpoe), where Y is the value at the compound concentration, X. The concentration response curve fitting was conducted using GraphPad Prism version 6.00 software.

### ALPK1 in vitro Kinase Assay

ALPK1 kinase activity was measured in an *in vitro* assay using ADP-Heptose as the ALPK1 ligand and activator of its kinase activity and TIFA protein as the ALPK1 phosphorylation substrate. Since phosphorylated TIFA proteins oligomerize, Homogeneous Time-Resolved Fluorescence (HTRF) was used to measure protein:protein interaction between HA-tagged TIFA proteins as an indicator of TIFA phosphorylation.

In brief, dose-response studies were performed with HEK293 cells cultured in Dulbecco's Modified Eagle Medium (DMEM) supplemented 10% fetal bovine serum (FBS, Hyclone^{™}) containing antibiotics (pen/strep, G418) in 384-well assay plates. Each well contained 0.1 mg TIFA, ALPK1 (2 nM final concentration in reaction mixture ) and kinase buffer (100 mM of HEPES pH 7.4, 4mM DTT, 40mM MgCl₂, 20 mM of β-Glycerol phosphate disodium salt, 0.4 mM of Na₃VO₄, 0.16 mg/mL). Titrations of the test compounds were prepared in dimethylsulphoxide (DMSO). The reaction was initiated by addition of ATP and ADP-Heptose.

For HTRF, samples were incubated with a Tb cryptate-labeled anti-HA antibody for capturing HA-tagged proteins according to the manufacturer's instructions (PerkinElmer^{™}, CisBio^{™}) and the fluorescence signal was quantified (Tecan Infinite F NANO+). HTRF signals were calculated as the HTRF ratio (ratio of fluorescence measured at 665 nm and 620 nm) × 104 (thereby using the signal at 620 nm as an internal standard).

All compounds exhibited a dose-dependent decrease in TIFA phosphorylation in this assay. IC50 values were determined using 3- or 4- parameter logistic equation using GraphPad Prism version 6.00. The reference compound, A027, was used as a positive control for each plate. This compound has an IC50 of ~50 nanomolar (nM) in this assay. IC50 values for the test compounds ranged from 1 to 1000 nM and are shown in Tables 4-7.

### NFκB Gene Reporter Alkaline Phosphatase Assay

An alkaline phosphatase reporter assay system was used to measure inhibition of ALPK1-dependent NFκB reporter gene activation. Briefly, HEK293 cells stably expressing an NF-kB reporter (referred to herein as "G9 cells") were maintained in DMEM as described above. For the assay, cells were seeded into 96-well plates at a density of 10,000 cells/well in Freestyle^{™} 293 Expression Medium (ThermoFisher), and allowed to attach overnight. Cells were pretreated with serially diluted compounds for 30 min and then stimulated with D-glycero-D-manno-6-fluoro-heptose-1β-S-ADP. This compound is an analog of ADP-heptose that shows increased stability *in vitro* along with a similar ability to activate ALPK1 kinase activity. NFkB gene activation was detected using the chromogenic substrate, para-nitrophenyl phosphate (pNPP) according to the manufacturer's protocols (pNPP Phosphatase Assay, Beyotime Biotechnology). All compounds exhibited a dose-dependent decrease in NFkB promoter-driven gene expression in this assay. IC50 values ranged from 1-10 micromolar (uM) and are shown in Tables 4-7.

### Inhibition of activated ALPK1

Activating mutations in ALPK1 are associated with diseases and disorders such as cancer, spiroandenoma, spiroandenocarcinoma, ROSAH syndrome, and PFAPA syndrome. We conducted further experiments to evaluate the ability of representative compounds to inhibit ALPK1 in the context of two activating mutations, T237M and V1092A. In preliminary experiments we determined that IL-8 protein secretion was elevated in cells transiently transfected with human ALPK1 expression vectors containing each of these activating mutations. Accordingly, we used IL-8 secretion as an indicator of activated ALPK1 inhibition in cells expressing these mutations.

First, in preliminary experiments, we established that IL-8 secretion was significantly increased in cells transiently expressing either of the two activating mutations, T237M or V1092A. HEK293 cells were cultured as described above prior to transient transfection with either empty vector or an expression vector encoding (i) human ALPK1 (hALPK1), (ii) hALPK1 with the T237M activating mutation (hALPK1-T237M) (iii) hALPK1 with the V1092A activating mutation (hALPK1- V1092A), or (iv) a kinase dead ALPK1 mutant (hALPK1-T237M-D1194S). Transfection was performed according to manufacturer's protocols (Lipofectamine^{™} 3000, ThermoFisher). Transfected cells were selected, seeded onto 96-well plates and treated with serial dilutions of the test compounds for 6.5 hr. Following treatment, cell viability was determined using a luminescent cell viability assay (Cell Counting-Lite Assay or "CCL Assay" from Vazyme Biotech Co., Ltd.) and cell free supernatants were collected and analyzed for IL-8 protein by IL-8 ELISA as described above. Figure 1 shows IL-8 secretion for each of the test groups. As shown in the figure, very little IL-8 was detectable in cells transfected with any of the empty vector, hALPK1, or the kinase dead hALPK1 mutant. In contrast, both of the activating mutations in hALPK1 induced significant IL-8 secretion.

Next, we tested a representative set of compounds for inhibition of IL-8 secretion in cells expressing each of the activating ALPK1 mutants, T237M and V1092A. Table 8 shows inhibition of IL-8 secretion in cells transfected with the T237M and Table 9 shows inhibition of IL-8 secretion in cells transfected with the V1092A mutant. For the T237M mutant study, we produced an HEK293 cell line ("A2") stably expressing the T237M hALPK1 mutant. A2 cells were cultured in the presence of test compound for 40 hours total. Fresh medium and compound were added at 24 hours. Cell viability and IL-8 secretion were determined 16 hours after the second addition of compound, using the CCL assay and IL-8 ELISA as described above. Table 8 shows half maximal inhibitory concentration (IC50) of IL-8 secretion in A2 cells, relative to IL-8 secretion from wild-type HEK293 cells, such that knockdown to the level of IL-8 from wild-type cells was considered to be 100% inhibition.

**Table 8. Half maximal inhibitory concentration (IC50) of IL-8 secretion in cells expressing T237M mutant:**

| Compd. ID | IC50 (uM) |
|---|---|
| A175 | 1.880 |
| A176 | 0.110 |
| A251 | 4.454 |
| A252 | 0.419 |
| A245 | 0.304 |
| A249 | 3.191 |
| A292 | 0.261 |
| A261 | 0.133 |
| A276 | 0.499 |
| A281 | 0.237 |
| A309 | 0.130 |
| A310 | 0.850 |
| A311 | 0.155 |
| A312 | 1.406 |
| A313 | 1.721 |
| A314 | 0.765 |
| A315 | 0.942 |
| A316 | 5.372 |
| A317 | 0.593 |
| A318 | 0.699 |
| A319 | 1.178 |
| A289 | 0.129 |
| A244 | 1.548 |
| B067 | 0.195 |
| B068 | 0.941 |
| B099 | 0.144 |
| B100 | 0.129 |
| B074 | 0.120 |
| B076 | 0.066 |
| B102 | 3.125 |
| C048 | 6.018 |
| C034 | 0.073 |
| C035 | 0.049 |
| C039 | 0.056 |
| C037 | 0.885 |
| C038 | 0.070 |
| C046 | 2.812 |
| C041 | 0.545 |
| C040 | 0.131 |
| C042 | 0.153 |
| C008 | 0.030 |
| C002 | 0.925 |
| C016 | 0.666 |
| C018 | 0.476 |
| C011 | 0.353 |

For the V1092A mutant study shown in Table 9, HEK293 cells were transiently transfected with hALPK1-V1092A or hALPK1 (wildtype) expression vectors and then treated with test compounds for 24 hours. Fresh medium and compound were added at 18 hours. Cell viability and IL-8 secretion were determined 6 hours after the second addition of compound, using the CCL assay and IL-8 ELISA as described above. Table 9 shows half maximal inhibitory concentration (IC50) of IL-8 secretion relative to wild-type HEK293 cells.

**Table 9. Half maximal inhibitory concentration (IC50) of IL-8 secretion in cells expressing V1092A mutant**

| Compd. ID | IC50(uM) |
|---|---|
| A175 | 4.025 |
| A176 | 1.546 |
| A251 | 0.892 |
| A252 | 0.723 |
| A245 | 0.304 |
| A249 | 11.690 |
| A292 | 0.547 |
| A261 | 0.573 |
| A276 | 1.650 |
| A281 | 0.486 |
| A310 | 1.907 |
| A311 | 1.156 |
| A312 | 16.020 |
| A313 | 10.170 |
| A314 | 3.996 |
| A315 | 2.769 |
| A316 | 16.450 |
| A317 | 1.930 |
| A319 | 7.860 |
| A289 | 0.439 |
| A244 | 4.787 |
| B067 | 0.215 |
| B068 | 27.090 |
| B099 | 0.427 |
| B100 | 0.429 |
| B074 | 0.264 |
| B075 | 12.990 |
| B076 | 0.128 |
| C048 | 5.572 |
| C034 | 0.277 |
| C035 | 0.067 |
| C039 | 0.959 |
| C037 | 8.297 |
| C038 | 0.352 |
| C046 | 4.447 |
| C041 | 0.954 |
| C040 | 0.068 |
| C042 | 0.225 |
| C008 | 0.048 |
| C002 | 3.369 |
| C016 | 1.585 |
| C018 | 1.917 |
| C011 | 0.353 |

### Inhibition of activated ALPK1 in kidney

To test the effects of the ALPK1 inhibitors on the expression of innate immunity genes activated upon ALPK1 activation, SD rats were orally administered ALPK1 inhibitors followed by activation of innate immunity genes by intraperitoneal administration of an ALPK1 agonist, D-glycero-D-manno-6-fluoro-heptose-1β-S-ADP. Kidney tissue was harvested and assayed for gene expression. Briefly, Twenty male Sprague-Dawley (SD) rats were randomly divided into five groups. The normal group was given vehicle (0.5% MC) orally. After 2 hours, PBS was given by ip injection. The control group was given vehicle (0.5% MC) orally. After 2 hours, D-glycero-D-manno-6-fluoro-heptose-1β-S-ADP (50 µpk) was given by ip injection. The other 3 groups were given the ALPK1 inhibitor A0176 (4, 10 and 25mpk) orally. After 2 hours, D-glycero-D-manno-6-fluoro-heptose-1β-S-ADP (50µpk) was given by ip injection. The kidney from each group were collected after 3 hours of ip injection of D-glycero-D-manno-6-fluoro-heptose-1β-S-ADP (50µpk). Samples were isolated for the RT-PCR to identify MCP-1 (CCL-2), CCL-7, CXCL-1, CXCL-10, IL-1β, IL-6 mRNA expression levels. Total RNA was extracted from kidney following the protocol of the Rneasy Mini Kit(QIAGEN, Germany). Messenger RNA was reverse transcribed to cDNA using HiScript Q RT SuperMix for qPCR Kit (Vazyme, Nanjing, China). Quantitative PCR was conducted using AceQ qPCR SYBR Green Master Mix Kit (Vazyme, Nanjing, China) on the QuantStudio 5 applied biosystems (Thermo scientific, USA). The relative mRNA levels were calculated using the 2-ΔΔCT method, and HPRT was used as a reference for gene expression normalization. Data were presented as the gene fold change. As shown in FIG. 2, A0176 showed a dose-dependent inhibition of gene expression levels for a number of innate immunity genes including MCP-1 (CCL-2), CCL-7, CXCL-1, CXCL-10, IL-1β, and IL-6 .

### Efficacy study in sepsis induced acute kidney injury animal model

Polymicrobial sepsis induced by cecal ligation and puncture (CLP) is the most frequently used model because it closely resembles the progression and characteristics of human sepsis. We used a rat CLP model to evaluate the effects of compounds described here on sepsis. Briefly, the rat cecum was ligated with sterile silk thread, and then cecum was punctured twice with a needle, gently squeezed to express a small amount of fecal material, then the abdominal incision was closed. Compounds C008 and A0176 (20 mg/kg) were dosed 2 hours prior to surgery, and survival was recorded over the next 24 hours. In addition, at 24 hours post-surgery, the kidneys were collected for gene expression analysis by Q-PCR and plasma was collected for measurement of plasma MCP-1 concentrations by ELISA. The results are shown in Figures 3, 4, and 5. Briefly, the ALPK1 inhibitors improved the animals' survival (FIG. 3); decreased kidney proinflammatory genes expression (FIG. 4); and improved plasma MCP-1 levels (FIG. 5).

Those skilled in the art will recognize or be able to ascertain using no more than routine experimentation, many equivalents to the specific embodiments of the invention as described herein. Such equivalents are intended to be encompassed by the following claims.

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims.

## Claims

1. A compound of Formula XI-A-1 or XI-B 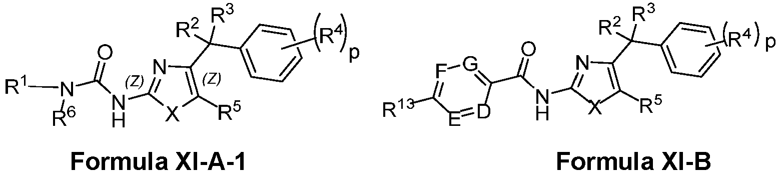
or a pharmaceutically acceptable salt thereof, wherein
X is selected from -S-, -O-, and -NR^{a}-, wherein
R^{a} is H, or C₁-C₆ alkyl;
R⁶ is selected from H, D, -OH, optionally substituted C₁-C₆ alkyl, wherein
the optionally substituted R⁶ moieties comprise 0-3 substituents independently selected from -D, halo, and -OH;
R¹ is selected from H, and optionally substituted C₁-C₆ alkyl;
wherein optionally substituted R¹ moieties comprise 0-4 substituents independently selected from -D, halo, -OH, -NH₂, =O, -CN, C₁-C₆ hydroxyalkyl, C₁-C₆ hydroxy-deuterated alkyl, C₁-C₆ alkoxyl, -O-X¹-R^{7a}, -C(O)N(R^{7a}R^{8a}), S(O)₂R^{7a}, -S(O)₂N(R^{7a}R^{8a}), -N(R^{7a}R^{8a}), saturated C₃-C₆ cycloalkyl, saturated or unsaturated 3-7 membered heterocyclyl containing 1-2 heteroatom ring vertices selected from N, O, and S, phenyl, 5-10 membered heteroaryl containing 1-4 heteroatom ring vertices selected from N, O, and S; wherein
each X¹ is independently C₁₋₆ alkylene;
each R^{7a} and R^{8a} are independently selected from H, C₁-C₆ alkyl, C₁-C₆ hydroxyalkyl, C₁-C₆ aminoalkyl, phenyl , saturated 3-7 membered heterocyclyl containing 1-2 heteroatom ring vertices selected from N, O, and S, wherein the phenyl and 3-7 membered heterocyclyl groups are substituted with 0-3 substituents selected from halo, -OH, and -NH₂, C₁-C₆ alkyl; and
the C₃-C₆ cycloalkyl, 3-7 membered heterocyclyl, the phenyl, the 5-10 membered heteroaryl are each independently substituted with 0 to 3 moieties selected from halo, -OH, -NH₂, C₁-C₆ alkyl, C₁-C₆ hydroxyalkyl, C₁-C₆ haloalkyl, C₁-C₆ alkoxyl, saturated 3-7 membered heterocyclyl containing 1-2 heteroatom ring vertices selected from N, O, and S, -C(O)N(R^{7b}R^{8b}), -S(O)₂ N(R^{7b}R^{8b}) and -N(R^{7b}R^{8b}), wherein
each R^{7b} and R^{8b} are independently selected from H, C₁-C₆ alkyl, and C₁-C₆ hydroxyalkyl; or
R¹ and R⁶ combine to form a 3-6 membered heterocycloalkyl substituted with 0-3 moieties independently selected from the group consisting of -OH, -NH₂, and C₁-C₆ hydroxyalkyl;
R⁵ is selected from H, deuterium, halo, C₁-C₆ alkyl, C₁-C₆ deuteroalkyl, and C₁-C₆ haloalkyl;
R² and R³ are each independently selected from H, OH, C₁-C₆ alkyl, C₂-C₆ alkynyl, C₃-C₆ cycloalkyl, and the phenyl, wherein C₁-C₆ alkyl,C₂-C₆ alkynyl, C₃-C₆ cycloalkyl, and the phenyl are each substituted with 0-3 moieties independently selected from -OH, and C₁-C₆ alkoxyl; provided that R² and R³ are not both H; or
R² and R³ combine to form a C₃-C₆ cycloalkyl ring or a 3-7 membered heterocyclyl containing 1-2 heteroatom ring vertices independently selected from N, O, and S;
each R⁴ is independently selected from halo, -OH, C₁-C₆ alkyl, C₁-C₆ alkoxyl, saturated C₃-C₆ cycloalkyl, OR^{7e}, wherein
R^{7e} is selected from H, C₁-C₆ alkyl, and C₁-C₆ haloalkyl, and
the subscript p is 1, 2 or 3;
D is CR¹⁰ or N;
E is CR¹⁴ or N;
F is CR¹² or N;
G is CR¹¹ or N;
provided that no more than three of D, E, F, and G are N;
R¹⁰, R¹¹, R¹², and R¹⁴, when present, are each independently selected from H, halo, - OH, C₁-C₆ alkoxyl, -O-X¹-R^{7a}, -C(O)N(R^{7a}R^{8a}), and saturated or unsaturated 3-7 membered heterocyclyl containing 1-2 heteroatom ring vertices selected from N, O, and S; wherein
each X¹ is independently C₁₋₆ alkylene;
each R^{7a} and R^{8a} are independently selected from H, C₁-C₆ alkyl, and C₁-C₆ alkoxyl;
R¹³ is H, saturated or unsaturated 3-7 membered heterocyclyl containing 1-2 heteroatom ring vertices selected from N, O, and S, saturated or unsaturated 7-8 membered bridged heterocyclyl containing 1-2 heteroatom ring vertices selected from N, O, and S, wherein
the 3-7 membered heterocyclyl, the 7-8 membered bridged heterocyclyl are optionally substituted with 0-2 moieties independently selected from - OH, C₁-C₆ alkyl, C₁-C₆ aminoalkyl, -NH₂, and C₁-C₆ hydroxyalkyl.

2. The compound of claim 1, wherein
X is -S-, -O-, or -NH-.

3. The compound of claim 1, having:
Formula XI-A-1-a 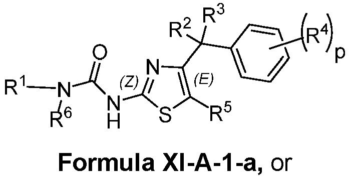
a pharmaceutically acceptable salt thereof,
or, R⁶ is selected from H, C₁-C₆ alkyl, C₁-C₆ hydroxyalkyl and C₁-C₆ hydroxy-deuterated alkyl.

4. The compound of any one of claims 1 to 3, wherein:
a) R¹ combines with R⁶ to form a 3-6 membered heterocycloalkyl substituted with 0-3 moieties independently selected from the group consisting of -OH, -NH₂, and C₁-C₆ hydroxyalkyl,
b) R¹ is C₁-C₆ alkyl substituted with 0-4 substituents independently selected from - OH, C₁-C₆ hydroxyalkyl, C₁-C₆ alkoxyl, -S(O)₂N(R^{7a}R^{8a}) and -N(R^{7a}R^{8a}), wherein each R^{7a} and R^{8a} are independently selected from H, C₁-C₆ alkyl, and C₁-C₆ aminoalkyl,
or
c) R¹ is C₁-C₆ alkyl substituted with 0-2 substituents independently selected from - OH, C₁-C₆ hydroxyalkyl, and -S(O)₂N(R^{7a}R^{8a}), wherein
each R^{7a} and R^{8a} are independently selected from H, and C₁-C₆ alkyl.

5. The compound of claim 1, wherein:
a) D, E, F and G are CR¹⁰, CR¹⁴, CR¹², and CR¹¹, respectively, or
b) F and G are CR¹⁴ and CR¹¹, respectively, E is N or CR¹⁴ and D is N or CR¹⁰.

6. The compound of claim 1 or 5, wherein:
a) R¹⁰ and R¹¹ are each H;
R¹² and R¹⁴ are each independently selected from halo, -OH, C₁-C₆ alkoxyl, and - C(O)N(R^{7b}R^{8b}), wherein
R^{7b} and R^{8b} are each independently selected from H, C₁-C₆ alkyl, and C₁-C₆ alkoxyl; and
R¹³ is selected from 3-7 membered heterocyclyl containing 1-2 heteroatom ring vertices selected from N, O, and S, and saturated or unsaturated 7-8 membered bridged heterocyclyl containing 1-2 heteroatom ring vertices selected from N, O, and S, wherein
the 3-7 membered heterocyclyl, and the 7-8 membered bridged heterocyclyl, are optionally substituted with 0-2 moieties independently selected from -OH, C₁-C₆ alkyl, C₁-C₆ hydroxyalkyl, and C₁-C₆ aminoalkyl,
b) R¹² and R¹⁴ are H;
R¹⁰ and R¹¹ are each independently selected from halo, -OH, C₁-C₆ alkoxyl, and - C(O)N(R^{7b}R^{8b}), wherein
R^{7b} and R^{8b} are each independently selected from H, C₁-C₆ alkyl, and C₁-C₆ alkoxyl; and
R¹³ is selected from 3-7 membered heterocyclyl containing 1-2 heteroatom ring vertices selected from N, O, and S, and saturated or unsaturated 7-8 membered bridged heterocyclyl containing 1-2 heteroatom ring vertices selected from N, O, and S, wherein
the 3-7 membered heterocyclyl, and the 7-8 membered bridged heterocyclyl, are optionally substituted with 0-2 moieties independently selected from -OH, C₁-C₆ alkyl, C₁-C₆ hydroxyalkyl, and C₁-C₆ aminoalkyl,
c) R¹⁰, R¹¹, R¹² and R¹⁴, when present, are each H; and
R¹³ is selected from saturated or unsaturated C₃-C₆ cycloalkyl, 3-7 membered heterocyclyl containing 1-2 heteroatom ring vertices selected from N, O, and S, and saturated or unsaturated 7-8 membered bridged heterocyclyl containing 1-2 heteroatom ring vertices selected from N, O, and S, wherein the 3-7 membered heterocyclyl, and the 7-8 membered bridged heterocyclyl,
are optionally substituted with 0-2 moieties independently selected from -OH, C₁-C₆ alkyl, C₁-C₆ hydroxyalkyl, and C₁-C₆ aminoalkyl,
d) R¹⁰, R¹¹, R¹² and R¹⁴, when present, are each H; and
R¹³ is a 3-7 membered heterocyclyl containing 1-2 heteroatom ring vertices selected from N, O, and S substituted with 0-2 moieties independently selected from -OH, C₁-C₆ alkyl, C₁-C₆ hydroxyalkyl, and C₁-C₆ aminoalkyl,
or
e) R¹⁰, R¹¹, R¹² and R¹⁴, when present, are each H; and
R¹³ is optionally substituted saturated or unsaturated 7-8 membered bridged heterocyclyl containing 1-2 heteroatom ring vertices selected from N, O, and S substituted with 0-2 substituents selected from -OH, and-C₁-C₆ alkyl.

7. The compound of claim 1, having Formula XI-B-1 or XI-B-2 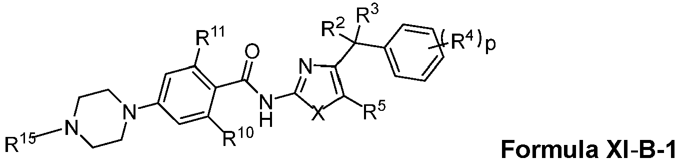 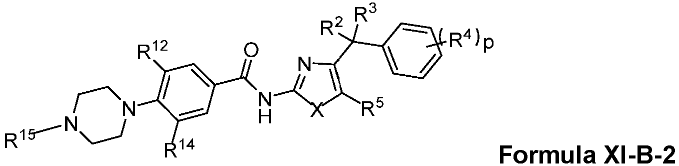 or a pharmaceutically acceptable salt thereof, wherein
R¹⁵ is selected from -OH, C₁-C₆ alkyl, C₁-C₆ hydroxyalkyl, and C₁-C₆ aminoalkyl,
optionally wherein R¹⁰, R¹¹, R¹² and R¹⁴are each independently selected from halo and C₁-C₆ alkyl.

8. The compound of claim 7, wherein:
a) R¹⁵ is selected from C₁-C₆ alkyl,
b) X is -S-, having formula XI-B-1-a or formula XI-B-2-a

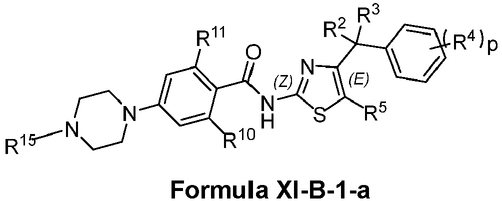

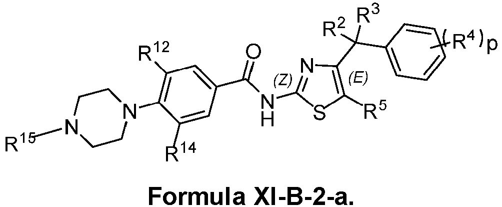

9. The compound of claim 7 or 8, having:
a) Formula XI-B-1-a-I or Formula XI-B-2-a-I 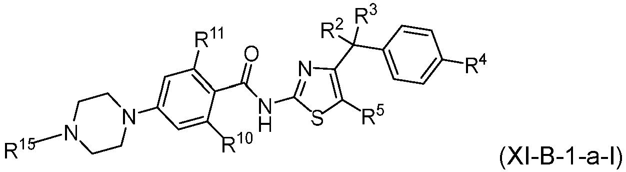 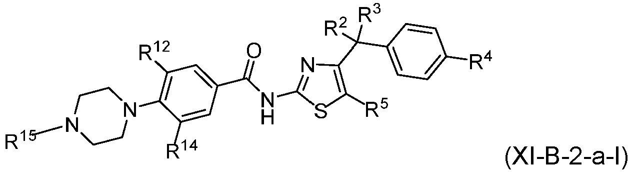 or a pharmaceutically acceptable salt thereof, wherein R⁴ is halo,
b) Formula XI-B-1-a-II or Formula XI-B-2-a-II 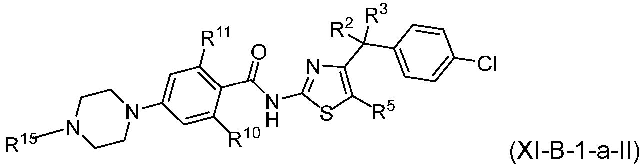 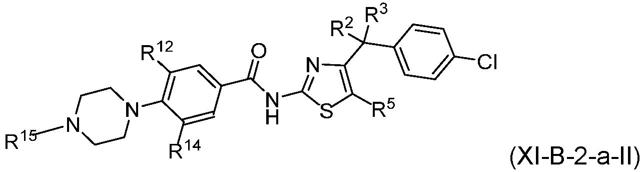 or a pharmaceutically acceptable salt thereof,
c) Formula XI-B-1-a-III or Formula XI-B-2-a-III 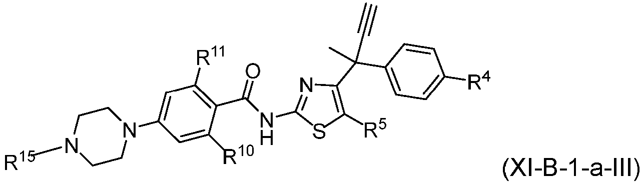 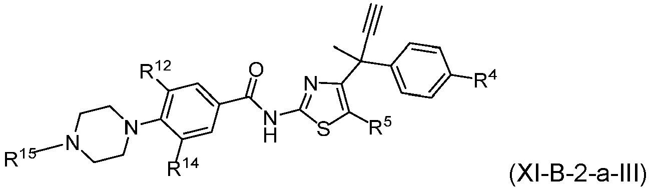 or a pharmaceutically acceptable salt thereof,
or
d) Formula XI-B-1-a-IV, or Formula XI-B-2-a-IV 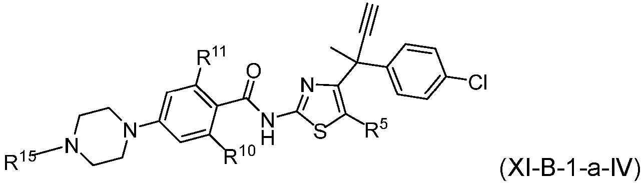 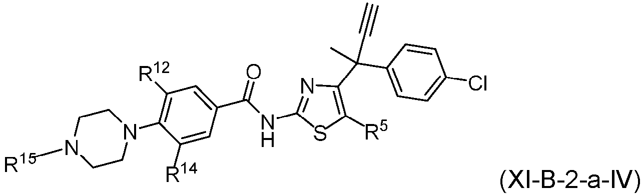 or a pharmaceutically acceptable salt thereof.

10. The compound of any one of claims 1 to 9, wherein the subscript p is 1, and R⁴ is:
a) attached to the phenyl ring as shown below: 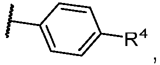 wherein the wavy line represents the point of attachment to the remainder of the formula,
b) halo attached to the phenyl ring as shown below: 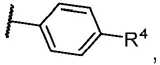 wherein the wavy line represents the point of attachment to the remainder of the formula,
c) chloro attached to the phenyl ring as shown below: 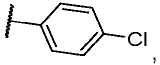 wherein the wavy line represents the point of attachment to the remainder of the formula,
or
d) methoxy attached to the phenyl ring as shown below: 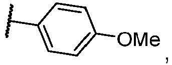 wherein the wavy line represents the point of attachment to the remainder of the formula.

11. The compound of any one of claims 1 to 10, wherein the carbon atom attached to R² and R³ is: a) the S isomer, or b) the R isomer.

12. The compound of claim 1, wherein the compound of Formula XI-A-1 or XI-B is selected from 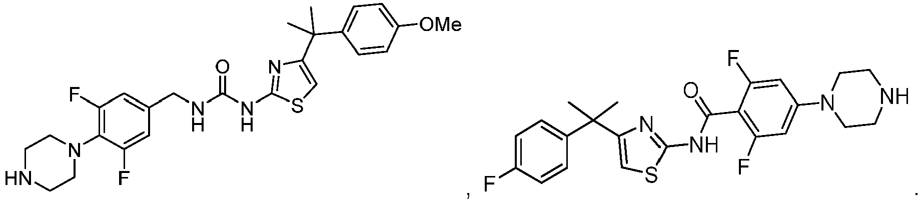 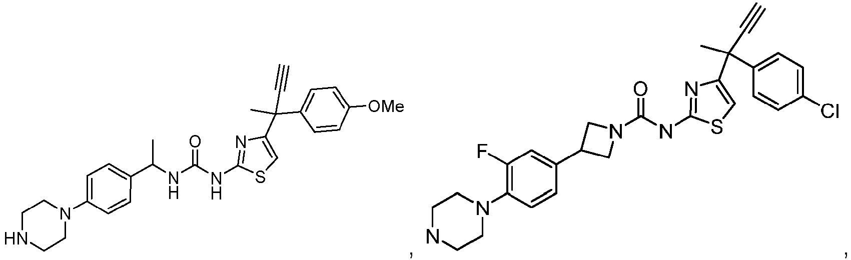 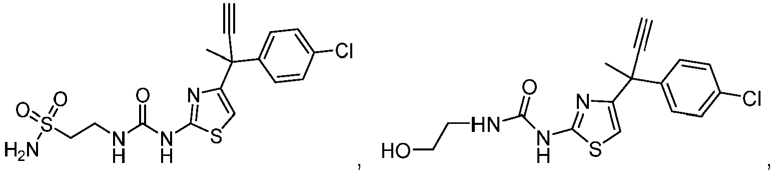 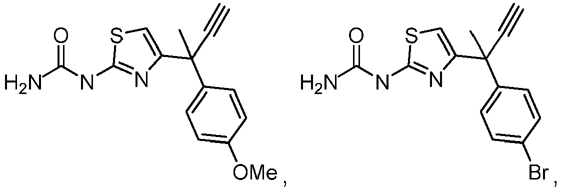 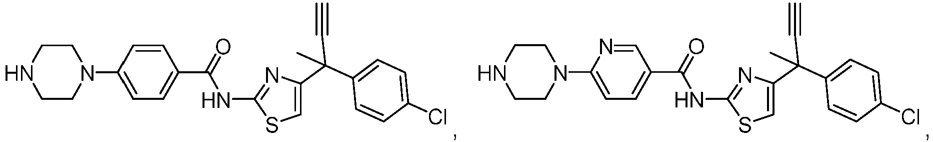 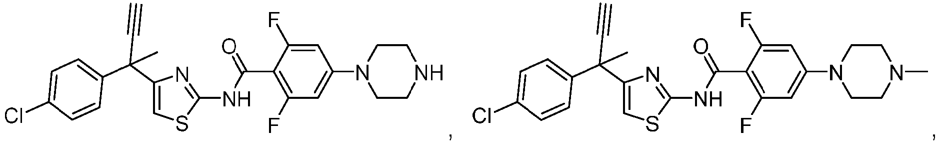 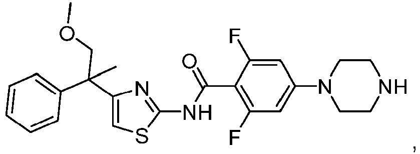 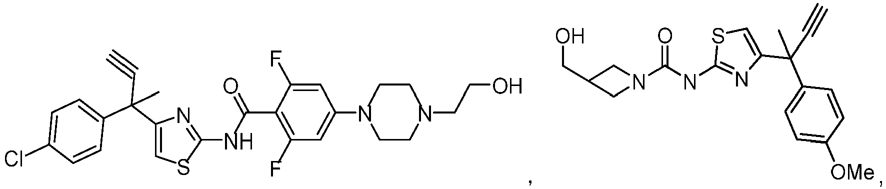 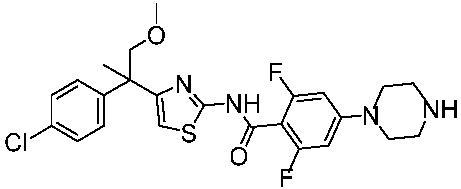 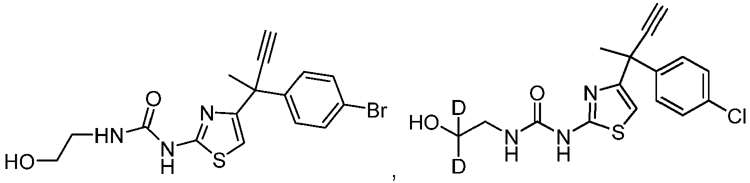 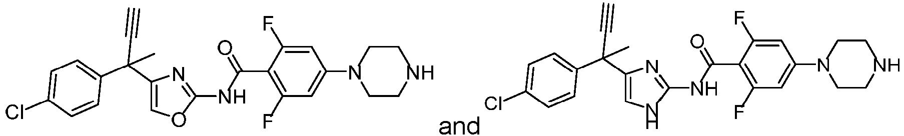 or
wherein the compound of Formula XI is selected from

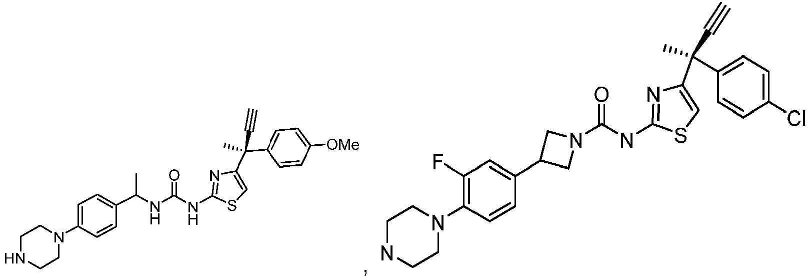

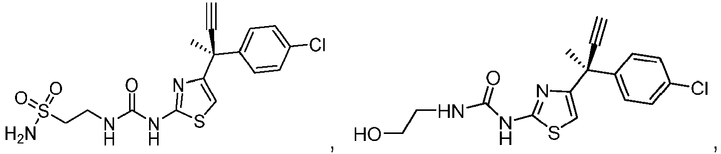

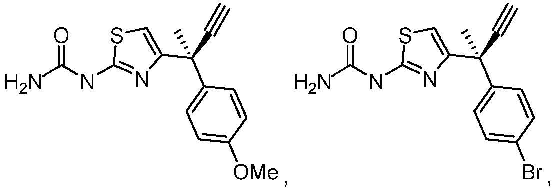

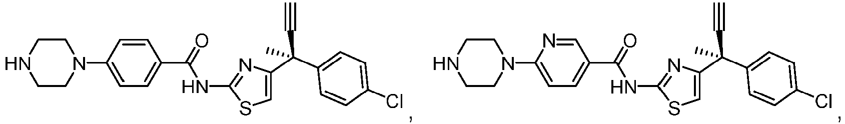

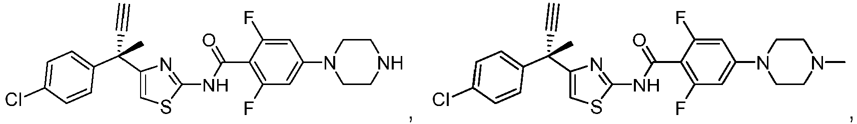

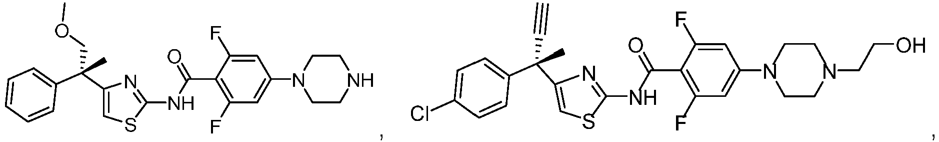

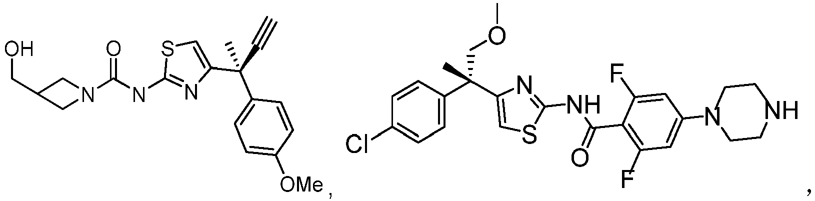

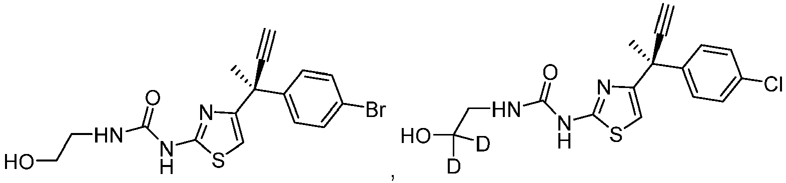

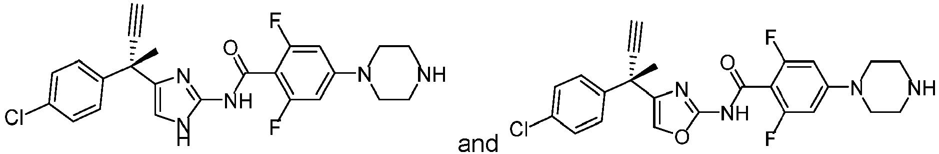

13. The compound of claim 1, selected from any one of the following compounds:

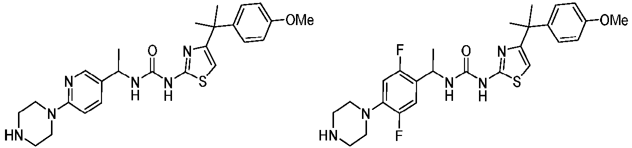

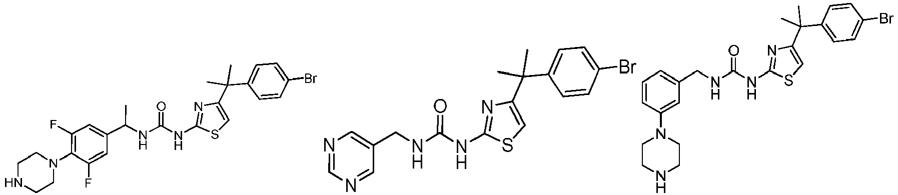

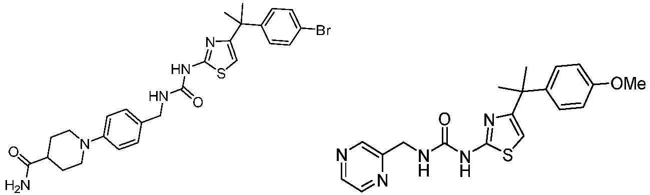

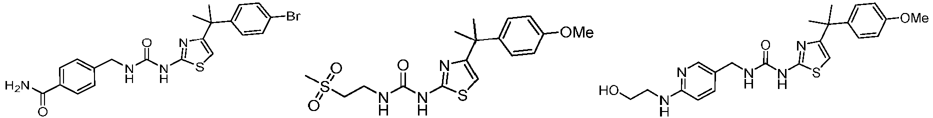

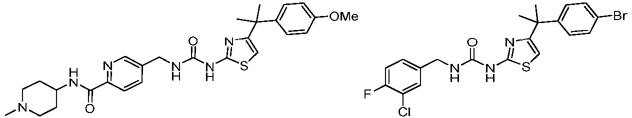

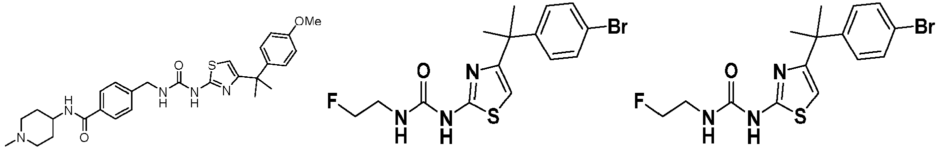

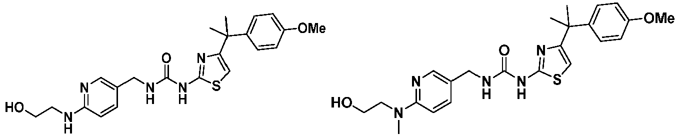

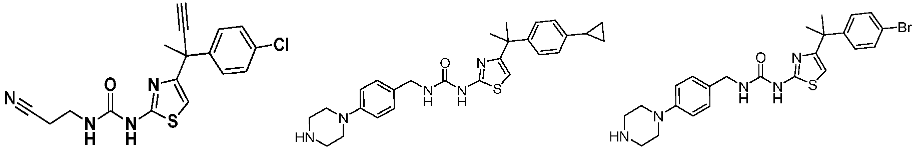

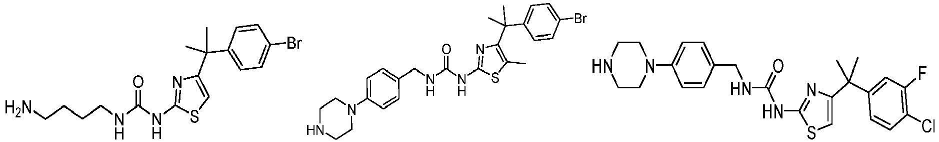

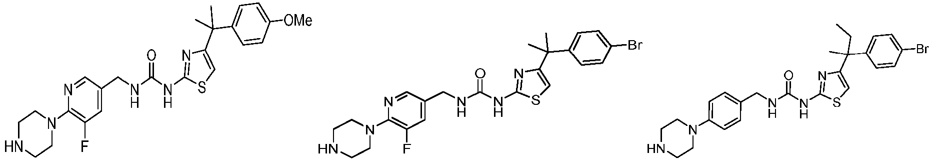

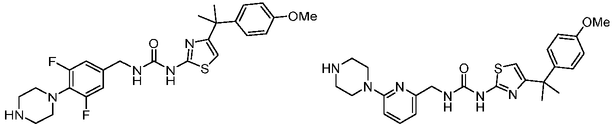

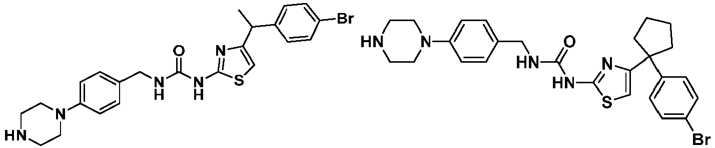

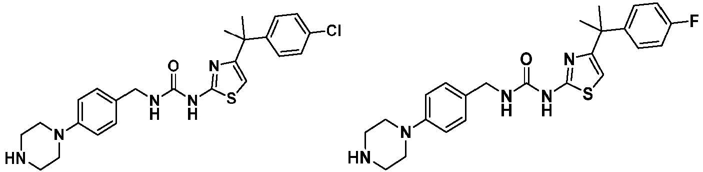

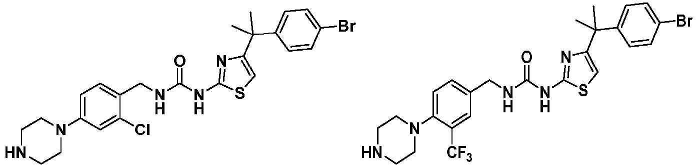

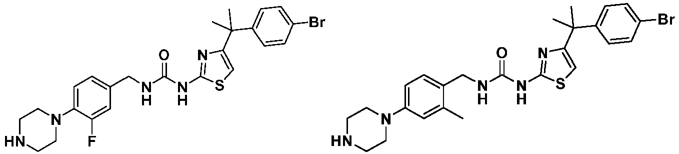

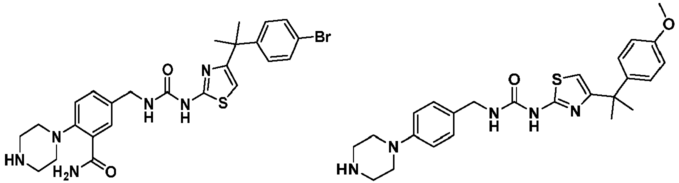

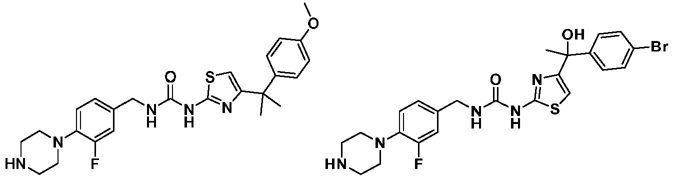

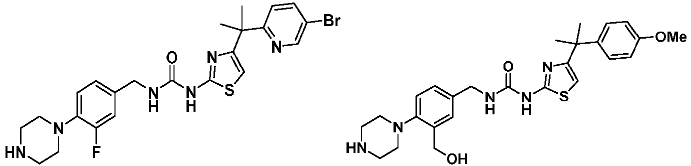

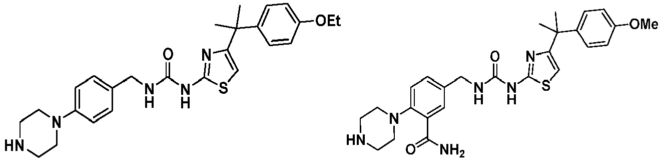

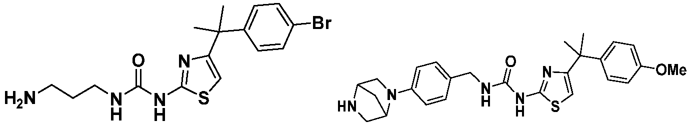

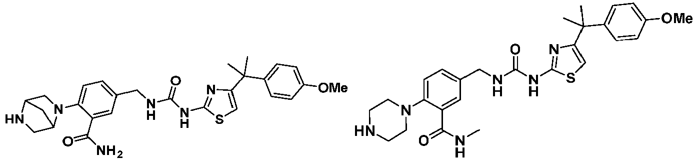

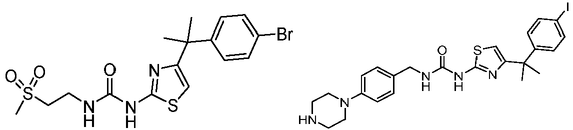

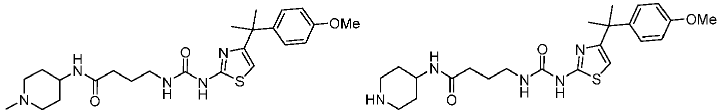

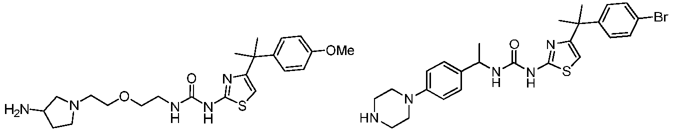

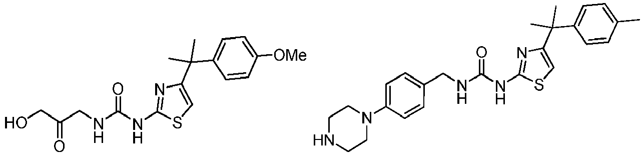

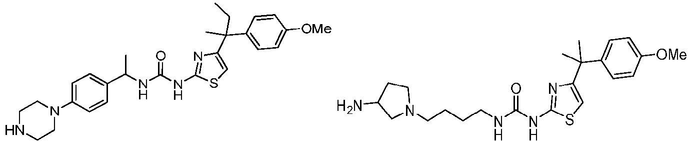

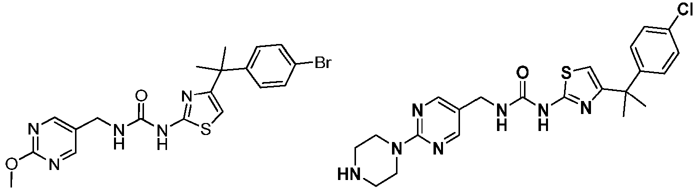

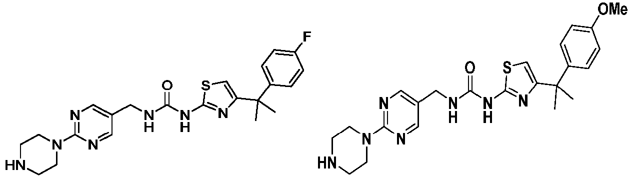

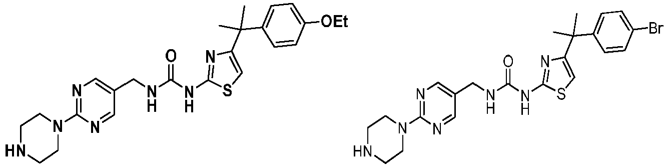

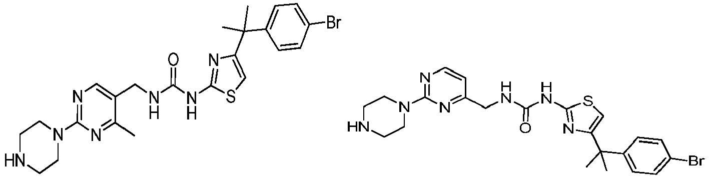

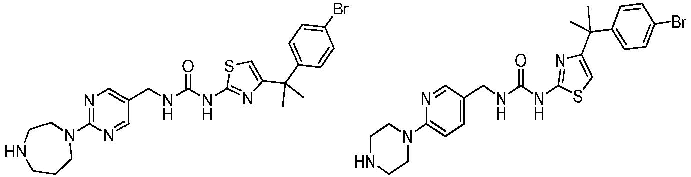

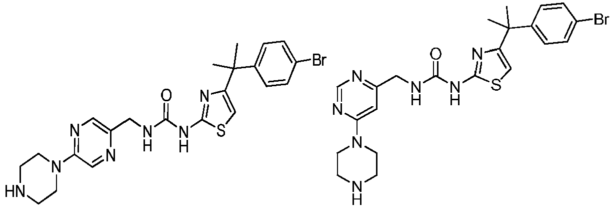

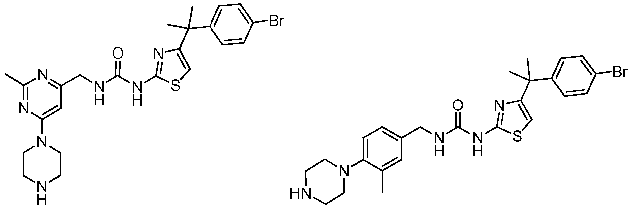

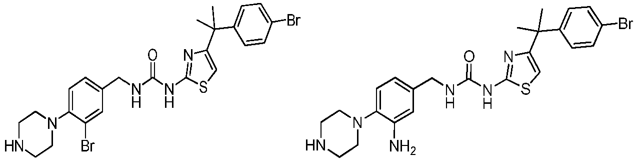

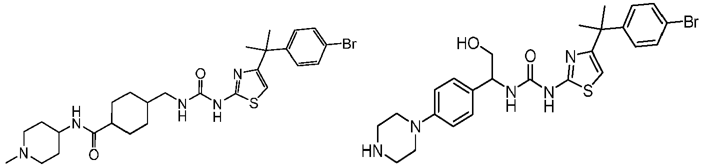

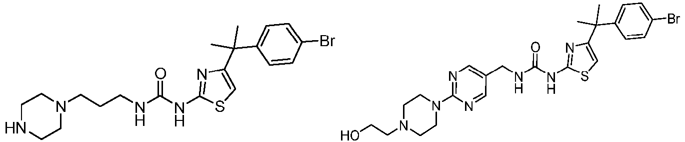

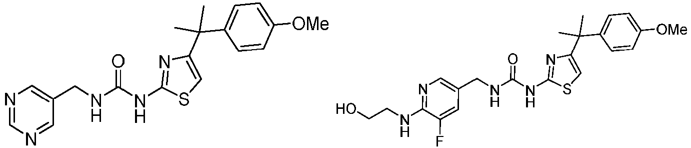

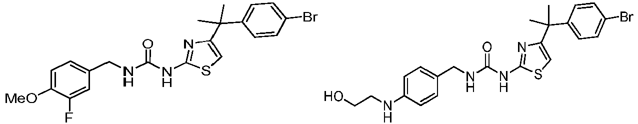

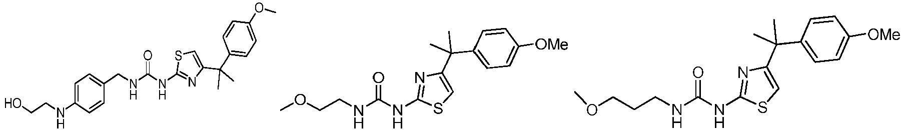

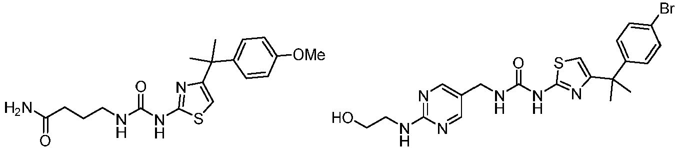

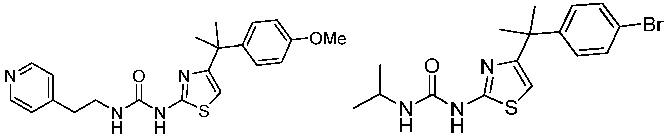

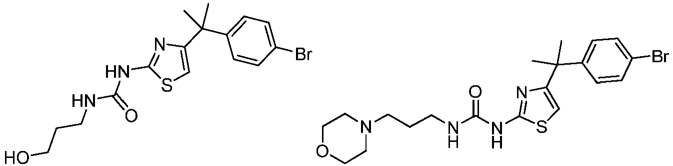

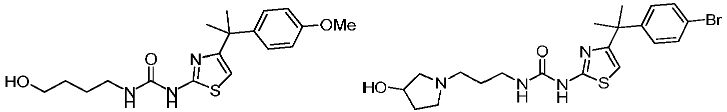

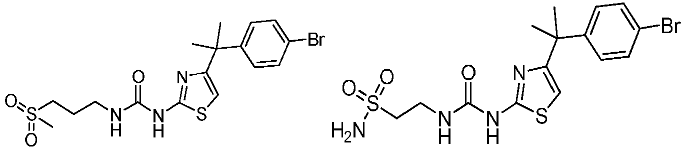

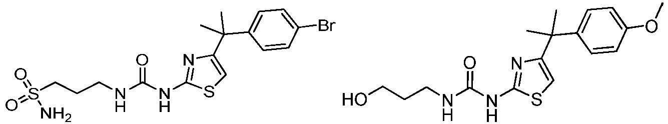

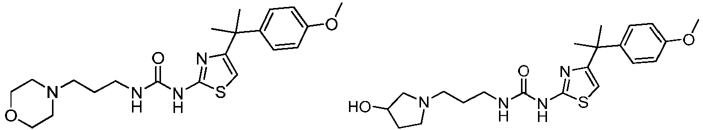

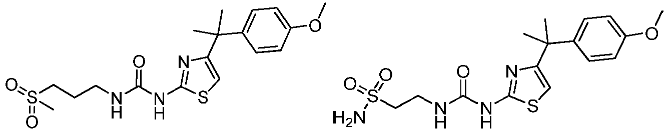

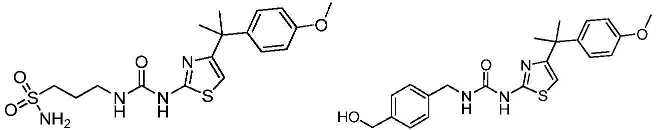

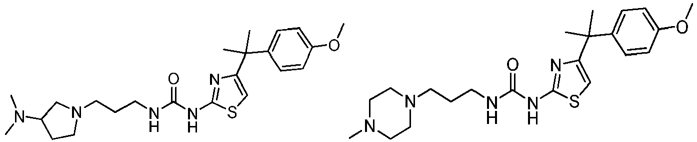

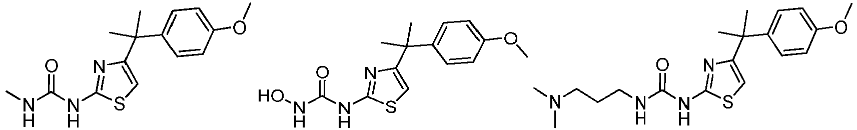

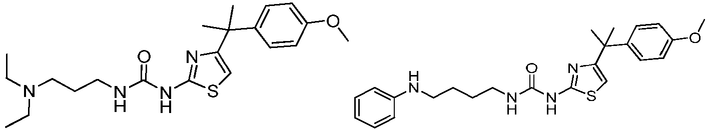

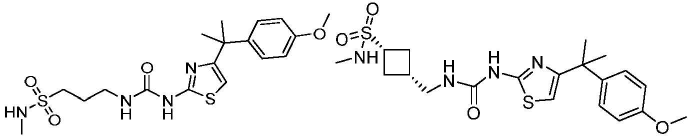

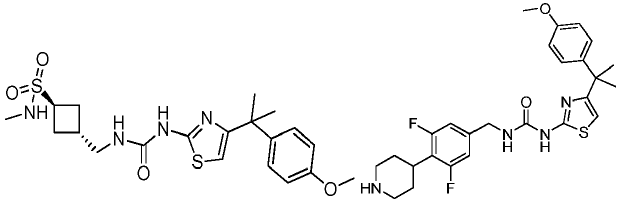

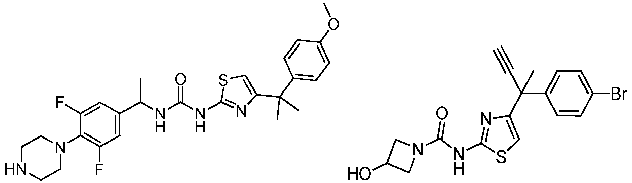

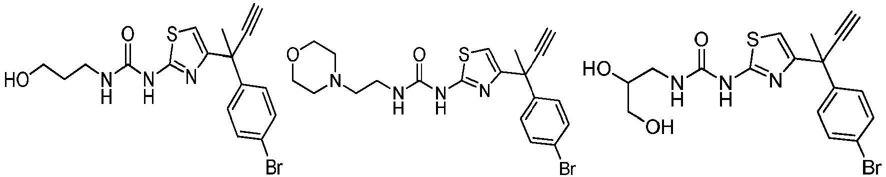

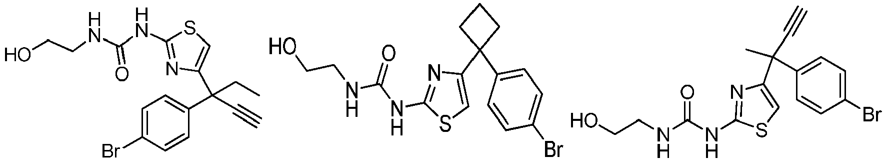

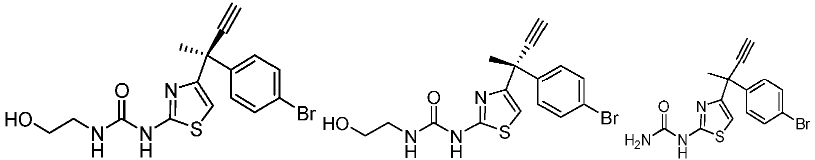

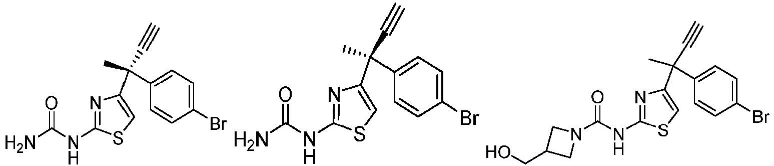

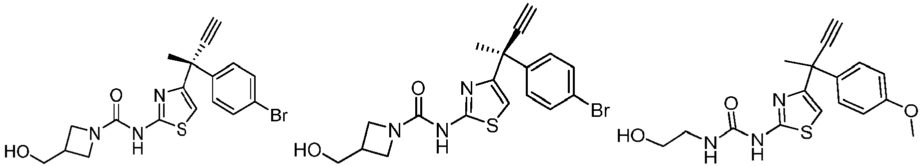

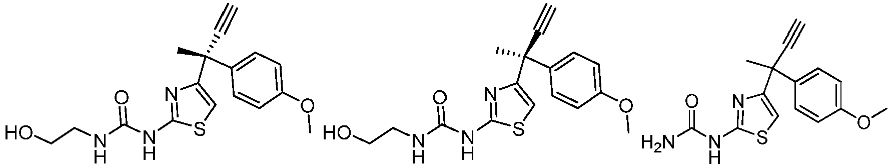

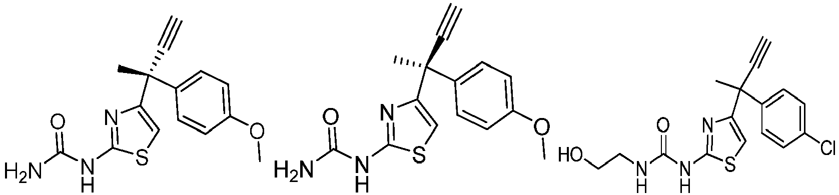

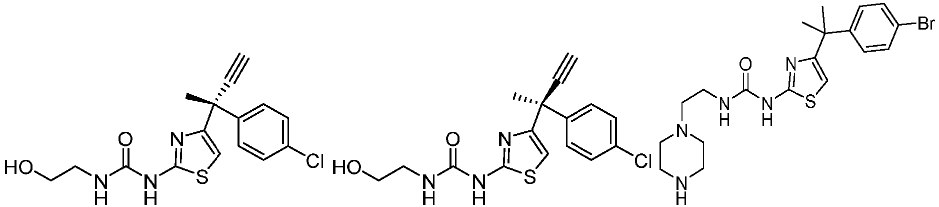

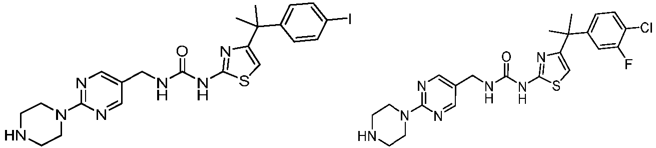

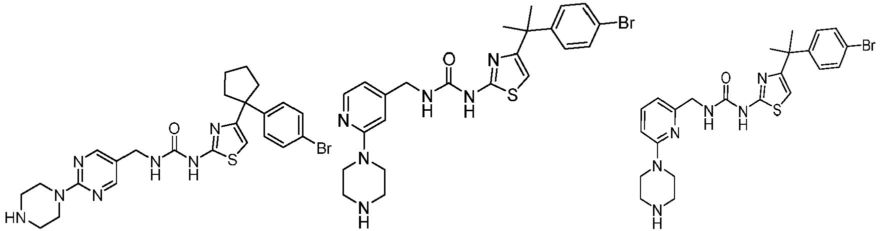

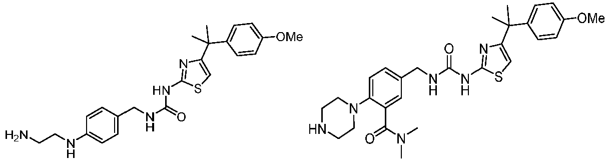

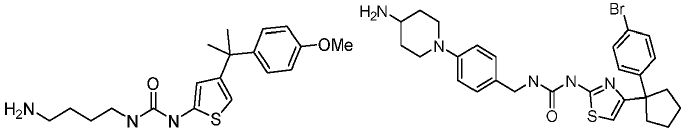

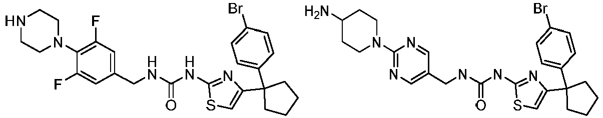

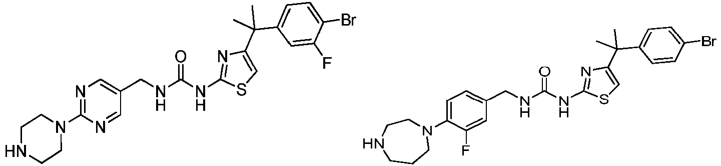

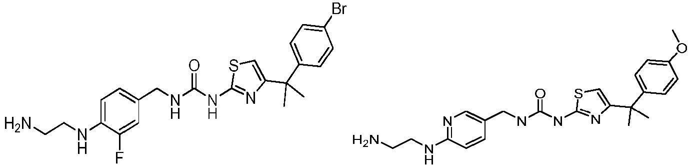

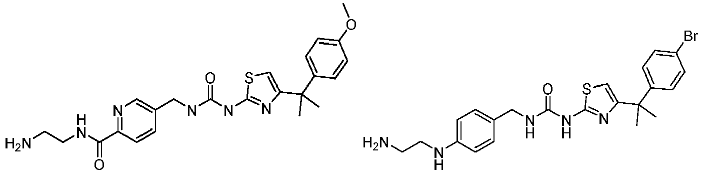

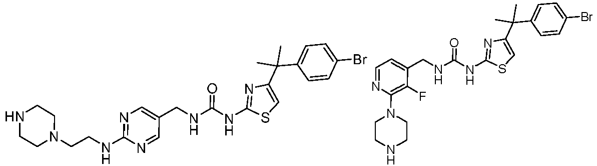

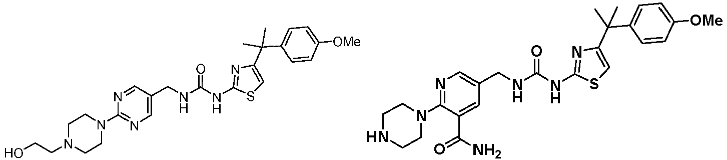

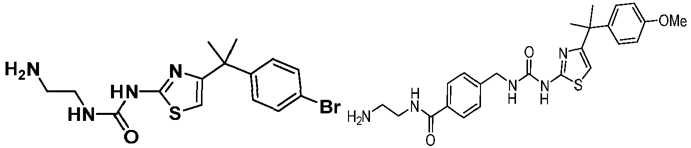

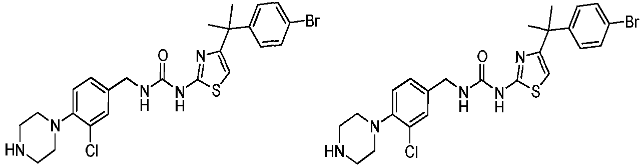

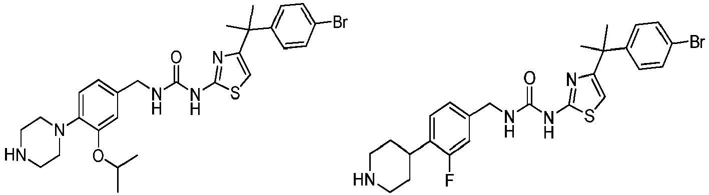

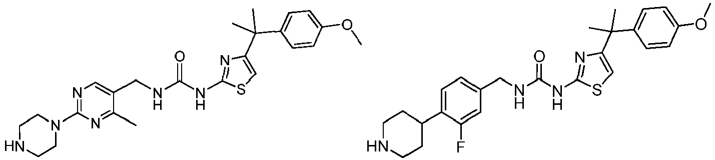

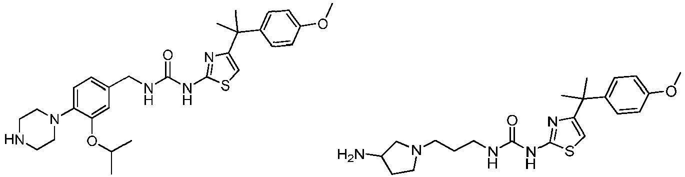

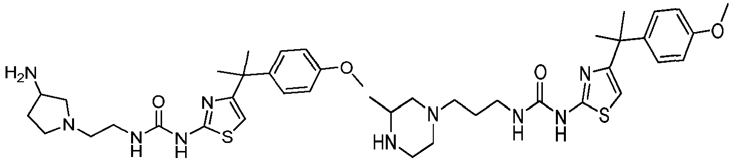

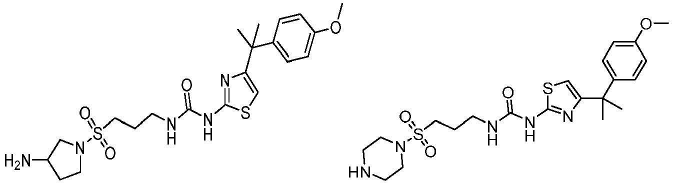

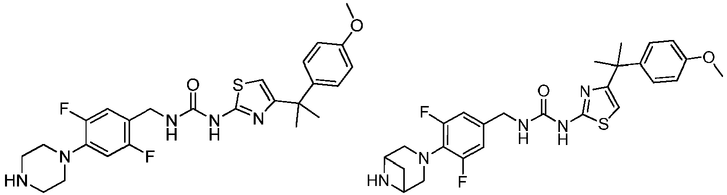

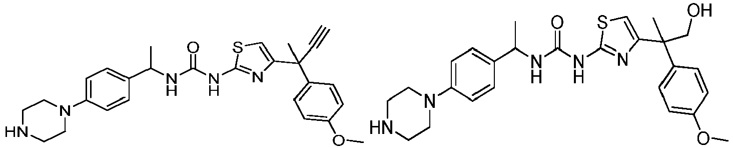

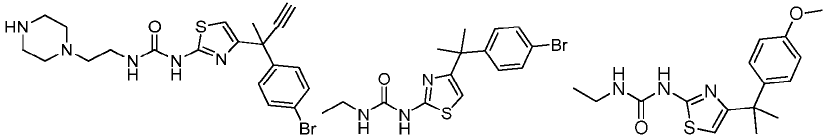

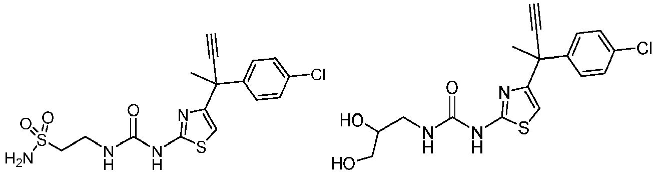

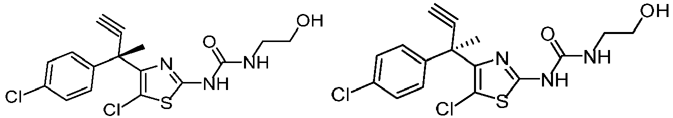

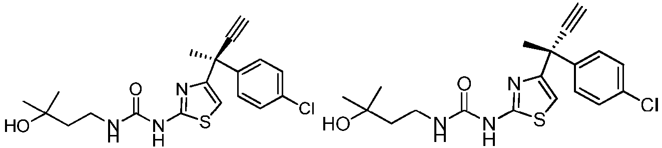

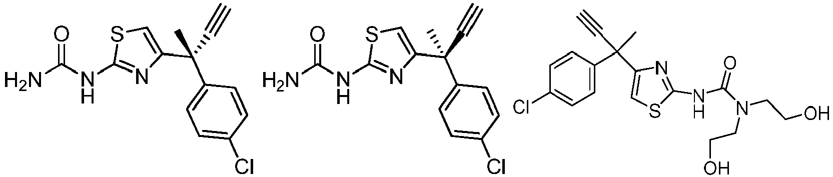

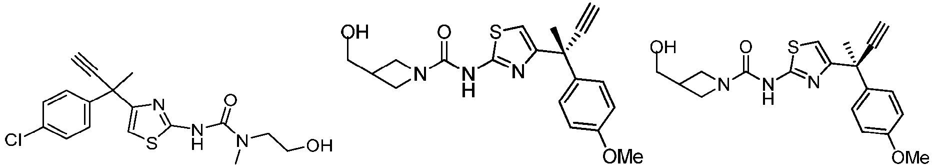

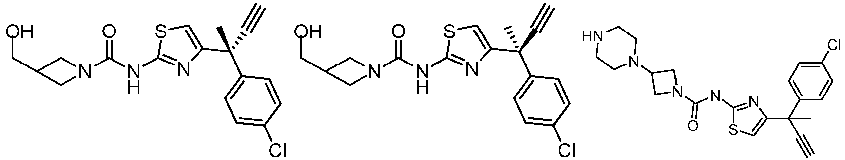

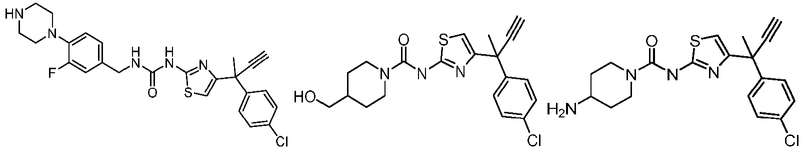

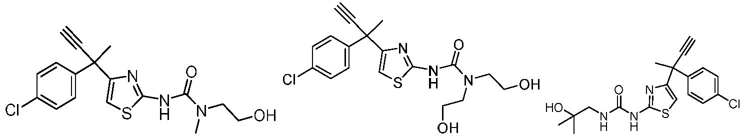

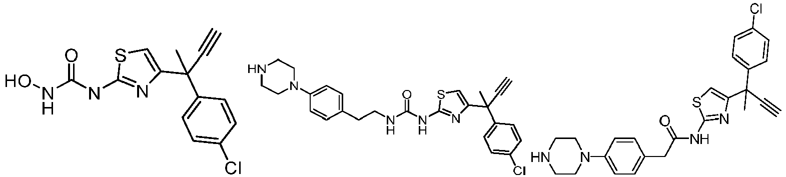

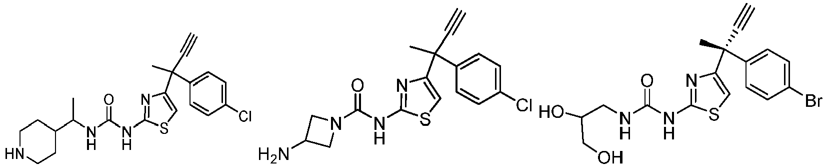

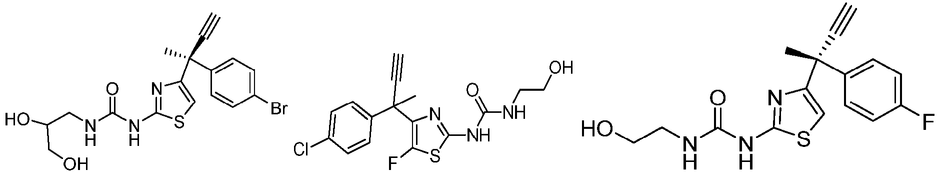

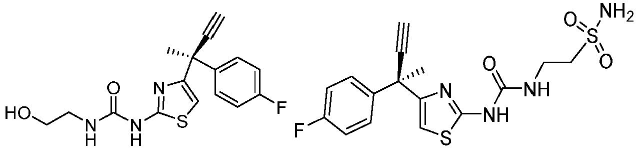

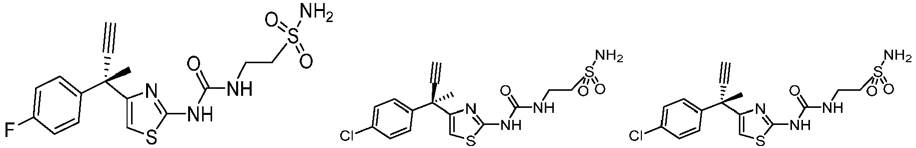

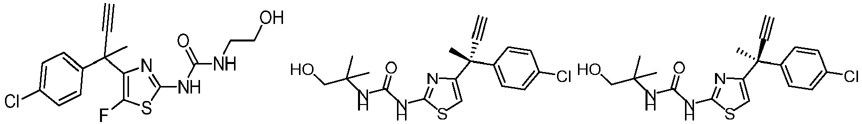

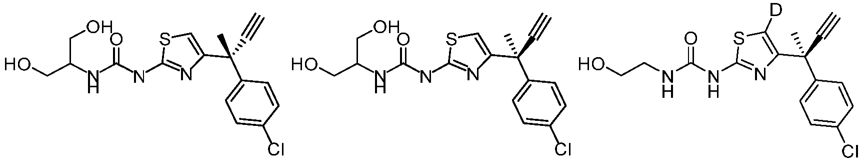

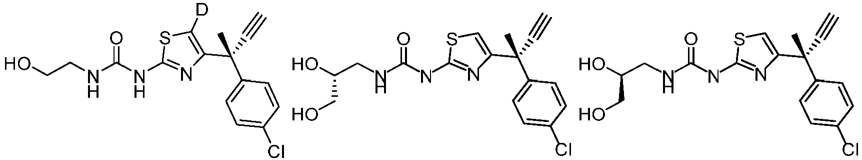

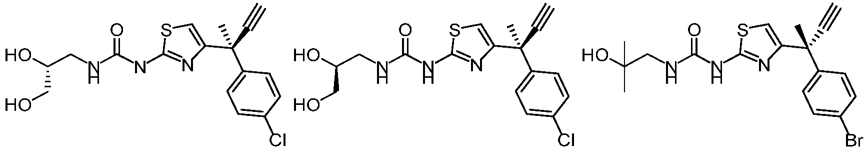

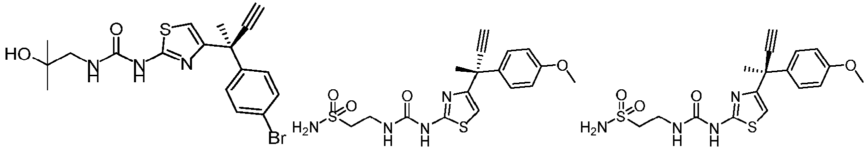

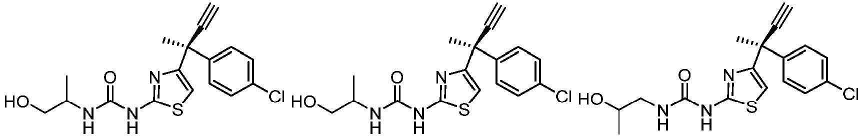

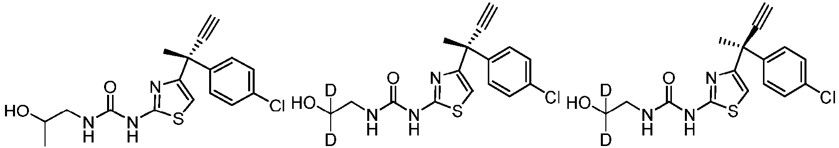

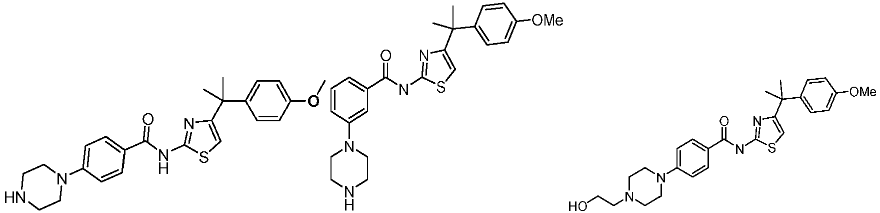

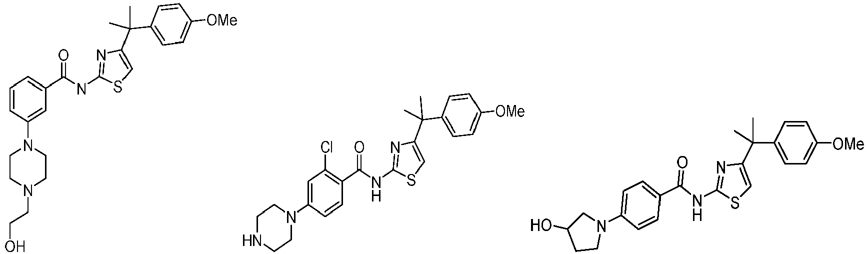

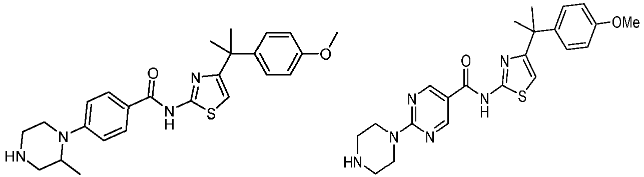

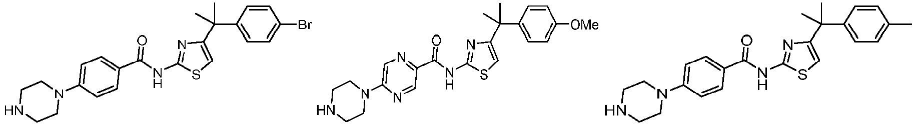

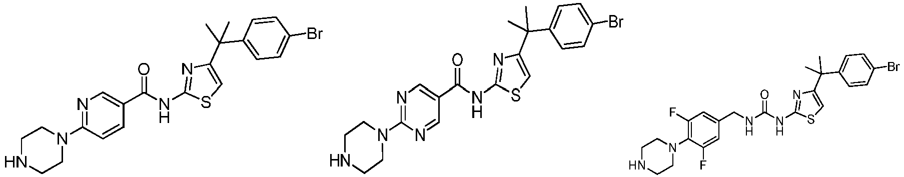

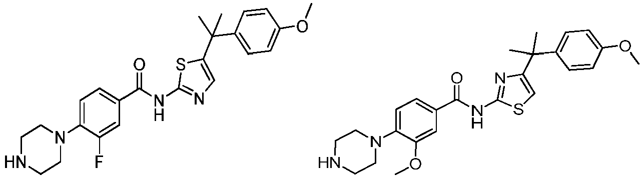

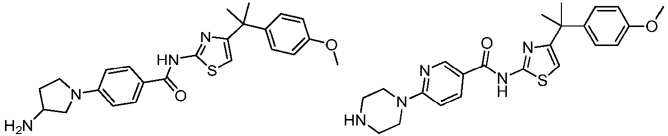

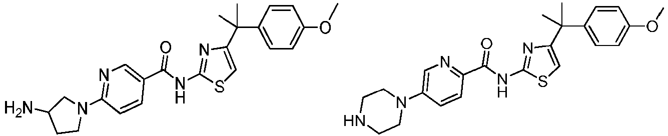

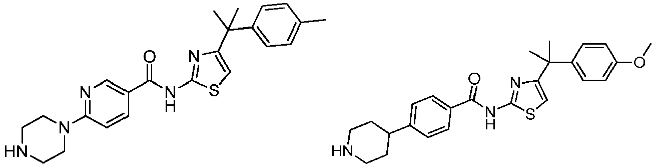

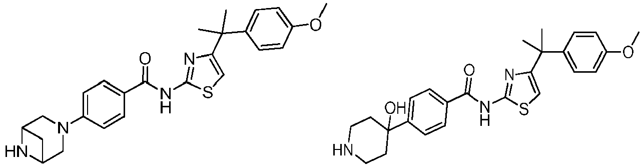

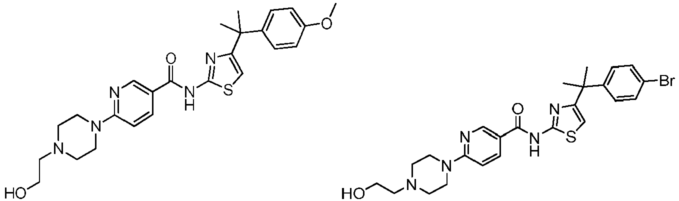

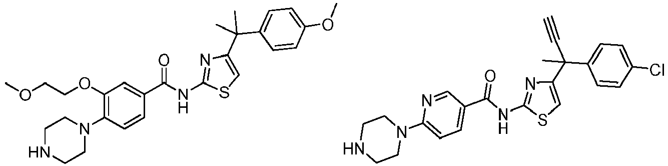

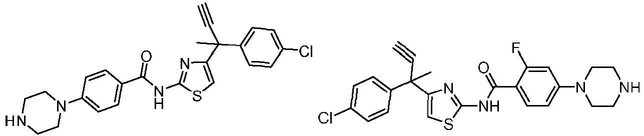

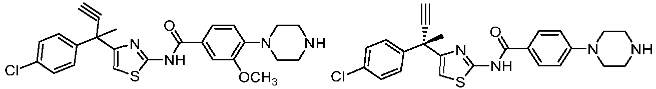

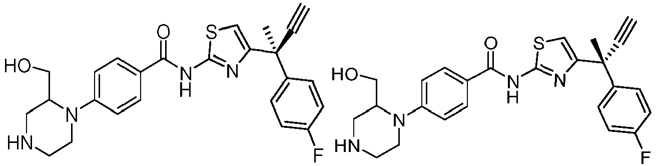

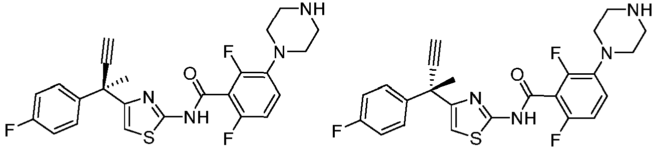

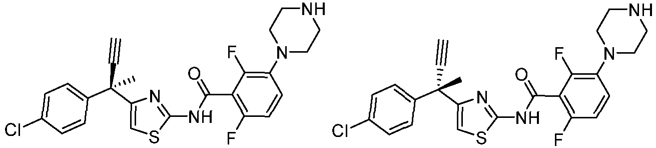

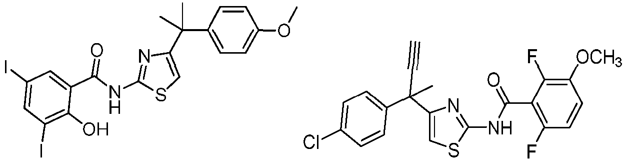

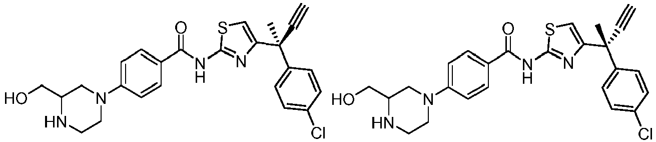

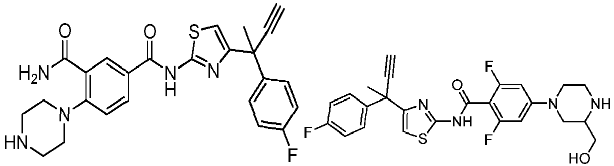

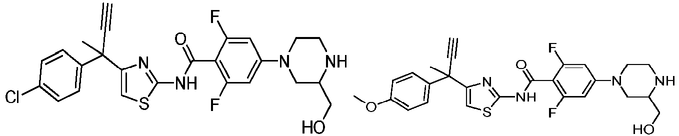

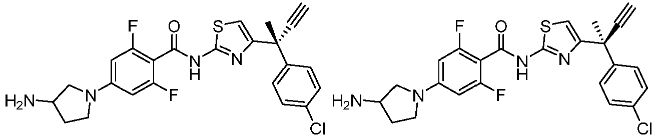

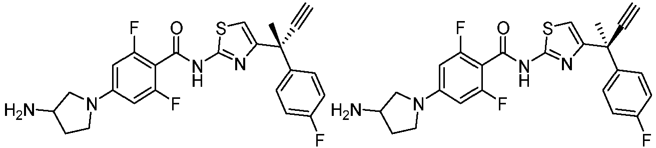

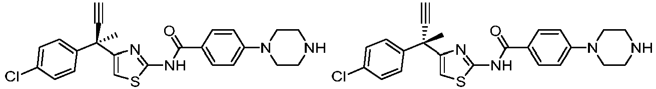

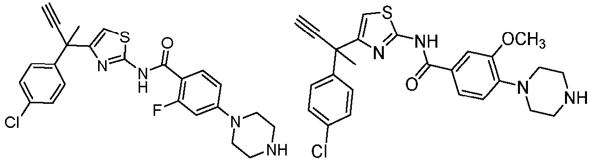

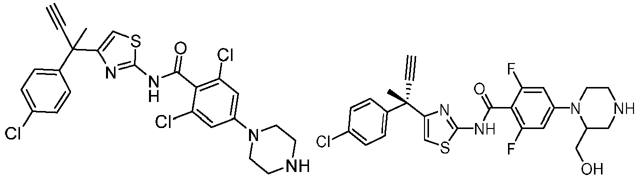

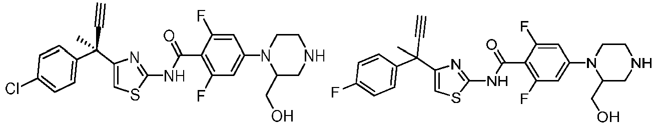

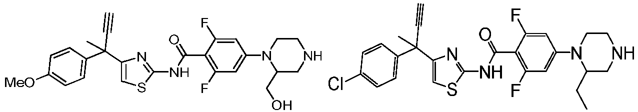

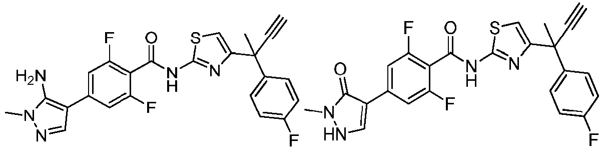

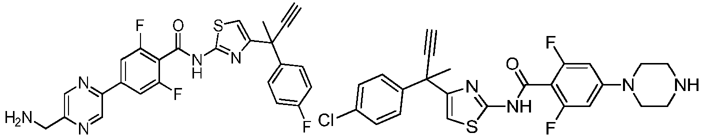

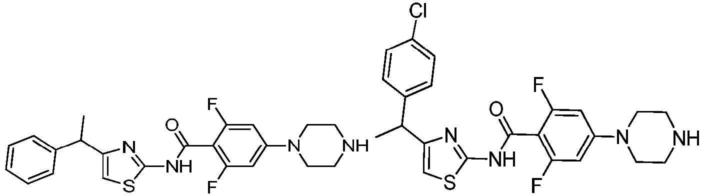

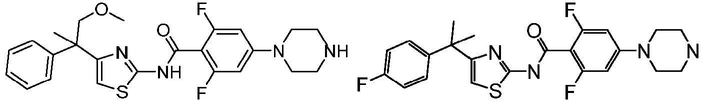

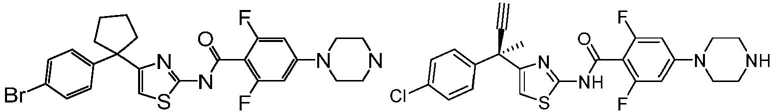

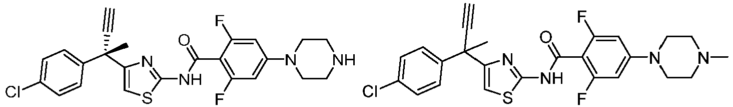

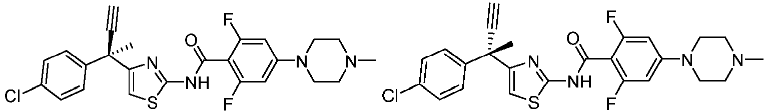

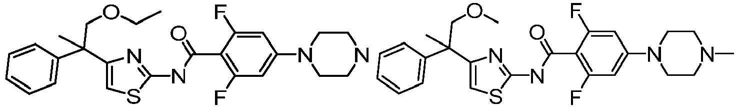

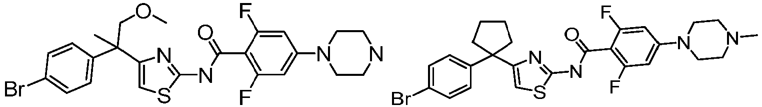

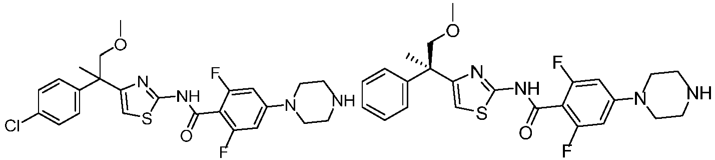

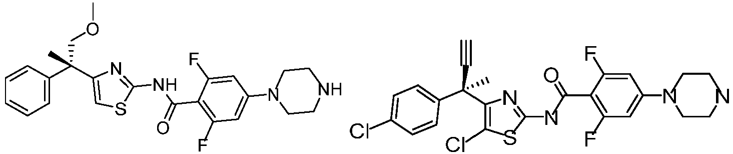

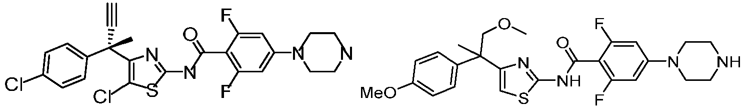

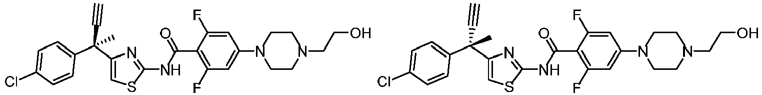

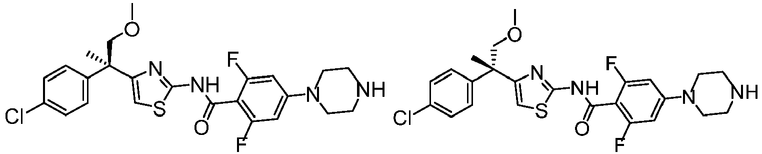

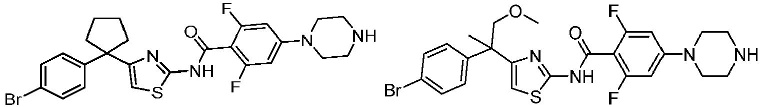

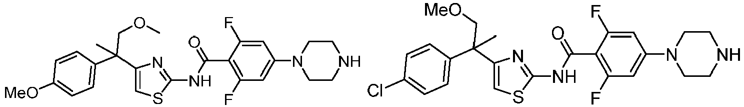

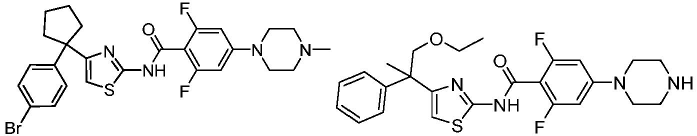

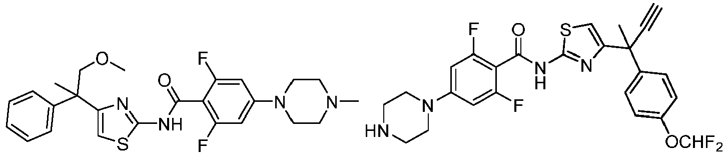

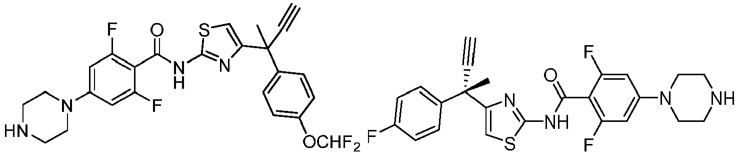

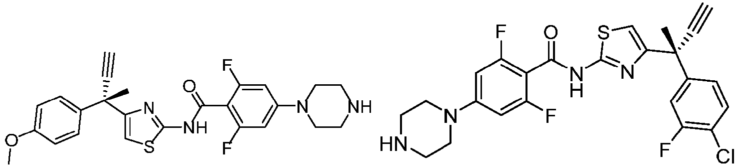

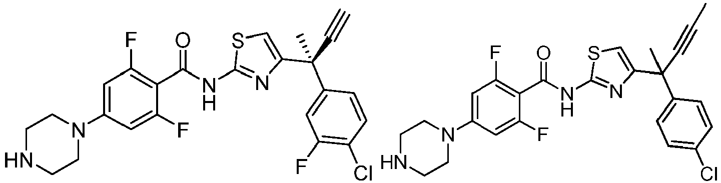

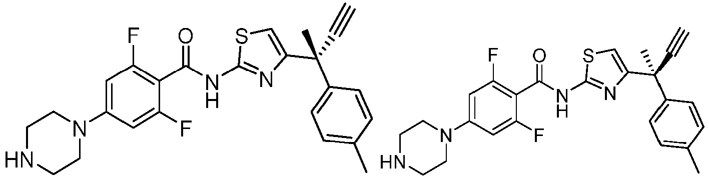

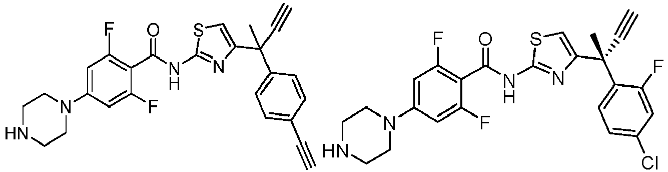

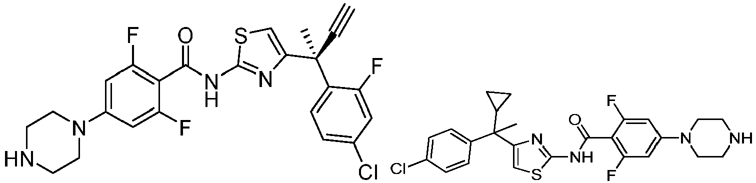

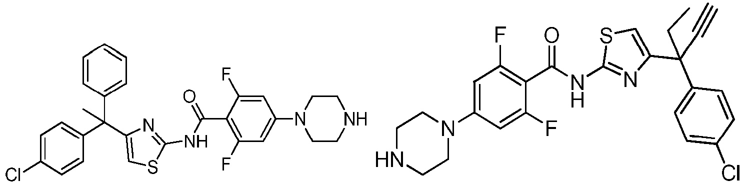

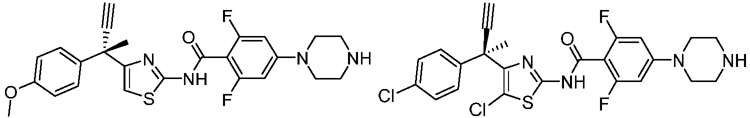

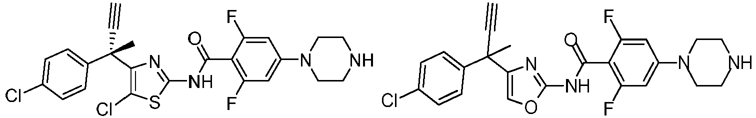

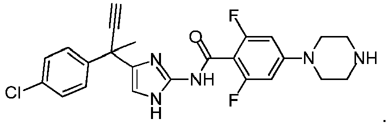

14. A pharmaceutical composition comprising a compound of any one of claims 1 to 13, and a pharmaceutically acceptable carrier or excipient.

15. The compound of any one of claims 1 to 13 for use in a method for inhibiting ALPK1 kinase activity in a cell or tissue of a subject in need of such therapy, the method comprising administering the compound to the subject.

16. The compound of any one of claims 1 to 13 for use in a method for inhibiting or reducing inflammation in a target tissue of a subject in need of such treatment, the method comprising administering the compound to the subject.

17. The compound of any one of claims 1 to 13 for use in a method for treating a disease, disorder, or condition **characterized by** excessive or inappropriate ALPK1-dependent proinflammatory signaling in a subject in need of such therapy, the method comprising administering the compound to the subject
optionally wherein the disease, disorder, or condition is selected from sepsis, cancer, spiroandenoma, spiroandenocarcinoma, "Retinal dystrophy, Optic nerve edema, Splenomegaly, Anhidrosis and migraine Headache" ("ROSAH") syndrome, and "Periodic Fever, Aphthous Stomatitis, Pharyngitis, and Adenitis" ("PFAPA") syndrome.

18. The compound for the use of claim 17, wherein the subject in need of such therapy is a subject carrying one or more genetic mutations in ALPK1.

## Patentansprüche

1. Eine Verbindung der Formel XI-A-1 oder XI-B 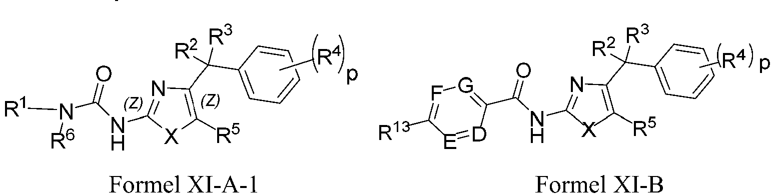
oder ein pharmazeutisch akzeptables Salz davon, wobei
X ausgewählt ist aus -S-, -O- und -NR^{a}-, wobei
R^{a} H oder ein C₁-C₆-Alkyl ist;
R⁶ ausgewählt ist aus H, D, -OH, einem gegebenenfalls substituierten C₁-C₆-Alkyl, wobei
die gegebenenfalls substituierten R⁶-Reste 0-3 Substituenten umfassen, die unabhängig voneinander ausgewählt sind aus -D, Halogen und -OH;
R¹ ausgewählt ist aus H und einem gegebenenfalls substituierten C₁-C₆-Alkyl;
wobei die gegebenenfalls substituierten R¹-Reste 0-4 Substituenten umfassen, die unabhängig voneinander ausgewählt sind aus -D, Halogen, -OH, -NH₂, =O, - CN, einem C₁-C₆-Hydroxyalkyl, einem C₁-C₆-hydroxy-deuterierten Alkyl, einem C₁-C₆-Alkoxy, -O-X¹-R^{7a}, -C(O)N(R^{7a}R^{8a}), S(O)2R^{7a}, -S(O)₂N(R^{7a}R^{8a}), - N(R^{7a}R^{8a}), einem gesättigten C₃-C₆-Cycloalkyl, einem gesättigten oder ungesättigten 3-7-gliedriges Heterocyclyl mit 1- 2 Heteroatomen an den Ringvertices, ausgewählt aus N, O und S, Phenyl, einem 5-10-gliedrigen Heteroaryl mit 1-4 Heteroatomen an den Ringvertices, ausgewählt aus N, O und S; wobei
jedes X₁ unabhängig ein C₁₋₆-Alkylen ist;
jedes R^{7a} und R^{8a} unabhängig voneinander ausgewählt sind aus H, einem C₁-C₆-Alkyl, einem C₁-C₆-Hydroxyalkyl, einem C₁-C₆-Aminoalkyl, Phenyl, einem gesättigten 3-7-gliedrigen Heterocyclyl mit 1-2 Heteroatomen an den Ringvertices, ausgewählt aus N, O und S, wobei die Phenyl- und 3-7-gliedrigen Heterocyclyl-Gruppen mit 0-3 Substituenten substituiert sind, die gewählt sind aus Halogen, -OH, - NH₂, und einem C₁-C₆-Alkyl, und
das C₃-C₆-Cycloalkyl-, das 3-7-gliedrige Heterocyclyl-,das Phenyl und das 5-10-gliedrige Heteroaryl jeweils unabhängig voneinander mit 0-3 Resten substituiert sind, die ausgewählt aus Halogen, -OH, -NH₂, einem C₁-C₆-Alkyl, einem C₁-C₆-Hydroxyalkyl, einem C₁-C₆-Halogenalkyl, einem C₁-C₆-Alkoxy, einem gesättigtem 3-7-gliedrigen Heterocyclyl mit 1-2 Heteroatomen an den Ringvertices, ausgewählt aus N, O und S, -C(O)N(R^{7b}R^{8b}), -S(O)₂ N(R^{7b}R^{8b}) und -N(R^{7b}R^{8b}), wobei
jedes R^{7b} und R^{8b} unabhängig voneinander ausgewählt sind aus H, einem C₁-C₆-Alkyl und einem C₁-C₆-Hydroxyalkyl; oder
R¹ und R⁶ miteinander verbunden sind, um ein 3-6 gliedriges Heterocycloalkyl zu bilden, das mit 0-3 Resten substituiert ist, die unabhängig voneinander ausgewählt sind aus der Gruppe bestehend aus -OH, -NH₂ und einem C₁-C₆-Hydroxyalkyl;
R⁵ ausgewählt ist aus H, Deuterium, Halogen, einem C₁-C₆-Alkyl, einem C₁-C₆-Deuteroalkyl und einem C₁-C₆-Halogenalkyl;
R² und R³ jeweils unabhängig voneinander ausgewählt sind aus H, OH, einem C₁-C₆-Alkyl, einem C₂-C₆-Alkinyl, einem C₃-C₆-Cycloalkyl und Phenyl, wobei das C₁-C₆-Alkyl, das C2-C₆-Alkinyl, das C₃-C₆-Cycloalkyl und das Phenyl jeweils mit 0-3 Resten substituiert sind, die unabhängig voneinander ausgewählt sind aus -OH und einem C₁-C₆-Alkoxy;
mit der Maßgabe, dass R² und R³ nicht beide H sind; oder
R² und R³ miteinander verbunden sind, um einen C₃-C₆-Cycloalkylring oder ein 3-7-gliedriges Heterocyclyl mit 1-2 Heteroatomen an den Ringvertices, ausgewählt unabhängig voneinander aus N, O und S, zu bilden;
jedes R⁴ unabhängig voneinander ausgewählt ist aus Halogen, -OH, einem C₁-C₆-Alkyl,
einem C₁-C₆-Alkoxy, einem gesättigten C₃-C₆-Cycloalkyl, O-R^{7e}, wobei
R^{7e} ausgewählt ist aus H, einem C₁-C₆-Alkyl und einem C₁-C₆-Halogenalkyl, und
der Index p 1, 2 oder 3 ist;
D CR¹⁰ oder N ist;
E CR¹⁴ oder N ist;
F CR¹² oder N ist;
G CR¹¹ oder N ist;
mit der Maßgabe, dass höchstens drei von D, E, F und G N sind;
R¹⁰, R¹¹, R¹², und R¹⁴, falls vorhanden, jeweils unabhängig voneinander ausgewählt sind aus H, Halogen, -OH, einem C₁-C₆-Alkoxy, -O-X¹-R^{7a}, -C(O)N(R^{7a}R^{8a}) und einem gesättigtem oder ungesättigten 3-7-gliedrigen Heterocyclyl mit 1-2 Heteroatomen an den Ringvertices, ausgewählt aus N, O und S; wobei
jedes X₁ unabhängig C₁₋₆ -Alkylen ist;
jedes R^{7a} und R^{8a} unabhängig voneinander ausgewählt sind aus H, einem C₁-C₆-Alkyl und einem C₁-C₆-Alkoxy;
R¹³ H, ein gesättigtes oder ungesättigtes 3- 7-gliedriges Heterocyclyl mit 1-2 Heteroatomen an den Ringvertices, ausgewählt aus N, O und S, ein gesättigtes oder ungesättigtes 7-8-gliedriges überbrücktes Heterocyclyl mit 1-2 Heteroatomen an den Ringvertices, ausgewählt aus N, O und S, ist, wobei
das 3- 7-gliedrige Heterocyclyl, das 7-8-gliedrige überbrücktes Heterocyclyl gegebenenfalls mit 0-2 Resten substituiert sind, die unabhängig voneinander ausgewählt sind aus -OH, einem C₁-C₆-Alkyl, einem C₁-C₆-Aminoalkyl, -NH₂ und einem C₁-C₆-Hydroxyalkyl .

2. Die Verbindung nach Anspruch 1, wobei X -S-, -O- oder -NH- ist.

3. Die Verbindung nach Anspruch 1, aufweisend:
Formel XI-A-1-a 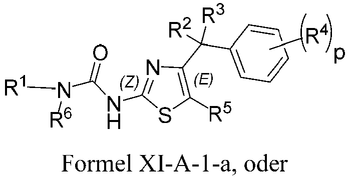
ein pharmazeutisch akzeptables Salz davon, oder, R⁶ ausgewählt ist aus H, einem C₁-C₆-Alkyl,
einem C₁-C₆-Hydroxyalkyl und C₁-C₆-hydroxy-deuteriertem Alkyl.

4. Die Verbindung nach einem der Ansprüche 1 bis 3, wobei:
a) R¹ mit R⁶ zu einem 3-6-gliedrigen Heterocycloalkyl verbindet ist, das mit 0-3 Resten substituiert ist, die unabhängig voneinander ausgewählt sind aus der Gruppe bestehend aus - OH, -NH₂ und einem C₁-C₆-Hydroxyalkyl ,
b) R₁ ein C₁-C₆-Alkyl ist, das mit 0-4 Substituenten substituiert ist, die unabhängig voneinander ausgewählt sind aus -OH, einem C₁-C₆-Hydroxyalkyl, einem C₁-C₆-Alkoxy, - S(O)₂N(R^{7a}R^{8a}) und -N(R^{7a}R^{8a}), wobei
jedes R^{7a} und R^{8a} unabhängig voneinander ausgewählt sind aus H, einem C₁-C₆-Alkyl und einem C₁-C₆-Aminoalkyl,
oder
c) R¹ ein C₁-C₆-Alkyl ist, das mit 0-2 Substituenten substituiert ist, die unabhängig voneinander ausgewählt sind aus -OH, einem C₁-C₆-Hydroxyalkyl und -S(O)₂N(R^{7a}R^{8a}), wobei
jedes R^{7a} und R^{8a} unabhängig voneinander ausgewählt aus H und einem C₁-C₆-Alkyl.

5. Die Verbindung nach Anspruch 1, wobei:
a) D, E, F und G jeweils CR¹⁰, CR¹⁴, CR¹², bzw. CR¹¹ sind, oder
b) F und G jeweils CR¹⁴ bzw. CR¹¹ sind, E N oder CR¹⁴ ist und D N oder CR¹⁰ ist.

6. Die Verbindung nach Anspruch 1 oder 5, wobei:
a) R¹⁰ und R¹¹ jeweils H sind;
R¹² und R¹⁴ jeweils unabhängig voneinander ausgewählt sind, aus Halogen, -OH, einem C₁-C₆-Alkoxy und -C(O)N(R^{7b}R^{8b}) wobei
R^{7b} und R^{8b} jeweils unabhängig voneinander ausgewählt sind aus H, einem C₁-C₆-Alkyl und einem C₁-C₆-Alkoxy; und
R¹³ ausgewählt ist aus 3-7-gliedrigem Heterocyclyl mit 1-2 Heteroatomen an den Ringvertices, ausgewählt aus N, O und S, und einem gesättigten oder ungesättigten 7-8-gliedrigen überbrückten Heterocyclyl mit 1-2 Heteroatomen an den Ringvertices, ausgewählt aus N, O und S, wobei
das 3-7-gliedrige Heterocyclyl und das 7-8-gliedrige überbrückte Heterocyclyl gegebenenfalls mit 0-2 Resten substituiert sind, die unabhängig voneinander ausgewählt sind aus -OH, einem C₁-C₆-Alkyl, einem C₁-C₆-Hydroxyalkyl und einem C₁-C₆-Aminoalkyl,
b) R¹² und R¹⁴ H sind;
R¹⁰ und R¹¹ jeweils unabhängig voneinander ausgewählt sind aus Halogen, -OH, einem C₁-C₆-Alkoxy und -C(O)N(R^{7b}R^{8b}), wobei
R^{7b} und R^{8b} jeweils unabhängig voneinander ausgewählt sind aus H, einem C₁-C₆-Alkyl und einem C₁-C₆-Alkoxy; und
R¹³ ausgewählt ist aus einem 3-7-gliedrigen Heterocyclyl mit 1-2 Heteroatomen an den Ringvertices, ausgewählt aus N, O und S, und einem gesättigten oder ungesättigten 7-8-gliedrigem überbrückten Heterocyclyl mit 1-2 Heteroatomen an den Ringvertices, ausgewählt aus N, O und S, wobei
das 3-7-gliedrige Heterocyclyl und das 7- 8-gliedrige überbrückte Heterocyclyl gegebenenfalls mit 0-2 Resten substituiert sind, die unabhängig voneinander ausgewählt sind aus -OH, einem C₁-C₆-Alkyl, einem C₁-C₆-Hydroxyalkyl und einem C₁-C₆-Aminoalkyl,
c) R¹⁰, R¹¹, R¹² und R¹⁴, falls vorhanden, jeweils H sind; und
R¹³ ausgewählt ist aus einem gesättigten oder ungesättigten C₃-C₆-Cycloalkyl, einem 3-7-gliedrigen Heterocyclyl mit 1-2 Heteroatomen an den Ringvertices, ausgewählt aus N, O und S, und einem gesättigten oder ungesättigten 7-8-gliedrigen überbrückten Heterocyclyl mit 1-2 Heteroatomen an den Ringvertices, ausgewählt aus N, O und S, wobei
das 3-7-gliedrige Heterocyclyl und das 7-8-gliedrige überbrückte Heterocyclyl gegebenenfalls mit 0-2 Resten substituiert sind, die unabhängig voneinander ausgewählt sind aus -OH, einem C₁-C₆-Alkyl, einem C₁-C₆-Hydroxyalkyl und einem C₁-C₆-Aminoalkyl,
d) R¹⁰, R¹¹, R¹² und R¹⁴, falls vorhanden, jeweils H sind; und
R¹³ ein 3-7-gliedriges Heterocyclyl mit 1-2 Heteroatomen an den Ringvertices, ausgewählt aus N, O und S, ist, das mit 0-2 Resten substituiert ist, die unabhängig voneinander ausgewählt sind aus -OH, einem C₁-C₆-Alkyl, einem C₁-C₆-Hydroxyalkyl und einem C₁-C₆-Aminoalkyl
oder
e) R¹⁰, R¹¹, R¹² und R¹⁴, falls vorhanden, jeweils H sind; und
R¹³ ein gegebenenfalls substituiertes gesättigtes oder ungesättigtes 7-8-gliedriges überbrücktes Heterocyclyl ist mit 1-2 Heteroatomen an den Ringvertices, ausgewählt aus N, O und S, das mit 0-2 Substituenten substituiert ist, die ausgewählt sind aus -OH und einem C₁-C₆-Alkyl.

7. Die Verbindung nach Anspruch 1, aufweisend die Formel XI-B-1 oder XI-B-2 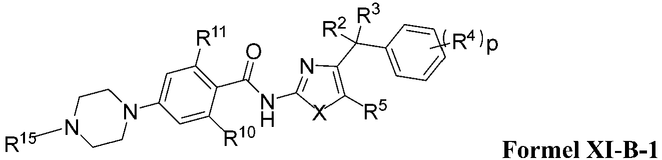 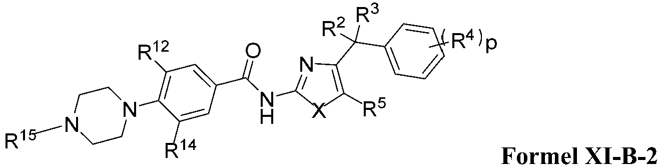
oder ein pharmazeutisch akzeptables Salz davon, wobei
R¹⁵ ausgewählt ist aus -OH, einem C₁-C₆-Alkyl, einem C₁-C₆-Hydroxyalkyl und einem C₁-C₆-Aminoalkyl,
gegebenenfalls wobei R¹⁰, R¹¹, R¹² und R¹⁴ jeweils unabhängig voneinander ausgewählt sind aus Halogen und einem C₁-C₆-Alkyl.

8. Die Verbindung nach Anspruch 7, wobei:
a) R¹⁵ ausgewählt ist aus einem C₁-C₆-Alkyl,
b) X -S- ist, aufweisend die Formel XI-B-1-a oder die Formel XI-B-2-a

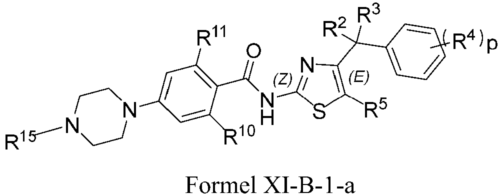

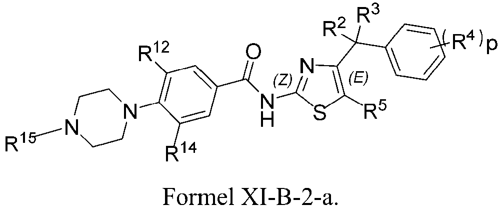

9. Die Verbindung nach Anspruch 7 oder 8, aufweisend:
a) die Formel XI-B-1-a-I oder Formel XI-B-2-a-I 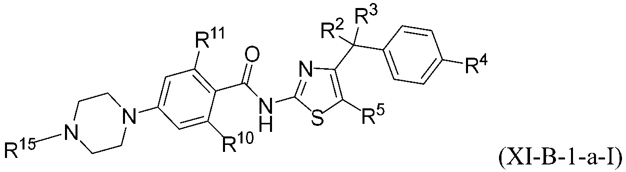 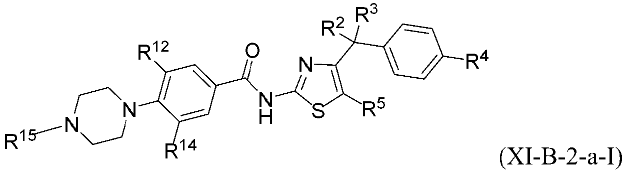 oder ein pharmazeutisch akzeptables Salz davon, wobei R⁴ Halogen ist,
b) die Formel XI-B-1-a-II oder Formel XI-B-2-a-II 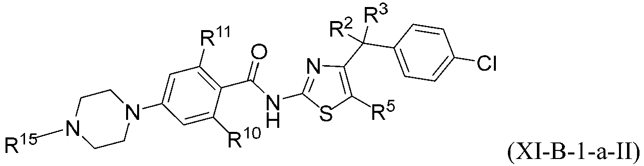 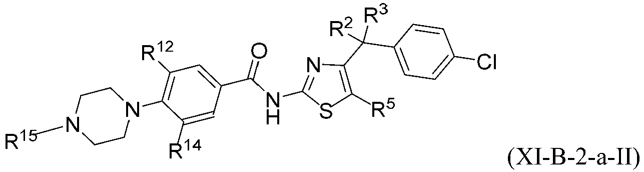 oder ein pharmazeutisch akzeptables Salz davon,
c) die Formel XI-B-1-a-III oder Formel XI-B-2-a-III 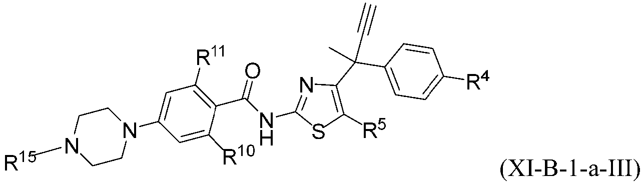 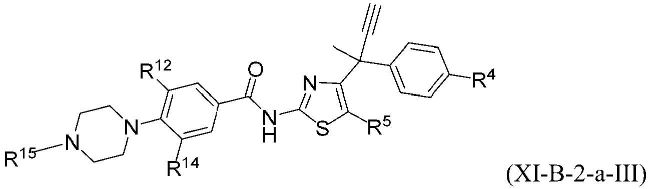 oder ein pharmazeutisch akzeptables Salz davon,
oder
d) Die Formel XI-B-1-a-IV oder Formel XI-B-2-a-IV 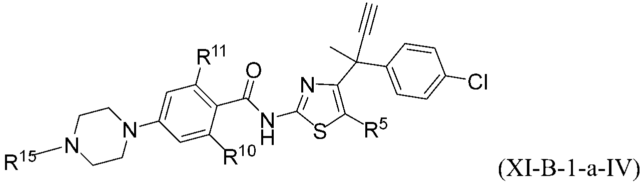 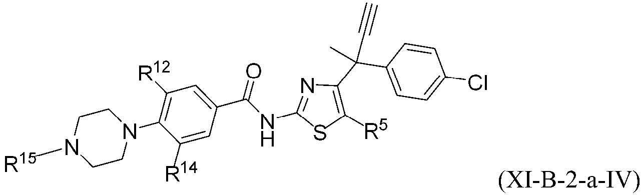 oder ein pharmazeutisch akzeptables Salz davon.

10. Die Verbindung nach einem der Ansprüche 1 bis 9, wobei der Index p 1 ist und R⁴:
a) an den Phenylring gebunden ist, wie nachstehend dargestellt: 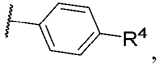 wobei die Wellenlinie die Bindungsstelle zum übrigen Teil der Formel darstellt,
b) Halogen, das an den Phenylring gebunden ist, wie nachstehend dargestellt: 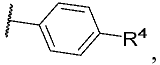 wobei die Wellenlinie die Bindungsstelle zum übrigen Teil der Formel darstellt,
c) Chlor, das an den Phenylring gebunden ist, wie nachstehend dargestellt: 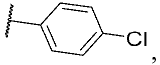 wobei die Wellenlinie die Bindungsstelle zum übrigen Teil der Formel darstellt,
oder
d) Methoxy, das an den Phenylring gebunden ist, wie nachstehend dargestellt: 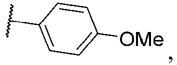 wobei die Wellenlinie die Bindungsstelle zum übrigen Teil der Formel darstellt.

11. Die Verbindung nach einem der Ansprüche 1 bis 10, wobei das Kohlenstoffatom, an das R² und R³ gebunden sind: a) das S-Isomer, oder b) das R-Isomer ist.

12. Die Verbindung nach Anspruch 1, wobei die Verbindung der Formel XI-A-1 oder XI-B ausgewählt ist aus 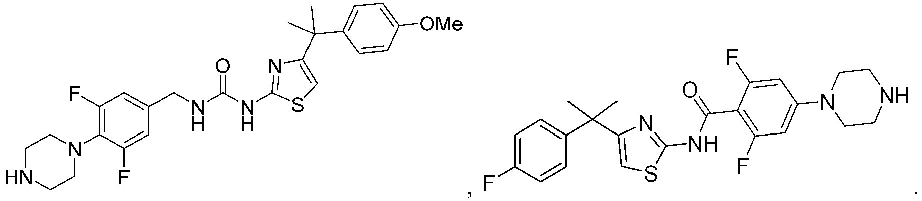 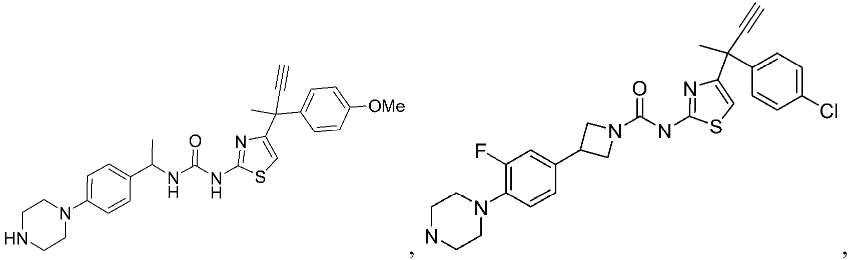 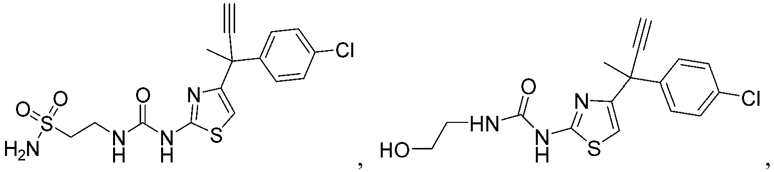 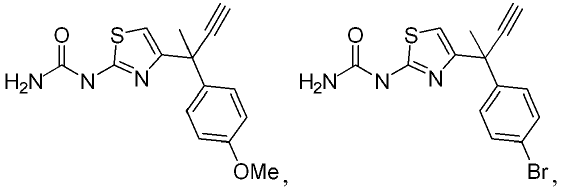 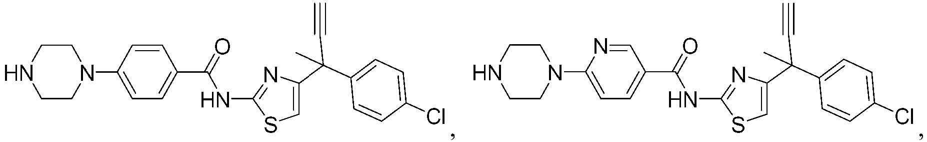 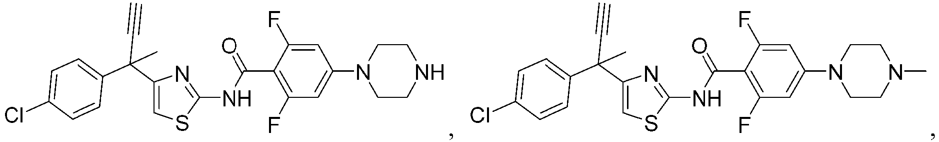 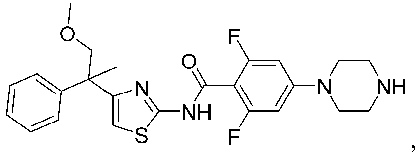 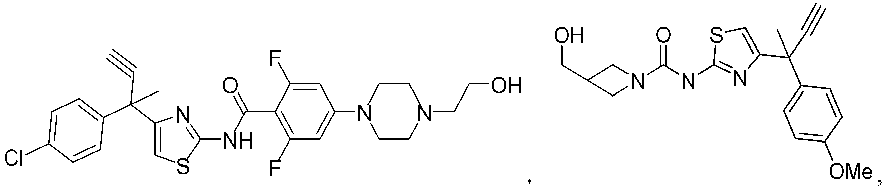 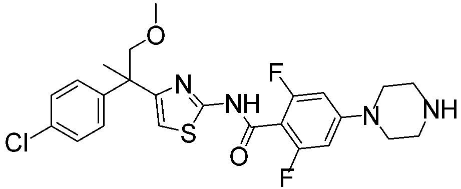 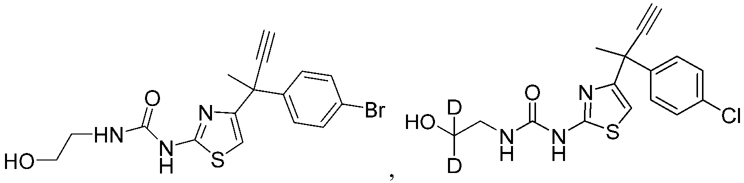 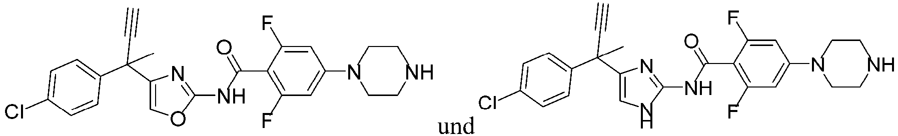 oder
wobei die Verbindung der Formel XI ausgewählt ist aus

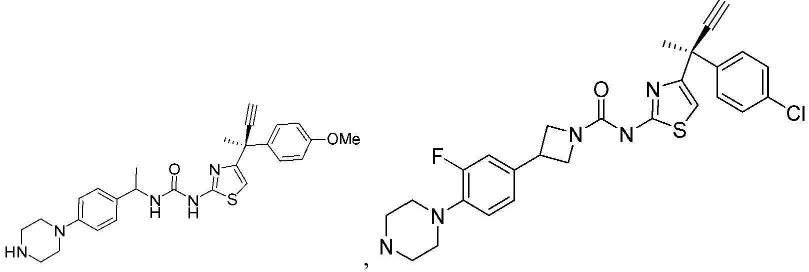

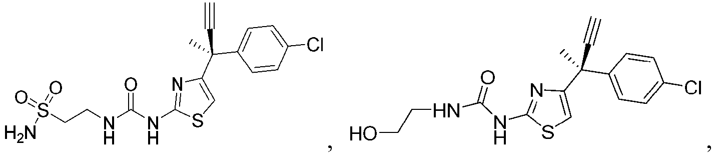

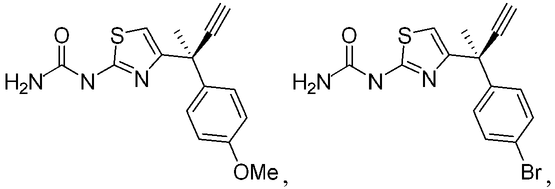

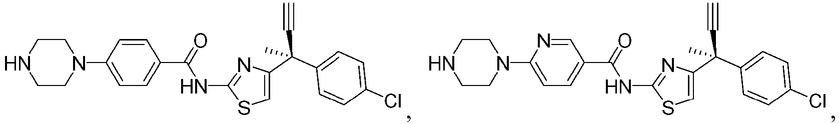

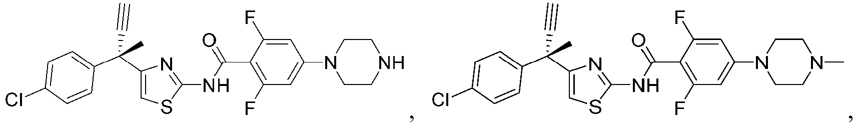

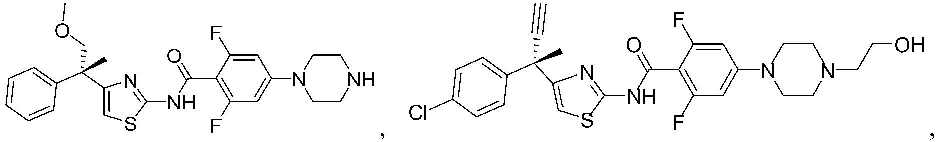

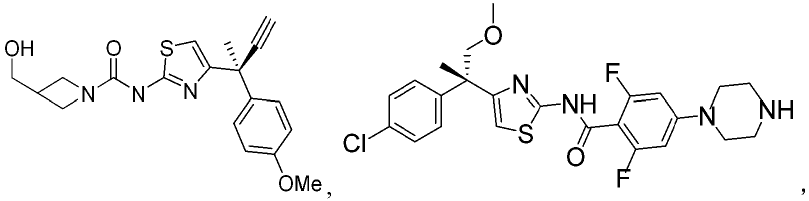

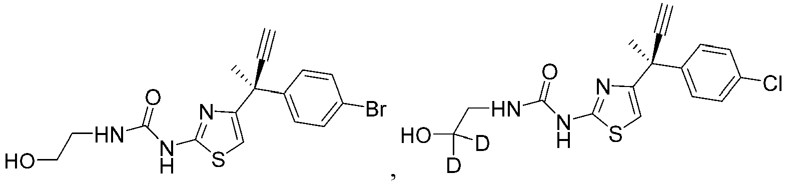

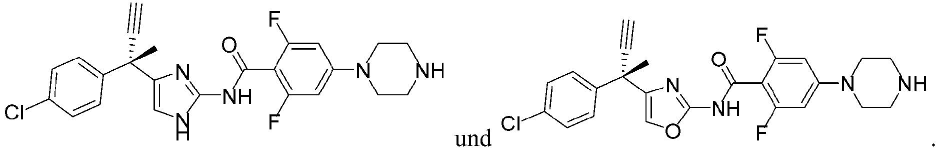

13. Die Verbindung nach Anspruch 1, ausgewählt aus einer der folgenden Verbindungen:

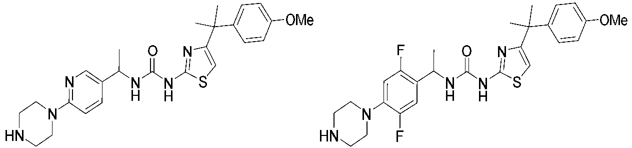

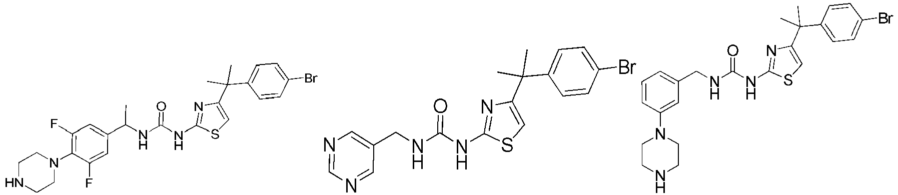

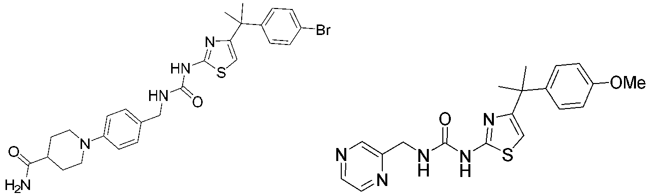

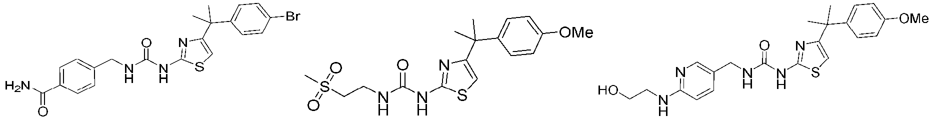

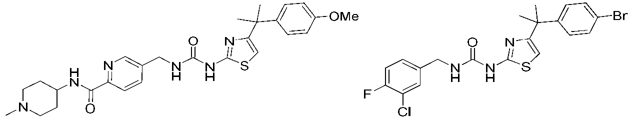

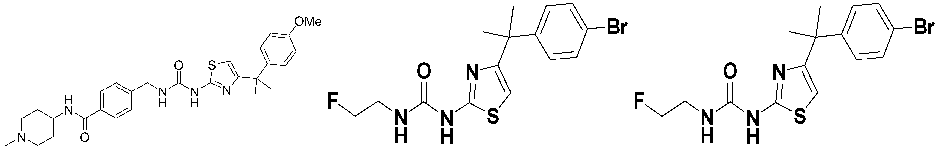

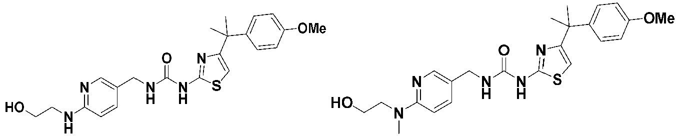

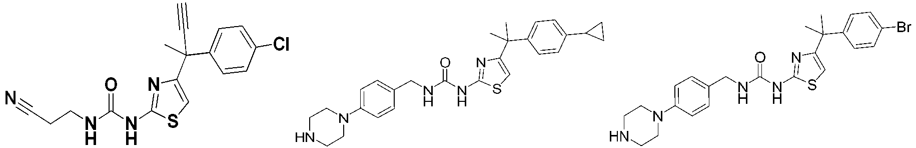

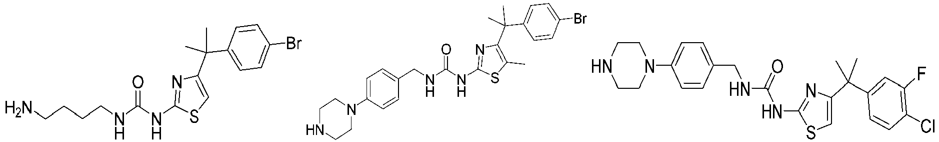

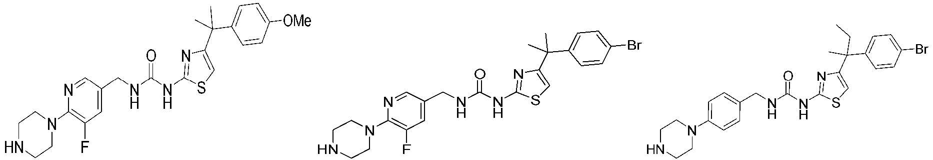

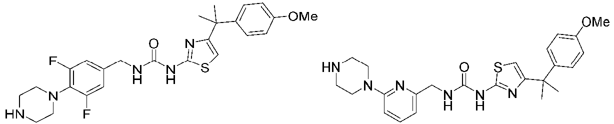

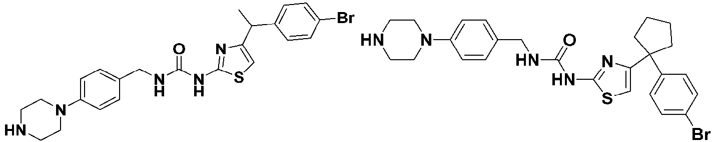

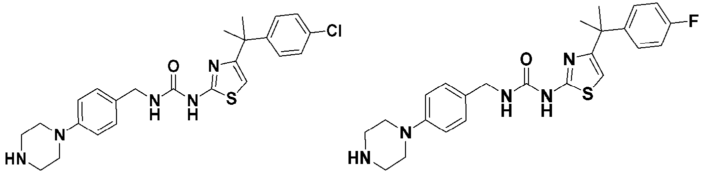

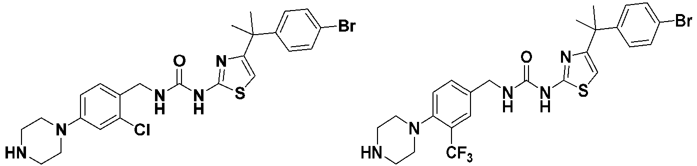

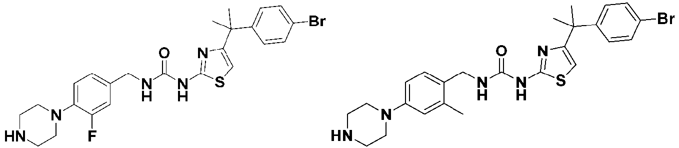

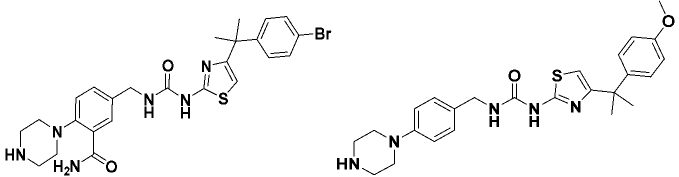

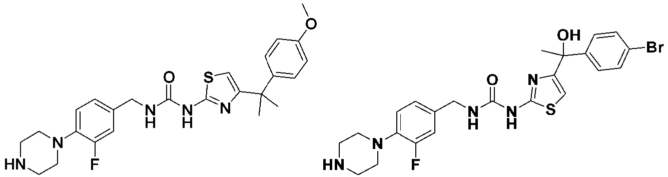

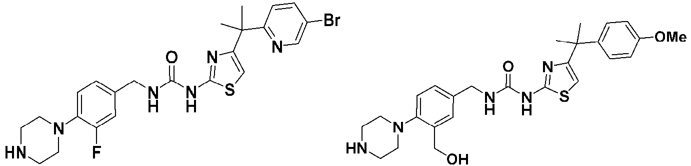

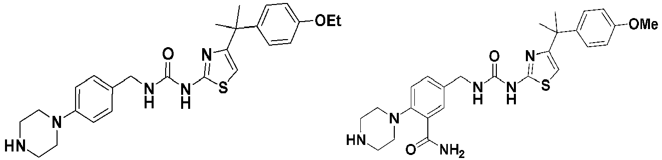

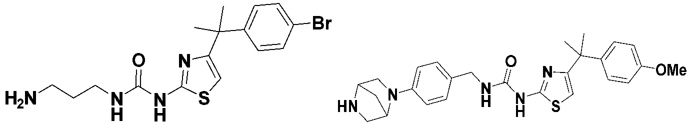

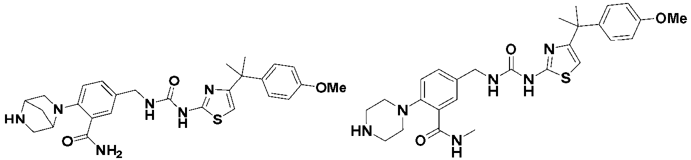

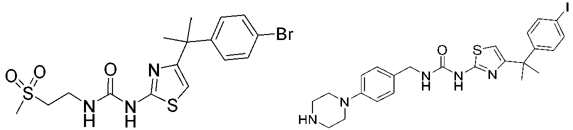

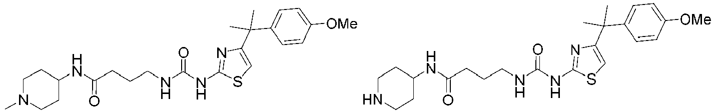

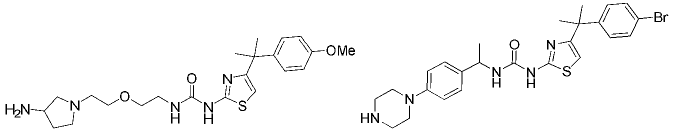

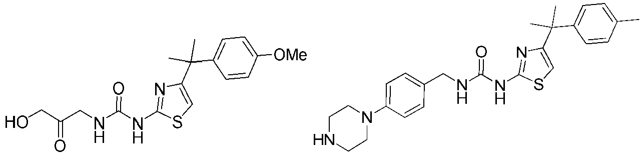

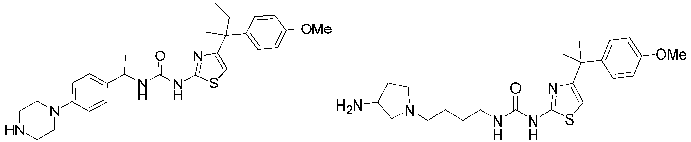

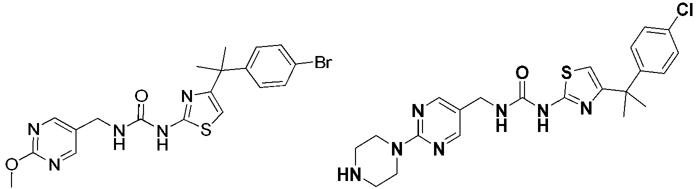

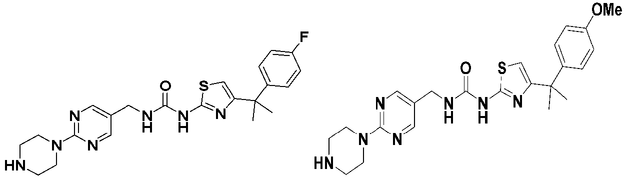

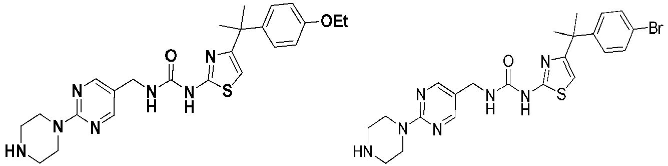

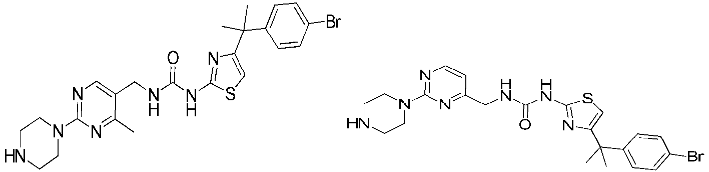

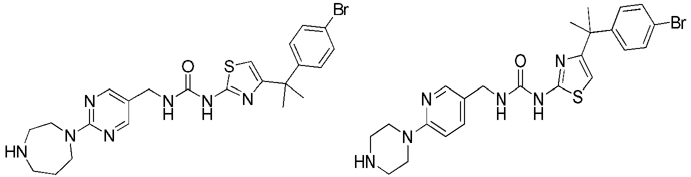

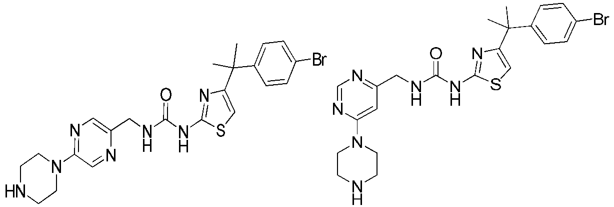

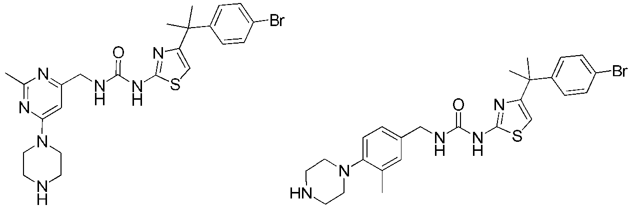

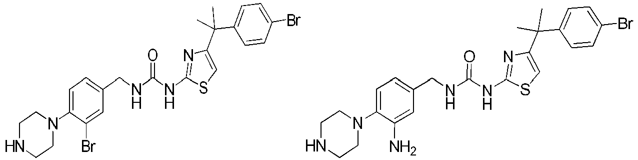

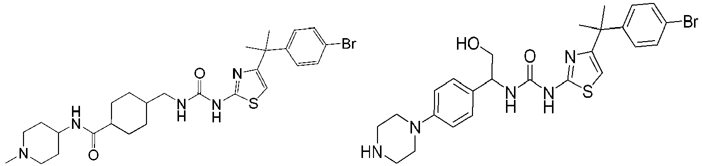

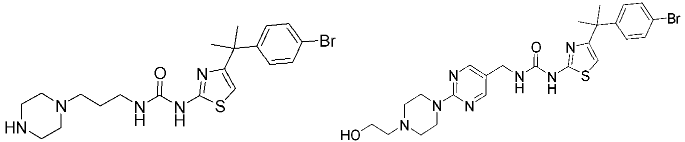

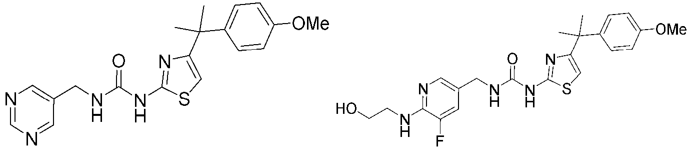

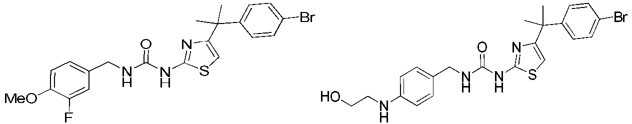

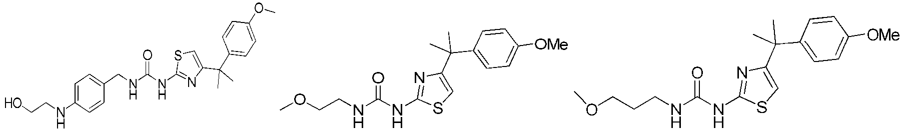

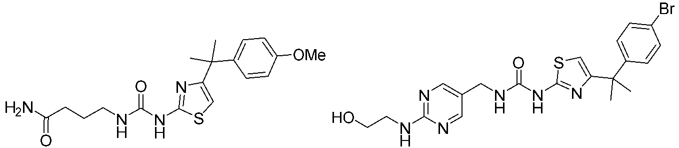

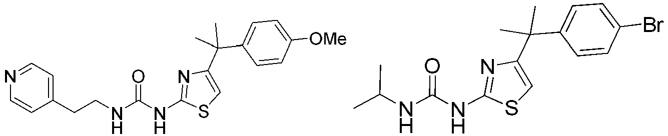

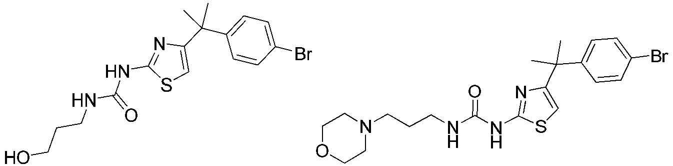

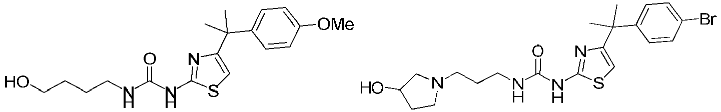

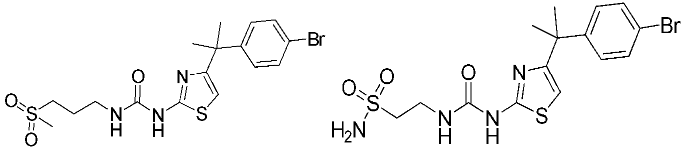

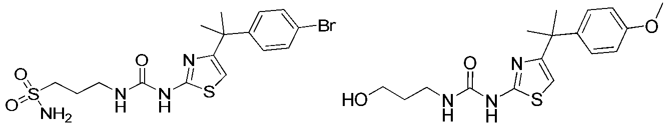

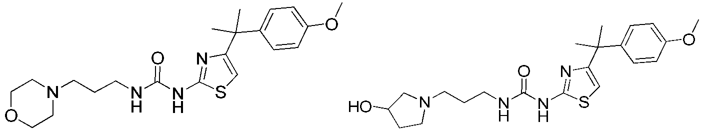

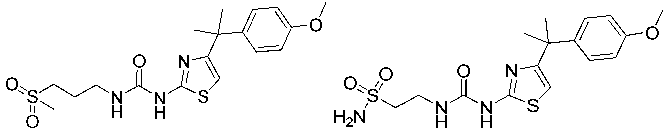

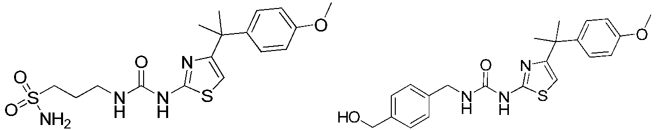

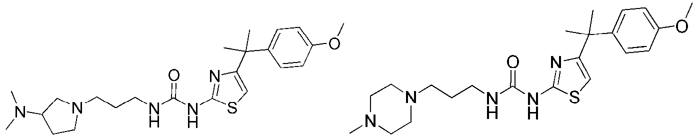

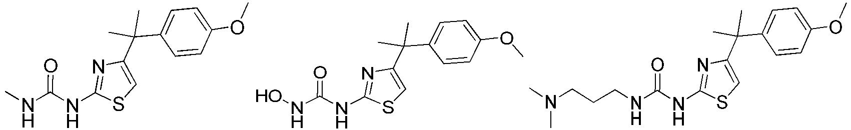

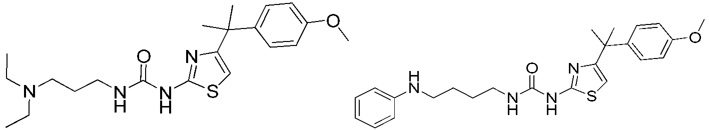

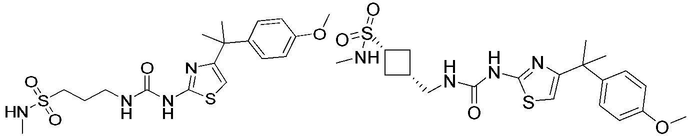

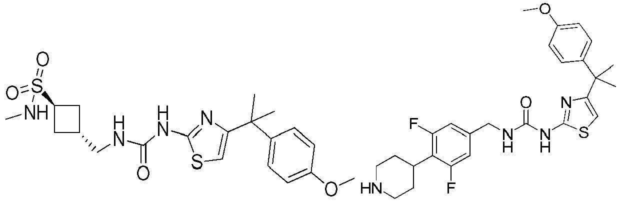

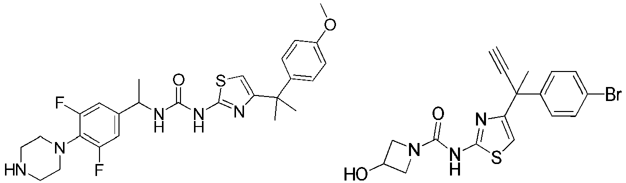

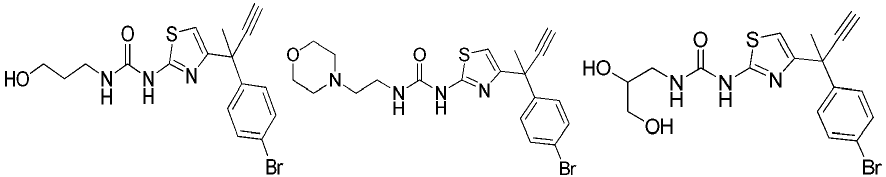

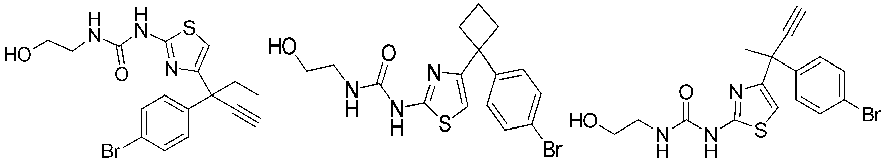

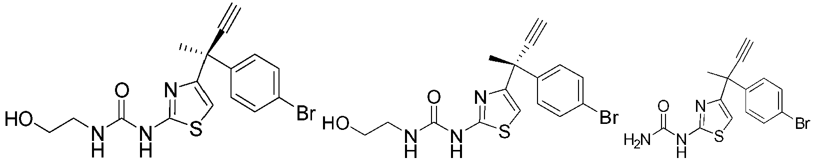

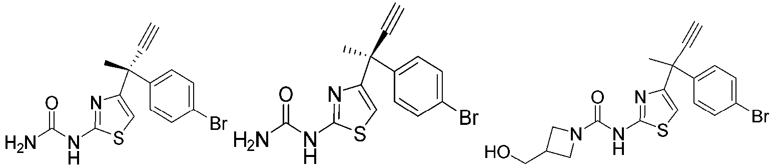

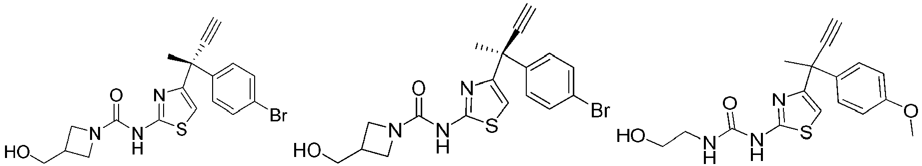

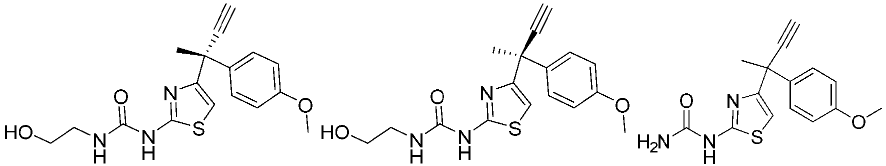

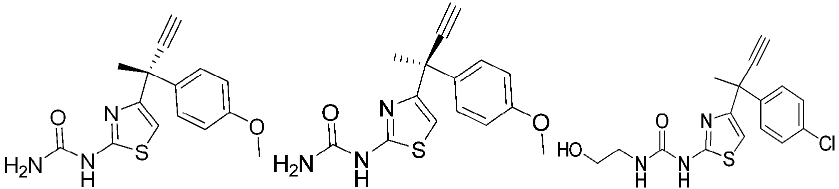

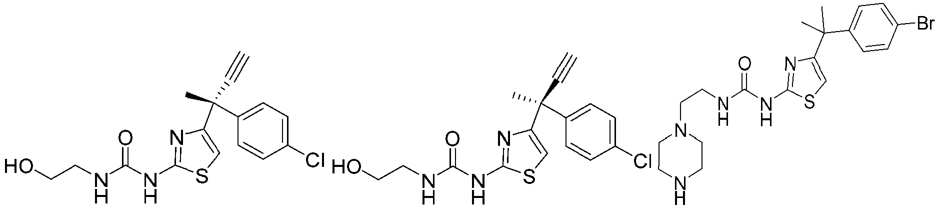

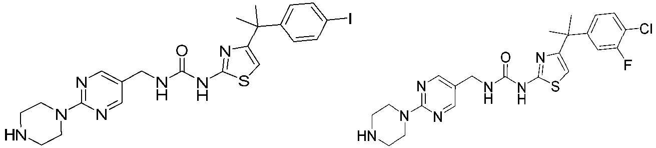

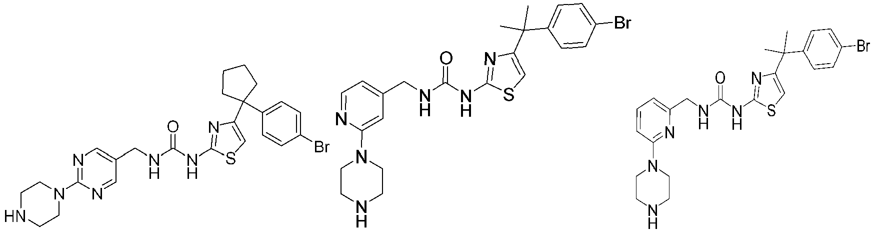

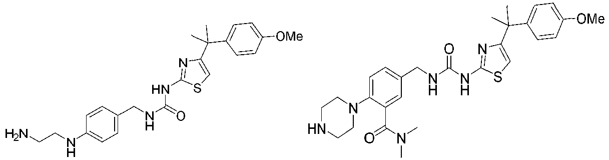

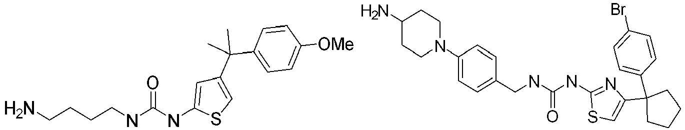

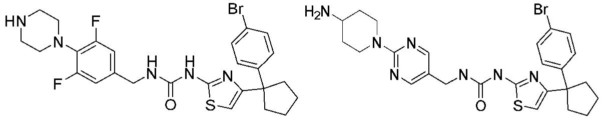

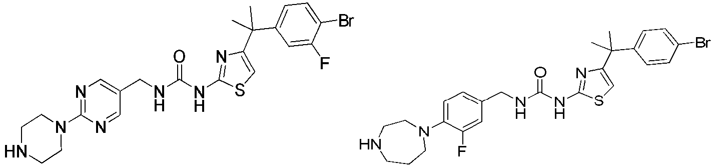

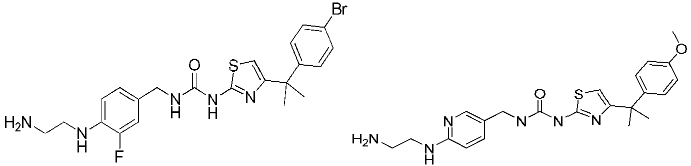

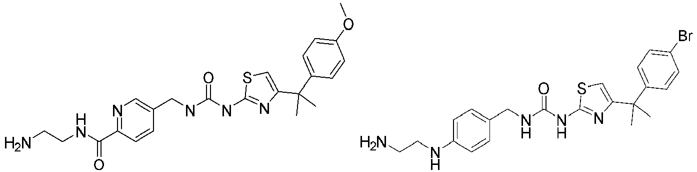

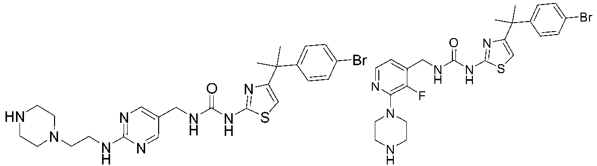

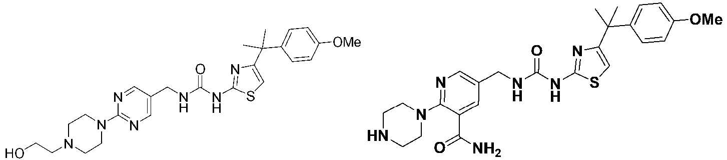

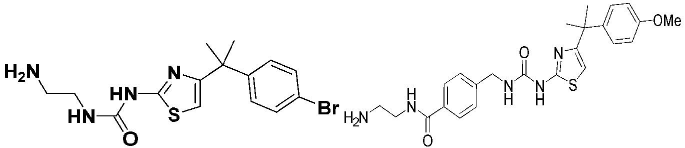

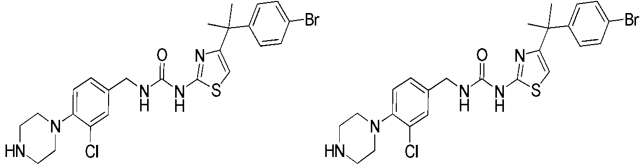

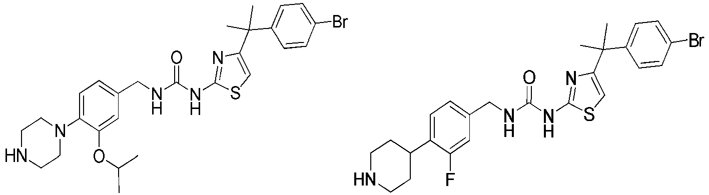

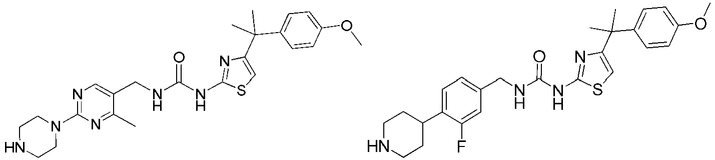

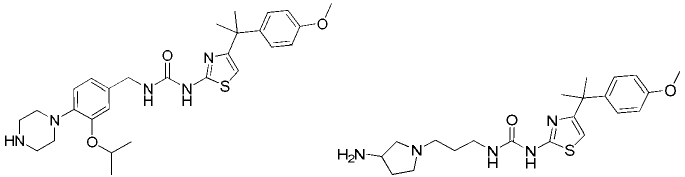

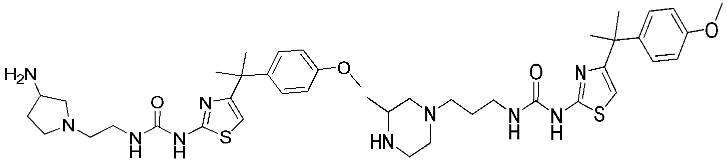

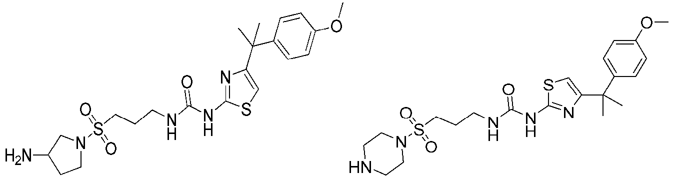

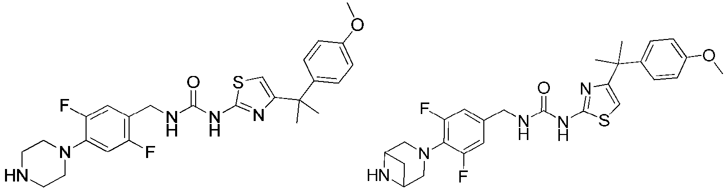

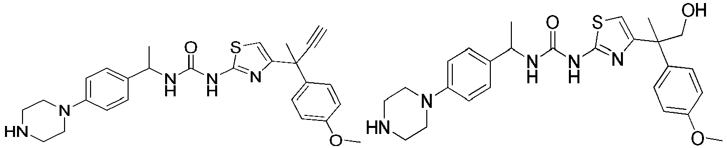

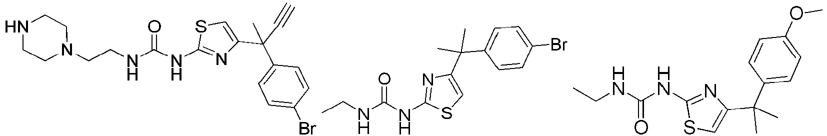

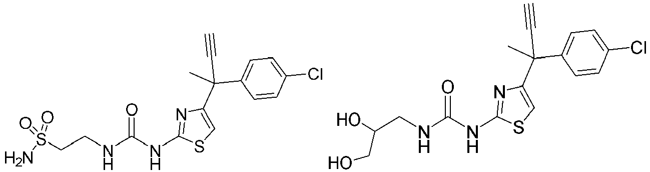

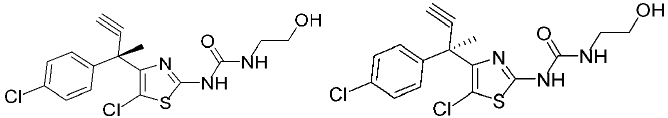

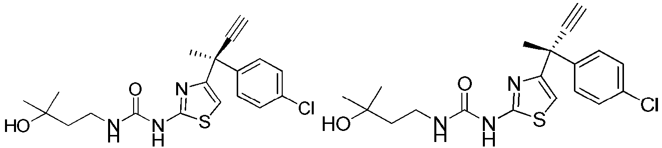

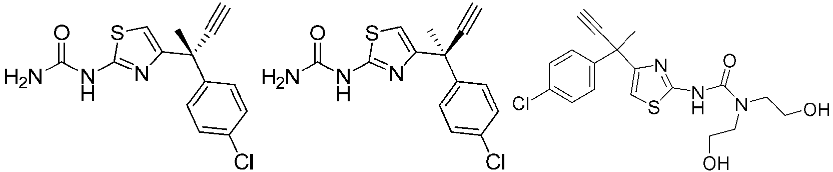

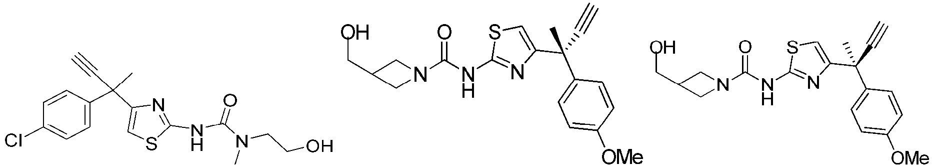

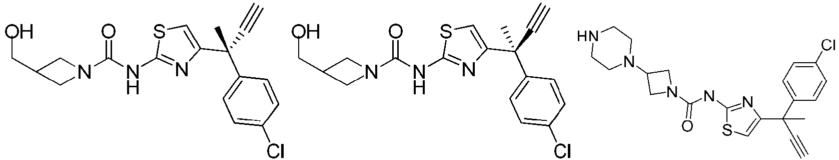

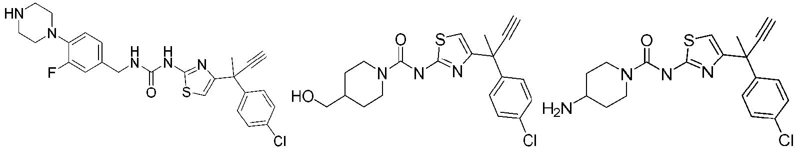

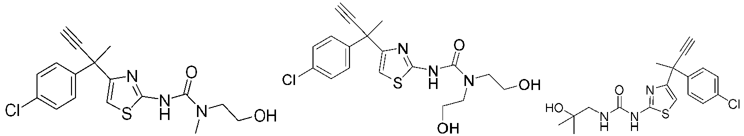

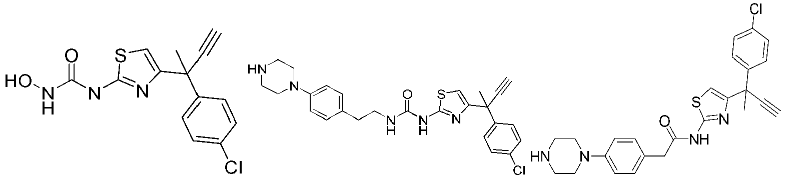

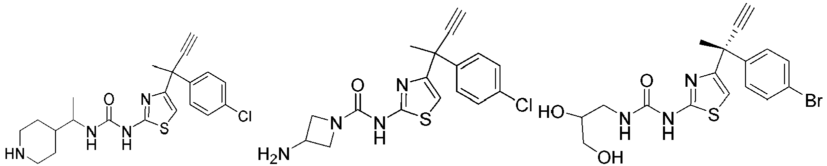

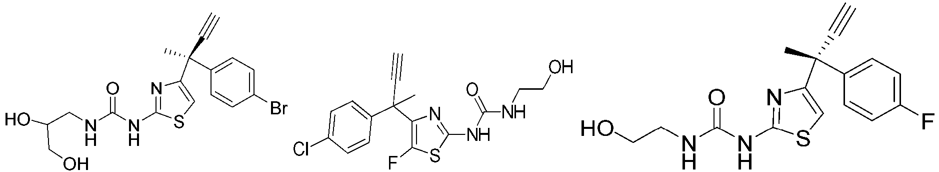

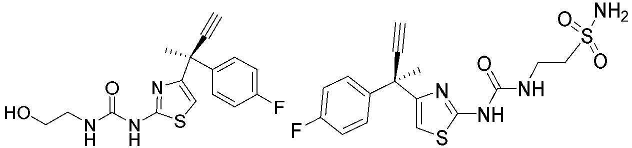

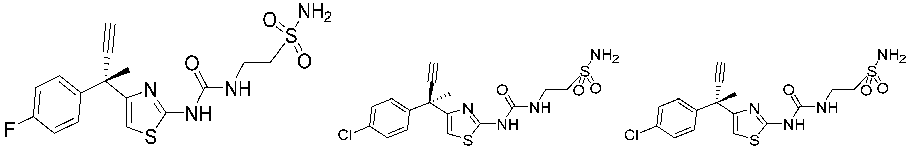

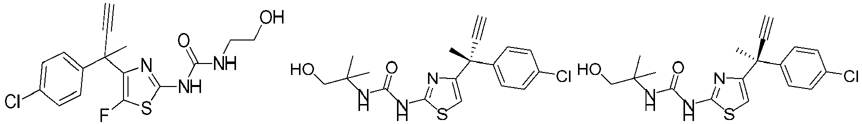

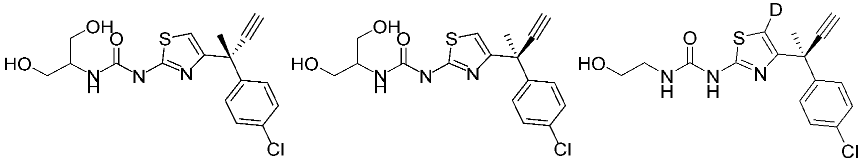

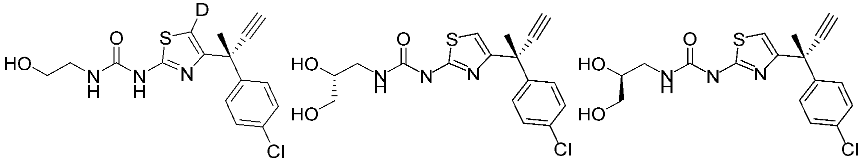

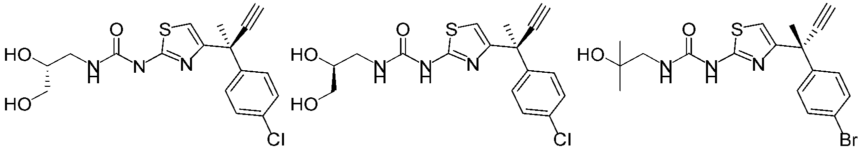

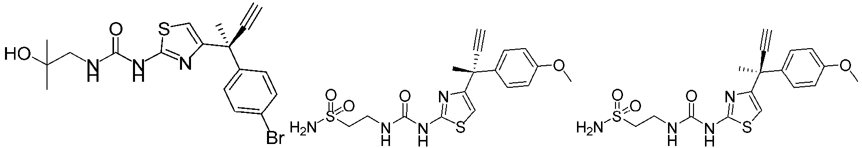

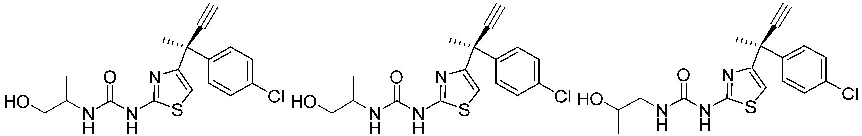

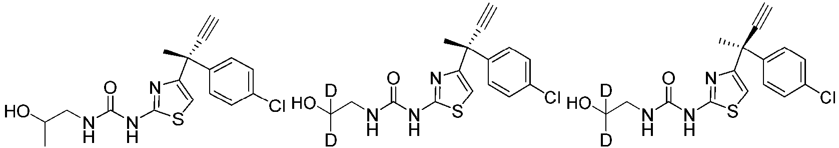

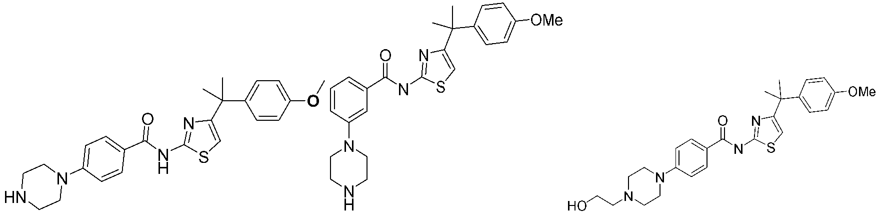

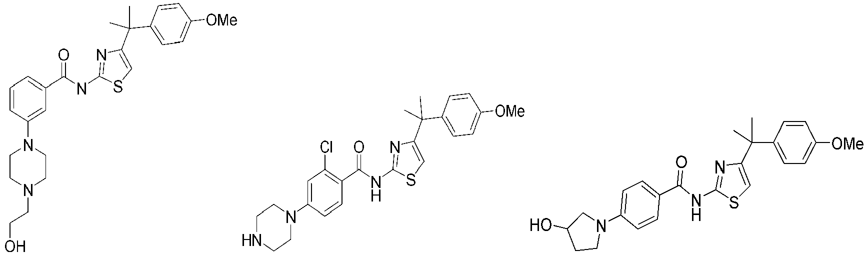

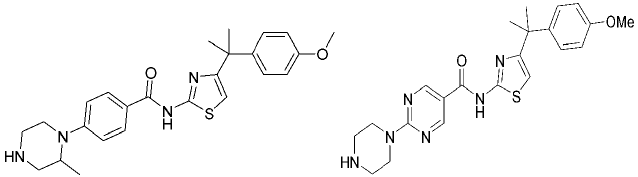

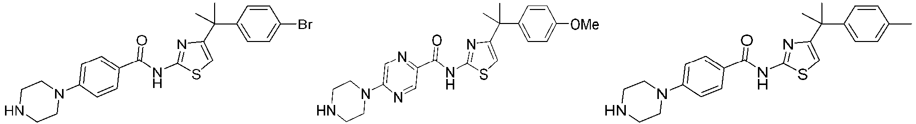

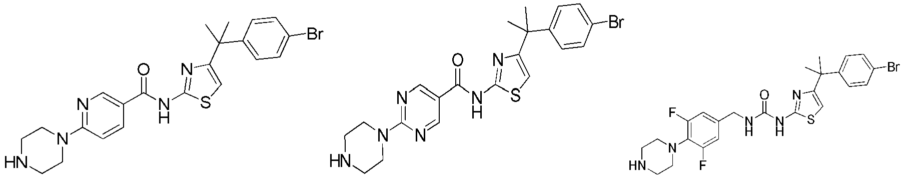

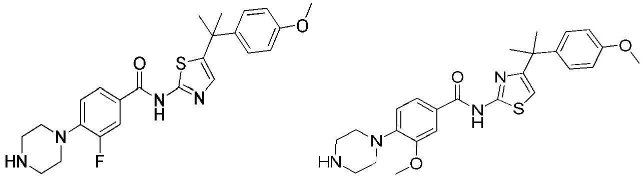

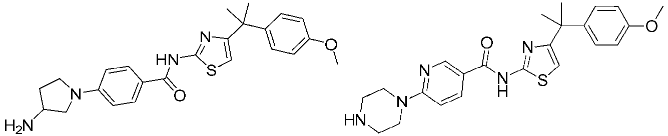

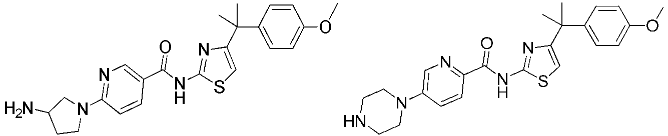

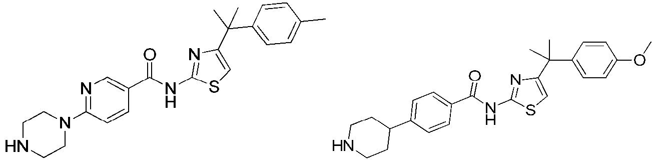

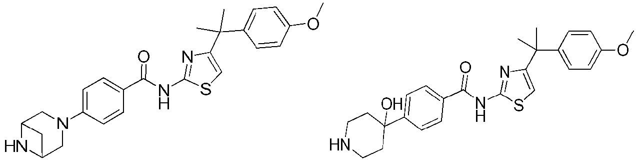

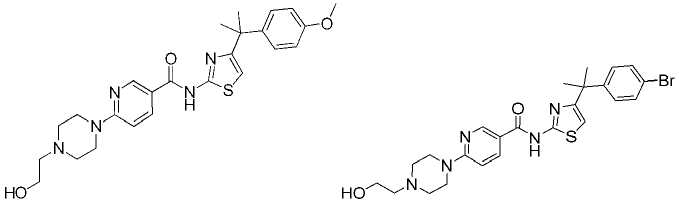

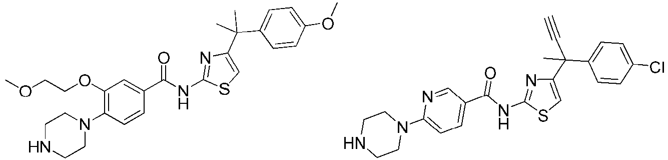

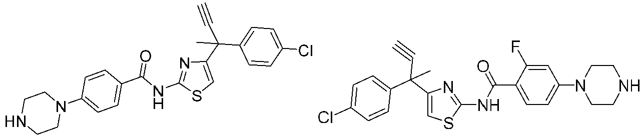

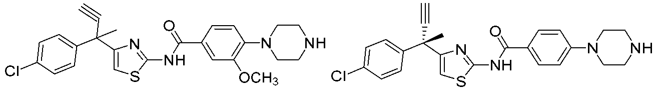

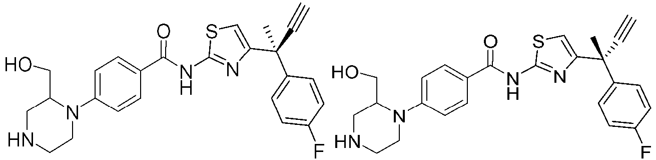

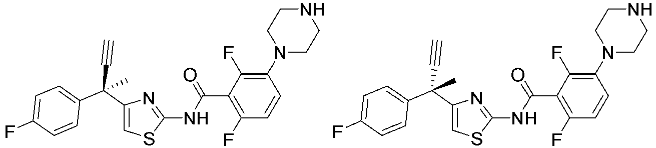

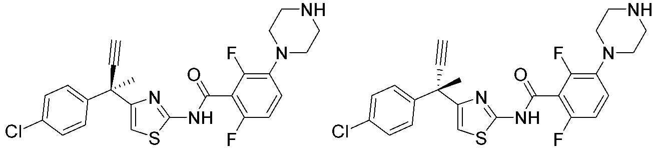

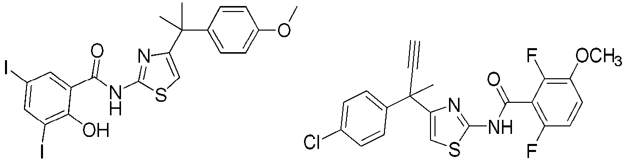

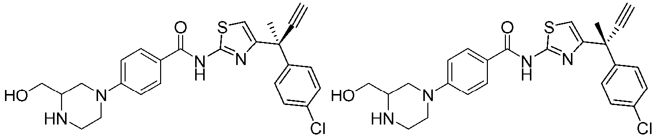

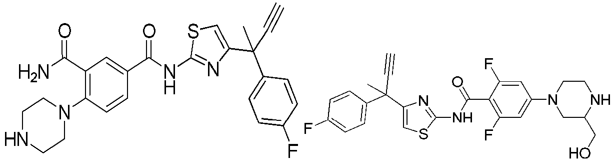

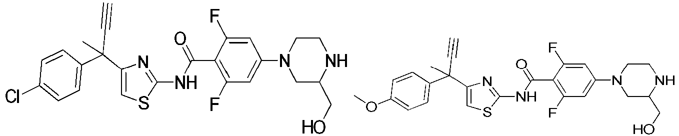

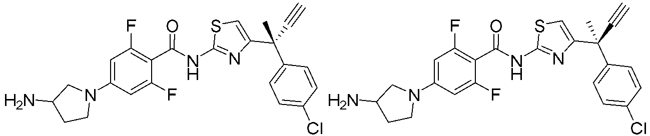

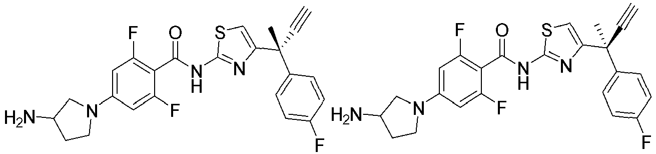

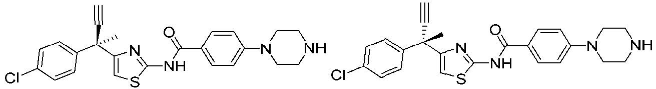

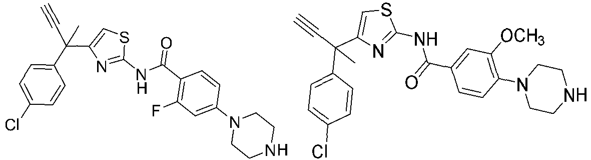

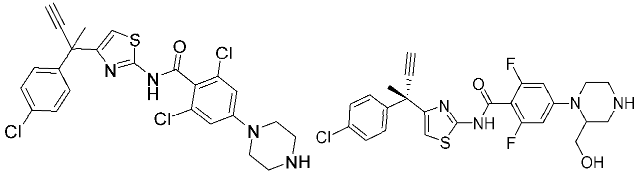

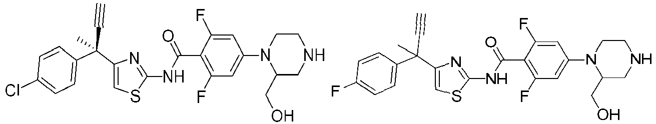

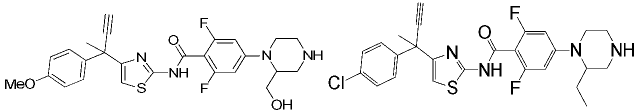

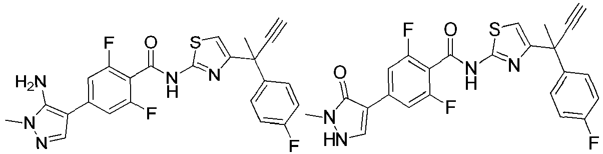

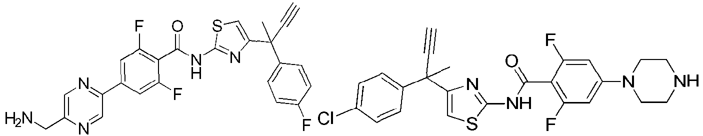

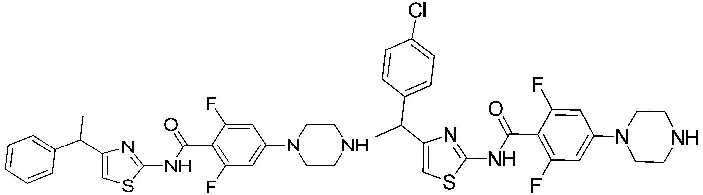

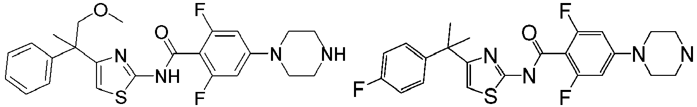

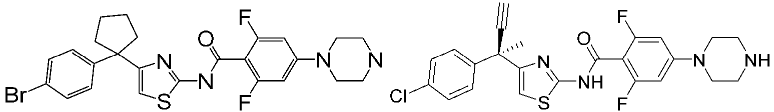

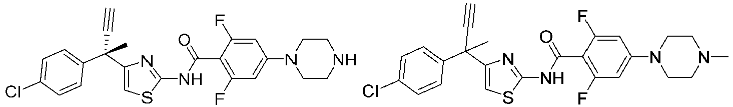

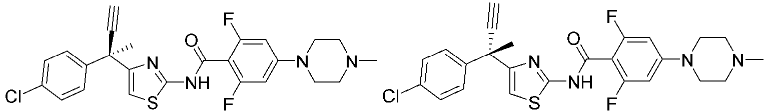

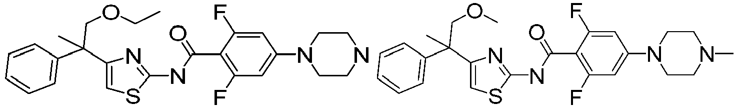

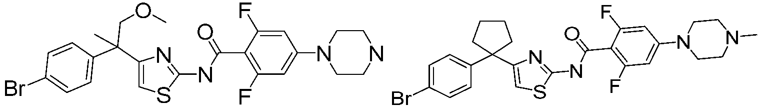

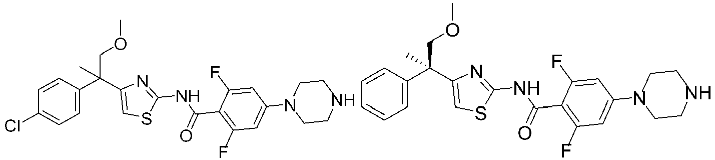

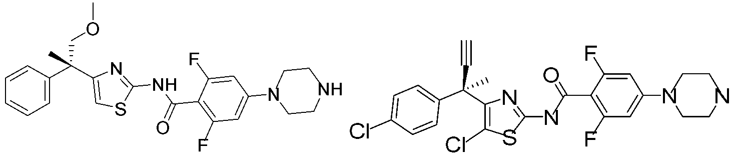

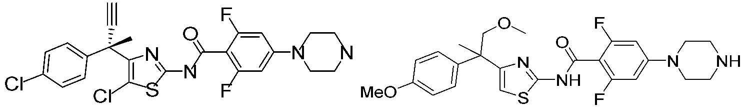

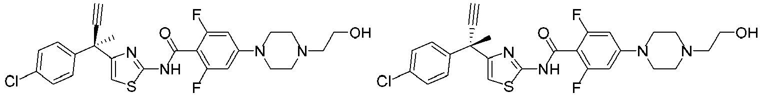

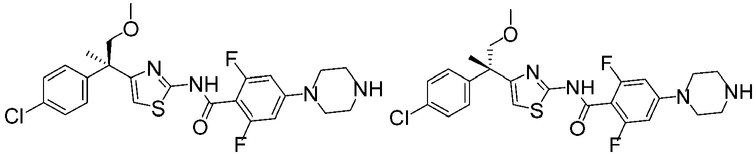

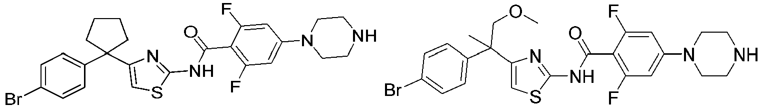

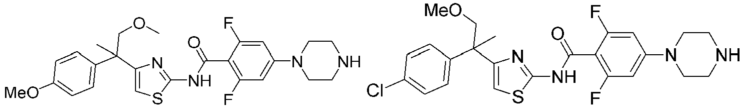

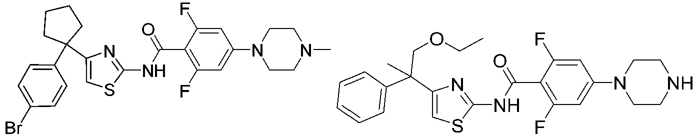

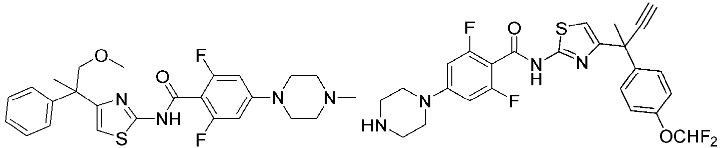

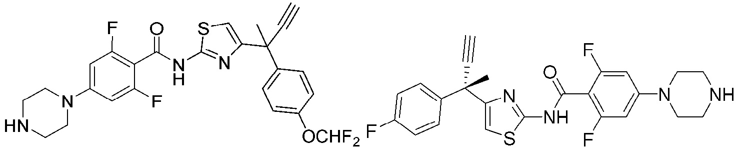

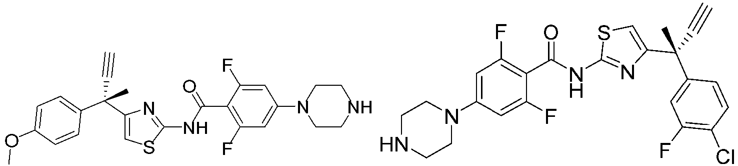

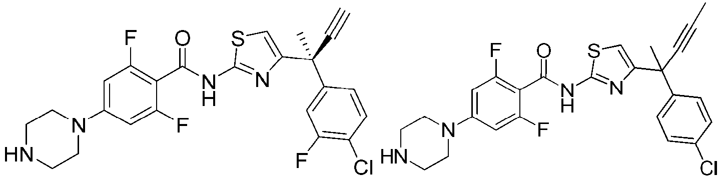

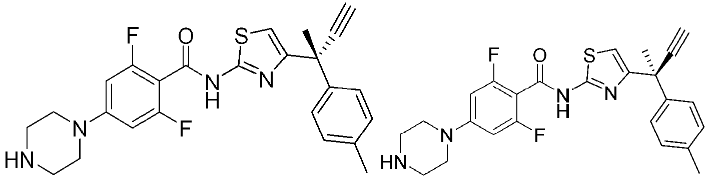

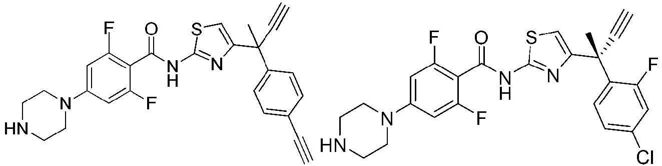

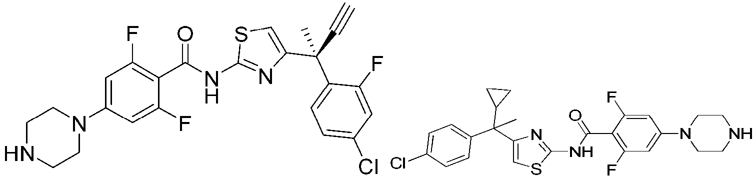

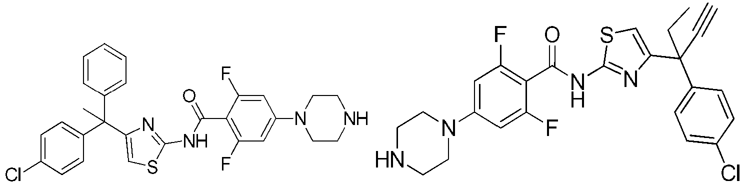

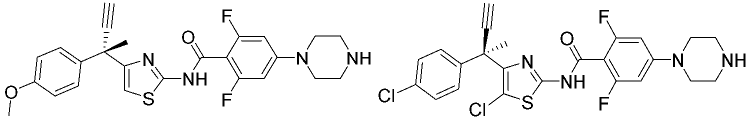

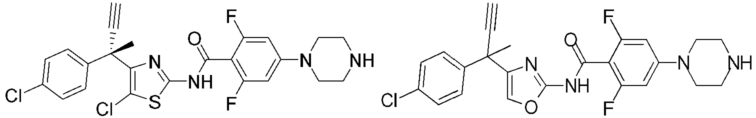

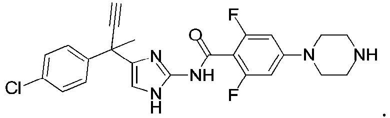

14. Pharmazeutische Zusammensetzung, umfassend eine Verbindung nach einem der Ansprüche 1 bis 13 sowie einen pharmazeutisch akzeptablen Träger oder Hilfsstoff.

15. Verbindung nach einem der Ansprüche 1 bis 13 zur Verwendung in einem Verfahren zur Hemmung der ALPK1-Kinaseaktivität in einer Zelle oder einem Gewebe eines Subjekts, das einer solchen Therapie bedarf, wobei das Verfahren die Verabreichung der Verbindung an das Subjekt umfasst.

16. Verbindung nach einem der Ansprüche 1 bis 13 zur Verwendung in einem Verfahren zur Hemmung oder Verminderung einer Entzündung in einem Zielgewebe eines Subjekts, das einer solchen Behandlung bedarf, wobei das Verfahren die Verabreichung der Verbindung an das Subjekt umfasst.

17. Verbindung nach einem der Ansprüche 1 bis 13 zur Verwendung in einem Verfahren zur Behandlung einer Krankheit, Störung oder eines Zustands, der durch übermäßige oder unangemessene ALPK1-abhängige proinflammatorische Signalgebung gekennzeichnet ist, bei einem Subjekt, das einer solchen Therapie bedarf, wobei das Verfahren die Verabreichung der Verbindung an das Subjekt umfasst,
gegebenenfalls wobei die Krankheit, Störung oder der Zustand ausgewählt ist aus Sepsis, Krebs, Spiroadenom, Spiroadenokarzinom, dem Syndrom "Retinale Dystrophie, Optikusnervenödem, Splenomegalie, Anhidrose und Migränekopfschmerzen" ("ROSAH") und dem Syndrom "Periodisches Fieber, aphthöse Stomatitis, Pharyngitis und Adenitis" ("PFAPA").

18. Verbindung zur Verwendung nach Anspruch 17, wobei das einer solchen Therapie bedürftige Subjekt ein Subjekt ist, das eine oder mehrere genetische Mutationen in ALPK1 trägt.

## Revendications

1. Un composé de formule XI-A-1 ou XI-B 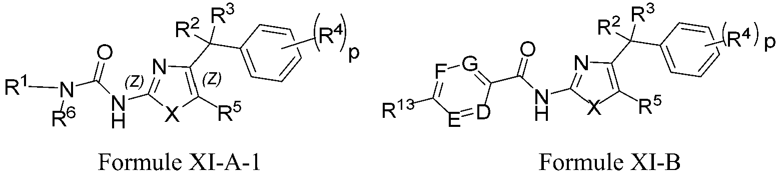
ou un sel pharmaceutiquement acceptable de celui-ci, dans lequel
X est choisi parmi -S-, -O- et -NR^{a}-, dans lequel
R^{a} est H, ou un alkyle en C₁-C₆;
R⁶ est choisi parmi H, D, -OH, un alkyle en C₁-C₆ éventuellement substitué, dans lequel
les fragments R⁶ éventuellement substitués comprennent 0 à 3 substituants choisis indépendamment les uns des autres parmi -D, halogéno et -OH;
R¹ est choisi parmi H, et un alkyle en C₁-C₆ éventuellement substitué;
dans lequel les fragments R¹ éventuellement substitués comprennent 0 à 4 substituants choisis indépendamment les uns des autres parmi -D, halogéno, -OH, -NH₂, =O, -CN, un hydroxyalkyle en C₁-C₆, un alkyle en C₁-C₆ hydroxy-deutéré, un groupe alcoxy en C₁-C₆, -O-X¹-R^{7a}, -C(O)N(R^{7a}R^{8a}), S(O)₂R^{7a}, - S(O)₂N(R^{7a}R^{8a}), -N(R^{7a}R^{8a}), un cycloalkyle saturé en C₃-C₆, un hétérocyclyle saturé ou insaturé à 3 à 7 chaînons contenant 1 à 2 sommets de cycle hétéroatomiques choisis parmi N, O et S, phényle, hétéroaryle à 5 à 10 chaînons contenant 1 à 4 sommets de cycle hétéroatomiques choisis parmi N, O et S; dans lequel
chaque X₁ est indépendamment un alkylène en C₁₋₆;
chaque R^{7a} et R^{8a} sont choisis indépendamment les uns des autres parmi H, un alkyle en C₁-C₆ , un hydroxyalkyle en C₁-C₆, un aminoalkyle en C₁-C₆ , phényle, un hétérocyclyle saturé à 3 à 7 chaînons contenant 1 à 2 sommets de cycle hétéroatomiques choisis parmi N, O et S, dans lequel les groupes phényle et hétérocyclyle à 3 à 7 chaînons sont substitués par 0 à 3 substituants choisis parmi halogéno, -OH et -NH₂, et un alkyle en C₁-C₆; et
les groupes cycloalkyle en C₃-C₆, l'hétérocyclyle à 3 à 7 chaînons, le phényle, et hétéroaryle à 5 à 10 chaînons sont chacun indépendamment substitués par 0 à 3 substituants choisis parmi halogéno, -OH, -NH₂, un alkyle en C₁-C₆, un hydroxyalkyle en C₁-C₆, un halogénoalkyle en C₁-C₆, un groupe alcoxy en C₁-C₆, un hétérocyclyle saturé à 3 à 7 chaînons contenant 1 à 2 sommets de cycle hétéroatomiques choisis parmi N, O et S, -C(O)N(R^{7b}R^{8b}), -S(O)₂N(R^{7b}R^{8b}) et -N(R^{7b}R^{8b}), dans lequel
chaque R^{7b} et R^{8b} sont choisis indépendamment les uns des autres parmi H, un alkyle en C₁-C₆ et un hydroxyalkyle en C₁-C₆; ou
R¹ et R⁶ se combinent pour former un hétérocycloalkyle à 3 à 6 chaînons substitué par 0 à 3 substituants choisis indépendamment les uns des autres parmi le groupe constitué de - OH, -NH₂ et un hydroxyalkyle en C₁-C₆;
R⁵ est choisi parmi H, deutérium, halogéno, un alkyle en C₁-C₆, un deutéroalkyle en C₁-C₄ et un halogénoalkyle en C₁-C₆;
R² et R³ sont chacun indépendamment choisis parmi H, OH, un alkyle en C₁-C₆, un alcynyle en C₂-C₆, un cycloalkyle en C₃-C₆ et phényle, dans lequel l'alkyle en C₁-C₆, l'alcynyle en C₂-C₆, le cycloalkyle en C₃-C₆ et le phényle sont chacun substitués par 0 à 3 substituants choisis indépendamment les uns des autres parmi -OH et un groupe alcoxy en C₁-C₆;
à condition que R₂ et R₃ ne soient pas tous les deux H; ou
R² et R³ se combinent pour former un cycle cycloalkyle en C₃-C₆ ou un hétérocyclyle à 3 à 7 chaînons contenant 1 à 2 sommets de cycle hétéroatomiques indépendamment choisis parmi N, O et S;
chaque R⁴ est indépendamment choisi parmi halogéno, -OH, un alkyle en C₁-C₆, un groupe alcoxy en C₁-C₆, un cycloalkyle saturé en C₃-C₆, O-R^{7e}, dans lequel
R^{7e} est choisi parmi H, un alkyle en C₁-C₆ et un halogénoalkyle en C₁-C₆, et l'indice p vaut 1, 2 ou 3;
D est CR¹⁰ ou N;
E est CR¹⁴ ou N;
F est CR¹² ou N;
G est CR¹¹ ou N;
à condition que pas plus de trois de D, E, F et G soient N;
R¹⁰, R¹¹, R¹² et R¹⁴, lorsqu'ils sont présents, sont chacun indépendamment choisis parmi H, halogéno, -OH, un groupe alcoxy en C₁-C₆, -O-X¹-R^{7a}, -C(O)N(R^{7a}R^{8a}) et un hétérocyclyle saturé ou insaturé à 3 à 7 chaînons contenant 1 à 2 sommets de cycle hétéroatomiques choisis parmi N, O et S; dans lequel
chaque X¹ est indépendamment un alkylène en C₁₋₆;
chaque R^{7a} et R^{8a} sont choisis indépendamment les uns des autres parmi H, un alkyle en C₁-C₆ et un groupe alcoxy en C₁-C₆;
R¹³ est H, un hétérocyclyle saturé ou insaturé à 3 à 7 chaînons contenant 1 à 2 sommets de cycle hétéroatomiques choisis parmi N, O et S, hétérocyclyle ponté saturé ou insaturé à 7 à 8 chaînons contenant 1 à 2 sommets de cycle hétéroatomiques choisis parmi N, O et S, dans lequel
l'hétérocyclyle à 3 à 7 chaînons et l'hétérocyclyle ponté à 7 à 8 chaînons sont éventuellement substitués par 0 à 2 substituants choisis indépendamment les uns des autres parmi -OH, un alkyle en C₁-C₆, un aminoalkyle en C₁-C₆, -NH₂ et un hydroxyalkyle en C₁-C₆.

2. Le composé selon la revendication 1, dans lequel X est -S-, -O- ou -NH-,

3. Le composé selon la revendication 1, présentant : Formule XI-A-1-a 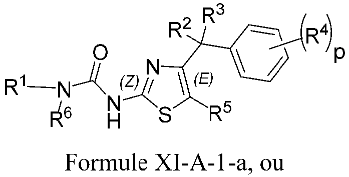 un sel pharmaceutiquement acceptable de celui-ci, ou, R⁶ est choisi parmi H, un alkyle en C₁-C₆, un hydroxyalkyle en C₁-C₆ et un alkyle en C₁-C₆ hydroxy-deutéré.

4. Le composé selon l'une quelconque des revendications 1 à 3, dans lequel:
a) R¹ se combine avec R⁶ pour former un hétérocycloalkyle à 3 à 6 chaînons substitué par 0 à 3 substituants choisis indépendamment les uns des autres parmi le groupe constitué de -OH, - NH₂ et un hydroxyalkyle en C₁-C₆,
b) R¹ est un groupe alkyle en C₁-C₆ substitué par 0 à 4 substituants choisis indépendamment les uns des autres parmi -OH, un hydroxyalkyle en C₁-C₆, un groupe alcoxy en C₁-C₆, - S(O)2N(R^{7a}R^{8a}) et -N(R^{7a}R^{8a}), dans lequel
chaque R^{7a} et R^{8a} sont choisis indépendamment les uns des autres parmi H, un alkyle en C₁-C₆ et un aminoalkyle en C₁-C₆,
ou
c) R¹ est un groupe alkyle en C₁-C₆ substitué par 0 à 2 substituants choisis indépendamment les uns des autres parmi -OH, un hydroxyalkyle en C₁-C₆ et -S(O)₂N(R^{7a}R^{8a}), dans lequel chaque R^{7a} et R^{8a} sont choisis indépendamment les uns des autres parmi H et un alkyle en C₁-C₆.

5. Le composé selon la revendication 1, dans lequel:
a) D, E, F et G sont respectivement CR¹⁰, CR¹⁴, CR¹² et CR¹¹, ou
b) F et G sont respectivement CR¹⁴ et CR¹¹, E est N ou CR¹⁴ et D est N ou CR¹⁰.

6. Le composé selon la revendication 1 ou 5, dans lequel:
a) R¹⁰ et R¹¹ sont chacun H;
R¹² et R¹⁴ sont choisis indépendamment les uns des autres parmi halogéno, -OH, un groupe alcoxy en C₁-C₆ et -C(O)N(R^{7b}R^{8b}), dans lequel
R^{7b} et R^{8b} sont choisis indépendamment les uns des autres parmi H, un alkyle en C₁-C₆ et un groupe alcoxy en C₁-C₆; et
R¹³ est choisi parmi un hétérocyclyle à 3 à 7 chaînons contenant 1 à 2 sommets de cycle hétéroatomiques choisis parmi N, O et S, et un hétérocyclyle ponté saturé ou insaturé à 7 à 8 chaînons contenant 1 à 2 sommets de cycle hétéroatomiques choisis parmi N, O et S, dans lequel
l'hétérocyclyle à 3 à 7 chaînons, et l'hétérocyclyle ponté à 7 à 8 chaînons, sont éventuellement substitués par 0 à 2 substituants choisis indépendamment les uns des autres parmi -OH, un alkyle en C₁-C₆, un hydroxyalkyle en C₁-C₆ et un aminoalkyle en C₁-C₆,
b) R¹² et R¹⁴ sont chacun H;
R¹⁰ et R¹¹ sont choisis indépendamment les uns des autres parmi halogéno, -OH, un groupe alcoxy en C₁-C₆ et -C(O)N(R^{7b}R^{8b}), dans lequel
R^{7b} et R^{8b} sont choisis indépendamment les uns des autres parmi H, un alkyle en C₁-C₆ et un groupe alcoxy en C₁-C₆; et
R¹³ est choisi parmi hétérocyclyle à 3 à 7 chaînons contenant 1 à 2 sommets de cycle hétéroatomiques choisis parmi N, O et S, et hétérocyclyle ponté saturé ou insaturé à 7 à 8 chaînons contenant 1 à 2 sommets de cycle hétéroatomiques choisis parmi N, O et S, dans lequel
l'hétérocyclyle à 3 à 7 chaînons, et l'hétérocyclyle ponté à 7 à 8 chaînons, sont éventuellement substitués par 0 à 2 substituants choisis indépendamment les uns des autres parmi -OH, un alkyle en C₁-C₆, un hydroxyalkyle en C₁-C₆ et un aminoalkyle en C₁-C₆,
c) R¹⁰, R¹¹, R¹² et R¹⁴, lorsqu'ils sont présents, sont chacun H; et
R¹³ est choisi parmi un cycloalkyle saturé ou insaturé en C₃-C₆, hétérocyclyle à 3 à 7 chaînons contenant 1 à 2 sommets de cycle hétéroatomiques choisis parmi N, O et S, et hétérocyclyle ponté saturé ou insaturé à 7 à 8 chaînons contenant 1 à 2 sommets de cycle hétéroatomiques choisis parmi N, O et S, dans lequel
l'hétérocyclyle à 3 à 7 chaînons, et l'hétérocyclyle ponté à 7 à 8 chaînons, sont éventuellement substitués par 0 à 2 substituants choisis indépendamment les uns des autres parmi -OH, un alkyle en C₁-C₆, un hydroxyalkyle en C₁-C₆ et un aminoalkyle en C₁-C₆,
d) R¹⁰, R¹¹, R¹² et R¹⁴, lorsqu'ils sont présents, sont chacun H; et
R¹³ est un hétérocyclyle à 3 à 7 chaînons contenant 1 à 2 sommets de cycle hétéroatomiques choisis parmi N, O et S substitué par 0 à 2 substituants indépendamment choisis parmi -OH, un alkyle en C₁-C₆, un hydroxyalkyle en C₁-C₆ et un aminoalkyle en C₁-C₆,
ou
e) R¹⁰, R¹¹, R¹² et R¹⁴, lorsqu'ils sont présents, sont chacun H; et
R¹³ est un hétérocyclyle ponté saturé ou insaturé à 7 à 8 chaînons éventuellement substitué contenant 1 à 2 sommets de cycle hétéroatomiques choisis parmi N, O et S substitué par 0 à 2 substituants choisis parmi -OH et alkyle en C₁-C₆.

7. Le composé selon la revendication 1, de formule XI-B-1 ou XI-B-2 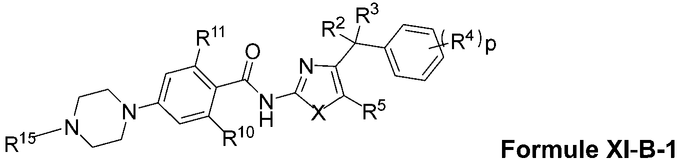 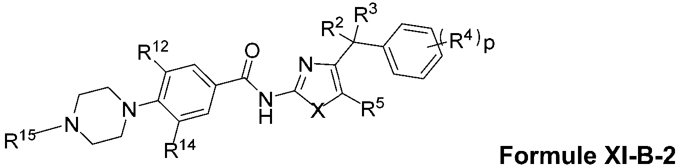
ou un sel pharmaceutiquement acceptable de celui-ci, dans lequel
R¹⁵ est choisi parmi -OH, un alkyle en C₁-C₆, un hydroxyalkyle en C₁-C₆ et un aminoalkyle en C₁-C₆,
éventuellement dans lequel R¹⁰, R¹¹, R¹² et R¹⁴,sont choisis indépendamment les uns des autres parmi halogéno et un alkyle en C₁-C₆.

8. Le composé selon la revendication 7, dans lequel:
a) R¹⁵ est choisi parmi un alkyle en C₁-C₆,
b) X est -S-, présentant la formule XI-B-1-a ou la formule XI-B-2-a

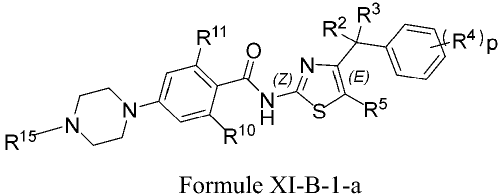

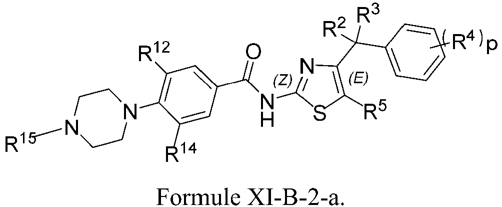

9. Le composé selon la revendication 7 ou 8, présentant:
a) Formule XI-B-1-a-I ou Formule XI-B-2-a-I 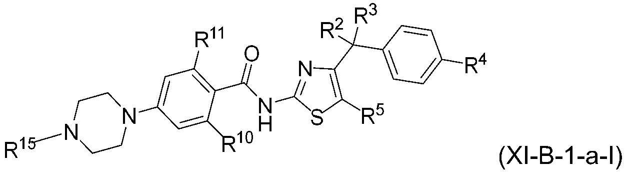 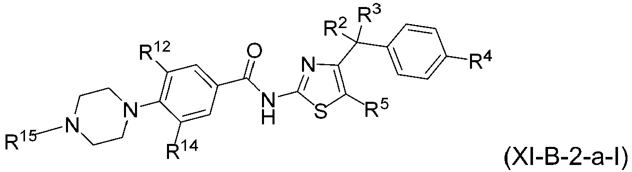 ou un sel pharmaceutiquement acceptable de celui-ci, dans lequel R⁴ est halogéno,
b) Formule XI-B-1-a-II ou Formule XI-B-2-a-II 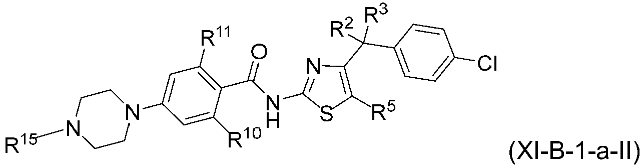 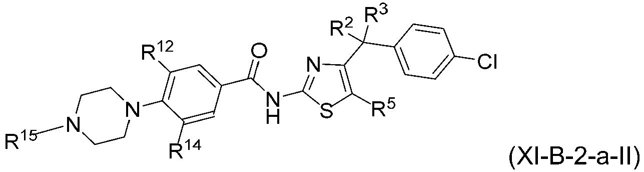 ou un sel pharmaceutiquement acceptable de celui-ci,
c) Formule XI-B-1-a-III ou Formule XI-B-2-a-III 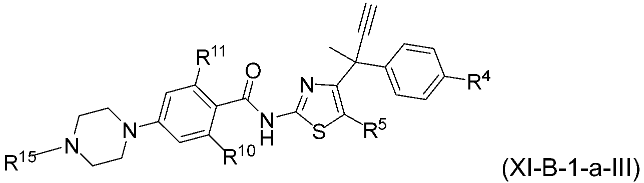 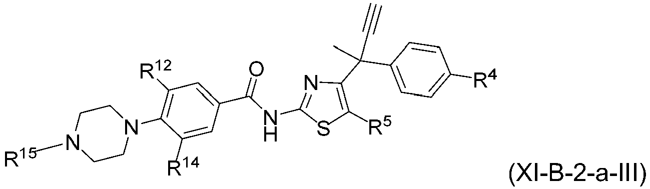 ou un sel pharmaceutiquement acceptable de celui-ci,
ou
d) Formule XI-B-1-a-IV, ou Formule XI-B-2-a-IV 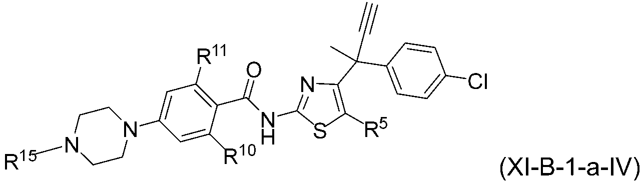 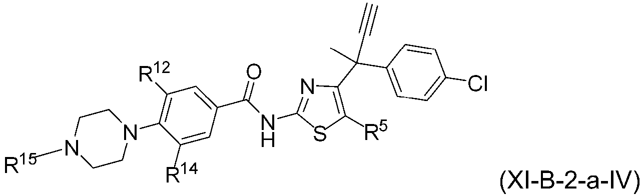 ou un sel pharmaceutiquement acceptable de celui-ci.

10. Le composé selon l'une quelconque des revendications 1 à 9, dans lequel l'indice p vaut 1 et R⁴ est:
a) attaché au cycle phényle comme indiqué ci-dessous: 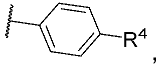 dans lequel la ligne ondulée représente le point de rattachement au reste de la formule,
b) halogéno attaché au cycle phényle comme indiqué ci-dessous: 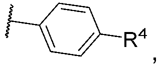 dans lequel la ligne ondulée représente le point de rattachement au reste de la formule,
c) chloro attaché au cycle phényle comme indiqué ci-dessous: 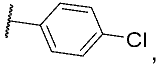 dans lequel la ligne ondulée représente le point de rattachement au reste de la formule,
ou
d) méthoxy attaché au cycle phényle comme indiqué ci-dessous: 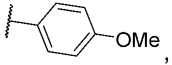 dans lequel la ligne ondulée représente le point de rattachement au reste de la formule.

11. Le composé selon l'une quelconque des revendications 1 à 10, dans lequel l'atome de carbone attaché à R² et R³ est: a) l'isomère S, ou b) l'isomère R.

12. Le composé selon la revendication 1, dans lequel le composé de formule XI-A-1 ou XI-B est choisi parmi 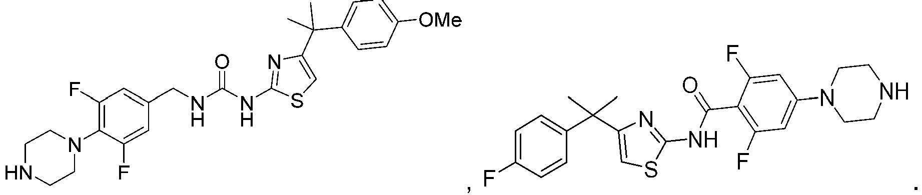 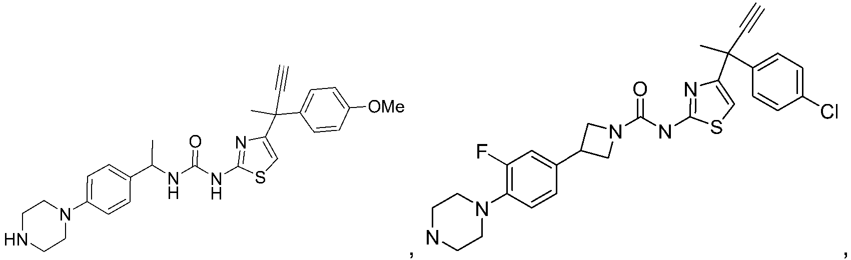 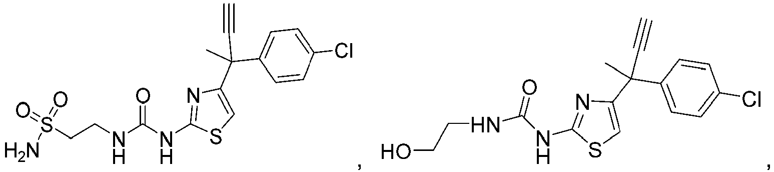 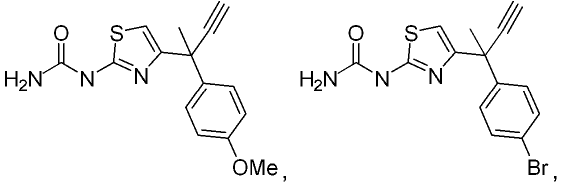 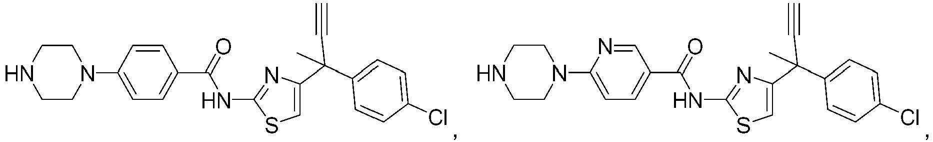 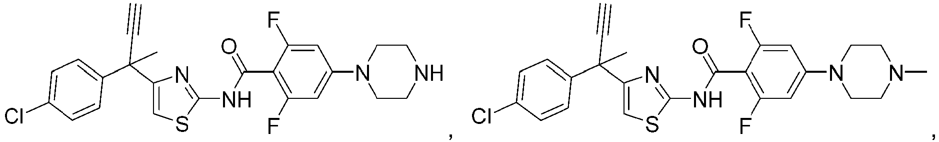 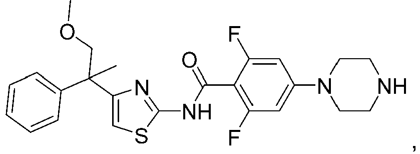 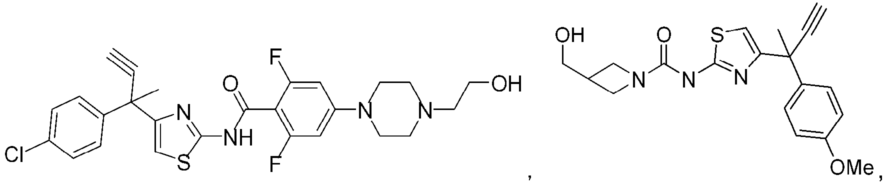 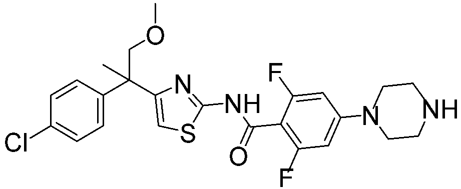 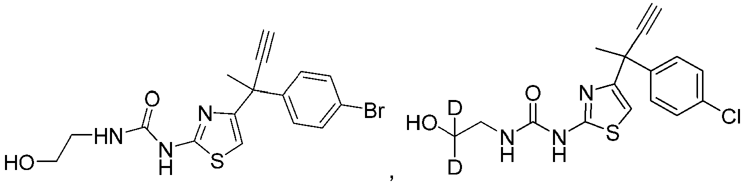 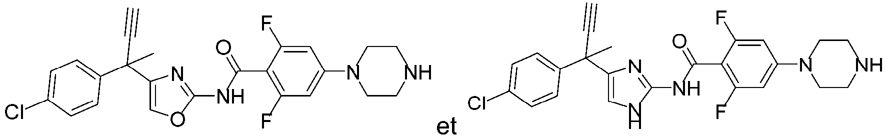 ou
dans lequel le composé de formule XI est choisi parmi

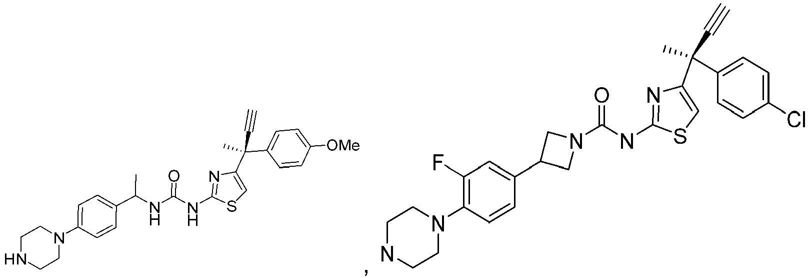

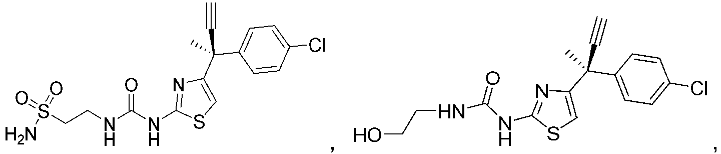

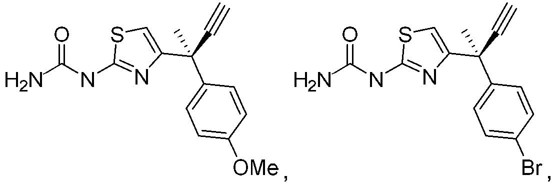

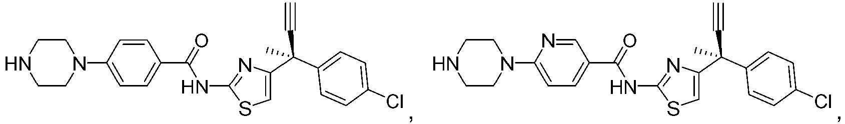

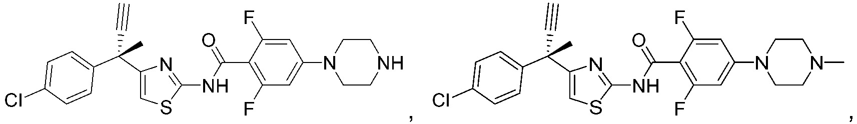

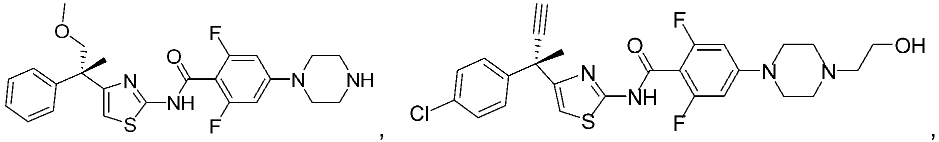

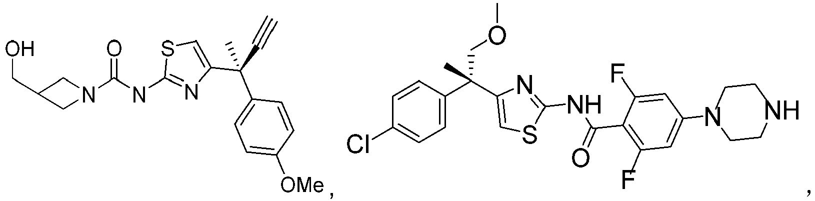

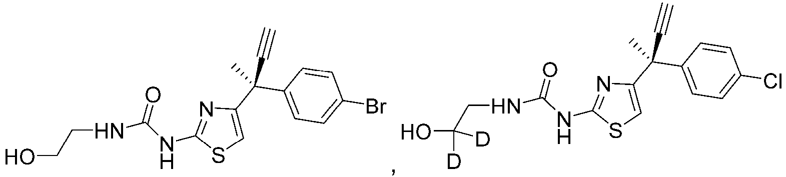

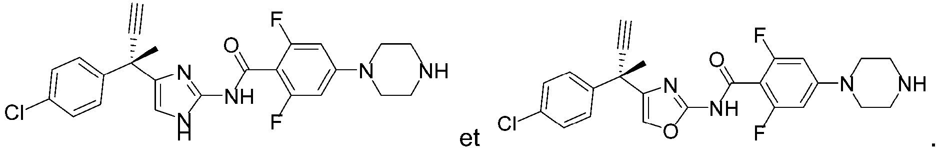

13. Le composé selon la revendication 1, choisi parmi l'un quelconque des composés suivants:

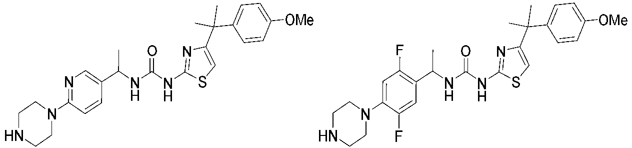

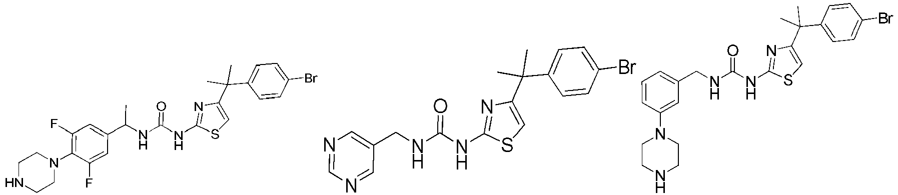

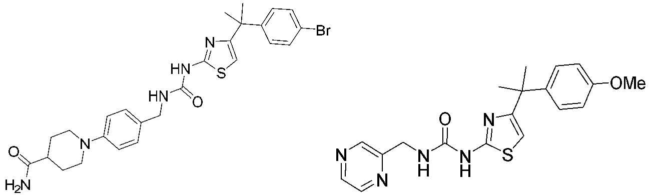

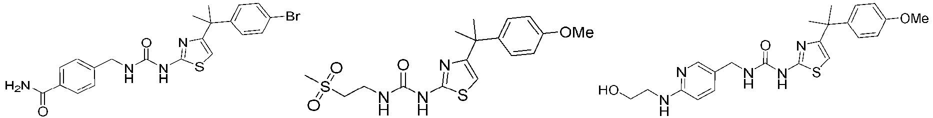

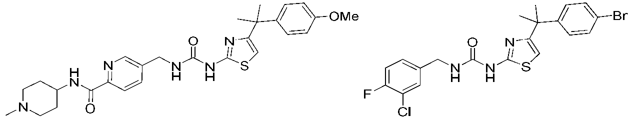

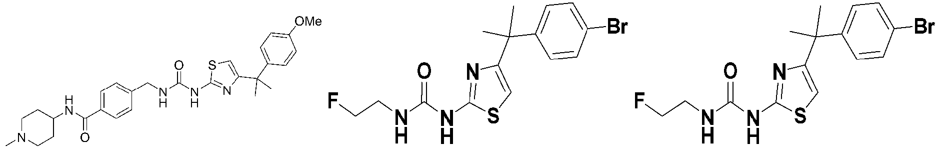

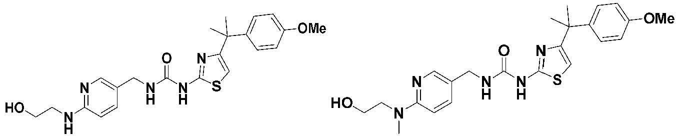

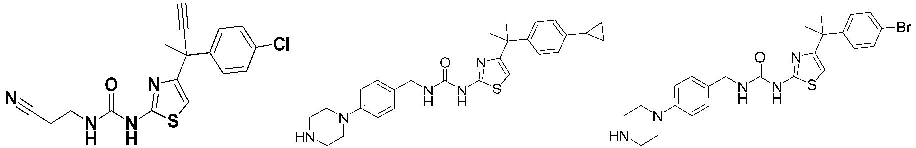

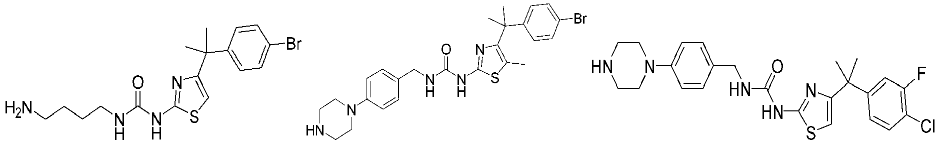

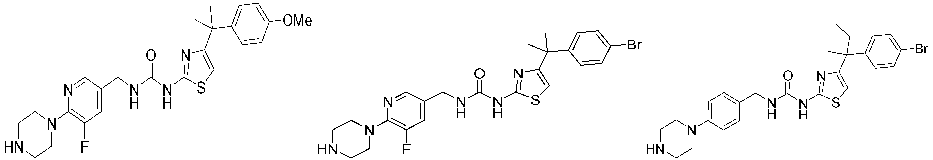

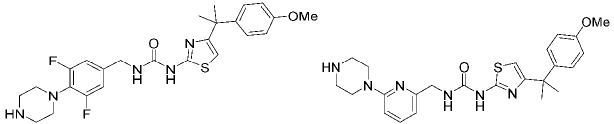

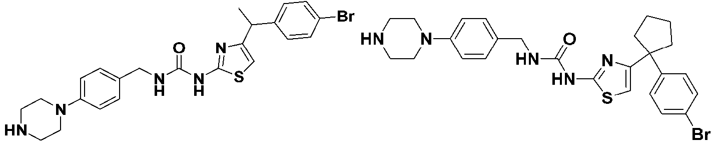

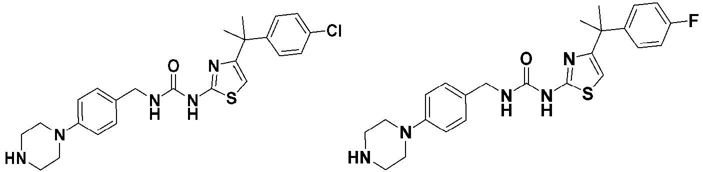

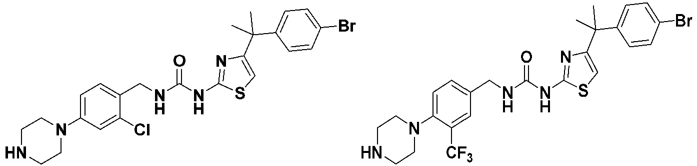

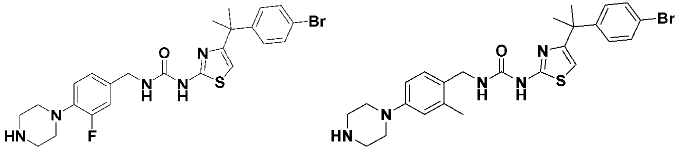

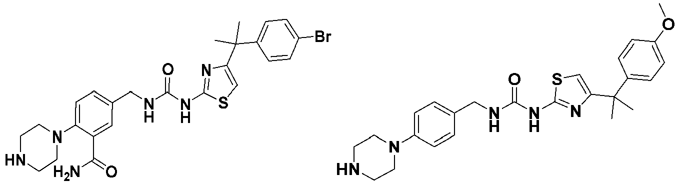

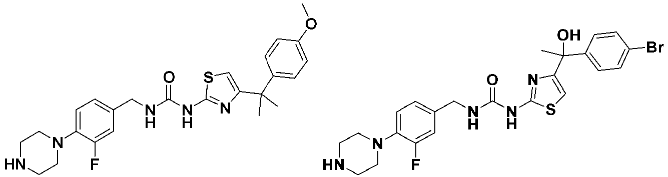

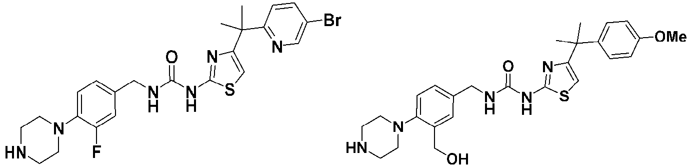

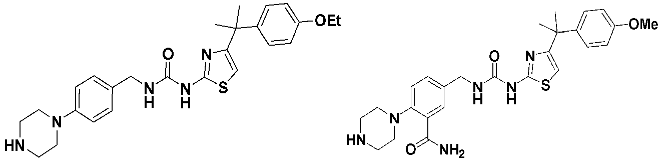

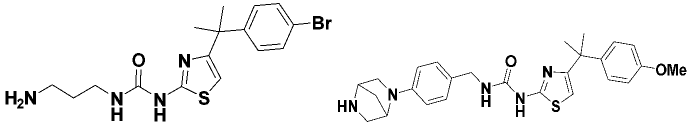

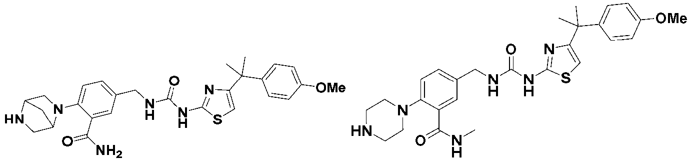

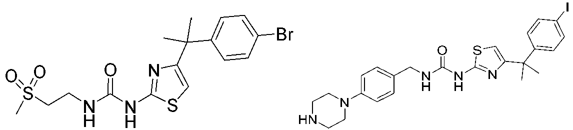

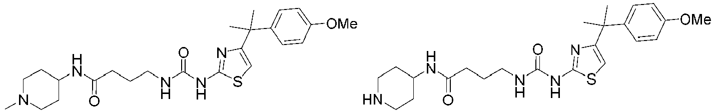

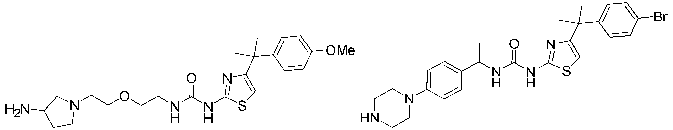

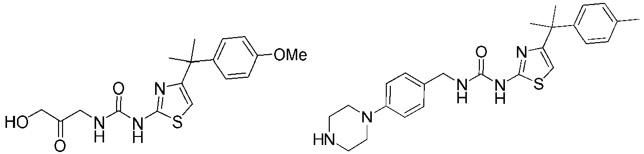

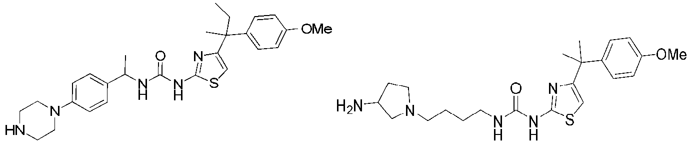

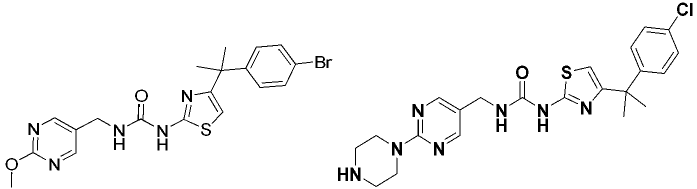

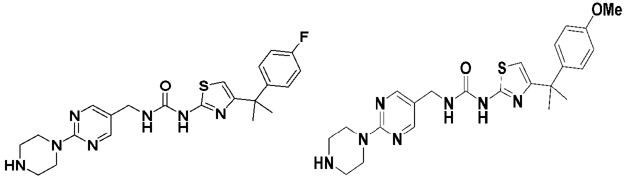

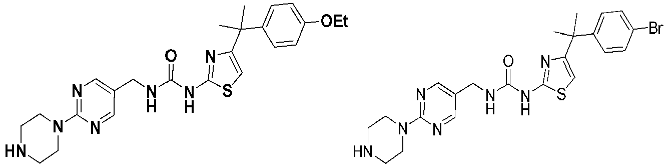

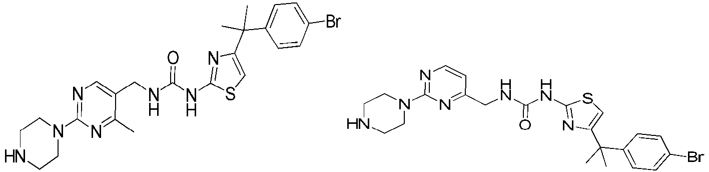

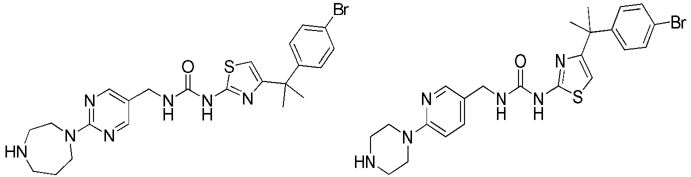

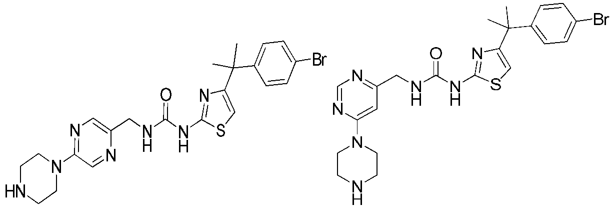

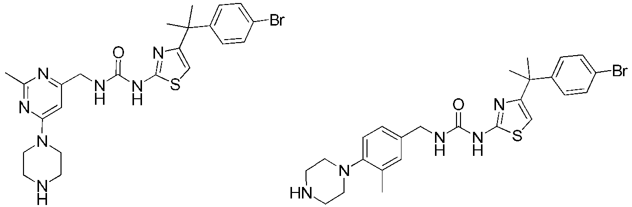

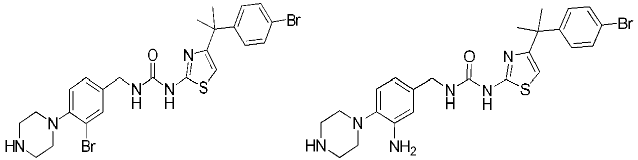

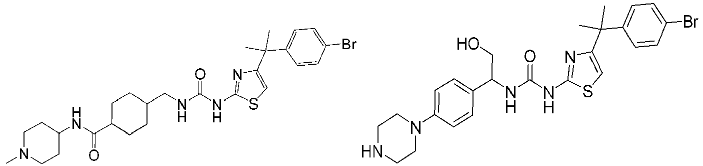

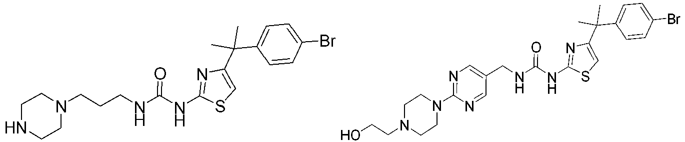

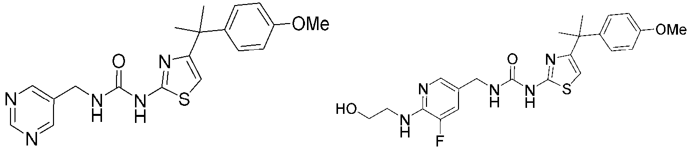

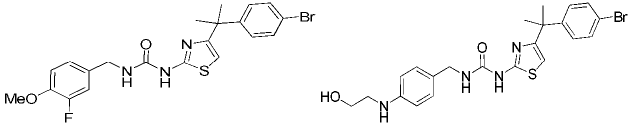

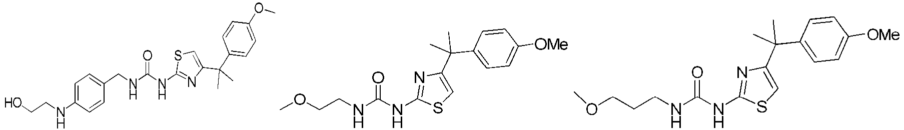

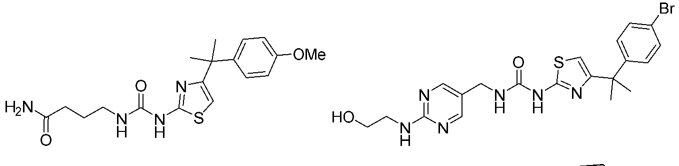

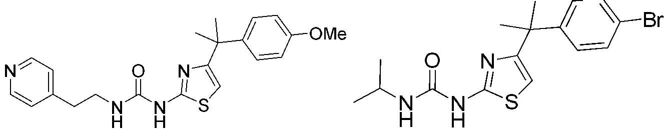

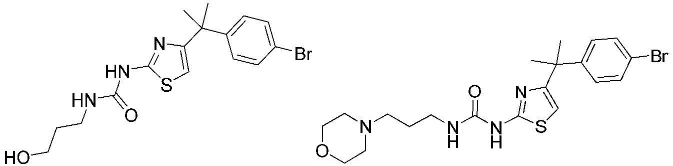

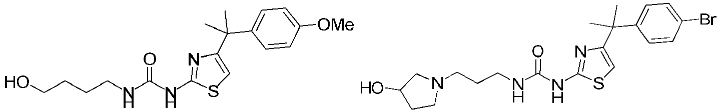

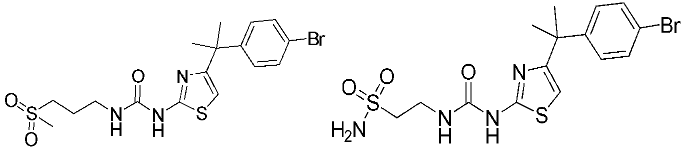

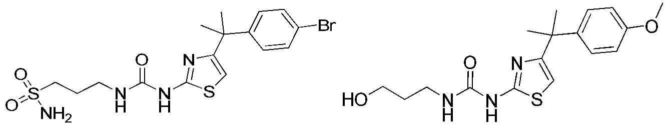

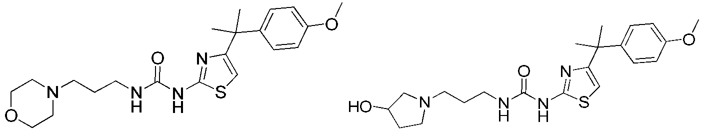

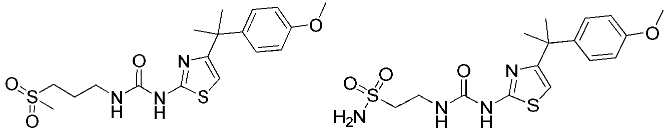

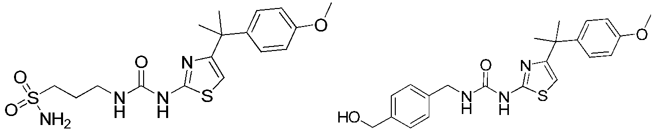

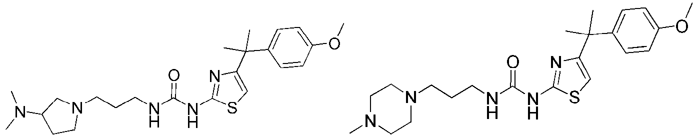

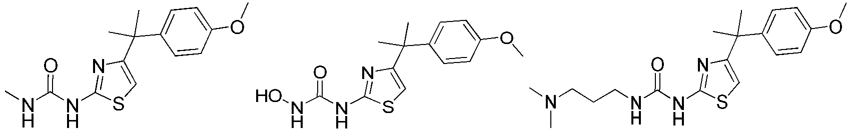

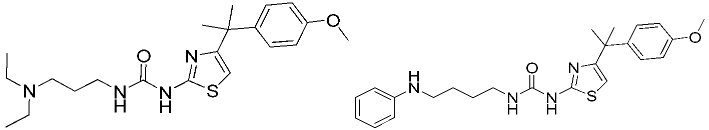

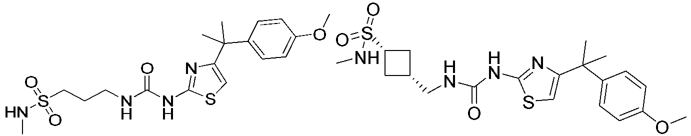

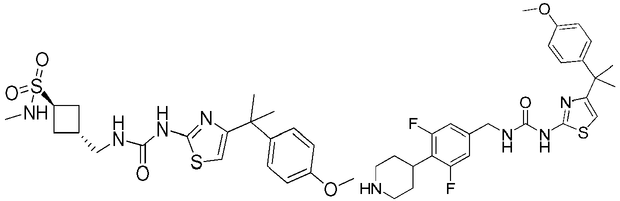

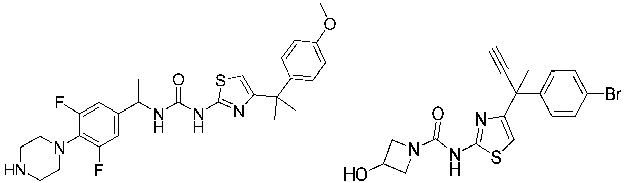

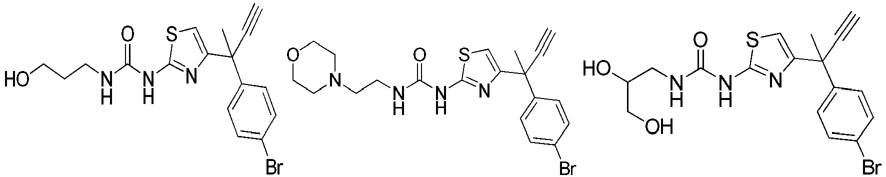

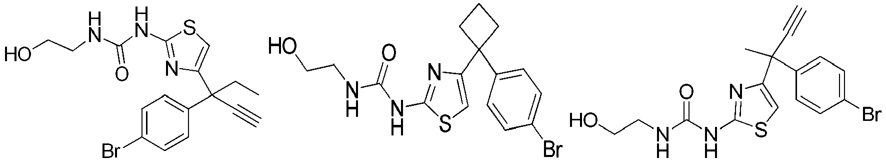

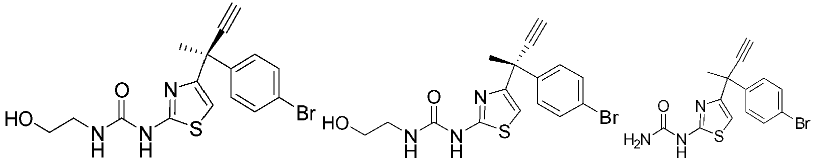

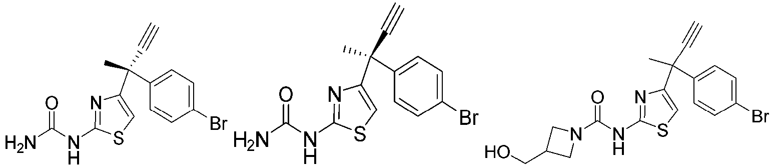

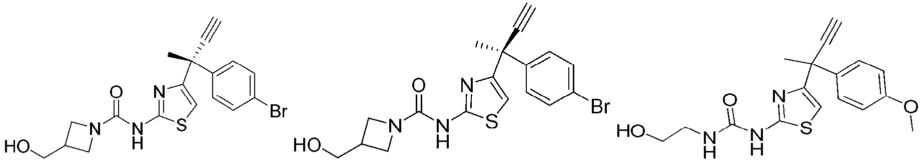

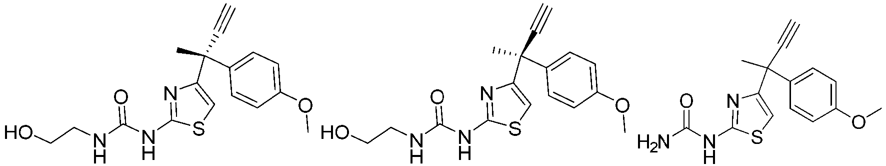

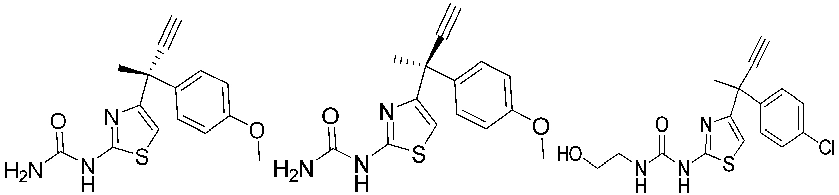

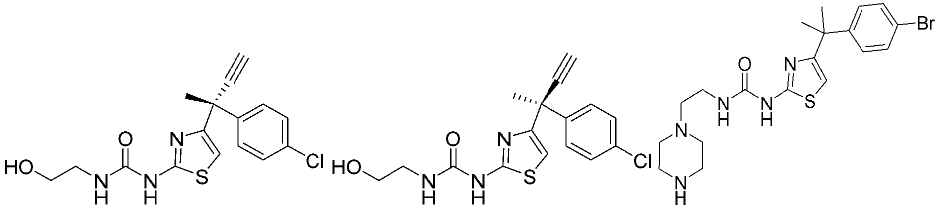

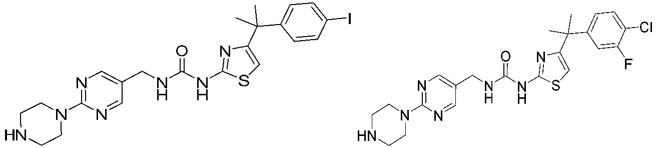

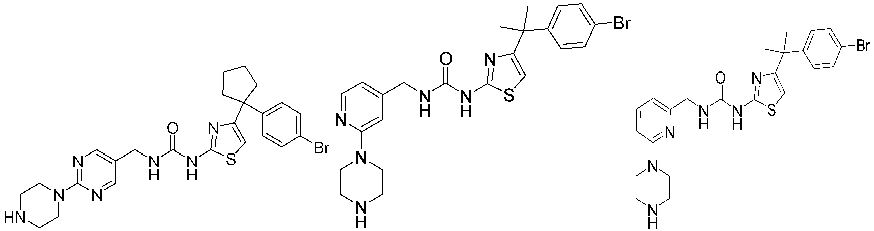

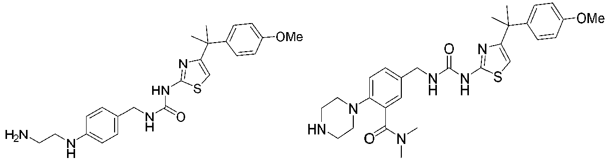

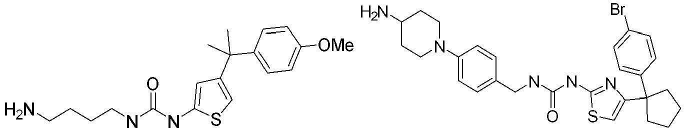

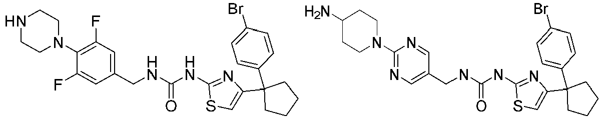

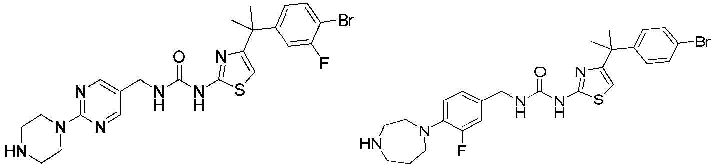

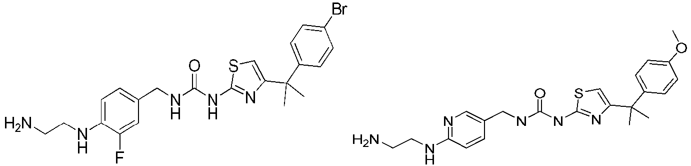

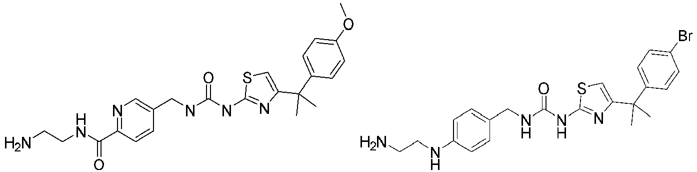

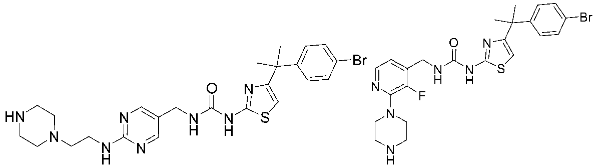

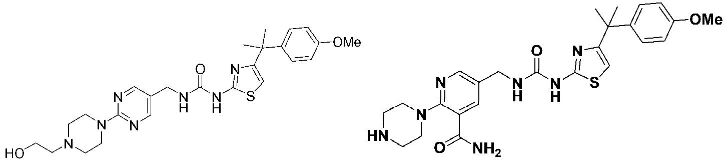

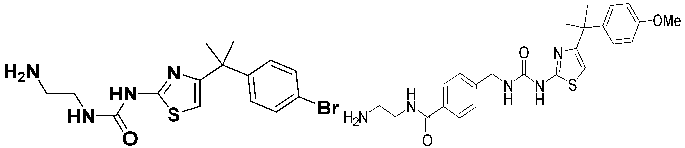

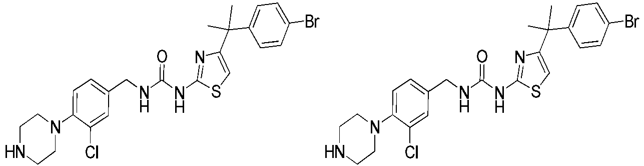

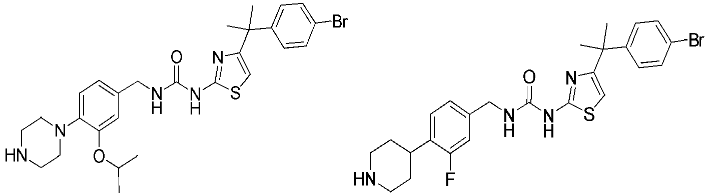

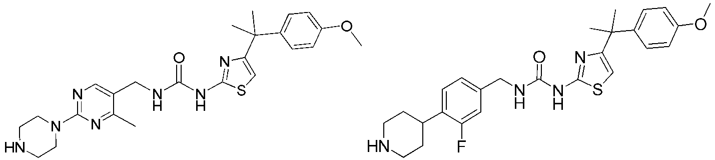

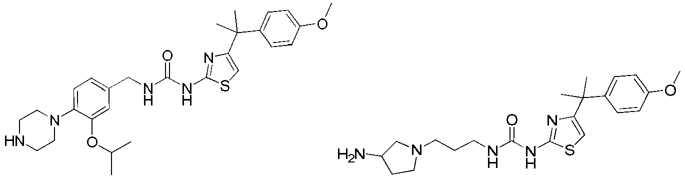

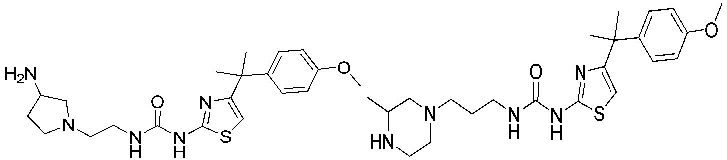

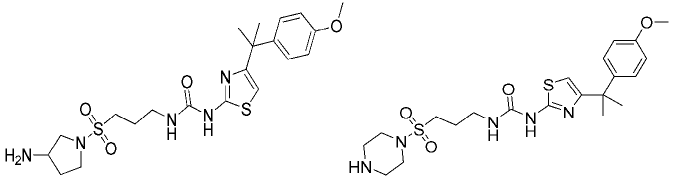

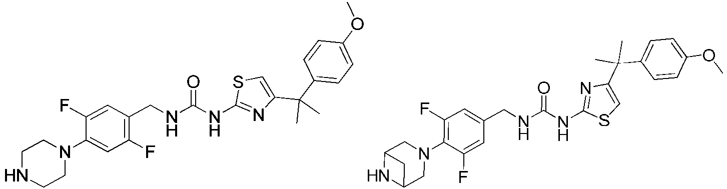

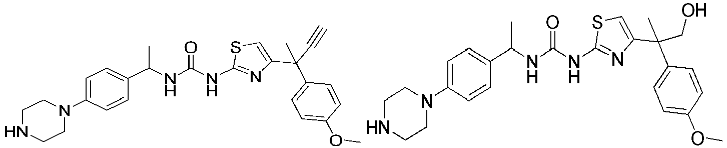

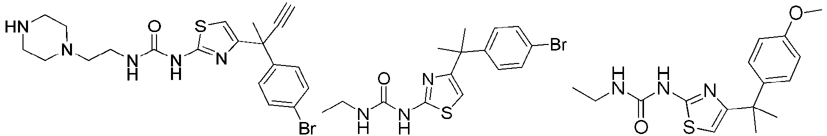

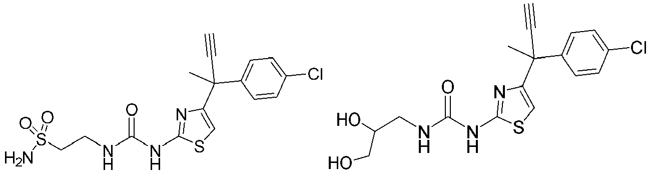

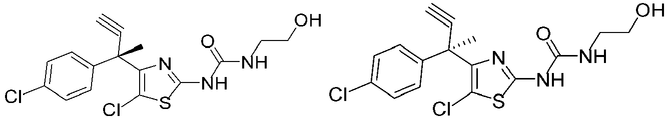

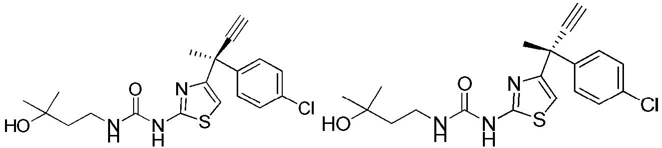

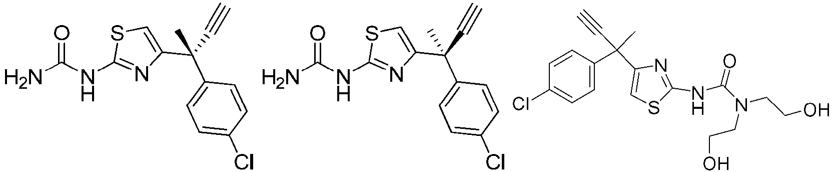

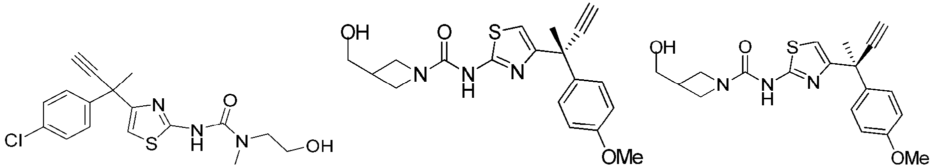

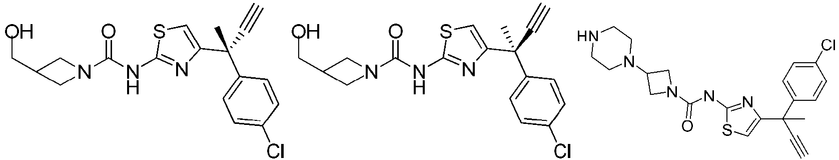

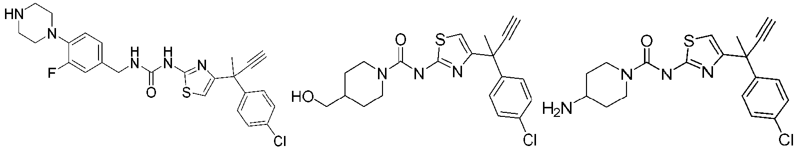

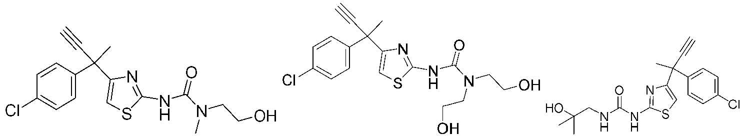

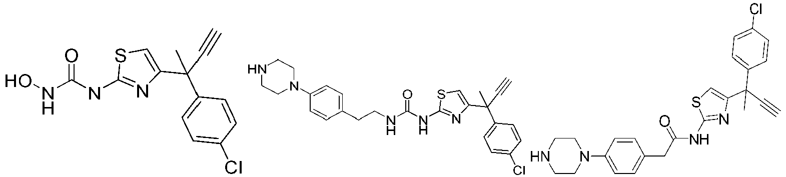

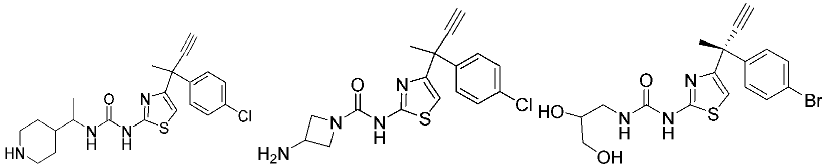

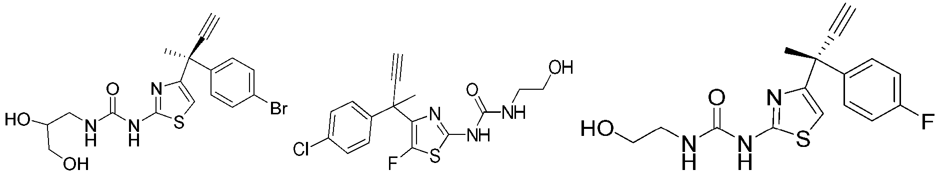

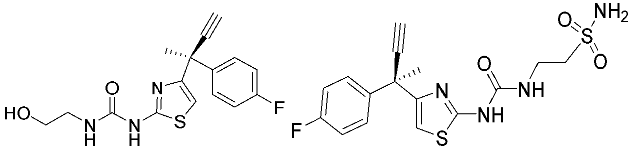

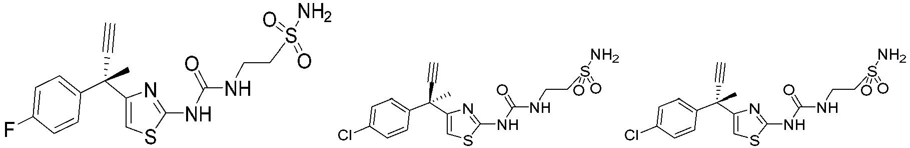

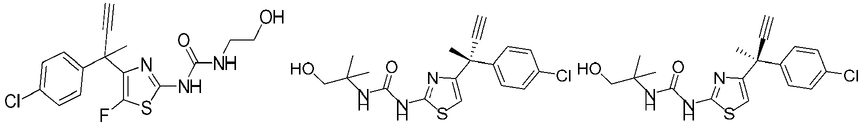

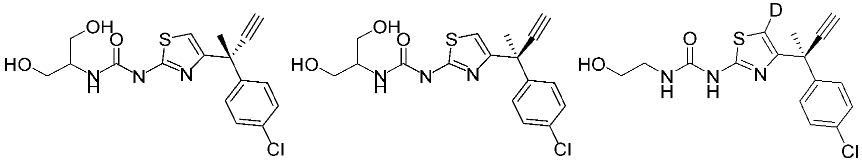

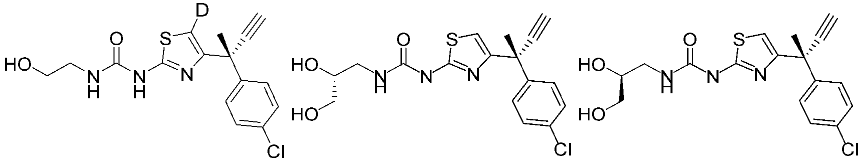

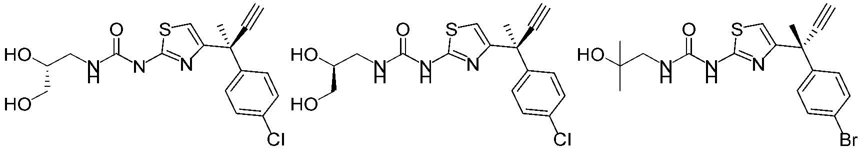

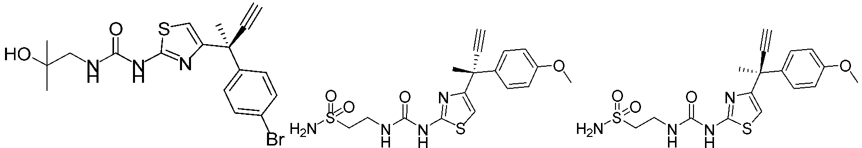

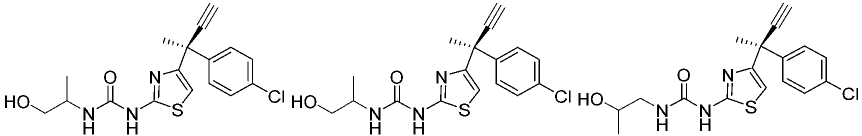

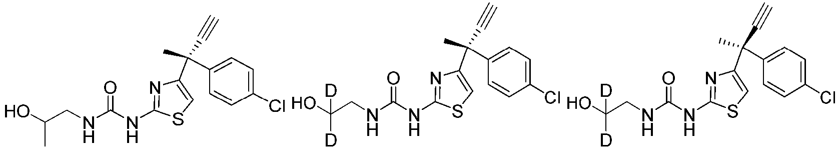

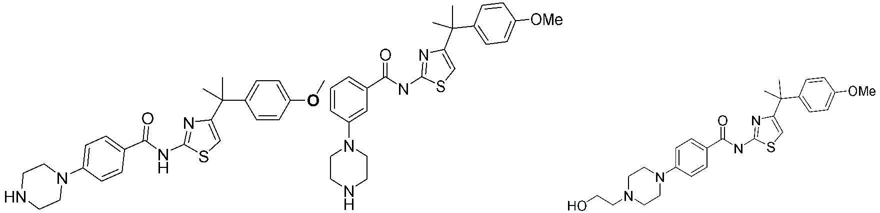

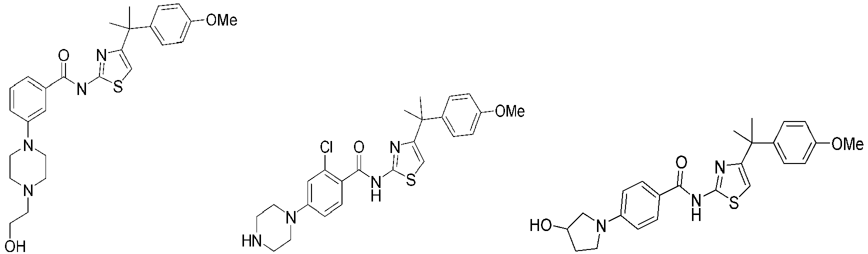

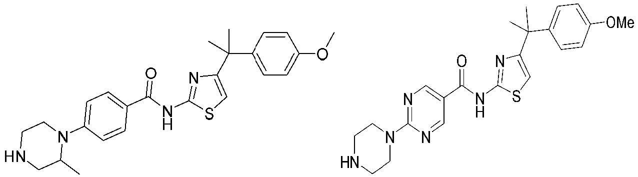

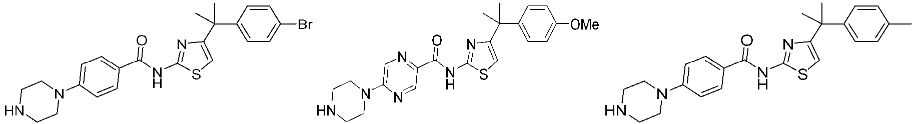

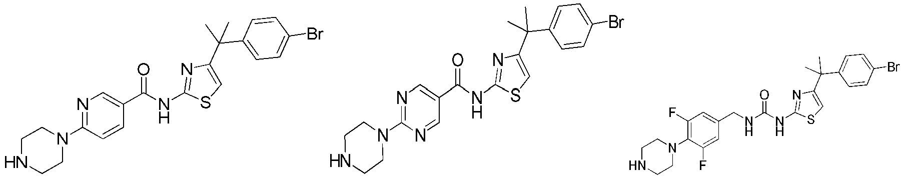

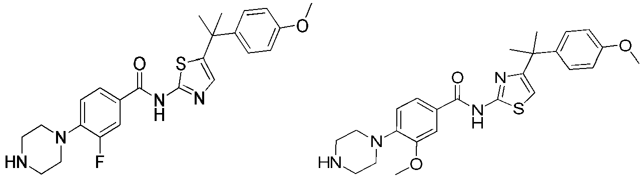

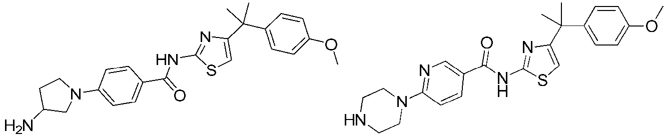

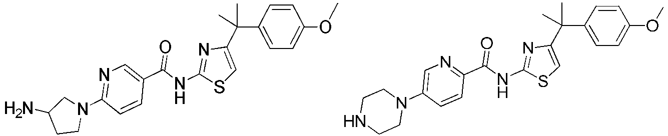

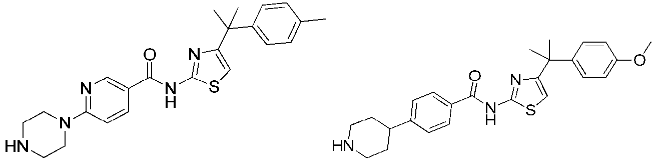

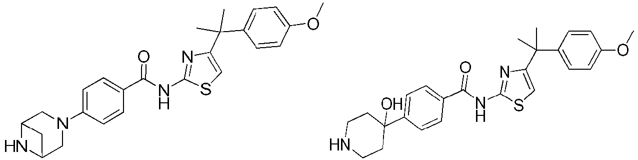

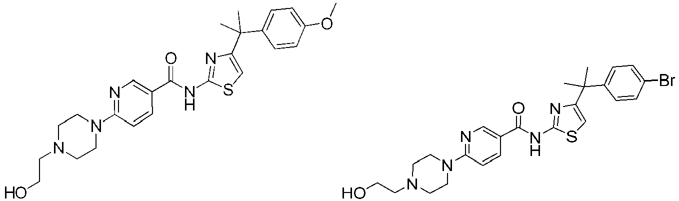

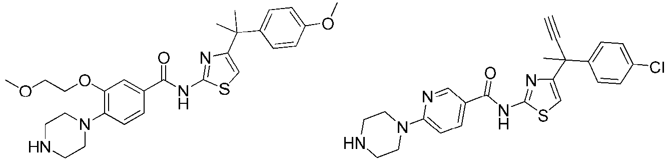

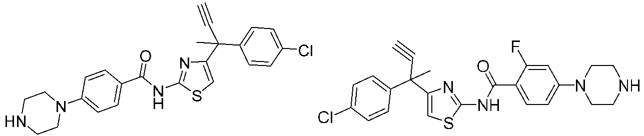

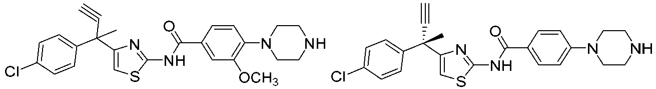

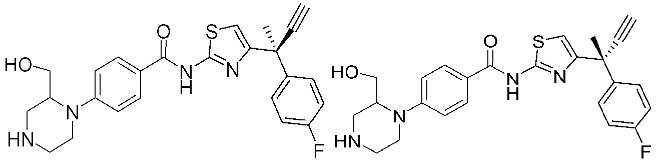

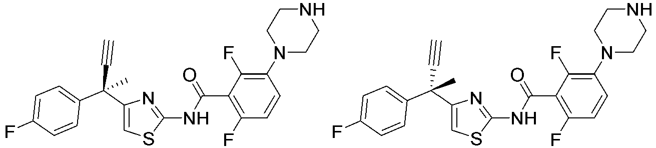

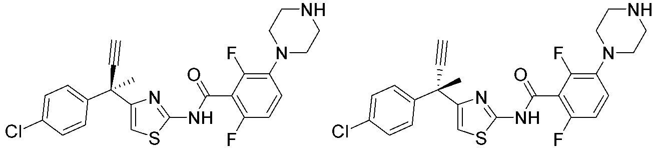

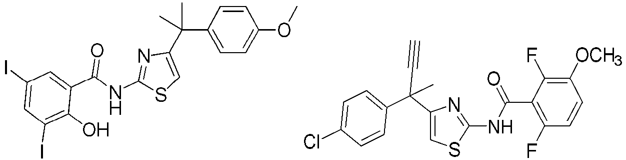

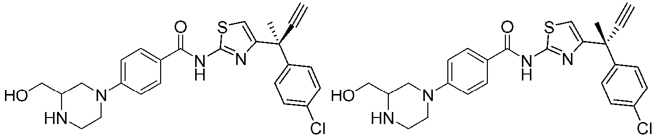

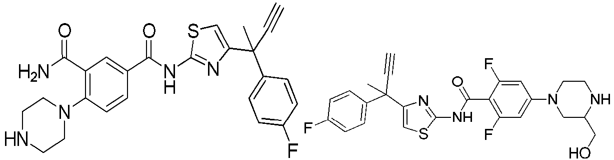

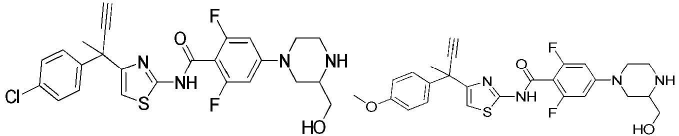

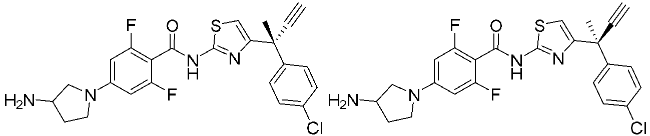

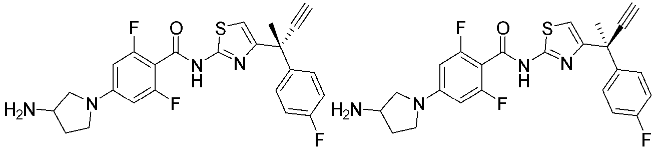

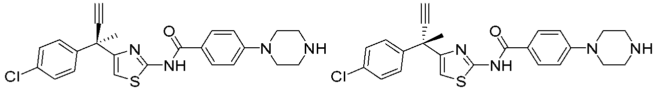

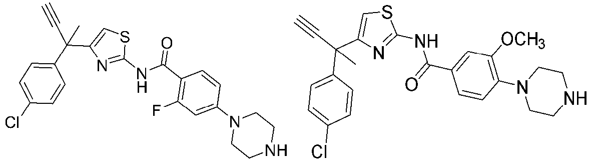

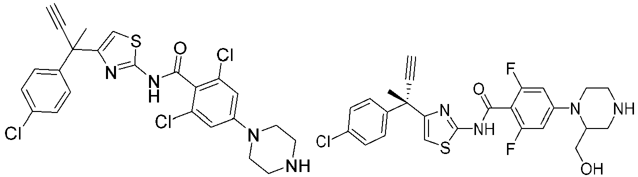

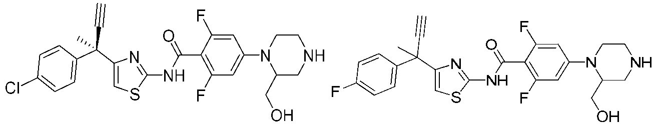

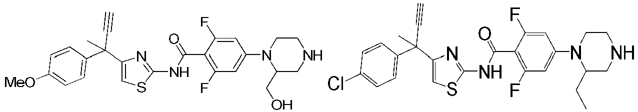

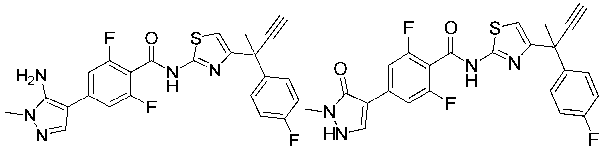

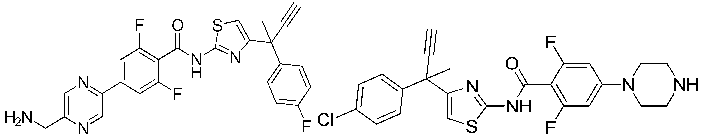

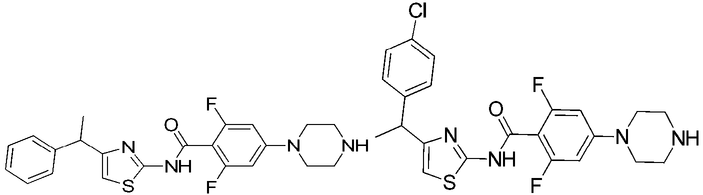

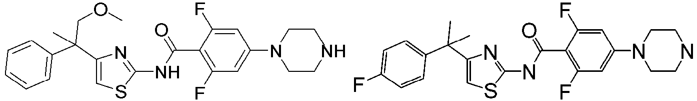

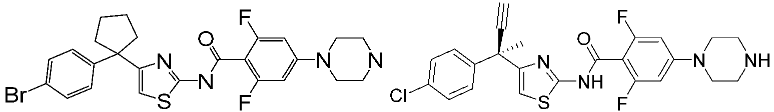

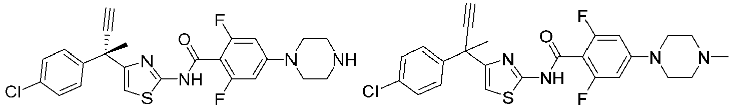

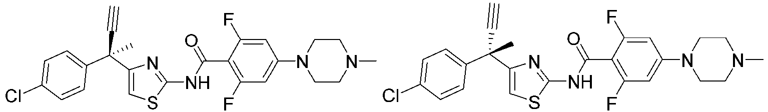

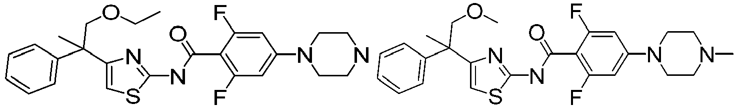

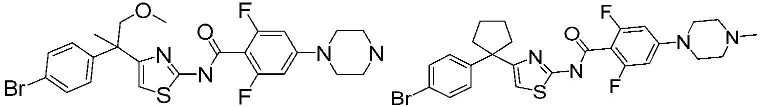

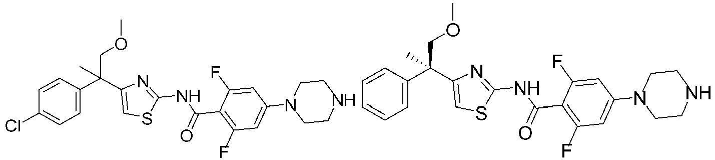

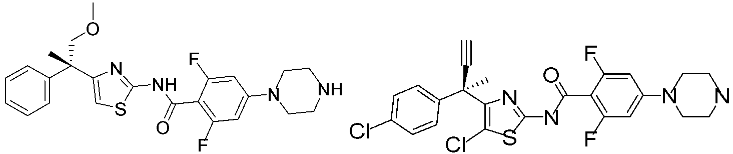

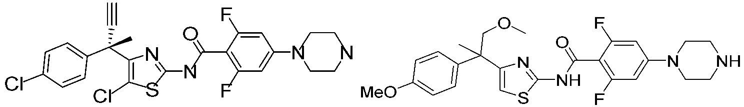

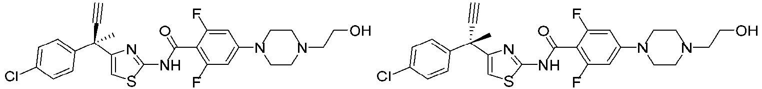

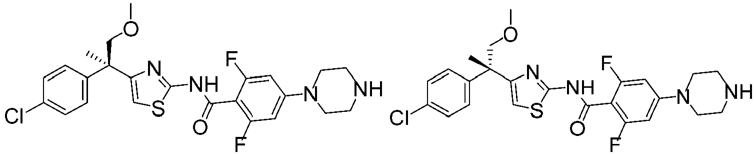

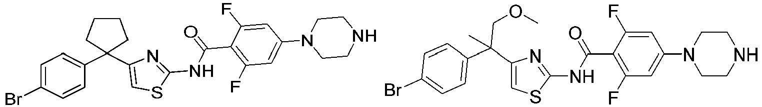

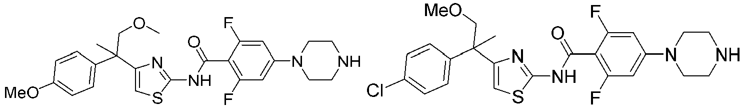

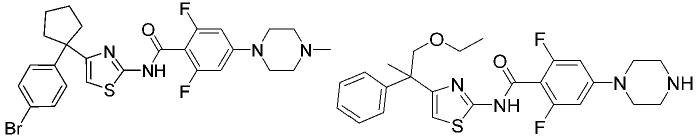

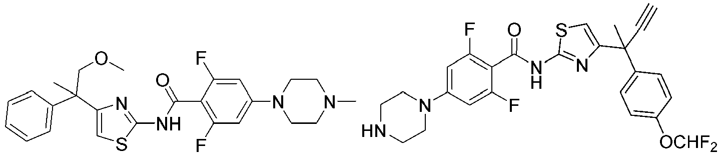

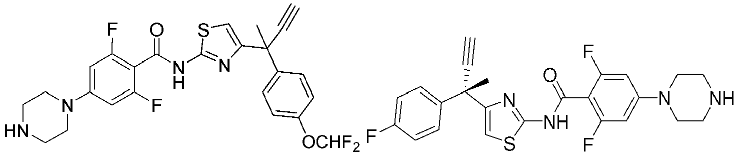

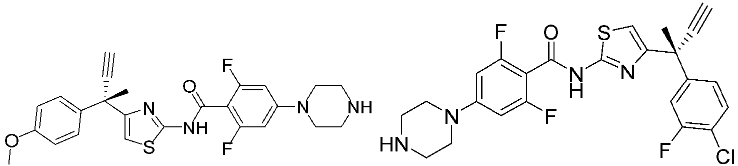

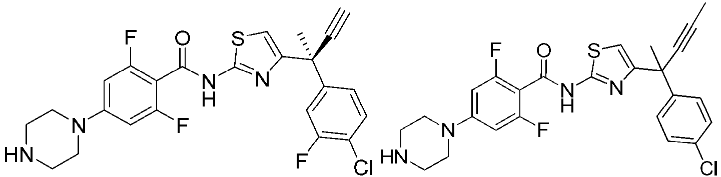

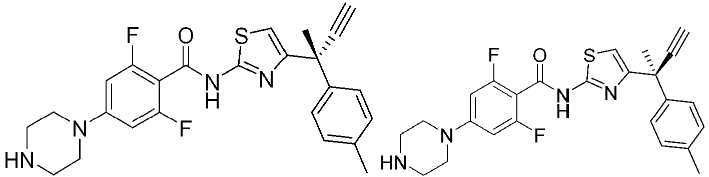

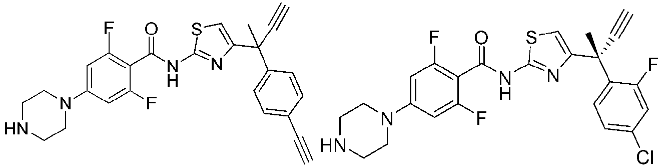

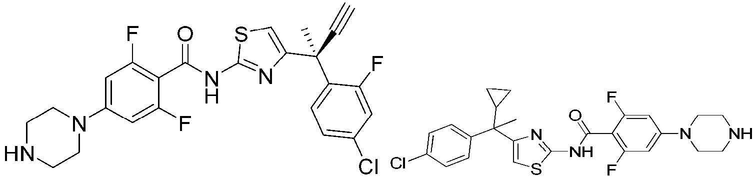

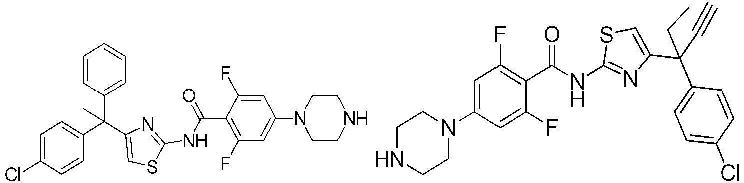

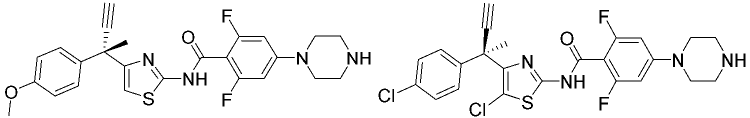

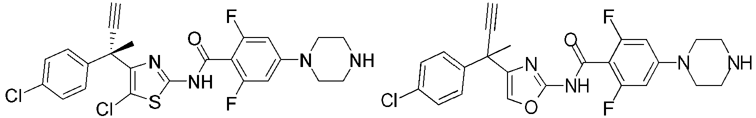

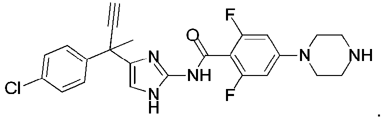

14. Une composition pharmaceutique comprenant un composé selon l'une quelconque des revendications 1 à 13, et un véhicule ou un excipient pharmaceutiquement acceptable.

15. Le composé selon l'une quelconque des revendications 1 à 13 destiné à être utilisé dans un procédé d'inhibition de l'activité de la kinase ALPK1 dans une cellule ou un tissu d'un sujet ayant besoin d'une telle thérapie, ledit procédé comprenant l'administration du composé au sujet.

16. Le composé selon l'une quelconque des revendications 1 à 13 destiné à être utilisé dans un procédé d'inhibition ou de réduction de l'inflammation dans un tissu cible d'un sujet nécessitant un tel traitement, ledit procédé comprenant l'administration du composé au sujet.

17. Le composé selon l'une quelconque des revendications 1 à 13 destiné à être utilisé dans un procédé de traitement d'une maladie, d'un trouble ou d'une affection **caractérisée par** une signalisation pro-inflammatoire excessive ou inappropriée dépendante d'ALPK1 chez un sujet nécessitant une telle thérapie, ledit procédé comprenant l'administration du composé au sujet
éventuellement dans lequel la maladie, le trouble ou l'affection sont choisi parmi septicémie, cancer, spiroadénome, spiroadénocarcinome, syndrome de «Dystrophie rétinienne, Œdème du nerf optique, Splénomégalie, Anhidrose et Céphalée migraineuse» («ROSAH»), et syndrome de «Fièvre périodique, Stomatite aphteuse, Pharyngite et Adénite» («PFAPA»).

18. Le composé selon l'utilisation de la revendication 17, dans lequel le sujet nécessitant une telle thérapie est un sujet porteur d'une ou plusieurs mutations génétiques d'ALPK1.
